(19) Europäisches Patentamt / European Patent Office / Office européen des brevets

(11) **EP 1 354 887 B1**

(12) **EUROPEAN PATENT SPECIFICATION**

(45) Date of publication and mention of the grant of the patent: **25.04.2007 Bulletin 2007/17**

(51) Int Cl.: *C07F 9/50* (2006.01) *C07C 209/10* (2006.01) *C07C 41/16* (2006.01) *C07C 45/68* (2006.01)

(21) Application number: **03009560.8**

(22) Date of filing: **09.07.1999**

(54) **Ligands for metals and improved metal-catalyzed processes based thereon**

Ligande für Metalle und verbesserte Metall-katalysierte Verfahren, die darauf basieren

Ligands pour métaux et procédés améliorés catalysés par des métaux,basés sur ceux-ci

(84) Designated Contracting States: **AT BE CH CY DE DK ES FI FR GB GR IE IT LI LU MC NL PT SE**

(30) Priority: **10.07.1998 US 113478**
**20.11.1998 US 196855**
**13.01.1999 US 231315**
**27.01.1999 US 239024**

(43) Date of publication of application: **22.10.2003 Bulletin 2003/43**

(62) Document number(s) of the earlier application(s) in accordance with Art. 76 EPC: **99933785.0 / 1 097 158**

(73) Proprietor: **Massachussetts Institute of Technology**
**Cambridge, MA 02139 (US)**

(72) Inventors:
- **Ahman, Jens**
  **Sandwich,**
  **Kent CT139NJ (GB)**
- **Wolfe, John, P.**
  **Ann Arbor, MI 48103-2076 (US)**
- **Kamikawa, Ken**
  **Sakai, Osaka 599-8531 (JP)**
- **Sadighi, Joseph, P**
  **Cambridge, MA 02139 (US)**
- **Old, David, W**
  **Irvine, CA 92620-3273 (US)**
- **Palucki, Michael**
  **Belle Meade, New Jersey 08502 (US)**
- **Chieffi, Andrew**
  **Somerville, Massachusetts 02143 (US)**
- **Singer, Robert, A.**
  **New York 10028 (US)**
- **Buchwald, Stephen L.**
  **Newton, MA 02558 (US)**

(74) Representative: **Vossius & Partner**
**Siebertstrasse 4**
**81675 München (DE)**

(56) References cited:
**WO-A-98/15515**

- **JOHN P WOLFE ET AL: "An Improved Catalyst System for Aromatic Carbon-Nitrogen Bond Formation: The Possible Involvement of Bis (Phosphine) Palladium Complexes as Key Intermediates" JOURNAL OF THE AMERICAN CHEMICAL SOCIETY, AMERICAN CHEMICAL SOCIETY, WASHINGTON, DC, US, vol. 118, no. 30, 31 July 1996 (1996-07-31), pages 7215-7216, XP002082634 ISSN: 0002-7863**
- **PALUCKI M ET AL: "PALLADIUM-CATALYZED INTERMOLECULAR CARBON-OXYGEN BOND FORMATION: A NEW SYNTHESIS OF ARYL ETHERS" JOURNAL OF THE AMERICAN CHEMICAL SOCIETY, AMERICAN CHEMICAL SOCIETY, WASHINGTON, DC, US, vol. 119, no. 14, 9 April 1997 (1997-04-09), pages 3395-3396, XP002053323 ISSN: 0002-7863**
- **WOLFE J P: "Highly active palladium catalysts for Suzuki coupling reactions" JOURNAL OF THE AMERICAN CHEMICAL SOCIETY, AMERICAN CHEMICAL SOCIETY, WASHINGTON, DC, US, vol. 121, no. 41, 20 October 1999 (1999-10-20), pages 9550-9561, XP002132767 ISSN: 0002-7863**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- OLD D W ET AL: "A HIGHLY ACTIVE CATALYST FOR PALLADIUM-CATALYZED CROSS-COUPLING REACTIONS: ROOM-TEMPERATURE SUZUKI COUPLINGS AND AMINATION OF UNACTIVATED ARYL CHLORIDES" JOURNAL OF THE AMERICAN CHEMICAL SOCIETY, AMERICAN CHEMICAL SOCIETY, WASHINGTON, DC, US, vol. 120, no. 37, 23 September 1998 (1998-09-23), pages 9722-9723, XP000776234 ISSN: 0002-7863
- DING K: "Highly efficient and practical optical resolution of 2-amino-2'- hydroxy-1,1'-binaphtyl by molecular complexation with N-benzylcinchonidium chloride" CHEMISTRY - A EUROPEAN JOURNAL, VCH PUBLISHERS, US, vol. 5, no. 6, June 1999 (1999-06), pages 1734-1737, XP002124322 ISSN: 0947-6539
- VYSKOCIL S: "Derivatives of 2-amino-2'-diphenylphosphimo-1,1'-binaphtyl (MAP) and their application in asymmetric Palladium (o)-catalyzrd allylic substitution" JOURNAL OF ORGANIC CHEMISTRY, AMERICAN CHEMICAL SOCIETY. EASTON, US, vol. 63, no. 22, 30 October 1998 (1998-10-30), pages 7738-7748, XP002124321 ISSN: 0022-3263
- ARANYOS A: "Novel electron-rich bulky phosphine ligands facilitate the Palladium-catalyzed preparation of diaryl ethers" JOURNAL OF THE AMERICAN CHEMICAL SOCIETY, AMERICAN CHEMICAL SOCIETY, WASHINGTON, DC, US, vol. 121, no. 18, 12 May 1999 (1999-05-12), pages 4369-4378, XP002124320 ISSN: 0002-7863
- VYSKOCIL S ET AL: "Synthesis of 2-Amino-2'-diphenylphosphino-1,1'-binaphth yl (MAP) and its Accelerating Effect on the Pd(0)-Catalyzed N-Arylation" TETRAHEDRON LETTERS, ELSEVIER SCIENCE PUBLISHERS, AMSTERDAM, NL, vol. 39, no. 50, 10 December 1998 (1998-12-10), pages 9289-9292, XP004142698 ISSN: 0040-4039

## Description

The present invention was made with support provided by the National Institutes of Health and the Office of Naval Research; the government, therefore, has certain rights in the invention.

Transition metal catalyst complexes play important roles in many areas of chemistry, including the preparation of polymers and pharmaceuticals. The properties of these catalyst complexes are recognized to be influenced by both the characteristics of the metal and those of the ligands associated with the metal atom. For example, structural features of the ligands can influence reaction rate, regioselectivity, and stereoselectivity. Electron-withdrawing ligands, in coupling reactions, can be expected to slow oxidative addition to, and speed reductive elimination from, the metal center; and electron-rich ligands, in coupling reactions, conversely, can be expected to speed oxidative addition to, and slow reductive elimination from, the metal center.

In many cases, the oxidative addition step in the accepted mechanism of a coupling reaction is deemed to be rate limiting. Therefore, adjustments to the catalytic system as a whole that increase the rate of the oxidative addition step should increase overall reaction rate. Additionally, the rate of oxidative addition of a transtion metal catalyst to the carbon-halogen bond of an aryl halide is known to decrease as the halide is varied from iodide to bromide to chloride, all other factors being equal. Because of this fact, the most stable, lowest molecular weight, least expensive, and arguably most easy to obtain, members of the set of reactive organic halides - the chlorides - are the poorest substrates for traditional transition metal catalyzed coupling reactions and the like.

To date, the best halogen-containing substrates for transition metal catalyzed carbon-heteroatom and carbon-carbon bond forming reactions have been the iodides. Bromides have often been acceptable substrates, but typically required higher temperatures, longer reaction times, and gave lower yields of products.

Formation of a new carbon-carbon bond at a position $\alpha$ to an electron withdrawing group, i.e. at an activated methyl, methylene or methine carbon, is typically achieved by nucleophilic attack of the conjugate base of the activated carbon, e.g., an enolate or ketene acetal, at an electrophilic carbon, e.g., a carbonyl carbon or a carbon bearing a good leaving group. While this paradigm is effective in a range of contexts, e.g., the aldol condensation and enolate alkylation, its scope, in fact, is limited. For example, existing methods for the formation of a carbon-carbon bond between an $sp^2$-hybridized carbon of an aromatic nucleus and an activated carbon require that the aromatic nucleus be susceptible to nucleophilic aromatic substitution, e.g., that it bear a number of electron withdrawing groups in appropriate positions. Furthermore, corresponding limitations exist in the art vis-a-vis the ability to install a vinyl group at an activated carbon, i.e. the requirement that the vinylating group be predisposed to an addition-elimination mechanism. A general method for the arylation and/or vinylation of activated methyl, methylene, and methine carbons, that utilizes readily available starting materials and affords products in high regioselectivity, is not known in the art.

A. Aranyos et al. disclose electron-rich bulky phosphine ligands which are stated to facilitate the palladium-catalyzed preparation of diaryl ethers (J. Am. Chem. Soc., **1999**, *121*, 4369-4378). Among others a binaphthyl ligand was investigated.

The synthesis of $\alpha$-aryl ketones has received much attention over the past two decades. A number of stoichiometric arylating reagents have been successfully developed for this purpose, however, their utility is decreased because each synthesis of an $\alpha$-aryl ketone requires the synthesis of a different arylating reagent. In contrast, the direct coupling of aryl halides with ketones would provide a convenient method for the synthesis of $\alpha$-aryl ketones. Semmelhack et al. have demonstrated that $Ni(COD)_2$ catalyzes the intramolecular coupling of an aryl iodide with a ketone enolate. While there are reports of Pd or Ni-catalyzed intermolecular coupling reactions that afford $\alpha$-aryl ketones, these methods require the use of stoichiometric amounts of tin reagents, and/or the use of enol ether, enamine or $\alpha$-chloroketone derivatives instead of the ketone. Thus, a general method which utilizes readily available starting materials and affords products in high regioselectivity has not been realized.

The invention is summarized in the following items:

1. The compound represented by general structure 7:

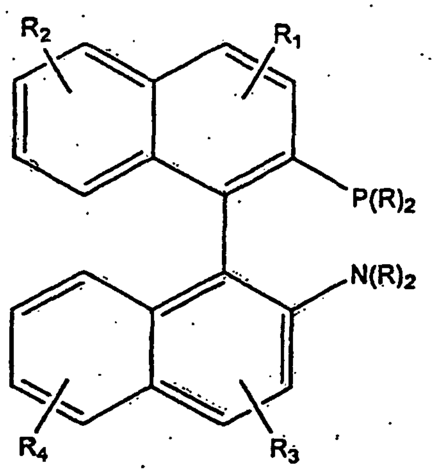

7

wherein

R is selected, independently for each occurrence, from the set consisting of alkyl, heteroalkyl, cycloalkyl, heterocycloalkyl, aryl, heteroaryl, aralkyl, heteroaralkyl, and $-(CH_2)_m-R_{80}$;

the A, B, A', and B' rings of the binaphthyl core independently may be unsubstituted or substituted with $R_1$, $R_2$, $R_3$, and $R_4$, respectively, any number of times up to the limitations imposed by stability and the rules of valence;

$R_1$, $R_2$, $R_3$, and $R_4$, are selected, independently for each occurrence, from the set consisting of alkyl, heteroalkyl, cycloalkyl, heterocycloalkyl, aryl, heteroaryl, aralkyl, heteroaralkyl, halogen, $-SiR_3$, and $-(CH_2)_m-R_{80}$;

$R_{80}$ represents an unsubstituted or substituted aryl, a cycloalkyl, a cycloalkenyl, a heterocycle, or a polycycle;

m is an integer in the range 0 to 8 inclusive; and

the ligand, when chiral, may be provided in the form of a mixture of enantiomers or as a single enantiomer.

2. The process represented by the generalized reaction depicted in Scheme 1:

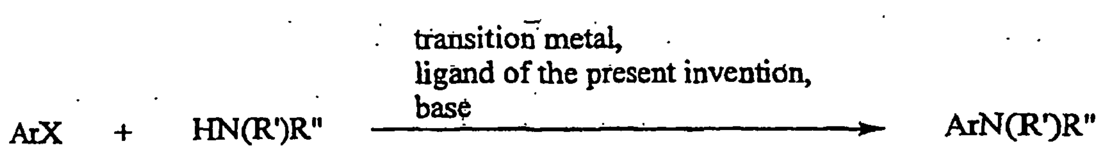


Scheme 1

wherein

Ar is selected from the set consisting of optionally substituted monocyclic and polycyclic aromatic and heteroaromatic moieties;

X is selected from the set consisting of Cl, Br, I, $-OS(O)_2$alkyl, and $-OS(O)_2$aryl;

R' and R" are selected, independently for each occurrence, from the set consisting of H, alkyl, heteroalkyl, aryl, heteroaryl, aralkyl, alkoxyl, amino, trialkylsilyl, and triarylsilyl;

R' and R", taken together, may form an optionally substituted ring consisting of 3-10 backbone atoms inclusive; said ring optionally comprising one or more heteroatoms beyond the nitrogen to which R' and R" are bonded;

R' and/or R" may be covalently linked to Ar such that the animation reaction is intramolecular;

the transition metal is selected from the set of groups 5-12 metals, preferably the Group VIIIA metals;

the ligand is represented by general structure 7; and

the base is selected from the set consisting, of hydrides, carbonates, fluorides, phosphates, alkoxides, phenoxides, amides, carbanions, and silyl anions.

3. The process represented by the generalized coupling reaction depicted in Scheme 2:

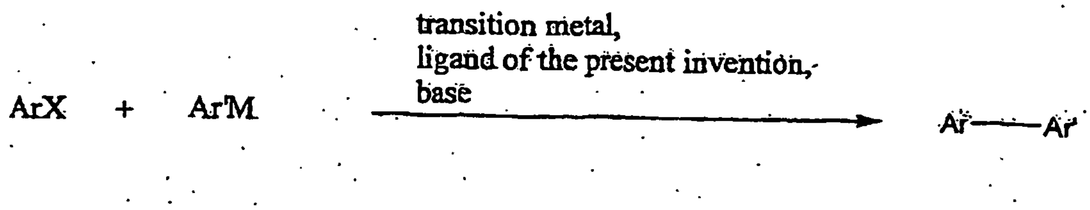


Scheme 2

wherein

Ar and Ar' are independently selected from the set consisting of optionally substituted aromatic, heteroaromatic, and alkenyl moieties;

X is selected from the set consisting of Cl, Br, I, $-OS(O)_2alkyl$, and $-OS(O)_2aryl$;

M represents $B(OR)_2$, Mg(halide), or Zn(halide);

R represents independently for each occurrence H, methyl, alkyl, heteroalkyl, aryl, or heteroaryl; or the two instances of R in an occurrence of $B(OR)_2$, taken together, may represent an optionally substituted two or three carbon tether between the two instances of O;

Ar and Ar' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

4. The process represented by the generalized coupling reaction depicted in Scheme 3:

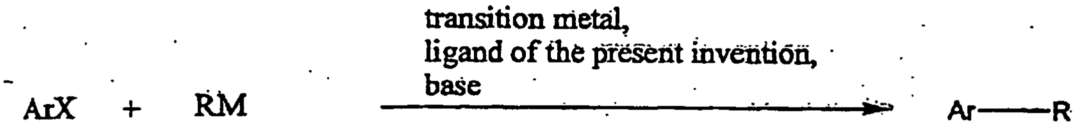


Scheme 3

wherein

Ar is selected from the set consisting of optionally substituted aromatic, heteroaromatic, and alkenyl moieties;

R is selected from the set consisting of optionally substituted alkyl, heteroalkyl, and aralkyl;

R' is selected, independently for each occurrence, from the set of alkyl and heteroalkyl; the carbon-boron bond of said alkyl and heteroalkyl groups being inert under the reaction conditions, e.g., $BR'_2$ taken together represents 9-borobicyclo[3.3.1]nonyl.

M represents $B(R')_2$, Mg(halide), or Zn(balide);

X of ArX is selected from the set consisting of Cl, Br, I, $-OS(O)_2$alkyl, and $-OS(O)_2$aryl;

Ar and R may be covalently linked such that the reaction is intramolecular;

the transition metal is selected from the set of groups 5-12 metals; preferably the Group VIIIA metals;

the ligand is represented by general structure 7; and

the base is selected from the set consisting of carbonates; phosphates, fluorides, alkoxides, amides, carbanions, and silyl anions.

5. The process represented by the generalized $\alpha$-arylation reaction depicted in Scheme 4:

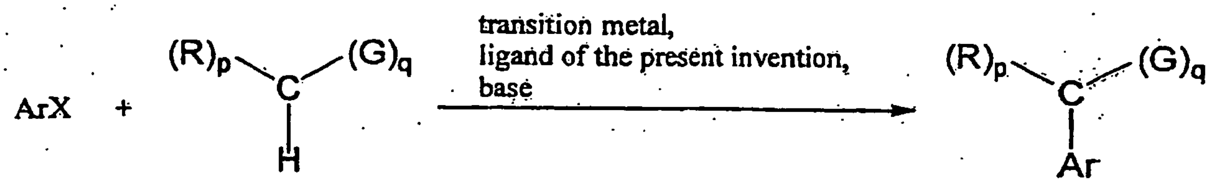


Scheme 4

wherein

Ar is selected from the set consisting of optionally substituted monocyclic and polycyclic aromatic and heteroaromatic moieties;

X is selected from the set consisting of Cl, Br, I, $-OS(O)_2$alkyl, and $-OS(O)_2$aryl;

G represents, independently for each occurrence, an electron withdrawing group selected from the group consisting of formyl, acyl, -CN, -C(O)OR, $-C(O)NR_2$, nitro, nitroso, $-S(O)_2R$, $-SO_3R$, $-S(O)_2NR_2$, -C(NR)-R, -C(NOR)-R, and $-C(NNR_2)-R$;

R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

q is an integer selected from the range 1 to 3 inclusive;

p is an integer equal to (3-q).

Ar and one instance of R may be covalently linked such that the reaction is intramolecular,

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

6. The process represented by the generalized α-vinylation reaction depicted in Scheme 5:

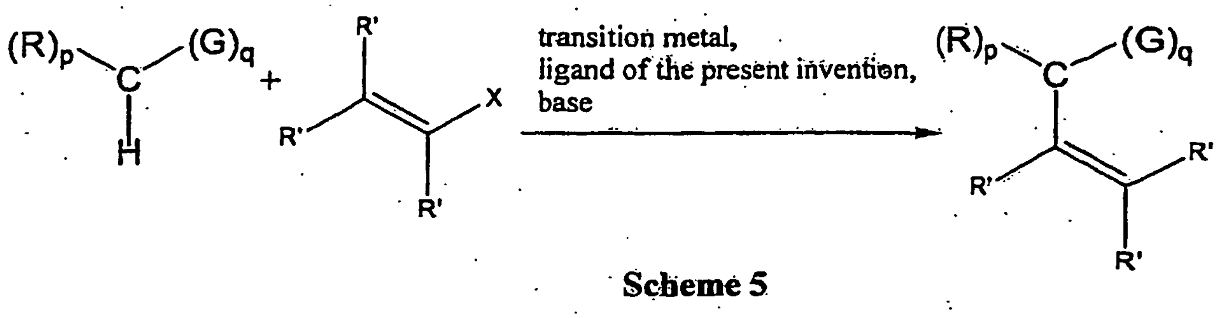


Scheme 5

wherein

X is selected from the set consisting of Cl, Br, I, $-OS(O)_2$alkyl, and $-OS(O)_2$aryl;
G represents, independently for each occurrence, an electron withdrawing group selected from the group consisting of formyl, acyl, -CN, -C(O)OR, $-C(O)NR_2$, nitro, nitroso, $-S(O)_2R$, $-SO_3R$, $-S(O)_2NR_2$, -C(NR)-R, -C(NOR)-R, and $-C(NNR)_2$-R,
R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;
R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
q is an integer selected from the range 1 to 3 inclusive;
p is an integer equal to (3-q).
an instance of R and an instance of R' may be covalently linked such that the reaction is intramolecular,
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

7. The process represented by the generalized O-arylation reaction depicted in Scheme 6:

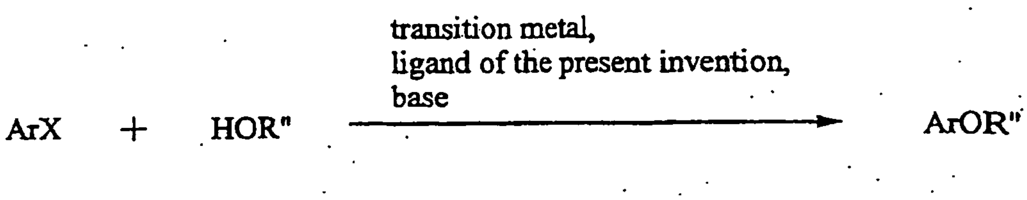


Scheme 6

wherein

Ar is selected from the set consisting of optionally substituted monocyclic and polycyclic aromatic and heteroaromatic moieties;

X is selected from the set consisting of Cl, Br, I, $-OS(O)_2$alkyl, and $-OS(O)_2$aryl;
R" represents, independently for each occurrence, alkyl, aryl, aralkyl, heteroalkyl, heteroaryl, heteroaralkyl, -Si (alkyl)$_3$, -Si(aryl)$_3$, or $-(CH2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
Ar and R" may be covalently linked such that the reaction is intramolecular;
the transition metal is selected from the set of groups 5-12 metals, preferably the Group VIIIA metals;
the ligand is represented by general structure 7;
the base is selected from the set consisting of carbonates, phosphates, fluorides, alkoxides, amides, carbanions, and silyl anions; and
the process is conducted at room temperature.

8. The process represented by the generalized O-vinylation reaction depicted in Scheme 7:

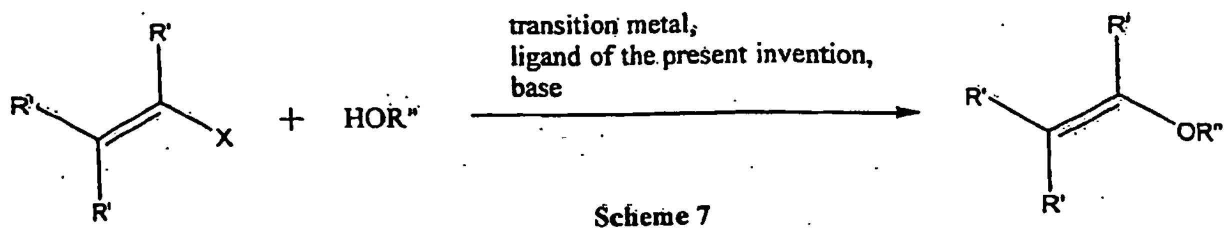


Scheme 7

wherein

X is selected from the set consisting of Cl, Br, I, $-OS(O)_2$alkyl, and $-OS(O)_2$aryl;

R' represents, independently for each occurrence, hydrogen, alkyl aryl, aralkyl, heteroalkyl, heteroaryl, heteroaralkyl, alkylamino, arylamino, alkylthio, arylthio, alkoxy, aryloxy, or $-(CH_2)_m-R_{80}$,

R" represents, independently for each occurrence, alkyl, aryl, aralkyl, heteroalkyl, heteroaryl, heteroaralkyl, -Si (alkyl)$_3$, -Si(aryl)$_3$, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

R" and an instance of R' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

9. The process represented by the generalized Heck reaction depicted in Scheme 8:

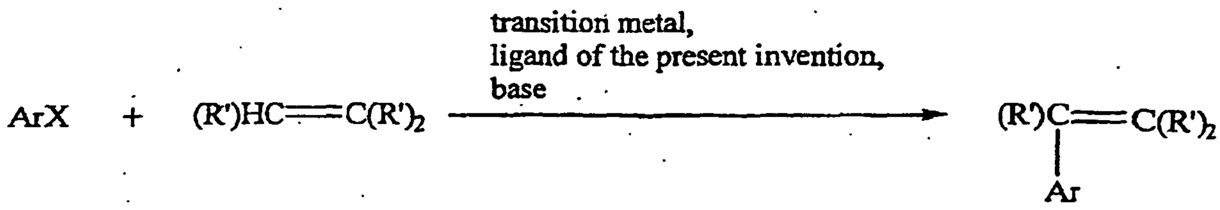


**Scheme 8**

wherein

Ar is selected from the set consisting of optionally substituted aromatic, heteroaromatic, and alkenyl moieties;

X is selected from the set consisting of Cl, Br, I, $-OS(O)_2$alkyl, and $-OS(O)_2$aryl;

R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

Ar and an instance of R' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

10. The process represented by the generalized Heck reaction depicted in Scheme 9:

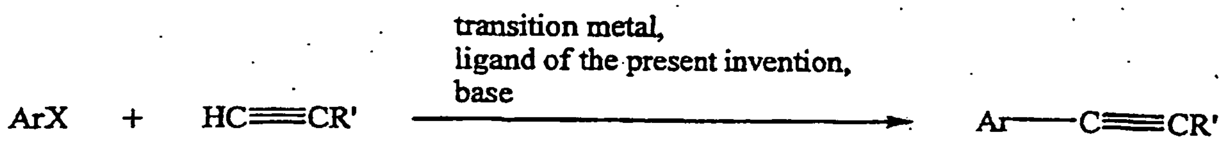


**Scheme 9**

wherein

Ar is selected from the set consisting of optionally substituted aromatic, heteroaromatic, and alkenyl moieties;

X is selected from the set consisting of Cl, Br, I, $-OS(O)_2$alkyl, and $-OS(O)_2$aryl;

R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl heterocycle or polycycle;

m, independently for each occurrence, is an integer selected from the range 0 to 8 inclusive;

Ar and R' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

The methods of the present invention, which use catalysts comprising ligands represented by general structure 7 in transition metal catalyzed carbon-heteroatom and carbon-carbon bond-forming reactions, provide improvements in many features of the transition metal-catalyzed reactions, including the range of suitable substrates, number of catalyst turnovers, reaction conditions, and efficiency.

Unexpected, pioneering improvements over the prior art have been realized in transition metal-catalyzed aryl amination reactions, Suzuki couplings to give both biaryl and alkylaryl products, arylations and vinylations at the position $\alpha$ to carbonyl and related functional groups, and carbon-oxygen bond formation. The ligands and methods of the present invention enable for the first time, the general and efficient use of aryl chlorides in the aforementioned reactions. Additionally, the ligands and methods of the present invention enable for the first time transformations utilizing aryl bromides or chlorides to proceed efficiently at room temperature. Furthermore, the ligands and methods of the present invention enable the aforementioned reactions to occur at synthetically useful rates using extremely small amounts of catalyst, e.g., 0.000001 mol% relative to the limiting reagent.

**Figure 1** depicts the method of preparation and reactions screened for certain ligands.

**Figure 2** depicts ligands pertaining to Figures 3-11 and Examples 59-65.

**Figure 3** depicts certain room temperature Pd-catalyzed aminations of aryl chlorides.

**Figure 4** depicts cartain Pd-catalyzed aminations of unactivated aryl chlorides.

**Figure 5** depicts certain Pd-catalyzed aminations of unactivated aryl chlorides.

**Figure 6** depicts certain Pd-catalyzed aminations of aryl chlorides at low catalyst loading.

**Figure 7** depicts certain Pd-catalyzed aminations of chloropyridines.

**Figure 8** depicts certain Pd-catalyzed aminations of functionalized aryl chlorides.

**Figure 9** depicts certain Pd-catalyzed aminations of aryl bromides.

**Figure 10** depicts the results of a Pd-catalyzed amination using one equivalent each (relative to the amine) of 4-bromo- and 4-chlorotoluene.

**Figure 11** depicts certain Pd-catalyzed aminations of aryl triflates.

**Figure 12** depicts certain Pd-catalyzed one-pot syntheses of triarylamines.

**Figure 13** depicts certain Pd-catalyzed one-pot syntheses of triarylamines.

**Figure 14** depicts the results of a Pd-catalyzed amination using a mixture of three congeneric aryl amines and three aryl bromides.

**Figure 15** depicts certain Pd-catalyzed arylations of diphenylamine.

**Figure 16** depicts certain Pd-catalyzed arylations of cyclopropylamine.

**Figure 17** depicts certain Pd-catalyzed arylations of indole.

**Figure 18** depicts certain ligands.

**Figure 19** depicts certain ligands.

**Figure 20** depicts certain Pd-catalyzed α-arylations of ketones.

**Figure 21** depicts certain Pd-catalyzed arylations of various substituted indoles.

**Figure 22** depicts certain Pd-catalyzed arylations of indole.

**Figure 23** depicts certain Pd-catalyzed arylations of sodium *tert*-butoxide.

**Figure 24** depicts certain Pd-catalyzed arylations of sodium *tert*-butoxide.

**Figure 25** depicts certain Pd-catalyzed arylations of sodium *tert*-butyldimethylsilyloxide.

**Figure 26** depicts certain Pd-catalyzed asymetric α-arylations of ketones.

**Figure 27** depicts the enantiomeric excess obtained in the Pd-catalyzed asymmetric α-arylation of 2-methyl-1-tetralone with an aryl bromide as a function of the ligand utilized.

**Figure 28** depicts certain Pd-catalyzed α-arylations of ketones conducted in the absence of phosphine ligands.

The subject ligands are represented by general structure 7:

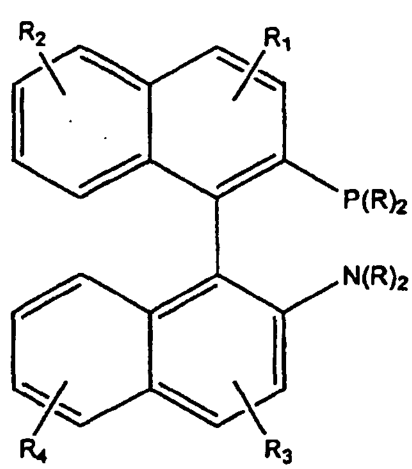

7

wherein

R is selected, independently for each occurrence, from the set consisting of alkyl, heteroalkyl, cycloalkyl, heterocycloalkyl, aryl, heteroaryl, aralkyl, heteroaralkyl, and $-(CH2)_m-R_{80}$;

the A, B, A', and B' rings of the binaphthyl core independently may be unsubstituted or substituted with $R_1$, $R_2$, $R_3$, and $R_4$, respectively, any number of times up to the limitations imposed by stability and the rules of valence;

$R_1$, $R_2$, $R_3$, and $R_4$, are selected, independently for each occurrence, from the set consisting of alkyl, heteroalkyl, cycloalkyl, heterocycloalkyl, aryl, heteroaryl, aralkyl, heteroaralkyl, halogen, $-SiR_3$, and $-(CH_2)_m-R_{80}$;

$R_{80}$ represents an unsubstituted or substituted aryl, a cycloalkyl, a cycloalkenyl, a heterocycle, or a polycycle;

m is an integer in the range 0 to 8 inclusive; and

the ligand, when chiral, may be provided in the form of a mixture of enantiomers or as a single enantiomer.

In certain embodiments, the ligands are represented by general structure **7**, and the associated definitions, wherein:

$R_1$, $R_2$, $R_3$, and $R_4$, are absent;

both instances of R on the N depicted explicitly are lower alkyl, preferably methyl; and

both instances of R on P depicted explicitly are cycloalkyl, preferably cyclohexyl.

In certain embodiments, the ligands are represented by general structure **7**, and the associated definitions, wherein the $PR_2$ group comprises an asymmetric P.
In certain embodiments, the ligands are represented by general structure **7**, and the associated definitions, wherein the $PR_2$ group comprises an asymmetric P; and the binaphthyl core is axially chiral.
In certain embodiments, the subject method is represented by the generalized reaction depicted in Scheme 1:

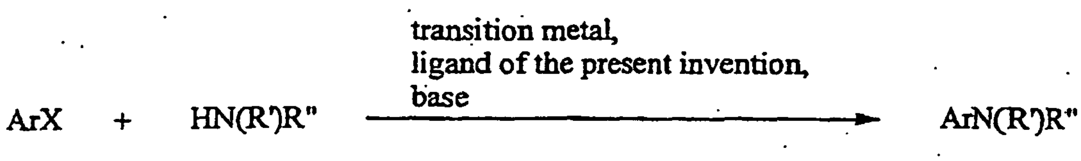


**Scheme 1**

wherein

Ar is selected from the set consisting of optionally substituted monocyclic and polycyclic aromatic and heteroaromatic moieties;

X is selected from the set consisting of Cl, Br, I, $-OS(O_2)$alkyl, and $-OS(O)_2$aryl;

R' and R" are selected, independently for each occurrence, from the set consisting of H, alkyl, heteroalkyl, aryl, heteroaryl, aralkyl, alkoxyl, amino, trialkylsilyl, and triarylsilyl;

R' and R", taken together, may form an optionally substituted ring consisting of 3-10 backbone atoms inclusive, said ring optionally comprising one or more heteroatoms beyond the nitrogen to which R' and R" are bonded;

R' and/or R" may be covalently linked to Ar such that the animation reaction is intramolecular,

the transition metal is selected from the set of groups 5-12 metals, preferably the Group VIBA metals;

the ligand is represented by general structure 7; and

the base is selected from the set consisting of hydrides, carbonates, fluorides, phosphates, alkoxides, phenoxides, amides, carbanions, and silyl anions.

Those of ordinary skill in the art will recognize that in the described embodiments based on Scheme 1, (alkenyl) X may serve as a surrogate for ArX.
In certain embodiments, the subject method is represented by Scheme 1 and the associated definitions, wherein the ligand is covalently linked to a solid support or soluble polymer, or is adsorbed onto a solid.
In certain embodiments, the subject method is represented by Scheme 1 and the associated definitions, wherein:

X of ArX is Cl, $-OS(O)_2$alkyl, or $-OS(O)_2$aryl; and

the method is conducted at room temperature.

In certain embodiments, the subject method is represented by Scheme 1 and the associated definitions, wherein the transition metal is palladium; and the base is a phosphate or fluoride.

In certain embodiments, the subject method is represented by Scheme 1 and the associated definitions, wherein the transition metal is palladium; and the base is potassium phosphate.

In certain embodiments, the subject method is represented by Scheme 1 and the associated definitions, wherein: HN(R')R" represents an optionally substituted heteroaromatic compound.

In certain embodiments, the subject method is represented by Scheme 1 and the associated definitions, wherein the product is provided in a yield of greater than 50%.

In certain embodiments, the subject method is represented by Scheme 1 and the associated definitions, wherein the product is provided in a yield of greater than 70%.

In certain embodiments, the subject method is represented by Scheme 1 and the associated definitions, wherein the product is provided in a yield of greater than 85%.

In certain embodiments, the subject method is represented by Scheme 1 and the associated definitions, wherein the method is conducted at room temperature.

In certain embodiments, the subject method is represented by Scheme 1 and the associated definitions, wherein X, of ArX is chloride.

In certain embodiments, the subject method is represented by Scheme 1 and the associated definitions, wherein X of ArX is chloride; and the process is conducted at a temperature less than about 100°C.

In certain embodiments, the subject method is represented by Scheme 1 and the associated definitions, wherein X of ArX is chloride; and the process is conducted at a temperature less than about 80°C.

In certain embodiments, the subject method is represented by Scheme 1 and the associated definitions, wherein X of ArX is chloride; and the process is conducted at room temperature.

In certain embodiments, the subject method is represented by Scheme 1 and the associated definitions, wherein less than 0.01 mol% of the catalyst relative to the limiting reagent is utilized.

In certain embodiments, the subject method is represented by Scheme 1 and the associated definitions, wherein less than 0.0001 mol% of the catalyst relative to the limiting reagent is utilized.

In certain embodiments, the subject method is represented by Scheme 1 and the associated definitions, wherein less than 0.000001 mol% of the catalyst relative to the limiting reagent is utilized.

In certain embodiments, the subject method is represented by Scheme 1 and the associated definitions, wherein the limiting reagent is consumed in less than 48 hours.

In certain embodiments, the subject method is represented by Scheme 1 and the associated definitions, wherein the limiting reagent is consumed in less than 24 hours.

In certain embodiments, the subject method is represented by Scheme 1 and the associated definitions, wherein the limiting reagent is consumed in less than 12 hours.

In certain embodiments, the subject method is represented by Scheme 1 and the associated definitions, wherein the method is not set-up or performed or both under an inert atmosphere.

In certain embodiments, the subject method is represented by Scheme 1 and the associated definitions, wherein the method is not set-up or performed or both under anhydrous conditions.

In certain embodiments, the subject method is represented by Scheme 1 and the associated definitions, wherein the method is not set-up or performed or both under an oxygen-free atmosphere.

In certain embodiments, the subject method is represented by the generalized coupling reaction depicted in Scheme 2:

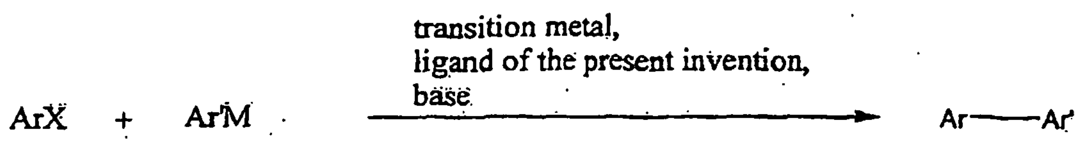


Scheme 2

wherein

Ar and Ar' are independently selected from the set consisting of optionally substituted aromatic, heteroaromatic, and alkenyl moieties;

X is selected from the set consisting of Cl, Br, I, -OS(O)$_2$alkyl, and -OS(O)$_2$aryl;

M. represents B(OR)$_2$, Mg(halide), or Zn(halide);

R represents independently for each occurrence H, methyl, alkyl, heteroalkyl, aryl, or heteroaryl; or the two instances of R in an occurrence of B(OR)$_2$, taken together, may represent an optionally substituted two or three carbon tether between the two instances of O;

Ar and Ar' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme 2 and the associated definitions, wherein the ligand is covalently linked to a solid support or soluble polymer, or is adsorbed onto a solid.

In certain embodiments, the subject method is represented by Schema 2 and the associated definitions, wherein the ligand is chiral and non-racemic;, and the product is not racemic.

In certain embodiments, the subject method is represented by Scheme 2 and the associated definitions, wherein M represents B(OR)$_2$.

In certain embodiments, the subject method is represented by Scheme 2 and the associated definitions, wherein:

X of ArX is Cl, -OS(O)$_2$alkyl, or -OS(O)$_2$aryl; and

the method is conducted at room temperature.

In certain embodiments, the subject method is represented by Scheme 2 and the associated definitions, wherein the transition metal is palladium; and
the base is a phosphate or fluoride.

In certain embodiments, the subject method is represented by Scheme 2 and the associated definitions, wherein:

the transition metal is palladium; and

the base is potassium phosphate.

In certain embodiments, the subject method is represented by Scheme 2 and the associated definitions, wherein the product is provided in a yield of greater than 50%.

In certain embodiments, the subject method is represented by Scheme 2 and the associated definitions, wherein the product is provided in a yield of greater than 70%.

In certain embodiments, the subject method is represented by Scheme 2 and the associated definitions, wherein the product is provided in a yield of greater than 85%.

In certain embodiments, the subject method is represented by Scheme 2 and the associated definitions, wherein the method is conducted at room temperature.

In certain embodiments, the subject method is represented by Scheme 2 and the associated definitions, wherein X of ArX is chloride.

In certain embodiments, the subject method is represented by Scheme 2 and the associated definitions, wherein X of ArX is chloride; and the process is conducted at a temperature less than about 100˚C.

In certain embodiments, the subject method is represented by Scheme 2 and the associated definitions, wherein X of ArX is chloride; and the process is conducted at a temperature less than about 80˚C.

In certain embodiments, the subject method is represented by Scheme 2 and the associated definitions, wherein X of ArX is chloride; and the process is conducted at room temperature.

In certain embodiments, the subject method is represented by Scheme 2 and the associated definitions, wherein

no more than one of the four ortho and ortho' substitutents of Ar-Ar' is hydrogen.

In certain embodiments, the subject method is represented by Scheme 2 and the associated definitions, wherein X of ArX is chloride; and no more than one of the four ortho and ortho' substitutents of Ar-Ar' is hydrogen.

In certain embodiments, the subject method is represented by Scheme 2 and the associated definitions, wherein less than 0.01 mol% of the catalyst relative to the limiting reagent is utilized.

In certain embodiments, the subject method is represented by Scheme 2 and the associated definitions, wherein less than 0.0001 mol% of the catalyst relative to the limiting reagent is utilized.

In certain embodiments, the subject method is represented by Scheme 2 and the associated definitions, wherein less than 0.000001 mol% of the catalyst relative to the limiting reagent is utilized.

In certain embodiments, the subject method is represented by Scheme 2 and the associated definitions, wherein the limiting reagent is consumed in less than 48 hours.

In certain embodiments, the subject method is represented by Scheme 2 and the associated definitions, wherein the limiting reagent is consumed in less than 24 hours.

In certain embodiments, the subject method is represented by Scheme 2 and the associated definitions, wherein the limiting reagent is consumed in less than 12 hours.

In certain embodiments, the subject method is represented by Scheme 2 and the associated definitions, wherein the method is not set-up or performed or both under an inert atmosphere.

In certain embodiments, the subject method is represented by Scheme 2 and the associated definitions, wherein the method is not set-up or performed or both under anhydrous conditions.

In certain embodiments, the subject method is represented by Scheme 2 and the associated definitions, wherein the method is not set-up or performed or both under an oxygen-free atmosphere.

In certain embodiments, the subject method is represented by the generalized coupling reaction depicted in Scheme 3:

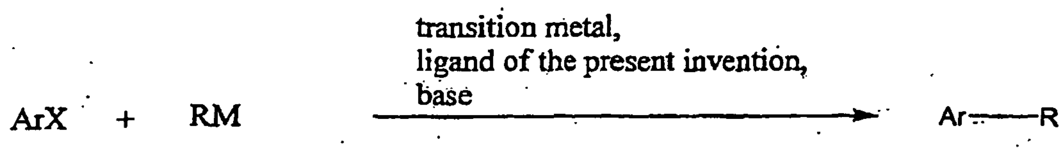


Scheme 3

wherein

Ar is selected from the set consisting of optionally substituted aromatic, heteroaromatic, and alkenyl moieties;

R is selected from the set consisting of optionally substituted alkyl, heteroalkyl, and aralkyl;

R' is selected, independently for each occurrence; from the set of alkyl and heteroalkyl; the carbon-boron bond of said alkyl and heteroalkyl groups being inert under the reaction conditions, e.g., $BR'_2$ taken together represents 9-borobicyclo[3.3.1]nonyl.

M represents $B(R')_2$, Mg(halide), or Zn(halide);

X of ArX is selected from the set consisting of Cl, Br, I, $-OS(O)_2$alkyl, and $-OS(O)_2$aryl;

Ar and R may be covalently linked such that the reaction is intramolecular;

the transition metal is selected from the set of groups 5-12. metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme 3 and the associated definitions, wherein the ligand is covalently linked to a solid support or soluble polymer, or is adsorbed onto a solid.

In certain embodiments, the subject method is represented by Scheme 3 and the associated definitions, wherein the ligand is chiral and non-racemic; and the product is not racemic.

In certain embodiments, the subject method is represented by Scheme 3 and the associated definitions, wherein M represents $B(R')_2$.

In certain embodiments, the subject method is represented by Scheme 3 and the associated definitions, wherein:

X of ArX is Cl, $-OS(O)_2$alkyl, or $-OS(O)_2$aryl; and

the method is conducted at room temperature.

In certain embodiments, the subject method is represented by Scheme 3 and the associated definitions, wherein the transition metal is palladium; and
the base is a phosphate or fluoride.
In certain embodiments, the subject method is represented by Scheme 3 and the associated definitions, wherein the transition metal is palladium; and
the base is potassium phosphate.
In certain embodiments, the subject method is represented by Scheme 3 and the associated definitions, wherein the product is provided in a yield of greater than 50%.
In certain embodiments, the subject method is represented by Scheme 3 and the associated definitions, wherein the product is provided in a yield of greater than 70%.
In certain embodiments, the subject method is represented by Scheme 3 and the associated definitions, wherein the product is provided in a yield of greater than 85%.
In certain embodiments, the subject method is represented by Scheme 3 and the associated definitions, wherein the method is conducted at room temperature.
In certain embodiments, the subject method is represented by Scheme 3 and the associated definitions, wherein X of ArX is chloride.
In certain embodiments, the subject method is represented by Scheme 3 and the associated definitions, wherein X of ArX is chloride; and the process is conducted at a temperature less than about 100°C.
In certain embodiments, the subject method is represented by Scheme 3 and the associated definitions, wherein X of ArX is chloride; and the process is conducted at a temperature less than about 80°C.
In certain embodiments, the subject method is represented by Scheme 3 and the associated definitions, wherein X of ArX is chloride; and the process is conducted at room temperature.
In certain embodiments, the subject method is represented by Scheme 3 and the associated definitions, wherein less than 0.01 mol% of the catalyst relative to the limiting reagent is utilized.
In certain embodiments, the subject method is represented by Scheme 3 and the associated definitions, wherein less than 0.0001 mol% of the catalyst relative to the limiting reagent is utilized.
In certain embodiments, the subject method is represented by Scheme 3 and the associated definitions, wherein less than 0.000001 mol% of the catalyst relative to the limiting reagent is utilized.
In certain embodiments, the subject method is represented by Scheme 3 and the associated definitions, wherein the limiting reagent is consumed in less than 48 hours.
In certain embodiments, the subject method is represented by Scheme 3 and the associated definitions, wherein the limiting reagent is consumed in less than 24 hours.
In certain embodiments, the subject method is represented by Scheme 3 and the associated definitions, wherein the limiting reagent is consumed in less than 12 hours.
In certain embodiments, the subject method is represented by Scheme 3 and the associated definitions, wherein the method is not set-up or performed or both under an inert atmosphere.
In certain embodiments, the subject method is represented by Scheme 3 and the associated definitions, wherein the method is not set-up or performed or both under anhydrous conditions.
In certain embodiments, the subject method is represented by Scheme 3 and the associated definitions, wherein the method is not set-up or performed or both under an oxygen-free atmosphere.
In certain embodiments, the subject method is represented by the generalized α-arylation reaction depicted in Scheme 4:

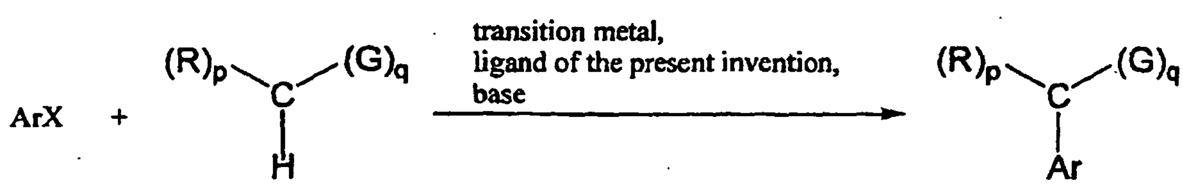


**Scheme 4**

wherein

Ar is selected from the set consisting of optionally substituted monocyclic and polycyclic aromatic and heteroaromatic moieties;

X is selected from the set consisting of Cl, Br, I, $-OS(O)_2$alkyl, and $-OS(O)_2$aryl;

G represents, independently for each occurrence, an electron withdrawing group selected from the group consisting of formyl, acyl, -CN, -C(O)OR, $-C(O)NR_2$, nitro, nitroso,$-S(O)_2R$, $-SO_3R$, $-S(O)_2NR_2$, -C(NR)-R, -C(NOR)-R, and -C $(NNR_2)$-R;

R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

q is an integer selected from the range 1 to 3 inclusive;

p is an integer equal to (3-q).

Ar and one instance of R may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme 4 and the associated definitions, wherein the ligand is covalently linked to a solid support or soluble polymer, or is adsorbed onto a solid.

In certain embodiments, the subject method is represented by Scheme 4 and the associated definitions, wherein the product is asymmetric and has an enantiomeric excess greater than about 50%.

In certain embodiments, the subject method is represented by Scheme 4 and the associated definitions, wherein the product is asymmetric and has an enantiomeric excess greater than about 70%.

In certain embodiments; the subject method is represented by Scheme 4 and the associated definitions, wherein the product is asymmetric and has an enantiomeric excess greater than about 90%.

In certain embodiments, the subject method is represented by Scheme 4 and the associated definitions, wherein the product is asymmetric and has an enantiomeric excess greater than about 95%.

In certain embodiments; the subject method is represented by Scheme 4 and the associated definitions, wherein the product is asymmetric and has an enantiomeric excess greater than about 50%; the transition metal is Pd; and less than 1.0 mol% of the catalyst relative to the limiting reagent is utilized.

In certain embodiments, the subject method is represented by Scheme 4 and the associated definitions, wherein the product is asymmetric and has an enantiomeric excess greater than about 70%; the transition metal is Pd; and less than 1.0 mol% of the catalyst relative to the limiting reagent is utilized.

In certain embodiments, the subject method is represented by Scheme 4 and the associated definitions, wherein the product is asymmetric and has an enantiomeric excess greater than about 90%; the transition metal is Pd; and less than 1.0 mol% of the catalyst relative to the limiting reagent is utilized.

In certain embodiments, the subject method is represented by Scheme 4 and the associated definitions, wherein the product is asymmetric and has an enantiomeric excess greater than about 95%; the transition metal is Pd; and less than 1.0 mol% of the catalyst relative to the limiting reagent is utilized.

In certain embodiments, the subject method is represented by Scheme 4 and the associated definitions, wherein the ligand is chiral and non-racemic; and the product is not racemic.

In certain embodiments, the subject method is represented by Scheme 4 and the associated definitions, wherein the ligand is chiral and non-racemic; the product is not racemic; and the product has an enantiomeric excess greater than about 50%.

In certain embodiments, the subject method is represented by Scheme 4 and the associated definitions, wherein the ligand is chiral and non-racemic; the product is not racemic; and the product has an enantiomeric excess greater than about 70%.

In certain embodiments, the subject method is represented by Scheme 4 and the associated definitions, wherein

the ligand is chiral and non-racemic; the product is not racemic; and the product has an enantiomeric excess greater than about 90%.

In certain embodiments, the subject method is represented by Scheme 4 and the associated definitions, wherein the ligand is chiral and non-racemic; the product is not racemic; and the product has an enantiomeric excess greater than about 95%.

In certain embodiments, the subject method is represented by Scheme 4 and the associated definitions, wherein the ligand is chiral and non-racemic; the product is not racemic; and the product has an enantiomeric excess greater than about 50%; the transition metal is Pd; and less than 1.0 mol% of the catalyst relative to the limiting reagent is utilized.

In certain embodiments, the subject method is represented by Scheme 4 and the associated definitions, wherein the ligand is chiral and non-racemic; the product is not racemic; and the product has an enantiomeric excess greater than about 70%; the transition metal is Pd; and less than 1.0 mol% of the catalyst relative to the limiting reagent is utilized.

In certain embodiments, the subject method is represented by Scheme 4 and the associated definitions, wherein the ligand is chiral and non-racemic; the product is not racemic; and the product has an enantiomeric excess greater than about 90%; the transition metal is Pd; and less than 1.0 mol% of the catalyst relative to the limiting reagent is utilized.

In certain embodiments, the subject method is represented by Scheme 4 and the associated definitions, wherein the ligand is chiral and non-racemic; the product is not racemic; and the product has an enantiomeric excess greater than about 95%; the transition metal is Pd; and less than 1.0 mol% of the catalyst relative to the limiting reagent is utilized.

In certain embodiments, the subject method is represented by Scheme 4 and the associated definitions, wherein q is 1.

In certain embodiments, the subject method is represented by Scheme 4 and the associated definitions, wherein q is 2.

In certain embodiments, the subject method is represented by Scheme 4 and the associated definitions, wherein:

X of ArX is Cl, $OS(O)_2$alkyl, or -$OS(O)_2$aryl; and

the method is conducted at room temperature.

In certain embodiments, the subject method is represented by Scheme 4 and the associated definitions, wherein the transition metal is palladium; and
the base is a phosphate or fluoride.

In certain embodiments, the subject method is represented by Scheme 4 and the associated definitions, wherein the transition metal is palladium; and
the base is potassium phosphate.

In certain embodiments, the subject method is represented by Scheme 4 and the associated definitions, wherein X of ArX represents Br; and
the reaction occurs at room temperature.

In certain embodiments, the subject method is represented by Scheme 4 and the associated definitions, wherein the product is provided in a yield of greater than 50%.

In certain embodiments, the subject method is represented by Scheme 4 and the associated definitions, wherein the product is provided in a yield of greater than 70%.

In certain embodiments, the subject method is represented by Scheme 4 and the associated definitions, wherein the product is provided in a yield of greater than 85%.

In certain embodiments, the subject method is represented by Scheme 4 and the associated definitions, wherein the method is conducted at room temperature.

In certain embodiments, the subject method is represented by Scheme 4 and the associated definitions, wherein X of ArX is chloride.

In certain embodiments, the subject method is represented by Scheme 4 and the associated definitions, wherein X of ArX is chloride; and the process is conducted at a temperature less than about 100˚C.

In certain embodiments, the subject method is represented by Scheme 4 and the associated definitions, wherein X of ArX is chloride; and the process is conducted at a temperature less than about 80˚C.

In certain embodiments, the subject method is represented by Scheme 4 and the associated definitions, wherein X of ArX is chloride; and the process is conducted at room temperature.

In certain embodiments, the subject method is represented by Scheme 4 and the associated definitions, wherein less than 0.01 mol% of the catalyst relative to the limiting reagent is utilized.

In certain embodiments, the subject method is represented by Scheme 4 and the associated definitions, wherein less than 0.0001 mol% of the catalyst relative to the limiting reagent is utilized.

In certain embodiments, the subject method is represented by Scheme 4 and the associated definitions, wherein less than 0.000001 mol% of the catalyst relative to the limiting reagent is utilized.

In certain embodiments, the subject method is represented by Scheme 4 and the associated definitions, wherein the limiting reagent is consumed in less than 48 hours.
In certain embodiments, the subject method is represented by Scheme 4 and the associated definitions, wherein the limiting reagent is consumed in less than 24 hours.
In certain embodiments, the subject method is represented by Scheme 4 and the associated definitions, wherein the limiting reagent is consumed in less than 12 hours.
In certain embodiments, the subject method is represented by Scheme 4 and the associated definitions, wherein the method is not set-up or performed or both under an inert atmosphere.
In certain embodiments, the subject method is represented by Scheme 4 and the associated definitions, wherein the method is not set-up or performed or both under anhydrous conditions.
In certain embodiments, the subject method is represented by Scheme 4 and the associated definitions, wherein the method is not set-up or performed or both under an oxygen-free atmosphere.
In certain embodiments, the subject method is represented by the generalized α-vinylation reaction depicted in Scheme 5:

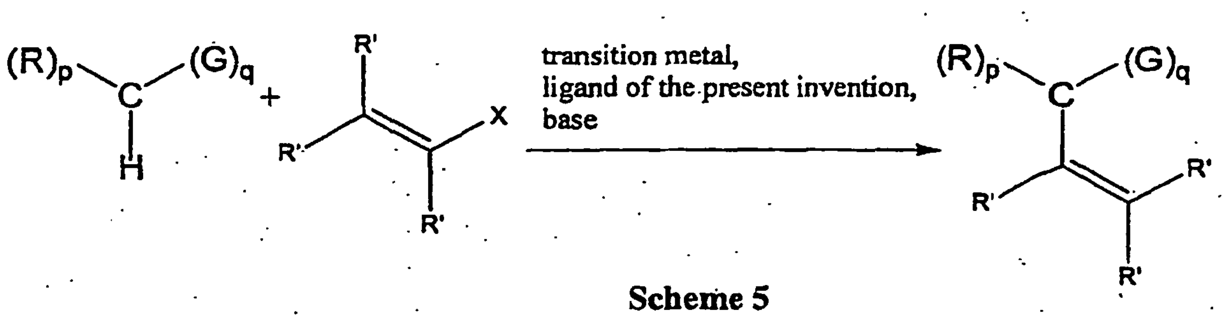


Scheme 5

wherein

X is selected from the set consisting of Cl, Br, I, $-OS(O)_2$alkyl, and $-OS(O)_2$aryl;

G represents, independently for each occurrence, an electron withdrawing group selected from the group consisting of formyl, acyl, -CN, -C(O)OR, $-C(O)NR_2$, nitro, nitroso, $-S(O)_2R$, $-SO_3R$; $-S(O)_2NR_2$, -C(NR)-R, -C(NOR)-R, and $-C(NNR_2)-R$;

R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;

R' represents, independently for each occurrence, hydrogen, alkyl aryl, aralkyl, heteroalkyl, heteroaryl, heteroaralkyl, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

q is an integer selected from the range 1 to 3 inclusive;

p is an integer equal to (3-q).

an instance of R and an instance of R' may be covalently linked such that the reaction is intramolecular,

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions,

and silyl anions.

In certain embodiments, the subject method is represented by Scheme 5 and the associated definitions, wherein the ligand is covalently linked to a solid support or soluble polymer, or is adsorbed onto a solid.

In certain embodiments, the subject method is represented by Scheme 5 and the associated definitions, wherein the product is asymmetric and has an enantiomeric excess greater than about 50%.

In certain embodiments, the subject method is represented by Scheme 5 and the associated definitions, wherein the product is asymmetric and has an enantiomeric excess greater than about 70%.

In certain embodiments, the subject method is represented by Scheme 5 and the associated definitions, wherein the product is asymmetric and has an enantiomeric excess greater than about 90%.

In certain embodiments, the subject method is represented by Scheme 5 and the associated definitions, wherein the product is asymmetric and has an enantiomeric excess greater than about 95%.

In certain embodiments, the subject method is represented by Scheme 5 and the associated definitions, wherein the product is asymmetric and has an enantiomeric excess greater than about 50%; the transition metal is Pd; and less than 1.0 mol% of the catalyst relative to the limiting reagent is utilized.

In certain embodiments, the subject method is represented by Scheme 5 and the associated definitions, wherein the product is asymmetric and has an enantiomeric excess greater than about 70%; the transition metal is Pd; and less than 1.0 mol% of the catalyst relative to the limiting reagent is utilized.

In certain embodiments, the subject method is represented by Scheme 5 and the associated definitions, wherein the product is asymmetric and has an enantiomeric excess greater than about 90%; the transition metal is Pd; and less than 1.0 mol% of the catalyst relative to the limiting reagent is utilized.

In certain embodiments, the subject method is represented by Scheme 5 and the associated definitions, wherein the product is asymmetric and has an enantiomeric excess greater than about 95%; the transition metal is Pd; and less than 1.0 mol% of the catalyst relative to the limiting reagent is utilized.

In certain embodiments, the subject method is represented by Scheme 5 and the associated definitions, wherein the ligand is chiral and non-racemic; and the product is not racemic.

In certain embodiments, the subject method is represented by Scheme 5 and the associated definitions, wherein the ligand is chiral and non-racemic; the product is not racemic; and the product has an enantiomeric excess greater than about 50%.

In certain embodiments, the subject method is represented by Scheme 5 and the associated definitions, wherein the ligand is chiral and non-racemic; the product is not racemic; and the product has an enantiomeric excess greater than about 70%.

In certain embodiments, the subject method is represented by Scheme 5 and the associated definitions, wherein the ligand is chiral and non-racemic; the product is not racemic; and the product has an enantiomeric excess greater than about 90%.

In certain embodiments, the subject method is represented by Scheme 5 and the associated definitions, wherein the ligand is chiral and non-racemic; the product is not racemic; and the product has an enantiomeric excess greater than about 95%.

In certain embodiments, the subject method is represented by Scheme 5 and the associated definitions, wherein the ligand is chiral and non-racemic; the product is not racemic; and the product has an enantiomeric excess greater than about 50%; the transition metal is Pd; and less than 1.0 mol% of the catalyst relative to the limiting reagent is utilized.

In certain embodiments, the subject method is represented by Scheme 5 and the associated definitions, wherein the ligand is chiral and non-racemic; the product is not racemic; and the product has an enantiomeric excess greater than about 70%; the transition metal is Pd; and less than 1.0 mol% of the catalyst relative to the limiting reagent is utilized.

In certain embodiments, the subject method is represented by Scheme 5 and the associated definitions, wherein the ligand is chiral and non-racemic; the product is not racemic; and the product has an enantiomeric excess greater than about 90%; the transition metal is Pd; and less than 1.0 mol% of the catalyst relative to the limiting reagent is utilized.

In certain embodiments, the subject method is represented by Scheme 5 and the associated definitions, wherein the ligand is chiral and non-racemic; the product is not racemic; and the product has an enantiomeric excess greater than about 95%; the transition metal is Pd; and less than 1.0 mol% of the catalyst relative to the limiting reagent is utilized.

In certain embodiments, the subject method is represented by Scheme 5 and the associated definitions, wherein q is 1.

In certain embodiments, the subject method is represented by Scheme 5 and the associated definitions, wherein q is 2.

In certain embodiments, the subject method is represented by Scheme 5 and the associated definitions, wherein:

X of $(R')_2CC(R')X$ is $-OS(O)_2$alkyl, or $-OS(O)_2$aryl; and

the method is conducted at room temperature.

In certain embodiments, the subject method is represented by Scheme 5 and the associated definitions, wherein the transition metal is palladium; and
the base is a phosphate or fluoride.

In certain embodiments, the subject method is represented by Scheme 5 and the associated definitions, wherein the transition metal is palladium; and
the base is potassium phosphate.

In certain embodiments, the subject method is represented by Scheme 5 and the associated definitions, wherein X of $(R')_2CC(R')X$ represents Br; and
the reaction occurs at room temperature.

In certain embodiments, the subject method is represented by Scheme 5 and the associated definitions, wherein the product is provided in a yield of greater than 50%.

In certain embodiments, the subject method is represented by Scheme 5 and the associated definitions, wherein the product is provided in a yield of greater than 70%.

In certain embodiments, the subject method is represented by Scheme 5 and the associated definitions, wherein the product is provided in a yield of greater than 85%.

In certain embodiments, the subject method is represented by Scheme 5 and the associated definitions, wherein the method is conducted at room temperature.

In certain embodiments, the subject method is represented by Scheme 5 and the associated definitions, wherein X of $(R')_2CC(R')X$ is chloride.

In certain embodiments, the subject method is represented by Scheme 5 and the associated definitions, wherein X of $(R')_2CC(R')X$ is chloride; and the process is conducted at a temperature less than about 100°C.

In certain embodiments, the subject method is represented by Scheme 5 and the associated definitions, wherein X of $(R')_2CC(R')X$ is chloride; and the process is conducted at a temperature less than about 80°C.

In certain embodiments, the subject method is represented by Scheme 5 and the associated definitions, wherein X of $(R')_2CC(R')X$ is chloride; and the process is conducted at room temperature.

In certain embodiments, the subject method is represented by Scheme 5 and the associated definitions, wherein less than 0.01 mol% of the catalyst relative to the limiting reagent is utilized.

In certain embodiments, the subject method is represented by Scheme 5 and the associated definitions, wherein less than 0.0001 mol% of the catalyst relative to the limiting reagent is utilized.

In certain embodiments, the subject method is represented by Scheme 5 and the associated definitions, wherein less than 0.000001 mol% of the catalyst relative to the limiting reagent is utilized.

In certain embodiments, the subject method is represented by Scheme 5 and the associated definitions, wherein the limiting reagent is consumed in less than 48 hours.

In certain embodiments, the subject method is represented by Scheme 5 and the associated definitions, wherein the limiting reagent is consumed in less than 24 hours.

In certain embodiments, the subject method is represented by Scheme 5 and the associated definitions, wherein the limiting reagent is consumed in less than 12 hours.

In certain embodiments, the subject method is represented by Scheme 5 and the associated definitions, wherein the method is not set-up or performed or both under an inert atmosphere.

In certain embodiments, the subject method is represented by Scheme 5 and the associated definitions, wherein the method is not set-up or performed or both under anhydrous conditions.

In certain embodiments, the subject method is represented by Scheme 5 and the associated definitions, wherein the method is not set-up or performed or both under an oxygen-free atmosphere.

In certain embodiments, the subject method is represented by the generalized O-arylation reaction depicted in Scheme 6:

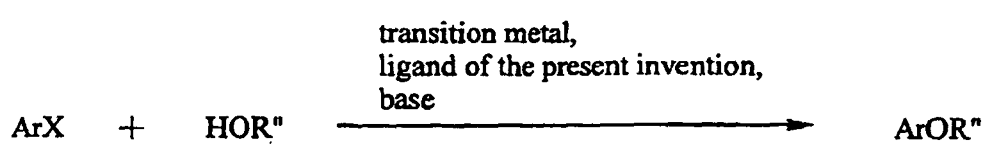


Scheme 6

wherein

Ar is selected from the set consisting of optionally substituted monocyclic and polycyclic aromatic and heteroaromatic moieties;

X is selected from the set consisting of Cl, Br, I, $-OS(O)_2$alkyl, and $-OS(O)_2$aryl;

R" represents, independently for each occurrence, alkyl, aryl, aralkyl, heteroalkyl, heteroaryl, heteroaralkyl, -Si(alkyl)$_3$, -Si(aryl)$_3$, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

Ar and R" may be covalently linked such that the reaction is intramolecular;

the transition metal is selected from the set of groups 5-12 metals, preferably the Group VIIIA metals;

the ligand is represented by general structure 7;

the base is selected from the set consisting of carbonates, phosphates, fluorides, alkoxides, amides, carbanions, and silyl anions; and

the method is conducted at room temperature.

In certain embodiments, the subject method is represented by Scheme 6 and the associated definitions, wherein the ligand is covalently linked to a solid support or soluble polymer, or is adsorbed onto a solid.
In certain embodiments, the subject method is represented by Scheme 6 and the associated definitions, wherein R"OH is a primary alcohol.
In certain embodiments, the subject method is represented by Scheme 6 and the associated definitions, wherein:

X of ArX is Cl, $-OS(O)_2$alkyl, or $-OS(O)_2$aryl.

In certain embodiments; the subject method is represented by Scheme 6 and the associated definitions, wherein the transition metal is palladium; and
the base is a phosphate or fluoride.
In certain embodiments, the subject method is represented by Scheme 6 and the associated definitions, wherein the transition metal is palladium; and
the base is potassium phosphate.
In certain embodiments, the subject method is represented by Scheme 6 and the associated definitions, wherein X of ArX represents Br.
In certain embodiments, the subject method is represented by Scheme 6 and the associated definitions, wherein the product is provided in a yield of greater than 50%.
In certain embodiments, the subject method is represented by Scheme 6 and the associated definitions, wherein the product is provided in a yield of greater than 70%.
In certain embodiments, the subject method is represented by Scheme 6 and the associated definitions, wherein the product is provided in a yield of greater than 85%.
In certain embodiments, the subject method is represented by Scheme 6 and the associated definitions, wherein X of ArX is chloride.
In certain embodiments, the subject method is represented by Scheme 6 and the associated definitions, wherein less than 0.01 mol% of the catalyst relative to the limiting reagent is utilized.
In certain embodiments, the subject method is represented by Scheme 6 and the associated definitions, wherein less than 0.0001 mol% of the catalyst relative to the limiting reagent is utilized.
In certain embodiments, the subject method is represented by Scheme 6 and the associated definitions, wherein less than 0.000001 mol% of the catalyst relative to the limiting reagent is utilized.
In certain embodiments, the subject method is represented by Scheme 6 and the associated definitions, wherein the limiting reagent is consumed in less than 48 hours.

In certain embodiments, the subject method is represented by Scheme 6 and the associated definitions, wherein the limiting reagent is consumed in less than 24 hours.

In certain embodiments, the subject method is represented by Scheme 6 and the associated definitions, wherein the limiting reagent is consumed in less than 12 hours.

In certain embodiments, the subject method is represented by Scheme 6 and the associated definitions, wherein the method is not set-up or performed or both under an inert atmosphere.

In certain embodiments, the subject method is represented by Scheme 6 and the associated definitions, wherein the method is not set-up or performed or both under anhydrous conditions.

In certain embodiments, the subject method is represented by Scheme 6 and the associated definitions, wherein the method is not set-up or performed or both under an oxygen-free atmosphere.

In certain embodiments, the subject method is represented by the generalized O-vinylation reaction depicted in Scheme 7:

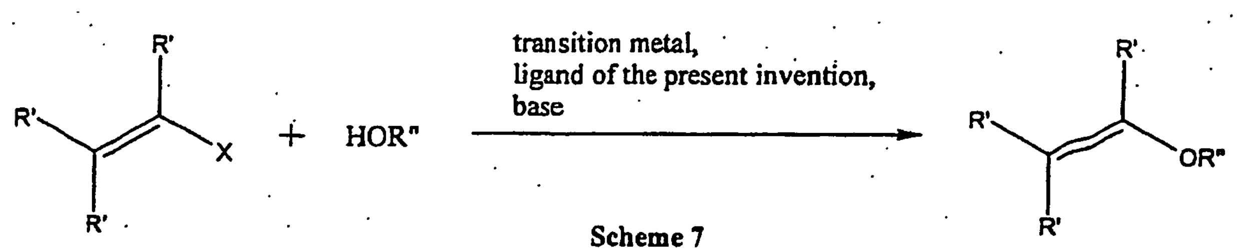


Scheme 7

wherein

X is selected from the set consisting of Cl, Br, I, $-OS(O)_2$alkyl, and $-OS(O)_2$aryl;

R' represents, independently for each occurrence, hydrogen, alkyl, aryl, aralkyl, heteroalkyl, heteroaryl, heteroaralkyl, alkylamino, arylamino; alkylthio, arylthio, alkoxy, aryloxy, or $-(CH_2)_m-R_{80}$;

R" represents, independently for each occurrence, alkyl, aryl, aralkyl, heteroalkyl, heteroaryl, heteroaralkyl, -Si (alkyl)$_3$, -Si(aryl)$_3$, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

R" and an instance of R' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme- 7 and the associated definitions, wherein the ligand is covalently linked to a solid support or soluble polymer, or is adsorbed onto a solid.

In certain embodiments, the subject method is represented by Scheme 7 and the associated definitions, wherein R"OH is a primary alcohol.

In certain embodiments, the subject method is represented by Scheme 7 and the associated definitions, wherein:

X of $(R')_2CC(R')X$ is $-OS(O)_2$alkyl, or $-OS(O)_2$aryl; and

the method is conducted at room temperature.

In certain embodiments, the subject method is represented by Scheme 7 and the associated definitions, wherein

the transition metal is palladium; and
the base is a phosphate or fluoride.

In certain embodiments, the subject method is represented by Scheme 7 and the associated definitions, wherein the transition metal is palladium; and
the base is potassium phosphate.

In certain embodiments, the subject method is represented by Scheme 7 and the associated definitions, wherein X of $(R')_2CC(R')X$ represents Br; and
the reaction occurs at room temperature.

In certain embodiments, the subject method is represented by Scheme 7 and the associated definitions, wherein the product is provided in a yield of greater than 50%.

In certain embodiments, the subject method is represented by Scheme 7 and the associated definitions, wherein the product is provided in a yield of greater than 70%.

In certain embodiments, the subject method is represented by Scheme 7 and the associated definitions, wherein the product is provided in a yield of greater than 85%.

In certain embodiments, the subject method is represented by Scheme 7 and the associated definitions, wherein the method is conducted at room temperature.

In certain embodiments, the subject method is represented by Scheme 7 and the associated definitions, wherein X of $(R')_2CC(R')X$ is chloride.

In certain embodiments, the subject method is represented by Scheme 7 and the associated definitions, wherein X of $(R')_2CC(R')X$ is chloride; and the process is conducted at a temperature less than about 100°C.

In certain embodiments, the subject method is represented by Scheme 7 and the associated definitions, wherein X of $(R')_2CC(R')X$ is chloride; and the process is conducted at a temperature less than about 80°C.

In certain embodiments, the subject method is represented by Scheme 7 and the associated definitions, wherein X of $(R')_2CC(R')X$ is chloride; and the process is conducted at room temperature.

In certain embodiments, the subject method is represented by Scheme 7 and the associated definitions, wherein less than 0.01 mol% of the catalyst relative to the limiting reagent is utilized.

In certain embodiments, the subject method is represented by Scheme 7 and the associated definitions, wherein less than 0.0001 mol% of the catalyst relative to the limiting reagent is utilized.

In certain embodiments, the subject method is represented by Scheme 7 and the associated definitions, wherein less than 0.000001 mol% of the catalyst relative to the limiting reagent is utilized.

In certain embodiments, the subject method is represented by Scheme 7 and the associated definitions, wherein the limiting reagent is consumed in less than 48 hours.

In certain embodiments, the subject method is represented by Scheme 7 and the associated definitions, wherein the limiting reagent is consumed in less than 24 hours.

In certain embodiments, the subject method is represented by Scheme 7 and the associated definitions, wherein the limiting reagent is consumed in less than 12 hours.

In certain embodiments, the subject method is represented by Scheme 7 and the associated definitions, wherein the method is not set-up or performed or both under an inert atmosphere.

In certain embodiments, the subject method is represented by Scheme 7 and the associated definitions, wherein the method is not set-up or performed or both under anhydrous conditions.

In certain embodiments, the subject method is represented by Scheme 7 and the associated definitions, wherein the method is not set-up or performed or both under an oxygen-free atmosphere.

In certain embodiments, the subject method is represented by the generalized Heck reaction depicted in Scheme 8:

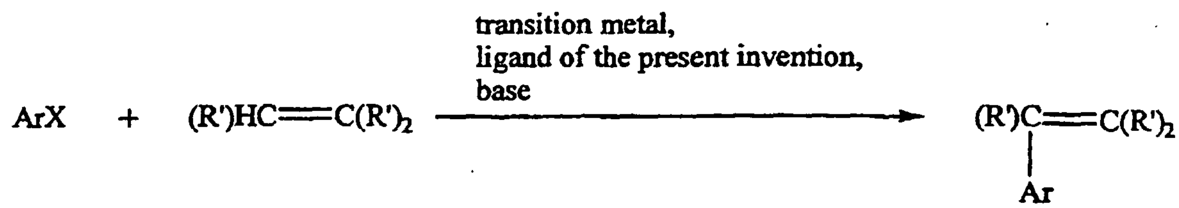


Scheme 8

wherein

Ar is selected from the set consisting of optionally substituted aromatic, heteroaromatic, and alkenyl moieties;

X is selected from the set consisting of Cl, Br, I, $-OS(O)_2$alkyl, and $-OS(O)_2$aryl;

R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

Ar and an instance of R' may be covalently linked such that the reaction is intramolecular;

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme 8 and the associated definitions, wherein the ligand is covalently linked to a solid support or soluble polymer, or is adsorbed onto a solid.

In certain embodiments, the subject method is represented by Scheme 8 and the associated definitions, wherein:

X of ArX is Cl, $-OS(O)_2$alkyl, or $-OS(O)_2$aryl; and

the method is conducted at room temperature.

In certain embodiments, the subject method is represented by Scheme 8 and the associated definitions, wherein the transition metal is palladium; and
the base is a phosphate or fluoride.

In certain embodiments, the subject method is represented by Scheme 8 and the associated definitions, wherein the transition metal is palladium; and
the base is potassium phosphate.

In certain embodiments, the subject method is represented by the generalized Heck reaction depicted in Scheme 9:

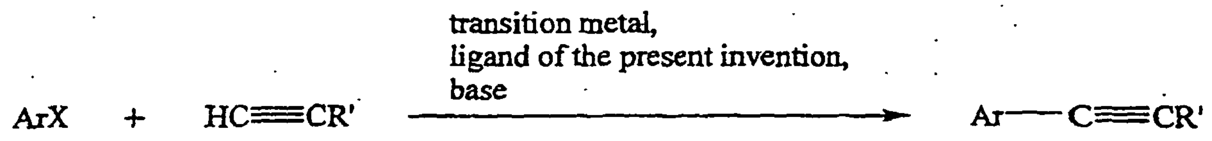


**Scheme 9**

wherein

Ar is selected from the set consisting of optionally substituted aromatic, heteroaromatic, and alkenyl moieties;

X is selected from the set consisting of Cl, Br, I, $-OS(O)_2$alkyl, and $-OS(O)_2$aryl;

R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;

$R_{80}$ represents independently for each occurrence a substituted or unsubstituted aryl, cycloalkyl, cycloalkenyl, heterocycle or polycycle;

m, independently for each occurrence, is an integer selected from the range 0 to 8 inclusive;

Ar and R' may be covalently linked such that the reaction is intramolecular,

the transition metal is selected from the set of groups 5-12 metals, preferably the Group VIIIA metals;

the ligand is represented by general structure 7; and

the base is selected from the set consisting of carbonates, phosphates, fluorides, alkoxides, amides, carbanions, and silyl anions.

In certain embodiments, the subject method is represented by Scheme 9 and the associated definitions, wherein the ligand is covalently linked to a solid support or soluble polymer, or is adsorbed onto a solid.

In certain embodiments, the subject method is represented by Scheme 9 and the associated definitions, wherein:

X of ArX is Cl, $-OS(O)_2$alkyl, or $-OS(O)_2$aryl; and

the method is conducted at room temperature.

In certain embodiments, the subject method is represented by Scheme 9 and the associated definitions, wherein the transition metal is palladium; and
the base is a phosphate or fluoride.

In certain embodiments, the subject method is represented by Scheme 9 and the associated definitions, wherein the transition metal is palladium; and
the base is potassium phosphate.

In preferred embodiments of the reactions of the invention, there is no need to use large excesses of reactants, e.g., amine, boronic acid, ketone and the like, or aromatic compound. The reactions proceed quickly and in high yield to the desired products using substantially stoichiometric amounts of reagents. For example, in the amination reactions of the invention, the amine may be present in as little as a two-fold excess and preferably in no greater than a 20% excess relative to the aromatic compound. Alternatively, the aromatic compound may be present in as little as a two-fold excess and preferably in no greater than a 20% excess relative to the amine. An analogous discussion applies to the subject Suzuki couplings and α-arylations.

The reactions typically proceed at mild temperatures and pressures to give high yields of the product aryl amines, biaryls, α-aryl ketones, and the like. Thus, yields of desired products greater than 45%, preferably greater than 75%, and even more preferably greater than 80%, may be obtained from reactions at mild temperatures according to the invention. The reaction may be carried out at temperature less than 120˚C, and preferably in the range of 20-100˚C. In certain preferred embodiments, the reactions are carried out at ambient temperature.

The reactions can be run in a wide range of solvent systems, including polar aprotic solvents. Alternatively, in certain embodiments, the subject reactions may be carried in the absence of added solvent.

The ability to provide synthesis schemes for aryl amines, biaryls, α-aryl ketones, and the like, which can be carried out under mild conditions and/or with non-polar solvents has broad application, especially in the agricultural and pharmaceutical industries, as well as in the polymer industry. In this regard, the subject reactions are particularly well-suited to reactants or products which include sensitive functionalities, e.g., which would otherwise be labile under harsh reaction conditions.

The subject amine arylation, Suzuki coupling, ketone α-arylation reactions and the like can be used as part of combinatorial synthesis schemes to yield libraries of aryl amines, biaryls, α-aryl ketones, and the like. Accordingly, another aspect of the present invention relates to use of the subject method to generate variegated libraries of aryl amines, biaryls, α-aryl ketones, and the like, and to the libraries themselves. The libraries can be soluble or linked to insoluble supports, e.g., through a substituent of a reactant (prior to carrying out a reaction of the present invention), e.g., the aryl group, amine, boronic acid, ketone, or the like, or through a substituent of a product (subsequent to carrying out a reaction of the present invention), e.g., the aryl amine, biaryl, α-aryl ketone, or the like.

The ligands of the present invention and the methods based thereon enable the formation of carbon-heteroatom and carbon-carbon bonds -- via transition metal catalyzed aminations, Suzuki couplings, α-arylations of carbonyls, and the like -- under conditions that would not yield appreciable amounts of the observed product(s) using ligands and methods known in the art. In preferred embodiments, the ligands and methods of the present invention catalyze the

aforementioned transformations at temperatures below 50 °C, and in certain embodiments they occur at room temperature. When a reaction is said to occur under a given set of conditions it means that the rate of the reaction is such the bulk of the starting materials is consumed, or a significant amount of the desired product is produced, within 48 hours, and preferably within 24 hours, and most preferably within 12 hours. In certain embodiments, the ligands and methods of the present invention catalyze the aforementioned transformations utilizing less than 1 mol% of the catalyst complex relative to the limiting reagent, in certain preferred embodiments less than 0.01 mol% of the catalyst complex relative to the limiting reagent, and in additional preferred embodiments less than 0.0001 mol% of the catalyst complex relative to the limiting reagent.

The ligands of the present invention and the methods based thereon can be used to produce synthetic intermediates that, after being subjected to additional methods known in the art, are transformed to desired end products, e.g., lead compounds in medicinal chemistry programs, pharmaceuticals, insecticides, antivirals and antifungals. Furthermore, the ligands of the present invention and the methods based thereon may be used to increase the efficiency of and/or shorten established routes to desired end products, e.g., lead compounds in medicinal chemistry programs, pharmaceuticals, insecticides, antivirals and antifungals.

*II. Definitions*

For convenience, before further description of the present invention, certain terms employed in the specification, examples; and appended claims are collected here.

The term "binaphthylene" refers to the ring system below. The numbers around the peripheries of the ring system are the positional numbering systems used herein. Likewise, the capital letters contained within the individual rings of the ring system are the ring descriptors used herein.

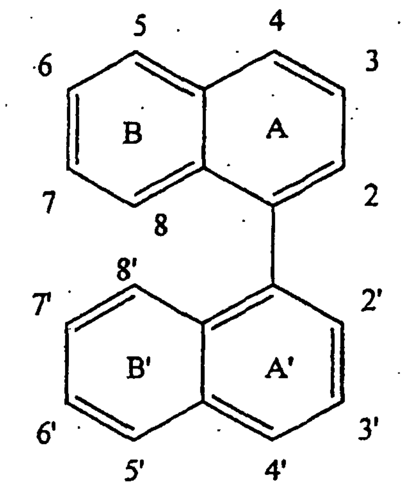

*binaphthyl*

The term "substrate aryl group" refers to an aryl group containing an electrophilic atom which is susceptible to the subject cross-coupling reaction, e.g., the electrophilic atom bears a leaving group. In reaction scheme 1, the substrate aryl is represented by ArX, and X is the leaving group. The aryl group, Ar, is said to be substituted if, in addition to X, it is substituted at yet other positions. The substrate aryl group can be a single ring molecule, or can be a component of a larger molecule.

The term "nucleophile" is recognized in the art, and as used herein means a chemical moiety having a reactive pair of electrons.

The term "electrophile" is art-recognized and refers to chemical moieties which can accept a pair of electrons from a nucleophile as defined above. Electrophilic moieties useful in the method of the present invention include halides and sulfonates.

The terms "electrophilic atom", "electrophilic center" and "reactive center" as used herein refer to the atom of the substrate aryl moiety which is attacked by, and forms a new bond to the nucleophilic heteroatom of the hydrazine and the like. In most (but not all) cases, this will also be the aryl ring atom from which the leaving group departs.

The term "electron-withdrawing group" is recognized in the art, and denotes the tendency of a substituent to

attract valence electrons from neighboring atoms, i.e., the substituent is electronegative.with respect to neighboring atoms. A quantification of the level of electron-withdrawing capability is given by the Hammett sigma (s) constant. This well known constant is described in many references, for instance, J. March, Advanced Organic Chemistry, McGraw Hill Book. Company, New York, (1977 edition) pp. 251-259. The Hammett constant values are generally negative for electron donating groups (s[P] = - 0.66 for $NH_2$) and positive for electron with drawing groups (s[P] = 0.78 for a nitro group), s[P] indicating para substitution. Exemplary electron-withdrawing groups include nitro, ketone, aldehyde, sulfonyl, trifluoromethyl, -CN, chloride, and the like. Exemplary electron-donating groups include amino, methoxy, and the like.

The term "reaction product" means a compound which results from the reaction of the hydrazine or the like and the substrate aryl group. In general, the term "reaction product" will be used herein to refer to a stable, isolable aryl ether adduct, and not to unstable intermediates or transition states.

The term "room temperature" is recognized in the art and means a comfortable indoor temperature, generally between 20 and 25°C.

The term "catalytic amount" is recognized in the art and means a substoichiometric amount of a reagent (a catalyst) relative to the limiting reagent(s).

The term "meso compound" is recognized in the art and means a chemical compound which has at least two chiral centers but is achiral due to the presence of an internal plane or point of symmetry.

The term "chiral" refers to molecules which have the property of non-superirnposability of their mirror image partner, while the term "achiral" refers to molecules which are superimposable on their mirror image partner. A "prochiral molecule" is a molecule which has the potential to be converted to a chiral molecule in a particular process.

The term "stereoisomers" refers to compounds which have identical chemical constitution, but differ with regard to the arrangement of the atoms or groups in space. In particular, "enantiomers" refer to two stereoisomers of a compound which are non-superimposable mirror images of one another. "Diastereomers", on the other hand, refers to stereoisomers with two or more centers of dissymmetry and whose molecules are not mirror images of one another.

Furthermore, a "stereoselective process" is one which produces a particular stereoisomer of a reaction product in preference to other possible stereoisomers of that product. An "enantioselective process" is one which favors production of one of the two possible enantiomers of a reaction product. The subject method is said to produce a "stereoisomerically-enriched" product (e.g., enantiomerically-enriched or diastereomerically-enriched) when the yield of a particular stereoisomer of the product is greater by a statistically significant amount relative to the yield of that stereoisomer resulting from the same reaction run in the absence of a chiral catalyst. For example, a reaction which routinely produces a racemic mixture will, when catalyzed by one of the subject chiral catalysts, yield an e.e. for a particular enantiomer of the product.

The term "regioisomers" refers to compounds which have the same molecular formula but differ in the connectivity of the atoms. Accordingly, a "regioselective process" is one which favors the production of a particular regioisomer over others, e.g., the reaction produces a statistically significant majority of a certain regioisomer.

As discussed more fully below, the reactions contemplated in the present invention include reactions which are enantioselective, diastereoselective, and/or regioselective. An enantioselective reaction is a reaction which converts an achiral reactant to a chiral product enriched in one enantiomer. Enantioselectivity is generally quantified as "enantiomeric excess" (ee) defined as follows:

$$\text{\% enantiomeric excess A (ee)} = (\text{\% enantiomer A}) - (\text{\% enantiomer B})$$

where A and B are the enantiomers formed. Additional terms that are used in conjunction with enatioselectivity include "optical purity" or "optical activity". An enantioselective reaction yields a product with an e.e. greater than zero. Preferred enantioselective reactions yield a product with an e.e. greater than 20%, more preferably greater than 50%, even more preferably greater than 70%, and most preferably greater than 80%.

A diastereoselective reaction converts a reactant or reactants (which may be achiral, racemic, non-racemic or enantiomerically pure) to a product enriched in one diastereomer. If the chiral reactant is racemic, in the presence of a chiral, non-racemic reagent or catalyst, one reactant enantiomer may react more slowly than the other. This effect is termed a kinetic resolution, wherein the reactant enantiomers are resolved by differential reaction rate to yield an enantiomerically enriched product. Kinetic resolution is usually achieved by the use of sufficient reagent to react with only one reactant enantiomer (i.e. one-half mole of reagent per mole of racemic substrate). Examples of catalytic reactions which have been used for kinetic resolution of racemic reactants include the Sharpless epoxidation and the Noyori hydrogenation.

A regioselective reaction is a reaction which occurs preferentially at one reactive center rather than another reactive center. For example, a regioselective $\alpha$-arylation of methyl isopropyl ketone would preferentially occur at one

of the two α-carbons of the ketone.

The term "non-racemic" means a preparation having greater than 50% of a desired stereoisomer, more preferably at least 75%. "Substantially non-racemic" refers to preparations which have greater than 90% ee for a desired stereoisomer, more preferably greater than 95% ee.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. In preferred embodiments, a straight chain or branched chain alkyl has 30 or fewer carbon atoms in its backbone (e.g., $C_1$-$C_{30}$ for straight chain, $C_3$-$C_{30}$ for branched chain), and more preferably 20 or fewer. Likewise, preferred cycloalkyls have from 3-10 carbon atoms in their ring structure, and more preferably have 5, 6 or 7 carbons in the ring structure.

Moreover, the term "alkyl" (or "lower alkyl") as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, a halogen, a hydroxyl, a carbonyl (such as a carboxyl, an ester, a formyl, or a ketone), a thiocarbonyl (such as a thioester, a thioacetate, or a thioformate), an alkoxyl, a phosphoryl, a phosphonate, a phosphinate, an amino, an amido, an amidine, an imine, a cyano, a nitro, an azido, a sulfhydryl, an alkylthio, a sulfate, a sulfonate, a sulfamoyl, a sulfonamido, a sulfonyl, a heterocyclyl, an aralkyl, or an aromatic or heteroaromatic moiety. It will be understood by those skilled in the art that the moieties substituted on the hydrocarbon chain can themselves be substituted, if appropriate. For instance, the substituents of a substituted alkyl may include substituted and unsubstituted forms of amino, azido, imino, amido, phosphoryl (including phosphonate and phosphinate), sulfonyl (including sulfate, sulfonamido, sulfamoyl and sulfonate), and silyl groups, as well as ethers, alkylthios, carbonyls (including ketones, aldehydes, carboxylates, and esters), $-CF_3$, -CN and the like. Exemplary substituted alkyls are described below. Cycloalkyls can be further substituted with alkyls, alkenyls, alkoxys, alkylthios, aminoalkyls, carbonyl-substituted alkyls, $-CF_3$, -CN, and the like.

The term "aralkyl", as used herein, refers to an alkyl group substituted with an aryl group (e.g., an aromatic or heteroaromatic group).

The terms "alkenyl" and "alkynyl" refer to unsaturated aliphatic groups analogous in length and possible substitution to the alkyls described above, but that contain at least one double or triple bond respectively.

Unless the number of carbons is otherwise specified, "lower alkyl" as used herein means an alkyl group, as defined above, but having from one to ten carbons, more preferably from one to six carbon atoms in its backbone structure. Likewise, "lower alkenyl" and "lower alkynyl" have similar chain lengths. Preferred alkyl groups are lower alkyls. In preferred embodiments, a substituent designated herein as alkyl is a lower alkyl.

The term "aryl" as used herein includes 5-,6- and 7-membered single-ring aromatic groups that may include from zero to four heteroatoms, for example, benzene, pyrrole, furan, thiophene, imidazole, oxazole, thiazole, triazole, pyrazole, pyridine, pyrazine, pyridazine and pyrimidine, and the like. Those aryl groups having heteroatoms in the ring structure may also be referred to as "aryl heterocycles" or "heteroaromatics". The aromatic ring can be substituted at one or more ring positions with such substituents as described above, for example, halogen, azide, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, sulfonamido, ketone, aldehyde, ester, heterocyclyl, aromatic or heteroaromatic moieties, $-CF_3$, -CN, or the like. The term "aryl" also includes polycyclic ring systems having two or more cyclic rings in which two or more carbons are common to two adjoining rings (the rings are "fused rings") wherein at least one of the rings is aromatic, e.g., the other cyclic rings can be cycloalkyls, cycloalkenyls, cycloalkynyls, aryls and/or heterocyclyls.

The abbreviations Me, Et, Ph, Tf, Nf, Ts, Ms, and dba represent methyl, ethyl, phenyl, trifluoromethanesulfonyl, nonafluorobutanesulfonyl, *p*-toluenesulfonyl, methanesulfonyl, and dibenzylideneacetone, respectively. A more comprehensive list of the abbreviations utilized by organic chemists of ordinary skill in the art appears in the first issue of each volume of the *Journal of Organic Chemistry;* this list is typically presented in a table entitled <u>Standard List of Abbreviations.</u> The abbreviations contained in said list, and all abbreviations utilized by organic chemists of ordinary skill in the art are hereby incorporated by reference.

The terms *ortho, meta* and *para* apply to 1,2-, 1,3- and 1,4-disubstituted benzenes, respectively. For example, the names 1,2-dimethylbenzene and *ortho*-dimethylbenzene are synonymous.

The terms "heterocyclyl" or "heterocyclic group" refer to 3- to 10-membered ring structures, more preferably 3- to 7-membered rings, whose ring structures include one to four heteroatoms. Heterocycles can also be polycycles. Heterocyclyl groups include, for example, thiophene, thianthrene, furan, pyran, isobenzofuran, chromene, xanthene, phenoxathiin, pyrrole, imidazole, pyrazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, pyrimidine, phenanthroline, phenazine, phenarsazine, phenothiazine, furazan, phenoxazine, pyrrolidine, oxolane, thiolane, oxazole, piperidine, piperazine, morpholine, lactones, lactams such as azetidinones and pyrrolidinones, sultams, sultones, and the like. The heterocyclic ring can be substituted at one or more positions with such substituents as described above, as for example, halogen,

alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, $-CF_3$, -CN, or the like.

The terms "polycyclyl" or "polycyclic group" refer to two or more rings (e.g., cycloalkyls, cycloalkenyls, cycloalkynyls, aryls and/or heterocyclyls) in which two or more carbons are common to two adjoining rings, e.g., the rings are "fused rings". Rings that are joined through non-adjacent atoms are termed "bridged" rings. Each of the rings of the polycycle can be substituted with such substituents as described above, as for example, halogen, alkyl, aralkyl, alkenyl, alkynyl, cycloalkyl, hydroxyl, amino, nitro, sulfhydryl, imino, amido, phosphonate, phosphinate, carbonyl, carboxyl, silyl, ether, alkylthio, sulfonyl, ketone, aldehyde, ester, a heterocyclyl, an aromatic or heteroaromatic moiety, $-CF_3$, -CN, or the like.

The term "carbocycle", as used herein, refers to an aromatic or non-aromatic ring in which each atom of the ring is carbon.

The term "heteroatom" as used herein means an atom of any element other than carbon or hydrogen. Preferred heteroatoms are nitrogen, oxygen, sulfur and phosphorous.

As used herein, the term "nitro" means $-NO_2$; the term "halogen" designates -F, -Cl, -Br or -I; the term "sulfhydryl" means -SH; the term "hydroxyl" means -OH; and the term "sulfonyl" means $-SO_2-$.

The terms "amine" and "amino" are art recognized and refer to both unsubstituted and substituted amines, e.g., a moiety that can be represented by the general formula:

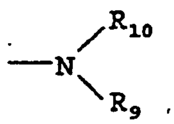
or
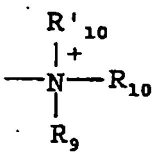

wherein $R_9$, $R_{10}$ and $R'_{10}$ each independently represent a hydrogen, an alkyl, an alkenyl, $-(CH_2)_m-R_8$, or $R_9$ and $R_{10}$ taken together with the N atom to which they are attached complete a heterocycle having from 4 to 8 atoms in the ring structure; $R_8$ represents an aryl, a cycloalkyl, a cycloalkenyl, a heterocycle or a polycycle; and m is zero or an integer in the range of 1 to 8. In preferred embodiments, only one of $R_9$ or $R_{10}$ can be a carbonyl, e.g., $R_9$, $R_{10}$ and the nitrogen together do not form an imide. In even more preferred embodiments, $R_9$ and $R_{10}$ (and optionally $R'_{10}$) each independently represent a hydrogen, an alkyl, an alkenyl, or $-(CH_2)_m-R_8$. Thus, the term "alkylamine" as used herein means an amine group, as defined above, having a substituted or unsubstituted alkyl attached thereto, i.e., at least one of $R_9$ and $R_{10}$ is an alkyl group.

The term "acylamino" is art-recognized and refers to a moiety that can be represented by the general formula:

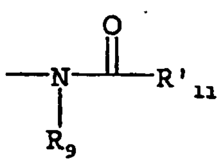

wherein $R_9$ is as defined above, and $R'_{11}$ represents a hydrogen, an alkyl, an alkenyl or $-(CH_2)_m-R_8$, where m and $R_8$ are as defined above.

The term "amido" is art recognized as an amino-substituted carbonyl and includes a moiety that can be represented by the general formula:

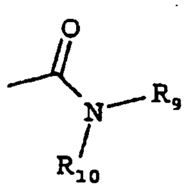

wherein $R_9$, $R_{10}$ are as defined above. Preferred embodiments of the amide will not include imides which may be unstable.

The term "alkylthio" refers to an alkyl group, as defined above, having a sulfur radical attached thereto. In

preferred embodiments, the "alkylthio" moiety is represented by one of-S-alkyl, -S-alkenyl, -S-alkynyl, and -S-$(CH_2)_m$-$R_8$, wherein m and $R_8$ are defined above. Representative alkylthio groups include methylthio, ethyl thio, and the like.

The term "carbonyl" is art recognized and includes such moieties as can be represented by the general formula:

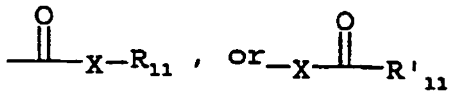

wherein X is a bond or represents an oxygen or a sulfur, and $R_{11}$ represents a hydrogen, an alkyl, an alkenyl, -$(CH_2)_m$-$R_8$ or a pharmaceutically acceptable salt, $R'_{11}$ represents a hydrogen, an alkyl, an alkenyl or -$(CH_2)_m$-$R_8$, where m and $R_8$ are as defined above. Where X is an oxygen and $R_{11}$ or $R'_{11}$ is not hydrogen, the formula represents an "ester". Where X is an oxygen, and $R_{11}$ is as defined above, the moiety is referred to herein as a carboxyl group, and particularly when $R_{11}$ is a hydrogen, the formula represents a "carboxylic acid". Where X is an oxygen, and $R'_{11}$ is hydrogen, the formula represents a "formate". In general, where the oxygen atom of the above formula is replaced by sulfur, the formula represents a "thiolcarbonyl" group. Where X is a sulfur and $R_{11}$ or $R'_{11}$ is not hydrogen, the formula represents a "thiolester." Where X is a sulfur and $R_{11}$ is hydrogen, the formula represents a "thiolcarboxylic acid." Where X is a sulfur and $R_{11}'$ is hydrogen, the formula represents a "thiolformate." On the other hand, where X is a bond, and $R_{11}$ is not hydrogen, the above formula represents a "ketone" group. Where X is a bond, and $R_{11}$ is hydrogen, the above formula represents an "aldehyde" group.

The terms "alkoxyl" or "alkoxy" as used herein refers to an alkyl group, as defined above, having an oxygen radical attached thereto. Representative alkoxyl groups include methoxy, ethoxy, propyloxy, tert-butoxy and the like. An "ether" is two hydrocarbons covalently linked by an oxygen. Accordingly, the substituent of an alkyl that renders that alkyl an ether is or resembles an alkoxyl, such as can be represented by one of -O-alkyl, -O-alkenyl, -O-alkynyl, -O-$(CH_2)_m$-$R_8$, where m and Rg are described above.

The term "sulfonate" is art recognized and includes a moiety that can be represented by the general formula: :

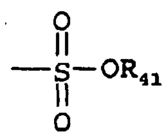

in which $R_{41}$ is an electron pair, hydrogen, alkyl, cycloalkyl, or aryl.

The terms triflyl, tosyl, mesyl, and nonaflyl are art-recognized and refer to trifluoromethanesulfonyl, *p*-toluenesulfonyl, methanesulfonyl, and nonafluorobutanesulfonyl groups, respectively. The terms triflate, tosylate, mesylate, and nonaflate are art-recognized and refer to trifluoromethanesulfonate ester, *p*-toluenesulfonate ester, methanesulfonate ester, and nonafluorobutanesulfonate ester functional groups and molecules that contain said groups, respectively.

The term "sulfate" is art recognized and includes a moiety that can be represented by the general formula:

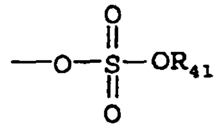

in which $R_{41}$ is as defined above.

The term "sulfonamido" is art recognized and includes a moiety that can be represented by the general formula:

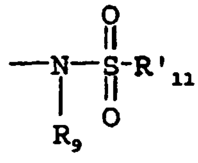

in which $R_9$ and $R'_{11}$ are as defined above.

The term "sulfamoyl" is art-recognized and includes a moiety that can be represented by the general formula:

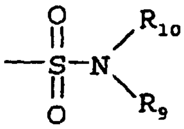

in which $R_9$ and $R_{10}$ are as defined above.

The terms "sulfoxido" or "sulfinyl", as used herein, refers to a moiety that can be represented by the general formula:

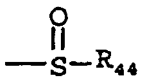

in which $R_{44}$ is selected from the group consisting of hydrogen, alkyl, alkenyl, alkynyl, cycloalkyl, heterocyclyl, aralkyl, or aryl.

A "phosphoryl" can in general be represented by the formula:

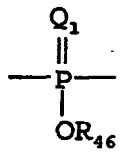

wherein $Q_1$ represented S or O, and $R_{46}$ represents hydrogen, a lower alkyl or an aryl. When used to substitute, e.g., an alkyl, the phosphoryl group of the phosphorylalkyl can be represented by the general formula:

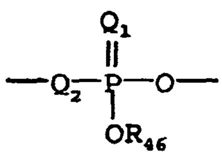
, or
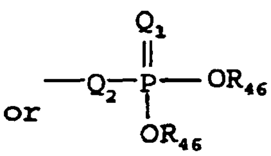

wherein $Q_1$ represented S or 0, and each $R_{46}$ independently represents hydrogen, a lower alkyl or an aryl, $Q_2$ represents 0, S or N. When $Q_1$ is an S, the phosphoryl moiety is a "phosphorothioate".

A "phosphoramidite" can be represented in the general formula:

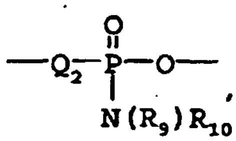
, or
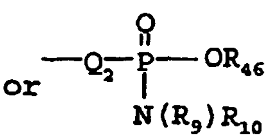

wherein $R_9$ and $R_{10}$ are as defined above, and $Q_2$ represents O, S or N.

A "phosphonamidite" can be represented in the general formula:

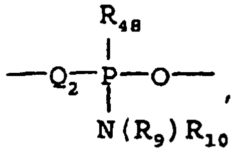
, or
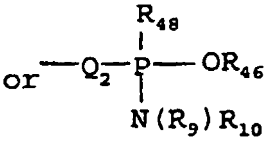

wherein $R_9$ and $R_{10}$ are as defined above, $Q_2$ represents O, S or N, and $R_{48}$ represents a lower alkyl or an aryl, $Q_2$

represents O, S or N.

A "selenoalkyl" refers to an alkyl group having a substituted seleno group attached thereto. Exemplary "selenoethers" which may be substituted on the alkyl are selected from one of -Se-alkyl, -Se-alkenyl, -Se-alkynyl, and -Se-$(CH_2)_m$-$R_8$, m and $R_8$ being defined above.

Analogous substitutions can be made to alkenyl and alkynyl groups to produce, for example, aminoalkenyls, aminoalkynyls, amidoalkenyls, amidoalkynyls, iminoalkenyls, iminoalkynyls, thioalkenyls, thioalkynyls, carbonyl-substituted alkenyls or alkynyls.

The phrase "protecting group" as used herein means temporary modifications of a potentially reactive functional group which protect it from undesired chemical transformations. Examples of such protecting groups include esters of carboxylic acids, silyl ethers of alcohols, and acetals and ketals of aldehydes and ketones, respectively. The field of protecting group chemistry has been reviewed (Greene, T.W.; Wuts, P.G.M *Protective Groups in Organic Synthesis,* $2^{nd}$ ed.; Wiley: New York, 1991).

It will be understood that "substitution" or "substituted with" includes the implicit proviso that such substitution is in accordance with permitted valence of the substituted atom and the substituent, and that the substitution results in a stable compound, e.g., which does not spontaneously undergo transformation such as by rearrangement, cyclization, elimination, etc.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, those described above. The permissible substituents can be one or more and the same or different for appropriate organic compounds. For purposes of this invention, the heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

A "polar solvent" means a solvent which has a dielectric constant ($\varepsilon$) of 2.9 or greater, such as DMF, THF, ethylene glycol dimethyl ether (DME), DMSO, acetone, acetonitrile, methanol, ethanol, isopropanol, n-propanol, t-butanol or 2-methoxyethyl ether. Preferred polar solvents are DMF, DME, NMP, and acetonitrile.

An "aprotic solvent" means a non-nucleophilic solvent having a boiling point range above ambient temperature, preferably from about 25°C to about 190°C, more preferably from about 80°C to about 160°C, most preferably from about 80°C to 150°C, at atmospheric pressure. Examples of such solvents are acetonitrile, toluene, DMF, diglyme, THF or DMSO.

A "polar, aprotic solvent" means a polar solvent as defined above which has no available hydrogens to exchange with the compounds of this invention during reaction, for example DMF, acetonitrile, diglyme, DMSO, or THF.

For purposes of this invention, the chemical elements are identified in accordance with the Periodic Table of the Elements, CAS version, Handbook of Chemistry and Physics, 67th Ed., 1986-87, inside cover. Also for purposes of this invention, the term "hydrocarbon" is contemplated to include all permissible compounds having at least one hydrogen and one carbon atom. In a broad aspect, the permissible hydrocarbons include acyclic and cyclic, branched and unbranched, carbocyclic and heterocyclic, aromatic and nonaromatic organic compounds which can be substituted or unsubstituted.

## *III. Exemplary Catalyzed Reactions*

As described above, one of the methods of the invention is a transition metal-catalyzed amination reaction which comprises combining an amine with a substrate aryl group bearing an activated group X. The reaction includes at least a catalytic amount of a transition metal catalyst, comprising a novel ligand, and the combination is maintained under conditions appropriate for the metal catalyst to catalyze the arylation of the amine.

The two ligands (**24** and **25**; not according to the invention) shown below are referred to by number in the illustrative reactions in this section.

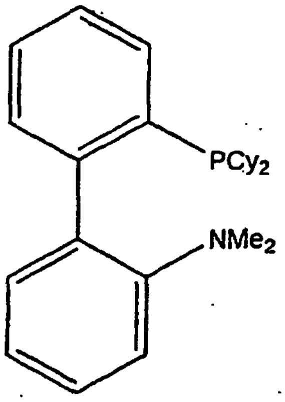

24

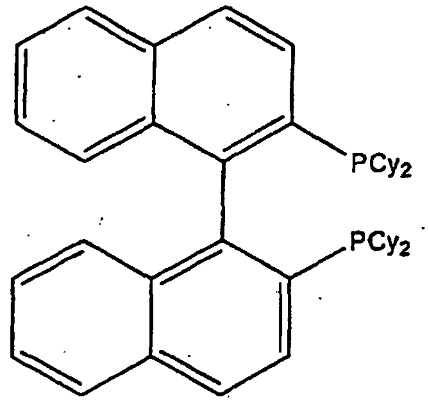

25

An illustrative reaction is the intermolecular reaction between an electron-rich aryl chloride and pyrrolidine to give an *N*-aryl pyrrolidine:

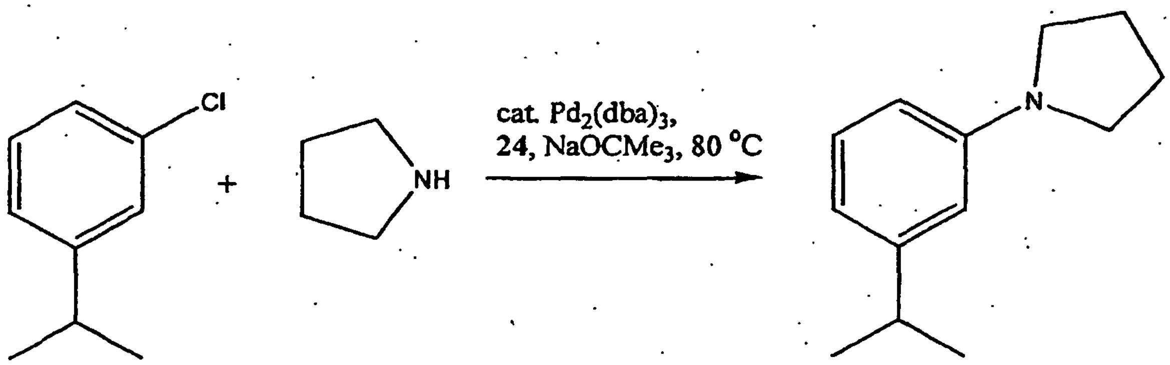


A second illustrative reaction is the *N*-arylation of indole with an electron-rich aryl bromide:

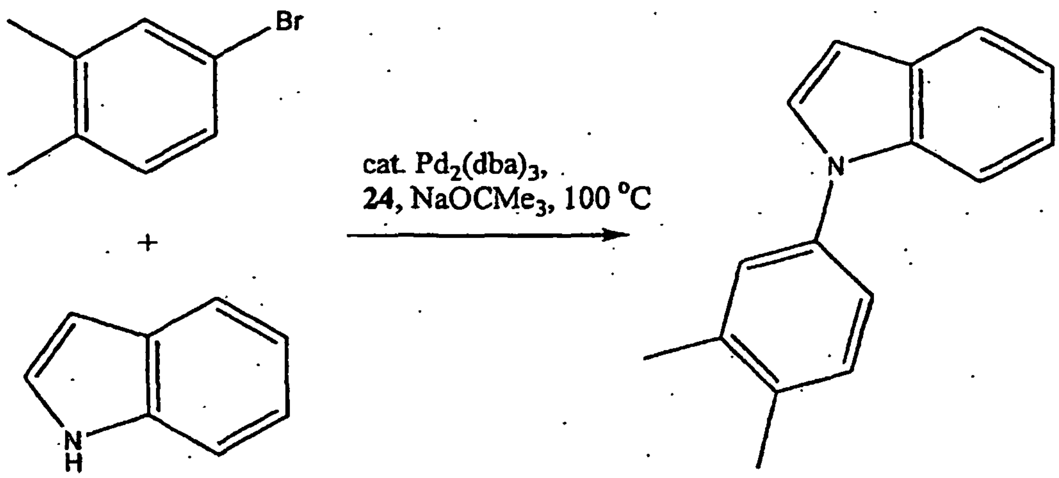


In the following the catalysis by Pd/4 of the amination of electron-poor aryl chlorides is depicted as an illustrative

transformation.

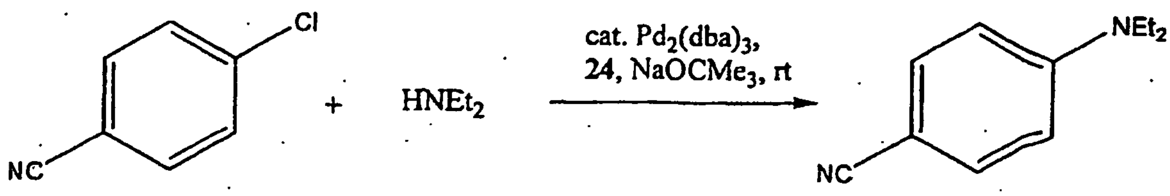


An additional reaction is the room temperature amination of aryl iodides or bromides, as depicted in the following illustrative transformation involving an aryl iodide.

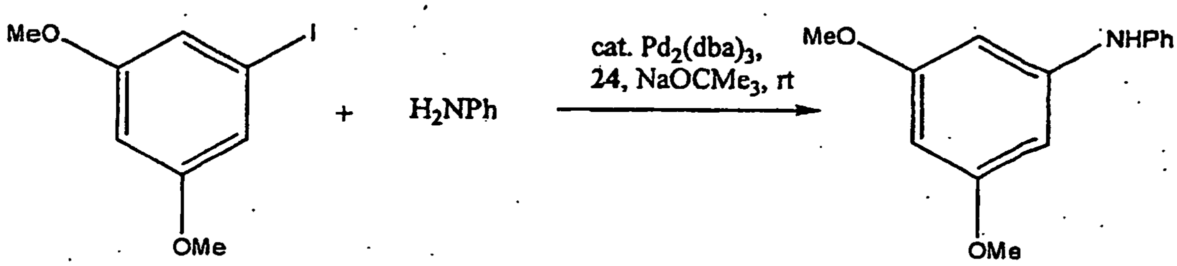


Another illustrative reaction is a palladium-catalyzed amination of an electron-neutral aryl chloride.

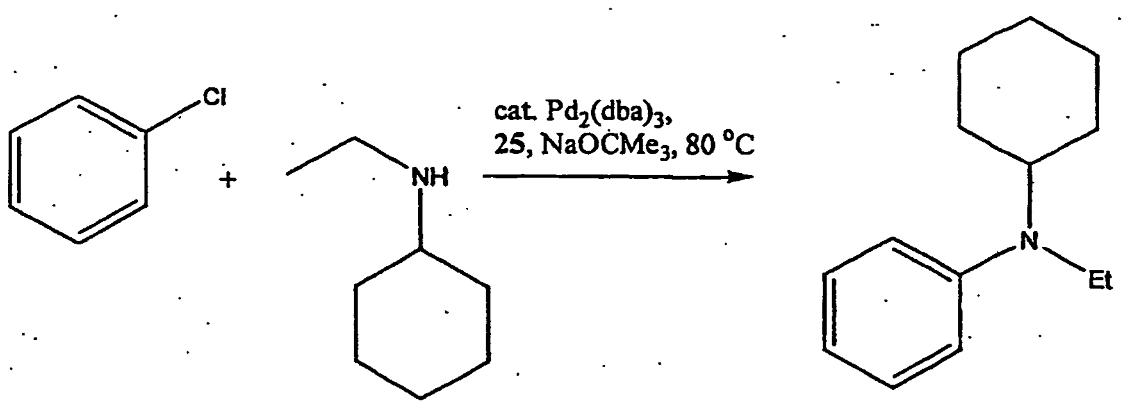


One of ordinary skill in the art will be able to envision intramolecular variants of the amination methods. An illustrative reaction follows:

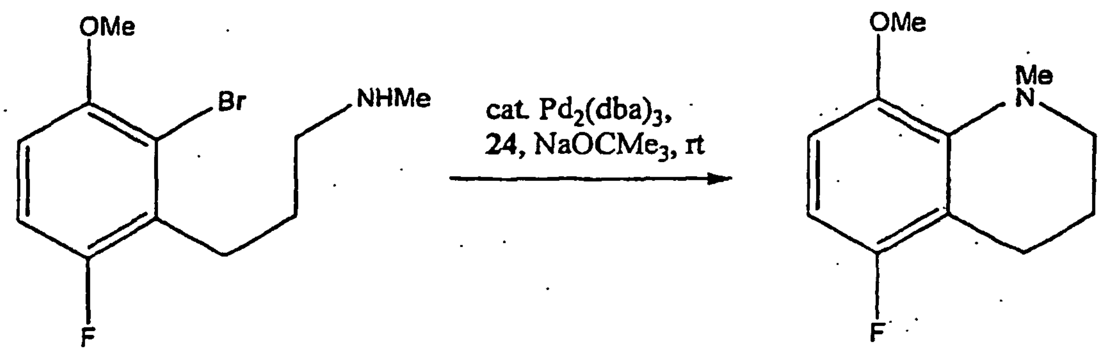


Another aspect of the Applicants' invention features a transition metal-catalyzed Suzuki cross-coupling reaction between an arylboronic acid, arylboronic ester, alkylborane, or the like and a substrate aryl bearing an activated group X. The reaction includes at least a catalytic amount of a transition metal catalyst, comprising a novel ligand, and the combination is maintained under conditions appropriate for the metal catalyst to catalyze the cross-coupling reaction between the boron-containing reactant and the substrate aryl reactant.

For illustration purposes, certain reactions using ligand 24 (not according to the invention) are shown in the following.

As an illustration of the Suzuki coupling reaction, the methods may be exploited in the preparation of 3,5-dimethoxybiphenyl, at room temperature, from 1-chloro-3,5-dimethoxybenzene and phenylboronic acid:

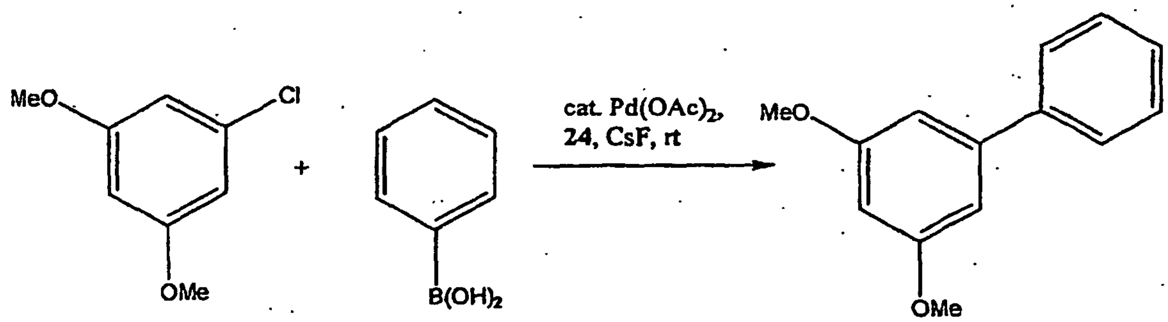


In a second illustration of the Suzuki coupling reaction, the methods may be exploited in the formation of a *sp*$^2$-*sp*$^3$ carbon-carbon bond; an electron-rich aryl chloride reacts with an alkyl borane to give an alkylarene:

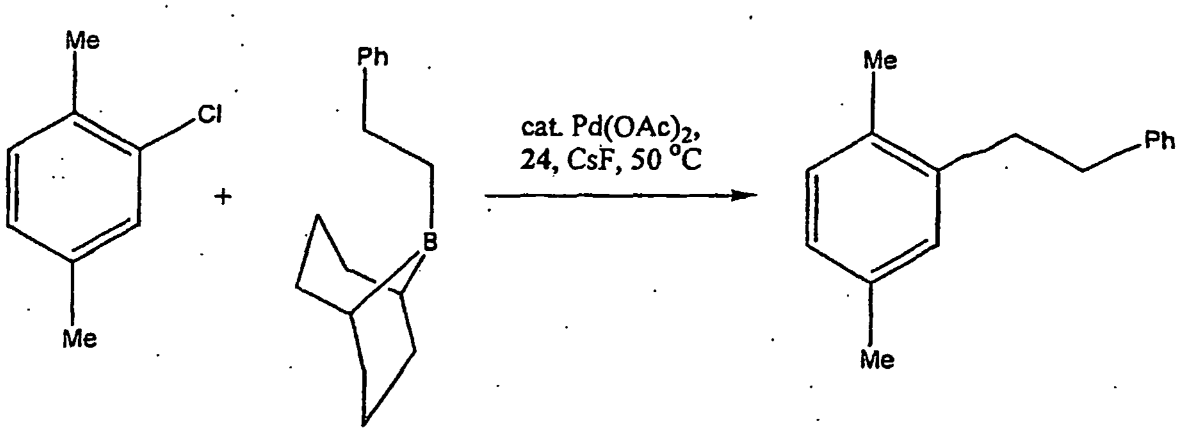


One of ordinary skill in the art will be able to envision intramolecular variants of the Suzuki coupling methods. An illustrative embodiment follows:

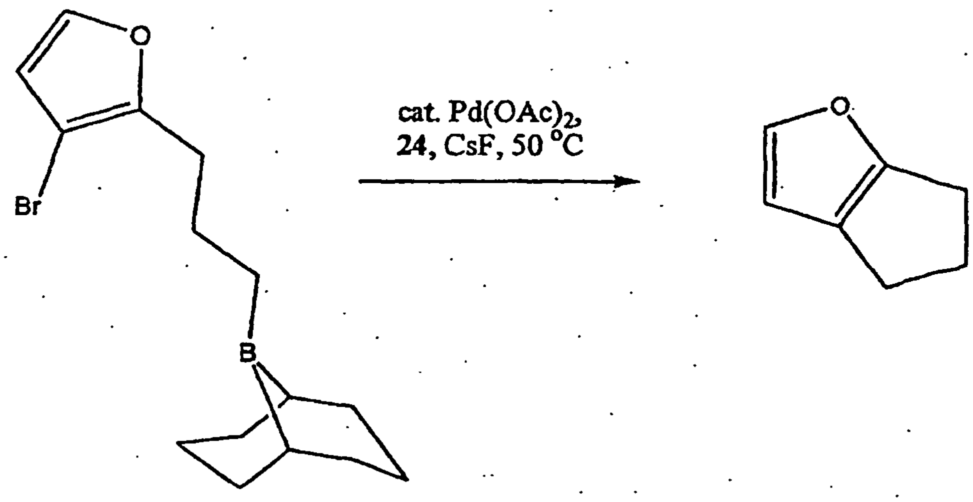


Still another aspect of the Applicants' invention features a transition metal-catalyzed α-arylation of ketones involving the reaction of an enolizable ketone with a substrate aryl bearing an activated group X. The reaction includes at least a catalytic amount of a transition metal catalyst, comprising a novel ligand, and the combination is maintained under conditions appropriate for the metal catalyst to catalyze the α-arylation of the enolizable ketone.

For illustration purposes, certain reactions using ligand 24 (not according to the invention) are shown in the following.

As an illustration of the α-arylation reaction, the methods may be exploited in the preparation of 6-methyl-2-(3,4-dimethylphenyl)cyclohexanone, at room temperature, from 1-bromo-3,4-dimethylbenzene and 2-methylcyclohexanone:

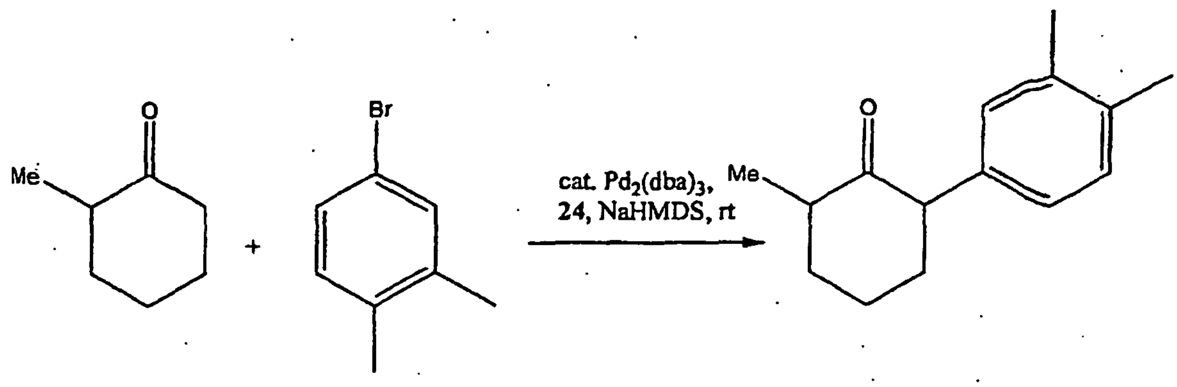


One of ordinary skill in the art will be able to envision intramolecular variants of the α-arylation methods. An illustrative reaction follows:

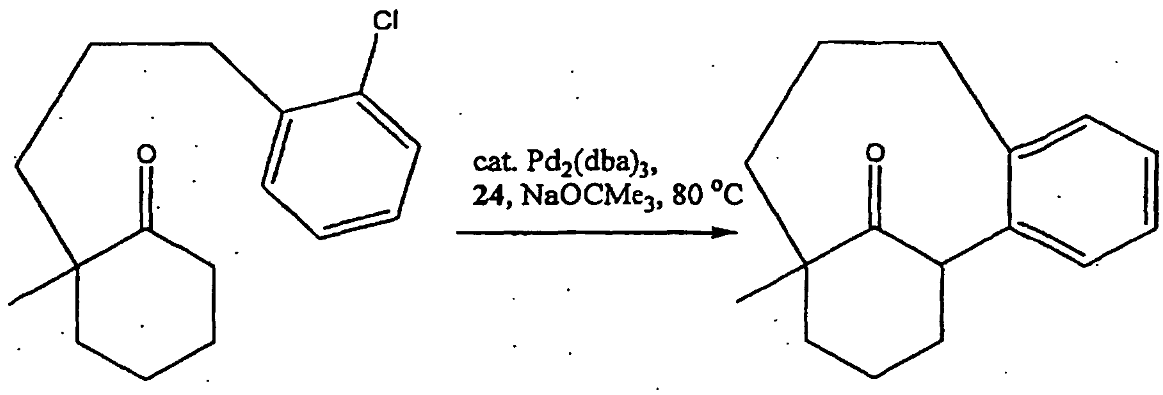


The substrate aryl compounds include compounds derived from simple aromatic rings (single or polycylic) such as benzene, naphthalene, anthracene and phenanthrene; or heteroaromatic rings (single or polycyclic), such as pyrrole, thiophene, thianthrene, furan, pyran, isobenzofuran, chromene, xanthene, phenoxathiin, pyrrole, imidazole, pyrazole, thiazole, isothiazole, isoxazole, pyridine, pyrazine, pyrimidine, pyridazine, indolizine, isoindole, indole, indazole, purine, quinolizine, isoquinoline, quinoline, phthalazine, naphthyridine, quinoxaline, quinazoline, cinnoline, pteridine, carbazole, carboline, phenanthridine, acridine, perimidine, phenanthroline, phenazine, phenarsazine, phenothiazine, furazan, phenoxazine, pyrrolidine, oxolane, thiolane, oxazole, piperidine, piperazine, morpholine and the like. In preferred embodiment, the reactive group, X, is substituted on a five, six or seven membered ring (though it can be part of a larger polycycle).

In preferred embodiments, the aryl substrate may be selected from the group consisting of phenyl and phenyl derivatives, heteroaromatic compounds, polycyclic aromatic and heteroaromatic compounds, and functionalized derivatives thereof. Suitable aromatic compounds derived from simple aromatic rings and heteroaromatic rings, include but are not limited to, pyridine, imidizole, quinoline, furan, pyrrole, thiophene, and the like. Suitable aromatic compounds derived from fused ring systems, include but are not limited to naphthalene, anthracene, tetralin, indole and the like. Suitable aromatic compounds may have the formula $Z_pArX$, where X is an activated substituent. An activated substituent, X, is characterized as being a good leaving group. In general, the leaving group is a group such as a halide or sulfonate. Suitable activated substituents include, by way of example only, halides such as chloride, bromide and iodide, and sulfonate esters such as triflate, mesylate, nonaflate and tosylate. In certain embodiments, the leaving group is a halide selected from iodine, bromine, and chlorine.

Z represents one or more optional substituents on the aromatic ring, though each occurence of Z (p>1) is independently selected. By way of example only, each incidence of substitution independently can be, as valence and stability permit, a halogen, a lower alkyl, a lower alkenyl, a lower alkynyl, a carbonyl (e.g., an ester, a carboxylate, or a formate), a thiocarbonyl (e.g., a thiolester, a thiolcarboxylate, or a thiolformate), a ketyl, an aldehyde, an amino, an acylamino, an amido, an amidino, a cyano, a nitro, an azido, a sulfonyl, a sulfoxido, a sulfate, a sulfonate, a sulfamoyl, a sulfonamido, a phosphoryl, a phosphonate, a phosphinate, $-(CH_2)_m-R_8$, $-(CH_2)_m-OH$, $-(CH_2)_m$-O-lower alkyl, $-(CH_2)_m$-O-lower alkenyl, $-(CH_2)_m-O-(CH_2)_n-R_8$, $-(CH_2)_m-SH$, $-(CH_2)_m$-S-lower alkyl, $-(CH_2)_m$-S-lower alkenyl, $-(CH_2)_m-S-(CH_2)_n-R_8$, or protecting groups of the above or a solid or polymeric support; $R_8$ represents a substituted or unsubstituted aryl, aralkyl, cycloalkyl, cycloalkenyl, or heterocycle; and n and m are independently for each occurrence zero or an integer in the range of 1 to 6. P is preferably in the range of 0 to 5. For fused rings, where the number of substitution sites on the aryl group increases, p may be adjusted appropriately.

In certain embodiments, suitable substituents Z include alkyl, aryl, acyl, heteroaryl, amino, carboxylic ester, carboxylic acid, hydrogen, ether, thioether, amide, carboxamide, nitro, phosphonic acid, hydroxyl, sulfonic acid, halide, pseudohalide groups, and substituted derivatives thereof, and p is in the range of 0 to 5. In particular, the reaction is anticipated to be compatible with acetals, amides and silyl ethers. For fused rings, where the number of substitution sites on the aromatic ring increases, p may be adjusted appropriately.

A wide variety of substrate aryl groups are useful in the methods of the present invention. The choice of substrate will depend on factors such as the amine, boronic acid, ketone, or the like to be employed and the desired product, and an appropriate aryl substrate will be made apparent to the skilled artisan by these teachings. It will be understood that the aryl substrate preferably will not contain any interfering functionalities. It will further be understood that not all activated aryl substrates will react with every amine, boronic acid, ketone, or the like.

The reactive the amine, boronic acid, ketone, or the like can be a molecule separate from the substrate aryl group, or a substituent of the same molecule (e.g., for intramolecular variations).

The amine, boronic acid, ketone, or the like is selected to provide the desired reaction product. The amine, boronic acid, ketone, or the like may be functionalized. The amine, boronic acid, ketone, or the like may be selected

from a wide variety of structural types, including but not limited to, acyclic, cyclic or heterocyclic compounds, fused ring compounds or phenol derivatives. The aromatic compound and the amine, boronic acid, ketone, or the like may be included as moieties of a single molecule, whereby the arylation reaction proceeds as an intramolecular reaction.

In certain embodiments, the amine, boronic acid, ketone, or the like is generated *in situ* by conversion of a precursor under the reaction conditions.

In certain embodiments, the aryl substrate and/or the amine, boronic acid, ketone, or the like is attached, either directly or via a tether, to a solid support.

Alternatively, the corresponding salt of the amine, boronic acid, ketone, or the like, may be prepared and used in place of the amine, boronic acid, ketone, or the like. When the corresponding salt of the amine, boronic acid, ketone, or the like is used in the reaction, an additional base may not be required.

The active form of the transition metal catalyst is not well characterized. Therefore, it is contemplated that the "transition metal catalyst" of the present invention, as that term is used herein, shall include any catalytic transition metal and/or catalyst precursor as it is introduced into the reaction vessel and which is, if necessary, converted *in situ* into the active form, as well as the active form of the catalyst which participates in the reaction.

In preferred embodiments, the transition metal catalyst complex is provided in the reaction mixture is a catalytic amount. In certain embodiments, that amount is in the range of 0.0001 to 20 mol%, and preferably 0.05 to 5 mol%, and most preferably 1-3 mol%, with respect to the limiting reagent, which may be either the aromatic compound the amine, boronic acid, ketone, or the like (or the corresponding salt thereof), depending upon which reagent is in stoichiometric excess. In the instance where the molecular formula of the catalyst complex includes more than one metal, the amount of the catalyst complex used in the reaction may be adjusted accordingly. By way of example, $Pd_2(dba)_3$ has two metal centers; and thus the molar amount of $Pd_2(dba)_3$ used in the reaction may be halved without sacrificing catalytic activity.

Catalysts containing palladium and nickel are preferred. It is expected that these catalysts will perform similarly because they are known to undergo similar reactions, namely oxidative-addition reactions and reductive-elimination reactions, which are thought to be involved in the formation of the products of the present invention. The novel ligands are thought to modify the catalyst performance by, for example, modifying reactivity and preventing undesirable side reactions.

As suitable, the catalysts employed in the subject method involve the use of metals which can mediate cross-coupling of the aryl groups ArX and the amine, boronic acid, ketone, or the like as defined above. In general, any transition metal (e.g., having d electrons) may be used to form the catalyst, e.g., a metal selected from one of Groups 3-12 of the periodic table or from the lanthanide series. However, in preferred embodiments, the metal will be selected from the group of late transition metals, e.g. preferably from Groups 5-12 and even more preferably Groups 7-11. For example, suitable metals include platinum, palladium, iron, nickel, ruthenium and rhodium. The particular form of the metal to be used in the reaction is selected to provide, under the reaction conditions, metal centers which are coordinately unsaturated and not in their highest oxidation state. The metal core of the catalyst should be a zero valent transition metal, such as Pd or Ni with the ability to undergo oxidative addition to Ar-X bond. The zero-valent state, M(0), may be generated in situ, e.g., from M(II).

To further illustrate, suitable transition metal catalysts include soluble or insoluble complexes of platinum, palladium and nickel. Nickel and palladium are particularly preferred and palladium is most preferred. A zero-valent metal center is presumed to participate in the catalytic carbon-heteroatom or carbon-carbon bond forming sequence. Thus, the metal center is desirably in the zero-valent state or is capable of being reduced to metal(0). Suitable soluble palladium complexes include, but are not limited to, tris(dibenzylideneacetone) dipalladium [$Pd_2(dba)_3$], bis(dibenzylideneacetone) palladium [$Pd(dba)_2$] and palladium acetate. Alternatively, particularly for nickel catalysts, the active species for the oxidative-addition step may be in the metal (+1) oxidation state.

Catalysts containing palladium and nickel are preferred. It is expected that these catalysts will perform comparably because they are known in the art to undergo similar reactions, namely cross-coupling reactions, which may be involved in the formation of the products of the present invention, e.g., arylamines, diaryls, $\alpha$-arylketones, or the like.

The coupling can be catalyzed by a palladium catalyst which palladium may be provided in the form of, for illustrative purposes only, Pd/C, $PdCl_2$, $Pd(OAc)_2$, $(CH_3CN)_2PdCl_2$, $Pd[P(C_6H_5)_3]_4$, and polymer supported Pd(0). In other embodiments, the reaction can be catalyzed by a nickel catalyst which nickel may be provided in the form of, for illustrative purposes only, $Ni(acac)_2$, $NiCl_2[P(C_6H_5)]_2$, Ni(1,5-cyclooctadiene)$_2$, Ni(1,10-phenanthroline)$_2$, $Ni(dppf)_2$, $NiCl_2$(dppf), $NiCl_2$(1,10.phenanthroline), Raney nickel and the like, wherein "acac" represents acetylacetonate.

The catalyst will preferably be provided in the reaction mixture as metal-ligand complex comprising a bound supporting ligand, that is, a metal-supporting ligand complex. The ligand effects can be key to favoring, *inter alia,* the reductive elimination pathway or the like which produces the products, rather than side reactions such as $\beta$-hydride elimination. In preferred embodiments, the subject reaction employs bidentate ligands such as bisphosphines or aminophosphines. The ligand, if chiral can be provided as a racemic mixture or a purified stereoisomer. In certain instances, e.g. the improved method for the synthesis of aryl amines, the use of a racemic, chelating ligand is preferred.

The ligand, as described in greater detail below, may be a chelating ligand. Weakly or non-nucleophilic stabilizing

ions are preferred to avoid undesired side reactions involving the counter ion. The catalyst complex may include additional ligands as required to obtain a stable complex. Moreover, the ligand can be added to the reaction mixture in the form of a metal complex, or added as a separate reagent relative to the addition of the metal.

The supporting ligand may be added to the reaction solution as a separate compound or it may be complexed to the metal center to form a metal-supporting ligand complex prior to its introduction into the reaction solution. Supporting ligands are compounds added to the reaction solution which are capable of binding to the catalytic metal center. In some preferred embodiments, the supporting ligand is a chelating ligand. Although not bound by any theory of operation, it is hypothesized that the supporting ligands suppress unwanted side reactions as well as enhance the rate and efficiency of the desired processes. Additionally, they typically prevent precipitation of the catalytic transition metal. Although the present invention does not require the formation of a metal-supporting ligand complex, such complexes have been shown to be consistent with the postulate that they are intermediates in these reactions and it has been observed the selection of the supporting ligand has an affect on the course of the reaction.

The supporting ligand is present in the range of 0.0001 to 40 mol% relative to the limiting reagent, i.e., amine, boronic acid, ketone or the like, or aromatic compound. The ratio of the supporting ligand to catalyst complex is typically in the range of about 1 to 20, and preferably in the range of about 1 to 4 and most preferably 2. These ratios are based upon a single metal complex and a single binding site ligand. In instances where the ligand contains additional binding sites (i.e., a chelating ligand) or the catalyst contains more than one metal, the ratio is adjusted accordingly.

In certain embodiments of the subject method the transition metal catalyst includes a ligand of general structure 7 and optionally one or more phosphine or aminophosphine ligands, e.g., as a Lewis basic ligand that controls the stability and electron transfer properties of the transition metal catalyst, and/or stabilizes the metal intermediates. Phosphine ligands are commercially available or can be prepared by methods similar to known processes. The phosphines can be monodentate phosphine ligands, such as trimethylphosphine, triethylphosphine, tripropylphosphine, triisopropylphosphine, tributylphosphine, tricyclohexylphosphine, trimethyl phosphite, triethyl phosphite, tripropyl phosphite, triisopropyl phosphite, tributyl phosphite and tricyclohexyl phosphite, in particular triphenylphosphine, tri(*o*-tolyl)phosphine, triisopropylphosphine or tricyclohexylphosphine; or a bidentate phosphine ligand such as 2,2'-bis(diphenylphosphino)-1,1'-binaphthyl (BINAP), 1,2-bis(dimethylphosphino)ethane, 1,2-bis(diethylphosphino)ethane, 1,2-bis(dipropylphosphino)ethane, 1,2-bis(diisopropylphosphino)ethane, 1,2-bis(dibutylphosphino)ethane, 1,2-bis(dicyclohexylphosphino) ethane, 1,3-bis(dicyclohexylphosphino)propane, 1,3-bis(diiso-propylphosphino)propane, 1,4-bis(diisopropylphosphino)-butane and 2,4-bis(dicyclohexylphosphino)pentane. The aminophosphines may be monodentate, e.g. each molecule of aminophosphine donates to the catalytic metal atom only a Lewis basic nitrogen atom or a Lewis basic phosphorus atom. Alternatively, the aminophosphine may be a chelating ligand, e.g. capable of donating to the catalytic metal atom both a Lewis basic nitrogen atom and a Lewis basic phosphorus atom.

In some instances, it may be necessary to include additional reagents in the reaction mixture to promote reactivity of either the transition metal catalyst or activated aryl nucleus. In particular, it may be advantageous to include a suitable base. In general, a variety of bases may be used in practice of the present invention. It has not been determined at which point(s) in the mechanisms of the subject transformations the base participates. The base may optionally be sterically hindered to discourage metal coordination of the base in those circumstances where such coordination is possible. i.e., alkali metal alkoxides. Exemplary bases include such as, by way of example only: alkoxides such as sodium *tert*-butoxide; alkali metal amides such as sodium amide, lithium diisopropylamide, and alkali metal bis(trialkylsilyl) amide, e.g., such as lithium bis(trimethylsuyl)amide (LiHMDS) or sodium bis(trimethylsilyl)amide (NaHMDS); tertiary amines (e.g. triethylamine, trimethylamine, 4-(dimethylamino)pyridine (DMAP), 1,5-diazabicycl[4.3.0]non-5-ene (DBN), 1,5-diazabicyclo[5.4.0]undec-5-ene (DBU); alkali or alkaline earth carbonate, bicarbonate or hydroxide (e.g. sodium, magnesium, calcium, barium, potassium carbonate, phosphate, hydroxide and bicarbonate). By way of example only, suitable bases include NaH, LiH, KH, $K_2CO_3$, $Na_2CO_3$, $Tl_2CO_3$, $Cs_2CO_3$, K(OtBu), Li(O*t*Bu), Na(O*t*Bu) K(OAr), Na(OAr), and triethylamine, or mixtures thereof. Preferred bases include CsF, $K_3PO_4$, DBU, NaOt-Bu, KOt-Bu, $LiN(i\text{-}Pr)_2$ (LDA), $KN(SiMe_3)_2$, $NaN(SiMe_3)_2$, and $LiN(SiMe_3)_2$.

Base is used in approximately stoichiometric proportions in the subject methods. The present invention has demonstrated that there is no need for large excesses of base in order to obtain good yields of the desired products under mild reaction conditions. No more than four equivalents of base, and preferably no more than two equivalents, are needed. Furthermore, in reactions using the corresponding salt of an amine, boronic acid, ketone or the like, additional base may not be required.

As is clear from the above discussion, the products which may be produced by the amination, Suzuki coupling, and $\alpha$-arylation reactions of this invention can undergo further reaction(s) to afford desired derivatives thereof. Such permissible derivatization reactions can be carried out in accordance with conventional procedures known in the art. For example, potential derivatization reactions include esterification, oxidation of alcohols to aldehydes and acids, *N*-alkylation of amides, nitrile reduction, acylation of alcohols by esters, acylation of amines and the like.

*IV. Reaction Conditions*

The reactions of the present invention may be performed under a wide range of conditions, though it will be understood that the solvents and temperature ranges recited herein are not limitative and only correspond to a preferred mode of the process of the invention.

In general, it will be desirable that reactions are run using mild conditions which will not adversely affect the reactants, the catalyst, or the product. For example, the reaction temperature influences the speed of the reaction, as well as the stability of the reactants and catalyst. The reactions will usually be run at temperatures in the range of 25˚C to 300˚C, more preferably in the range 25˚C to 150˚C.

In general, the subject reactions are carried out in a liquid reaction medium. The reactions may be run without addition of solvent. Alternatively, the reactions may be run in an inert solvent, preferably one in which the reaction ingredients, including the catalyst, are substantially soluble. Suitable solvents include ethers such as diethyl ether, 1,2-dimethoxyethane, diglyme, t-butyl methyl ether, tetrahydrofuran and the like; halogenated solvents such as chloroform, dichloromethane, dichloroethane, chlorobenzene; and the like; aliphatic or aromatic hydrocarbon solvents such as benzene, xylene, toluene, hexane, pentane and the like; esters and ketones such as ethyl acetate, acetone, and 2-butanone; polar aprotic solvents such as acetonitrile, dimethylsulfoxide, dimethylformamide and the like; or combinations of two or more solvents.

The invention also contemplates reaction in a biphasic mixture of solvents, in an emulsion or suspension, or reaction in a lipid vesicle or bilayer. In certain embodiments, it may be preferred to perform the catalyzed reactions in the solid phase with one of the reactants anchored to a solid support.

In certain embodiments it is preferable to perform the reactions under an inert atmosphere of a gas such as nitrogen or argon.

The reaction processes of the present invention can be conducted in continuous, semi-continuous or batch fashion and may involve a liquid recycle operation as desired. The processes of this invention are preferably conducted in batch fashion. Likewise, the manner or order of addition of the reaction ingredients, catalyst and solvent are also not generally critical to the success of the reaction, and may be accomplished in any conventional fashion. In a order of events that, in some cases, can lead to an enhancement of the reaction rate, the base, e.g. *t*-BuONa, is the last ingredient to be added to the reaction mixture.

The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials to the metal catalyst. When complete conversion is not desired or not obtainable, the starting materials can be separated from the product and then recycled back into the reaction zone.

The processes may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

Furthermore, one or more of the reactants can be immobilized or incorporated into a polymer or other insoluble matrix by, for example, derivativation with one or more of substituents of the aryl group.

*V. Combinatorial Libraries*

The subject reactions readily lend themselves to the creation of combinatorial libraries of compounds for the screening of pharmaceutical, agrochemical or other biological or medically-related activity or material-related qualities. A combinatorial library for the purposes of the present invention is a mixture of chemically related compounds which may be screened together for a desired property; said libraries may be in solution or covalently linked to a solid support or soluble polymer, or is adsorbed onto a solid. The preparation of many related compounds in a single reaction greatly reduces and simplifies the number of screening processes which need to be carried out. Screening for the appropriate biological, pharmaceutical, agrochemical or physical property may be done by conventional methods.

Diversity in a library can be created at a variety of different levels. For instance, the substrate aryl groups used in a combinatorial approach can be diverse in terms of the core aryl moiety, e.g., a variegation in terms of the ring structure, and/or can be varied with respect to the other substituents.

A variety of techniques are available in the art for generating combinatorial libraries of small organic molecules. See, for example, Blondelle et al. (1995) Trends Anal. Chem. 14:83; the Affymax U.S. Patents 5,359,115 and 5,362,899:

the Ellman U.S. Patent 5,288,514: the Still et al. PCT publication WO 94/08051; Chen et al. (1994) JACS 116:2661: Kerr et al. (1993) JACS 115:252; PCT publications WO92/10092, WO93/09668 and WO91/07087; and the Lerner et al. PCT publication WO93/20242). Accordingly, a variety of libraries on the order of about 16 to 1,000,000 or more diversomers can be synthesized and screened for a particular activity or property.

In an exemplary embodiment, a library of substituted diversomers can be synthesized using the subject reactions adapted to the techniques described in the Still et al. PCT publication WO 94/08051, e.g., being linked to a polymer bead by a hydrolyzable or photolyzable group, e.g., located at one of the positions of substrate. According to the Still et al. technique, the library is synthesized on a set of beads, each bead including a set of tags identifying the particular diversomer on that bead. In one embodiment, which is particularly suitable for discovering enzyme inhibitors, the beads can be dispersed on the surface of a permeable membrane, and the diversomers released from the beads by lysis of the bead linker. The diversomer from each bead will diffuse across the membrane to an assay zone, where it will interact with an enzyme assay. Detailed descriptions of a number of combinatorial methodologies are provided below.

A) Direct Characterization

A growing trend in the field of combinatorial chemistry is to exploit the sensitivity of techniques such as mass spectrometry (MS), e.g., which can be used to characterize sub-femtomolar amounts of a compound, and to directly determine the chemical constitution of a compound selected from a combinatorial library. For instance, where the library is provided on an insoluble support matrix, discrete populations of compounds can be first released from the support and characterized by MS. In other embodiments, as part of the MS sample preparation technique, such MS techniques as MALDI can be used to release a compound from the matrix, particularly where a labile bond is used originally to tether the compound to the matrix. For instance, a bead selected from a library can be irradiated in a MALDI step in order to release the diversomer from the matrix, and ionize the diversomer for MS analysis.

B) Multipin Synthesis

The libraries of the subject method can take the multipin library format Briefly, Geysen and co-workers (Geysen et al. (1984) PNAS 81:3998-4002) introduced a method for generating compound libraries by a parallel synthesis on -polyacrylic acid-grated polyethylene pins arrayed in the microtitre plate format. The Geysen technique can be used to synthesize and screen thousands of compounds per week using the multipin method, and the tethered compounds may be reused in many assays. Appropriate linker moieties can also been appended to the pins so that the compounds may be cleaved from the supports after synthesis for assessment of purity and further evaluation (c.f., Bray et al. (1990) Tetrahedron Lett 31:5811-5814; Valerio et al. (1991) Anal Biochem 197:168-177; Bray et al. (1991) Tetrahedron Lett 32:6163-6166).

C) Divide-Couple-Recombine

In yet another embodiment, a variegated library of compounds can be provided on a set of beads utilizing the strategy of divide-couple-recombine (see, e.g., Houghten (1985) PNAS 82:5131-5135; and U.S. Patents 4,631,211; 5,440,016; 5,480,971). Briefly, as the name implies, at each synthesis step where degeneracy is introduced into the library, the beads are divided into separate groups equal to the number of different substituents to be added at a particular position in the library, the different substituents coupled in separate reactions, and the beads recombined into one pool for the next iteration.

In one embodiment, the divide-couple-recombine strategy can be carried out using an analogous approach to the so-called "tea bag" method first developed by Houghten, where compound synthesis occurs on resin sealed inside porous polypropylene bags (Houghten et al. (1986) PNAS 82:5131-5135). Substituents are coupled to the compound-bearing resins by placing the bags in appropriate reaction solutions, while all common steps such as resin washing and deprotection are performed simultaneously in one reaction vessel. At the end of the synthesis, each bag contains a single compound.

D) Combinatorial Libraries by Light-Directed, Spatially Addressable Parallel Chemical Synthesis

A scheme of combinatorial synthesis in which the identity of a compound is given by its locations on a synthesis substrate is termed a spatially-addressable synthesis. In one embodiment, the combinatorial process is carried out by controlling the addition of a chemical reagent to specific locations on a solid support (Dower et al. (1991) Annu Rep Med Chem 26:271-280; Fodor, S.P.A. (1991) Science 251:767; Pirrung et al. (1992) U.S. Patent No. 5,143,854; Jacobs et al. (1994) Trends Biotechnol 12:19-26). The spatial resolution of photolithography affords miniaturization. This technique can be carried out through the use protection/deprotection reactions with photolabile protecting groups.

The key points of this technology are illustrated in Gallop et al. (1994) J Med Chem 37:1233-1251. A synthesis substrate is prepared for coupling through the covalent attachment of photolabile nitroveratryloxycarbonyl (NVOC) protected amino linkers or other photolabile linkers. Light is used to selectively activate a specified region of the synthesis support for coupling. Removal of the photolabile protecting groups by light (deprotection) results in activation of selected areas. After activation, the first of a set of amino acid analogs, each bearing a photolabile protecting group on the amino terminus, is exposed to the entire surface. Coupling only occurs in regions that were addressed by light in the preceding step. The reaction is stopped, the plates washed, and the substrate is again illuminated through a second mask, activating a different region for reaction with a second protected building block. The pattern of masks and the sequence of reactants define the products and their locations. Since this process utilizes photolithography techniques, the number of compounds that can be synthesized is limited only by the number of synthesis sites that can be addressed with appropriate resolution. The position of each compound is precisely known; hence, its interactions with other molecules can be directly assessed.
In a light-directed chemical synthesis, the products depend on the pattern of illumination and on the order of addition of reactants. By varying the lithographic patterns, many different sets of test compounds can be synthesized simultaneously; this characteristic leads to the generation of many different masking strategies.

E) Encoded Combinatorial Libraries

In yet another embodiment, the subject method utilizes a compound library provided with an encoded tagging system. A recent improvement in the identification of active compounds from combinatorial libraries employs chemical indexing systems using tags that uniquely encode the reaction steps a given bead has undergone and, by inference, the structure it carries. Conceptually, this approach mimics phage display libraries, where activity derives from expressed peptides, but the structures of the active peptides are deduced from the corresponding genomic DNA sequence. The first encoding of synthetic combinatorial libraries employed DNA as the code. A variety of other forms of encoding have been reported, including encoding with sequenceable bio-oligomers (e.g., oligonucleotides and peptides), and binary encoding with additional non-sequenceable tags.

1) Tagging with sequenceable bio-oligomers

The principle of using oligonucleotides to encode combinatorial synthetic libraries was described in 1992 (Brenner et al. (1992) PNAS 89:5381-5383), and an example of such a library appeared the following year (Needles et al. (1993) PNAS 90:10700-10704). A combinatorial library of nominally $7^7$ (= 823,543) peptides composed of all combinations of Arg, Gln, Phe, Lys, Val, D-Val and Thr (three-letter amino acid code), each of which was encoded by a specific dinucleotide (TA, TC, CT, AT, TT, CA and AC, respectively), was prepared by a series of alternating rounds of peptide and oligonucleotide synthesis on solid support. In this work, the amine linking functionality on the bead was specifically differentiated toward peptide or oligonucleotide synthesis by simultaneously preincubating the beads with reagents that generate protected OH groups for oligonucleotide synthesis and protected $NH_2$ groups for peptide synthesis (here, in a ratio of 1:20). When complete, the tags each consisted of 69-mers, 14 units of which carried the code. The bead-bound library was incubated with a fluorescently labeled antibody, and beads containing bound antibody that fluoresced strongly were harvested by fluorescence-activated cell sorting (FACS). The DNA tags were amplified by PCR and sequenced, and the predicted peptides were synthesized. Following such techniques, compound libraries can be derived for use in the subject method, where the oligonucleotide sequence of the tag identifies the sequential combinatorial reactions that a particular bead underwent, and therefore provides the identity of the compound on the bead.
The use of oligonucleotide tags permits exquisitely sensitive tag analysis. Even so, the method requires careful choice of orthogonal sets of protecting groups required for alternating co-synthesis of the tag and the library member. Furthermore, the chemical lability of the tag, particularly the phosphate and sugar anomeric linkages, may limit the choice of reagents and conditions that can be employed for the synthesis of non-oligomeric libraries. In preferred embodiments, the libraries employ linkers permitting selective detachment of the test compound library member for assay.
Peptides have also been employed as tagging molecules for combinatorial libraries. Two exemplary approaches are described in the art, both of which employ branched linkers to solid phase upon which coding and ligand strands are alternately elaborated. In the first approach (Kerr JM et al. (1993) J Am Chem Soc 115:2529-2531), orthogonality in synthesis is achieved by employing acid-labile protection for the coding strand and base-labile protection for the compound strand.
In an alternative approach (Nikolaiev et al. (1993) Pept Res 6:161-170), branched linkers are employed so that the coding unit and the test compound can both be attached to the same functional group on the resin. In one embodiment, a cleavable linker can be placed between the branch point and the bead so that cleavage releases a molecule containing both code and the compound (Ptek et al. (1991) Tetrahedron Lett 32:3891-3894). In another embodiment, the cleavable linker can be placed so that the test compound can be selectively separated from the bead, leaving the code behind. This last construct is particularly valuable because it permits screening of the test compound

without potential interference of the coding groups. Examples in the art of independent cleavage and sequencing of peptide library members and their corresponding tags has confirmed that the tags can accurately predict the peptide structure.

2) Non-sequenceable Tagging: Binary Encoding

An alternative form of encoding the test compound library employs a set of non-sequencable electrophoric tagging molecules that are used as a binary code (Ohlmeyer et al. (1993) PNAS 90:10922-10926). Exemplary tags are haloaromatic alkyl ethers that are detectable as their trimethylsilyl ethers at less than femtomolar levels by electron capture gas chromatography (ECGC). Variations in the length of the alkyl chain, as well as the nature and position of the aromatic halide substituents, permit the synthesis of at least 40 such tags, which in principle can encode $2^{40}$ (e.g., upwards of $10^{12}$) different molecules. In the original report (Ohlmeyer et al., supra) the tags were bound to about 1% of the available amine groups of a peptide library via a photocleavable o-nitrobenzyl linker. This approach is convenient when preparing combinatorial libraries of peptide-like or other amine-containing molecules. A more versatile system has, however, been developed that permits encoding of essentially any combinatorial library. Here, the compound would be attached to the solid support via the photocleavable linker and the tag is attached through a catechol ether linker via carbene insertion into the bead matrix (Nestler et al. (1994) J Org Chem 59:4723-4724). This orthogonal attachment strategy permits the selective detachment of library members for assay in solution and subsequent decoding by ECGC after oxidative detachment of the tag sets.

Although several amide-linked libraries in the art employ binary encoding with the electrophoric tags attached to amine groups, attaching these tags directly to the bead matrix provides far greater versatility in the structures that can be prepared in encoded combinatorial libraries. Attached in this way, the tags and their linker are nearly as unreactive as the bead matrix itself. Two binary-encoded combinatorial libraries have been reported where the electrophoric tags are attached directly to the solid phase (Ohlmeyer et al. (1995) PNAS 92:6027-6031) and provide guidance for generating the subject compound library. Both libraries were constructed using an orthogonal attachment strategy in which the library member was linked to the solid support by a photolabile linker and the tags were attached through a linker cleavable only by vigorous oxidation. Because the library members can be repetitively partially photoeluted from the solid support, library members can be utilized in multiple assays. Successive photoelution also permits a very high throughput iterative screening strategy: first, multiple beads are placed in 96-well microtiter plates; second, compounds are partially detached and transferred to assay plates; third, a metal binding assay identifies the active wells; fourth, the corresponding beads are rearrayed singly into new microtiter plates; fifth, single active compounds are identified; and sixth, the structures are decoded.

***Examples***

The invention may be understood with reference to the following examples, which are presented for illustrative purposes only and which are non-limiting. The substrates utilized in these examples were either commercially available, or were prepared from commercially available.reagents.

Ligands 1 and 2 are not according to the invention.

***Example 1***

A Highly Active Catalyst for Palladium-Catalyzed Cross-Coupling Reactions: Room Temperature Suzuki Couplings and Amination of Unactivated Aryl Chlorides

A highly active palladium catalyst which employs the aminophosphine ligand 1-(*N,N*-dimethylamino)-1'-(dicyclohexylphosphino)biphenyl (2) has been developed. This catalyst is effective for the cross-coupling of aryl chlorides with amines, boronic acids, and ketone enolates. The system is sufficiently reactive to allow for the room temperature amination of aryl bromides and electron-deficient aryl chlorides, and promotes room temperature Suzuki coupling reactions of both electron-rich and electron-deficient aryl chlorides. The coordination of the amine moiety may be key to the enhanced reactivity and catalyst stability of this system.

Palladium-catalyzed C-N bond-forming reactions have evolved into a versatile and efficient synthetic transformation. The use of palladium catalysts supported by bidentate phosphine ligands has made possible substitution of aryl halides and triflates with nitrogen,[1] oxygen,[2] and certain carbon nucleophiles.[3] The lack of a general palladium-based catalyst for aryl chloride substitution reactions,[4,5] as well as the elevated reaction temperatures often required prompted us to search for new ligands which might overcome these limitations.

$^1$H NMR studies in our laboratories of the amination reactions of aryl bromides catalyzed by BINAP/$Pd(OAc)_2$ suggested that oxidative addition was rate limiting.[6] For aryl chlorides, oxidative addition can be anticipated to be even

more sluggish. To facilitate this slow step, we began to explore the use of electron-rich phosphine ligands.[4, 5d, 7a] An initial experiment which employed $PCy_3$ as the palladium-supporting ligand demonstrated that although this type of catalyst was capable of activating the carbon-chlorine bond, the process suffered from facile β-hydride elimination and subsequent formation of reduced arene.[5a] Based on our knowledge that bidentate ligands suppressed β-hydride elimination in arylations of primary amines,[1c] we focused our efforts on the preparation of electron-rich bidentate phosphines.[6] We first prepared the known 2,2-bis(dicyclohexylphosphino)binaphthyl (**1**).[8] Initial screening demonstrated that **1**/Pd(0) constituted a reasonably effective catalyst for the coupling of pyrrolidine with chlorotoluene. This important result, taken together with our experience with bidentate monophosphines PPF-OMe and PPFA[1d] prompted us to prepare aminophosphine ligand **2**.[9] In comparison to **1**, use of ligand **2** is generally superior and significantly expands the scope of palladium-catalyzed aryl chloride transformations. Herein, we demonstrate that the **2**/Pd(0) catalyst system is highly active and allows for the room temperature amination of aryl bromides and the first example of a room temperature amination of an aryl chloride. Moreover, this system functions as the first general catalyst for room temperature Suzuki coupling reactions of aryl chlorides.

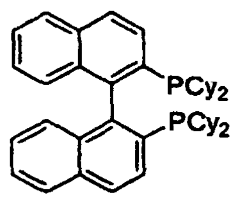 **1** 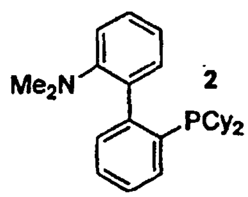 **2**

To demonstrate the efficacy of the **2**/Pd(0) catalyst system, we have prepared several aniline derivatives from aryl chlorides (Table 1, entries 1-2,4-6, 8-9, 13, 16). Secondary amines give excellent results in the coupling procedure (Table 1, entries 1-2, 4-6, 8-9), and the arylation of a primary aniline can also be accomplished (Table 1, entry 16). Primary alkyl amines are efficient coupling partners provided the aryl chloride is substituted at the ortho position (Table 1, entry 13), or through the use of ligand **1** (Table 1, entries 14,17). Catalyst levels as low as 0.05 mol% Pd have been achieved in the reaction of chlorotoluene with di-*n*-butylamine (Table 1, entry 1).

Given the high reactivity of this catalyst, we explored the possibility of carrying out room temperature aminations. We found that both aryl iodides and aryl bromides (Table 1, entries 3, 7, 10, 15) reacted readily at room temperature when DME was employed as the solvent The experimentally simple procedure did not require crown ether or other additives.[1e] Broadly speaking, the room temperature amination of aryl bromides displays the same scope as the reactions of aryl chlorides at 80 °C. Aryl bromides containing functional groups sensitive to NaO*t*-Bu could be converted to the corresponding aniline derivative by using $K_3PO_4$ as the base. In these reactions (Table 1, entries 11 and 12), heating at 80 °C was required due to the decreased basicity and/or solubility of $K_3PO_4$.

Using **2**/Pd(0) the first amination of an aryl chloride (albeit an activated one) at room temperature could also be achieved for the first time.[10] Thus, the coupling of *p*-chlorobenzonitrile and morpholine was catalyzed by 2.5 mol% $Pd_2(dba)_3$, 7.5 mol% **2** and NaO*t*-Bu in DME at room temperature to provide the corresponding aniline derivative in 96% yield (Table 1, entry 9).

**Table 1: Catalytic Amination[a] of Aryl Chlorides and Bromides**

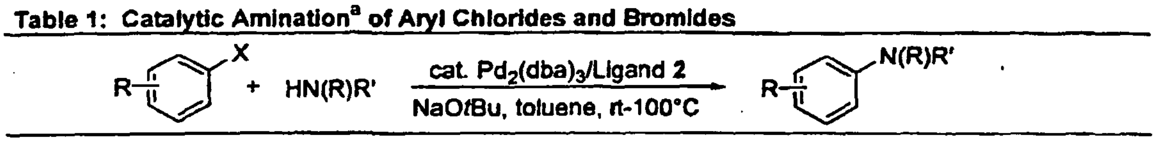


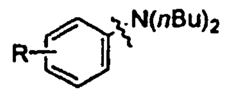

1: R=4-Me, X=Cl; 95%
2: R=4-MeO, X=Cl; 90%
3: R=4-Me, X=Br; 96%[b,c,k]

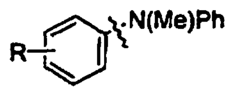

4: R=4-Me, X=Cl; 98%
5: R=4-MeO, X=Cl; 95%
6: R=2,5-$Me_2$, X=Cl; 96%
7: R=4-Me, X=Br; 95%[b,j]

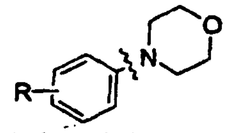

8: R=4-MeO, X=Cl; 91%
9: R=4-CN, X=Cl; 96%[b,d,k]
10: R=2,5-$Me_2$, X=Br; 97%[b,c,l]
11: R=4-$CO_2$Me, X=Br; 81%[e]
12: R=4-C(O)Me, X=Br; 82%[f,g]

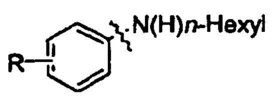

13: R=2,5-$Me_2$, X=Cl; 99%
14: R=4-$CO_2$Me, X=Cl; 83%[h,i]
15: R=2,6-$Me_2$, X=Br; 88%[b,d,k]

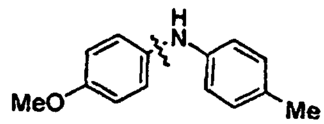

16: X=Cl, 93%[h]

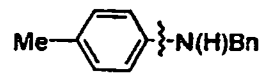

17: X=Cl, 89%[j,m]

☐ = Reaction run at rt.

(a) Reaction Conditions: 1.0 equiv. aryl halide, 1.2 equiv. amine, 1.4 equiv. NaO*t*Bu, 0.5 mol% $Pd_2(dba)_3$, 1.5 mol% ligand (1.5L/Pd), toluene (2 mL/mmol halide), 80 °C. Reactions were complete in 11-27 h; reaction times have not been minimized. (b) Reaction run at room temperature in DME solvent. (c) Reaction run with 1.5 mol% $Pd_2(dba)_3$. (d) Reaction run with 2.5 mol% $Pd_2(dba)_3$. (e) Reaction run using $K_3PO_4$, DME solvent. (f) Reaction run using $Pd(OAc)_2$, $K_3PO_4$, DME solvent. (g) One of two runs only proceeded to 98% conversion. (h) Reaction run at 100 °C. (i) Reaction run with $Pd(OAc)_2$, ligand 1, $Cs_2CO_3$ as catalyst, ligand, and base. (j) Using1 as ligand. (k) [ArBr]=1M. (l) [ArBr]=2M. (m) 1.5 equiv. benzylamine used.

In light of the high reactivity of this new catalyst system in amination reactions, we proceeded to examine its utility in several different Pd-catalyzed C-C bond forming reactions. Pd-catalyzed Suzuki coupling reactions[11] which use aryl chlorides as substrates generally require fairly high reaction temperatures (>90 °C), and are usually inefficient if the aryl halide does not contain electron-withdrawing substituents.[7] While nickel catalysts are more efficient at promoting Suzuki coupling reactions of electronically-neutral or electron-rich aryl chlorides, sterically hindered substrates are often problematic due to the small size of nickel relative to palladium.[12] Furthermore, examples of Suzuki coupling reactions which proceed at room temperature are rare,[13] and often require stoichiometric amounts of highly toxic thallium hydroxide. [13b,c,d] To the best of our knowledge, no examples of room temperature Suzuki couplings of an aryl chloride have been reported.

We have found that Suzuki coupling reactions of both aryl bromides and aryl chlorides proceed in high yield at room temperature using the **2**/Pd(0) catalyst system and CsF[14] in dioxane solvent (Table 2, entries 2,5,7-10).[15,16] These conditions allow for the coupling of both electron-rich and electron-deficient aryl chlorides, and tolerate the presence of base-sensitive functional groups. An aryl-alkyl coupling reaction of an aryl chloride using an alkylboron reagent generated *in situ* from 1-hexene and 9-BBN[17] was achieved at 50 °C; the higher temperature presumably being necessary due to the increased size of the boron reagent and the slower rate of transmetallation of alkyl groups relative to aryl groups.[17] Suzuki coupling reactions of electron-rich aryl chlorides could also be carried out using inexpensive $K_3PO_4$ with only 0.5 mol% palladium catalyst, although temperatures of 100 °C were required.

We also found the **2**/Pd(0) catalyst system was effective for the Pd-catalyzed $\alpha$-arylation of ketones.[3] Coupling of 5-bromo-*m*-xylene with 2-methyl-3-pentanone was performed at room temperature using NaHMDS as base (Table 2, entry 12). Interestingly, while the BINAP catalyst system was selective at promoting the monoarylation of methyl ketones, **2**/Pd was selective for the diarylation of methyl ketones (Table 2, entry 11). This may be due to the decreased steric bulk of the dimethylamine portion of **2** relative to the diphenylphosphine group of BINAP.

Other Pd-catalyzed cross couplings of aryl chlorides were surveyed using this catalyst. Stille couplings,[18] Sonogashira couplings,[19] and cross-couplings of aryl halides with organozinc reagents gave no detectable products.[20] The Heck arylation[21] of styrene gave some conversion to product at 110 °C.

**Table 2: Suzuki Coupling[a] and Ketone Arylation**

| Entry | Halide | Coupling Partner | Temp | mol% Pd | Yield | Product |
|---|---|---|---|---|---|---|
| 1 | 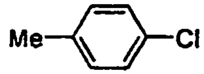 | $PhB(OH)_2$ | 100 | 0.5 | 96[b] | 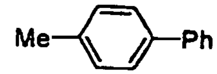 |
| 2 | | | rt | 2.0 | 94 | |
| 3 | | *o*-$MeOPhB(OH)_2$ | 100 | 1.0 | 94[d] | 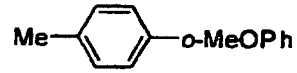 |
| 4 | 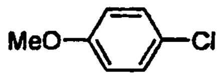 | $PhB(OH)_2$ | 100 | 0.5 | 93[b] | 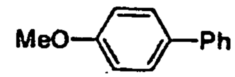 |
| 5 | | | rt | 2.0 | 92 | |
| 6 | | 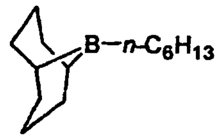 | 50 | 2.0 | 88[c] | 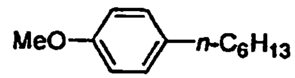 |
| 7 | 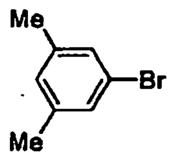 | $PhB(OH)_2$ | rt | 1.0 | 92 | 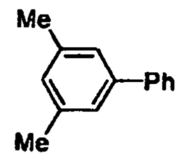 |
| 8 | 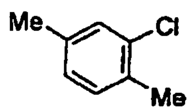 | *m*-$TolB(OH)_2$ | rt | 2.0 | 94 | 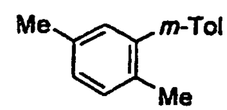 |
| 9 | 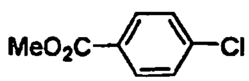 | $PhB(OH)_2$ | rt | 2.0 | 90 | 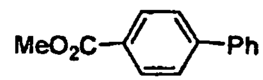 |
| 10 | 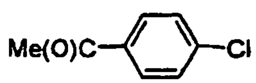 | *m*-$TolB(OH)_2$ | rt | 2.0 | 92 | 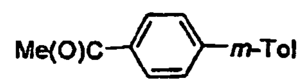 |
| 11 | 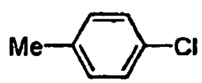 | 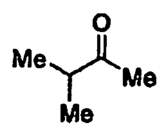 | 80 | 3.0 | 79[d] | 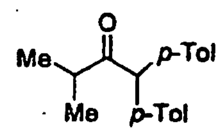 |

(continued)

| Entry | Halide | Coupling Partner | Temp | mol% Pd | Yield | Product |
|---|---|---|---|---|---|---|
| 12 | 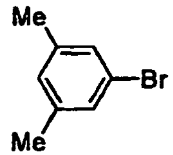 | 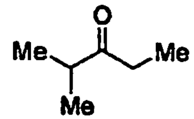 | rt | 3.0 | 82[e] | 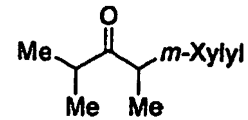 |

(a) Reaction Conditions: 1.0 equiv. aryl halide, 1.5 equiv. boron reagent, 3.0 equiv. CsF, 0.5-2.0 mol% $Pd(OAc)_2$, 0.75-3.0 mol% **2** (1.5UPd), dioxane (3 mL/mmol halide). Reactions were complete in 19-30h; reaction times have not been minimized. (b) 2.0 equiv. $K_3PO_4$ used in place of CsF. (c) One of two runs only proceeded to 98% conversion. (d) $Pd_2(dba)_3$, NaO*t*Bu used as catalyst, base. (e) $Pd_2(dba)_3$. NaHMDS used as catalyst, base

While the precise mechanistic details of the reactions promoted by the **2**/Pd(0) catalyst system remain unknown, we believe that the overall catalytic cycle for the amination reaction is similar to that postulated for the BINAP/Pd catalyzed amination of aryl bromides.[1c] However, in reactions catalyzed by **2**/Pd there may be different pathways available for the amine coordination/deprotonation step. Our current view is a pathway which involves binding of the amine to four-coordinate complex **I**, followed by deprotonation of the resulting five-coordinate complex **II** to give **III** (Figure 1, path A). Alternatively, coordination of the amine substrate may occur after initial dissociation of the dimethylamino moiety of the ligand, followed by nucleophilic attack of the amine substrate on three-coordinate[22b] complex **IV** to give **V**. Deprotonation of **V** is followed by rapid recomplexation of the ligand amine group to give **III** (Figure 1, path B).[22] If path B is operative, the recomplexation of the amine is presumably fast relative to β-hydride elimination since little or no reduced side product is observed. This notion is supported by the fact that $Cy_2PPh$ was not an effective ligand for any of these Pd-catalyzed processes;[15,16] amination reactions conducted with electron-rich monodentate phosphines as ligands such as $Cy_3P$ or $Cy_2PPh$ demonstrated that reduction via β-hydride elimination can be a significant problem without a chelating group on the ligand. The relatively small size of the amine group in **2** allows for the efficient coupling of both cyclic and acyclic secondary amines.[1d] That **2**/Pd(0) can be employed in an amination procedure at the 0.05 mol% level (Table 1, entry **1**) suggests that the dimethylamino group also contributes to the stability of the catalyst.

**Figure 1**

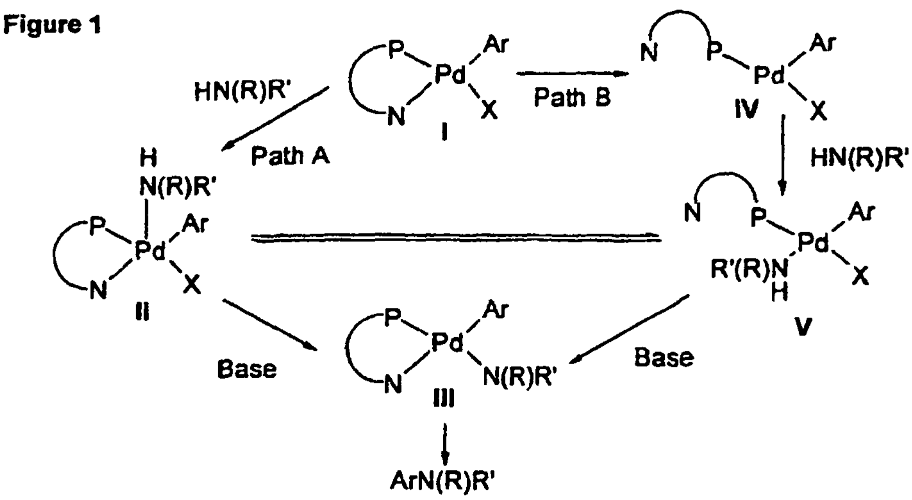


The failure of the **2**/Pd(0) catalyst system to promote the Heck, Stille, Sonogashira, and zinc cross-coupling reactions suggests the C-C bond forming reactions discussed in this paper proceed through four-coordinate intermediates with both the amine and phosphine moieties bound to the metal during the key steps in the catalytic cycle. If the ligand is bound in a bidentate fashion, transmetallation from Sn, Cu or Zn, or olefin coordination would be slow.[21,23] This argument is supported by the fact that Suzuki couplings and ketone arylation reactions are generally efficient with chelating phosphine ligands, while Stille reactions are not. Although in some cases Heck reactions are efficient with chelating ligands, these are usually with cationic complexes or for intramolecular reactions.[21]

We hope that modification of the design of this ligand or further optimization of reaction conditions may lead to efficient Heck olefinations of electron-rich aryl chlorides.[24] Further studies towards development of highly active catalysts for these and other processes are currently underway.

**References and Notes for Example 1**

(1) (a) Guram, A. S.; Rennels, R. A.; Buchwald, S. L. *Angew. Chem. Int. Ed. Engl.* **1995**, *34*, 1348-1349; (b) Wolfe, J. P.; Rennels, R. A.; Buchwald, S. L. *Tetrahedron* **1996**, *52*, 7525-7546. (c) Wolfe, J. P.; Wagaw, S.; Buchwald, S. L. *J Am. Chem. Soc.* **1996**, *118*, 7215-7216; (d) Marcoux, J.-F.; Wagaw, S.; Buchwald, S. L. *J. Org. Chem.* **1997**, *62,* 1568-1569; (e) Wolfe, J. P.; Buchwald, S. L. *J. Org. Chem.* **1997**, *62*, 6066-6068. (f) Wolfe, J. P.; Wagaw, S.; Marcoux, J. -F.; Buchwald, S. L. *Acc. Chem. Res.* Submitted for publication; (g). Louie, J.; Hartwig, J. *Tetrahedron Lett.* **1995**, *36*, 3609-3612; (h) Driver, M. S.; Hartwig, J. F. *J. Am. Chem. Soc.* **1996**, *118*, 7217-7218; (i) Barañano, D.; Mann, G.; Hartwig, J. F. *Cur. Org. Chem.* **1997**, *1*, 287-305. (j) Hartwig, J. F. *Synlett* **1997**, 329-340.

(2) (a) Palucki, M.; Wolfe, J. P.; Buchwald, S. L. *J. Am. Chem. Soc.* **1996,** *118,* 10333-10334; (b) Palucki, M.; Wolfe,

J. P.; Buchwald, S. L. *J. Am. Chem, Soc.* **1997,** *119,* 3395-3396; (d) - Mann, G.; Hartwig, J. F. *J. Am. Chem. Soc.* **1996**, *118,* 13109-13110; (e) Mann, G.; Hartwig, J. F. *J. Org. Chem.* **1997**, *62*, 5413-5418.

(3) (a) Palucki, M.; Buchwald. S. L. *J. Am. Chem. Soc.* **1997**, *119*, 11108-11109; (b) Ahman, J.; Wolfe, J. P.; Troutman, M. V.; Palucki, M.; Buchwald, S. L. *J. Am. Chem. Soc.* **1998**, *120,* 1918; (c) Hamann, B. C.; Hartwig, J. F. *J. Am. Chem. Soc.* **1997**, *119,* 12382-12383; (d) Satoh, T.; Kawamura, Y.; Miura, M.; Nomura, M. *Angew. Chem. Int. Ed. Engl.* **1997,** *46*, 1740-1742.

(4) Aryl chlorides are attractive starting materials from the perspective of cost and availability, but are less reactive than aryl bromides and iodides. See: Grushin, V. V.; Alper, H. *Chem. Rev.* **1994,** *94*, 1047-1062.

(5) Existing protocols for the amination of aryl chlorides include our work in nickel catalysis as well as two palladium-based methods. Our nickel-based work, while quite effective for a wide variety of aryl chloride substrates, is not effective for amination of other aryl halides and does not tolerate base-sensitive functional groups. The palladium methods are quite limited in scope and often result in mixtures of products. See: (a) Wolfe, J. P.; Buchwald, S. L; *J. Am. Chem. Soc.* **1997,** *119,* 6054-6058; (b) Beller, M.; Riermeier, T. H.; Reisinger, C.-P.; Herrmann, W. A. *Tetrahedon Lett.* **1997,** *38*, 2073-2074; (c) Riermeier, T. H.; Zapf, A.; Beller, M. *Top. Catal.* **1997,** *4,* 301-309; (d) Reddy, N. P.; Tanaka, M. *Tetrahedon Lett.* **1997**, *38,* 4807-4810. (e) Nishiyama, M.; Yamamoto, T.; Koie, Y. *Tetrahedron Lett.* **1998,** *39*, 617-620; (f) Yamamoto, T.; Nishiyama, M.; Koie, Y. *Tetrahedron Lett.* **1998**, *39,* 2367-2370.

(6) Hartwig and Hamann have recently reported similar NMR experiments. They have also shown that electron-rich bidentate bis-phosphines can be used for the Pd-catalyzed amination of aryl chlorides: Hartwig, J. F.; Hamann, B**.** C. Submitted for Publication.

(7) (a) Shen, W. *Tetrahedron Lett.* **1997,** *38*, 5575-5578. (b) Beller, M.; Fischer, H.; Herrmann, W. A.; Öfele, K.; Brossmer, C. *Angew. Chem. Int. Ed. Engl.* **1995, 34,** 1848-1849.

(8) Zhang, X.; Mashima, K.; Koyano, K.; Sayo, N.; Kumobayashi, H.; Akutagawa, S.; Takaya, H. *J. Chem. Soc. Perkin Trans. I* **1994,** 2309-2322.

(9) Ligand **2** was prepared in 3 steps from *N,N*-dimethyl-2-bromoaniline. The ligand is obtained as a crystalline solid and is stored and handled in the air without any special precautions. Under these conditions, the ligand is stable for at least a month without any detectable oxidation. See supporting information for complete experimental details.

(10) Control experiments conducted in the absence of palladium afforded no coupled products after 24 h at room temperature.

(11) Suzuki, A. in *Metal-Catalyzed Cross-Coupling Reactions* Diederich, F.; Stang, P. J. Eds., Wiley-VCH, Weinheim, Germany, 1998, Ch. 2.

(c) Bumagin, N. A.; Bykov. V. V. *Tetrahedron* **1997**, *53*, 14437-14450. (d) Mitchell, M. B.; Wallbank, P. J. *Tetrahedron Lett.* **1991,** *32*, 2273-2276. (e) Firooznia, F.; Gude, C.; Chan, K.; Satoh, Y. *Tetrahedron Lett.* **1998,** *39*, 3985-3988. (f) Cornils, B. *Orgn. Proc. Res. Dev.* **1998,** *2*, 121-127.

(12) (a) Indolese, A. F. *Tetrahedron Lett.* **1997**, *38,* 3513-3516. (b) Saito, S.; Oh-tani, S.; Miyaura, N. *J. Org. Chem*. **1997,** *62,* 8024-8030.

(13) (a) Campi, E. M.; Jackson, W. R.; Marcuccio, S. M.; Naeslund, C. G. M *J. Chem. Soc., Chem. Commun* **1994,** 2395. (b) Anderson, J. C.; Namli, H.; Roberts, C. A. *Tetrahedron* **1997**, *53,* 15123-15134. (c) Anderson, J. C.; Namli, H. *Synlett* **1995**, 765-766. (d) Uenishi, J.-i.; Beau, J. -M.; Armstrong, R. W.; Kishi, Y. *J. Am. Chem. Soc.* 1987, *109,* 4756-4758.

(14) Wright, S. W.; Hageman, D. L.; McClure, L. D. *J. Org. Chem.* **1994***, 59,* 6095-6097.

(15) See supporting information for complete experimental details.

(16) Control experiments conducted using dicyclohexylphenylphosphine in place of **2** gave low conversions and low yields of products.[15]

(17) Miyaura, N.; Ishiyama, T.; Sasaki, H.; Ishikawa, M.; Satoh, M.; Suzuki, A. *J. Am. Chem. Soc.* **1989,** *111,* 314-321.

(18) Stille, J. K. *Angew Chem Int. Ed Engl.* **1986,** *25,* 508.

(19) Sonogashira, K. in ref 11, Ch 5.

(20) Knochel, P. in ref 11, Ch 9.

(21) (a) de Meijere, A.; Meyer, F. E. *Angew. Chem. Int. Ed. Engl.* **1994,** *33,* 2379-2411; (b) Bräse, S.; de Meijere, A. in ref. 11, Ch. 3.

(22) (a) It is also possible that reductive elimination occurs from a 3-coordinate intermediate[1j] formed by deprotonation of **V**. (b) There is precedent for the dissociation of one phosphine of a chelating bis-phosphine.[1j] (c) In reactions which employ NaO*t*-Bu as base it is possible that complexes shown in Figure 1 may contain X=O*t*Bu.[2d] In reactions which employ $Cs_2CO_3$ or $K_3PO_4$ as base it is unlikely that carbonate or phosphate complexes form due to the low solubility and low nucleophilicity of $Cs_2CO_3$ and $K_3PO_4$ relative to NaO*t*-Bu.

(23) Farina, V. *Pure Appl. Chem.* **1996***, 68,* 73-78.

(24) Heck reactions of aryl chlorides generally require high reaction temperatures, and are often inefficient for electron-rich aryl chlorides. See ref 5a and references therein. (a) Herrmann, W. A.; Brossmer, C.; Reisinger, C: -P.; Riermeier, T. H.; Öfele, K.; Beller, M. *Chem. Eur. J.* **1997**, *3*, 1357-1364. (b) Reetz, M. T.; Lohmer, G.; Schwickardi, R. *Angew. Chem. Int. Ed. Engl.* **1998**, *37*, 481-483. (c) Ohff, M.; Ohff, A.; van der Boom, M. E.; Milstein, D. *J. Am. Chem. Soc.* **1997,** *119,* 11687-11688.

**Supporting Information for Example 1**

General. All reactions were carried out under an argon atmosphere in oven-dried glassware. Elemental analyses were performed by E & R Microanalytical Laboratory Inc., Parsippany, N.J. Toluene was distilled under nitrogen from molten sodium. THF was distilled under argon from sodium benzophenone ketyl. Unless stated otherwise, commercially obtained materials were used without purification. Aryl halides were purchased from Aldrich Chemical company except for 4-chloroacetophenone which was purchased from Fluka Chemical company. N,N-dimethyl-2-bromoaniline[1] was prepared by alkylation of 2-bromoaniline with iodomethane in DMF in the presence of sodium carbonate. Tribasic potassium phosphate was purchased from Fluka Chemical company. Cesium fluoride was purchased from Strem Chemical company and was ground with a mortar and pestle before use. Cesium carbonate was obtained from Chemetal and was ground with a mortar and pestle before use. Phenylboronic acid, chlorodicyclohexylphosphine, palladium acetate, tris(dibenzylideneacetone)dipalladium(0), (±)-2,2'-dibromo-1,1'-binaphthyl, and *n*-butyllithium were purchased from Strem Chemical company. 2-Methoxyphenylboronic acid[2] and 3-methylphenylboronic acid[2] were prepared by lithiation of the corresponding halide and reaction with $B(OMe)_3$ according to a general literature procedure.[2] These boronic acids were obtained in ~85-95% purity following crystallization from pentane/ether and were used without further purification. Trimethyl borate, triisopropyl borate, 9-BBN (0.5 M THF solution), NaHMDS (95%), 2-methyl-3-pentanone, 3-methyl-2-butanone, anhydrous dioxane, anhydrous DME, dicyclohexylphenylphosphine, and 1-hexene were purchased from Aldrich Chemical company. (±)-2,2'-Bis(dicyclohexylphosphino)-1,1'-binaphthyl 1[3] was prepared by metallation of the corresponding dibromobinaphthyl with *t*-butyllithium and quenching with chlorodicyclohexylphosphine using a procedure analogous to the synthesis of (±)-BINAP.[4] It was characterized by elemental analysis and by comparison of its $^1H$ and $^{31}P$ NMR spectra with literature data.[3] Tetrakis(triphenylphosphine)palladium was prepared according to a literature procedure.[5] Sodium *t*-butoxide was purchased from Aldrich Chemical Company; the bulk of this material was stored under nitrogen in a Vacuum Atmospheres glovebox. Small portions (1-2 g) were removed from the glovebox in glass vials, stored in the air in desiccators filled with anhydrous calcium sulfate, and weighed in the air. IR spectra reported in this paper were obtained by placing neat samples directly on the DiComp probe of an ASI REACTIR *in situ* IR instrument. Yields in Tables 1 and 2 refer to isolated yields (average of two runs) of compounds estimated to be 95% pure as determined by $^1H$ NMR, and GC analysis or combustion analysis. Entries 1,[6] 2,[7] 3,[6] 4,[6] 5,[8] 6,[9] 7,[6] 8,[6] 9,[11] 13,[6] and 14,[10] from Table I have been previously reported by this group and were characterized by comparison of their $^1H$ NMR spectra to those of samples prepared prior to this work; their purity was confirmed by GC analysis. The procedures described in this section are representative, thus the yields may differ from those given in Tables 1 and 2.

2-(*N,N*-Dimethylamino)-2'-(dicyclohexylpbosphino)biphenyl (2).

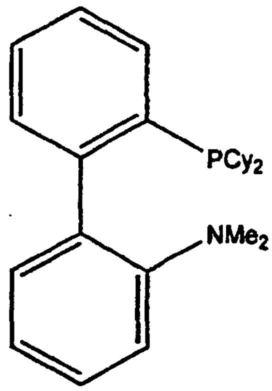

2

*N,N*-dimethylamino-2-bromoaniline[1] (4.0 g, 20.0 mmol) was loaded into an oven-dried flask which had been cooled to room temperature under an argon purge. The flask was purged with argon and THF (20 mL) was added. The solution was cooled to -78 ˚C and n-butyllithium (13.1 mL, 21.0 mmol, 1.6 M in hexanes) was added dropwise with stirring. After the addition was complete the reaction mixture was stirred at -78 ˚C for 75 min during which time a white precipitate formed. An additional 70 mL of THF was added, and the aryllithium suspension was then transferred via cannula to a separate flask containing a solution of triisopropyl borate (9.2 mL, 40.0 mmol) in THF (20 mL) which had been cooled to -78 ˚C. The reaction mixture was stirred at -78 ˚C for 1 h, then warmed to room temperature and allowed to stir overnight (25 h). The reaction was quenched with 1 M aqueous HCl (250 mL), and stirred at room temperature for 15 min. The pH of the mixture was adjusted to pH 7 with 6 M aqueous NaOH, and the mixture was transferred to a separatory funnel. The mixture was extracted with ether (3 x 150 mL), and the combined organic extracts were dried over anhydrous magnesium sulfate, and concentrated *in vacuo* to give a brown oil which contained substantial amounts of N,IV-dimethylaniline. This oil was then taken up in ether (100 mL), and extracted with 1 M aqueous NaOH (3 x 100 mL). The organic layer was discarded and the aqueous extracts were adjusted to pH 7 with 6 M aqueous HCl. The aqueous phase was then extracted with ether (3 x 100 mL), and the combined organic layers were dried over anhydrous magnesium sulfate, filtered, and concentrated *in vacuo* to give 1.85 g of 2-(*N,N*-dimethylamino)phenylboronic acid[12] as a viscous tan oil which was found to be ~50-60% pure by $^1$H NMR. This material was used without further purification.

The crude boronic acid was taken up in ethanol (5 mL) and was added to a flask containing a solution of tetrakis (triphenylphosphine)palladium[5] (700 mg, 0.61 mmol, 5 mol%) and 2-bromoiodobenzene (4.1 g, 14.5 mmol) in DME (100 mL) under argon. A solution of $Na_2CO_3$ (6.42 g, 60.6 mmol) in degassed water (30 mL) was added to the reaction vessel, and the mixture was heated to reflux for 48 h. The reaction mixture was then cooled to room temperature, diluted with ether (200 mL), and poured into a separatory funnel. The layers were separated, and the aqueous layer was extracted with ether (200 mL). The layers were separated and the aqueous layer was discarded. The combined organic layers were then washed with 1 M aqueous NaOH (50 mL), and the aqueous wash was discarded. The combined organic fractions were then extracted with 1 M aqueous HCl (4 x 150 mL). The organic fraction was discarded, and the combined aqueous acid extracts were basified to pH 14 with 6 M aqueous NaOH. The aqueous phase was extracted with ether (3 x 150 mL), and the combined organic layers were dried over anhydrous magnesium sulfate, filtered, and concentrated *in vacuo* to give 2.1 g of a white solid which was judged to be ~90-95% pure by $^1$H NMR. This material was used without further purification.

An oven-dried round-bottomed flask was cooled to room temperature under an argon purge and charged with the crude 1-(N,N-dimethylamino)-1'-bromobiphenyl. The flask was purged with argon, and THF (120 mL) was added. The solution was cooled to -78 ˚C with stirring, and *n*-butyllithium (5.2 mL, 8.37 mmol, 1.6 M in hexanes) was added dropwise. The solution was stirred at -78˚C for 35 min, then a solution of chlorodicyclohexylphosphine (2.21 g, 9.51 mmol) in THF (30 mL) was added dropwise to the reaction vessel. The reaction mixture was stirred at -78 ˚C and allowed to warm slowly to room temperature overnight. The reaction was then quenched with saturated aqueous $NH_4Cl$ (30 mL), diluted with ether (200 mL), and poured into a separatory funnel. The layers were separated and the aqueous phase was extracted with ether (50 mL). The combined organic layers were dried over anhydrous sodium sulfate, filtered, and

concentrated to give a white solid. The crude material was recrystallized from degassed, hot ethanol under an argon atmosphere to afford 2.25 g (29% overall yield for 3 steps) of a white solid: mp 110 ˚C; $^1$H NMR (300 MHz, $CDCl_3$) δ 7.54 (d, 1H, *J*=6.8 Hz), 7.26-7.40 (m, 4H), 7.02-7.05 (m, 1H), 6.93-6.98 (m, 3H), 2.44 (s, 6H), 1.98-2.05 (m, 1H), 1.40-1.82 (m, 11 H), 0.75-1.38 (m, 10 H); $^{13}$C NMR (125 MHz, $CDCl_3$) δ 151.5, 149.8, 149.5, 135.8, 135.5, 135.3, 132.7, 132.4, 130.54, 130.49, 128.5, 128.1, 125.8, 120.6, 117.3, 43.2, 36.8, 36.7, 33.5, 33.4, 30.9, 30.8, 30.6, 30.4, 29.8, 29.7, 28.5, 27.6, 27.54, 27.46, 27.3, 27.2, 26.7, 26.4 (observed complexity due to P-C splitting; definitive assignments have not yet been made); $^{31}$P NMR (121.5 MHz, $CDCl_3$) δ -9.2; IR (neat, cm$^{-1}$) 2922, 1444, 745. Anal Calcd for $C_{26}H_{36}NP$: C, 79.35; H, 9.22. Found: C, 79.43; H, 9.48.

**General procedure for the palladium-catalyzed amination of aryl chlorides:** An oven-dried Schlenk tube or test tube fitted with a rubber septum was purged with argon and charged with tris(dibenzylidineacetone)dipalladium (0.005 mmol, 1 mol% Pd), ligand **2** (0.015 mmol, 1.5 mol%), and NaO*t*-Bu (1.4 mmol). The tube was purged with argon, and toluene (2.0 mL), the aryl chloride (1.0 mmol) and the amine (1.2 mmol) were added. The mixture was stirred in an 80 ˚C oil bath until the starting aryl chloride had been completely consumed as judged by GC analysis. The reaction mixture was then cooled to room temperature, diluted with ether (20 mL), filtered through celite and concentrated *in vacuo.* The crude material was then purified by flash chromatography on silica gel.

***N*-(4-methylphenyl)-*p*-anisidine.**[13]

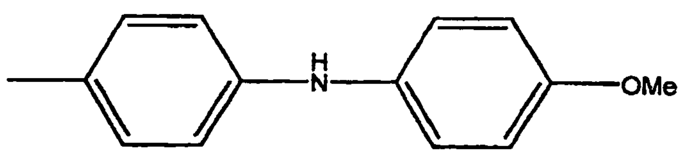

The general procedure except using a reaction temperature of 100 ˚C gave 198 mg (93%) of a tan solid: mp 80-81 ˚C (lit.[13] mp 84-85 ˚C). $^1$H NMR (300 MHz, $CDCl_3$) δ 6.98-7.05 (m, 4H), 6.80-6.86 (m, 4H), 5.37 (s, br 1H), 3;76 (s, 3H), 2.26 (s, 3H); $^{13}$C NMR (125 MHz, $CDCl_3$) δ 154.8, 142.4, 136.7, 129.7, 129.3, 121.1, 116.6, 114.7, 55.6, 20.5; IR (neat, cm-$^1$) 3416, 2910, 1513, 1304, 815.

***N*-benzyl-*p*-toluidine.**[14]

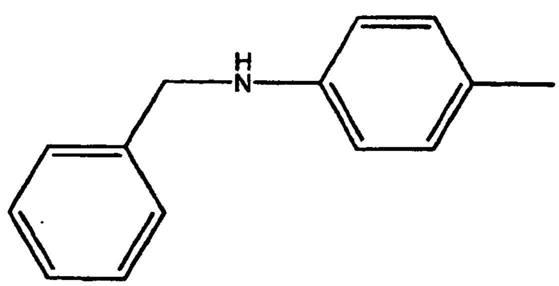

The general procedure except using **1** as the ligand, and 1.5 equiv of benzyl amine, gave 177 mg (90%) of a pale yellow oil: $^1$H NMR (250 MHz, $CDCl_3$) δ 7.25-7.39 (m, 5H), 6.98 (d, 2H, *J*=8.1 Hz), 6.56 (d, 2.H, *J*=8.5 Hz), 4.31 (s, 2H), 3.90 (br s, 1H), 2.23 (s, 3H); $^{13}$C NMR (125 MHz, $CDCl_3$) δ 145.9, 139.7, 129.7, 128.5, 127.4, 127.1, 126.7, 113.0, 48.6, 20.3; IR (neat, cm$^{-1}$) 3416, 3026, 1521, 807.

***N*-(4-Cyanophenyl)morpholine.**[11]

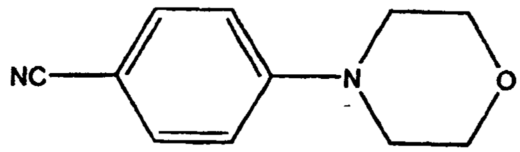

An oven-dried resealable Schlenk tube was purged with argon and charged with $Pd_2(dba)_3$ (11.5 mg, 0.025 mmol, 5 mol% Pd), **2** (14.8 mg, 0.075 mmol, 7.5 mol%), NaO*t*-Bu (68 mg, 0.71 mmol) and 4-chlorobenzonitrile (69 mg, 0.50 mmol). The tube was purged with argon then DME (0.5 mL) and morpholine (53 $\mu$L, 0.61 mmol) were added through a rubber septum. The septum was removed, the tube was sealed with a teflon screw cap and the mixture was stirred at room temperature for 26 h, then diluted with EtOAc, filtered through celite and concentrated *in vacuo.* The crude material was purified by flash chromatography on silica gel to afford 91 mg (96%) of a tan solid.

**Amination using 0.05 mol% Pd.** An oven-dried resealable Schlenk tube was purged with argon and charged with $Pd_2(dba)_3$ (2.3 mg, 0.0025 mmol, 0.05 mol% Pd), ligand **2** (2.9 mg, 0.0075 mmol, 0.075 mol%), and NaO*t*-Bu (1.34 g, 13.9 mmol). Toluene (10 mL), di-*n*-butylamine (2.00 mL, 11.9 mmol), and 4-chlorotoluene (1.18 mL, 10.0 mmol) were added and the mixture was degassed using three freeze-pump-thaw cycles. The reaction vessel was placed under argon, sealed with a teflon screw cap, and stirred in a 100°C oil bath for 20 h after which time GC analysis showed the aryl halide had been completely consumed. The reaction mixture was cooled to room temperature, diluted with ether (100 mL) and extracted with 1 M HCl (3 x 100 mL). The combined aqueous acid phase was basified with 3N NaOH, then extracted with ether (3 x 150 mL). The ethereal extracts were dried over anhydrous sodium sulfate, filtered and concentrated to afford 2.01 g (95%) of di-*n*-butyltoluidine[6] as a pale yellow oil.

**General procedure for the room-temperature palladium-catalyzed amination of aryl bromides**: An oven-dried resealable Schlenk tube was purged with argon and charged with $Pd_2(dba)_3$ (0.005-0.025 mmol, 1-5 mol% Pd), ligand **2** (0.015-0.075 mmol, 1.5-7.5 mol%), and NaO*t*-Bu (1.4 mmol) [see Table 1, for amount of Pd and ligand used]. The tube was purged with argon, fitted with a rubber septum and then DME (0.5 mL-1.0 mL), the aryl bromide (1.0 mmol) and the amine (1.2 mmol) were added via syringe. The septum was removed, the tube was sealed with a teflon screw cap and the mixture was stirred at room temperature for 24 h. The reaction mixture was then diluted with ether (20 mL), filtered through celite and concentrated *in vacuo.* The crude material was purified by flash chromatography on silica gel.

**2,6-Dimethyl-*N*-(*n*-hexyl)aniline.**

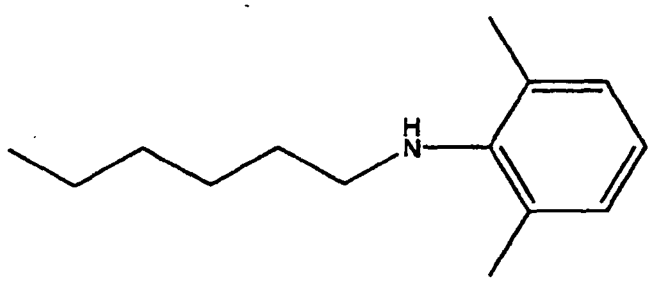

The general procedure was conducted with with 0.5 mmol of aryl bromide and afforded 90 mg (87%) of a colorless oil: $^1H$ NMR (300 MHz, $CDCl_3$) $\delta$ 6.98 (d, 2H, *J*=7.5 Hz), 6.79 (t, 1H, *J*=7.5 Hz), 2.97 (t, 2H, *J*=7.2 Hz), 2.94-2.99 (br, 1H), 2.28 (s, 6H), 1.52-1.60 (m, 2H), 1.28-1.41 (m, 6H), 0.89 (t, 3H, *J*=6.8 Hz); $^{13}C$ NMR (125 MHz, $CDCl_3$) $\delta$ 146.5, 129.1, 128.8, 121.5, 48.7, 31.7, 31.2, 26.9, 22.6, 18.5, 14.0; IR (neat, $cm^{-1}$) 3384, 2926, 1472, 1256, 1219, 762. Anal Calcd for $C_{14}H_{23}N$: C, 81.89; H, 11.29. Found: C, ; H,.

***N*-(2,5-Dimethylphenyl)morpholine.**

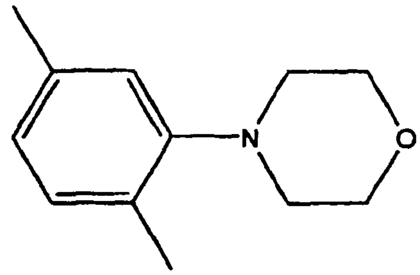

The general procedure was conducted at 2.0 M concentration and afforded 185 mg (95%) of a colorless oil: $^1H$ NMR (300 MHz, $CDCl_3$) δ 7.06 (d, 1H, *J*=7.7 Hz), 6.80-6.82 (m, 2H), 3.84 (t, 4H, *J*=4.6 Hz), 2.89 (t, 4H, *J*=4.6 Hz), 2.31 (s, 3H), 2.26 (s, 3H); $^{13}C$ NMR (125 MHz, $CDCl_3$) δ 151.1, 136.2, 131.0, 129.3, 124.0, 119.7, 67.5, 52.3, 21.1, 17.4; IR (neat, $cm^{-1}$) 2955, 2851, 1505, 1242, 1117, 807. Anal Calcd for $C_{12}H_{17}NO$: C, 75.35; H, 8.96. Found: C,; H,.

***N*-(4-carbomethoxyphenyl)morpholine.**[15]

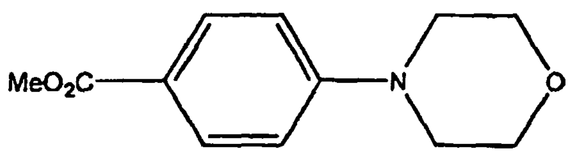

The general procedure was conducted with 0.5 mmol of aryl bromide except using $K_3PO_4$ in place of NaO*t*-Bu at 80 ˚C, EtOAc as the workup solvent and gave 89 mg (80%) of a colorless solid: mp 152-154 ˚C (lit.[15] mp 157-160 ˚C). $^1H$ NMR (300 MHz, $CDCl_3$) δ 7.94 (d, 2H, *J*=8.6 Hz), 6.86 (d, 2H, *J*=8.8 Hz), 3.87 (s, 3H), 3.86 (t, 4H, *J*=4.8 Hz), 3.29 (t, 4H, *J*=4.8 Hz); $^{13}C$ NMR (125 MHz, $CDCl_3$) δ 167.0, 154.2, 131.2, 120.4, 113.5, 66.6, 51.6, 47.8; IR (neat, $cm^{-1}$) 2968, 1698, 1289, 1116, 768. Anal Calcd for $C_{12}H_{15}NO_3$: C, 65.14; H, 6.83. Found: C, ; H,.

***N*-(4-acetylphenyl)morpholine.**[16]

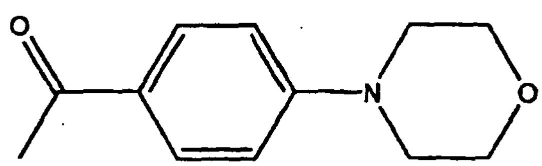

The general procedure except using $Pd(OAc)_2$, $K_3PO_4$ in place of $Pd_2(dba)_3$, NaO*t*Bu; at a reaction temperature of 80 ˚C, and 1/1 $Et_2O$/EtOAc as the workup solvent gave 169 mg (82%) of a pale yellow solid: m.p. 93-94 ˚C (lit.[14] mp 97-98 ˚C). $^1H$ NMR (300 MHz, $CDCl_3$) δ 7.89 (d, 2H, *J*=9.1 Hz), 6.87 (d, 2H, *J*=9.1 Hz), 3.86 (t, 4H, *J*=4.8 Hz), 3.31 (t, 4H, *J*=5.1 Hz), 2.54 (s, 3H); $^{13}C$ NMR (125 MHz, $CDCl_3$) δ 196.4, 154.1, 130.2, 128.1, 113.2, 66.5, 47.5, 26.0; IR (neat, $cm^{-1}$) 2972, 1660, 1243, 1119, 818. Anal Calcd for $C_{12}H_{15}NO_2$: C, 70.22; H, 7.37. Found: C, 70.31; H, 722.

**Aminations with dicyclohexylphenylphosphine as the supporting Ligand.** The coupling of 4-chlorotoluene and di-*n*-butylamine following the general procedure for catalytic amination of aryl chlorides using dicyclohexylphenylphosphine (not according to the invention) in place of **2** led to 96% conversion (17% GC yield) in 12 h. In the same amount of time, the reaction using ligand **2** (not according to the invention) was complete, affording a 97% isolated yield of the desired product. The coupling of 2-bromo-p-xylene and morpholine following the room temperature procedure above, replacing **2** with dicyclohexylphenylphosphine (1.5L/Pd), led to 2.5% consumption of the starting aryl bromide, with a trace amount of product detected (GC). When a ratio of 3L/Pd was used, no reaction was observed.

**General procedure for the room-temperature Suzuki coupling of aryl halides:** An oven-dried resealable Schlenk tube was purged with argon and charged with $Pd(OAc)_2$ (0.02 mmol, 2 mol%), ligand **2** (not according to the invention; 0.03 mmol, 3 mol%), the boronic acid (1.5 mmol), and cesium fluoride (3.0 mmol). The tube was purged with argon, and' dioxane (3 mL) and the aryl halide (1.0 mmol) were added through a rubber septum. The septum was removed, the tube was sealed with a teflon screw cap and the mixture was stirred at room temperature until the starting aryl halide had been completely consumed as judged by GC analysis. The reaction mixture was then diluted with ether (20 mL) and poured into a separately funnel. The mixture was washed with 1 M NaOH (20 mL), and the layers were separated. The aqueous layer was extracted with ether (20 mL), and the combined organic layers were dried over anhydrous magnesium sulfate, filtered, and concentrated *in vacuo.* The crude material was then purified by flash chromatography on silica gel.

**3,5-Dimethylbiphenyl.**[17]

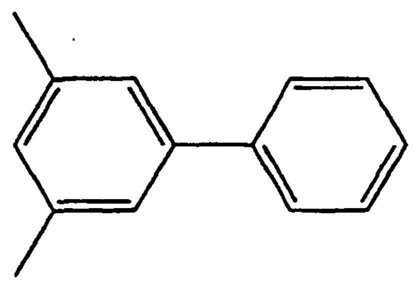

The general procedure using 1 mol% $Pd(OAc)_2$ and 1.5 mol% ligand **2** gave 171 mg (94%) of a colorless oil. $^1H$ NMR (300 MHz, $CDCl_3$) δ 7.57 (d, 2H, *J*=6.8 Hz), 7.42 (t, 2H, *J*=7.2 Hz), 7.31-7.34 (m, 1H), 7.21 (s, 2H), 7.00 (s, 1H). 2.38 (s, 6H); $^{13}C$ NMR (125 MHz, $CDCl_3$) δ 141.5, 141.3, 138.2, 128.9, 128.6, 127.2, 127.0, 125.1, 21.4; IR (neat, $cm^{-1}$) 3030, 1602, 849, 760. Anal Calcd for $C_{14}H_{14}$: C, 92.26; H, 7.74. Found: C, 91.98; H, 8.02.

**2,5,3'-Trimethylbiphenyl.**[18]

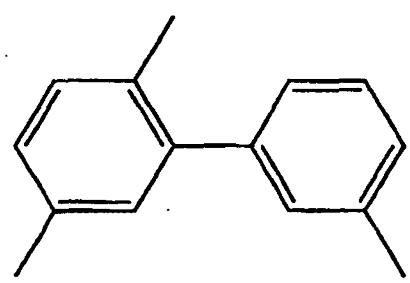

The general procedure gave 192 mg (98%) of a colorless oil which contained 4% 3,3'-dimethylbiphenyl as determined by $^1H$ NMR: $^1H$ NMR (300 MHz, $CDCl_3$) δ 7.25-728 (m, 1H), 7.04-7.16 (m, 6H), 2.39 (s, 3H), 2.34 (s, 3H), 2.23 (s, 3H); $^{13}C$ NMR (125 MHz, $CDCl_3$) δ 142.1, 141.9, 137.5, 135.0, 132.1, 130.5, 130.2, 129.9, 127.85, 127.80, 127.3, 126.2, 21.4, 20.9, 19.9; IR (neat, $cm^{-1}$) 2949, 1451, 811, 703. Anal Calcd for $C_{15}H_{15}$: C, 92.26; H, 7.74. Found: C, 92.34; H, 7.66.

**4-Acetyl-3'-methylbiphenyl.**[19]

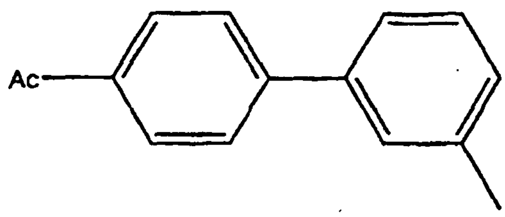

The general procedure gave 190 mg (90%) of a white solid: mp 84-86 °C (lit.[19] mp 92 °C). $^1H$ NMR (300 MHz, $CDCl_3$) δ 8.02 (d, 2H, *J*=8.5 Hz), 7.68 (d, 2H, *J*=8.5 Hz), 7.33-7.44 (m, 3H), 7.20-7.26 (m, 1H), 2.64 (s, 3H), 2.43 (s, 3H); $^{13}C$ NMR (125 MHz, $CDCl_3$) δ 197.6, 145.8, 139.7, 138.5, 135.7, 128.9, 128.8, 127.9, 127.1, 124.3, 26.5, 21.4; IR (neat, $cm^{-1}$) 3019, 1683, 1270, 787. Anal Calcd for $C_{15}H_{14}O$: C, 85.68; H, 6.71. Found: C, 85.79; H, 6.92

**Methyl 4-phenylbenzoate.**[20]

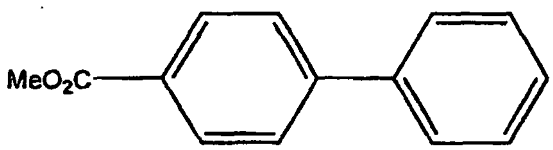

The general procedure (except using water for the aqueous workup in place of 1 M aqueous NaOH) gave 193 mg (91 %) of a white solid: mp 113 °C (lit.[20] mp 117-118 °C). $^1H$ NMR (300 MHz, $CDCl_3$) δ 8.11 (d, 2H, *J*=8.3 Hz), 7.61-7.68 (m, 4H), 7.39-7.49 (m, 3H), 3.94 (s, 3H); $^{13}C$ NMR (125 MHz, $CDCl_3$) δ 166.9, 145.5, 139.9, 130.0, 128.8, 128.1, 127.2, 126.9, 52.0; IR (neat, $cm^{-1}$) 2945, 1710, 1270, 1112, 749. Anal Calcd for $C_{14}H_{13}O_2$: C, 78.85; H, 6.14. Found: C, 79.04; H, 6.16.

**4-Hexylanisole.**[21]

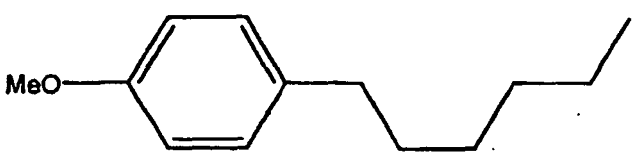

An oven-dried resealable Schlenk tube was capped with a rubber septum, cooled under an argon purge, charged with 1-hexene (0.19 mL, 1.5 mmol), and cooled to 0 °C. A solution of 9-BBN in THF (3 mL, 1.5 mmol, 0.5 M) was added, the flask was stirred at 0 °C for 15 min, then warmed to room temperature and stirred for 5 h. 4-Chloroanisole (0.12 mL, 1.0 mmol) was added, the septum was removed, and palladium acetate (4.4 mg, 0.02 mmol, 2 mol%), ligand **2** (11.9 mg, 0.03 mmol, 3 mol%), and cesium fluoride (456 mg, 3.0 mmol) were added under a stream of argon. The septum was replaced and the flask was purged with argon for 30 s. Dioxane (2 mL) was added, the septum was removed, the tube was sealed with a teflon screw cap, and the mixture was stirred at rt for 2 min. The reaction mixture was then heated to 50 °C with stirring for 22 h, at which time GC analysis showed the aryl chloride had been completely consumed. The mixture was cooled to room temperature, diluted with ether (20 mL), and poured into a separatory funnel. The mixture was washed with 1 M aqueous NaOH (20 mL), the layers were separated, and the aqueous phase was extracted with ether (20 mL). The combined organic layers were dried over anhydrous magnesium sulfate, filtered, and concentrated *in vacuo.* The crude material was purified by flash chromatography to afford 170 mg (89%) of a colorless oil: $^1H$ NMR (300 MHz, $CDCl_3$) δ 7.09 (d; 2H, *J*=8.8 Hz), 6.82 (d, 2H, *J*=8.6 Hz), 3.78 (s, 3H), 2.54 (t, 2H, *J*=7.5 Hz), 1.54-1.60 (m, 2H), 1.28-1.35 (m, 6H), 0.88 (t, 3H, *J*=6.8 Hz); $^{13}C$ NMR (125 MHz, $CDCl_3$) δ 157.6, 135.0, 129.2, 113.6, 552, 35.0, 31.73, 31.70, 28.9, 22.6, 14.1; IR (neat, $cm^{-1}$) 2926, 1513, 1243, 1038, 822. Anal Calcd for $C_{13}H_{20}O$: C, 81.20; H, 10.48. Found: C, 81.19; H, 10.62.

**General procedure for $K_3PO_4$ promoted Suzuki coupling of aryl chlorides**: An oven-dried resealable Schlenk tube was purged with argon and charged with $Pd(OAc)_2$ (0.01 mmol, 0.5 mol%), ligand **2** (not according to the invention; 0.015 mmol, 0.75 mol%), the boronic acid (3.0 mmol), and potassium phosphate (4.0 mmol). The tube was purged with argon, and dioxane (6 mL) and 4-chlorotoluene (2.0 mmol) were added through a rubber septum. The septum was removed, the tube was sealed with a teflon screw cap and the mixture was stirred at room temperature for 2 min, then heated to 100 °C with stirring until the starting aryl chloride had been completely consumed as judged by GC analysis. The reaction mixture was then cooled to room temperature, diluted with ether (20 mL) and poured into a separatory funnel. The mixture was washed with 1 M NaOH (20 mL), and the layers were separated. The aqueous layer was extracted with ether (20 mL), and the combined organic layers were dried over anhydrous magnesium sulfate, filtered, and concentrated *in vacuo*. The crude material was then purified by flash chromatography on silica gel. **4-Methoxybiphenyl.**[22]

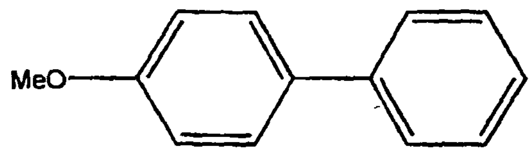

The general procedure gave 347 mg (94%) of a white solid: mp 83-84 °C (lit.[22] mp 87 °C); $^1H$ NMR (250 MHz, $CDCl_3$) δ 7.52-7.58 (m, 4H), 7.42 (t, 2H, *J*=7.8 Hz), 7.26-7.38 (m, 1H), 6.97 (d, 2H, *J*=6.7 Hz), 3.86 (s, 3H); $^{13}C$ NMR (125 MHz, $CDCl_3$) δ 159.1, 140.8, 133.7, 128.7, 128-1, 126.7, 126.6, 114.2, 55.3; IR (neat, $cm^{-1}$) 3003, 1251, 1034, 834, 760. Anal Calcd for $C_{13}H_{12}O$: C, 84.75; H, 6.57. Found: C, 85.06; H, 6.72.

**4-Methylbiphenyl.**[23]

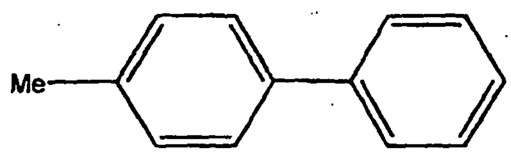

The general procedure gave 319 mg (95%) of a white solid: mp 44-46 °C (lit.[23] mp 49-50 °C); $^1H$ NMR (250 MHz, $CDCl_3$) δ 7.57 (d, 2H, *J*=8.8 Hz), 7.39-7.51 1 (m, 4H), 7.23-7.35 (m, 3H), 2.40 (s, 3H); $^{13}C$ NMR (125 MHz, $CDCl_3$) δ 141.2, 138.4, 136.9, 129.4, 128.7, 126.94, 126.92, 21.0; IR (neat, $cm^{-1}$) 3030, 1486, 822, 753. Anal Calcd for $C_{13}H_{12}$: C, 92.81; H, 7.19. Found: C, 92.86; H, 7.15.

**4-Methyl-2'-methoxybiphenyl.**[24]

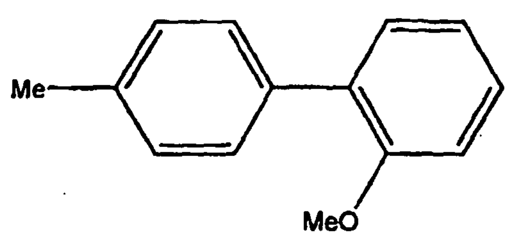

The general procedure was conducted on a 1 mmol scale using 1 mol% $Pd(OAc)_2$, 1.5 mol% ligand **2**, and 3 eq CsF in place of $K_3PO_4$ to give 196 mg (99%) of a white solid, mp 74-75 °C (lit.[24] mp 70-72 °C); $^1H$ NMR (250 MHz, $CDCl_3$) δ 7.42 (d, 2H, *J*=8.1 Hz), 7.21-7.33 (m, 4H), 7.16-7.04 (m, 2H), 3.81 (s, 3H), 2.39 (s, 3H); $^{13}C$ NMR (125 MHz, $CDCl_3$) δ 156.5, 136.5; 135.6, 130.7, 129.4, 128.6, 128.3, 120.8, 111.2, 55.5, 21.2; IR (neat, $cm^{-1}$) 2964, 1227, 1023, 757. Anal Calcd for $C_{14}H_{14}O$: C, 84.81; H, 7.12. Found: C, 84.94; H, 7.36.

**Suzuki coupling with dicyclohexylphenylphosphine as the supporting ligand.** Two coupling reactions of 4-chlorotoluene with phenylboronic acid using the general procedures for Suzuki couplings described above were carried out with dicyclohexylphenylphosphine (not according to the invention; 2L/Pd) in place of **2**. The reaction conducted at room temperature with CsF as the base proceeded to 10% conversion (5% GC yield) after 2 days, while the reaction at 100 ˚C with $K_3PO_4$ as the base proceeded to 27% conversion (18% GC yield) in 2 days.

**2-Methyl-4-(3,5-xylyl)-3-pentanone.**

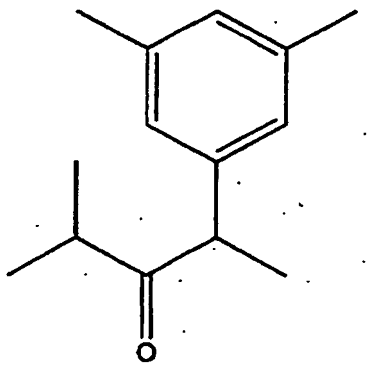

An oven-dried resealable Schlenk tube was charged with NaHMDS (238 mg, 13 mmol) under nitrogen in a Vacuum Atmospheres glovebox. The tube was capped with a teflon screw cap and removed from the glovebox. The screwcap was removed and $Pd_2(dba)_3$ (13.7 mg, 0.015 mmol, 3 mol% Pd) and **2** (14.1 mg, 0.036 mmol, 3.6 mol%) were added under a stream of argon. The tube was capped with a rubber septum and toluene (3 mL) was added with stirring. The flask was then charged with 5-bromo-m-xylene (0.135 mL, 1.0 mmol), 2-methyl-3-pentanone (0.15 mL, 1.2 mmol), and additional toluene (3 mL). The septum was replaced with a teflon screw cap and the reaction mixture was stirred at room temperature for 22 h until the starting aryl bromide had been completely consumed as judged by GC analysis. The reaction was quenched with 5 mL of saturated aqueous $NH_4Cl$, diluted with ether (20 mL), and poured into a separatory funnel. The layers were separated, and the aqueous phase was extracted with ether (10 mL). The combined organic layers were dried over anhydrous magnesium sulfate, filtered, and concentrated *in vacuo.* The crude material was purified by flash chromatography on silica gel to give 163 mg (80%) of a colorless oil. GC and NMR analysis showed the material obtained was a mixture of the desired product and a regioisomer containing the aryl group at the 2-position of the ketone (46/1 ratio by GC analysis; 40/1 ratio by $^1H$ NMR analysis). NMR data are given for the major product only. $^1H$ NMR (250 MHz, $CDCl_3$) $\delta$ 6.88 (s, 1H), 6.81 (s, 2H), 3.83 (q, 1H, *J*=6.9 Hz), 2.68 (p, 1H, *J*=6.9 Hz), 2.29 (s, 6H), 1.34 (d, 3H, *J*=6.9 Hz), 1.07 (d, 3H, *J*=7.0 Hz), 0.92 (d, 3H, *J*=6.6 Hz); $^{13}C$ NMR (125 MHz, $CDCl_3$) $\delta$ 214.7, 140.7, 138.3, 128.6, 125.7, 50.9, 39.0, 21.2, 19.3, 18.2, 18.1; IR (neat, $cm^{-1}$) 2972, 1710, 1141, 849. Anal (for the mixture) Calcd for $C_{14}H_{20}O$: C, 82.3; H, 9.87. Found: C, 82.09; H, 9.85.

**1,1-Bis(4-methylphenyl)-3-methyl-2-butanone.**

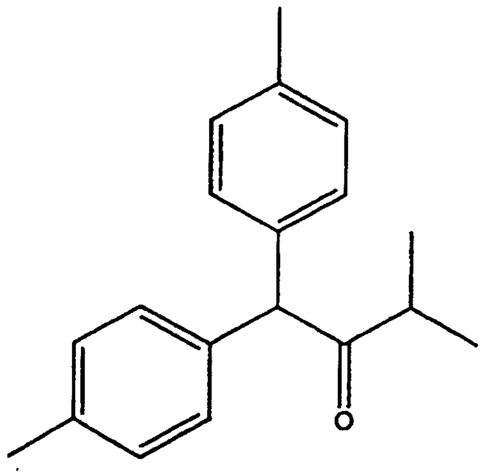

An oven-dried Schlenk tube was cooled under an argon purge and charged with $Pd_2(dba)_3$ (13.7 mg, 0.015 mmol, 3 mol% Pd), **2** (14.1 mg, 0.036 mmol, 3.6 mol%), and NaO*t*Bu (211 mg, 2.2 mmol). The flask was purged with argon, and toluene (3 mL) was added with stirring. The flask was then charged with 4-chlorotoluene (0.24 mL, 2.0 mmol), 3-methyl-2-butanone (0.105 mL, 1.0 mmol), and additional toluene (3 mL). The reaction mixture was stirred at room temperature for 2 min, then heated to 80˚C with stirring for 22 h at which time GC analysis showed the starting aryl chloride had been completely consumed. The reaction mixture was cooled to room temperature, quenched with saturated aqueous $NH_4Cl$ (5 mL), diluted with ether (20 mL), and poured into a separatory funnel. The layers were separated and the aqueous phase was extracted with ether (10 mL). The combined organic fractions were dried over anhydrous magnesium sulfate, filtered, and concentrated *in vacuo.* The crude material was purified by flash chromatography on silica gel to give 210 mg (79%) of a white solid: mp 48-51 ˚C; $^1$H NMR (300 MHz, $CDCl_3$) δ 7.00-7.18 (m, 8H), 5.22 (s, 1H), 2.79 (p, 1H, *J*=6.8 Hz), 2.31 (s, 6H), 1.10 (d, 6H, *J*=6.8 Hz); $^{13}$C NMR (125 MHz, $CDCl_3$) δ 212.3, 136.6, 135.8, 12924, 129.16, 128.9, 128.7, 61.4, 40.7, 21.0, 18.6; IR (neat, $cm^{-1}$) 2972, 1718, 1513, 1038, 803. Anal Calcd for $C_{14}H_{20}O$: C, 85.67; H, 8.32. Found: C, 86.02; H, 8.59.

**References for Supporting Information for Example 1**

(1) Parham, W. E.; Piccirilli, R. M. *J. Org. Chem.* **1977,** *42,* 257-260.

(2) Thompson, W. J.; Gaudino, J. *J. Org. Chem.* **1984,** *49,* 5237-5243.

(3) Zhang, X.; Mashima, K.; Koyano, K.; Sayo, N.; Kumobayashi, H.; Akutagawa, S.; Takaya, H. *J. Chem. Soc. Perkin Trans. 1* **1994**, 2309-2322.

(4) Miyashita, A.; Takaya, H.; Souchi, T.; Noyori, R. *Tetrahedron* **1984**, *40,* 1245-1253.

(5) Hegedus, L. S. in *Organometallics in Synthesis* Schlosser, M. Ed., John Wiley and Sons, West Sussex, England, 1994, p 448.

(6) Wolfe, J. P.; Buchwald, S. L. *J. Org. Chem.* **1996,** *61,* 1133-1135.

(7) Marcoux, J. -F.; Wagaw, S.; Buchwald, S. L. *J. Org. Chem.* **1997,** *62,* 1568-1569.

(8) Wolfe, J. P.; Buchwald, S. L. *J. Am. Chem. Soc.* **1997,** *119*, 6054-6058.

(9) Wolfe, J. P.; Buchwald, S. L. *J. Am. Chem. Soc.* **1996,** *118*, 7215-7216.

(10) Wolfe, J. P.; Buchwald, S. L. *Tetrahedron Lett.* **1997,** *38,* 6359-6362.

(11) Wolfe, J. P.; Buchwald, S. L. *J. Org. Chem.* **1997,** *62,* 1264-1267.

(12) Lauer, M.; Wulff, G. *J. Organomet. Chem.* **1983,** *256,* 1-9.

(13) Abe, M.; Takahashi, M. *Synthesis* **1990**, 939-942.

(14) Watanabe, Y.; Tsuji, Y.; Ige, H.; Ohsugi, Y.; Ohta, T. *J. Org. Chem.* **1984**, *49,* 3359-3363.

(15) Behringer, H.; Heckmaier, P. *Chem. Ber.* **1969,** *102***,** 2835-2850.

(16) Kotsuki, H.; Kobayashi, S.; Matsumoto, K.; Suenaga, H.; Nishizawa, H. *Synthesis* **1990**, 1147-1148.

(17) Häfelinger, G.; Beyer, M.; Burry, P.; Eberle, B.; Ritter, G.; Westermayer, G.; Westermayer, M. *Chem. Ber.* **1984,** *117,* 895-903.

(18) Novrocik, J.; Novrocikova, M.; Titz, M. *Coll. Czech. Chem. Commun.* **1980,** 3140-3149.

(19) Wirth, H. O.; Kern, W.; Schmitz, E. *Makromol. Chem.* **1963,** 68, 69-99.

(20) Barba, I.; Chinchilla, R.; Gomez, C. *Tetrahedron* **1990,** *46,* 7813-7822.

(21) Skraup, S.; Nieten, F. *Chem. Ber.* **1924,** 1294-1310.

(22) Darses, S.; Jeffery, T.; Brayer, J.-L.; Demoute, J.-P.; Genet, J.-P. *Bull. Soc. Chim. Fr.* **1996,** *133,* 1095-1102.

(23) Rao, M. S. C.; Rao, G. S. K. *Synthesis* **1987,** 231-233.

(24) Hatanaka, Y. Goda, K. -i.; Okahara, Y.; Hiyama, T. *Tetrahedron* **1994**, *50,* 8301-8316.

***Example 2***

Synthesis of *N*-(2,5-Dimethylphenyl)-*N*-methylaniline

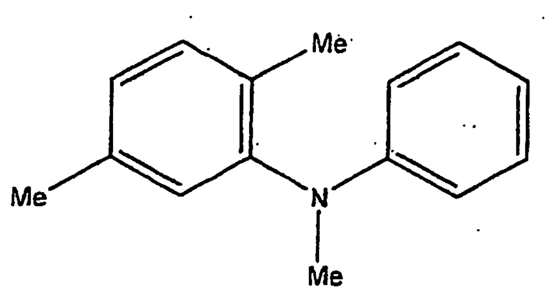

An oven-dried test tube was purged with argon and charged with $Pd_2(dba)_3$ (4.6 mg, 0.005 mmol, 1.0 mol% Pd), **2** [not according to the invention; Example 1] (6.0 mg, 0.015 mmol, 1.5 mol%), and NaO*t*-Bu (135 mg, 1.40 mmol). The test tube was fitted with a septum, then toluene (2.0 mL), *N*-methylaniline (135 µL, 1.25 mmol), and 2-chloro-*p*-xylene (135 µL, 1.01 mmol) were added. The mixture was stirred at 80˚C for 13 h, then cooled to room temperature, diluted with ether (20 mL), filtered and concentrated: The crude material was purified by flash chromatography on silica gel to afford 202 mg (95%) of a colorless oil.

***Example 3***

Synthesis of Di-*n*-butyl-*p*-toluidine.

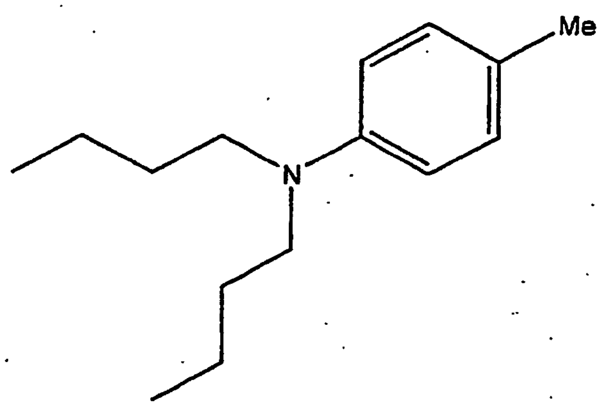

An oven-dried resealable Schlenk tube was purged with argon and charged with $Pd_2(dba)_3$ (2.3 mg, 0.0025 mmol, 0.05 mol% Pd), **2** [not according to the invention; Example 1] (2.9 mg, 0.0075 mmol, 0.075 mol%), and NaO*t*-Bu (1.34 g, 13.9 mmol). Toluene (10 mL), di-*n*-butylamine (2.00 mL, 11.9 mmol), and 4-chlorotoluene (1.18 mL, 10.0 mmol) were added and the mixture was degassed using three freeze-pump-thaw cycles. The reaction vessel was placed under argon, sealed with a teflon screw cap, and stirred in a 100 °C oil bath for 20 h after which time GC analysis showed the aryl halide had been completely consumed. The reaction mixture was cooled to room temperature, diluted with ether (100 mL) and extracted with 1 M HCl (3 x 100 mL). The combined aqueous acid phase was basified with 3N NaOH, then extracted with ether (3 x 150 mL). The ethereal extracts were dried over anhydrous sodium sulfate; filtered and concentrated to afford 2.01 g (95%) of a pale yellow oil.

***Example 4***

Synthesis of *N*-(4-Cyanophenyl)morpholine.

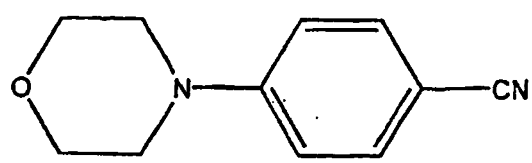

An oven-dried resealable Schlenk tube was purged with argon and charged with $Pd_2(dba)_3$ (11.5 mg, 0.025 mmol, 5 mol% Pd), **2** [not according to the invention; Example 1] (14.8 mg, 0.075 mmol, 7.5 mol%), NaO*t*-Bu (68 mg, 0.71 mmol) and 4-chlorobenzonitrile (69 mg, 0.50 mmol). The tube was purged with argon then DME (0.5 mL) and morpholine (53 μL, 0.61 mmol) were added through a rubber septum. The septum was removed, the tube was sealed with a teflon screw cap and the mixture was stirred at room temperature for 26 h. The reaction was then diluted with EtOAc (20 mL), filtered through celite and concentrated *in vacuo.* The crude material was purified by flash chromatography on silica gel to afford 91 mg (96%) of a tan solid.

***Example 5***

Synthesis of *N*-(2,5-Dimethylphenyl)morpholine.

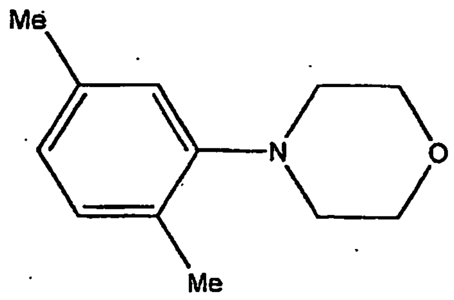

An oven-dried resealable Schlenk tube was purged with argon and charged with $Pd_2(dba)_3$ (13.9 mg, 0.015 mmol, 3.0 mol% Pd), **2** [not according to the invention; Example 1] (17.9 mg, 0.045 mmol, 4.5 mol%), and NaO*t*-Bu (140 mg, 1.4.mmol). The tube was purged with argon, fitted with a rubber septum and then DME (0.5 mL), 2-bromo-*p*-xylene (140 μL, 1.01 mmol) and morpholine (105 μL, 1.2 mmol) were added via syringe. The septum was removed, the tube was sealed with a teflon screw cap and the mixture was stirred at room temperature for 24 h. The reaction mixture was then diluted with ether (20 mL), filtered through celite and concentrated *in vacuo.* The crude material was purified by flash chromatography on silica gel to afford 185 mg (95%) of a colorless oil.

***Example 6***

Synthesis of *N*-(4-Carbomethoxyphenyl)morpholine.

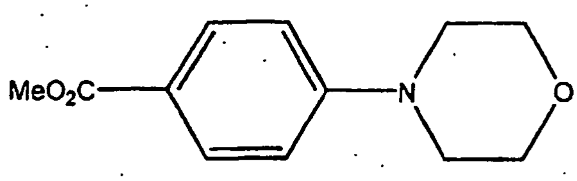

An oven-dried resealable Schlenk tube was purged with argon and charged with $Pd_2(dba)_3$ (2.3 mg, 0.0025 mmol, 1.0 mol% Pd), **2** [not according to the invention; Example 1] (3:0 mg, 0.0076 mmol, 1.5 mol%), $K_3PO_4$ (150 mg, 0.71 mmol), and methyl 4-bromobenzoate (108 mg, 0.50 mmol). The tube was purged with argon, fitted with a rubber septum and then DME (1.0 mL) and morpholine (55 μL, 0.63 mmol) were added. The septum was removed, the tube was sealed with a teflon screw cap and the mixture was stirred at 80 ˚C for 24 h. The reaction mixture was then cooled to room temperature, diluted with EtOAc (20 mL), filtered through celite and concentrated *in vacuo.* The crude material was purified by flash chromatography on silica gel to afford 89 mg (80%) of a colorless solid.

***Example 7***

Synthesis of *N*-benzyl-*p*-toluidine.

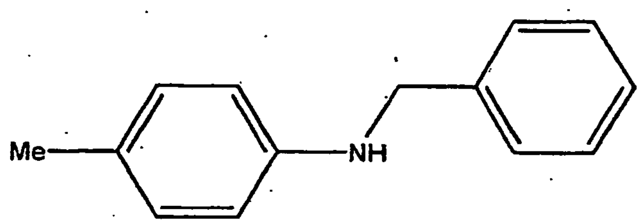

An oven dried Schlenk tube was purged with argon and charged with $Pd_2(dba)_3$ (4.6 mg, 0.005 mmol, 1.0 mol% Pd), Cy-BINAP (not according to the invention; 9.6 mg, 0.015 mmol, 1.5 mol%), and-NaO*t*Bu (135 mg, 1.4 mmol).

The tube was purged with argon and charged with toluene (2 mL), 4-chlorotoluene (0.12 mL, 1.0 mmol), and benzylamine (0.165 mL, 1.5 mmol). The mixture was heated to 100°C with stirring until the starting aryl chloride had been completely consumed as judged by GC analysis. The reaction mixture was cooled to room temperature, diluted with ether (20 mL), filtered through celite, and concentrated *in vacuo.* The crude material was then purified by flash chromatography on silica gel to give 177 mg (90%) of a pale yellow oil.

***Example 8***

Synthesis of 3.5-dimethylbiphenyl via Suzuki Coupling

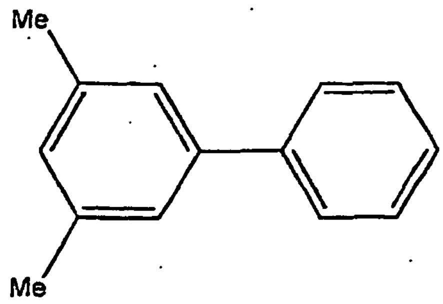

An oven dried resealable Schlenk tube was purged with argon and charged with palladium acetate (2.2 mg, 0.01 mmol, 1 mol%), ligand **2** [not according to the invention; Example 1] (5.9 mg, 0.015 mmol, 1.5 mol%), phenylboron dihydroxide (183 mg, 1.5 mmol), and cesium fluoride (456 mg, 3.0 mmol). The tube was purged with argon, and dioxane (3 mL) and 5-bromo-m-xylene (0.135 mL, 1.0 mmol) were added through a rubber septum. The septum was removed, the tube was sealed with a teflon screw cap and the mixture was stirred at room temperature until the starting aryl bromide had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel to give 171 mg (94%) of a colorless oil.

***Example 9***

Synthesis of 4-methylbiphenyl via Suzuki Coupling

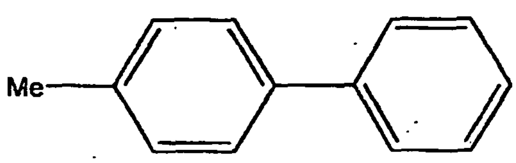

An oven dried resealable Schlenk tube was purged with argon and charged with palladium acetate (4.4 mg, 0.02 mmol, 2 mol%), ligand **2** [not according to the invention; Example 1] (11.9 mg, 0.03 mmol, 3 mol%), phenylboron dihydroxide (183 mg, 1.5 mmol), and cesium fluoride (456 mg, 3.0 mmol). The tube was purged with argon, and dioxane (3 mL) and 4-chlorotoluene (0.12mL, 1.0 mmol) were added through a rubber septum. The septum was removed, the tube was sealed with a teflon screw cap and the mixture was stirred at room temperature until the starting, aryl chloride had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated in *vacuo.* The crude material was then purified by flash chromatography on silica gel to give 157 mg (93%) of a glassy solid.

***Example 10***

Synthesis of 3-methyl-4'-acetylbiphenyl via Suzuki Coupling

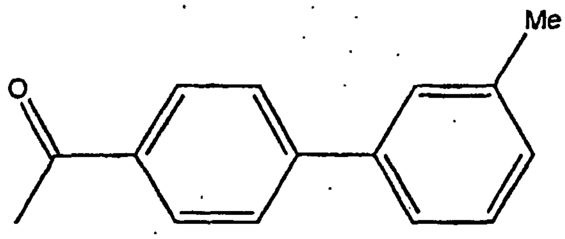

An oven dried resealable Schlenk tube was purged with argon and charged with palladium acetate (4.4 mg, 0.02 mmol, 2 mol%), ligand **2** [not according to the invention; Example 1] (11.9 mg, 0.03 mmol, 3 mol%), 3-methylphenylboronic acid (204 mg, 1.5 mmol), and cesium fluoride (456 mg, 3.0 mmol). The tube was purged with argon, and dioxane (3 mL), and 4-chloroacetophenone (0.13 mL, 1.0 mmol) were added through a rubber septum. The septum was removed, the tube was sealed with a teflon screw cap and the mixture was stirred at room temperature until the starting aryl chloride had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel to give 195 mg (93%) of a white solid.

***Example 11***

Synthesis of 4-methoxybiphenyl via Suzuki Coupling

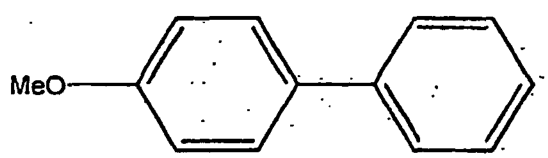

An oven dried resealable. Schlenk tube was purged with argon and charged with palladium acetate (2.2 mg, 0.01 mmol, 0.5 mol%), ligand **2** [not according to the invention; Example 1] (5.9 mg, 0.015 mmol, 0.75 mol%), phenylboron dihydroxide (366 mg, 3.0 mmol), and potassium phosphate (850 mg, 4.0 mmol). The tube was purged with argon, and dioxane (6 mL), and 4-chloroanisole (0.24 mL, 2.0 mmol) were added through a rubber septum. The septum was removed, the tube was sealed with a teflon screw cap and the mixture was stirred at room temperature for two minutes, then heated to 100 ˚C with stirring until the starting aryl chloride had been completely consumed as judged by GC analysis. The reaction mixture was then diluted with ether (40 mL) and poured into a separatory funnel. The mixture was washed with 1M NaOH (40 mL), and the layers were separated. The aqueous layer was extracted with ether (40 mL), and the combined organic extracts were dried over anhydrous magnesium sulfate, filtered, and concentrated *in vacuo.* The crude material was then purified by flash chromatography on silica gel to give 347 mg (94%) of a white solid.

***Example 12***

Synthesis of 2-amino-2'-bromo-1,1'-binaphthyl benzophenone imine

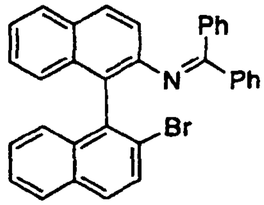

An oven-dried 100 mL round-bottom flask was fitted with a reflux condenser, purged with argon and charged with 2,2'-dibromo-1,1'-binaphthyl (5.0 g, 12.1 mmol), benzophenone imine (2.9 g, 15.7 mmol), NaO*t*-Bu (1.7 g, 18.0 mmol), $Pd_2(dba)_3$ (110 mmol, 0.12mmol), bis(2-(diphenylphosphino)phenyl)ether (129 mg , 0.24 mmol), and toluene (50 mL). The mixture was stirred for 18 hours at 100 ˚C then cooled to room temperature and two-thirds of the solvent was removed under reduced pressure. Ethanol (25 mL) and water (3 mL) were added to the resulting mixture. The yellow crystals were collected on a Büchner funnel and washed with ethanol (10 mL) to afford 5.7 g (92%) of crude material which was used in the following Example without further purification.

***Example 13***

Synthesis of 2-amino-2'-bromo-1,1'-binaphthyl

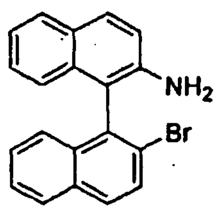

The crude imine from Example 12 (3.0 g, 5.9 mmol) was suspended in dichloromethane (100 mL) in a 300 mL round bottom flask. Concentrated hydrochloric acid (1.5 mL, 17. 6 mmol) was added to the suspension which became homogeneous within 15 min. The reaction mixture was stirred for 18 hours at room temperature during which time a precipitate formed. The mixture was them treated with 1 M NaOH (25 mL), and the layers were separated. The aqueous layer was extracted with additional dichloromethane (10 mL). The combined organic layers were washed with brine, dried over anhydrous magnesium sulfate, filtered, and concentrated *in vacuo.* The crude material was then purified by flash chromatography on silica gel to give 1.5 g (73%) of colorless crystals.

***Example 14***

Synthesis of 2-*N,N*-dimethylamino-2'-bromo-1,1'-binaphthyl

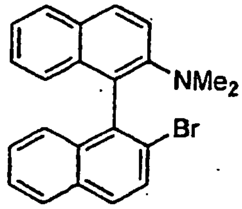

A 20 mL round-bottom flask was charged with amine from Example 13 (480 mg, 1.4 mmol), iodomethane (0.25 mL, 4.2 mmol), sodium carbonate (318 mg, 3.0 mmol), and DMF (8 mL), and then purged with argon. The mixture was heated to 50 ˚C and stirred until the starting material had been completely consumed. The reaction mixture was diluted with ether (5 mL) and water (1 mL) and then passed through a plug of silica gel. The filtrate was dried over anhydrous

magnesium sulfate, filtered, and concentrated *in vacuo* to give 473 mg (91%) of colorless crystals.

***Example 15***

Synthesis of 2-*N*,*N*-dimethylamino-2'-diphenylphosphino-1,1'-binaphthyl (**26**)

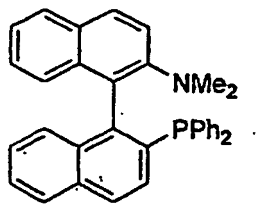

**26**

An oven-dried 20 mL round-bottom flask was charged with bromide from Example 14 (300 mg, 0.8 mmol) and THF (8 mL). The mixture was purged with argon and cooled to -78 ˚C, then *n*-butyllithium (0.6 mL, 0.9 mmol) was added dropwise. The solution and stirred at -78˚C for 45 min, then chlorodiphenylphosphine (229 mg, 1.0 mmol) was added dropwise. The reaction was stirred for 1 hour at -78 ˚C, then was allowed to warm to room temperature and stirred for 18 hours. Saturated aqueous ammonium chloride (2 mL) was added and the reaction mixture was extracted with ether (2x10 mL). The combined organic extracts were dried over anhdrous magnesium sulfate, filtered, and concentrated *in vacuo*. The crude material was purified by flash chromatography on silica gel to give 340 mg (88%) of **26** as colorless crystals. This ligand is according to the invention.

***Example 16***

Synthesis of 2-*N,N*-dimethylamino-2'-dicyclohexylphoshino-1,1'-binaphthyl (**27**)

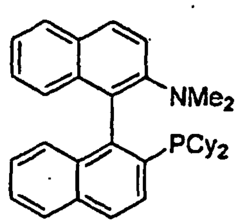

**27**

An oven-dried 20 mL round-bottom flask was charged with bromide from Example 14 (600 mg , 1.6 mmol) and THF (16 mL). The mixture was purged with argon and cooled to -78 ˚C, then *n*-butyllithium (1.1 mL, 1.8 mmol) was added dropwise. The solution was stirred at -78 ˚C for 45 min, then chlorodicyclohexylphosphine (484 mg, 2.1 mmol) was added dropwise. The reaction was stirred for 1 hour at -78 ˚C, then was allowed to warm to room temperature and stirred for 18 hours. Saturated aqueous ammonium chloride (2 mL) was added and the reaction mixture was extracted with ether (2 x 10 mL). The combined organic extracts were dried over anhdrous magnesium sulfate, filtered, and concentrated *in vacuo.* The crude material was recrystalized from dichloromethane and methanol to give 623 mg (79%) of **27** as colorless crystals. This ligand is according to the invention.

***Example 17***

Synthesis of N-(4-methoxyphenyl)pyrrolidine

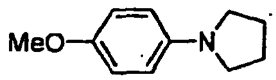

An oven dried test tube was charged with $Pd_2(dba)_3$ (4.5 mg, 0.005 mmol), **27** (according to the invention; Example 16, 7.4 mg, 0.015 mmol), 4-chloroanisole (140 mg, 0.98 mmol), pyrrolidine (85 mg, 1.2 mmol), NaO*t*-Bu (135 mg, 1.4 mmol), toluene (2 mL), and purged with argon. The mixture was heated to 80 ˚C and stirred for 18 hours. The reaction mixture was cooled to room temperature, diluted with ether (5 mL), filtered through a plug of celite, and concentrated *in vacuo.* The residue was purified by flash chromatography on silica gel to give 165 mg (95%) of the title product as colorless crystals.

***Example 18***

Synthesis of N-benzyl-*p*-toluidine

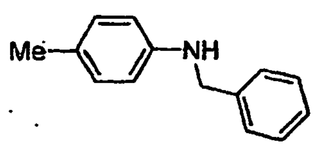

An oven dried resealable Schlenk tube was purged with argon and charged with $Pd_2(dba)_3$ (4.6 mg, 0.005 mmol, 1.0 mol% Pd), **27** (according to the invention; Example 16, 7.4 mg, 0.015 mmol, 1.5 mol%), and NaO*t*Bu (135 mg; 1.4 mmol). The tube was purged with argon and toluene (2 mL), 4-chlorotoluene (0.12 mL, 1.0 mmol), and benzylamine (0.165 mL, 1.5 mmol) were added through a rubber septum. The septum was replaced with a teflon screw cap, the tube was sealed, and the mixture was heated to 100 ˚C with stirring until the starting aryl chloride had been completely consumed as judged by GC analysis. A small amount of diarylated benzylamine was detected in the crude reaction mixture (GC ratio of product/diarylated benzylamine=16/1). The reaction mixture was cooled to room temperature, diluted with ether (20 mL), and extracted with 1 M HCl (5x40 mL). The organic phase was discarded and the combined aqueous extracts were basisified to pH 14 with 6M NaOH and extracted with ether (4x50 mL). The combined organic extracts were dried over anhydrous magnesium sulfate, filtered, and concentrated *in vacuo* to give give 175 mg (89%) of a pale yellow oil.

***Example 19***

Synthesis of *N*-(4-methylphenyl)indole

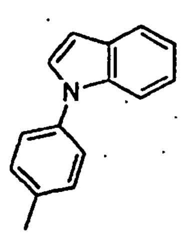

An oven-dried resealable Schlenk tube was purged with argon and charged with $Pd_2(dba)_3$ (11.2 mg, 0.012

mmol, 2.5 mol% Pd), **2** [not according to the invention; Example 1] (14.4 mg, 0.036 mmol, 7.5 mol%), NaO*t*-Bu (130 mg, 1.35 mmol) and indole (115 mg, 0.98 mmol). The tube was purged with argon then toluene (1.0 mL) and 4-bromotoluene (120 μL, 0.98 mmol) were added through a rubber septum. The septum was removed, the tube was sealed with a teflon screw cap and the mixture was stirred at 100 °C for 21 h. The reaction was then diluted with ether (20 mL), filtered through celite and concentrated *in vacuo.* The crude material was purified by flash chromatography on silica gel to afford 191 mg (94%) of a colorless oil.

***Example 20***

Synthesis of *N*-(4-fluorophenyl)indole

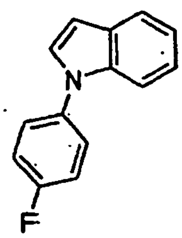

An oven-dried resealable Schlenk tube was purged with argon and charged with $Pd_2(dba)_3$ (11.5 mg, 0.013 mmol, 5 mol% Pd); **2** [not according to the invention; Example 1] (14.8 mg, 0.038 mmol, 7.5 mol%). NaO*t*-Bu (68 mg, 0.71 mmol) and indole (60 mg, 0.51 mmol). The tube was purged with argon then toluene (0.5 mL) and 1-bromo-4-fluorobenzene (55 μL, 0.50 mmol) were added through a rubber septum. The septum was removed, the tube was sealed with a teflon screw cap and the mixture was stirred at 100 °C for 36 h. The reaction was then diluted with ether (20 mL), filtered through celite and concentrated *in vacuo.* The crude material was purified by flash chromatography on silica gel to afford 81 mg (77%) of a colorless oil.

***Example 21***

Synthesis of *N*-(4-methylphenyl)indole

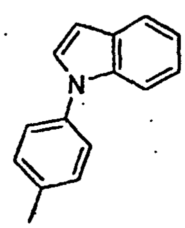

An oven-dried resealable Schlenk tube was purged with argon and charged with $Pd_2(dba)_3$ (11.6 mg, 0.012 mmol, 5 mol% Pd), **2** [not according to the invention; Example 1] (11.0 mg, 0.028 mmol, 5.5 mol%), $Cs_2CO_3$ (230 mg, 0.75 mmol) and indole (60 mg, 0.51 mmol). The tube was purged with argon then toluene (1.0 mL) and 4-chlorotoluene (60 μL, 0.51 mmol) were added through a rubber septum. The septum was removed, the tube was sealed with a teflon screw cap and the mixture was stirred at 100 °C for 24 h. The reaction was then diluted with ether (20 mL), filtered through celite and concentrated *in vacuo.* The crude material was purified by flash chromatography on silica gel to afford 94 mg (89%) of a colorless oil.

***Example 22***

Synthesis of 2-Bromo-2'-methoxyl-1,1'-biphenyl

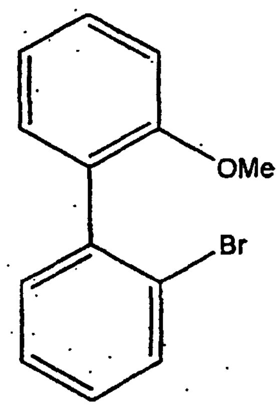

2-Bromoiodobenzene (640 μL, 5.0 mmol) was added to a suspension of $Pd(PPh_3)_4$ (305 mg, 0.26 mmol) in DME (100 mL) at room temperature under argon. After 15 min at room temperature, a solution of 2 methoxyphenylboronic acid (760 mg, 5.0 mmol) in ethanol (2 mL) was added, followed by aqueous $Na_2CO_3$ (2.0 M, 5 mL, 10 mmol). The reaction vessel was fitted with a reflux condenser and heated to reflux under argon for 22.5 h. The reaction mixture was then cooled to room temperature and filtered through Celite. The filter cake was washed with ether and water, and the filtrate was concentrated *in vacuo.* The resulting aqueous residue was diluted with brine and extracted with ether. The ethereal layer was dried ($MgSO_4$), filtered and concentrated. The crude residue was purified by flash chromatography on silica gel to afford 823 mg (63%) of a colorless oil.

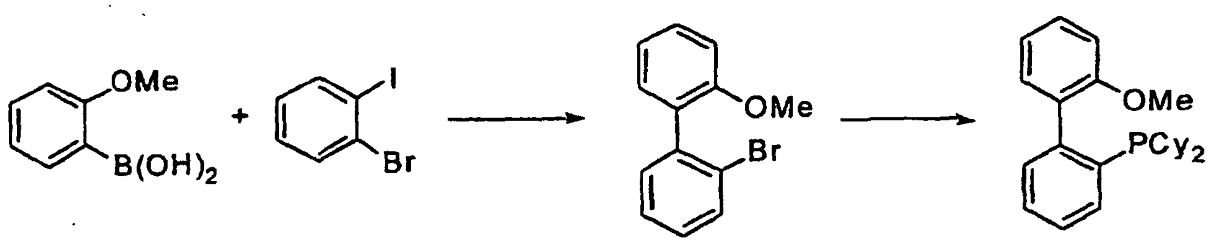

1

2

***Example 23***

Synthesis of 2-Dicyclohexylphosphino-2'-methoxy-1,1'-biphenyl

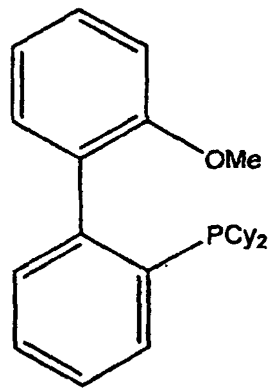

A solution of **1** (Example 22, 535 mg, 2.03 mmol) in THF (20 mL) was cooled to -78 °C under argon, then *n*-BuLi (1.6 M in hexane, 1.35 mL, 2.16 mmol) was added dropwise. After 2.5 h at -78 °C, a solution of chlorodicyclohexylphosphine (570 mg, 2.45 mmol) in THF (3 mL) was added over 10 min. The reaction mixture was then allowed to warm to room temperature overnight, then quenched with saturated aqueous $NaHCO_3$ and concentrated in vacuo. The resulting aqueous suspension was extracted with ether (2x50 mL), and the combined ethereal layers were dried ($Na_2SO_4$), filtered and concentrated *in vacuo.* The resulting crude solid was recrystallized from ethanol to afford 420 mg (54 %) of a white solid.

***Example 24***

Synthesis of *N*-(4-methylphenyl)indole

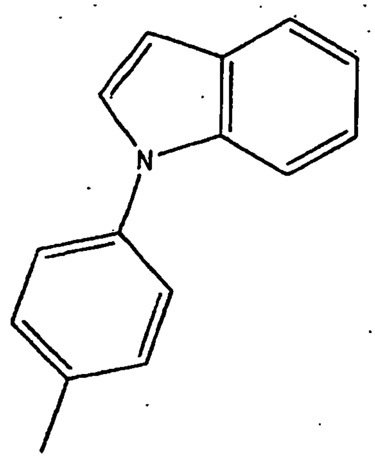

An oven-dried test tube was purged with argon and then charged with 2-dicyclohexylphosphino-2'-methoxy-1,1'-biphenyl (not according to the invention; 14.5 mg, 0.038 mmol, 7.5 mol%) and $Pd_2(dba)_3$ (11.6 mg, 0.013 mmol, 5.0 mol% Pd). Toluene (1.0 mL), indole (71 mg, 0.61 mmol), 4-chlorotoluene (60 mL, 0.51 mmol); and NaOt-Bu (70 mg, 0.73 mmol) were then added. The tube was fitted with a septum, purged with argon and heated at 100 °C for 28 h. The reaction was then cooled to room temperature, diluted with ether (20 mL), filtered through Celite and concentrated *in vacuo.* The residue was purified by flash chromatography on silica gel to afford 99 mg (94%) of a colorless oil.

***Example 25***

Synthesis of 2-(di-*tert*-butylphosphino)biphenyl

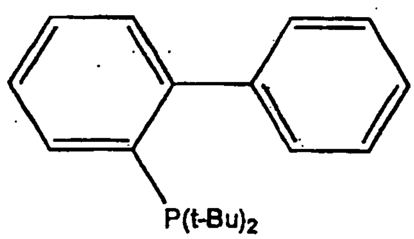

A solution of 2-bromobiphenyl (5.38 g, 23.1 mmol) and a few iodine crystals in 40 mL of THF with magnesium turnings (617 mg, 25.4 mmol) was heated to a reflux for 2 h. Heat was temporarily removed for the addition of cuprous chloride (2.40 g, 24.2 mmol) followed by chloro-di-*tert*-butylphosphine. Heating was resumed for 8 h. The reaction mixture was then removed from heat and allowed to cool to rt. The reaction mixture was poured onto 200 mL of 1:1 hexane/ether. The suspension was filtered and the filtercake was washed with 60 mL of hexane. The solid was partitioned between 150 mL of 1:1 hexane/ethyl acetate and 60 mL of concentrated ammonium hydroxide with 100 mL of water. The organic layer was washed with 100 mL of brine, dried over anhydrous sodium sulfate, and concentrated *in vacuo.* The white solid was recrystallized from 30 mL of MeOH to give white crystals of 2-(di-*tert*- butylphosphino)biphenyl (4.01 g, 58%). A second crop (464 mg, 67%) was obtained by recrystallization from 50 mL of MeOH and 25 mL of water.

***Example 26***

General procedure for determining the effect of various additives on the preparation of 4-methylbiphenyl via Suzuki coupling

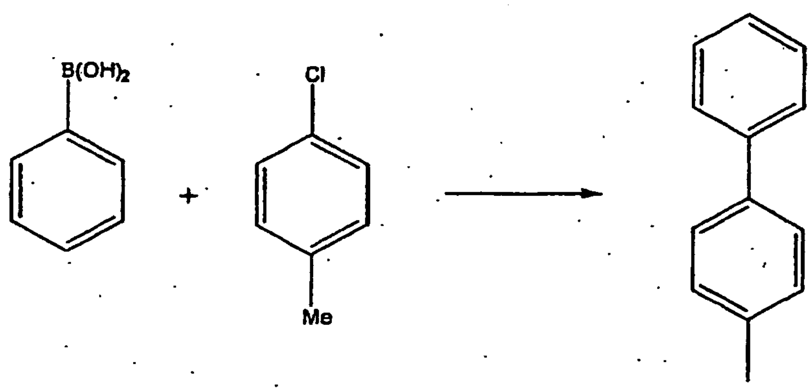

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 4.5 mg, 0.015 mmol, 1.5 mol%), phenylboron dihydroxide (183 mg, 1.5 mmol), additive (3.0 mmol), and 4-chlorotoluene (0.12 mL, 1.0 mmol). The tube was evacuated and backfilled with argon, and THF (2 mL) was added through a rubber septum. The reaction mixture was stirred at room temperature for 20 hours. The reaction mixture was then diluted with ethyl acetate (30 mL) and poured into a separatory funnel. The mixture was washed with 2.0M NaOH (20 mL), followed by brine (20 mL). The organic layer was submitted for GC analysis giving the results tabulated below.

| *Additive* | *Conversion* |
|---|---|
| Cesium Fluoride | 55% |
| Potassium Fluoride | 62% |
| Potassium Carbonate | 10% |
| Potassium Phosphate | 38% |
| Sodium Acetate | 0% |

***Example 27***

Synthesis of 4-*t*-butylbiphenyl using $K_3PO_4$ as base with 0.1 mol% Pd

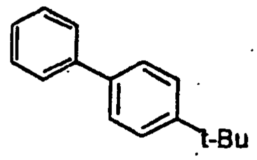

An oven dried resealable Schlenk tube was evacuated and backfilled with argon and charged with phenylboron dihydroxide (183 mg, 1.5 mmol), and potassium phosphate (425 mg, 2.0 mmol). The tube was evacuated and' backfilled with argon, and DME (1.5 mL) and 1-bromo-4-*t*-butylbenzene (0.17 mL, 1.0 mmol) were added through a rubber septum. A separate flask was charged with $Pd_2(dba)_3$ (4.6 mg, 0.005 mmol), 2-(di-tert-butylphosphino)biphenyl (not according to the invention; 4.5 mmol 0.015 mmol), and DME (1 mL). The mixture was stirred for 1 minute at room temperature, then 100 μL of this solution (0.1 mol% Pd, 0.15 mol% 2-(di-tert-butylphosphino)biphenyl) was added to the Schlenk tube followed by additional THF (1.5 mL). The septum was removed, the tube was sealed with a teflon screw cap and the mixture was stirred at room temperature for 2 minutes, then heated to 80 ˚C with stirring until the starting aryl bromide had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel to give 199 mg (95%) of a glassy solid.

***Example 28***

Synthesis of 4-*t*-butylbiphenyl using CsF as base with 0.05 mol% Pd

An oven dried resealable Schlenk tube was evacuated and backfilled with argon and charged with phenylboron dihydroxide (183 mg, 1.5 mmol), and cesium fluoride (456 mg, 3.0 mmol). The tube was evacuated and backfilled with argon, and THF (1.5 mL) and 1-bromo-4-*t*-butylbenzene (0.17 mL, 1.0 mmol) were added through a rubber septum. A separate flask was charged with $Pd_2(dba)_3$ (4.6 mg, 0.005 mmol), 2-(di-tert-butylphosphino)biphenyl (not according to the invention; 4.5 mmol, 0.015 mmol), and THF (1 mL). The mixture was stirred for 1 minute at room temperature, then 50 μL of this solution (0.05 mol% Pd, 0.075 mol% 2-(di-tert-butylphosphino)biphenyl) was added to the Schlenk tube followed by additional THF (1.5 mL). The septum was removed, the tube was sealed with a teflon screw cap and the mixture was stirred at room temperature for 2 minutes, then heated to 80 ˚C with stirring until the starting aryl bromide had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica get to give 202 mg (96%) of a glassy solid.

***Example 29***

Optimized synthesis of 4-methylbiphenyl utilizing KF

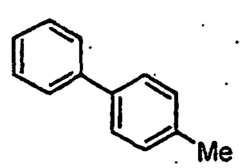

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), phenylboron dihydroxide (183 mg, 1.5 mmol), and potassium fluoride (174 mg, 3.0 mmol). The tube was evacuated and backfilled with argon, and THF (1 mL) and 4-chlorotoluene (0.12 mL, 1.0 mmol) were added through a rubber septum. The tube was sealed with a teflon screwcap, and the reaction mixture was stirred at room temperature until the starting aryl chloride had been completely consumed as judged by GC analysis. The reaction mixture was then diluted with ether (30. mL) and poured into a separatory funnel. The mixture was washed with. 1.0 M NaOH (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 158 mg (94%) of the title compound.

***Example 30***

Synthesis of 2-cyanomethylbiphenyl

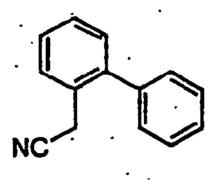

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), phenylboron dihydroxide (183 mg, 1.5 mmol), and potassium fluoride (174 mg, 3.0 mmol). The tube was evacuated and backfilled with argon, and THF (1 mL) and 2-chlorobenzyl cyanide (152 mg, 1.0 mmol) were added through a rubber septum. The tube was sealed with a teflon screwcap, and the reaction mixture was stirred at room temperature until the starting aryl chloride had been completely consumed as judged by GC analysis. The reaction mixture was then diluted with ether (30 mL) and poured into a separatory funnel. The mixture was washed with 1.0 M NaOH (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica, gel to afford 178 mg (92%) of the title compound.

***Example 31***

Synthesis of 4-carbomethoxy-3'-acetylbiphenyl

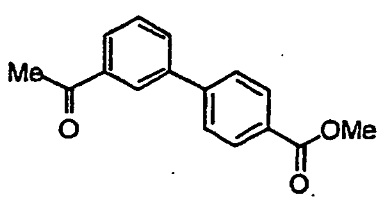

An oven dried resealable Schlenk tube was evacuated and backfilled with argon and charged with palladium acetate (2,2 mg, 0.01 mmol, 1.0 mol%), 2-(di-tert-butylphosphino)biphenyl (not according to the invention; 6.0 mg, 0.020 mmol, 2.0 mol%), 3-acetylphenyl boronic acid (246 mg, 1.5 mmol), potassium fluoride (174 mg, 3.0 mmol), and methyl-4-chlorobenzoate (171 mg, 1.0 mmol). The tube was evacuated and backfilled with argon, and THF (1 mL) was added through a rubber septum. The tube was sealed with a teflon screwcap, and the reaction mixture was stirred at room temperature until the starting aryl chloride had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash

chromatography on silica gel to afford 229 mg (90%) of the title compound.

***Example 32***

Synthesis of 4-cyanobiphenyl

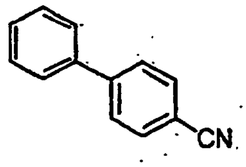

An oven dried resealable Schlenk tube was evacuated and backfilled with argon and charged with palladium acetate. (2,2 mg, 0.01 mmol, 1.0 mol%), 2-(di-tert-butylphosphino)biphenyl (not according to the invention; 6.0 mg, 0.020 mmol, 2.0 mol%), phenylboronic acid (183 mg, 1.5 mmol), potassium fluoride (174 mg, 3.0 mmol), and 4-chlorobenzonitrile (136 mg, 1.0 mmol). The tube was evacuated and backfilled with argon; and THF (1 mL) was added through a rubber septum. The tube was sealed with a teflon screwcap, and the reaction mixture was stirred at room temperature until the starting aryl chloride had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 159 mg (89%) of the title compound.

***Example 33***

Synthesis of 4-formyl-4'-ethoxybiphenyl

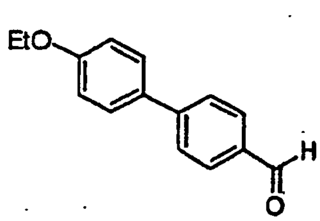

An oven dried resealable Schlenk tube was evacuated and backfilled with argon and charged with palladium acetate (1.1 mg, 0.005 mmol, 0.5 mol%), 2-(di-tert-butylphosphino)biphenyl (not according to the invention; 3.0 mg, 0.01 mmol, 1.0 mol%), 4-ethoxyphenylboronic acid (249 mg, 1.5 mmol), potassium fluoride (174 mg, 3.0 mmol), and 4-bromobenzaldehyde (185 mg, 1.0 mmol). The tube was evacuated and backfilled with argon, and THF (1 mL) was added through a rubber septum. The tube was sealed with a teflon screwcap, and the reaction mixture was stirred at room temperature until the starting aryl bromide had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 203 mg (90%) of the title compound.

***Example 34***

Synthesis of 4-hydroxybiphenyl

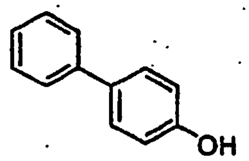

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.02 mmol, 2.0 mol%), phenylboronic acid (183 mg, 1.5 mmol), potassium fluoride (174 mg, 3.0 mmol), and 4-bromophenol (173 mg, 1.0 mmol). The tube was evacuated and backfilled with argon, and THF (1 mL) was added through a rubber septum. The tube was sealed with a teflon screwcap, and the reaction mixture was stirred at room temperature until the starting aryl bromide had been completely consumed as judged by GC analysis. The reaction mixture was then diluted with ether (30 mL), filtered through celite, and concentrated. The crude material was purified by flash chromatography on silica gel to afford 154 mg (91%) of the title compound.

***Example 35***

Synthesis of 2-hydroxymethylbiphenyl

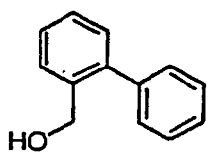

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.02 mmol, 2.0 mol%), phenylboronic acid (183 mg, 1.5 mmol), potassium fluoride (174 mg, 3.0 mmol), and 2-bromobenzyl alcohol (187 mg, 1.0 mmol). The tube was evacuated and backfilled with argon, and THF (1 mL) was added through a rubber septum. The tube was sealed with a teflon screwcap, and the reaction mixture was stirred at 50 C until the starting aryl bromide had been completely consumed as judged by GC analysis. The reaction mixture was then diluted with ether (30 mL), filtered througth celite, and concentrated. The crude material was purified by flash chromatography on silica gel to afford 153 mg (83%) of the title compound.

***Example 36***

Synthesis of 2,5-dimethylbiphenyl

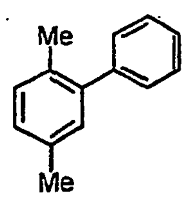

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol 2.0 mol%), phenylboronic acid (183 mg, 1.5 mmol); and potassium fluoride (174 mg, 3.0 mmol). The tube was evacuated and backfilled with argon, and THF (1 mL) and 2-bromo-*p*-xylene. (0.138 mL, 1.0 mmol) were added through a rubber septum. The tube was sealed with a teflon screwcap, and the reaction mixture was stirred at room temperature until the starting aryl bromide had been completely consumed as judged by GC analysis. The reaction mixture was then

diluted with ether (30 mL), filtered through celite, and concentrated, The crude material was purified by flash chromatography on silica gel to afford 149 mg (82%) of the title compound.

***Example 37***

Synthesis of 4-methoxybiphenyl

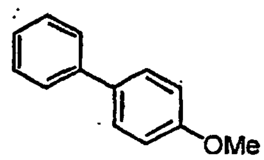

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), phenylboronic acid (183 mg, 1.5 mmol), and potassium fluoride (174 mg, 3.0 mmol). The tube was evacuated and backfilled with argon, and THF (1 mL) and 4-chloroanisole (0.123 mL, 1.0 mmol) were added through a rubber septum. The tube was sealed with a teflon screwcap, and the reaction mixture was stirred at room temperature until the starting aryl chloride had been completely consumed as judged by GC analysis. The reaction mixture was then diluted with ether (30 mL), filtered through celite, and concentrated. The crude material was purified by flash chromatography on silica gel to afford 176 mg (96%) of the title compound.

***Example 38***

Synthesis of N-acetyl-4-aminobiphenyl

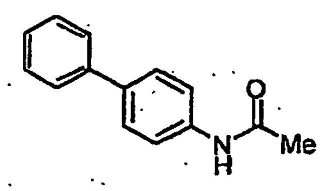

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.02 mmol, 2.0 mol%), phenylboronic acid (183 mg, 1.5 mmol), potassium fluoride (174 mg, 3.0 mmol), and 4'-bromoacetanilide (214 mg, 1.0 mmol). The tube was evacuated and backfilled with argon, and THF (1 mL) was added through a rubber septum. The tube was sealed with a teflon screwcap, and the reaction mixture was stirred at 50 C until the starting aryl bromide had been completely consumed as judged by GC analysis. The reaction mixture was then diluted with ether (30 mL), filtered through celite, and concentrated. The crude material was purified by flash chromatography on silica gel to afford 182 mg (86%) of the title compound.

***Example 39***

Synthesis of 4-nitrobiphenyl

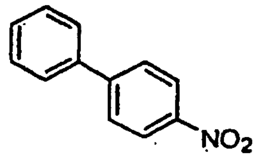

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.02 mmol, 2.0 mol%), phenylboronic acid (183 mg, 1.5 mmol), potassium fluoride (174 mg, 3.0 mmol), and 1-chloro-4-nitrobenzene (158 mg, 1.0 mmol). The tube was evacuated and backfilled with argon, and THF (1 mL) was added through a rubber septum. The tube was sealed with a teflon screwcap, and the reaction mixture was stirred at room temperature until the starting aryl chloride had been completely consumed as judged by GC analysis. The reaction mixture was then diluted with ether (30 mL), filtered through celite, and concentrated. The crude material was purified by flash chromatography on silica gel to afford 196 mg (98%) of the title compound.

***Example 40***

Synthesis or 2.6-dimethylbiphenyl

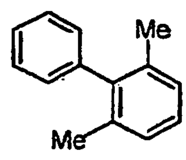

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), phenylboronic acid (183 mg, 1.5 mmol), and potassium fluoride (174 mg, 3.0 mmol). The tube was evacuated and backfilled with argon, and THF (1 mL) and 2-bromo-*m*-xylene (0.144 mL, 1.0 mmol) were added through a rubber septum. The tube was sealed with a teflon screwcap, and the reaction mixture was stirred at 65 ˚C until the starting aryl bromide had been completely consumed as judged by GC analysis. The reaction mixture was then diluted with ether (30 mL), filtered through celite, and concentrated. The crude material was purified by flash chromatography on silica gel to afford 144 mg (79%) of the title compound.

***Example 41***

Synthesis of 2-methoxy-4'-methylbiphenyl

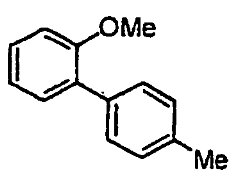

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), 2-methoxyphenylboronic acid (228 mg, 1.5 mmol), and potassium fluoride (174 mg, 3.0 mmol). The tube was evacuated and backfilled with argon, and THF (1 mL) and 4-chlorotoluene (0.144 mL, 1.0 mmol) were added through a rubber septum. The tube was sealed with a teflon screwcap, and the reaction mixture was stirred at 65 ˚C until the starting aryl chloride had been completely consumed as judged by GC analysis. The reaction mixture was then

diluted with ether (30 mL), filtered through celite, and concentrated. The crude material was purified by flash chromatography on silica gel to afford 188 mg (95%) of the title compound.

***Example* 42**

Synthesis of 2-methoxy-2'-acetylbiphenyl

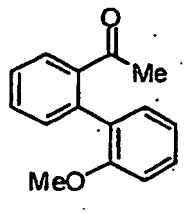

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), 2-methoxyphenylboronic acid (228 mg, 1.5 mmol), and potassium phosphate (425 mg, 2.0 mmol). The tube was evacuated and backfilled with argon, and toluene (3 mL) and 2'-chloroacetophenone (0.13 mL, 1.0 mmol) were added through a rubber septum. The tube was sealed with a teflon screwcap, and the reaction mixture was heated to 65 ˚C with stirring until the starting aryl chloride had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 201 mg (89%) of the title compound.

***Example 43***

Synthesis of 3-(3-acetylphenyl)pyridine

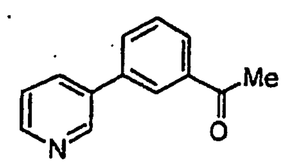

An oven dried resealable Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), 3-acetylphenylboronic acid (246 mg, 1.5 mmol), and potassium fluoride'(173 ing, 3.0 mmol). The tube was evacuated and backfilled with argon, and THF (1 mL) and 3-chloropyridine (0.095 mL, 1.0 mmol) were added through a rubber septum. The tube was sealed with a teflon screwcap, and the reaction mixture was heated to 50 ˚C with stirring until the starting aryl chloride had been completely consumed as judged by GC analysis. The reaction mixture was then diluted with ether (30 mL) and poured into a separatory funnel. The mixture was washed with.water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 181 mg (92%) of the title compound.

***Example 44***

Synthesis of 4-acetylbiphenyl from an aryl chloride utilizing 0.02 mol% Pd

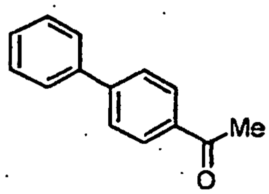

An oven dried resealable Schlenk tube was evacuated and backfilled with argon and charged with phenylboronic acid (228 mg, 1.5 mmol), and potassium phosphate (425 mg, 2.0 mmol). The tube was evacuated and backfilled with argon, and toluene (1.5 mL) and 4-chloroacetophenone (0.13 mL, 1.0 mmol) were added through a rubber septum. In a separate flask, palladium acetate (2.2 mg, 0.01 mmol) and 2-(di-tert-butylphosphino)biphenyl (not according to the invention; 6.0 mg, 0.02 mmol) were dissolved in 5 mL THF under argon. A portion of this solution (100 μL, 0.0002 mmol Pd, 0.02 mol% Pd) was added to the reaction mixture, followed by additional toluene (1.5 mL) through a rubber septum. The tube was sealed with a teflon screwcap, and the reaction mixture was heated to 100 °C with stirring until the starting aryl chloride had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 178 mg (91%) of the title compound.

***Example 45***

Synthesis of 4-acetylbiphenyl from an aryl bromide utilizing 0.000001 mol% Pd

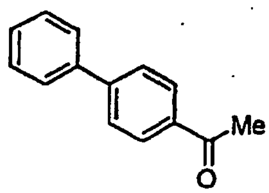

An oven dried resealable Schlenk tube was evacuated and backfilled with argon and charged with phenylboronic acid (228 mg, 1.5 mmol), and potassium phosphate (425 mg, 2.0 mmol), and 4-bromoacetophenone (199 mg, 1.0 mmol). The tube was evacuated and backfilled with argon, and toluene (1.5 mL) was added through a rubber septum. In a separate flask in a nitrogen filled glovebox, palladium acetate (4.5 mg, 0.02 mmol) and 2-(di-tert-butylphosphino)biphenyl (not according to the invention; 12.0 mg, 0.04 mmol) were dissolved in 20 mL THF under argon. A portion of this solution (10 μL, 0.00001 mmol Pd, 0.001 mol% Pd) was added to a second flask contaninig 10 mL THF). A portion of this second solution (10 μL, 0.00000001 mmol Pd, 0.000001 mol% Pd) was added to the reaction mixture, followed by additional toluene (1.5 mL) through a rubber septum. The tube was sealed with a teflon screwcap, and the reaction mixture was heated to 100°C with stirring until the starting aryl bromide had been completely consumed as judged by GC analysis. The reaction mixture was then diluted with ether (30 mL) and poured into a separatory funnel. The mixture was washed with water (20 mL), and the aqueous layer was extracted with ether (20 mL). The combined organic layers were washed with brine (20 mL), dried over anhydrous magnesium sulfate, filtered, and concentrated. The crude material was purified by flash chromatography on silica gel to afford 176 mg (90 %) of the title compound.

***Example 46***

Optimized synthesis of 2-acetylbiphenyl utilizing potassium fluoride

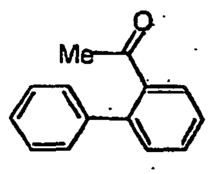

An oven dried Schlenk tube was evacuated and backfilled with argon and charged with palladium acetate (4.5 mg, 0.02 mmol, 1.0 mol%), 2-(di-tert-butylphosphino)biphenyl (not according to the invention; 11.9 mg, 0.040 mmol, 2.0-mol%), phenylboron dihydroxide (366 mg, 3.0 mmol), and potassium fluoride (349 mg, 6.0 mmol). The tube was evacuated and backfilled with argon, and THF (2 mL) and 2-chloroacetophenone (0.26 mL, 2.0 mmol) were added through a rubber septum. The reaction mixture was stirred at room temperature until the starting aryl chloride had been completely consumed as judged by GC analysis. The reaction mixture was then diluted with ethyl acetate (30 mL) and poured into a separatory funnel. The mixture was washed with 2.0M NaOH (20 mL). The organic layer was washed with brine (20 mL), dried over anhydrous sodium sulfate, filtered, and concentrated. The crude material was purified by flash chromatography on silica gel to afford 369 mg (94%) of the title compound.

***Example 47***

Optimized synthesis of 2-formyl-4'-diphenylketiminebiphenyl utilizing potassium fluoride

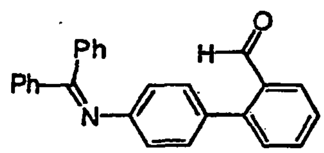

An oven dried Schlenk tube was evacuated and backfilled with argon and charged with palladium acetate (4.5 mg, 0.02 mmol, 1.0 mol%), 2-(di-tert-butylphosphino)biphenyl (not according to the invention; 11.9 mg, 0.040 mmol, 2.0 mol%), 4-(diphenylketimine)phenyl bromide (672 mg, 2.0 mmol), 2-formylphenylboron dihydroxide (450 mg, 3.0 mmol), and potassium fluoride (349 mg, 6.0 mmol). The tube was evacuated and backfilled with argon, and THF (2 mL) was added through a rubber septum. The reaction mixture was stirred at room temperature until the starting aryl bromide had been completely consumed as judged by GC analysis. The reaction mixture was then diluted with ethyl acetate (30 mL) and poured into a separatory funnel. The mixture was washed with 2.0M NaOH (20 mL). The organic layer was washed with brine (20 mL), dried over anhydrous sodium sulfate, filtered, and concentrated. The crude material was purified by flash chromatography on silica gel to afford 647 mg (90%) of the title compound.

***Example 48***

Synthesis of 3-acetyl-3'-5'-dimethoxybiphenyl

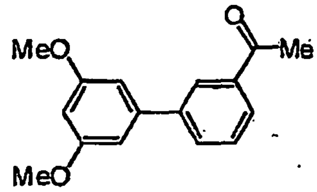

An oven dried. Schlenk tube was evacuated and backfilled with argon and charged with palladium acetate (2.2 mg, 0.01 mmol, 1.0 mol%), 2-(di-tert-butylphosphino)biphenyl (not according to the invention; 60 mg, 0.020 mmol, 2.0 mol%), 3,5-dimethoxyphenyl chloride (173 mg, 1.0 mmol), 3-acetylphenylboron dihydroxide (246 mg, 1.5 mmol), and potassium fluoride (174 mg, 3.0 mmol). The tube was evacuated and backfilled with argon, and THF (1 mL) was added

through a rubber septum. The reaction mixture was stirred at room temperature until the starting aryl chloride had been completely consumed as judged by GC analysis. The reaction mixture was then diluted with ethyl acetate (30 mL) and poured into a separatory funnel. The mixture was washed with 2.0 M NaOH (20 mL). The organic layer was washed with brine (20 mL); dried over anhydrous magnesium sulfate, filtered, and concentrated. The crude material was purified by flash chromatography on silica gel to afford 232 mg (91%) of the title compound.

***Example 49***

Synthesis of 2-phenylthiophene

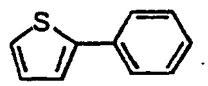

An oven dried Schlenk tube was evacuated and backfilled with argon and charged with palladium acetate (2.2 mg, 0.01 mmol, 1.0 mol%), 2-(di-tert-butylphosphino)biphenyl (not according to the invention; 6.0 mg, 0.020 mmol, 2.0 mol%), phenylboron dihydroxide (183 mg, 1.5 mmol), and potassium fluoride (174 mg, 3.0 mmol). The tube was evacuated and backfilled with argon, and THF (1 mL) and 2-bromothiophene (0.097 mL, 1.0 mmol) were added through a rubber septum. The reaction mixture was stirred at room temperature until the starting aryl bromide had been completely consumed as judged by GC analysis. The reaction mixture was then diluted with ethyl acetate (30 mL) and poured into a separatory funnel. The mixture was washed with 2.0M NaOH (20 mL). The organic layer was washed with brine (20 mL), dried over anhydrous magnesium sulfate, filtered, and concentrated. The crude material was purified by flash chromatography on silica gel to afford 159 mg (99%) of the title compound.

***Example 50***

Room temperature synthesis of 4-methylbiphenyl utilizing the ligand 2,6-dimethoxyphenyl-di-*t*-butylphosphine

An oven dried resealable Schlenk tube was evacuated and backfilled with argon and charged with palladium acetate (4.4 mg, 0.01. mmol, 1 mol%), 2,6-dimethoxyphenyl-di-*t*-butylphosphine (not according to the invention; 4.2 mg, 0.015 mmol, 1.5 mol%), phenylboron dihydroxide (183 mg, 1.5 mmol), and cesium fluoride (456 mg, 3.0 mmol). The tube was evacuated and backfilled with argon, and THF (3 mL) and 4-chlorotoluene (0.12 mL, 1.0 mmol) were added through a rubber septum. The septum was removed, the tube was sealed with a teflon screw cap and the mixture was stirred at room temperature until the starting aryl chloride had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel to give 164 mg (98%) of a glassy solid.

***Example 51***

Room temperature synthesis of 4-methylbiphenyl utilizing the ligand 2,4,6-trimethoxyphenyl-di-*t*-butylphosphine

An oven dried resealable Schlenk tube was evacuated and backfilled with argon and charged with palladium acetate (4.4 mg, 0.01 mmol, 1 mol%), 2,4,6-trimethoxyphenyl-di-*t*-butylphosphine (not according to the invention; 4.7 mg, 0.015 mmol, 1.5 mol%), phenylboron dihydroxide (183 mg, 1.5 mmol), and cesium fluoride (456 mg, 3.0 mmol). The tube was evacuated and backfilled with argon, and THF (3 mL) and 4-chlorotoluene (0.12 mL, 1.0 mmol) were added through a rubber septum. The septum was removed, the tube was sealed with a teflon screw cap and the mixture was stirred at room temperature until the starting aryl chloride had been completely consumed as judged by GC analysis. The reaction mixture was then diluted with ether (20 mL) and poured into a separatory funnel. The mixture was washed with 1M NaOH (20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel to give 165 mg (98%) of a glassy solid.

***Example 52***

Synthesis of 4-(trifluoromethyl)phenylboronic acid

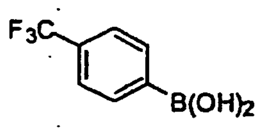

An oven dried Schlenk tube was charged with magnesium turnings (766 mg, 31.5 mmol), evacuated, and backfilled with argon. To the reaction vessel was added 10 mL of ether followed by 4-(trifluoromethyl)phenyl bromide (4.20 mL, 30.0 mmol). The reaction mixture was stirred without external heating for 1 hour, during which time an exotherm occurred and then subsided. The solution was diluted with ether (10 mL) and transferred via cannula to a flask containing triisopropylborate (13.8 mL, 60.0 mmol) in 1:1 THF/ether (20 mL) at -78 ˚C. The resulting reaction mixture was kept at -78 ˚C for 15 minutes and then was allowed to warm to room temperature. After stirring at room temperature for 15 minutes, the reaction mixture was poured onto 2.0 M HCl (60 mL). The mixture was transferred to a separatory funnel, extracted with ethyl acetate (60 mL), washed with water (60 mL), and brine (60 mL). The organic solution was dried over anhydrous sodium sulfate and concentrated in vacuo. The crude material was dissolved in 2:1 hexane/ethyl acetate (90 mL) and activated charcoal was added. The mixture was filtered and the product crystallized upon cooling. The crystals were collected by filtration to afford 1.98 g (35%) of pale yellow needles (not according to the invention).

***Example 53***

Synthesis of 2-bromo-4'-(trifluoromethyl)biphenyl

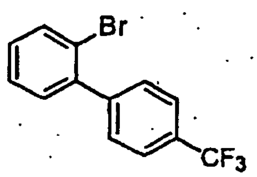

An oven dried Schlenk tube was evacuated and backfilled with argon and charged with tetrakis(triphenylphosphine)palladium (289 mg, 0.25 mmol, 5.0 mol%), 2-bromoiodobenzene (0.83 mL, 6.50 mmol), 4-(trifluoromethyl)phenylboronic acid (950 mg, 5.0 mmol), and sodium carbonate (2.86 g, 27.0 mmol). The tube was evacuated and backfilled with argon. To the tube was added (degassed) dimethoxyethane (45 mL), ethanol (2 mL), and water (15 mL) through a rubber septum. The reaction mixture was heated to 85 ˚C with stirring for 32 hours. The reaction mixture was then diluted with 2:1 hexane/ethyl acetate (100 mL) and poured into a separatory funnel. The mixture was washed with water (80 mL), and brine (80 mL). The organic layer was dried over anhydrous sodium sulfate, decanted, and concentrated in vacuo. The crude material was purified by flash chromatography on silica gel to afford 1.01 g (67%) of the product (not according to the invention).

***Example 54***

Synthesis of 2-(di-*t*-butylphosphino)-4'-(trifluoromethyl)biphenyl

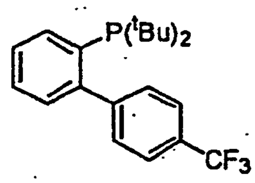

An oven dried Schlenk tube was evacuated and backfilled with argon and charged with magnesium turnings (90 mg, 3.69 mmol), 2-bromo-4'-(trifluoromethyl)biphenyl (1.01 g, 3.35 mmol), and a crystal of iodine. The tube was purged with argon for 5 minutes, then THF (6 mL) was added through a rubber septum and the reaction mixture was heated to reflux for 1 hour. The reaction mixture was cooled to room temperature and cuprous chloride (365 mg, 3.69 mmol) and chloro-di-*t*-butylphosphine (0:765 mL, 4.03 mmol) were added. Heating was resumed for 14 hours. The reaction mixture was then cooled to room temperature and diluted with ether (40 mL). The suspension was filtered to isolate the solid. The solid was partitioned between ethyl acetate (60 mL) and 38% ammonium hydroxide (75 mL). The aqueous layer was extracted with ethyl acetate (60 mL). The combined organic layers were washed with brine (50 mL), dried over anhydrous sodium sulfate, decanted, and concentrated in vacuo. The product was crystallized from MeOH (10 mL) to afford 131 mg (11%) of pale yellow needles. A second crop was isolated by concentrating the mother liquor and recrystallizing the solid from MeOH (20 mL) and water (2 mL) to afford 260 mg (21 %) of the product (not according to the invention).

***Example 55***

Synthesis of 2-(di-1-adamantylphosphino)biphenyl

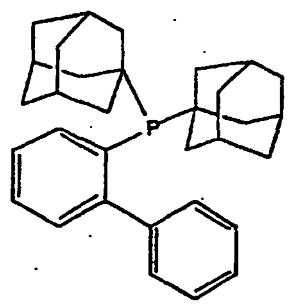

An oven-dried, round-bottom flask was charged with magnesium turnings (15.3 g, 0.63 mol) and 1-bromoadamantane (9.0 g, 0.041 mol). The flask was evacuated and backfilled with argon two times. To the reaction vessel 45 mL ether was added and the mixture was gently refluxed for 15 hours, without mechanical stirring. The resulting solution of Grignard reagent was taken up in a syringe, and added very slowly dropwise to a separate flame-dried, two-necked, round-bottom flask equipped with a reflux condenser which had been charged with $PCl_3$ (0.9 mL, 10 mmol) and 15. mL ether which had been coooled to -40 °C. During the addition the temperature was monitored and kept below -25 °C. The resulting mixture was stirred for 30 minutes at -45 °C, then the cooling bath was removed and the reaction mixture was allowed to warm slowly to room temperature. After stirring for an additional 30 minutes at room temperature, the reaction vessel was placed into a heated oilbath (37 °C) and was gently refluxed for 22 hours. The mixture was cooled to room temperature, and the solution was filtered through a cannula filter. The solvent as well as some of the adamantane byproduct was removed in vacuo, without exposing the product to air to afford crude di-1-adamantylchlorophosphine.

An oven dried Schlenk tube was charged with magnesium turnings (240 mg, 9.89 mmol), 2-bromo-biphenyl (1.55 mL, 7.5 mmol). The tube was evacuated and backfilled with argon two times. To the above mixture THF (15 mL) was added through a rubber septum and the reaction mixture was heated to a mild reflux for 3 hours. The reaction mixture was then temporarily cooled to room temperature for the addition of cuprous chloride (930 mg, 9.45 mmol) followed by a solution of the di-1-adamantylchlorophosphine in 5 mL THF. Heating was resumed for an addtional 3 hours. The reaction mixture was cooled to room temperature, and ether (50 mL) and pentane (50 mL) were added. The resulting suspension was stirred for 10 minutes, during which time a heavy dark-brown precipitate formed. The suspension was filtered and the solid was collected on a fritted funnel. The solid was partitioned between ethyl acetate-ether (100

mL 1:1) and 3 8% ammonium hydroxide-water (100 mL 1:1). The mixture was vigorously shaken several times over 30 minutes. The aqueos layer washed twice with ether-ethyl acetate (1:1, 100 mL). The combined organic layers were washed with brine (2x 50 mL), dried over anhydrous magnesium sulfate, decanted, and concentrated in vacuo. The product was crystallized from toluene/methanol to afford 450 mg (5.8%) product as a white solid (not according to the invention).

***Example 56***

Synthesis of 2-(di-*t*-butylphosphino)-2'-(isopropyl)biphenyl

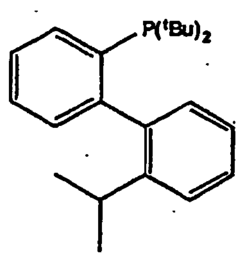

A flame-dried Schlenk tube was evacuated and backfilled with argon two times, and was charged with 2-(bromo)-2'-(isopropyl)biphenyl (1.5 g, 5.45 mmol) and ether (15 mL). The reaction mixture was cooled to -78 ˚C, and *t*-BuLi (6.7 mL, 1.7 M in penate) was added dropwize *via* a syringe, through a rubber septum. After the addition was complete, the reaction mixture was stirred for an additional 15 minutes at -78 ˚C. The cooling bath was removed, and $t$-$Bu_2PCl$ was added dropwise. After reaching room temperature, the reaction vessel was placed into a heated oil bath (37 ˚C), and the reaction mixture was refluxed for 48 hours. The mixture was cooled to room temperature, a saturated solution of aqueous ammonium chloride (10 mL) was added, and the resulting mixture was partitioned between ether (100 mL) and water (50 mL). The organic layer was dried over a 1:1 mixture of anhydrous magnesium sulfate and sodium sulfate, decanted and concentrated in vacuo. The product was crystallized from MeOH to afford 601 mg (30 %) of white needles (not according to the invention).

***Example 57***

Synthesis of di-*t*butyl-(*o*-cyclohexyl)phenyl phosphine (**3**)

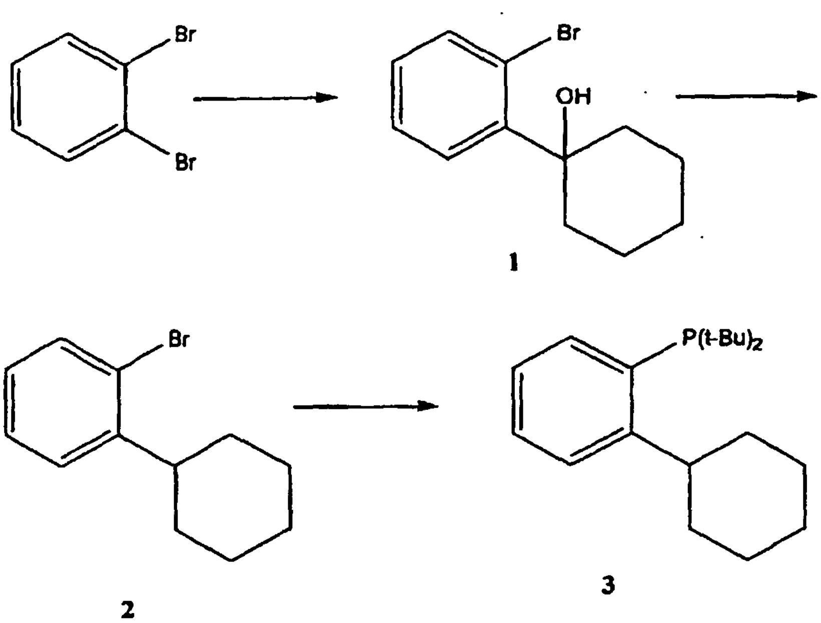

1

2

3

An oven-dried Schlenk flask was allowed to cool to room temperature under an argon purge, and was charged with 1,2-dibromobenzene (1.2 mL, 10.0 mmol), ether (20 mL), and THF (20 mL). The mixture was cooled to -119 ˚C with stirring using an ethanol/$N_2$ cold bath. *n*-butyllithium in hexanes (5.8 mL, 1.6 M, 9.3 mmol) was added slowly dropwise. The mixture was stirred at -119 ˚C for 45 min, then cyclohexanone (0.98 mL, 9.5 mmol) was added to the mixture. The mixture was stirred at -78 ˚C for 30 min, then warmed to room temperature and stirred for 17 h. The mixture was quenched with saturated aqueous ammonium chloride (20 mL), diluted with ether (50 mL), and poured into a separatory funnel. The layers were separated and the aqueous phase was extracted with ether (1x20 mL). The organic layers were combined and washed with brine (20 mL), dried over anhydrous magnesium sulfate, filtered, and concentrated *in vacuo.* The crude material was purified by flash chromatography on silica gel to afford 1.91g of 1 which was judged to be ~86% pure by GC analysis. This material was used without further purification.

A round bottomed flask was purged with argon and charged with alcohol **1** (1.78 g, 7.0 mmol), dichloromethane (28 mL), triethylsilane (1.5 mL, 9.1 mmol), and trifluoroacetic acid (1.1 mL, 14.7 mmol). The mixture was stirred at room temperature for 1.5 h, then was quenched with solid potassium carbonate (ca 2g). The mixture was diluted with ether (50 mL) and transferred to a separatory funnel. The mixture was washed with saturated aqueous $NaHCO_3$ (50 mL), and the organic phase was dried over anhydrous magnesium sulfate, filtered, and concentrated *in vacuo* to afford a mixture of **2** and 1-(2-bromophenyl)cyclohexene. The crude material was placed into a round bottomed flask, and the flask was purged with argon. THF (2 mL) was added, and the mixture was cooled to 0 ˚C with stirring. A solution of $BH_3$ in THF (7 mL, 1M, 7.0 mmol) was added dropwise to the mixture. The mixture was stirred at 0 ˚C for 1.5 h, then warmed to room temperature and stirred for 19 h. Acetic acid (4 mL) was added and the mixture was stirred at room temperature for 6h. The mixture was then diluted with ether (50 mL) and poured into a separatory funnel. The mixture was washed with 1M NaOH (50 mL), the layers were separated, and the aqueous phase was extracted with ether (50 mL). The combined organic phases were washed with brine (50 mL), dried over anhydrous magnesium sulfate, filtered, and concentrated *in vacuo.* The crude material was purified by flash chromatography on silica gel to afford 555 mg of **2** which was judged to be 93% pure by GC analysis. This material was used without further purification.

An oven-dried Schlenk tube was cooled to room temperature under an argon purge, and was charged with magnesium turnings (27 mg, 1.1 mmol), THF (1 mL), and 1,2-dibromoethane (8 μL). The mixture was stirred at room temperature for 10 min, then **2** (239 mg, 1.0 mmol) was added in one portion. The mixture was stirred at rt for 20 min, then heated to 60 ˚C for 15 min. The mixture was cooled to room temperature, the septum was removed from the flask, and copper (I) chloride (104 mg, 1.05 mmol) was added. The tube was capped with the septum and purged with argon for 1 min. The tube was charged with di-*t*-butylchlorophosphine (.23 mL, 1.2 mmol) and additional THF (1 mL). The mixture was heated to 60 ˚C with stirring for 26 h. The mixture was cooled to room temperature and filtered, and the solids were washed with ether/hexanes (50 mL, 1/1 v/v). The organic solution was poured into a separatory funnel and washed with ammonium hydroxide solution (3x50 mL), and brine (50 mL). The organic phase was then dried over anhydrous sodium sulfate, filtered, and concentrated. The crude material was purified by flash chromatography on silica gel to afford **3** as a white solid (141 mg), which was judged to be 92% pure by GC analysis. This material was recrystallized from hot methanol to afford 101 mg (~3% overall from 1,2-dibromobenzene) of 3 as a white, crystalline solid.

***Example 58***

Preparation of *o*-di-*t*-butylphosphino-*o*-terphenyl (**3**)

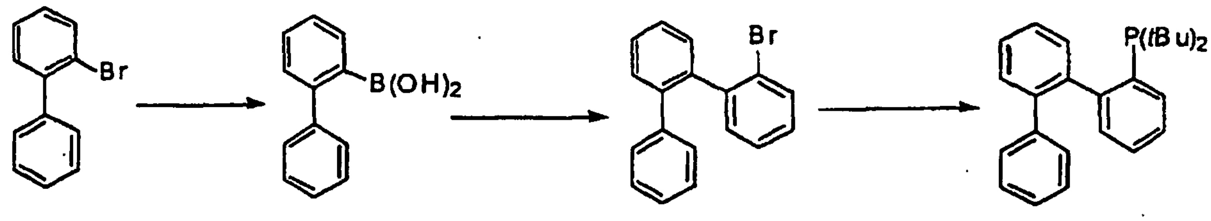

1 2 3

An oven-dried Schlenk tube was cooled to room temperature under an argon purge, and was charged with magnesium turnings (243 mg, 11.0 mmol), ether (7 mL), and 1,2-dibromoethane (38 μL). The mixture was stirred at room temperature until the evolution of gases ceased, then a solution of 2-bromobiphenyl (1.7 mL, 10.0 mmol) in 5 mL ether was added dropwise. The mixture was stirred at room temperature for 1.75 h. The solution was then transferred to a separate flask containing a solution of triisopropyl borate (4.6 mL, 20.0 mmol) in THF (20 mL) which had been cooled to 0 ˚C. The mixture was stirred at 0 ˚C for 15 min, then warmed to room temperature and stirred for 21 h. The reaction was quenched with 1M HCl (40 mL) and stirred at room temperature for 10 min. The solution was basisified to pH 14 with 6M NaOH, then extracted with ether (1x10 mL). The organic phase was discarded and the aqueous phase was acidified to pH 2 with 6M HCl. The aqueous phase was extracted with ether (3x50 mL), and the combined organic layers were dried over anhydrous sodium sulfate, filtered, and concentrated *in vacuo.* The crude material was recrystallized from ether/pentane at -20 ˚C to afford 1.0g (51%) of **1** as a white, crystalline solid.

An oven-dried Schlenk flask was cooled to room temperature under an argon purge, and was charged with tetrakis(triphenylphosphine)palladium (289 mg, 0.25 mmol, 5 mol%) sodium carbonate (2.86 g, 27 mmol), and **1** (1.0 g, 5.0 mmol). The flask was purged with argon and DME (50 mL), ethanol (2 mL), water (15 mL), and 2-bromoiodobenzene (0.83 mL, 6.05 mmol) were added through a rubber septem. The mixture was heated to 85 ˚C with stirring for 3 days. The mixture was cooled to room temperature, diluted with ether (100 mL), and poured into a separatory funnel. The layers were separated and the organic phase was washed with 1M NaOH (2x50 mL), washed with brine, dried over anhydrous magnesium sulfate, filtered, and concentrated *in vacuo.* The crude material was purified by flash chromatography on silica gel to afford 1.23 g (79%) of **2** as a colorless oil.

An oven-dried Schlenk tube was cooled to room temperature under an argon purge, and was charged with magnesium turnings (54 mg, 2.2 mmol), THF (2 mL), and 1,2-dibromoethane (9 μL). The mixture was stirred at room temperature for 15 min, then a solution of **2** (618 mg, 2.0 mmol) in 1 mL THF was added dropwise. The mixture was stirred at rt for 1h, then the septum was removed from the flask, and copper (I) chloride (283 mg, 2.1 mmol) was added. The tube was capped with the septum and purged with argon for 1 min. The tube was charged with di-*t*-butylchlorophosphine (.46 mL, 2.4 mmol) and additional THF (1 mL). The mixture was heated to 60 ˚C with stirring for 26 h. The mixture was cooled to room temperature and filtered, and the solids were washed with ether/hexanes (50 mL, 1/1 v/v). The organic solution was poured into a separatory funnel and washed with ammonium hydroxide solution (3x50 mL), and brine (50 mL). The organic phase was then dried over anhydrous sodium sulfate, filtered, and concentrated. The crude material was recrystallized from hot methanol to afford 191 mg (26%) of 3 as a white, crystalline solid.

***Example 59***

Room-temperature catalytic aminations of aryl chlorides

**General procedure**. An oven-dried resealabale Schlenk flask was evacuated and backfilled with argon. The flask was charged with $Pd(OAc)_2$ (2.2 mg, 0.01 mmol, 1 mol %), **3** (not according to the invention; 6.0 mg, 0.02 mmol, 2 mol %), and NaO*t*Bu (135 mg, 1.4 mmol). The flask was evacuated and backfilled with argon, then capped with a rubber septum. Toluene (0.5 mL), the aryl chloride (1.0 mmol) (aryl chlorides which were solids at room temperature were added as solids following the addition of NaO*t*Bu); the amine (1.2 mmol), and additional toluene (0.5 mL) were added through the septum. The septum was replaced with a teflon screwcap, the flask was sealed, and the mixture was

stirred at room temperature until the starting aryl chloride had been completely consumed as judged by GC analysis. During the course of the reaction the mixture was observed to form a gel (at around 50% conversion) and then liquefy again as the reaction proceed to completion. Following the complete consumption of the aryl chloride starting material, the mixture was diluted with ether (20 mL), filtered through celite, and concentrated *in vacuo.* The crude material was purified by flash chromatography on silica gel.

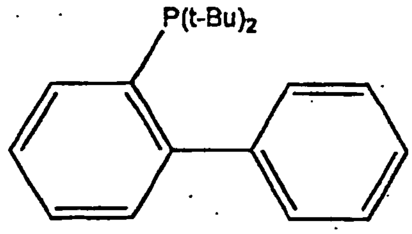

**3**

***N*-Methyl-*N*-phenyl-*p*-toluidine** (Figure 3, entry 1) The general procedure was modified such that when the aryl halide had been completely consumed, the mixture was diluted with ether (50 mL) and transferred to a separatory funnel. The mixture was washed with aqueous HCl (1 M, 2x20 mL), washed with brine (20 mL), dried over anhydrous magnesium sulfate, filtered and concentrated *in vacuo.* The crude product was purified by flash chromatography on silica gel to give 197 mg (100%) of the title compound as a colorless oil. $^1$H NMR (250 MHz, $CDCl_3$) δ 7.35-7.19 (m, 2H), 7.15-7.09 (m, 2H), 7.02-6.82 (m, 5H), 3.28 (s, 3H), 2.32 (s, 3H).

***N*-(4- Methylphenyl) morpholine** (Figure 3, entry 2) The general procedure gave 166 mg (94%) of the title compound as a white solid, mp 47-48 ˚C (lit mp=48 ˚C). $^1$H NMR (300 MHz, $CDCl_3$) δ 7.09 (d, 2H, *J*=8.5 Hz), 6.84 (d, 2H, *J*=8.2 Hz), 3.86 (t, 4H, *J*=4.7 Hz), 3.11 (t, 4H, *J*=4.6 Hz), 2.28 (s, 3H).

***N*,*N*-Dibutyl-*p*-toluidine** (Figure 3, entry 3) The general procedure was modified such that 2 mol % $Pd(OAc)_2$ and 3 mol % 3 were employed. When the aryl halide had been completely consumed, 30% $H_2O_2$ (1 mL) was added to the reaction mixture in order to oxidize the phosphine ligand. The mixture was stirred at room temperature for 5 min, then diluted with ether (20 mL) and transferred to a separatory funnel. The layers were separated and the organic layer was washed with water (20 mL), and saturated aqueous $Fe(SO)_4$ (20 mL). The combined aqueous layers were extracted with ether, the organic extracts were combined and the aqueous layer was discarded. The combined organic extracts were extracted with aqueous HCl (1 M, 4x50 mL), the organic layer was discarded, and the aqueous extracts were combined and basicified to pH 14. The aqueous layer was extracted with ether (4x50 mL), and the combined organic extracts were washed with brine, dried over anhydrous magnesium sulfate, filtered through a plug of silica gel, and concentrated *in vacuo* to afford 172 mg (79%) of the title compound as a colorless oil. $^1$H NMR (300 MHz, $CDCl_3$) δ 7.00 (d, 2H, *J*=8.6 Hz), 6.57 (d, 2H, *J*=8.6 Hz), 3.22 (t, 4H, *J*=7.7 Hz), 2.23 (s, 3H), 1.56-1.48 (m, 4H), 1.37-1.29 (m, 4H), 0.94 (t, 6H, *J*=7.4 Hz).

***N*-(2,5-Xylyl)pyrrolidine** (Figure 3, entry 4). The general procedure gave 169 mg (97%) of the title compound as a colorless oil which was determined to contain <1 % of **3** as judged by $^1$H NMR and GC analysis. $^1$H NMR (250 MHz, $CDCl_3$) δ 6.99 (d, 1H, *J*=7.5 Hz), 6.69-6.63 (m, 2H), 3.25-3.10 (m, 4H), 2.29 (s, 3H), 2.28 (s, 3H), 1.95-1.85 (m, 4H).

***N*-(2,5- Xylyl) benzylamine** (Figure 3, entry 5) The general procedure was modified such that 2 mol % $Pd(OAc)_2$ and 4 mol % **3** were employed. When the aryl halide had been completely consumed, 30% $H_2O_2$ (2 mL) and THF (1 mL) was added to the reaction mixture. The mixture was stirred at room temperature for 5 min, then diluted with ether (20 mL) and transferred to a separatory funnel. The layers were separated and the organic layer was washed with water (20 mL), and saturated aqueous $Fe(SO)_4$ (20 mL). The combined aqueous layers were extracted with ether, the organic extracts were combined, dried over anhydrous magnesium sulfate, filtered, and concentrated *in vacuo.* The crude material was then purified by flash chromatography on silica gel to afford 196 mg (99%) of the title compound as a colorless oil. $^1$H NMR (250 MHz, $CDCl_3$) δ 7.41-7.26 (m, 5H), 6.96 (d, 1H, *J*=7.3 Hz), 6.51-6.46 (m, 2H), 4.36 (s, 2H), 3.790 (s, br, 1H), 2.26 (s, 3H), 2.12 (s, 3H); $^{13}$C NMR (75 MHz, $CDCl_3$) δ 145.9, 139.5, 129.9, 128.6, 127.6, 127.2, 118.9, 117.8, 110.8, 48.3, 21.5, 17.1; IR (neat, $cm^{-1}$) 3438, 1582, 1453, 795. Anal Calcd for $C_{15}H_{17}N$: C, 85.26; H, 8.11. Found: C, 85.14; H, 8.12.

***N*-(4- Methoxyphenyl) morpholine** (Figure 3, entry 6) The general procedure was modified such that 2 mol % $Pd(OAc)_2$ and 4 mol % **3** were employed. The procedure afforded 173 mg (90%) of the title compound as a white solid, mp 73-74 ˚C (lit mp 71 ˚C). $^1$H NMR (300 MHz, $CDCl_3$) δ 6.91-6.81 (m, 4H), 3.88-3.85 (m, 4H), 3.78 (s, 3H), 3.08-3.05 (m, 4H).

***N*-(4- Cyanophenyl) morpholine** (Figure 3, entry 7) The general procedure gave 158 mg (84%) of the title compound as a white solid, mp 85 ˚C (lit mp 75-76.5 ˚C). $^1$H NMR (300 MHz, $CDCl_3$) δ 7.52 (d, 2H, *J*=9.1 Hz), 6.86 (d, 2H, *J*=9.1

Hz), 3.87-3.84 (m, 4H), 3.29-3.26 (m, 4H).

***N*-(2- Methoxyphenyl) benzylamine** (Figure 3, entry 8) The general procedure gave 211 mg (99%) of the title compound as a colorless oil. $^1H$ NMR (300 MHz, $CDCl_3$) $\delta$ 7.40-7.20 (m, 5H), 6.96-6.76 (m, 2H), 6.70-6.57 (m, 2H), 4.66 (s, br, 1H), 4.35 (s, 2H), 3.84 (s, 3H); $^{13}C$ NMR (75 MHz, $CDCl_3$) $\delta$ 146.7, 139.5, 138.0, 128.5, 127.4, 127.0, 121.2, 116.5, 110.0, 109.3, 55.3, 47.9, IR (neat, $cm^{-1}$) 3425, 2937, 1511, 1027, 735. Anal Calcd for $C_{14}H_{15}NO$: C, 78.84; H, 7.09. Found; C, 79.54; H, 6.79.

***N*-Methyl-*N*-(3,5- dimethoxyphenyl) aniline** (Figure 3, entry 9) The general procedure gave 238 mg (98%) of the title compound as a colorless oil. $^1H$ NMR (300 MHz, $CDCl_3$) $\delta$ 7.32-7.26 (m, 2H), 7.10-7.01 (m, 3H), 6.12-6.06 (m, 3H), 3.73 (s, 6H), 3.29 (s, 3H); $^{13}C$ NMR (75 MHz, $CDCl_3$) $\delta$ 161.3, 150.9, 148.6, 129.2, 122.3, 97.5, 92.4, 55.2, 40.3; IR (neat, $cm^{-1}$) 2939, 1586, 1150, 1065, 700. Anal Calcd for $C_{15}H_{17}NO_2$: C, 74.05; H, 7.04. Found: C, 73.90; H, 7.01.

***Example 60***

Catalytic amination of aryl chlorides at 80-110 °C

**General Procedure.** An oven-dried resealable Schlenk flask was evacuated and backfilled with argon. The flask was charged with palladium acetate (0.5 mol %), **3** (not according to the invention; 1.0 mol %), NaO*t*Bu (1.4 equiv), and evacuated and backfilled with argon. The flask was capped with a rubber septum and toluene (2 mL/mmol halide), the aryl halide (1.0 equiv), and the amine (1.2 equiv) were added through the septum. The septum was replaced with a teflon screwcap, the flask was sealed, and the mixture was heated to 80 °C with stirring until the starting aryl halide had been completely consumed as judged by GC analysis. The mixture was cooled to rt, diluted with ether (30 mL), filtered through celite, and concentrated *in vacuo.* The crude product was purified by flash chromatography on silica gel.

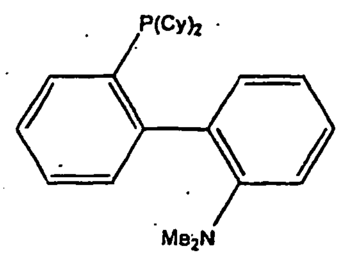
2

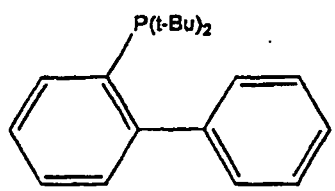
3

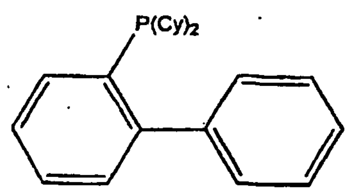
4

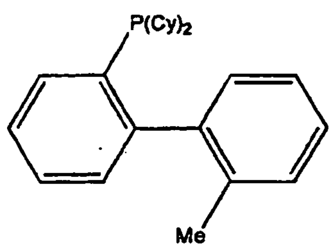
5

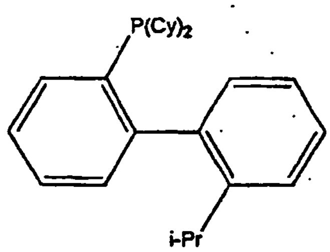
6

Compounds 2 to 6 are not ligands according to the invention.

***N*-(4- Methylphenyl) hexylamine** (Figure 4, entry 1). The general procedure was conducted on a 2 mmol scale using 1.5 equiv amine. Following completion of the reaction, the mixture was cooled to room-temperature, diluted with ether (40 mL), and extracted with 1 M aqueous HCl (3x50 mL). The organic phase was discarded, and the aqueous layer was basicified to pH 14 with 6 M aqueous NaOH. The aqueous phase was extracted with ether (3 x 50 mL), and the ether layers were combined, dried over anhydrous magnesium sulfate, filtered, and concentrated *in vacuo* to afford 318 mg (83%) of a white solid, mp 37 °C (lit mp 37.1-37.3 °C). This material contained 1% **3** as judged by GC and $^1H$ NMR analysis: $^1H$ NMR ($CDCl_3$, 300 MHz) $\delta$ 6.97 (d, 2H, *J* = 8.89 Hz), 6.54 (d, 2H, *J* = 8.7 Hz), 3.45 (s, br, 1H), 3.07 (t, 2H, *J*= 7.5 Hz), 1.64-126 (m, 8H), 0.89 (m, 3H).

***N*-(4-methylphenylmorpholine)** (Figure 4, entry 2) The general procedure conducted on a 2 mmol scale gave 316 mg (95%) of a white solid. See above for NMR data.

**Di-*p*-tolylamine** (Figure 4, entry 3) The general procedure using $Pd_2(dba)_3$ gave 185 mg (93%) of a white

solid: mp 78-79°C (lit. 79 °C); $^1$H NMR (300 MHz, $CDCl_3$) δ 7.10 (d, *J* = 8.2 Hz, 4H), 6.98 (d, *J* = 8.4 Hz, 4H), 5.53 (s, 1H), 2.33 (s, 6H); $^{13}$C NMR (75 MHz, $CDCl_3$) δ 141.3, 130.3, 130.0, 118.1, 20.8; IR (neat, $cm^{-1}$) 3419, 3026, 2914, 2860, 1609, 1589, 1515, 1320, 1239, 1227, 1177, 1123, 1108, 1381, 1040, 880, 805, 772, 704. Anal. Calcd for $C_{14}H_{11}N$: C, 85.24; H, 7.66. Found: C, 85.29; H, 8.02..

***N*-(*p*-Tolyl) diphenylamine** (Figure 4; entry 4) The general procedure gave 242 mg (93%) of a pale yellow solid: mp 66-67.5 °C (lit. 68.8 °C); $^1$H NMR (300 MHz, $CDCl_3$) δ 7.23 (d, 7.3 Hz, 4H), 7.11-6.96 (m, 10H), 2.34 (s, 3H); $^{13}$C NMR (75 MHz, $CDCl_3$) δ 148.2, 145.4, 132.9, 130.1, 129.3, 125.1, 123.8, 122.4, 21.0; IR (neat, $cm^{-1}$) 3085, 3058, 3033, 3004, 2975, 2919, 2860, 1594, 1582, 1509, 1490, 1449, 1323, 1293, 1274, 1171, 1150, 1111, 1075, 1028, 917, 899, 888, 814, 749, 712, 695. Anal. Calcd for $C_{19}H_{17}N$: C, 87.99; H, 6.61. Found: C, 88.01; H, 6.84.

***N*-Benzyl-*p*-toluidine** (Figure 4, entry 5). The general procedure was conducted on a 2 mmol scale using 1.5 equiv amine. The reaction was subjected to the same workup described above for *N*-(4-methylphenyl)heacylamine to afford 344 mg (87%) of a pale yellow oil. This material contained 1% **3** as judged by GC and $^1$H NMR analysis. See above for NMR data.

***N,N*-Dibutyl-*p*-toluidine** (Figure 4, entry 6). The general procedure using $Pd_2(dba)_3$ and **5** (not according to the invention) gave 193 mg (88%) of a pale yellow oil. See above for NMR data.

***N,N*-Dibutyl-*p*-toluidine** (Figure 4, entry 6). The general procedure using $Pd_2(dba)_3$ and **6** (not according to the invention) gave 199 mg (91%) of a pale yellow oil. See above for NMR data.

***N*-Ethyl-*N*-phenyl-*p*-toluidine** (Figure 4, entry 7). The general procedure gave 196 mg (93%) of a pale yellow oil: $^1$H NMR (250 MHz, $CDCl_3$) δ 7.25-7.18 (m, 4H), 7.11 (d, 2H, *J* = 8.3 Hz), 6.99-6.79 (m; 3H), 3.74 (q, 2H, *J* = 7.1 Hz), 2.32 (s, 3H), 120 (t, 3H, *J* = 7.0 Hz); $^{13}$C NMR (75 MHz, $CDCl_3$) δ 148.2, 145.0, 132.1, 130.0, 129.0, 123.4, 119.3, 118.2, 46.4, 20.8, 12.6; IR (neat, $cm^{-1}$) 2974, 1599, 1498, 1259, 810. Anal. Calcd for $C_{15}H_{17}N$: C, 85.26; H, 8.11: Found: C, 85.25; H, 8.15.

***N*-(4- Methylphenyl) piperidine.** (Figure 4, entry 8). The general procedure gave 149 mg (85%) of a colorless oil: $^1$H NMR ($CDCl_3$, 300 MHz) δ 7.05 (d, 2H, *J* = 8.4 Hz), 6.85 (d, 2H, *J* = 8.4 Hz), 3.09 (t, 4H, *J* = 5.4 Hz), 2.26 (s, 3H), 1.73-1.50 (m, 6H).

***N*-Methyl-*N*-phenyl- 2,5- xylidene** (Figure 4, entry 9). The general procedure conducted on a 2 mmol scale gave 374 mg (89%) of a colorless oil: $^1$H NMR ($CDCl_3$, 300 MHz) δ 7.19-7.14 (m, 3H), 7.01-6.95 (m, 2H), 6.72-6.67 (m, 1H), 6.54-6.51 (m, 2H), 3.20 (s, 3H), 2.30 (s, 3H), 2.09 (s, 3H).

***N*-(2,5- Xylyl) cyclohexylamine** (Figure 4, entry 10). The general procedure gave 197 mg (97%) of a colorless oil: $^1$H NMR (300 MHz, $CDCl_3$) δ 6.92 (d, 1H, *J* = 7.4 Hz), 6.45-6.40 (m, 2H), 3.35-3.25 (m, 2H), 2.28 (s, 3H), 2.08 (s, br, 5H), 1.80-1.55 (m, 3H), 1.45-1.10 (m, 5H); $^{13}$C NMR (75 MHz, $CDCl_3$) δ 145.1, 136.6, 130.0, 118.6, 116.8, 110.9, 51.4, 33.6, 26.0, 25.0, 21.6, 17.1; IR (neat, $cm^{-1}$) 3427, 2927, 1520, 789. Anal. Calcd for $C_{14}H_{21}N$: C, 82.70; H, 10.41. Found: C, 82.51; H, 10.78.

***N*-(2,5-Xylyl)pyrrolidine** (Figure 4, entry 11). The general procedure conducted on a 2 mmol scale gave 346 mg (99%) of a colorless oil. See above for NMR data.

***N*-(2,5- Xylyl) morpholine** (Figure 4, entry 12). The general procedure conducted on a 2 mmol scale gave 340 mg (89%) of a colorless oil: $^1$H-NMR (300 MHz, $CDCl_3$) δ 7.05 (d, 1 H, *J* = 7.6 Hz), 6.81 (s, 1 H), 6.80 (d, 1 H, *J* = 7.6 Hz), 3.84 (m, 4 H, *J* = 4.6 Hz), 2.90 (m, 4 H, *J* = 4.6 Hz), 2.31 (s, 3 H), 2.27 (s, 3 H); $^{13}$C NMR (75 MHz, $CDCl_3$) δ 151.1, 136.3, 131.1, 129.4, 124.1, 119.8, 67.6, 52.4, 21.4, 17.7; IR (neat, $cm^{-1}$) 2962, 2856, 1243, 1117, 996.

**2-Methoxy-2',4'-dimethyldiphenylamine** (Figure 4, entry 13). The general procedure using $Pd_2(dba)_3$ gave 228 mg (94%) of a white solid: mp = 90-91.5 °C; $^1$H NMR (300 MHz, $CDCl_3$) δ 7.16 (s, 1H), 7.12 (d, *J* = 7.5 Hz, 1H), 7.05 (dd, *J* = 7.66, 2.2 Hz, 1H), 6.93-6.84 (m, 3H), 6.79 (d, *J* = 7.6 Hz, 1H), 5.86 (s, 1H), 3.93 (s, 3H), 2.31 (s, 3H), 2.26 (s, 3H); $^{13}$C NMR (75 MHz, $CDCl_3$) δ 148.2, 140.8, 136.5, 134.2, 130.9, 126.4, 123.1, 121.0, 120.4, 119.3, 114.6, 110.5, 55.8, 21.4, 17.7; IR (neat, $cm^{-1}$) 3411, 3062, 3045, 3006, 2964, 2933, 2919, 2856, 2836, 1598, 1576, 1521, 1501, 1449, 1412, 1341, 1293, 1243, 1220, 1179, 1115, 1048, 1030, 1000, 886, 809, 772, 741, 708. Anal. Calcd for $C_{15}H_{17}NO$: C, 79.26; H, 7.54. Found: C, 79.18; H, 7.56.

***N*-Benzyl-2,5-xylidene** (Figure 4, entry 14). The general procedure conducted on a 2 mmol scale gave 387 mg (92%) of a colorless oil. See above for NMR data.

***N*-(2,5- dimethylphenyl) aminoacetoaldehyde diethyl acetal** (Figure 4, entry 15). The general procedure conducted on a 2 mmol scale gave 474 mg (100%) of a colorless oil: $^1$H-NMR (300MHz, $CDCl_3$) δ 6.93 (d, 1 H, *J* = 7.4 Hz), 6.48 (d, 1 H, *J* = 7.4 Hz), 6.45 (s, 1 H), 4.74 (t, 1H, *J*=5.8 Hz), 3.80-3.52 (m, 5 H), 3.28 (d, 2 H, *J* = 5.7 Hz), 2.28 (s, 3 H), 2.10 (s,3 H), 1.25 (t, 6 H *J*= 7.0 Hz); $^{13}$C NMR (75 MHz, $CDCl_3$) δ 145.8, 136.8, 130.1, 119.4, 118.0, 111.1, 101.0, 62.4, 46.4, 21.8, 17.2, 15.6. IR (neat, $cm^{-1}$) 3421, 1522, 1125, 1059, 793.

**N-*p*-Anisidylpyrrolidine** (Figure 5, entry 16). The general procedure gave 159 mg (90%) of a white solid, mp 40-41 °C (lit mp 40-41 °C):[22] $^1$H NMR ($CDCl_3$, 300 MHz) δ 6.84 (d, 2H, *J* = 9.1 Hz), 6.53 (d, 2H, *J* = 9.0 Hz), 3.76 (s, 3H), 3.28-3.20 (m, 4H), 2.02-1.90 (m, 4H).

**4-[2-(*p*-Anisidyl) ethyl] morpholine** (Figure, 5, entry 17). The general procedure conducted on a 2 mmol scale gave 420 mg (89%) of a pale yellow oil: $^1$H NMR (300 MHz, $CDCl_3$) 6.79 (d-like, 2 H, *J* = 9.0 Hz), 6.61 (m, 2 H, *J*

= 9.0 Hz), 4.04 (br s, 1 H), 3.75 (s, 3 H), 3.72 (m, 4 H, *J* = 4.5 Hz), 3.12 (t, 2 H, *J* = 6.0 Hz), 2.62 (t, 2 H, *J* = 6.0 Hz), 2.47 (m, 4 H, *J* = 4.5 Hz), $^{13}C$ NMR (75 MHz, $CDCl_3$) 152.3, 142.9, 115.1, 114.4, 67.1, 56.0, 57.5, 53.6, 41.1; IR (neat, $cm^{-1}$) 3367, 2950, 1235, 1115, 1036. Anal. Calcd for $C_{13}N_{20}N_2O_2$: C, 66.07; H, 8.53. Found: C, 65.87; H, 8.62.

**1-(4-Methoxyphenyl)-4-methylpiperazine** (Figure 5, entry 18). The general procedure conducted on a 2 mmol scale using ligand **4** (not according to the invention) gave 341 mg (83%) of a yellow solid, mp 67-68 °C (lit. mp 67-70 °C): $^1H$ NMR (300 MHz, $CDCl_3$) 6.90 (m, 2 H, *J* = 9.1 Hz), 6.83 (m, 2 H, *J* = 9.1 Hz), 3.75 (s, 3 H), 3.10 (m, 4 H, *J* = 4.9 Hz), 2.56 (m, 4 H, *J* = 4.9 Hz), 2.34 (s, 3 H); $^{13}C$ NMR (75 MHz, $CDCl_3$) 153.8, 145.7, 118.2, 114.4, 55.6, 55.4, 50.7, 46.3; IR (neat, $cm^{-1}$) 1509, 1246, 1223, 1036, 832.

**4,4'-Dimethoxydiphenylamine** (Figure 5, entry 19). The general procedure using $Pd_2(dba)_3$ gave 217 mg (95%) of a pale yellow solid: mp 99.5-101.5 °C (lit. 101-103 °C); $^1H$ NMR (300 MHz, $CDCl_3$) δ 6.96 (d, *J* = 8.9 Hz, 4H), 6.84 (d, *J* = 9.0 Hz, 4H), 5.32 (s, 1H), 3.80 (s, 6H); $^{13}C$ NMR (75 MHz, $CDCl_3$) δ 154.4, 138.1, 119.7, 114.9, 55.8; IR (neat, $cm^{-1}$) 3421, 3031, 3014, 2958, 2939, 2916, 2840, 1513, 1466, 1441, 1299, 1248, 1218, 1179, 1115, 1030, 830, 818, 762, 708. Anal. Calcd for $C_{14}H_{15}NO_2$: C, 73.34; H, 6.59. Found: C, 73.51; H, 6.74.

**Benzophenone *N*-(2-methoxyphenyl)hydrazone** (Figure 5, entry 20). The general procedure using $Pd_2(dba)_3$ gave 278 mg (92%) of a pale yellow solid: mp 101.5-102.5 °C (lit. 101°C); $^1H$ NMR (300 MHz, $CDCl_3$) δ 8.02 (s, 1H), 7.69 (d, *J* = 7.7 Hz, 1H), 7.66-7.50 (m, 5H), 7.40-7.30 (m, 5H), 7.01 (td, *J* = 7.7, 1.7 Hz, 1H), 6.85-6.77 (m, 2H), 3.59 (s, 3H); $^{13}C$ NMR (75 MHz, $CDCl_3$) δ 145.6, 145.0, 138.8, 134.4, 133.2, 129.7, 129.3, 129.2, 128.3, 128.1, 126.7, 121.7, 119.3, 112.5, 110.2, 55.7; IR (neat, $cm^{-1}$) 3342, 3060, 3010, 2970, 2945, 2840, 1602, 1561, 1511, 1494, 1457, 1441, 1432, 1322, 1256, 1218, 1181, 1127, 1025, 917, 766, 743, 702. Anal. Calcd for $C_{20}H_{18}N_2O$: C, 79.44; H, 6.00. Found: C, 79.48; H, 6.09.

***N*-(2-Methoxyphenyl)benzylamine** (Figure 5, entry 21) The general procedure conducted on a 2 mmol scale gave 423 mg (99%) of the title compound as a colorless oil. See above for NMR data.

***N*-(2- Methoxyphenyl) pyrrolidine** (Figure 5, entry 22) The general procedure conducted on a 2 mmol scale gave 314 mg (89%) of the title compound as a colorless oil. $^1H$ NMR (300 MHz, $CDCl_3$) δ 6.91-6.77 (m, 4H), 3.84 (s, 3H), 3.35-3.25 (m, 4H), 1.95-1.85 (m, 4H); $^{13}C$ NMR (75 MHz, $CDCl_3$) δ 150.4, 139.9, 121.0, 119.6, 115.4, 111.6, 55.5, 50.4, 24.6; IR (neat, $cm^{-1}$) 2960, 1596, 1503, 1229, 735. Anal. Calcd for $C_{11}H_{15}NO$: C, 74.54; H, 8.53. Found: C, 74.63; H, 8.46.

***N*-(*p*-Tolyl)-3,5-dimethoxyaniline** (Figure 5, entry 23) The general procedure using $Pd_2(dba)_3$ gave 228 mg (94%) of a white solid: mp 66.5-67.5 °C; $^1H$ NMR (300 MHz, $CDCl_3$) δ 7.11 (d, *J* = 8.6 Hz, 2H), 7.04 (d, *J* = 8.5 Hz, 2H), 6.20 (d, *J* = 2.1 Hz, 2H), 6.05 (t, *J* = 2.2 Hz, 1H), 5.64 (s, 1H), 3.77 (s, 6H), 2.33 (s, 3H); $^{13}C$ NMR (75 MHz, $CDCl_3$) δ 161.8, 146.3, 139.9, 131.6, 130.0, 120.0, 95.1, 92.5, 55.4, 20.9; IR (neat, $cm^{-1}$) 3367, 3012, 2966, 2937, 2840, 1594, 1513, 1478, 1459, 1254, 1200, 1189, 1165, 1144, 1057, 924, 822, 810, 770, 720,683. Anal. Calcd for $C_{15}H_{17}NO_2$: C, 74.05; H, 7.04. Found: C, 74.06; H, 7.23.

***N*-Benzhydrilidene-3,5-dimethoxyaniline** (Figure 5, entry 24) The general procedure using $Pd_2(dba)_3$ (1 mol % Pd) and ligand **4** (not according to the invention) gave 316 mg (100%) of a pale yellow solid, mp 101-102 °C: $^1H$ NMR (300 MHz, $CDCl_3$) 7.76-7.71 (m, 2 H), 7.49-7.12 (m, 8 H), 6.06 (t, 1 H, *J* = 2.1), 5.91 (d, 2 H, *J* = 2.1 Hz), 3.62 (s, 6 H); $^{13}C$ NMR (75 MHz, $CDCl_3$) 168.5, 160.8, 153.14, 139.6, 136.3, 130.9, 129.5, 129.4, 128.8; 128.3, 128.1, 99.4, 96.1, 55.4; IR (neat, $cm^{-1}$) 1594, 1208, 1154, 1123, 704 . Anal. Calcd for $C_{21}H_{19}NO_2$: C, 79.47; H, 6.03. Found: C, 79.61; H, 6.19.

***N*-(2,6-dimethylphenyl)morpholine** (Figure 5, entry 25) The general procedure using ligand **2** (not according to the invention) and a reaction temperature of 110 °C gave 162 mg (86%) of a white solid, mp 86-87 °C: $^1H$ NMR (300 MHz, $CDCl_3$) 7.01-6.92 (m, 3 H), 3.79 (m, 4 H, *J* = 4.5 Hz), 3 .08 (m, 4 H, *J* = 4.5 Hz), 2.34 (s, 6 H); $^{13}C$ NMR (75 MHz, $CDCl_3$) 148.1, 137.1, 129.2, 125.5, 68.3, 50.2, 19.8; IR (neat, $cm^{-1}$) 1260, 1109, 939, 843, 782. Anal. Calcd for $C_{12}H_{17}NO$: C, 75.36; H, 8.96. Found: C, 75.35; H, 9.26.

*N*-(2,6-dimethylphenyl)benzylamine (Figure 5, entry 26) The general procedure using 1.5 equiv benzylamine gave 183 mg (87%) of a colorless oil: $^1H$ NMR (300 MHz, $CDCl_3$) 7.39-7.22 (m, 5 H), 7.02 (m, 2 H, *J* = 7.4 Hz), 6.85 (m, 1 H, *J* = 7.4 Hz), 4.17 (s, 2 H), 3.20 (br s, 1H), 2.27 (s, 6 H); $^{13}C$-NMR (75 MHz, $CDCl_3$) 146.0, 140.5, 129.9, 128.9, 128.7, 128.1, 127.4, 122.3, 53.0, 18.7; IR (neat, $cm^{-1}$) 3361, 3029, 2941, 1218, 1096.

**2,6-Diisopropyl-2',6'-dimethyldiphenylamine** (Figure 5, entry 27). The general procedure using $Pd_2(dba)_3$ (4 mol % Pd) gave 212 mg (75%) of a white solid: mp 40.5-44 °C; $^1H$NMR (300 MHz, $CDCl_3$) δ 7.16-7.11 (m, 3H), 6.96 (d, *J* = 7.2 Hz, 2H), 6.74 (t, *J* = 7.5 Hz, 1H), 4.81 (s, 1H), 3.17 (sept, 6.8 Hz, 1H), 2.00 (s, 6H), 1.15 (s, 6H), 1.12 (s, 6H); $^{13}C$ NMR (75 MHz, $CDCl_3$) δ 144.4, 143.3, 139.0, 129.7, 125.8, 125.0, 123.4, 119.8, 28.3, 23.7, 19.6, IR (neat, $cm^{-1}$) 3421, 3064, 3041, 3027, 2958, 2925, 2867, 1590, 1470, 1447, 1378, 1362, 1333, 1275, 1225, 1098, 1179, 1162, 1057, 1034, 990, 938, 920, 888, 793, 768, 737, 695, 683. Anal. Calcd for $C_{20}H_{27}N$: C, 85.35; H, 9.67. Found: C, 85.11; H, 9.56.

***Example 61***

Catalytic amination of chloropyridines at 80-110 ˚C

**General Procedure**. An oven-dried resealable Schlenk flask was evacuated and backfilled with argon. The flask was charged with palladium acetate (0.5 mol %), **3** (not according to the invention; 1.0 mol %), NaO*t*Bu (1.4 equiv), and evacuated and backfilled with argon. The flask was capped with a rubber septum and toluene (2 mL/mmol halide), the aryl halide (1.0 equiv), and the amine (1.2 equiv) were added through the septum. The septum was replaced with a teflon screwcap, the flask was sealed, and the mixture was heated to 80 ˚C with stirring until the starting aryl halide had been completely consumed as judged by GC analysis. The mixture was cooled to rt, diluted with ether (30 mL), filtered through celite, and concentrated *in vacuo.* The crude product was purified by flash chromatography on silica gel.

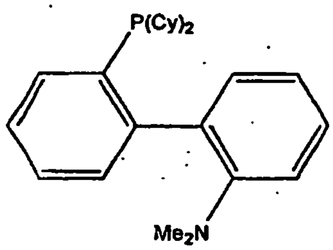

**2**

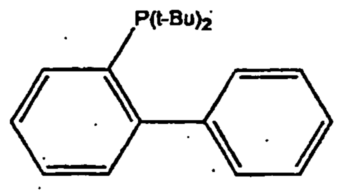

**3**

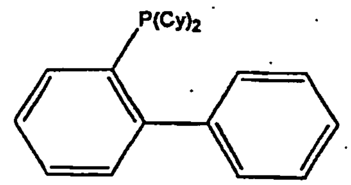

**4**

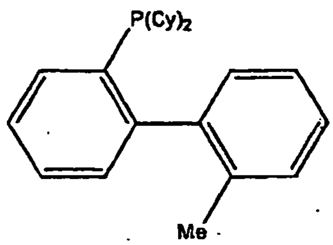

**5**

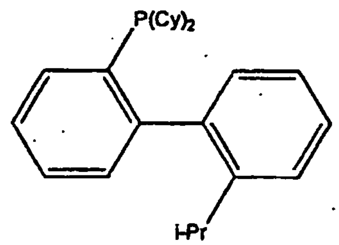

**6**

***N*-Benzyl- 2- aminopyridine** (Figure 7, entry 1). The general procedure conducted on a 2 mmol scale using a reaction temperature of 100 ˚C gave 358 mg (100 %) of a white solid, mp 93-94 ˚C which was determinded to .be a 9/1 mixture (by $^1H$ NMR) of the title compound and bis(2-pyridyl)benzylamine. Data are given for the title compound: $^1H$ NMR (300 MHz, $CDCl_3$) 8.07 (ddd, 1 H, *J* = 5.0, 1.8, 0,8 Hz), 1.40-7.23 (m, 6 H), 6.56 (ddd, 1 H, *J* = 7.1, 5.0, 0.9 Hz), 6.35 (ddd, 1 H, *J* = 8.4, 0.9, 0.8 Hz), 5.07 (br s, 1 H), 4.48 (d, 2 H, *J* = 5.8 Hz); $^{13}C$ NMR (75 M Hz, $CDCl_3$) 158.7, 148.3, 139.3, 137.6, 128.7, 127.5, 127.3, 113.2, 106.9, 46.5; IR (neat, $cm^{-1}$) 3222, 1598, 1441,770, 748, 699.

***N*-(2-Pyridyl)morpholine** (Figure 7, entry 2) The general procedure conducted on a 2 mmol scale using a reaction temperature of 100˚C and ligand **4** (not according to the invention) gave 319 mg (97%) of a pale yellow oil: $^1H$ NMR (300 MHz, $CDCl_3$) 8.20 (ddd, 1 H, *J* = 5.0, 2.0, 0.9 Hz), 7.49 (ddd, 1 H, *J* = 8.6, 7.1, 2.0 Hz), 6.66 (ddd, 1 H, *J* = 5.0, 2.0; 0.9 Hz), 6.63 (ddd, 1 H, *J* = 8.6, 0.9, 0.9 Hz), 3.82 (m, 4 H, *J* = 4.9 Hz), 3.49 (m, 4 H, *J* = 4.9 Hz); $^{13}C$ NMR (75 MHz, $CDCl_3$) 158.8, 148.0, 137.6, 113.9, 107.0, 66.9, 45.7; IR (neat, $cm^{-1}$) 2964, 2858, 1256, 1121, 982, 944.

***N*-Methyl-*N*-(3- pyridyl) aniline** (Figure 7, entry 3) The general procedure using -1 mol % $Pd(OAc)_2$, ligand **4** (not according to the invention) and a reaction temperature of 110 ˚C gave 178 mg (97%) of a pale yellow oil: $^1H$ NMR (300 MHz, $CDCl_3$) 8.31 (d, 1 H, *J* = 2.8 Hz), 8.12 (dd, 1 H, *J* = 4.6, 1.5 Hz), 7.33-7.02 (m, 7 H), 3.31 (s, 3 H); $^{13}C$ NMR (75 MHz, $CDCl_3$) 143.2, 140.4, 136.5, 135.9, 125.0, 120.1, 118.8, 118.6, 117.7, 37.5; IR (neat, $cm^{-1}$) 3035, 1582, 1345, 1256, 1133.

***N*-Methyl-*N*-(3- pyridyl) aniline** (Figure 7, entry 3) The general procedure using 1 mol % $Pd(OAc)_2$, ligand **2** (not according to the invention) and a reaction temperature of 110 ˚C gave 176 mg (96%) of a pale yellow oil.

***N*-Benzyl- 3- aminopyridine** (Figure 7, entry 4). The general procedure using 1 mol % $Pd(OAc)_2$ and a reaction temperature of 110 ˚C gave 161 mg (88%) of a pale green solid, mp 87-88 ˚C (lit mp 88-89 ˚C): $^1H$ NMR (300 MHz, $CDCl_3$) 8.07 (dd, 1 H, *J* = 3.0, 0.6 Hz), 7.96 (dd, 1 H, *J* = 4.6, 1.5 Hz), 7.38-7.26 (m, 5 H), 7.06 (ddd, 1 H, *J* = 8.4, 4.6, 0.6 Hz), 6.87 (ddd, 1 H, *J* = 8.4, 3.0, 1.5 Hz), 4.22 (br s, 1H), 4.33 (s, 2 H); $^{13}C$ NMR (75 MHz, $CDCl_3$) 144:1, 138.9, 138.6, 136.2, 128.8, 127.52, 127.46, 123.8, 118.6, 47.9; IR (neat, $cm^{-1}$) 3261, 1590, 1578, 1328, 712.

***N*-Hexyl- 3- aminopyridine** (Figure 7, entry 5) The general procedure using 3 equiv hexylamine, 1 mol % Pd

(OAc)$_2$, ligand **2** (not according to the invention) and a reaction temperature of 100 ˚C gave 134 mg (75%) of a white solid, mp 57-58 ˚C: $^1$H NMR (300 MHz, $CDCl_3$) 8.01 (d, 1 H, *J* = 2.8 Hz), 7.93 (dd, 1 H, *J* = 4.6, 1.0 Hz), 7.06 (dd, 1 H, *J* = 8.3, 4.6 Hz), 6.84 (ddd, 1 H, *J* = 8.3, 2_8, 1.0 Hz), 3.80 (br s, 1 H), 3.10 (t, 2 H, *J* = 6.9 Hz), 1.62 (tt, 2 H, 6.9, 6.9 Hz), 1.46-1.26 (m, 6 H), 0.90 (t, 3 H, *J* = 6.0 Hz); $^{13}$C NMR (75 MHz, $CDCl_3$) 144.5, 138.4, 136.0, 123.8, 118.3, 43.7, 31.8, 29.5, 26.9, 22.8, 14.2; FT-IR (neat, cm$^{-1}$) 3251, 1584, 1418, 789, 704.

**N-(3-Pyridyl)morpholine** (Figure 7, entry 6) The general procedure using 1 mol % Pd(OAc)$_2$ and a reaction temperature of 110˚C gave 116 mg (71%) of a pale yellow oil: $^1$H NMR (300 MHz, $CDCl_3$) 8.31 (dd, 1 H, *J* = 2.1, 1.5 Hz), 8.13 (dd, 1 H, *J* = 3.3, 2.4 Hz), 7.20-7.17 (m, 2 H), 3.87 (m, 4 H, *J* = 4.9 Hz), 3.18 (m, 4 H, *J* = 4.9 Hz); $^{13}$C NMR (75 MHz, $CDCl_3$) 146.9, 141.1, 138.3, 123.5, 122.1, 66.7, 48.6; IR (neat, cm$^{-1}$) 2856, 1582, 1246, 1123, 930.

***N,N*-Dibutyl- 3- aminopyridine** (Figure 7, entry 7) The general procedure using 1 mol % Pd(OAc)$_2$, ligand **2** (not according to the invention), and a reaction temperature of 110 ˚C gave 171 mg (83%) of a pale yellow oil: $^1$H NMR (300 MHz, $CDCl_3$) 8.05 (d, 1 H, *J* = 3.0 Hz), 7.87 (dd, 1 H, *J* = 4.6, 1.2 Hz), 7.08 (dd, 1 H, *J* = 8.5, 4.6 Hz), 6.88 (ddd, 1 H, *J* = 8.5, 3.0, 1.2 Hz), 3.26 (t, 4 H, *J* = 7.5 Hz), 1.56 (m, 4 H), 1.32 (qt, 4 H, *J* = 7.3, 7.3 Hz), 0.95 (t, 6 H, *J* = 7.3 Hz); $^{13}$C NMR (75 MHz, $CDCl_3$) 143.9, 136.5, 134.5, 123.6, 117.7, 50.6, 29.3, 20.4, 14.2; IR (neat, cm$^{-1}$) 2962, 2935, 2873, 1584, 1225, 1181.

***Example 62***

Catalytic amination of aryl bromides at 80-110 ˚C

**General Procedure.** An oven-dried resealable Schlenk flask was evacuated and backfilled with argon. The flask was charged with palladium acetate (0.5 mol %), **3** (not according to the invention; 1.0 mol %), NaO*t*Bu (1.4 equiv), and evacuated and backfilled with argon. The flask was capped with a rubber septum and toluene (2 mL/mmol halide), the aryl halide (1.0 equiv), and the amine (1.2 equiv) were added through the septum. The septum was replaced with a teflon screwcap, the flask was sealed, and the mixture was heated to 80 ˚C with stirring until the starting aryl halide had been completely consumed as judged by GC analysis. The mixture was cooled to rt, diluted with ether (30 mL), filtered through celite, and concentrated *in vacuo.* The crude product was purified by flash chromatography on silica gel.

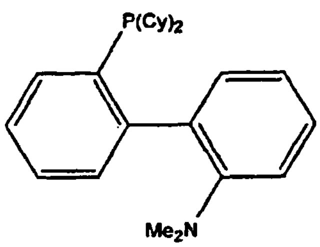

2

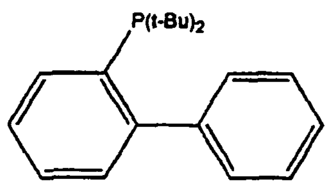

3

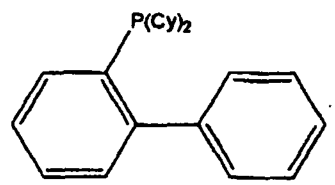

4

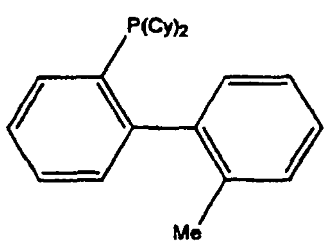

5

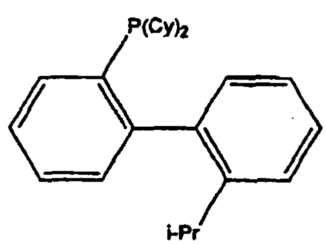

6

***N*-(4-methylphenyl)morpholine** (Figure 9, entry 1) The general procedure conducted on a 2 mmol scale gave 329 mg (93%) of a white solid. See above for NMR data.

**4-Methoxy-4'-(dimethylamino)diphenylamine** (Figure 9, entry 2) The general procedure using $Pd_2(dba)_3$ gave 229 mg (95%) of a pale yellow solid: mp 77-78 ˚C (lit. 78 ˚C); $^1$H NMR (300 MHz, $C_6D_6$) δ 6.97 (d, *J* = 8.9 Hz, 2H), 6.84 (d, *J* = 8.6 Hz, 2H), 6.83 (d, *J* = 8.6 Hz, 2H), 6.64 (d, *J* = 8.9 Hz, 2H), 4.78 (s, 1H), 3.37 (s, 3H), 2.57 (s, 6H); $^{13}$C NMR (75 MHz, $C_6D_6$) δ 154.6, 147.2, 140.1, 135.6, 121.8, 118.9, 115.4, 115.1, 55.6, 41.6; IR (neat, cm$^{-1}$) 3273, 3039, 3013, 2966, 2954, 2879, 2832, 2792, 1507, 1476, 1457, 1437, 1304, 1295, 1252, 1237, 1208, 1169, 1129, 1034, 938, 818, 797, 762, 731. Anal. Calcd for $C_{15}H_{18}N_2O$: C, 74.35; H, 7.49. Found: C, 74.23; H, 7.47.

***N*-(*p*-Tolyl)-*p*-anisidine** (Figure 9, entry 3) The general procedure using $Pd_2(dba)_3$ gave 194 mg (91%) of a

pale yellow solid: mp 81-83 ˚C (lit. 82-83 ˚C); $^1$H NMR (300 MHz, $CDCl_3$) δ 7.05 (d, *J* = 8.5 Hz, 2H), 7.04 (d, *J* = 8.8 Hz, 2H), 6.87 (d, *J* = 8.5 Hz, 2H), 6.86 (d, *J* = 8.8 Hz, 2H) 5.42 (s, 1H), 3.81 (s, 3H), 2.29 (s, 3H); $^{13}$C NMR (75 MHz, $CDCl_3$) δ 155.0, 142.5, 136.8, 130.0, 129.5, 121.3, 116.7, 114.8, 55.8, 20.8; IR (neat, $cm^{-1}$) 3415, 3026, 3014, 2952, 2911, 2858, 2836, 1613, 1582, 1515, 1466, 1316, 1295, 1241, 1225, 1179, 1125, 1105, 1032, 812, 768, 704. Anal. Calcd for $C_{14}H_{15}NO$: C, 78.84; H, 7.09. Found: C, 78.78; H, 7.02.

***N*-Ethyl-*N*-(3,5- dimethylphenyl) aniline** (Figure 9, entry 4). The general procedure gave 188 mg (84%) of a pale yellow oil: $^1$H NMR (300 MHz, $CDCl_3$) δ 7.29 (dd, *J* = 8.8, 8.5 Hz, 2H), 6.97 (d, *J* = 8.8 Hz, 2H), 6.92 (t, *J* = 8.5 Hz, 1H), 6.67 (s, 2H), 6.65 (s, 1H), 3.77 (q, *J* = 6.8 Hz, 2H), 2.28 (s, 6H), 1.23 (t, *J* = 6.8 Hz, 3H); $^{13}$C NMR (75 MHz, $CDCl_3$) δ 148.1, 147.8, 139.0, 129.3, 123.6, 120.6, 120.3, 119.6, 46.6, 21.7, 13.0; IR (neat, $cm^{-1}$) 3035, 2972, 2917, 2869, 1590, 1495, 1470, 1370, 1351, 1289, 1268,1250, 1191, 1129, 1106, 1071, 1032, 992, 847, 824, 809, 749, 693. Anal. Calcd for $C_{16}H_{19}N$: C, 85.28; H, 8.50. Found: C, 84.99; H, 8.69.

***N*-Benzyl-3,5-xylidene** (Figure 9, entry 5). The general procedure using $Pd_2(dba)_3$ and 1.5 equiv benzylamine gave 174 mg (82%) of a colorless oil. $^1$H NMR. (300 MHz, $CDCl_3$) δ 7.38-7.25 (m, 5H), 6.39 (s, 1H), 6.29 (s, 2H), 4.30 (s, 2H), 3.89 (s, br, 1H), 2.23 (s, 6H).

***N*-Mesityl- 3,4-(methylenedioxy) aniline** (Figure 9, entry 6) The general procedure gave 245 mg (96%) of a pale yellow solid: mp 104.5-106.5 ˚C (lit. 77-79 ˚C); $^1$H NMR (300 MHz, $CDCl_3$) δ 6.96 (s, 2H), 6.65 (d, *J* = 8.3 Hz, 1H), 6.14 (d, *J* = 2.1 Hz, 1H), 5.97 (dd, *J* = 8.3, 2.3 Hz, 1H), 5.87 (s, 2H), 4.98 (s, 1H), 2.33 (s, 3H), 2.21 (s, 6H); $^{13}$C NMR (75 MHz, $CDCl_3$) δ 148.5, 142.5, .140.1, 136.4, 135.7, 135.3, 129.4, 108.7, 105.4, 100.8, 96.5, 21.1, 18.4; IR (neat, $cm^{-1}$) 3369, 2953, 2917, 2885, 2856, 1632, 1615, 1497; 1482, 1245, 1227, 1194, 1038, 944, 932, 859, 822, 795. Anal. Calcd for $C_{16}H_{17}NO_2$: C, 75.27; H, 6.71. Found: C, 75.20; H, 6.76.

***N*-(3-Carbomethoxyphenyl)morpholine** (Figure 9, entry. 7). The general procedure using $Pd_2(dba)_3$, ligand **2** (not according to the invention), and a reaction temperature of 100 ˚C gave 193 mg (87%) of a pale yellow oil. See above for NMR data.

***N*-(4-*t*-Butylphenyl)piperidine** (Figure 9, entry 8). The general procedure using $Pd_2(dba)_3$ and ligand **4** (not according to the invention) gave 185 mg (85%) of a white solid. $^1$H NMR (300 MHz, $CDCl_3$) δ 7.26 (d, 2H, *J* = 9.8 Hz), 6.89 (d, 2H, *J* = 9.7 Hz), 3.11 (t, 4H, *J* = 5.5 Hz), 1.75-1.67 (m, 4H), 1.60-1.50 (m, 2H), 1.29 (s, 9H).

***N*,*N*-Dibutyl-4-*t*-butylaniline** (Figure 9, entry 9). The general procedure using $Pd_2(dba)_3$ and ligand **4** (not according to the invention) gave 222 mg (85%) of a colorless oil. $^1$H NMR (300 MHz, $CDCl_3$) δ 7.21 (d, 2H, *J* = 9.0 Hz), 6.59 (d, 2H, *J* = 8.7 Hz), 3.23 (t, 4H, *J* = 7.8 Hz), 1.62-1.50 (m, 4H), 1.41-1.28 (m, 4H), 1.28 (s, 9H), 0.94 (t, 6H, *J* = 7.5 Hz).

***N,N*-Dibutyl-4-*t*-butylaniline** (Figure 9, entry 9). The general procedure using $Pd_2(dba)_3$ and ligand **5** (not according to the invention) gave 237 mg (91%) of a colorless oil. See above for NMR. data.

***N*-Methyl-*N*-Phenyl-2,5-xylidene** (Figure 9, entry 10). The general procedure using $Pd_2(dba)_3$ and ligand **4** (not according to the invention) gave 159 mg (75%) of a colorless oil: See above for NMR data.

***Example 63***

Catalytic amination of aryl triflates at 80-110 ˚C

**General Procedure**. An oven-dried resealable Schlenk flask was evacuated and backfilled with argon. The flask was charged with palladium acetate (0.5 mol %), **3** not according to the invention; (1.0 mol %), NaO*t*Bu (1.4 equiv), and evacuated and backfilled with argon. The flask was capped with a rubber septum and toluene (2 mL/mmol halide), the aryl halide (1.0 equiv), and the amine (1.2 equiv) were added through the septum. The septum was replaced with a teflon screwcap, the flask was sealed, and the mixture was heated to 80˚C with stirring until the starting aryl halide had been completely consumed as judged by GC analysis. The mixture was cooled to rt, diluted with ether (30 mL), filtered through celite, and concentrated *in vacuo.* The crude product was purified by flash chromatography on silica gel.

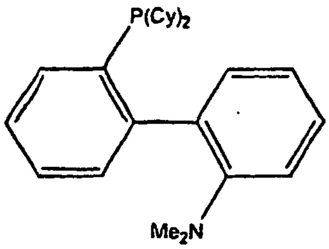

2

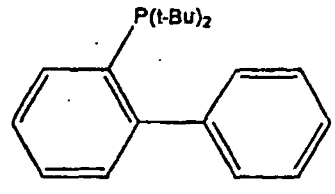

3

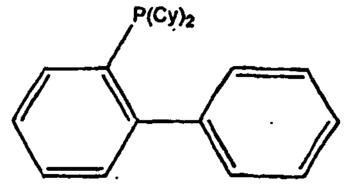

4

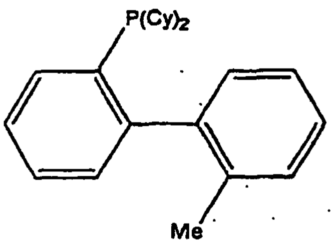

5

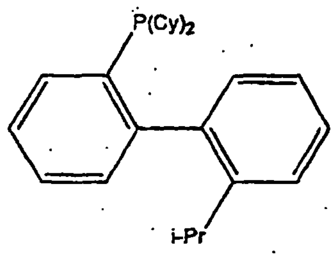

6

Compounds 2 to 6 are not ligands according to the invention.

***N*-(4-*t*-Butylphenyl)morpholine** (Figure 11, entry 1) The general procedure using $K_3PO_4$ as the base, 1 mol % $Pd(OAc)_2$, THF solvent, and a reaction temperature of 65 ˚C gave 201 mg (92%) of a white solid. $^1H$ NMR (300 MHz, $CDCl_3$,) δ 7.30 (d,2H, *J* = 8.9 Hz), 6.87 (d, 2H, *J* = 8.9 Hz), 3.86 (t, 4H, *J* = 4.7 Hz), 3.14 (t, 4H, *J* = 4.9 Hz), 1.30 (s, 9H).

***N*-(4-*t*-Butylphenyl)-*p*-anisidine** (Figure 11, entry 2) The general procedure using $K_3PO_4$ as the base, 0.5 mol % $Pd_2(dba)_3$, and DME solvent gave 221 mg (87%) of a pale yellow solid, mp xx ˚C. $^1H$ NMR (300 MHz, $CDCl_3$) δ 7.30-7.20 (m, 2H), 7.10-6.75 (m, 6H), 5.50 (s, br, 1H), 3.20 (s, 3H), 1.29 (s, 9H); $^{13}C$ NMR (75 MHz, $CDCl_3$) δ 154.8,142.6, 142.4, 136.6, 126.0, 121.3, 115.7, 114.6, 55.5, 34.0, 31.5.

***N*-(4-*t*-Butylphenyl) benzylamine** (Figure 11, entry 3) The general procedure using 1 mol % $Pd(OAc)_2$ and 1.5 equiv benzylamine gave 163 mg (68%) of a colorless oil. $^1H$ NMR (300 MHz, $CDCl_3$) δ 7.40-7.26 (m, 5H), 7.20 (d, 2H, *J* = 6.7 Hz), 6.60 (d, 2H, *J* = 6.5 Hz), 4.31 (s, 2H), 3.95 (s, br, 1H), 1.27 (s, 9H); $^{13}C$ NMR (75 MHz, $CDCl_3$) δ 145.8, 140.3, 139.6, 128.6, 127.5, 127.1, 126.0, 112.5, 48.6, 33.8, 31.5.

***N,N*-Dibutyl-*p*-*t*-butylaniline** (Figure 11, entry 4) The general procedure using 1 mol % $Pd(OAc)_2$ and 2 mol % **4** (not according to the invention) gave 185 mg (71%) of a colorless oil. See above for NMR data.

***N*-(4-Nitrophenyl)-*p*-anisidine** (Figure 11, entry 5) The general procedure using $K_3PO_4$ as the base, 0.5 mol % $Pd_2(dba)_3$, and DME solvent (the material was purified by crystallization from ethanol instead of flash chromatography) gave 183 mg (75%) of a yellow solid. See above for NMR data.

***N*-(3,4-Dimethylphenyl)pyrrolidine** (Figure 11, entry 6) The general procedure using $K_3PO_4$ as the base, 0.5 mol % $Pd_2(dba)_3$, ligand **4** (not according to the invention), and DME solvent gave 155 mg (89%) of a colorless oil. $^1H$ NMR (300 MHz, $CDCl_3$) δ 6.97 (d, 1H, *J*=8.1 Hz), 6.40-6.33 (m, 2H), 3.26-3.21 (m, 4H), 2.23 (s, 3H), 2.16 (s, 3H), 2.00-1.94 (m, 4H); $^{13}C$ NMR (75 MHz, $CDCl_3$) δ 146.6, 137.0,130.1, 113.3, 109.2, 47.8, 25.4, 20.2, 18.6.

**2 Methoxy-4'-cyanodiphenylamine** (Figure 11, entry 7) The general procedure using $K_3PO_4$ as the base, $Pd_2(dba)_3$, and THF solvent (the product was purified by recrystallization from ethanol/hexanes instead of chromatography) gave 194 mg (87%) of a pale yellow solid: mp 108-109 ˚C; $^1H$ NMR (300 MHz, $CDCl_3$) δ 7.50 (d, *J* = 8.7 Hz, 2H), 7.37 (d, *J* = 7.5 Hz, 1H), 7.08-6.94 (m, 5H), 6.38 (s, 1H), 3.89 (s, 3H); $^{13}C$ NMR (75 MHz, $CDCl_3$) δ 150.2, 147.6, 133.8, 129.7, 123.4, 120.9, 120.1, 118.9, 115.6, 111.2, 101.8, 55.8; IR (neat, $cm^{-1}$) 3321, 3045, 3002, 2968, 2929, 2831, 2217, 1611, 1598, 1586, 1522, 1507, 1488, 1459, 1341, 1328, 1295, 1248, 1175, 1111, 1028, 830, 820, 741. Anal. Calcd for $C_{14}H_{12}N_2O$: C, 74.98; H, 5.39. Found: C, 74.95; H, 5.32.

***N*-(4-Acetylphenyl)-*m*-toluidine** (Figure 11, entry 8) The general procedure using $K_3PO_4$ as the base, 0.5 mol % $Pd_2(dba)_3$, and DME solvent gave 211 mg (94%) of a yellow solid, mp xx ˚C. $^1H$ NMR (300 MHz, $CDCl_3$) δ 7.86 (d, 2H, *J*=6.9 Hz), 7.23 (t, 1H, *J*=6.6 Hz), 6.97-6.87 (m, 4H), 6.11 (s, 1H), 2.53 (s, 3H), 2.35 (s, 3H); $^{13}C$ NMR (75 MHz, $CDCl_3$) δ 196.4, 148.5, 140.5, 139.4, 130.6, 129.2, 128.7, 124:1, 121.3, 117.7, 114.3, 26.1, 21.4.

***N*-(4-Nitrophenyl)piperidine** (Figure 11, entry 9) The general procedure using $K_3PO_4$ as the base, 0.5 mol % $Pd_2(dba)_3$, and DME solvent gave 168 mg (82%) of a yellow solid. $^1H$ NMR 300 MHz, $CDCl_3$) δ 8.09 (d, 2H, *J* = 9.3 Hz), 6.79 (d, 2H, *J* = 9.9 Hz), 3.50 (m, 4H), 1.75-1.65 (m, 6H).

***Example 64***

Catalytic Amination of Aryl Chlorides at Low Catalyst Loadings

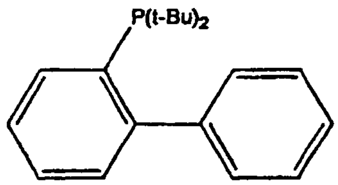

3

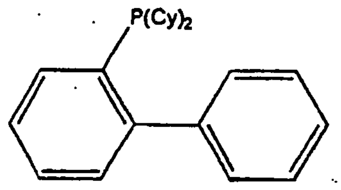

4

Compounds 3 and 4 are not ligands according to the invention.

***N*-Methyl-*N*-phenyl-*p*-toluidine** (Figure 6, entry 1) An oven-dried Schlenk flask was evacuated and backfilled with argon. The flask was charged with NaO*t*Bu (270 mg, 2.8 mmol), then evacuated and backfilled with argon and toluene (1 mL), 4-chlorotoluene (0.24 mL, 2.0 mmol), and *N*-methyl aniline (0.26 mL, 2.4 mmol) were added through a rubber septum. A separate flask was charged with $Pd_2(dba)_3$ (9.2 mg, 0.01 mmol) and ligand **3** (not according to the invention; 12.0 mg, 0.04 mmol), and was purged with argon. Toluene (4 mL) was added, the mixture was stirred for 1 minute at rt, then 200 μL of this solution (0.05. mol % Pd, 0.1 mol % ligand 3) was added to the Schlenk flask followed by additional toluene (1 mL). The septum was removed; the flask was sealed with a teflon screwcap and the mixture was stirred at rt for 2 minutes, then heated to 100 ˚C with stirring until the starting aryl chloride had been completely consumed as judged by GC analysis. The mixture was cooled to room-temperature, diluted with ether, and filtered through Celite. The crude product was purified by flash chromatography on silica gel to give 378 mg (96%) of the title compound as a colorless oil. See above for NMR data.

***N*-(4- Methylphenyl) morpholine** (Figure 6, entry 2) An oven-dried Schlenk flask was evacuated and backfilled with argon. The flask was charged with NaO*t*Bu (270 mg, 2.8 mmol), then evacuated and backfilled with argon and toluene (1 mL), 4-chlorotoluene (0.24 mL, 2.0 mmol), and morpholine (0.20 mL, 2.4 mmol) were added through a rubber septum. A separate flask was charged with $Pd_2(dba)_3$ (4.6 mg, 0.005 mmol) and ligand **4** (not according to the invention; 7.0 mg, 0.02 mmol), and was purged with argon. THF (1 mL) was added, the mixture was stirred for 1 minute at rt, then 100 μL of this solution (0.05 mol % Pd, 0.1 mol % ligand **4**) was added to the Schlenk flask followed by additional toluene (1 mL). The septum was removed; the flask was sealed with a teflon screwcap and the mixture was stirred at rt for 2 minutes, then heated to 100˚C with stirring until the starting aryl chloride had been completely consumed as judged by GC analysis. The mixture was cooled to room-temperature, diluted with ether, and filtered through Celite. The crude product was purified by flash chromatography on silica gel to give 311 mg (88%) of the title compound as a white solid. See above for NMR data.

***N*-Methyl-*N*-phenyl- 2,5- xylidene** (Figure 6, entry 3) An oven-dried Schlenk flask was evacuated and backfilled with argon. The flask was charged with NaO*t*Bu (270 mg, 2.8 mmol), then evacuated and backfilled with argon and toluene (1 mL), 2-chloro-*p*-xylene (not according to the invention; 0.24 mL, 2.0 mmol), and *N*-methyl aniline (0.26 mL, 2.4 mmol) were added through a rubber septum. A separate flask was charged with $Pd_2(dba)_3$(9.2 mg, 0.01 mmol) and ligand **3** (not according to the invention; 2.0 mg, 0.04 mmol), and was purged with argon. Toluene (4 mL) was added, the mixture was stirred for 1 minute at rt, then 200 μL of this solution (0.05 mol % Pd, 0.1 mol % ligand **3**) was added to the Schlenk flask followed by additional toluene (1 mL). The septum was removed; the flask was sealed with a teflon screwcap and the mixture was stirred at rt for 2 minutes, then heated to 100 ˚C with stirring until the starting aryl chloride had been completely consumed as judged by GC analysis. The mixture was cooled to room-temperature, diluted with ether, and filtered through celite. The crude product was purified by flash chromatography on silica gel to give 391 mg (93%) of the title compound as a colorless oil. See above for NMR data.

**2-Methoxy-2',4'-dimethyldiphenylamine** (Figure 6, entry 4). An oven-dried, resealable Schlenk tube equipped with a Teflon screwcap was evacuated and backfilled with argon. The cap was removed, and the tube was charged with sodium *tert*-butoxide (1.35 g, 14.0 mmol), tris(dibenzylideneacetone) dipalladium (2.3 mg, 0.0025 mmol, 0.05 mol % Pd) and **3** (not according to the invention; 3.0 mg, 0.010 mmol, 0.10 mol %). The tube was capped with the Teflon screwcap, evacuated and backfilled with argon. The screwcap was replaced with a rubber septum, and *o*-anisidine (1.35 mL, 12.0 mmol) was added via syringe, followed by 2-chloro-*p*-xylene (1.34 mL, 10.0 mmol) and toluene (5 mL). The tube was purged with argon for three minutes, then the septum was replaced with a Teflon screwcap; the tube was sealed and the contents were heated to 80 ˚C with stirring. Analysis by gas chromatography after 14 h indicated complete

consumption of the aryl chloride. The reaction mixture was cooled to room temperature, diluted with diethyl ether (50 mL) and washed with a 1 M aqueous solution of phosphoric acid (50 mL), followed by water (50 mL). The resulting solution was dried over anhydrous potassium carbonate, filtered and concentrated *in vacuo.* The residue was purified by recrystallization from methanol, affording the title compound as white crystals, 2.20 g (97 %). See above for NMR data.

***Example 65***

Catalytic amination of functionalized aryl halides

**General procedure.** An oven-dried resealable Schlenk flask was evacuated and backfilled with argon. The flask was charged with $Pd(OAc)_2$ (2.2 mg, 0.01 mmol), **3** (not according to the invention; 4.5 mg, 0.015 mmol); $K_3PO_4$ (297 mg, 1.4 mmol). The flask was evacuated and backfilled with argon and capped with a rubber septum. DME (2 mL), the aryl halide (1.0 mmol), and the amine (1.2 mmol) were added through the septum, the septum was removed, and the flask was sealed with a teflon screwcap. The mixture was heated to 80 ˚C with stirring until the starting aryl chloride had been completely consumed as judged by GC analysis. The mixture was cooled to room temperature, diluted with 1/1 ether/ethyl acetate (40 mL), filtered through celite, and concentrated *in vacuo.* The crude material was purified by flash chromatography on silica gel.

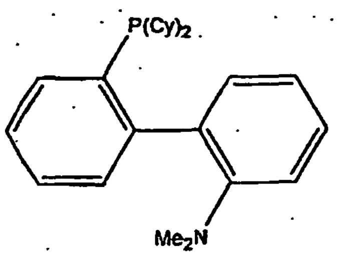

2

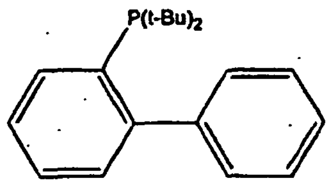

3

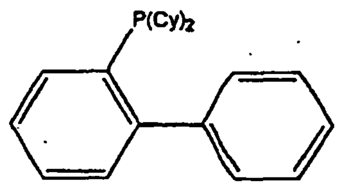

4

Compounds 2 to 4 are not ligands according to the invention.

***N*-(3- Cyanophenyl) benzylamine** (Figure 8, entry 1). The general procedure using 2 mol % $Pd(OAc)_2$, 4 mol % **4** (not according to the invention), and a reaction temperature of 100 ˚C gave 163 mg (78%) of a white solid, mp 69-70 ˚C: $^1$H-NMR (300 MHz, $CDCl_3$) T.40-7.14 (m, 6 ID, 6.97 (ddd, 1 H, 7.5, 1.4, 1.0 Hz), 6.83-6.78 (m, 2 H), 4.37 (br s, 1 H), 4.33 (s, 2 H); $^{13}$C-NMR (75 MHz, $CDCl_3$) 148.4, 138.3, 129.9, 128.9, 127.6, 127.4, 120.8, 119.6, 117.3, 115.1, 112.9, 47.8; FT-IR (neat) 3386, 2229, 1600, 776, 691 $cm^{-1}$. Anal. Calcd for $C_{14}H_{12}N_2$: C, 80.74; H, 5.81. Found: C, 80.89; H, 5.66.

***N*-(3-Cyanophenyl)pyrrolidine** (Figure 8, entry 2). The general procedure using ligand **4** (not according to the invention) gave 144mg (84%) of a pale yellow solid, mp 85-86 ˚C: $^1$H NMR (300 MHz, $CDCl_3$) 7.22 (ddd, 1 H, *J* = 7.5, 7.5, 2.1 Hz), 6.84 (d, 1 H, *J* = 7.5 Hz), 6.67-6.64 (m, 2 H), 3.29-3.24 (m, 4 H), 2.05-2.01 (m, 4 H); $^{13}$C NMR (75 MHz, $CDCl_3$) 147.7, 129.8, 120.0, 118.6, 115.8, 114.3, 112.7, 47.7, 25.6; FT-IR (neat, $cm^{-1}$ 2225, 1596, 1380, 791, 687.

***N*-(Diphenylmethylene)-4-nitroaniline** (Figure 8, entry 3). The general procedure using $Pd_2(dba)_3$, ligand **4** (not according to the invention), and a reaction tempeature of 100 ˚C (the product was purified by recrystallization from toluene/ethanol rather than chromatography) gave 249 mg (82%) of a pale yellow solid: mp 157-159 ˚C (lit. 156 ˚C); $^1$H NMR (300 MHz, $CDCl_3$) δ 8.05 (d, *J* = 8.8 Hz, 2H), 7.77 (broad s, 2H), 7.44 (broad s, 2H), 7.32 (broad s, 4H), 7.12 (broad s, 2H), 6.81 (d, *J* = 8.8 Hz, 2H); $^{13}$C NMR (75 MHz, $CDCl_3$) δ 169.8, 157.7, 143.5, 138.6, 135.4, 131.7, 129.8, 129.4, 128.5, 124.8, 121.1; IR (neat, $cm^{-1}$) 3064, 2927, 2844, 1586, 1511, 1441, 1339, 1318, 1293, 1231, 1110, 959, 849, 785, 756, 706, 693, 666. Anal. Calcd for $C_{19}H_{14}N_2O_2$: C, 75.48; H, 4.67. Found: C, 75.33; H, 4.65.

**4-Methoxy-4'-nitrodiphenylamine** (Figure 8, entry 4). The general procedure using $Pd_2(dba)_3$ and a reaction temperature of 100 ˚C (the product was purified by recrystallization from toluene/ethanol rather than chromatography) gave 222 mg (91%) of an orange solid: mp 152-152.5 ˚C (lit.[X] 151 ˚C); $^1$H NMR (300 MHz, $CDCl_3$) δ 8.09 (d, *J* = 9.2 Hz, 2H), 7.17 (d, *J* = 8.9 Hz, 2H), 6.95 (d, *J* = 8.9 Hz, 2H), 6.77 (d, *J* = 9.2 Hz, 2H), 6.15 (s, 1H), 3.85 (s, 3H); $^{13}$C NMR (75 MHz, $CDCl_3$) δ 157.6, 151.9, 1392, 132.2, 126.5, 125.7, 115.1, 112.8, 55.7; IR (neat, $cm^{-1}$) 3325, 3191, 3124, 3110, 3082, 3066, 3041, 3022, 2954, 2931, 2906, 2835, 1592, 1544, 1526, 1511, 1480, 1461, 1445, 1320, 1293, 1231, 1181, 1167, 1111, 1028, 1000, 830, 812, 801, 762, 749, 697, 675. Anal. Calcd for $C_{13}H_{12}N_2O_3$: C, 63.93; H, 4.95. Found: C, 63.73, H, 4.86.

**1-(3-Acetylphenyl)-4-methylpiperazine** (Figure 8, entry 5). The general procedure using $Pd_2(dba)_3$ and ligand **2** (not according to the invention) gave 163 mg (75%) of a pale orange oil: $^1$H NMR (300 MHz, $CDCl_3$) 7.52 (dd, 1 H, *J* = 2.2, 1.5 Hz), 7.41 (ddd, 1 H, *J* = 8.0,1.5,1.1 Hz), 7.33 (dd, 1 H, *J* = 8.0, 8.0 Hz), 7.12 (ddd, 1 H, *J* = 8.0, 2.2, 1.1 Hz), 3.27 (m, 4 H, *J* = 4.8 Hz), 2.58 (m, 4 H; *J* = 4.8 Hz), 2.58 (s, 3 H), 2.36 (s, 3 H); $^{13}$C NMR (75 MHz, $CDCl_3$) 198.6,151.5,

138.0, 129.3, 120.6, 120.0, 27.0,114.8, 55.2, 49.0, 46.3; IR (neat, $cm^{-1}$) 1681, 1441, 1296, 1260, 687.

***N*-(3-Acetylphenyl)aniline** (Figure 8, entry 6). The general procedure using $Pd_2(dba)_3$ and ligand **4** (not according to the invention) gave 174 mg (82%) of a yellow solid, mp 91.5-92.5: $^1H$ NMR (300 MHz, $CDCl_3$) 7.62 (dd, 1 H, *J* = 2.0, 1.4 Hz), 7.46 (ddd, 1 H, *J* = 7.5, 1.4, 1.4 Hz), 7.34-7.22 (m, 4 H), 7.11-7.05 (m, 2 H), 6.97 (tt, 1 H, *J* = 7.3, 1.1 Hz), 5.92 (br s, 1 H), 2.56 (s, 3 H); $^{13}C$ NMR (75 MHz, $CDCl_3$) 198.3, 143.9, 142.4, 138.4, 129.6, 121.9, 121.6, 120.8, 118.6, 116.6, 27.0; IR (neat, $cm^{-1}$) 3355,1667,1580,1324, 687.

***N*-(3-Acetylphenyl)aniline** (Figure 8, entry 6). The general procedure using $Pd_2(dba)_3$ and ligand **2** (not according to the invention) gave 179 mg (85%) of a yellow solid. See above for NMR data.

***N*-(4-Acetylphenyl)morpholine** (Figure 8, entry 7). The general procedure gave 187 mg (91%) of a pale yellow solid: mp 96-97 ˚C (lit. mp 97-98 ˚C). $^1H$ NMR (300 MHz, $CDCl_3$) 7.89 (d, 2H, *J*=9.1 Hz), 6.87 (d, 2H, *J*=9.1 Hz), 3.86 (t, 4H, *J*=4.8 Hz), 3.31 (t, 4H, *J*=5.1 Hz), 2.54 (s, 3H).

***N*-(4-Acetylphenyl)-*p*-toluidine** (Figure 8, entry 8). The general procedure using $Pd_2(dba)_3$ and ligand **4** (not according to the invention) gave 211 mg (94%) of a pink solid, mp 114-115 ˚C (lit mp 115 ˚C): $^1H$ NMR (300 MHz, $CDCl_3$) 7.83 (d, 2 H, *J* = 9.0 Hz), 7.14 (d, 2H, *J* = 8.5 Hz), 7.07 (m, 2 H, *J* = 8.5 Hz), 6.91 (m, 2 H, *J* = 9.0 Hz), 6.20 (br s, 1 H), 2.51 (s, 3 H), 2.33 (s, 3 H); $^{13}C$ NMR (75 MHz, $CDCl_3$) 196.5, 149.2, 137.9, 133.4, 130.8, 130.1, 128.5, 121.6, 113.9, 26.4, 21.1; FT-IR (neat, $cm^{-1}$) 3325,1648, 1563,1277, 809.

***N*-(4-Carbomethoxyphenyl)morpholine** (Figure 8, entry 9). The general procedure using ligand **4** (not according to the invention) gave 198 mg (90%) of a pale yellow solid, mp. 162-163. ˚C: (lit mp 152-154 ˚C) $^1H$ NMR (300 MHz, $CDCl_3$) δ 7.94 (d, 2H, *J* = 9.1 Hz), 6.86 (d, 2H, *J* = 9.1 Hz), 3.87 (s, 3H), 3.87-3.84 (m, 4H), 3.30-3.25 (m, 4H); $^{13}C$ NMR (75 MHz, $CDCl_3$) δ 167.0, 154.1, 131.2, 120.2, 113.4, 66.6, 51.7, 47.6; IR (neat, $cm^{-1}$) 2970, 1700, 1115, 770. Anal. Calcd for $C_{12}H_{15}NO_3$: C, 65.14; H, 6.83. Found: C, 65.18; H, 6.78.

***N*-(4-Cyanophenyl)hexylamine** (Figure 8, entry 10). The general procedure using $Pd_2(dba)_3$ and a reaction temperature of 110 ˚C gave 153 mg (76%) of a pale yellow solid, mp 36-37 ˚C: $^1H$-NMR (300 MHz, $CDCl_3$) 7.38 (m, 2 H, *J* = 8.3 Hz), 6.54 (m, 2 H, *J* = 8.3 Hz), 4.29 (br s, 1 H), 3.12 (t, 2H, *J* = 7.2 Hz), 1.62 (tt, 2 H, *J* = 7.2, 7.2 Hz), 1.44-1.28 (m, 6 H), 0.90 (t, 3 H, *J* = 6.7 Hz); $^{13}C$-NMR (75 MHz, $CDCl_3$) 151.6, 13 3.7, 120.8, 112.1, 98.1, 43.4, 31.7, 29.3, 26.9, 22.8, 14.2; FT-IR (neat, $cm^{-1}$) 3352, 2929, 2213, 1607,1532,1171.

***N*-(3-Carbomethoxyphenyl)morpholine** (Figure 8, entry 11). The general procedure using $Pd_2(dba)_3$ and ligand **4** (not according to the invention) and a reaction temperature of 100 ˚C gave 201 mg (91 %) of a pale yellow oil: $^1H$-NMR (300 MHz, $CDCl_3$) 7.58 (dd, 1 H, *J* = 2.6,1.5 Hz), 7.54 (ddd, 1 H, 7.9, 1.5, 0.9 Hz), 7.33 (dd, 1 H, *J* = 7.9, 7.9 Hz), 7.10 (ddd, 1 H, *J* = 7.9, 2.6, 0.9 Hz), 3.90 (s, 3 H), 3.87 (t-like, 4 H, *J* = 4.5 Hz), 3.20 (m, 4 H, *J* = 4.5 Hz); $^{13}C$-NMR (75 MHz, $CDCl_3$). 167.5, 151.4, 131.2, 129.3, 121.2, 120.2, 116.5, 67.0, 52.3, 49.3; IR (neat, $cm^{-1}$) 1717, 1262, 1117, 998, 756 .

***N*-(3-Carbomethoxyphenyl)-*N*-methylaniline** (Figure 8, entry 12). The general procedure using $Pd_2(dba)_3$ and ligand **4** (not according to the invention) and a reaction temperature of 100˚C gave 215 mg (89%) of a pale orange oil: $^1H$ NMR (300 MHz, $CDCl_3$) 7.64 (dd, 1 H, *J* = 2.7, 1.2 Hz), 7.56 (ddd, 1 H, *J* = 7.8, 2.7, 1.2 Hz), 7.35-7.25 (m, 3 H), 7.13 (ddd, 1 H, *J* = 7.2, 2.7, 1.2 Hz), 7.10-7.01 (m, 3 H), 3.88 (s, 3 H), 3.34 (s, 3 H); $^{13}C$ NMR (75 MHz, $CDCl_3$) 167.4, 149.3, 148.7, 1313, 129.6, 129.1, 123.4, 122.9, 122.3, 121.5, 119.4, 52.2, 40.4; IR (neat, $cm^{-1}$) 1723, 1592, 1289, 1262, 1111. Anal. Calcd for $C_{15}H_{15}NO_2$: C, 74.67; H, 6.27. Found: C, 74.65; H, 6.13.

***N*-(3-Carbomethoxyphenyl)-*N*-methylaniline** (Figure 8, entry 12). The general procedure using $Pd_2(dba)_3$ and ligand **2** (not according to the invention) and a reaction temperature of 100 ˚C gave 189 mg (78%) of a pale orange oil. See above for NMR data.

***Example 66***

A Highly Active Catalyst For the Room-Temoeerature Catalytic Amination and Suzuki Coupling of Aryl Chlorides

Mixtures of palladium acetate (1-2 mol % Pd) and *o*-(di-*tert*-butylphosphino)biphenyl (**4**; not according to the invention) catalyze the room-temperature cross-coupling of aryl chlorides with amines. This catalyst also functions for the room-temperature Suzuki coupling of aryl chlorides. Use of **4** or the related ligand *o*-(dicyclohexylphosphino)biphenyl (**3**; not according to the invention) allows for Suzuki coupling at very low catalyst loadings (0.000001 mol %-0.005 mol % Pd). The effectiveness of these ligands is believed to be due to a unique combination of steric and electronic properties which greatly accelerate the oxidative addition step while facilitating transmetallation (or Pd-N bond formation) and reductive elimination processes.

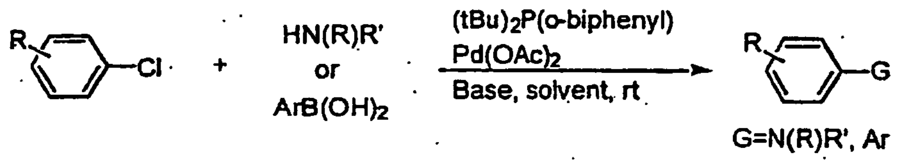


Palladium-catalyzed animation[1] and Suzuki coupling[2] reactions have found widespread use in many areas of organic synthesis. These methods permit the construction of $sp^2$-$sp^2$ C-C bonds or aryl C-N bonds which cannot be easily or efficiently formed using classical transformations. Most procedures commonly used for these processes employ triarylphosphine-based catalyst systems.[1,2] While these catalysts are readily available, they usually require elevated reaction temperatures (usually 50-100 ˚C) to function efficiently, and tend to be unreactive towards aryl chloride substrates.[3-5]

We recently reported that 2-dicyclohexylphosphino-2'-dimethylaminobiphenyl (**1**; not according to the invention) (Figure 1) was an excellent ligand for palladium-catalyzed cross-coupling reactions of aryl chlorides.[6] Although the Pd/**1** catalyst system was effective for the room-temperature Suzuki coupling of both electron-rich and electron-deficient aryl chloride substrates,[7] room-temperature catalytic aminations of aryl chlorides were inefficient; only the highly activated 4-chlorobenzonitrile was effectively transformed.

Subsequent studies demonstrated that the bulky phosphine **2** was a more effective ligand than 1 in palladium-catalyzed C-O bond forming reactions, presumably due to its ability to increase the rate of reductive elimination in these processes. [5g,8] Furthermore, experiments designed to determine whether the amino group on **2** was necessary for effective catalysis revealed that for some substrate combinations the desamino ligand **4** was as effective as **2** prompting us to examine its use in amination processes.[9]

Figure 1

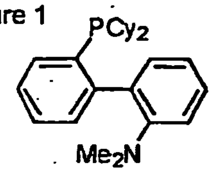 1 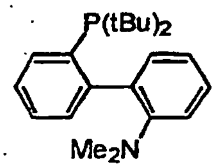 2 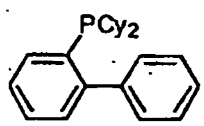 3 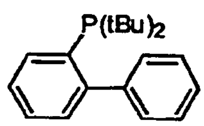 4

Compounds 1 to 4 are not ligands according to the invention.

As shown in Table 1, mixtures of palladium acetate and **4** effectively catalyzed the room-temperature amination of a variety of aryl chloride substrates, including substrates which are electron-rich and/or ortho-substituted. Secondary amines were found to be effective coupling partners, and primary amines were successfully arylated with ortho-substituted aryl halides. Reactions of primary amines with aryl halides which lack ortho substituents failed to proceed to completion under these conditions,[10] and substrates containing base-sensitive functional groups could not be transformed due to the inefficiency of the room-temperature reactions in the presence of bases weaker than NaO*t*Bu (e.g. $K_3PO_4$).[10] In a few cases the catalytic amination reactions of aryl chlorides were effected using low catalyst loadings with **3** or **4** as ligand (0.05 mol % Pd) at 100 ˚C (Table 1, entries 1-2). However, this low catalyst protocol is not yet general;[10] efforts to increase the: number of catalyst turnovers for a broader-set of substrates are currently underway.

Table 1: Room-Temperature Catalytic Amination of Aryl Chlorides[a]

| Entry | Halide | Amine | Product | %Pd | Rxn Time | Yield (%) |
|---|---|---|---|---|---|---|
| 1 | 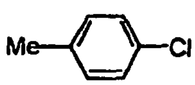 | 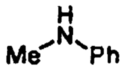 | 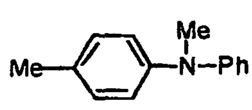 | 1.0<br>0.05 | 19 h<br>19 h | 98<br>95[b] |
| 2 | | 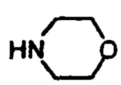 | 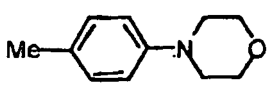 | 1.0<br>0.05/3 | 20 h<br>26 h | 94<br>89[b] |
| 3 | | HNBu$_2$ | 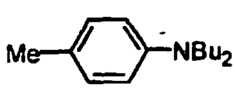 | 2.0 | 18 h | 81 |

(continued)

| Entry | Halide | Amine | Product | %Pd | Rxn Time | Yield (%) |
|---|---|---|---|---|---|---|
| 4 | 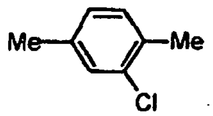 | 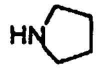 | 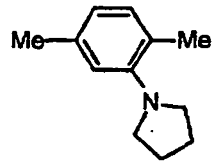 | 1.0 | 21 h | 98 |
| 5 | | $H_2NBn$ | 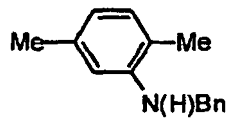 | 2.0 | 18 h | 99 |
| 6 | 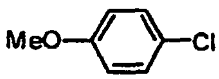 | 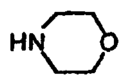 | 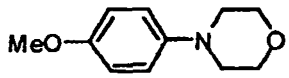 | 2.0 | 20 h | 90 |
| 7 | 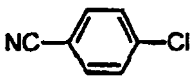 | 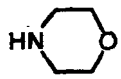 | 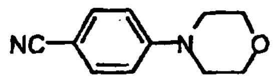 | 1.0 | 15 h | 86 |
| 8 | 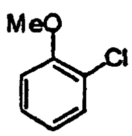 | $H_2NBn$ | 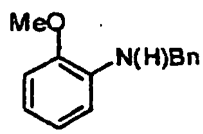 | 1.0 | 14 h | 99 |
| 9 | 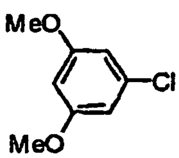 | 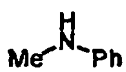 | 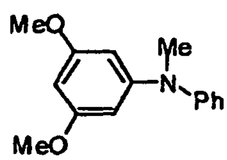 | 1.0 | 16 h | 97 |

[a] Reaction conditions: 1.0 equiv. aryl chloride, 1.2 equiv amine, 1.4 equiv NaO tBu, 1-2 mol % $Pd(OAc)_2$, 2-4 mol % 4, toluene (1 mL/mmol halide), rt. Reaction times have not been minimized. Yields represent isolated yields (average of two or more experiments) of compounds estimated to be 95 % pure as judged by $^1H$ NMR and GC analysis (known compounds) and combustion analysis (new compounds). [b] The reaction was run at 100 °C using $Pd_2(dba)_3$ in place of $Pd(OAc)_2$.

Catalysts based on ligand **4** were also effective for the room-temperature Suzuki coupling of aryl chlorides using 1.0-1.5 mol % Pd in the presence of a stoichiometric amount of KF. These conditions tolerate the presence of a wide variety of functional groups, and provide the desired products in excellent yields (Table 2).[11]

Table 2: Room-Temperature Suzuki Coupling of Aryl Chlorides[a]

| Entry | Halide | Boronic Acid | Product. | mol% Pd | Time (h) | Yield (%) |
|---|---|---|---|---|---|---|
| 1 | 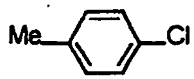 | 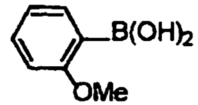 | 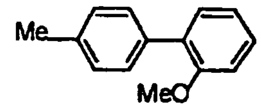 | 1 | 24 | 95 |
| 2 | 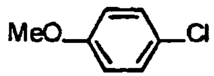 | 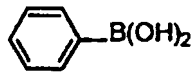 | 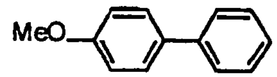 | 1.5 | 21 | 92 |
| 3 | 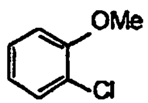 | 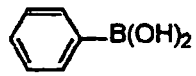 | 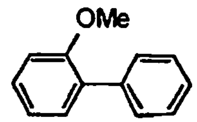 | 1 | 24 | 96 |
| 4 | 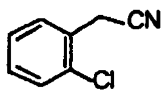 | 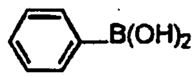 | 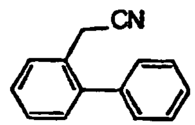 | 1 | 20 | 91 |
| 5 | 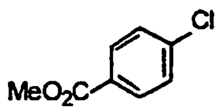 | 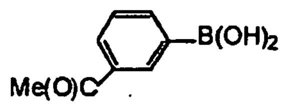 | 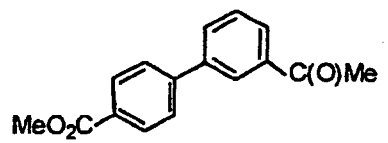 | 1 | 2 | 91 |

[a] Reaction conditions: 1.0 equiv aryl chloride, 1.5 equiv boronic acid, 3.0 equiv KF, cat $Pd(OAc)_2$, cat 4 (2L/Pd), THF (1 mL/mmo) aryl chloride), rt: reaction times have not been minimized.

Use of catalysts based on **3** or **4** at 100 °C allowed for effective Suzuki coupling at low catalyst loadings; higher turnover numbers were usually obtained with **3** (Table 3).[12] The coupling of 4-bromoacetophenone with phenylboronic acid (entry 3) was achieved at 0.000001 mol % Pd (100,000,000 turnovers),[13] although a control reaction conducted in the absence of phosphine ligands proceeded to completion with 0.001 mol % $Pd(OAc)_2$, suggesting that reactions of this substrate combination are particularly facile.[14] Aryl chlorides were effectively coupled at 0.02-0.05 mol % Pd, the lowest catalyst loadings reported thus far for the Suzuki coupling of aryl chlorides.[13a]

Table 3: Suzuki Coupling at Low Catalyst Loading[a]

| Entry | Halide | Boronic Acid | Product | mol% | Pd Ligand | Time (h) | Yield (%) |
|---|---|---|---|---|---|---|---|
| 1 | 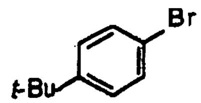 | 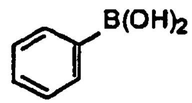 | 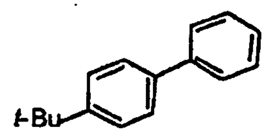 | 0.02 | 4 | 26 | 92 |
| | | | | 0.005 | 3 | 16 | 93 |
| 2 | 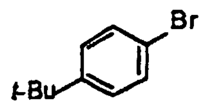 | 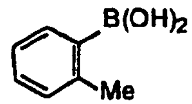 | 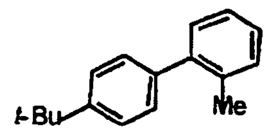 | 0.005 | 3 | 20 | 96 |
| 3 | 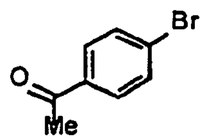 | 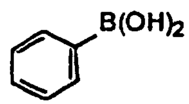 | 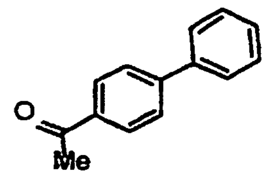 | 0.001 | 4 | 19 | 96[a] |
| | | | | 0.001 | - | 19 | 100[b] |
| | | | | 0.000001 | 4 | 24 | 91[c] |
| 4 | 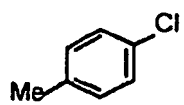 | 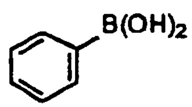 | 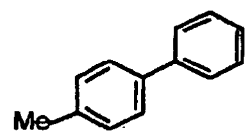 | 0.1 | 4 | 25 | 95 |
| | | | | 0.05 | 3 | 25 | 94[d] |
| 5 | 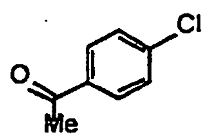 | 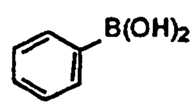 | 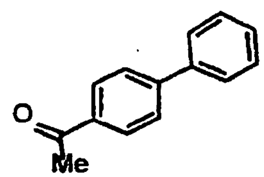 | 0.02 | 4 | 23 | 92 |

[a] Reaction Conditions: 1.0 equiv aryl halide; 1.5 equiv boronic acid; 2.0 equiv $K_3PO_4$; cat. $Pd(OAc)_2$, cat. Ligand (2L/Pd); toluene (3 mL/mmol halide); 100 C; reaction times have not been minimized; all reactions proceeded to completion unless otherwise noted. [b] GC yield. [c] Of two experiments, one proceeded to only 99% conversion. [d] The reaction proceeded to 99% conversion. [e] $Pd_2(dba)_3$ used in place of $Pd(OAc)_2$.

Although the reasons for the high activity of catalysts supported by **3** and **4** are not well understood at this time, we believe that several structural features of the ligands are of importance. The electron-rich phosphine facilitates oxidative addition of the aryl chloride[15] and binds tightly to the metal to prevent precipitation of the catalyst. The steric encumbrance of the ligands promote reductive elimination,[8] and examination of models reveals that the *o*-phenyl moiety may be oriented such that a favorable interaction between the aromatic $\pi$-system and the metal orbitals occurs.[16] This interaction may also orient the arene ring of the substrate perpendicular to the N-Pd bond placing it in the stereoelectronically optimum arrangement for reductive elimination to occur.[17] The combination of these effects serve to accelerate oxidative addition without inhibition of transmetallation or reductive elimination. Further mechanistic studies are currently underway.

In conclusion, we have developed a new, highly active catalyst system based on ligand **4** for the palladium-catalyzed amination and Suzuki coupling of aryl chlorides at room-temperature and at low catalyst loading. Although **4** is most effective for room-temperature reactions, **3** is frequently more effective for reactions which employ low levels of the palladium catalyst, and for Suzuki coupling reactions of very hindered substrates.[12] The mild reaction conditions employed for these transformations provide further evidence that the oxidative addition of aryl chlorides to complexes of $Pd^0$ can be induced to occur at low temperatures when catalysts which possess suitable steric and electronic properties are employed.

*References and Notes for Example 66*

[1] a) J. P. Wolfe, S. Wagaw, J. -F. Marcoux, S. L. Buchwald *Acc. Chem. Res.* **1998**, *31*, 805-818; b) J. F. Hartwig *Angew. Chem.* **1998**, *110,* 2154-2177; *Angew. Chem. Int. Ed. Engl.* **1998**, *37*, 2046-2067; c) B. H. Yang, S. L. Buchwald *J. Organomet. Chem*. **1999**, *576*, 125-146.

[2] A. Suzuki in *Metal-Catalyzed Cross-Coupling Reactions* (Eds.: F Diederich, P. J Stang), Wiley-VCH, Weinheim **1998**; Ch. 2.

[3] V. V. Grushin, H. Alper *Chem. Rev.* **1994***, 94*,1047-1062

[4] a) The Herrmann/BeHer palladacycle catalyst has been demonstrated to be effective for some C-C and C-N bond forming reactions of aryl chlorides at 135 ˚C. T. H. Riermeier, A. Zapf, M. Beller *Top. Catal.* **1997***, 4*, 301-309, and references cited therein; b) Herrmann has demonstrated the Suzuki coupling of 4-chloroacetophenone using palladium complexes bearing chelating, heterocyclic carbene ligands; see: W. A. Herrmann, C.-P. Reisinger, Spiegler, M. *J. Organomet. Chem.* **1998**, *557,* 93-96; c) Trudell and Nolan have recently reported the Suzuki coupling of aryl chlorides using bulky, heterocyclic carbene ligands; see: C. Zhang, J. Huang, M. L. Trudell, S. P. Nolan *J. Org. Chem.* In press.

[5] Recent work has led to the use of bulky, electron-rich phosphines as supporting ligands for palladium-catalyzed amination, diarylether formation, and Suzuki coupling reactions of aryl chloride substrates. These catalyst systems, however, still require elevated reaction temperatures, and Suzuki coupling reactions of electron-rich aryl chlorides are often ineffective. For catalytic amination reactions see [6] and: a) M. Nishiyama, T. Yamamoto, Y. Koie *Tetrahedron Lett.* **1998**, *39*, 617-620; b) M. Yamamoto, M. Nishiyama, Y. Koie *Tetrahedron Lett.* **1998**, *39,* 2367-2370; c) N. P. Reddy, M. Tanaka *Tetrahedron Lett.* **1998**, *39*, 617-620; d) B. C. Hamann, J. F. Hartwig *J. Am. Chem. Soc.* **1998**, *120*, 7369-7370; e) X. H. Bei, A. S. Guram, H. W. Turner, W. H. Weinberg *Tetrahedron Lett.* **1999**, *40,* 1237-1240; For diarylether formation see: f) G. Mann, C. Incarvito, A. C. Rheingold, J. F. Hartwig *J. Am. Chem. Soc.* **1999**, *121*, 3224-3225; g) A. Aranyos, D. W. Old, A. Kiyomori, J. P. Wolfe, J. P. Sadighi, S. L. Buchwald *J. Am. Chem. Soc.* **1999**, *121,* 4369-4378; For Suzuki coupling see [6] and: h) W. Shen *Tetrahedron Lett.* **1997**, *38*, 5575-5578; i) N. A. Bumagin, V. V. Bykov. *Tetrahedron* **1997**, *53*, 14437-14450; j) M. B. Mitchell, P. J. Wallbank *Tetrahedron Lett.* **1991,** *32*, 2273-2276; k) F. Firooznia, C. Gude, K. Chan, Y. Satoh *Tetrahedron Lett.* **1998***, 39,* 3985-3988; 1) B. Cornils *Orgn. Proc. Res. Dev.* **1998,** *2,* 121-127; m) Fu has recently reported the Suzuki coupling of electron rich aryl chlorides using palladium complexes with $P(t\mathrm{Bu})_3$ as the supporting ligand. A. F. Littke, G. C. Fu *Angew. Chem.* **1998,** *110,* 3586-3587; *Angew. Chem. Int. Ed. Engl.* **1998,** *37,* 3387-3388; n) X. Bei, T. Crevier, A. S. Guram, B. Jandeleit, T. S. Powers, H. W. Turner, T. Uno, H. Weinberg *Tetrahedron Lett.* **1999,** *40,* 3855-3858.

[6] D. W. Old, J. P. Wolfe, S. L. Buchwald *J. Am. Chem. Soc.* **1998,** *120***,** 9722-9723.

[7] The few previously reported methods for room-temperature Suzuki coupling frequently require toxic additives,

and do not function for aryl chloride substrates. See: J. C. Anderson, H. Namli, C. A. Roberts *Tetrahedron* ***1997,*** *53,* 15123-15134, and references therein.

[8] Bulky ligands have been shown to accelerate other palladium-catalyzed cross-coupling reactions. See: a) V. Farina in *Comprehensive Organometallic Chemistry,* 2nd Ed, Pergamon, Oxford, **1995**, *Vol. 12,* pp 161-240; b) J. F. Hartwig, S. Richards, D. Baranano, F. Paul *J. Am. Chem. Soc.* **1996**, *118*, 3626-3633.

[9] Ligands **3** and **4** are air-stable, crystalline solids which are prepared in one step. These ligands are now commercially available from Strem Chemical Co.

[10] While the scope of the room-temperature amination of aryl chlorides, and aminations at low catalyst loadings is somewhat limited, a much broader range of substrates are efficiently coupled at higher temperatures (80-100 ˚C) using 0.5-1.0 mol % Pd. Reactions of functionalized substrates may be carried out using $K_3PO_4$ in place of NaO*t*Bu at 80-100 ˚C. These results will be reported in full papers.

[11] The scope and limitations of Suzuki couplings which employ **3** or **4** will be the subject of a full paper.

[12] All reactions proceed to completion unless otherwise noted.

[13] Beller and Herrmann, and Bedford have reported catalysts which provide 74,000 and 1,000,000 turnovers for this reaction, respectively. See: a) M. Beller, H. Fischer, W. A. Herrmann, K. Öfele, C. Brossmer *Angew. Chem.* **1995**, *107,* 1992-1993; *Angew. Chem. Int. Ed. Engl.* **1995**, *34*, 1848-1849; b) D. A. Albisson, R. B. Bedford, S. E. Lawrence, P. N. Scully *Chem. Commun* **1998**, 2095-2096.

[14] Under these conditions, Suzuki coupling reactions of other substrates give little or no products in the absence of phosphine ligands.

[15] a) G. O. Spessard, G. L. Meissler *Organometallic Chemistry* Prentice-Hall: Upper Saddle River, New Jersey , **1996;** pp 171-175. b) M. Portnoy, D. Milstein *Organometallics* **1993**, *12*, 1665-1673.

[16] Metal-π interactions have been observed in other palladium complexes. See: a) H. Ossor, M. Pfeffer, J. T. B. H. Jastrzebski, C. H. Stam *Inorg. Chem.* **1987**, *26*, 1169-1171; b) L. R. Falvello, J. Fornies, R. Navarro, V. Sicilia, M. Tomas *Angew. Chem.* **1990,** *102,* 952-954; *Angew. Chem. Int. Ed. Engl.* **1990**, *29*, 891-893; c) C. -S. Li, C. -H. Cheng, F. -L. Liao, S. -L. Wang *J. Chem. Soc.. Chem. Commun.* **1991**, 710-711; d) S. Kannan, A. J. James, P. R. Sharp *J. Am. Chem. Soc.* **1998**, *120,* 215-216.

[17] Biaryl-forming reductive elimination from $Pt^{II}$ has been postulated to occur via a transition state in which both arenes are perpendicular to the coordination plane. See: P. S. Braterman, R J. Cross, G. B. Young *J. Chem. Soc. Dalton Trans 1* 1977, 1892-1897.

***Example 67***

*N*-(4-*t*-Butylphenyl)phenylalanine methyl ester

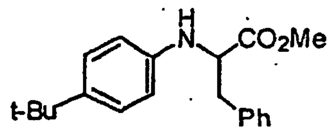

An oven-dried Schlenk tube was evacuated and backfilled with argon and charged with L-phenylalanine methylester hydrochloride (129.4 mg, 0.6 mmol, 99% ee), $Pd(OAc)_2$ (5.6 mg, 0.025 mmol, 5 mol %), 2-(isopropyl)-2'-(di-*t*-butylphosphino)biphenyl (not according to the invention; 13.1 mg, 0.0375 mmol) and $Cs_2CO_3$ (423.6 mg, 1.3 mmol). The tube was evacuated and backfilled with argon and toluene (1.0 mL) was added followed by 1-bromo-4-t-butylbenzene (0.0867 mL, 0.5 mmol), dodecane (85.8 mg, 0.504 mmol) and an additional portion of toluene (1.0 mL) through a rubber septum. The tube was sealed and heated to 40 ˚C with stirring for 20 hours at which time GC analysis showed 28%

conversion. The mixture was diluted with water (3.0 niL) and extracted with $Et_2O$ (2x4.0 mL). The combined organic layers were dried with $Na_2SO_4$, filtered, and concentrated in vacuo. The crude material was purified by flash chromatography on silica gel to afford 23.8 mg (15%) of the product as a white solid. HPLC analysis of the product showed the ee to be 83.3%.

***Example 68***

*N*-(4-Methylphenyl)phenylalanine methyl ester

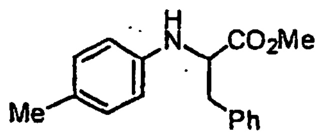

An oven-dried Schlenk tube was evacuated and backfilled with argon and charged with L-phenylalanine methylester hydrochloride (129.4 mg, 0.6 mmol, 99% ee), $Pd(OAc)_2$ (5.6 mg, 0.025 mmol, 5 mol %), 2-(isopropyl)-2'-(di-*t*-butylphosphino)biphenyl (not according to the invention; 13.1 mg, 0.0375 mmol) and $Cs_2CO_3$ (423.6 mg, 1.3 mmol). The tube was evacuated and backfilled with argon and toluene (1.0 mL) was added followed by p-chlorotoluene (0.0592 mL, 0.5 mmol), dodecane (83.0 mg, 0.487 mmol) and an additional portion of toluene (1.0 mL) through a rubber septum. The tube was sealed and heated to 40 ˚C with stirring for 20 hours at which time GC analysis showed 20% conversion. The mixture was diluted with water (3.0 mL) and extracted with $Et_2O$ (2x4.0 mL). The combined organic layers were dried with $Na_2SO_4$, filtered, and concentrated in vacuo. The crude material was purified by flash chromatography on silica gel to afford 12.7 mg (9%) of the product as a colorless oil. HPLC analysis of the product showed the ee to be 75.5%.

***Example 69***

*N*-(4-Methoxyphenyl)phenylalanine methyl ester

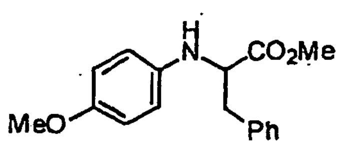

An oven-dried Schlenk tube was evacuated and backfilled with argon and charged with *DL*-phenylalanine methylester hydrochloride (129.4 mg, 0.6 mmol), $Pd(OAc)_2$ (5.6 mg, 0.025 mmol, 5 mol %), 2-(isopropyl)-2'-(di-*t*-butylphosphino)biphenyl (not according to the invention; 13.1 mg, 0.0375 mmol) and $Cs_2CO_3$ (423.6 mg, 1.3 mmol). The tube was evacuated and backfilled with argon and toluene (1.0 mL) was added followed by *p*-chloroanisole (0.0613 ml, 0.5 mmol), dodecane (82.9 mg, 0.487 mmol) and an additional portion of toluene (1.0 mL) through a rubber septum. The tube was sealed and heated to 80 ˚C with stirring for 20 hours at which time GC analysis showed 45% conversion. The mixture was diluted with water (3.0 mL) and extracted with $Et_2O$ (2 x4.0 mL). The combined organic layers were dried with $Na_2SO_4$, filtered, and concentrated in vacuo. The crude material was purified by flash chromatography on silica gel to afford 28.4 mg (20%) of the product as a colorless oil.

***Example 70***

*N*-(2,5-Dimethylphenyl)phenylalanine methyl ester

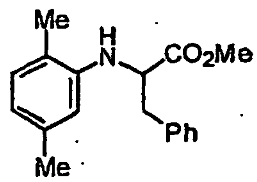

An oven-dried. Schlenk tube was evacuated and backfilled with argon and charged with L-phenylalanine methylester hydrochloride (129.4 mg, 0.6 mmol, 99% ee), $Pd(OAc)_2$(5.6 mg, 0.025 mmol, 5 mol %), 2-(isopropyl)-2'-(di-*t*-butylphosphino)biphenyl (not according to the invention; 13.1 mg, 0.0375 mmol) and $Cs_2CO_3$ (423.6 mg, 1.3 mmol). The tube was evacuated and backfilled with argon and toluene (1.0 mL) was added followed by 2-chloro-*p*-xylene(0.0670 mL, 0.5 mmol), dodecane (86.4 mg, 0.507 mmol) and an additional portion of toluene (1.0 mL) through a rubber septum. The tube was sealed and heated to 40 ˚C with stirring for 20 hours at which time GC analysis showed 9% conversion. The mixture was diluted with water (3.0 mL) and extracted with $Et_2O$ (2x4.0 mL). The combined organic layers were dried with $Na_2SO_4$, filtered, and concentrated in vacuo. The crude material was purified by flash chromatography on silica gel to afford 11.2 mg (8%) of the product as a colorless oil. HPLC analysis of the product showed the ee to be 69.9%.

***Example 71***

*N*-(3,5-Dimethoxyphenyl)phenylalanine methyl ester

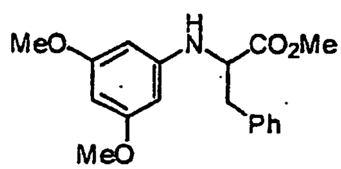

An oven-dried Schlenk tube was evacuated and backfilled with argon and charged with 5-chloro-1,3-dimethoxybenzne (86.3 mg, 0.5 mmol), *DL*-phenylalanine methylester hydrochloride (129.4 mg, 0.6 mmol), $Pd(OAc)_2$ (5.6 mg, 0.025 mmol, 5 mol %), 2-(isopropyl)-2'-(di-*t*-butylphosphino)biphenyl (not according to the invention; 13.1 mg, 0.0375 mmol) and $Cs_2CO_3$ (423.6 mg, 1.3 mmol). The tube was evacuated and backfilled with argon and toluene (1.0 mL) was added followed by dodecane (86.1 mg, 0.505 mmol) and an additional portion of toluene (1.0 mL) through a rubber septum. The tube was sealed and heated to 80 ˚C with stirring for 20 hours at which time GC analysis showed 40% conversion. The mixture was diluted with water (3.0 mL) and extracted with $Et_2O$ (2x4.0 mL). The combined organic layers were dried with $Na_2SO_4$, filtered, and concentrated in vacuo. The crude material was purified by flash chromatography on silica gel to afford 32.5 mg (21%) of the product as a colorless oil.

***Example 72***

*N*-(4-*t*-Butylphenyl)morpholine: Coupling of 4-*t*-butylbromobenzene with morpholine using CsF as the stoichiometric base

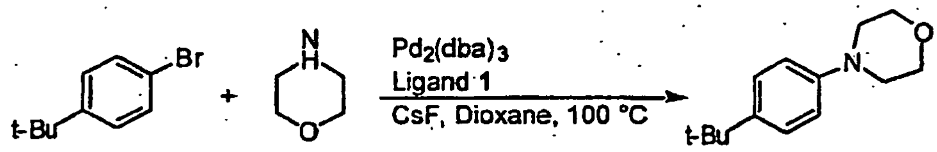


Ligand 1= 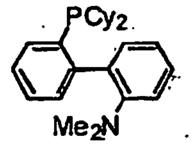

An oven-dried resealable Schlenk flask was purged with argon and charged with $Pd_2(dba)_3$ (4.6 mg, 0.005 mmol, 1.0 mol % Pd), ligand **1** (not according to the invention; 5.9 mg, 0.015 mmol, 1.5 mol %), and CsF (304 mg, 2.0 mmol). The flask was purged with argon and 4-*t*-butylbromobenzene (0.17 mL, 1.0 mmol), morpholine (0.10 mL, 1.2 mmol), and dioxane (2 mL) were added through a rubber septum. The flask was sealed with a teflon screwcap and was heated to 100 ˚C with stirring for 22h. GC analysis of the reaction mixture showed that the desired product had formed; the reaction had proceeded to ~76% conversion with a ratio of desired product to *t*-butylbenzene of 38:1.

## *Example 73*

*N*-(*3.5* dimethylphenyl)-*N*-(4-methoxyphenyl)-*N*-(2-pyridyl)amine

A resealable Schlenk tube was charged with $Pd_2(dba)_3$ (14.0 mg, 0.015 mmol, 3 mol %), 2-(di-cyclohexylphosphino)-2'-methyl-biphenyl (not according to the invention; 22.0 mg, 0.06 mmol, 6 mol %), sodium *tert*-butoxide (0.230 g, 2.4 mmol) and *p*-anisidine (0.123 g, 1 mmol) evacuated, backfilled with argon and fitted with a rubber septum. 5-Bromo-*m*-xylene (0.135 mL, 1.0 mmol), dodecane (0.227 mL, 1.0 mmol) and toluene (2 mL) were added via syringe and the flask was sealed with a teflon screwcap. The reaction was heated to 80 ˚C with stirring until the aryl bromide had been consumed (as judged by GC analysis). While maintaining the reaction at 80 ˚C, the teflon screwcap was replaced with a septum and purged with argon for 5 minutes. 2-Chloropyridine (0.194 mL, 2.0 mmol) was added via syringe while the reaction was purged with argon, then re-sealed. The reaction was further heated at 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes and ethyl acetate as eluent to yield 276 mg (91%) as a yellow solid.

## *Example 74*

*N*-(2-methoxyphenyl)-*N*-(3-methoxyphenyl)-*N*-(4-methoxyphenyl)amine

A resealable Schlenk tube was charged with $Pd_2(dba)_3$ (14.0 mg, 0.015 mmol, 3 mol %), 2-(di-*t*-butylphosphino) biphenyl (not according to the invention; 18.0 mg, 0.06 mmol, 6 mol %), sodium *tert*-butoxide (0.270 g, 2.8 mmol) and *p*-anisidine (0.123 g, 1 mmol) evacuated, backfilled with argon and fitted with a rubber septum. 3-Bromoanisole (0.126 mL, 1.0 mmol), 2-chloroanisole (0.133 mL, 1.05 mmol) dodecane (0.227 mL, 1.0 mmol) and toluene. (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture ofhexanes, toluene and ethyl acetate as eluent to yield 240 mg (72%) as a yellow oil.

## *Example 75*

*N*-(4-dimethylaminophenyl)-*N*-(4-methoxyphenyl)-*N*-(3-methylphenyl)amine

A resealable Schlenk tube was charged with $Pd_2(dba)_3$ (9.2 mg, 0.01 mmol, 2 mol %), 2-(di-*t*-butylphosphino) biphenyl (not according to the invention; 12.0 mg, 0.04 mmol, 4 mol %), sodium *tert*-butoxide (0.202 g, 2.1 mmol) and 4-bromo-*N,N*-dimethylaniline (0.200 g, 1 mmol) evacuated, backfilled with argon and fitted with a rubber septum. *m*-Toluidine (0.107 mL, 1.0 mmol), 4-chloroanisole (0.129 mL, 1.05 mmol) dodecane (0.227 mL, 1.0 mmol) and toluene (4 mL) were added via syringe and the flask was sealed with a teflon screwcap. The reaction was heated to 80 ˚C with stirring

until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 315 mg (95%) as a yellow oil.

***Example 76***

*N*-(2,4-dimethylphenyl)-*N*-(4-methoxyphenyl)-*N*-(3-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %) and sodium *tert*-butoxide (0.202 g, 2.1 mmol) evacuated, backfilled with argon and fitted with a rubber septum. 2,4-Dimethylaniline (0.124 mL, 1.0 mmol), 4-bromoanisole (0.118 mL, 1.0 mmol), 3-chlorotoluene (0.133 mL, 1.05 mmol), dodecane (0.227 mL, 1.0 mmol) and toluene (4 mL) were added via syringe and the flask was sealed with a teflon screwcap. The reaction was heated to 60 ˚C with stirring until the aryl bromide had been consumed (based on previous reactions), then heated to 80 ˚C until the diphenylamine had been consumed (as judged by GC analysis). The reaction mixture was then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 269 mg (85%) as a colorless oil.

***Example 77***

*N*-(2,4-dimethylphenyl)-*N*-(4-methoxyphenyl)-*N*-(3-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %) and sodium *tert*-butoxide (0.270 g, 2.8 mmol) evacuated, backfilled with argon and fitted with a rubber septum. 2,4-Dimethylaniline (0.124 mL, 1.0 mmol), 4-bromoanisole (0.118, mL, 1.0 mmol), 3-chlorotoluene (0.133 mL, 1.05 mmol), dodecane (0.227 mL, 1.0 mmol) and toluene (4 mL) were added via syringe and the flask was sealed with a teflon screwcap. The reaction was heated to 60 ˚C with stirring until the aryl bromide had been consumed (based on previous reactions), then heated to 80 ˚C until the diphenylamine had been consumed (as judged by GC analysis). The reaction mixture was then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 288 mg (90%) as a colorless oil.

***Example 78***

*N*-(2,5-dimethylphenyl)-*N*-(3-methoxyphenyl)-*N*-(4-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %), sodium *tert*-butoxide (0.202 g, 2.1 mmol) and *p*-toluidine (0.107 g, 1 mmol) evacuated, backfilled with argon and fitted with a rubber septum. 2-Bromo-*p*-xylene (0.138 mL, 1.0 mmol), 3-chloroanisole (0.128 mL, 1.05 mmol) dodecane (0.227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 295 mg (92%) as a yellow oil.

***Example 79***

*N*-(2,5-dimethylphenyl)-*N*-(3-methoxyphenyl)-*N*-(4-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %), sodium *tert*-butoxide (0.270 g, 2.8 mmol) and *p*-toluidine (0.107 g, 1 mmol) evacuated, backfilled with argon and fitted with a rubber septum. 2-Bromo-*p*-xylene (0.138 mL, 1.0 mmol), 3-chloroanisole (0.128 mL, 1.05 mmol) dodecane (0.227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl

ether (5 mL), washed with brine, dried over $MgSO_4$ and concentrated. The product was purified by flash chromatography on silica gel using a mixture of hexanes, toluene and ethyl acetate as eluent to yield 280 mg (87%) as a yellow oil.

***Example 80***

*N,N'*-(4-*tert*-butylphenyl)-*N, N'*-(4-methylphenyl)-1,3-phenylenediamine

A resealable Schlenk tube was charged with $Pd_2(dba)_3$ (14 mg, 0.015 mmol, 3-mol %), 2-(di-*t*-butylphosphino) biphenyl (not according to the invention; 18.0 mg, 0.06 mmol, 6 mol %), sodium, *tert*-butoxide(0.404 g, 4.2 mmol) and 1,3-phenylenediamine (0.108 g, 1 mmol) evacuated, backfilled with argon and fitted with a rubber septum. 4-Bromo-*tert*-butylbenzene (0.347 mL, 2.0. mmol), 3-chlorotoluene (0.236 mL, 2.1 mmol) and toluene (4 mL) were added via syringe and the flask was sealed with a teflon screwcap. The reaction was heated to 80 ˚C with stirring until the diphenylamine had been consumed (as judged by TLC), then cooled to room temperature, taken up in diethyl ether (10 mL), washed with brine, dried over $MgSO_4$ and concentrated. The product was purified by flash chromatography on silica gel using a mixture of hexanes and toluene as eluent to yield 518 mg (94%) as a white solid:

***Example 81***

*N*-(4-*n*-butylphenyl)-*N*-(4-methoxyphenyl)-*N*-(4-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %), sodium *tert*-butoxide (0.202 g, 2.1 mmol) and p-toluidine (0.107 g, 1 mmol) evacuated, backfilled with argon and fitted with a rubber septum. 4-Bromo-anisole (0.118 mL, 1.0 mmol), 1-*n*-butyl-4-chlorobenzene (0.177 mL, 1.05 mmol) dodecane (0.227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes and ethyl acetate as eluent to yield 297 mg (89%) as a yellow oil.

***Example 82***

*N*-(2,5-dimethylphenyl)-*N*-(3,5-dimethylphenyl)-*N*-(4-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %), sodium *tert*-butoxide (0.202 g, 2.1 mmol) and *p*-toluidine (0.107 g, 1 mmol) evacuated, backfilled with argon and fitted with a rubber septum. 5-Bromo-*m*-xylene (0.135 mL, 1.0 mmol), 2-chloro-*p*-xylene (0.141 mL, 1.05 mmol) dodecane (0-227 mL, 1.0 mmol) and toluene. (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 252 mg (80%) as a yellow oil.

***Example 83***

*N*-(2,5-dimethylphenyl)-*N*-(3-methoxyphenyl)-*N*-(4-methylphenyl)amine

A resealable Schlenk tube was charged with $Pd_2(dba)_3$ (4.6 mg, 0.005 mmol, 1 mol %) 2-(di-*t*-butylphosphino) biphenyl (not according to the invention; 6.0 mg, 0.02 mmol, 2 mol %), sodium *tert*-butoxide (0.270 g, 2.8 mmol) and *p*-toluidine (0.107 g, 1 mmol) evacuated, backfilled with argon and fitted with a rubber septum. 3-Bromoanisole (0.126 mL, 1.0 mmol), 2-chloro-*p*-xylene (0.141 mL, 1.05 mmol), dodecane (0227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture ofhexanes, toluene and ethyl acetate as eluent to yield 288 mg (91%) as a yellow oil.

***Example 84***

*N*-(2,4-dimethylphenyl)-*N,N*-(3-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %) and sodium *tert*-butoxide (0.270 g, 2.8 mmol) evacuated, backfilled with argon and fitted with a rubber septum. 2,4-dimethylaniline (0.123 mL, 1.0. mmol), 3-chlorotoluene (0.248 mL, 2.1 mmol), dodecane (0.227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 270 mg (87%) as a yellow solid.

***Example 85***

*N*-(3,5-dimethylphenyl)-*N*-*N*-(4-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol; 2 mol %), sodium *tert*-butoxide (0.270 g, 2.8 mmol) and *p*-toluidine (0.107 g, 1 mmol) evacuated, backfilled with argon and fitted with a rubber septum- 5-bromo-*m*-xylene (0.135 mL, 1.0 mmol), 4-chloroanisole (0.124 mL, 1.05 mmol), dodecane (0.227 mL, 1:0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes and ethyl acetate as eluent to yield 268 mg (89%) as a pale green oil.

***Example 86***

*N*-(4-methylphenyl)-*N,N*-(3-methylphenyl)amine

A resealable Schlenk tube was charged with $Pd_2(dba)_3$ (4.6 mg, 0.005 mmol, 1 mol %), 2-(di-*t*-butylphosphino) biphenyl (not according to the invention; 6.0 mg, 0.02 mmol, 2 mol %), *p*-toluidine (0.107 g, 1.0 mmol) and sodium *tert*-butoxide (0.270 g, 2.8 mmol) evacuated, backfilled with argon and fitted with a rubber septum. 3-Chlorotoluene (0.248 mL, 2.1 mmol), dodecane (0.227 mL, 1.0 mmol) and toluene (4 mL) were added via syringe and the flask was sealed with a teflon screwcap. The reaction was heated to 80 ˚C with stirring until the diphenylamine had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes, toluene and ethyl acetate as eluent to yield 197 mg (68%) as a white solid.

***Example 87***

*N*-(2-pyridyl)-*N,N*-diphenylamine

A resealable Schlenk tube was charged with $Pd_2(dba)_3$ (4.6 mg, 0.005 mmol, 1 mol %), 2-(di-cyclohexylphosphino)-2'-methyl-biphenyl (not according to the invention; 7.0 mg, 0.02 mmol, 2 mol %), sodium *tert*-butoxide (0.135 g, 1.4 mmol) and diphenylamine (0.169 g, .0 mmol) evacuated, backfilled with argon and fitted with a rubber septum. 2-Chloropyridine (0.196 mL, 2.0 mmol) and toluene (4 mL) were added via syringe and the flask was sealed with a teflon screwcap. The reaction was heated to 80 ˚C with stirring until the aryl bromide had been consumed (as judged by GC analysis), then cooled to room temperature, taken up in diethyl ether (5 mL), washed with brine, dried over $MgSO_4$ and concentrated. The product was purified by flash chromatography on silica gel using a mixture of hexanes and ethyl acetate as eluent to yield 245 mg (99%) as a yellow solid.

***Example 88***

*N*-(3-thiophenyl)-*N,N*-diphenylamine

A resealable Schlenk tube was charged with $Pd_2(dba)_3$ (9.2 mg, 0.01 mmol, 2 mol %), 2-(di-*t*-butylphosphino) biphenyl (not according to the invention; 12.0 mg, 0.04 mmol, 4 mol %), sodium *tert*-butoxide (0.135 g, 1.4 mmol) and

diphenylamine (0.169 g, 1.0 mmol) evacuated, backfilled with argon and fitted with a rubber septum. 3-Bromothiophene (0.188 mL, 2.0 mmol) and toluene (2 mL) were added via syringe and the flask was sealed with a teflon screwcap. The reaction was heated to 80 ˚C with stirring until the aryl bromide had been consumed (as judged by GC analysis). GC yield was determined to be 72%.

***Example 89***

*N*-(4-methoxyphenyl)-*N,N*-diphenylamine

A resealable Schlenk tube was charged with $Pd(OAc)_2$ (1.1 mg, 0.005 mmol, 0.05 mol %), 2-(di-*t*-butylphosphino)biphenyl (not according to the invention; 3.0 mg, 0.015 mmol, 0.15 mol %), sodium *tert*-butoxide (1.345 g, 14 mmol) and diphenylamine (1.692 g, 10 mmol) evacuated, backfilled with argon and fitted with a rubber septum. 4-Chloroanisole (1.255 mL, 10.25 mmol) and toluene (20 mL) were added via syringe and the flask was sealed with a teflon screwcap. The resulting mixture was stirred for one minute at room temperature. The reaction was heated to 80 ˚C with stirring until the aryl bromide had been consumed (as judged by GC analysis), then cooled to room temperature, taken up in diethyl ether (20 mL), washed with brine, dried over $MgSO_4$ and concentrated. The product was recrystallized from ethanol to yield 2.42 g (88%) as off-white crystals.

***Example 90***

*N*-(4-methylphenyl)indole

A test tube was charged with $Pd_2(dba)_3$ (2.3 mg, 0.0025 mmol, 1 mol %), 2-(di-*t*-butylphosphino)-2'-isopropylbiphenyl (not according to the invention; 2.6 mg, 0.0075 mmol, 1.5 mol %), indole (0.60 g, 0.51 mmol) and sodium *tert*-butoxide (0.067 g, 0.7 mmol) and fitted with a rubber septum with an argon tube 4-Chlorotoluene (0.059 mL, 0.50 mmol), and toluene (1 mL) were added via syringe and the reaction was purged with argon for 5 minutes. The reaction was heated to 100 ˚C with stirring until the aryl chloride had been consumed (as judged by GC analysis), then cooled to room temperature, taken up in diethyl ether (5 mL), filtered through celite and concentrated. The product was purified by flash chromatography on silica gel using a mixture of hexanes, toluene and ethyl acetate as eluent to yield 95 mg (92%) as a colorlress liquid.

***Example 91***

*N*-(4-*tert*-butylphenyl)indole

A test tube was charged with $Pd_2(dba)_3$ (2.3 mg, 0.0025 mmol, 1 mol %), 2-(di-*t* butylphosphino)-2'-isopropylbiphenyl (not according to the invention; 2.6 mg, 0.0075 mmol, 1.5 mol %), indole (0.60 g, 0.51 mmol) and sodium *tert*-butoxide (0.067 g, 0.7 mmol) and fitted with a rubber septum with an argon tube. 1-Bromo-4-*tert*-butylbenzene (0.087mL, 0.50 mmol), and toluene (1 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified, by flash chromatography on silica gel using a mixture of hexanes, toluene and ethyl acetate as eluent to yield 111 mg (90%) as a white solid.

***Example 92***

*N*-(4-methoxyphenyl)indole

A test tube was charged with $Pd_2(dba)_3$ (2.3 mg, 0.0025 mmol, 1 mol %), 2-(di-*t*-butylphosphino)-2'-isopropylbiphenyl (not according to the invention; 2.6 mg, 0.0075 mmol, 1.5 mol %), indole (0.60 g, 0.51 mmol) and sodium *tert*-butoxide (0.067 g, 0.7 mmol) and fitted with a rubber septum with an argon tube. 4-Bromoanisole (0.063 mL, 0.50 mmol), and toluene (1 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes, toluene and ethyl acetate as eluent to yield 93 mg (84%) as a white solid.

***Example 93***

*N*-(4-*N,N*-dimethylaminophenyl)indole

A test tube was charged with $Pd_2(dba)_3$ (2.3 mg, 0.0025 mmol, 1 mol %), 2-(di-*t*-butylphosphino)-2'-isopropyl-biphenyl (not according to the invention; 2.6 mg, 0.0075 mmol, 1.5 mol %), indole (0.60 g, 0.51 mmol), 4-bromo-*N,N*-dimethylaniline (0.100 g, 0.50 mmol) and sodium *tert*-butoxide (0.067 g, 0.7 mmol) and fitted with a rubber septum with an argon tube. Toluene (1 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes, toluene and ethyl acetate as eluent to yield 100 mg (90%) as a yellow liquid.

***Example 94***

*N*-(4-fluorophenyl)indole

A test tube was charged with $Pd_2(dba)_3$ (2.3 mg, 0.0025 mmol, 1 mol %), 2-(di-*t*-butylphosphino)-2'-isopropyl-biphenyl (not according to the invention; 2.6 mg, 0.0075 mmol, 1.5 mol %), indole (0.60 g, 0.51 mmol) and sodium *tert*-butoxide (0.067 g, 0.7 mmol) and fitted with a rubber septum with an argon tube 1-Bromo-4-fluorobenzene (0.055 mL, 0.50 mmol), and toluene (1 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes, toluene and ethyl acetate as eluent to yield 66 mg (63%) as a clear liquid.

***Example 95***

*N*-(3-pyridyl)indole

A test tube was charged with $Pd_2(dba)_3$ (6.9 mg, 0.0075 mmol, 3 mol %), 2-(di-*t*-butylphosphino)-2'-isopropyl-biphenyl (not according to the invention; 7.6 mg, 0.0225 mmol, 4.5 mol %), indole (0.60 g, 0.51 mmol) and sodium *tert*-butoxide (0.067 g, 0.7 mmol) and fitted with a rubber septum with an argon tube. 3-Bromopyridine (0.050 mL; 0.50 mmol), and toluene (1 mL) were added via syringe and the reaction was purged with argon for 5 minutes. The reaction was heated to 100 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes, toluene and ethyl acetate as eluent to yield 88 mg (91%) as a yellow liquid.

***Example 96***

Methyl 4-(*N*-indole)benzoate

A test tube was charged with $Pd_2(dba)_3$ (2.3 mg, 0.0025 mmol, 1 mol %), 2-(di-*t*-butylphosphino)-2'-isopropyl-bip,henyl (not according to the invention; 2.6 mg, 0.0075 mmol, 1.5 mol %), indole (0.60 g, 0.51 mmol), methyl 4-bromobenzoate (0.108 g, 0.50 mmol) and potassium phosphate (0.148 g, 0.7 mmol) and fitted with a rubber septum with an argon tube. Toluene (1 mL) were added via syringe and the reaction was purged with argon for 5 minutes. The reaction was heated to 80 ˚C with stirring until the aryl chloride had been consumed (as judged by GC analysis), then cooled to room temperature, taken up in diethyl ether (5 mL), filtered through celite and concentrated. The product was purified by flash chromatography on silica gel using a mixture ofhexanes, toluene and ethyl acetate as eluent to yield 103 mg (83%) as a yellow liquid.

***Example 97***

*N*-cyclopropyl-4-*tert*-butylaniline

A resealable Schlenk tube was charged with $Pd_2(dba)_3$ (4.6 mg, 0.005 mmol, 1 mol %), 2-(di-*t*-butylphosphino)-2'-isopropyl-biphenyl (not according to the invention; 7.0 mg, 0.015 mmol, 2.0 mol %) and sodium *tert*-butoxide (0.135

g, 1.4 mmol) evacuated, backfilled with argon and fitted with a rubber septum. 1-Bromo-4-*tert*-butylbenzene (0.173 mL, 1.0 mmol), cyclopropylamine (0.104 mL. 1.5 mmol), dodecane (0.227 mL, 1.0 mmol) and toluene (2 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes and ethyl acetate as eluent to yield 143 mg (76%) as a yellow oil.

***Example 98***

*N*-cyclopropyl-2,5-dimethylaniline

A resealable Schlenk tube was charged with $Pd_2(dba)_3$ (4.6 mg, 0.005 mmol, 1 mol %), 2-(di-*t*-butylphosphino)-2'-isopropyl-biphenyl (not according to the invention; 7.0 mg, 0.015 mmol, 2.0 mol %) and sodium *tert*-butoxide (0.135 g, 1.4 mmol) evacuated, backfilled with argon and fitted with a rubber septum. 2-Bromo-*p*-xylene (0.138 mL, 1.0 mmol), cyclopropylamine (0.104 mL, 1.5 mmol), dodecane (0.227 mL, 1.0 mmol) and toluene (2 mL) were added via syringe and the reaction was purged with argon for 5 minutes. The reaction was heated to 80 ˚C with stirring until the aryl bromide had been consumed (as judged by GC analysis), then cooled to room temperature, taken up in diethyl ether (5 mL), filtered through celite and concentrated. The product was purified by flash chromatography on silica gel using a mixture of hexanes and ethyl acetate as eluent to yield 139 mg (86%) as a yellow oil.

***Example 99***

A resealable Schlenk tube was charged with $Pd_2(dba)_3$ (92 mg, 0.01 mmol, 1.5 mol %), 2-(di-*t*-butylphosphino) biphenyl (not according to the invention; 12.0 mg, 0.04 mmol, 6 mol %), sodium *tert*-butoxide (0.317 g, 3.3 mmol), *p*-toluidine (0.107 g, 1 mmol), and *p*-anisidine (0.123 g, 1.0 mmol), evacuated, backfilled with argon and fitted with a rubber septum. Aniline (0.091 mL, 1.0 mmol), 4-bromoanisole (0.118 mL, 1.0 mmol), 1-bromo-4-*tert*-butylbenzene (0.173 mL, 1.0 mmol), 5-bromo-*m*-xylene (0.135 mL, 1.0 mmol), dodecane (0.227 mL, 1.0 mmol) and toluene (6 mL) were added via syringe and the flask was sealed with a teflon screwcap. The reaction was heated to 80 ˚C with stirring until the reagents had been consumed (as judged by GC analysis), then cooled to room temperature, taken up in diethyl ether (15 mL), washed with brine, dried over $MgSO_4$ and concentrated. The products were purified by flash chromatography on silica gel using a mixture of hexanes and ethyl acetate as eluent to yield 613 mg (93%) as a dark brown mixture of the 9 products. Characterized by GC/MS only. See Figure 14.

***Example 100***

Highly Active Palladium Catalysts For Suzuki Coupling Reactions

Mixtures of palladium acetate and *o*-(di-*t*-butylphosphino)biphenyl (not according to the invention; **4**) catalyze the room-temperature Suzuki coupling of aryl bromides and aryl chlorides with 0.5-1.0 mol % Pd. Use of *o*-(dicyclohexylphosphino)biphenyl (**2**) allows Suzuki couplings to be carried out at low catalyst loadings (0.000001-0.02 mol % Pd). The process tolerates a broad range of functional groups and substrate combinations including the use of sterically hindered substrates. This is the most active catalyst system in terms of reaction temperature, turnover number, and steric tolerance which has been reported to date.

The palladium-catalyzed cross coupling of aryl halides with organoboron reagents has become one of the most widely utilized methods for the formation of $sp^2$-$sp^2$ carbon-carbon bonds.[1] However, most protocols for Suzuki coupling do not effectively transform aryl chlorides, which are the cheapest and most readily available aryl halides.[1,2] The few existing methods for the palladium-catalyzed Suzuki coupling of aryl chloride substrates usually only function well for electron deficient substrates.[3a-f,4] Methods for the nickel-catalyzed Suzuki coupling of aryl chlorides have been reported which are more general than the protocols which use palladium-catalysts, but do not work well for very hindered substrates.[3g,h,5] Room-temperature Suzuki couplings of aryl halides are rare, usually require toxic additives such as thallium hydroxide, and do not work for aryl chloride substrates.[6]

We have recently reported that the aminophosphine ligand 2-dimethylamino-2'-dicyclohexylphosphinobiphenyl (**1**) promotes the palladium-catalyzed amination and room-temperature Suzuki coupling of aryl chlorides.[7] Although palladium catalysts supported by this ligand were sufficiently active to promote room-temperature Suzuki coupling reactions of aryl chlorides, this ligand required four steps to prepare from commercially available starting materials.

Related studies directed towards the development of improved catalysts for palladium-catalyzed C-O bond forming reactions led to the discovery that di-*t*-butylphosphino-aminobiphenyl ligand **3** was a highly efficient ligand for the coupling of aryl halides with phenols or NaO*t*Bu.[8] In some cases catalysts derived from the desamino derivative (*o*-

biphenyl)P(*t*-Bu)$_2$ (**4**) were found to be of comparable activity to those from **3**.

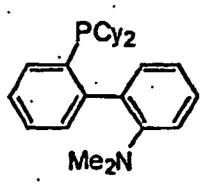

**1**

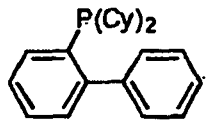

**2**

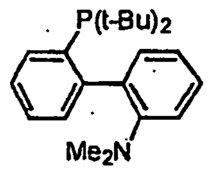

**3**

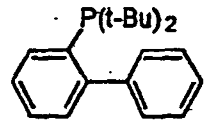

**4**

Compounds 1 to 4 are not ligands according to the invention.

Further studies involving ligands **2-4** revealed that catalysts supported by **4** were substantially more reactive than catalysts supported by **1** in Suzuki coupling reactions of aryl bromides and chlorides carried out at room temperature, and functioned efficiently for a wide variety of substrates with 0.5-1.0 mol % Pd.[9] Use of **2** as a ligand was successful for the Suzuki coupling of hindered substrates and provided for efficient reactions at very low catalyst loadings.[9] Of note is that **2** and **4** are prepared in high yield in a single step and are now commercially available.[10] Moreover, they are air stable, crystalline compounds which require no special handling. Herein we report detailed studies on the effectiveness of catalysts based on ligands **2** and **4** in Suzuki coupling reactions.

*Room-Temperature Suzuki Coupling of Aryl Halides*

The reaction of a wide variety of aryl halides and boronic acids was examined using conditions optimized for room-temperature Suzuki coupling; the results are shown in Tables 1 and 2. A catalyst comprised ofPd(OAc)$_2$/**4** efficiently promotes the room-temperature Suzuki coupling of both electron-rich and -poor aryl bromides (Table 1) and chlorides (Table 2). As is usually the case in Suzuki coupling reactions conducted under non-aqueous conditions,[1,11] a wide variety of functional groups are tolerated, and the catalyst is also active for heterocyclic halide substrates. Aryl halides with ortho substituents are usually efficiently coupled, although heating was occasionally required for reactions to proceed to completion. Reactions of ortho-substituted halides were often more efficient if **2** was used in place of **4** (see below). Cross-coupling of chloropyridine derivatives, or aryl halides containing acidic protons were slower at room temperature, and heating was required for reactions to proceed to completion in <24 h. The coupling of an aryl chloride with an alkyl 9-BBN derivative[12] (generated *in situ*) was effected at 65 °C (Table 2, entry 12).

Table 1: Suzuki Coupling of Aryl Bromides[a]

| Entry | Halide | Boronic Acid | Product | Ligand | Time (h) | Yield (%) |
|---|---|---|---|---|---|---|
| 1 | 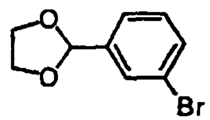 | 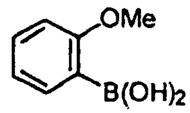 | 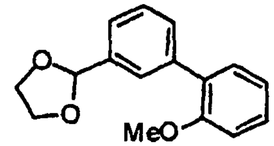 | 4<br>$(ArO)_3P$[b] | 2.5<br>2.5 | 86[c]<br>81[c] |
| 2 | 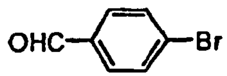 | 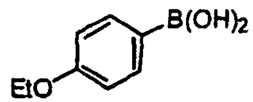 | 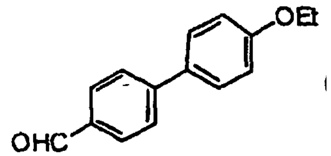 | 4<br>$(ArO)_3P$[b] | 2<br>2 | 90<br>82 |
| 3 | 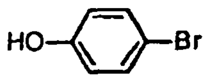 | 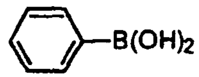 | 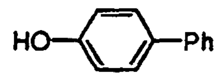 | 4 | 12 | 90[d] |
| 4 | 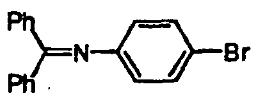 | 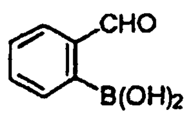 | 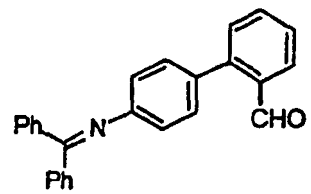 | 4 | 20 | 89 |
| 5 | 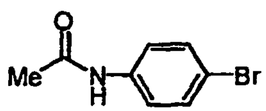 | 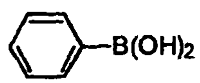 | 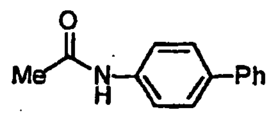 | 4 | 11 | 88[d] |
| 6 | 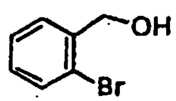 | 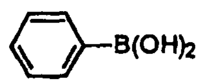 | 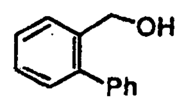 | 4 | 11 | 83[d] |
| 7 | 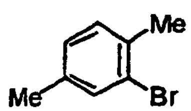 | 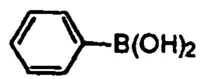 | 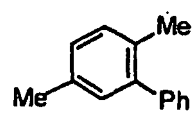 | 4<br>4<br>2 | 40<br>12<br>22 | 82<br>94[e]<br>93 |

(continued)

| Entry | Halide | Boronic Acid | Product | Ligand | Time (h) | Yield (%) |
|---|---|---|---|---|---|---|
| 8 | 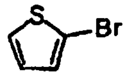 | 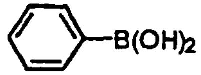 | 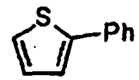 | 4 | 3 | 98 |
| 9 | 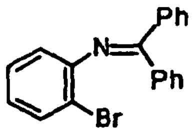 | 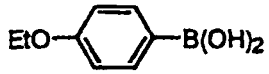 | 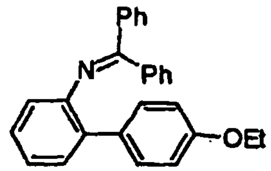 | 2 | 17 | 87[f,g] |

(a) Reaction conditions: 1.0 equiv aryl bromide, 1.5 equiv boronic acid, 3.0 equiv KF, 1 mol % $Pd(OAc)_2$, cat ligand (2UPd), THF (1 mL/mmol aryl bromide, rt; reaction times have not been minimized. Yields in tables 1-4 represent isolated yields (average of two or more experiments) of compounds estimated to be 95 % pure as judged by $^1H$ NMR and GC analysis (known compounds) and combustion analysis (new compounds); (b) $ArO_3P$= Tris(2,4-di-t-butylphenyl)phosphite; (c) The reaction was conducted with 0.5 mol % $Pd(OAc)_2$; (d) The reaction was conducted at 50 ˚C; (e) The reaction was conducted at 45 ˚C: (f) The reaction was conducted at 80˚C; (g) $K_3PO_4$ (2.0 equiv) used in place of KF.

During the course of our studies, we examined several different bases for the Suzuki coupling reactions. For room-temperature reactions, KF or CsF[10] were found to be the most effective of these. Other bases such as $K_3PO_4$, alkali metal carbonates, and sodium *t*-butoxide were substantially less effective at room-temperature, and alkali metal acetates failed to promote the reaction. Reactions conducted at room temperature were most efficient in THF or dioxane. Use of DME or $CH_3CN$ as solvent led to slower reactions, while reactions run in toluene or NMP gave very low conversions. Alcohols (MeOH, EtOH, *i*-PrOH) were poor solvents for the room-temperature Suzuki coupling, and their use led to reduction of the starting aryl halide.[13]

The correct combination of solvent and base was extremely important. While KF was ineffective in toluene, it was the most efficient promoter of room-temperature Suzuki coupling reactions in THF. Furthermore, while $K_3PO_4$ was less useful than KF for reactions in THF, reactions could be run at very low catalyst loadings using $K_3PO_4$ in toluene solvent at 100 ˚C (see below). Reactions conducted with low catalyst loadings were much less efficient in oxygenated solvents, such as THF, DME, or dioxane when $K_3PO_4$ was employed as the base. Use of biphasic solvent systems generally gave inferior results compared to reactions run without added water.

Table 2: Suzuki Coupling of Aryl Chlorides[a]

| Entry | Halide | Boronic Acid | Product | mol% Pd | Time (h) | Yield (%) |
|---|---|---|---|---|---|---|
| 1 | 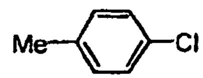 | 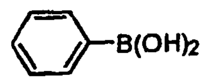 | 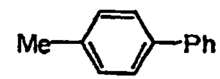 | 1<br>0.5 | 6<br>19 | 95<br>97 |
| 2 | 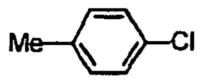 | 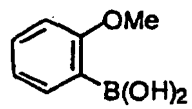 | 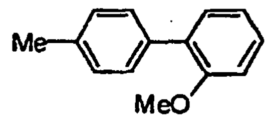 | 1 | 24 | 95 |
| 3 | 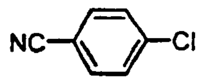 | 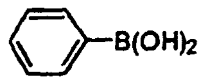 | 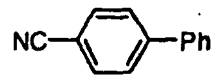 | 1 | 2 | 88 |
| 4 | 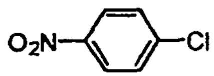 | 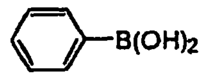 | 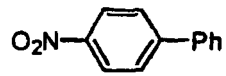 | 1<br>0.2 | 4<br>8 | 98<br>98 |
| 5 | 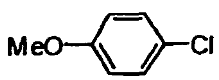 | 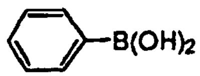 | 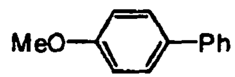 | 1<br>1.5 | 6<br>21 | 93[b]<br>92 |
| 6 | 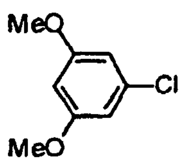 | 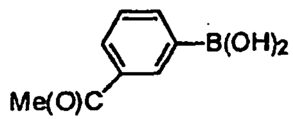 | 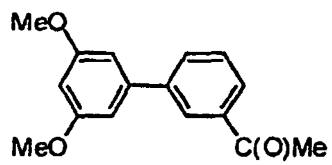 | 1 | 7 | 90 |
| 7 | 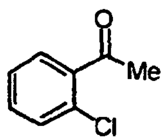 | 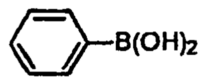 | 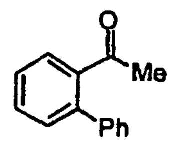 | 1 | 2.5 | 93 |
| 8 | 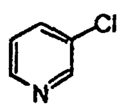 | 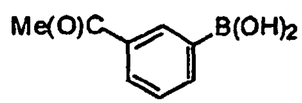 | 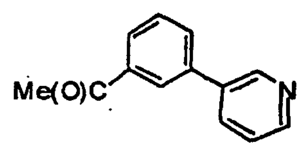 | 1 | 9 | 94[c] |

(continued)

| Entry | Halide | Boronic Acid | Product | mol% Pd | Time (h) | Yield (%) |
|---|---|---|---|---|---|---|
| 9 | 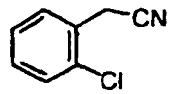 | 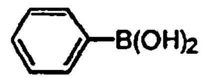 | 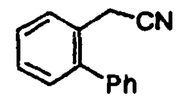 | 1 | 20 | 91 |
| 10 | 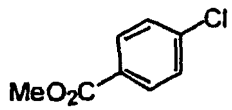 | 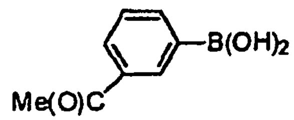 | 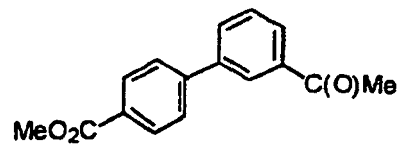 | 1 | 2 | 91 |
| 11 | 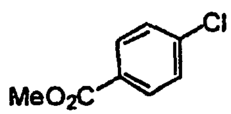 | 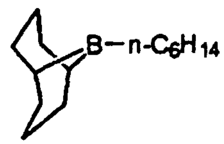 | 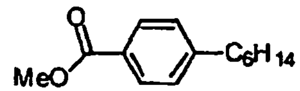 | 1 | 20 | 83[d] |
| 12 | 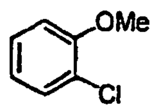 | 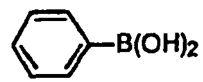 | 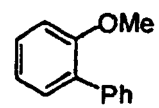 | 1 | 24 | 96 |

(a) Reaction conditions: 1.0 equiv aryl chloride, 1.5 equiv boronic acid, 3.0 equiv KF, cat $Pd(OAc)_2$. cat 4 (2L/Pd), THF (1 mL/mmol aryl chloride), rt; reaction times have not been minimized; (b) reaction conducted at 45 ˚C: (c) The reaction was conducted at 50 ˚C: (d) The reaction was conducted at 65 ˚C.

With respect to precatalyst, $Pd(OAc)_2$ was more effective than $Pd_2(dba)_3$; catalysts derived from the latter did not catalyze room-temperature couplings of aryl chlorides for the systems examined. The use of $Pd_2(dba)_3$ for reactions of aryl bromides at low catalyst loadings gave better results than $Pd(OAc)_2$ in THF at 65 °C, although $Pd(OAc)_2$ was a superior palladium source for reactions conducted in toluene at 100 °C.

Reactions of 5-chloro-1,3-dimethoxybenzene with phenylboronic acid at room-temperature proceeded more rapidly as the amount of boronic acid and KF added to the reaction mixture was increased; $21 \pm 1\%$ conversion was obtained after 1 h with 1.5 equiv boronic acid and 3.0 equiv KF while $32 \pm 2\%$ conversion was observed after the same period of time when 3.0 equiv boronic acid and 6.0 equiv KF was employed (after 4h $66 \pm 2\%$ and $96 \pm 3\%$ conversion were observed, respectively). This trend suggests that transmetallation may be the rate limiting step for this substrate combination. However; it is possible that the rate limiting step in the catalytic cycle may change when other substrates or reaction conditions are used.

A recent report described the use of a cyclometallated tris(2,4-di-*t*-butylphenyl)phosphite palladium complex as a highly active catalyst for the Suzuki coupling of aryl bromides.[14] The authors reported that the cyclometallated complex was sufficiently active to promote the room-temperature Suzuki coupling of 4-bromoacetophenone with phenylboronic acid, and speculated that the palladacycle was probably being cleaved *in situ* to a non-cyclometallated catalyst.[14] We examined the use of mixtures of palladium acetate and tris(2,4-di-*t*-butylphenyl)phosphite as a catalyst under our reaction conditions and found that this system gave results comparable to those obtained with **4**/$Pd(OAc)_2$ in room-temperature Suzuki couplings of aryl bromides (Table 1, entries 1-2), but was unreactive towards aryl chlorides. Even at 100 °C, the coupling of 4-chlorotoluene with phenylboronic acid catalyzed by 1 mol % palladium acetate and 2 mol % tris(2,4-di-*t*-butylphenyl)phosphite proceeded only to 5% conversion.

*Synthesis of Biaryls Containing More Than One Ortho Substituent*

Although examples of Suzuki coupling to form very hindered biphenyls from aryl iodides or bromides have been reported,[6b,15] reactions of this type are often problematic.[1,6b,15a] In some cases the use of certain bases (TlOH,[6b] $Ba(OH)_2$,[15a] or $K_3PO_4$[15a]), or solvent combinations (e.g. toluene/water/ethanol 3/3/1)[15e] have been reported to give improved results, although the generality of these protocols is not clear. To the best of our knowledge, only one example of the synthesis of biphenyls bearing three ortho substituents from aryl chloride substrates has been previously reported.[5]

Using our conditions, substrates with more than one ortho substituent were substantially less reactive than other aryl halides, and the **4**/$Pd(OAc)_2$ catalyst system was usually not very effective for reactions of these types of substrates. However, catalysts employing ligands **1, 2, 5,** or **6** functioned well for reactions of 2,6-disubstituted halides, 2,6-disubstituted boronic acids, and the coupling of *o*-substituted halides with *o*-substituted boronic acids. Ligands **1, 5** and **6** were equally effective for these reactions, while **2** provided catalysts which were slightly less efficient (Table 3, entries 7-8). The best results in reactions of hindered substrates were usually obtained with $K_3PO_4$ as the base in toluene solvent. While L/Pd ratios of 2/1 were usually employed, occasionally it was found that use of a 3-4/1 ratio was beneficial.

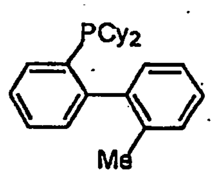

**5**

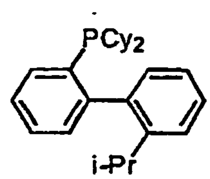

**6**

Compounds 5 and 6 are not ligands according to the invention.

While reactions which formed biaryls containing three ortho substituents were fairly efficient, reactions which formed tetra-ortho substituted biaryls were problematic and typically proceeded to <40% conversion.[16] Surprisingly, use of an increased quantity of catalyst did not give improved results.

Table 3: Suzuki Coupling of Sterically Hindered Substrates[a]

| Entry | Halide | Boronic Acid | Product | Ligand | Temp (˚C) | Time (h) | Yield (%) |
|---|---|---|---|---|---|---|---|
| 1 | 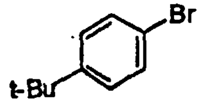 | 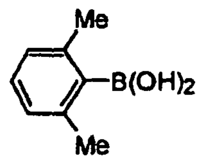 | 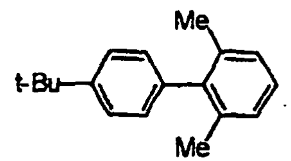 | 1 | 100 | 16 | 97 |
| 2 | 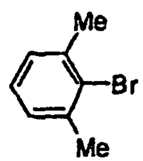 | 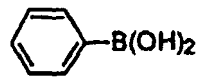 | 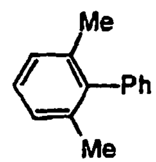 | 4<br>2 | 65<br>rt | 19<br>13 | 81[b,c]<br>92[b,c] |
| 3 | 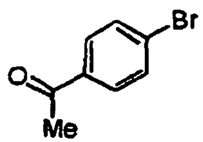 | 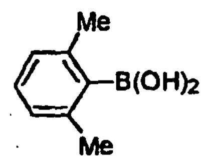 | 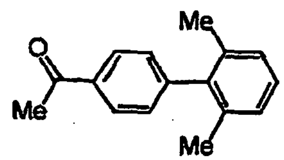 | 4 | 80 | 16 | 87[d] |
| 4 | 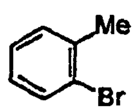 | 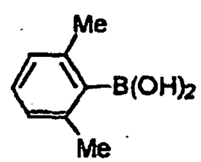 | 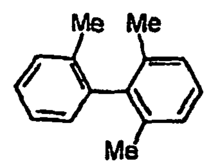 | 1 | 100 | 20 | 86 |
| 5 | 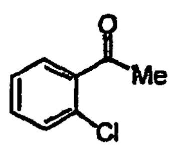 | 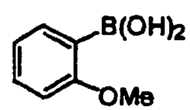 | 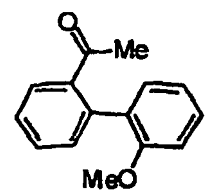 | 4 | 65 | 6 | 90 |
| 6 | 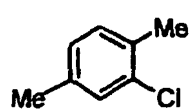 | 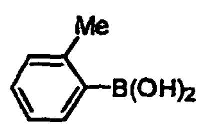 | 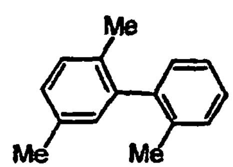 | 2 | 80 | 15 | 96 |

(continued)

| Entry | Halide | Boronic Acid | Product | Ligand | Temp (˚C) | Time (h) | Yield (%) |
|---|---|---|---|---|---|---|---|
| 7 | 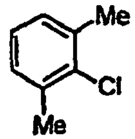 | 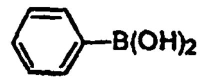 | 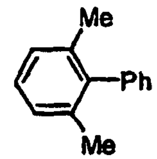 | 1 | 100 | 22 | 91 |
| | | | | 2 | 100 | 22 | 85[e] |
| 8 | 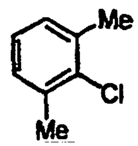 | 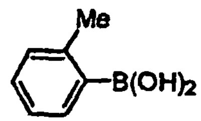 | 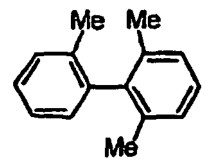 | 6 | 100 | 3 | 92 |
| | | | | 2 | 100 | 17 | 88[f] |

(a) Reaction conditions: 1.0 equiv aryl halide, 1.5 equiv boronic acid, 2.0 equiv $K_3PO_4$, 1 mol % $Pd(OAc)_2$, cat ligand (4L/Pd), toluene (3 mL/mmol halide); reaction times have not been minimized; (b) KF (3.0 equiv) used in place of $K_3PO_4$; (c) Ratio of L/Pd=2/1; (d) The reaction was conducted with 2 mol % $Pd(OAc)_2$; (e) Of two runs, one proceeded to 93% conversion, the other proceeded to 97% conversion; (f) Of two runs, one proceeded to 97% conversion.

*Suzuki Coupling at Low Catalyst Loading*

Studies to minimize the quantity of catalyst necessary further demonstrated the high activity of catalysts based on the dialkylphosphino(*o*-biphenyl) ligands. When **4** was used as the supporting ligand, reactions of electronically-neutral aryl bromides proceeded to completion with 0.025-0.05 mol % $Pd_2(dba)_3$ at 65 ˚C in THF, or with 0.1 mol % Pd$(OAc)_2$ in toluene at 100 ˚C in <24 h; the activated aryl chloride 4-chloroacetophenone was coupled with phenylboronic acid in 92% yield using 0.02 mol % Pd (Table 4, entry 6). Generally the best results were obtained with $K_3PO_4$ as the stoichiometric base in toluene solvent.

Higher turnover numbers were achieved when ligand **2** was employed in place of ligand **4**. Reactions of unactivated aryl bromides proceeded to completion with 0.005 mol % Pd (Table 4, entries 1-3), while the coupling of 4-chlorotoluene proceeded to 99% conversion (93% isolated yield) with 0.05 mol % Pd (Table 4, entry 5). Tris(2,4-di-*t*-butylphenyl)phosphite proved to be less effective than **2** for the coupling of 1-bromo-4-*t*-butylbenzene with phenylboronic acid when a catalyst loading of 0.001 mol % Pd was employed (Table 4, entry 2); the reaction catalyzed by Pd/**2** proceeded to an average of 92% conversion (93% avg. GC yield) while the Pd/phosphite catalyst system afforded an average conversion of 43% (44% avg. GC yield). Interestingly, the reaction of 1-bromo-4-*t*-butylbenzene with *o*-tolylboronic acid proceeded to 89% conversion (88% GC yield) using the phosphite ligand (Table 4, entry 3).

Remarkably low catalyst loadings could be used for the cross-coupling of 4'-bromoacetophenone with phenylboronic acid. Previous reports have described catalyst systems which provide 74,000 or 1,000,000 turnovers for this reaction at 135 ˚C.[3b,14] We were able to reproducibly obtain 100,000,000 turnovers in <24 h at 100 ˚C using the (*o*-biphenyl)P(*t*-Bu)$_2$ catalyst system for this reaction; a control experiment conducted in the absence of a phosphine ligand showed that 100,000 turnovers could be obtained using $Pd(OAc)_2$ in the absence of added ligand as the catalyst for this reaction. These exceptionally high turnover numbers were only obtained for this substrate combination suggesting that it is not a useful benchmark to test new catalysts in Suzuki couplings.

Table 4: Suzuki Coupling at Low Catalyst Loading

| Entry | Halide | Boronic Acid | Product | mol% Pd | Ligand | Time (h) | Yield (%) |
|---|---|---|---|---|---|---|---|
| 1 | 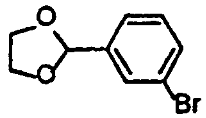 | 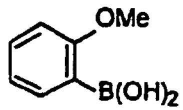 | 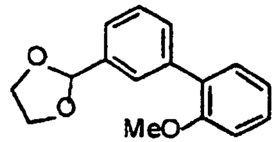 | 0.005 | 2 | 18 | 87 |
| 2 | 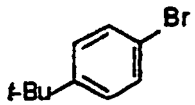 | 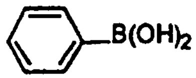 | 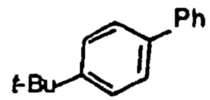 | 0.1 | 4 | 20 | 96[b,c,d] |
| | | | | 0.05 | 4 | 24 | 94[b,c,d,e] |
| | | | | 0.02 | 4 | 26 | 92 |
| | | | | 0.005 | 2 | 16 | 94 |
| | | | | 0.001 | 2 | 20 | 92 (GC)[g] |
| | | | | 0.001 | $ArO_3P$ | 20 | 43 (GC)[h,i] |
| | | | | 0.005 | $ArO_3P$ | 24 | 54 (GC)[i,k] |
| 3 | 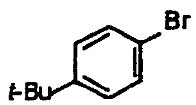 | 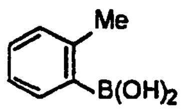 | 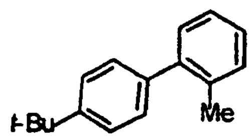 | 0.1 | 4 | 25 | 94[b,c,d] |
| | | | | 0.005 | 2 | 20 | 96 |
| | | | | 0.005 | $ArO_3P$ | 24 | 88 (GC)[i,l] |
| 4 | 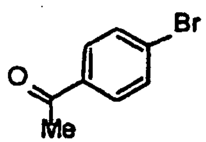 | 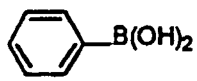 | 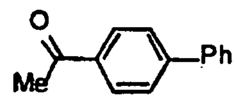 | 0.001 | 4 | 19 | 96[b] |
| | | | | 0.001 | - | 19 | 100 (GC) |
| | | | | 0.000001 | 4 | 24 | 91 |
| 5 | 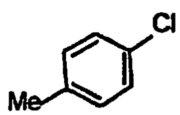 | 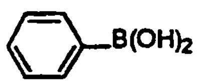 | 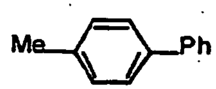 | 0.1 | 4 | 25 | 95 |
| | | | | 0.05 | 2 | 25 | 93[f] |

(continued)

| Entry | Halide | Boronic Acid | Product | mol% Pd | Ligand | Time (h) | Yield (%) |
|---|---|---|---|---|---|---|---|
| 6 | 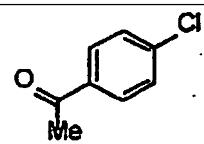 | 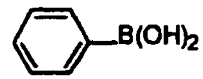 | 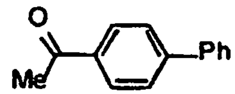 | 0.02 | 4 | 23 | 92 |

(a) Reaction conditions: 1.0 equiv aryl halide, 1.5 equiv boronic acid, 2.0 equiv $K_3PO_4$, cat $Pd(OAc)_2$, cat ligand (2L/Pd), toluene (3 mL/mmol halide), 100 °C; reaction times have not been minimized. All reactions proceed to completion unless otherwise noted; (b) $Pd_2(dba)_3$ used in place of $Pd(OAc)_2$; (c) THF used in place of toluene; (d) reaction conducted at 65 °C; (e) CsF (3.0 equiv) used in place of $K_3PO_4$; (f) reaction proceeded to 99% conversion; (g) reaction proceeded to 92% conversion; (h) reaction proceeded to 44% (average) conversion; (i) $ArO_3P$=tris(2,4-di-*t*-butylphenyl)phosphite; (j) Of two experiments, one proceeded to only 99% conversion; (k) The reaction proceeded to 52% (average) conversion; (l) The reaction proceeded 89% (average) conversion.

*Discussion*

Our results demonstrate that catalysts supported by ligands 2 or 4 are substantially more active for Suzuki coupling than catalysts based on triarylphosphines or trialkylphosphines which have been previously described.[1] The efficiency of catalysts based on **2** or **4** is most likely due to the combination of a number of factors. The electronic properties of the ligand are certainly of importance, as most triarylphosphines are not sufficiently electron-rich to promote the oxidative addition of aryl chlorides, particularly at room temperature.[2,17] However, previous studies have shown that electron-rich trialkylphosphines such as $PCy_3$ are rather inefficient ligands for the Suzuki coupling of electron-rich aryl halides;[3a,e] although these electron-rich ligands facilitate oxidative addition[18] they also decrease the rate of reductive elimination processes.[19] In contrast, Fu has shown that $t\text{Bu}_3\text{P}$ is an effective ligand for the Suzuki coupling of aryl chlorides.[4] These findings reflect that the combination of both steric bulk and electronics is important (see below). The higher efficiency of $t\text{Bu}_3\text{P}$ relative to $Cy_3P$ has previously been documented in aryl amination processes by the Tosoh group.[20]

Ligands **2** and **4** possess a fine balance of steric and electronic properties which allow for significantly accelerated oxidative addition while facilitating the other steps (transmetallation, reductive elimination) in the catalytic cycle. The basic phosphine group promotes oxidative addition, and binds tightly to the metal (relative to a triarylphosphine) to prevent precipitation of the catalyst. We believe that the ortho-phenyl moiety may provide a stabilizing interaction between the aromatic π-system and one of the metal d-orbitals,[21] and increases the steric bulk around the metal, which promotes reductive elimination and favors monophosphine palladium species.[22,23] This interaction also causes the aryl group of the substrate to be oriented perpendicular to the coordination plane which should be the most stereoelectronically favorable conformation for reductive elimination.[24]

Room-temperature Suzuki coupling reactions catalyzed by Pd/**4** were faster than those catalyzed by Pd/**2**. However, ligands **1, 2, 5**, and **6** were more effective for the Suzuki coupling of hindered substrates. Presumably this latter set of ligands are more efficient than **4** because their smaller size allows for relatively facile transmetallation to the $L_nPd(Ar)X$ intermediate when sterically encumbered aryl halides or boronic acids are used; the decreased steric properties of the ligand allows for the transformation of larger substrates. The ability of the electron-rich ligands to prevent precipitation of the palladium also may contribute to their efficiency in reactions of hindered substrates.

In general, **2** gave better results at low catalyst loading than were obtained with **4**. The larger *o*-(di-*t*-butylphosphino)biphenyl ligand (**4**) may tend to dissociate from the metal more readily than the smaller *o*-(dicyclohexylphosphino) biphenyl ligand (**2**) to form unligated palladium complexes which are unstable and lead to the precipitation of the metal. Therefore, catalysts based on the smaller dicyclohexylphosphino moiety are more stable than those based on larger ligands and thus allow for higher turnover numbers in reactions run at low catalyst loadings.

The fact that significantly higher turnover numbers are obtained with the electron-rich dialkyl(biphenyl)phosphine ligands than with less electron-rich phosphine or phosphite ligands is due in part to the basic nature of **2** and **4**. These ligands bind to the metal more tightly than triarylphosphine derivatives and may help to increase catalyst lifetime by keeping the metal in solution for extended periods of time. Although steric influences on coordination number are important for reactivity,[22] the basic phosphine is necessary to promote the oxidative addition step in the catalytic cycle.[2,18] This is highlighted by the results obtained with the tris(2,4-di-*t*-butylphenyl)phosphite ligand. While the steric bulk of the phosphite ligand may lead to pathways favoring the more reactive $L_1$ palladium complexes, it is not as electron-rich as **1-6,** and is ineffective as a ligand for Suzuki coupling reactions of aryl chloride substrates.

In conclusion, we have developed a new, highly active catalyst system for Suzuki coupling of aryl halides based on ligands **2** and **4**, which are easily prepared in one step and are commercially available.[10] While the use of **4** generally gives faster reaction rates in room-temperature Suzuki couplings, **2** is more effective for hindered substrates and operates more efficiently at low catalyst loadings. Of great importance is that with the use of ligands **2-6** the rate of oxidative addition is greatly enhanced while the rate of the other steps of the catalytic cycle are probably also increased. Thus, their use avoids the common pitfall in which speeding up the rate of one step slows that of another, resulting in little or no increase in the overall rate of the reaction. Our current view is that the success of these ligands is due to a combination of: 1) their electron-richness-to enhance the rate of oxidative addition and keep the palladium in solution; 2) their steric bulk-to enhance the rate of reductive elimination and to maximize the quantity of $L_1Pd$ complexes, increasing the rate of transmetallation. We also believe that the presence of the *o*-biphenyl moiety is important, helping to confer air-stability to the ligand, enhancing the rate of reductive elimination as well as stabilizing the catalyst by interacting with the Pd. Further studies concerning the effectiveness of this class of ligands for other palladium-catalyzed reactions, as well as mechanistic studies to determine the factors responsible for the high activities of these catalysts are currently underway.

**General Experimental Procedures.** All reactions were carried out under an argon atmosphere in oven-dried glassware. Elemental analyses were performed by E & R Microanalytical Laboratory Inc., Parsippany, NJ, or by Atlantic Microlabs Inc, Norcross, GA. Toluene was distilled under nitrogen from molten sodium. THF was distilled under argon from sodium benzophenone ketyl. DME was purchased anhydrous from Aldrich Chemical Co. and was used without further purification. Unless stated otherwise, commercially obtained materials were used without purification. Aryl halides were purchased

from Aldrich Chemical Co. except for 4-chloroacetophenone and 2-bromoisopropylbenzne which were purchased from Fluka Chemical Co., 2-bromobiphenyl which was purchased from Lancaster Synthesis Inc., and *N*-(diphenylmethylene)-4-bromoaniline[25] which was prepared according to a previously published procedure.[25] Dicyclohexylchlorophosphine, palladium acetate, and *n*-butyllithium were purchased from Strem Chemical Co. Boronic acids were purchased from Aldrich Chemical company and were used without further purification. Trimethyl borate, triisopropyl borate, 9-BBN, di-*t*-butylchlorophosphine, potassium fluoride, and 1-hexene were purchased from Aldrich Chemical Co. Tribasic potassium phosphate was purchased from Fluka Chemical Co. Sodium carbonate was purchased from Mallinckrodt Chemical Co. Tetrakis(triphenylphosphine)palladium was prepared according to a literature procedure,[26] or purchased from Strem Chemical company. 2-(*N,N*-Dimethylamino)-2'-(dicyclohexylphosphino)biphenyl (1) was prepared according to the previously published procedure.[7] 2,6-Dimethylphenylboronic acid was prepared according to a literature procedure[27] which was modified such that *n*-butyllithium and triisopropyl borate were used in place of *s*-butyllithium and trimethyl borate. IR spectra reported in this paper were obtained by placing neat samples directly on the DiComp probe of an ASI REACTIR *in situ* IR instrument. Yields in Tables 1 and 2 refer to isolated yields (average of two runs) of compounds estimated to be 95% pure as determined by $^1$H NMR, and GC analysis or combustion analysis. Entries 1,[7] and 5[7] from Table 2 have been previously reported by this group and were characterized by comparison of their $^1$H NMR spectra to those of samples prepared prior to this work; their purity was confirmed by GC analysis. The procedures described in this section are representative, thus the yields may differ from those given in Tables 1-4.

***o*-(Dicyclohexylphosphino)biphenyl (2).**[9] An oven-dried round-bottomed flask equipped with a magnetic stirbar and a rubber septum was allowed to cool to rt under an argon purge. The flask was charged with 2-bromobiphenyl (0.69 mL, 4.0 mmol) and THF (10 mL), and cooled to -78 ˚C in a dry ice/acetone bath. *n*-Butyllithium in hexanes (1.6 M, 2.75 mL, 4.4 mmol) was added dropwise with stirring. The resulting yellow solution was stirred at -78 ˚C for 45 min, during which time a yellow precipitate formed. A solution of dicyclohexylchlorophosphine (1.16 g, 5.0 mmol) in THF (2 mL) was added to the mixture dropwise at -78 ˚C, and the resulting solution was stirred at -78 ˚C for 15 min. The solution was then warmed to 0 ˚C in an ice water bath and allowed to warm slowly to rt overnight (14 h). The reaction was quenched with saturated aqueous ammonium chloride (10 mL), diluted with ether (50 mL), and transferred to a separatory funnel. The layers were separated, and the aqueous layer was extracted with ether (20 mL). The combined organic layers were washed with brine (50 mL), dried over anhydrous sodium sulfate, filtered, and concentrated to give a colorless oil. Methanol (5 mL) was added, and a white precipitate formed. The material was then recrystallized from hot methanol (two crops of crystals were collected) to afford 994 mg (71%) of a white solid, mp 103 ˚C. $^1$H NMR (300 MHz, $CDCl_3$) δ 7.62-7.51 (m, 1H), 7.40-7.10 (m, 8H), 1.95-1.45 (m, 13H), 1.35-0.95 (m, 9H) ; $^{31}$P NMR (121 MHz, $CDCl_3$) δ -12.7; $^{13}$C NMR (75 MHz, $CDCl_3$) δ 150.7, 150.4, 142.9, 142.8, 134.1, 133.8, I32.8, 130.61, 130.55, 130.5, 130.2, 1301, 128.1, 127.3, 127.14, 127.05, 126.7, 126.4, 34.7, 34.5, 30.5, 30.2, 29.3, 29.1, 27.3, 27.2, 27.1, 26.4 (observed complexity due to P-C splitting; definitive assignments have not yet been made); IR (neat, $cm^{-1}$) 2916, 1441, 749. Anal. Calcd for $C_{24}H_{31}P$: C, 82.25; H, 8.92. Found: C, 82.18; H, 9.04.

**2-(Di-*t*-butylphosphino)biphenyl (4).**[8,9] An oven-dried round-bottomed flask equipped with a magnetic stirbar and a rubber septum was allowed to cool to rt under an argon purge. The flask was charged with magnesium turnings (617 mg, 25.4 mmol) and a small crystal of iodine. The flask was purged with argon and a solution of 2-bromobiphenyl (5.38 g, 23.1 mmol) in THF (40 mL) was added. The mixture was heated to reflux with stirring for 2 h, then allowed to cool to rt. The septum was removed and anhydrous copper (I) chloride (2.40 g, 24.2 mmol) was added. The flask was capped with the septum and purged with argon for 2 min. Di-*t*-butylchlorophosphine (5.0 g, 27.7 mmol) was added via syringe, and the mixture was heated to reflux with stirring for 8 h. The mixture was cooled to rt and diluted with 1:1 hexanes: ether (200 mL). The resulting suspension was filtered, and the solids were washed with hexanes (60 mL). The solid material was transferred to a flask containing 1:1 hexane:ethyl acetate (150 mL) and water (100 mL) and 30% aqueous ammonium hydroxide (60 mL) were added. The resulting slurry was stirred at rt for 5 min then transferred to a separatory funnel. The layers were separated and the organic phase was washed with brine (100 mL), dried over anhydrous sodium sulfate, filtered, and concentrated *in vacuo.* The resulting solid was recrystallized from methanol (2 crops of crystals were collected) to afford 4.46 g (67%) of a white solid, mp 86-86.5 ˚C. $^1$H NMR (300 MHz, $CDCl_3$) δ 7.95-7.85 (m, 1H), 7.40-7.21 (m, 8H), 1.15 (d, 18H, *J* = 11.6 Hz); $^{31}$P NMR (121 MHz, $CDCl_3$) δ 18.7; $^{13}$C NMR (75 MHz, $CDCl_3$) δ 151.4, 150.9, 143.6, 143.5, 135.6, 135.2, 135.0, 130.5, 130.4, 130.1, 128.3, 127.0, 126.7, 126.5, 126.2, 126.0; 125.6, 32.7, 32.4, 30.8, 30.6 (observed complexity due to P-C splitting; definitive assignments have not yet been made); IR (neat, $cm^{-1}$) 2956, 1459, 1362, 1173; Anal. Calcd for $C_{20}H_{27}P$: C, 80.50; H, 9.12. Found: C, 80.67; H, 9.36.

**2-Dicyclohexylphosphino-2'-methylbiphenyl (5).** A flame-dried flask was cooled to rt under an argon purge and charged with tetrakis(triphenylphosphine)palladium (425 mg, 0.37 mmol), sodium carbonate (3.9 g, 36.8 mmol), and o-tolylboronic acid (1.0 g, 7.36 mmol). The flask was purged with argon and a degassed mixture of DME (60 mL), water (18 mL), and ethanol (3 mL) was added to the flask *via* cannula. 1-Bromo-2-iodobenzene (1.13 mL, 8.83 mmol) was added to the flask *via* syringe and the mixture was heated to 90 ˚C for 42 h. The mixture was cooled to rt, diluted with ether (50 mL) and transferred to a separatory funnel. The layers were separated and the organic layer was washed with aqueous sodium hydroxide (3 x 75 mL). The organic layer was concentrated *in vacuo* and the crude material was

dissolved in 1:1 ether:dichloromethane (200 mL), washed with brine (50 mL), dried over anhydrous sodium sulfate, filtered, and concentrated *in vacuo.* The crude material was purified by flash chromatography on silica gel to afford 1.57 g of 2-bromo-2'-methylbiphenyl which contained ~5% of 2-bromoiodobenzene as judged by GC analysis. This material was used without further purification.

An oven-dried Schlenk flask was cooled to rt under an argon purge and charged with 2-bromo-2'-methylbiphenyl (682 mg, 2.76 mmol) and THF (7 mL). The mixture was cooled to-78 ˚C with stirring and *n*-butyllithium (1.6 M, 1.9 mL, 3.04 mmol) was added dropwise. The mixture was stirred at -78 ˚C for 70 min, then a solution of dicyclohexylchlorophosphine (803 mg, 3.45 mmol) in THF (2 mL) was added dropwise at -78 ˚C *via* syringe. The mixture was stirred at -78 ˚C for 20 min, warmed to 0 ˚C and stirred for 20 min, then warmed to rt and stirred for 18 h. The mixture was quenched with saturated aqueous ammonium chloride (5 mL), diluted with ether (50 mL), and transferred to a separatory funnel. The layers were separated and the aqueous layer was extracted with ether (20 mL). The combined organic layers were washed with brine (30 mL), dried over anhydrous sodium sulfate, filtered, and concentrated *in vacuo.* The crude material was crystallized from ethanol to afford 754 mg (65% overall yield) of the title compound as a white solid, mp 107-109 ˚C. $^1H$ NMR (250 MHz, $CDCl_3$) δ 7.56 (s, br, 1H), 7.37-7.30 (m, 2H), 7.28-7.10 (m, 4 H), 7.06 (d, 1H, *J* = 73 Hz), 2.06 (s, 3H), (1.99-1.80 (m, 1H), 1.80-1.45 (m, 11H), 1.40-0.85 (m, 10H); $^{31}P$ NMR (121 MHz, $CDCl_3$) δ -10.97; $^{13}C$ NMR (75 MHz, $CDCl_3$) δ 149.9, 149.5, 142.4, 142.3, 135.5, 134.5, 134.2, 132.5, 130.7, 130.0, 129.9, 129.3, 128.2, 127.2, 126.3, 124.5,35.4, 35.2, 33.2, 33.0, 30.8, 30.6, 30.0, 29.8, 29.7, 29.6, 28.8, 28.7, 27.6, 27.44, 27.39, 27.2, 27.0, 26.4, 26.3, 20.7,20.6 (observed complexity due to P-C splitting; definitive assignments have not yet been made); IR (neat, $cm^{-1}$) 2927, 1445, 1177, 1007, 766. Anal. Calcd for $C_{25}H_{33}P$: C, 82.38; H, 9.13. Found: C, 82.11; H, 9.21.

**2-Dicyclohexylphosphino-2'-isopropylbiphenyl (6).** An oven-dried flask was cooled to rt under an argon purge and charged with 2-bromoisopropyl benzene (4.0 g, 20.0 mmol) and THF (80 mL). The solution was cooled to -78 ˚C and a solution of *n*-butyllithium in hexanes (1.65 M, 12.7 mL, 21.0 mmol) was added dropwise. The mixture was stirred at -78 ˚C for 1 h, then transferred *via* cannula to a separate flask containing a solution of triisopropyl borate (9.2 mL; 40.0 mmol) in THF (40 mL) under argon which had been cooled to -78 ˚C. The reaction mixture was stirred at -78 ˚C for 15 min, then warmed to rt and allowed to stir overnight (14 h). Aqueous HCl (1 M, 250 mL) was added and the mixture was stirred at rt for 15 min. The mixture was basisified to pH 14 with aqueous NaOH (6 M) and the mixture was transferred to a separatory funnel. The mixture was extracted with ether (100 mL), and the organic layer was discarded. The aqueous phase was acidified to ~pH 7 with aqueous HCl (1 M) and was extracted with ether (2 x 150 mL). The combined organic extracts were dried over anhydrous magnesium sulfate, filtered, and concentrated *in vacuo.* The crude material was precipitated from ether/pentane to give 2-isopropylphenylbbronic acid (2.4 g) which was used without further purification.

An oven-dried flask was charged with the crude 2-isopropylphenylboronic acid (2.4 g), tetrakis(triphenylphosphine) palladium (840 mg, 0.61 mmol, 5 mol %), and $K_3PO_4$ (4.6 g, 21.9 mmol). The flask was purged with argon, then DMF (100 mL) and 2-bromoiodobenzene (1.88 mL, 14.6 mmol) were added *via* syringe. The mixture was heated to 100 ˚C for 48 h, then cooled to rt, diluted with ether (200 mL) and water (100 mL) and transferred to a separatory funnel. The layers were separated and the aqueous layer was extracted with ether (200 mL). The combined organic layers were washed with aqueous NaOH (1 M), dried over anhydrous magnesium sulfate, filtered, and concentrated *in vacuo.* The crude material was purified by flash chromatography on silica gel to give 2-bromo-2'-isopropylbiphenyl (1.5 g). The crude material was used without further purification.

An oven-dried flask was cooled to rt under an argon purge and charged with the crude 2-bromo-2'-isopropylbiphenyl (1.1 g, 4.0 mmol), and THF (10 mL). The mixture was cooled to -78 ˚C and a solution of n-butyllithium in hexanes (1.6 M, 2.8 mL, 4.4 mmol) was added dropwise. The mixture was stirred at -78 ˚C for I h, then a solution of dicyclohexylchlorophosphine (1.16 g, 5.0 mmol) in THF (2 mL) under argon was added dropwise. The mixture was stirred at -78 ˚C for 15 min, then warmed to rt and stirred for 20 h. Aqueous ammonium chloride (10 mL) was added, and the mixture was diluted with ether (50 mL) and transferred to a separatory funnel. The layers were separated and the aqueous layer was extracted with ether (20 mL). The combined organic layers were dried over anhydrous sodium sulfate, filtered, and concentrated *in vacuo.* The crude material was crystallized from ethanol to afford 877 mg (~11% overall yield from 2-isopropylbromobenzene) of a white crystalline solid, mp 104 ˚C. $^1H$ NMR (250 MHz, $CDCl_3$) δ 7.58 (s, br, 1H), 7.36-7.10 (m, 6H), 7.00 (d, 1H, *J* = 7.5 Hz), 2.65 (p, 1H, *J* = 6.8 Hz), 1.99-1.85 (m, br, 1H), 1.75-1.45 (m, 11H), 1.28-0.85 (m, 17H); $^{31}P$ NMR (121 MHz, $CDCl_3$) δ -12.75; $^{13}C$ NMR (75 MHz, $CDCl_3$) δ 149.9, 149.5, 146.2, 141.15, 141.07, 134.9, 134.7, 132.7, 132.6, 130.9, 130.4, 130.3, 127.9, 127.6, 126.3, 124.7, 124.3, 35.8, 35.6, 33.9, 33.7, 30.9, 30.8, 30.3, 30.0, 29.9, 29.7, 29.6, 29.5, 28.9, 28.8, 27.6, 27.5, 27.4, 27.3, 27.2, 27.0, 26.5, 26.4, 25.3, 22.6 (observed complexity due to P-C splitting; definitive assignments have not yet been made); IR (neat, $cm^{-1}$) 2921, 1443, 1003, 753. Anal. Calcd for $C_{27}H_{37}P$: C, 82.61; H, 9.50. Found: C, 82.35; H, 9.55.

**N-(Diphenylmethylene)-2-bromoaniline** An oven-dried flask was charged with 2-bromoaniline (10.32 g, 60.0 mmol), benzophenone.(10.93 g, 60.0 mmol), 5Å molecular sieves (150 g), and toluene (300 mL); the mixture was heated to 100 ˚C with stirring under an argon atmosphere for 36 h. The mixture was cooled to rt, filtered, and concentrated *in vacuo* to afford a yellow solid which was recrystallized from methanol to afford 16.58 g (82%) of a yellow solid, mp 103-105 ˚C. $^1H$ NMR (300 MHz, $CDCl_3$) δ 7.81 (d, 2H, *J* = 7.0 Hz), 7.50-7.40 (m, 4H), 7.28-7.16 (m, 5H), 7.05-6.99 (m,

1H), 6.80-6.74 (m, 1H), 6.53 (d, 1H, *J* = 7.8 Hz); $^{13}C$ NMR (75 MHz, $CDCl_3$) δ 169.3, 150.1, 138.7, 135.9, 132.3, 131.0, 129.5, 128.8, 128.5, 128.2, 127.8, 127.4, 121.1, 115.2; IR (neat, $cm^{-1}$) 3056, 1615, 1447, 1275, 1025. Anal Calcd for $C_{19}H_{14}BrN$: C, 67.87; H, 4.20. Found: C, 67.76; H, 4.24.

**General Procedure for the Suzuki Coupling of Aryl Halides Using KF as base.** An oven-dried resealable Schlenk flask was evacuated and backfilled with argon[28] and charged with $Pd(OAc)_2$ (2.2 mg, 0.01 mmol, 1.0 mol %), ligand **4** (not according to the invention; 6.0 mg, 0.020 mmol, 2.0 mol %), the boronic acid (1.5 mmol), and potassium fluoride (174 mg, 3.0 mmol). The flask was evacuated and backfilled with argon, and THF (1 mL) and the aryl halide (1.0 mmol) were added through a rubber septum (aryl halides which were solids at rt were added prior to the second evacuation/ backfill cycle). The flask was sealed with a teflon screwcap, and the reaction mixture was stirred at rt until the starting aryl chloride had been completely consumed as judged by GC analysis. The reaction mixture was diluted with ether (30 mL) and poured into a separatory funnel. The mixture was washed with aqueous NaOH (1 M, 20 mL), and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel.

**2-Methoxy-3-(1,3-dioxolane)-biphenyl.** The coupling of 2-(3-bromophenyl)1,3-dioxolane with 2-methoxyphenylboronic acid was effected using the general procedure with 0.5 mol % $Pd(OAc)_2$ and 1.0 mol % 4 to afford 215 mg (84%) of the title compound as a colorless oil. $^{1}H$ NMR (300 MHz, $CDCl_3$) δ 7.62 (s, 1H), 7.55-7.50 (m, 1H), 7.43-7.40 (m, 2H), 7.38-7.28 (m, 2H), 7.06-6.96 (m, 2H), 5.59 (s, 1H), 4.17-4.01 (m, 4H), 3.80 (s, 3H); $^{13}C$ NMR (75 MHz, $CDCl_3$) δ 156.3, 138.5, 137.6, 130.8, 130.3, 130.2, 128.6, 127.8, 127.5, 124.8, 120.7, 111.1, 103.7, 65.1, 55.4; IR (neat, $cm^{-1}$) 2887, 1598, 1239, 1100, 753. Anal. Calcd for $C_{16}H_{16}O_3$: C, 74.98; H, 6.29. Found: C, 74.92; H, 6.49.

**2-Methoxy-3-(1,3-dioxolane)-biphenyl** (phosphite ligand used). The coupling of 2-(3-bromophenyl)-1,3-dioxolane with 2-methoxyphenylboronic acid was effected using the general procedure with 0.5 mol % $Pd(OAc)_2$ and 1.0 mol % tris (2,4-di-*t*-butylphenyl)phosphite to afford 211 mg (82%) of the title compound as a colorless oil.

**4-Formyl-4'-ethoxyhiphenyl.** The coupling of 4-bromobenzaldehyde with 4-ethoxyphenylboronic acid was effected using the general procedure with 0.5 mol % $Pd(OAc)_2$ and 1.0 mol % **4** to afford 203 mg (90%) of the title compound as a white solid, mp 102-103 °C. $^{1}H$ NMR (300 MHz, $CDCl_3$) δ 10.04 (s, 1H), 7.92 (d, 2H, *J* = 8.4 Hz), 7.72 (d, 2H, *J* = 8.3 Hz), 7.58 (d, 2H, *J* = 8.8 Hz), 6.99 (d, 2H, *J* = 8.8 Hz), 4.10 (q, 2H, *J* = 7.1 Hz), 1.45 (t, 3H, *J* = 7.1 Hz); $^{13}C$ NMR (75 MHz, $CDCl_3$) δ 191.7, 159.5, 146.7, 134.7, 131.8, 130.2, 128.4, 126.9, 115.0, 63.6, 14.7; IR (neat, $cm^{-1}$) 2984, 1679, 1602, 1185, 822. Anal Calcd for $C_{15}H_{14}O_2$: C, 79.62; H, 6.24. Found: C, 80.02; H, 6.47.

**4-Formyl-4'-ethoxybiphenyl.** The coupling of 4-bromobenzaldehyde with 4-ethoxyphenylboronic acid was effected using the general procedure with 0.5 mol % $Pd(OAc)_2$ and 1.0 mol % tris(2,4-di-*t*-butylphenyl)phosphite to afford 178 mg (79%) of the title compound as a white solid.

**4-Phenylphenol.**[29] The coupling of 4-bromophenol with phenylboronic acid was effected using the general procedure with a reaction temperature of 50 °C to afford 152 mg (89%) of the title compound as a white solid, mp 146-147 °C (lit mp 165 °C).[29] $^{1}H$ NMR (300 MHz, $CDCl_3$) δ 7.6-6.8 (m, 9H), 4.88 (s, 1H); $^{13}C$ NMR (75 MHz, $CDCl_3$) δ 155.0, 140.7, 134.0, 129.4, 128.7, 128.4, 126.7, 115.6; IR (neat, $cm^{-1}$) 3350, 1262, 1116, 834, 757. Anal. Calcd for $C_{12}H_{10}O$: C, 84.68; H, 5.92. Found: C, 84.96; H, 5.64.

**2-Formyl-4'-diphenylketiminebiphenyl.** The coupling of *N*-(diphenylinethylene)-4-bromoaniline[25] with 2-formylphenylboronic acid was effected using the general procedure (carried out on a 2 mmol scale) to afford 647 mg (90%) of the title compound as a yellow powder, mp 96-98 °C. $^{1}H$ NMR (300 MHz, $CDCl_3$) δ 9.88 (s, 1H), 7.98 (d, 1H, *J* = 8.0 Hz), 7.79 (d, 2H, *J* = 7.3 Hz), 7.59 (t, 1H, *J* = 7.3 Hz), 7.39-7.50 (m, 6H), 7.30 (d, 2H, *J* = 5.9 Hz), 7.17 (d, 4H, *J* = 7.8 Hz), 6.84 (d, 2H, *J* = 7.8 Hz); $^{13}C$ NMR (75 MHz, $CDCl_3$) δ 192.5, 168.8, 151.3, 145.7, 139.3, 135.9, 133.6, 133.4, 132.2, 130.9, 130.6, 130.3, 129.4, 129.3, 128.8, 128.2, 127.9, 127.4, 127.3, 121.0; IR (neat, $cm^{-1}$) 3059, 2845, 2747, 1691, 1595, 1472, 1445, 1392. Anal Calcd for $C_{26}H_{19}NO$: C, 86.40; H, 5.30. Found: C, 86.43; H, 5.09.

***N*-Acetyl-4-aminobiphenyl.**[30] The coupling of 4'-bromoacetanilide with phenylboronic acid was effected using the general procedure with a reaction temperature of 50 °C to afford 188 mg (89%) of the title compound as a white solid, mp 150-153°C (lit mp 171°C).[30] $^{1}H$ NMR (300 MHz, $CDCl_3$) δ 7.7-7.3 (m, 9H), 2.19 (s, 3H), 2.15 (s, 1H); $^{13}C$ NMR (75 MHz, $CDCl_3$) δ 168.6, 140.4, 137.1, 131.9, 128.8, 127.5, 127.1, 126.8, 120.3, 24.5; IR (neat, $cm^{-1}$) 3308, 3192, 1660, 1606, 1454, 1490, 1405, 1370, 1324. Anal. Calcd for $C_{14}H_{13}NO$: C, 79.59; H, 6.20. Found: C, 79.49; H, 6.00.

**2-Phenylbenzyl alcohol**.[31] The coupling of 2-bromobenzyl alcohol with phenylboronic acid was effected using the general procedure with a reaction temperature of 50 °C to afford 162 mg (88%) of the title compound as a colorless oil. $^{1}H$ NMR (300 MHz, $CDCl_3$) δ 7.5-7.2 (m, 9H), 4.54 (s, 2H), 2.09 (s, 1H); $^{13}C$ NMR (75 MHz, $CDCl_3$) δ 141.1, 140.6, 137.9, 130.0, 129.0, 128.3, 128.2, 127.6, 127.5, 127.2; IR (neat, $cm^{-1}$) 3350, 3061,1478, 1451, 1193, 1007, 749. Anal. Calcd for $C_{13}H_{12}O$: C, 84.75; H, 6.57. Found: C, 84.94; H, 6.91.

**2,5-Dimethylbiphenyl.**[32] The coupling of 2-bromo-*p*-xylene with phenylboronic acid was effected using the general procedure with a reaction temperature of 45 °C to afford 171 mg (94%) of the title compound as a colorless oil. $^{1}H$ NMR (300 MHz, $CDCl_3$) δ 7.4-7.0 (m, 8H), 2.34 (s, 3H), 2.22 (s, 3H); $^{13}C$ NMR (75 MHz, $CDCl_3$) δ 142.0, 141.7, 135.1, 132.1, 130.5, 130.2, 129.2, 128.0, 127.9, 126.6; IR (neat, $cm^{-1}$) 3026, 2922, 1490, 144-4, 811, 776, 703. Anal. Calcd for $C_{14}H_{14}$:

C, 92.26; H, 7.74. Found: C, 92.11; H, 7.86.

**2,5-Dimethylbiphenyl**[32] (room temperature). The coupling of 2-bromo-*p*-xylene with phenylboronic acid was effected using the general procedure to afford 149 mg (82%) of the title compound as a colorless oil.

**2,5-Dimethylbiphenyl**[32] (room temperature, ligand **2** (not according to the invention) used). The coupling of 2-bromo-*p*-xylene with phenylboronic acid was effected using the general procedure with ligand **2** to afford 175 mg (96%) of the title compound as a colorless oil.

**2-Phenylthiophene.**[33] The coupling of 2-bromothiophene with phenylboronic acid was effected using the general procedure to afford 159 mg (99%) of the title compound as a white solid, mp 34-35 °C (lit mp 34-36 °C).[33] $^1$H NMR (300 MHz, $CDCl_3$) δ 7.69 (d, 2H, *J* = 7.2 Hz), 7.44 (dd, 2H, *J* = 7.8 Hz, 7.2 Hz), 7.44 (t, 1H, *J* = 7.8 Hz), 7.37 (d, 1H, *J* = 3.8 Hz), 7.33 (d, 1H, *J* = 5.0 Hz), 7.13 (dd, 1H, *J* = 5.0 Hz, 3.8 Hz); $^{13}$C NMR (75 MHz, $CDCl_3$) δ 144.4, 134.3, 128.8, 128.0, 127.4, 125.9, 124.7, 123.0; IR (neat, $cm^{-1}$) 3076, 1596, 1488, 1446, 1426, 1334, 1257, 1074, 852, 824, 757, 690. Anal Calcd for $C_{10}H_8S$: C, 74.96; H, 5.03. Found: C, 75.06; H, 5.00.

**2,6-Dimethylbiphenyl.**[34] The coupling of 2-bromo-*m*-xylene with phenylboronic acid was effected using the general procedure with a reaction temperature of 65 °C to afford 149 mg (82%) of the title compound as a colorless oil. $^1$H NMR (300 MHz, $CDCl_3$) δ 7.5-7.0 (m, 8H), 2.02 (s, 6H); $^{13}$C NMR (75 MHz, $CDCl_3$) δ 141.8, 141.0, 136.0, 129.0, 128.4, 127.2, 127.0, 126.6. IR (neat, $cm^{-1}$) 3057, 3022, 1463, 1444, 768; Anal. Calcd for $C_{14}H_{14}$: C, 92.26; H, 7.74. Found: C, 91.92; H, 8.00.

**2,6-Dimethylbiphenyl**[34] (room temperature, ligand **2** (not according to the invention) used). The coupling of 2-bromo-*m*-xylene with phenylboronic acid was effected using the general procedure with ligand **2** to afford 168 mg (92%) of the title compound as a colorless oil.

*Suzuki Coupling of Aryl Chlorides*

**4-Methylbiphenyl.**[7] The coupling of 4-chlorotoluene with phenylboronic acid was effected using the general procedure to afford 161 mg (96%) of the title compound as a white solid, mp 42-45 °C (lit mp 44-46 °C).[7]

**4-Methylbiphenyl.**[7] The coupling of 4-chlorotoluene with phenylboronic acid was effected using the general procedure with 0.5 mol % $Pd(OAc)_2$ and 1.0 mol % **4** to afford 161 mg (96%) of the title compound as a white solid.

**2-Methoxy-4'-methylbiphenyl.**[7] The coupling of 4-chlorotoluene with 2-methoxyphenyl boronic acid was effected using the general procedure to afford 188 mg (95%) of the title compound as a white solid, mp 71-72 °C (lit mp 74-75 °C).[7] $^1$H NMR (300 MHz, $CDCl_3$) δ 7.43 (d, 1H, *J* = 8.3 Hz), 7.3-6.9 (m, 7H), 3.81 (s, 3H), 2.39 (s, 3H); $^{13}$C NMR (75 MHz, $CDCl_3$) δ 156.4, 136.6, 135.5, 130.8, 130.6, 129.4, 128.7, 128.3, 120.7, 111.1, 55.5, 21.2; IR (neat, $cm^{-1}$) 3022, 1598, 1486, 1463, 1258, 1235, 1031, 818, 753. Anal. Calcd for $C_{14}H_{14}O$: C, 84.81; H, 7.12. Found: C, 85.02; H, 7.33.

**4-Cyanobiphenyl.**[35] The coupling of 4-chlorobenzonitrile with phenylboronic acid was effected using the general procedure to afford 156 mg (87%) of the title compound as a white solid, mp 86-87 °C (lit mp 82-84 °C).[35] $^1$H NMR (300 MHz, $CDCl_3$) δ 7.76-7.68 (m, 4H), 7.60-7.58 (m, 2H), 7.52-7.43 (m, 3H); $^{13}$C NMR (75 MHz, $CDCl_3$) δ 145.6, 139.1, 132.5, 129.0, 128.6, 127.6, 127.4, 118.9, 110.8; IR (neat, $cm^{-1}$) 2227, 1605, 1485, 768. Anal Calcd for $C_{13}H_9N$: C, 87.12; H, 5.06. Found: C, 87.04; H, 5.06.

**4-Nitrobiphenyl**.[36] The coupling of 4-chloronitrobenzene with phenylboronic acid was effected using the general procedure with 0.2 mol % $Pd(OAc)_2$ and 0.4 mol % 4 to afford 196 mg (98%) of the title compound as a yellow solid, mp 102-103 °C (lit mp 114-114.5 °C).[36] $^1$H NMR (300 MHz, $CDCl_3$) δ 8.29 (d, 2H, *J* = 8.5 Hz), 7.73 (d, 2H, *J* = 8.5 Hz), 7.63 (d, 2H, *J* = 7.6 Hz), 7.52-7.44 (m, 3H); $^{13}$C NMR (75 MHz, $CDCl_3$) δ 147.6, 147.0, 138.7, 129.1, 128.9, 127.8, 127.3, 124.1; IR (neat, $cm^{-1}$)1594, 1513, 1447, 1351, 1104, 1077. Anal. Calcd for $C_{12}H_9NO_2$: C, 72.35; H, 4.55. Found: C, 72.63; H, 4.20.

**4-Nitrobiphenyl**[36] (1.0 mol % Pd used). The coupling of 4-chloronitrobenzene with phenylboronic acid was effected using the general procedure to afford 196 mg (98%) of the title compound as a yellow solid.

**4-Methoxybiphenyl.**[7] The coupling of 4-chloroanisole with phenylboronic acid was effected using the general procedure and a reaction temperature of 45 °C to afford 163 mg (88%) of the title compound as a white solid, mp 77-78.5 °C (lit mp 83-84 °C).[7]

**4-Methoxybiphenyl**.[7] The coupling of 4-chloroanisole with phenylboronic acid was effected using the general procedure with 1.5 mol % $Pd(OAc)_2$ and 3.0 mol % **4** to afford 170 mg (92%) of the title compound as a white solid.

**3-Acetyl-3',5'-dimethoxybiphenyl.** The coupling of 5-chloro-1,3-dimethoxybenzene with 3-acetylphenylboronic acid was effected using the general procedure to afford 232 mg (91%) of the title compound as a white solid, mp 73-74 °C. $^1$H NMR (300 MHz, $CDCl_3$) δ 8.15 (s, 1H), 7.92 (d, 1H, *J* = 7.5 Hz), 7.75 (d, 1H, *J* = 7.5 Hz), 7.50 (t, 1H, *J* = 7.5 Hz), 6.73 (s, 2H), 6.48 (s, 1H), 3.84 (s, 6H), 2.64 (s, 3H); $^{13}$C NMR (75 MHz, $CDCl_3$) δ 197.9, 161.0, 142.2, 141.5, 137.4, 131.7, 128.9, 127.4, 126.8, 105.4, 99.5, 55.3, 26.6; IR (neat, $cm^{-1}$) 3006, 2937, 1459, 1402, 1349, 1266, 1204, 1155. Anal Calcd for $C_{16}H_{16}O_3$: C, 74.98; H, 629. Found: C, 75.07; H, 5.94.

**2-Acetylbiphenyl.**[37] The coupling of 2-chloroacetophenone with phenylboronic acid was effected using the general procedure (carried out on a 2 mmol scale) to afford 369 mg (94%) of the title compound as a colorless oil: $^1$H NMR (300

MHz, $CDCl_3$) δ 7.50-7.57 (m, 2H), 7.33-7.44 (m, 7H), 2.01 (s, 3H); $^{13}C$ NMR (75 MHz, $CDCl_3$) δ 204.8, 140.8, 140.7, 140.4, 130.7, 130.2, 128.8, 128.8, 128.6, 127.8, 127.4, 30.4; IR (neat, $cm^{-1}$) 3058, 3024, 1687, 1594, 1471, 1449, 1354, 1269, 1233. Anal Calcd for $C_{14}H_{12}O$: C, 85.68; H, 6.16. Found: C, 85.76; H, 6.39.

**3-(3-Acetylphenyl)pyridine.** The coupling of 3-chloropyridine with 3-acetylphenylboronic acid was effected using the general procedure with a reaction temperature of 50°C to afford 181 mg (92%) of the title compound as a colorless oil. $^1H$ NMR (300 MHz, $CDCl_3$) δ 8.88 (d, 1H, *J* = 2.3 Hz), 8.64 (d, 1H, *J* = 4.7 Hz), 8.18 (s, 1H), 8.00 (d, 1H, *J* = 7.7 Hz), 7.92 (d, 1H, *J* = 8.0 Hz), 7.79 (d, 1H, *J* = 7.7 Hz), 7.60 (t, 1H, *J* = 7.8 Hz), 7.43-7.39 (m, 1H), 2.67 (s, 3H); $^{13}C$ NMR (75 MHz, $CDCl_3$) δ 197.5, 149.0, 148.2, 138.4, 138.3, 135.7, 134.4, 131.5, 129.3, 127.9, 126.8, 123.6, 26.6; IR (neat, $cm^{-1}$) 3034, 1683, 1239, 791. Anal Calcd for $C_{13}H_{11}NO$: C, 79.17; H, 5.62. Found: C, 79.12; H, 5.62.

**2-Cyanomethylbiphenyl.**[38] The coupling of 2-chlorobenzyl cyanide with phenylboronic acid was effected using the general procedure to afford 177 mg (92%) of the title compound as a colorless oil. $^1H$ NMR (300 MHz, $CDCl_3$) δ 7.56-7.53 (m, 1H), 7.48-7.38 (m, 5H), 7.30-7.26 (m, 3H), 3.63 (s, 2H); $^{13}C$ NMR (75 MHz, $CDCl_3$) δ 141.8, 139.9, 130.4, 128.9, 128.6, 128.2, 127.7, 118.1, 22.0; IR (neat, $cm^{-1}$) 3061, 2250, 1482, 749. Anal Calcd for $C_{14}H_{11}N$: C, 87.01; H, 5.74. Found: C, 87.25; H, 5.60.

**4-Carbomethoxy-3'-acetylbiphenyl.** The coupling of methyl-4-chlorobenzoate with 3-acetylphenylboronic acid was effected using the general procedure to afford 229 mg (90%) of the title compound as a white solid, mp 109-110 °C. $^1H$ NMR (300 MHz, $CDCl_3$) δ 8.20 (s, 1H), 8.12 (d, 2H, *J* = 8.3 Hz), 7.96 (d, 1H, *J* = 7.8 Hz), 7.82 (d, 1H, *J* = 6.5 Hz), 7.68 (d, 2H, *J* = 8.8 Hz), 7.57 (t, 1H, *J* = 7.7 Hz), 3.94 (s, 3H), 2.66 (s, 3H); $^{13}C$ NMR (75 MHz, $CDCl_3$) δ 197.5, 166.6, 144.3, 140.3, 137.7, 131.5, 130.1, 129.3, 129.1, 127.9, 126.9, 126.8, 52.0, 26.6; IR (neat, $cm^{-1}$) 3003, 1722, 1679, 1293, 1111, 768. Anal Calcd for $C_{16}H_{14}O_3$: C, 75.58; H, 5.55. Found: C, 75.96; H, 5.27.

**Methyl-(4-n-hexyl)benzoate.** An oven-dried Schlenk flask was charged with 9-BBN (146 mg, 1.2 mmol) in a nitrogen-filled glovebox. The flask was capped with a rubber septum and removed from the glovebox. THF (2 mL) was added, the suspension was cooled to 0 °C, and 1-hexene (0.175 mL, 1.4 mmol) was added via syringe. The mixture was stirred at 0 °C for 5 min, then warmed to rt and stirred overnight (~14 h). The solution was then transferred *via* cannula to a separate oven-dried Schlenk flask which had been cooled to rt under an argon purge and charged with methyl 4-chlorobenzoate (171 mg, 1.0 mmol), potassium fluoride (174 mg, 3.0 mmol), $Pd(OAc)_2$ (2.2 mg, 0.01 mmol, 1 mol %), **2** (not according to the invention; 7.0 mg, 0.02 mmol, 2 mol %), and THF (1 mL). The mixture was heated to 65 °C with stirring until the starting aryl chloride had been completely consumed as judged by GC analysis (20 h). The mixture was cooled to rt, diluted with ethyl acetate (30 mL), and transferred to a separatory funnel. The mixture was washed with aqueous NaOH (2 M, 20 mL), and washed with brine (20 mL). The organic layer was concentrated *in vacuo,* and the crude material was purified by flash chromatography on silica gel to afford 186 mg (84%) of the title compound as a colorless oil. $^1H$ NMR (300 MHz, $CDCl_3$) δ 7.95 (d, 2H, *J* = 8.2 Hz), 7.24 (d, 2H, *J* = 8.2 Hz), 3.90 (s, 3H), 2.65 (t, 2H, *J* = 7.7 Hz), 1.69-1.57 (m, 2H), 1./60-1.25 (m, 6H), 0.88 (t, 3H, *J* = 6.4 Hz); $^{13}C$ NMR (75 MHz, $CDCl_3$) δ 167.2, 148.5, 129.6, 1.28.4, 127.6, 51.9, 36.0, 31.6, 31.1, 28.9, 22.6, 14.0; IR (neat, $cm^{-1}$) 2928, 2856, 1724, 1436, 1278, 1109. Anal Calcd for $C_{14}H_{20}O$: C, 76.33; H, 9.15. Found: C, 76.57; H, 9.43.

**2-Methoxybiphenyl.**[39] The coupling of 2-chloroanisole with phenylboronic acid was effected using the general procedure to afford 181 mg (98%) of the title compound as a colorless oil. $^1H$ NMR (300 MHz, $CDCl_3$) δ 7.59 (d, 2H, *J* = 8.0 Hz), 7.46 (t, 2H, *J* = 8.0 Hz), 7.40-7.35 (m, 3H), 7.08 (t, 1H, *J* = 7.4 Hz), 7.03 (d, 1H, *J* = 8.6 Hz), 3.85 (s, 3H); $^{13}C$ NMR (75 MHz, $CDCl_3$) δ 156.4, 138.5, 130.8, 130.7, 129.5, 128.6, 127.9, 126.9, 120.8, 111.2, 55.5; IR (neat, $cm^{-1}$) 3059, 3025, 2955, 2833, 1597, 1584, 1504, 1483, 1463, 1430, 1260, 1236, 1180, 1123, 1056, 1028, 1009, 754, 732, 699. Anal Calcd for $C_{13}H_{12}O$: C, 84.75; H, 6.57. Found: C, 84.43; H, 6.68.

**General Procedure for the Suzuki Coupling of Aryl Halides Using $K_3PO_4$ as Base.** An oven-dried resealable Schlenk flask was evacuated and backfilled with argon and charged with $Pd(OAc)_2$ (2.2 mg, 0.01 mmol, 1.0 mol %), ligand 4. (6.0 mg, 0.020 mmol, 2.0 mol %), the boronic acid (1.5 mmol), and $K_3PO_4$ (425 mg; 2.0 mmol). The flask was evacuated and backfilled with argon, and toluene (3 mL) and the aryl halide (1.0 mmol) were added through a rubber septum (aryl halides which were solids at rt were added prior to the second evacuation/backfill cycle). The flask was sealed with a teflon screwcap, and the reaction mixture was heated to 65 °C with stirring until the starting aryl halide had been completely consumed as judged by GC analysis. The reaction mixture was then cooled to rt, diluted with ether (30 mL) and poured into a separatory funnel. The mixture was washed with aqueous NaOH (1 M, 20 mL), and the aqueous layer was extracted with ether (20 mL). The combined organic layers were washed with brine (20. mL), dried over anhydrous magnesium sulfate, filtered, and concentrated *in vacuo.* The crude material was purified by flash chromatography on silica gel.

**2-(Diphenylketimine)-4'-ethoxybiphenyl.** The coupling of *N*-(diphenylmethylene)-2-bromoaniline with 4-ethoxyphenylboronic acid was effected using the general procedure with ligand **2** (not according to the invention) and a reaction temperature of 80 °C to afford 328 mg (87%) of the title compound as a yellow solid, mp 97-98 °C. $^1H$ NMR (250 MHz, $CDCl_3$) δ 7.64 (d, 2H, *J* = 6.7 Hz), 7.49-7.30 (m, 3H), 7.20-6.90 (m, 8H), 6.86 (d, 1H, *J* = 7.9 Hz), 6.77 (d, 2H, *J* = 6.8 Hz), 6.65 (d, 2H, *J* = 7.0 Hz), 3.99 (q, 2H, *J* = 7.0 Hz), 1.39 (t, 3H, *J* = 6.9 Hz); $^{13}C$ NMR (75 MHz, $CDCl_3$) δ 167.5, 157.4, 148.9, 139.3, 136.3, 132.2, 131.0, 130.3, 129.8, 129.1, 128.7, 128.2, 128.0, 127.3, 126.9, 63.2, 14.8; IR (neat, $cm^{-1}$)

2983, 1607, 1470, 1243, 1052. Anal Calcd for $C_{27}H_{23}NO$: C, 85.91; H, 6.14. Found: C, 85.81; H, 6.08.

**2,6-Dimethyl-4'-*t*-butylbiphenyl.** The coupling of 1-bromo-4-*t*-butylbenzene with 2,6-dimethylphenylboronic acid was effected using the general procedure (carried out on a 0.5 mmol scale) with 4 mol % **1** (not according to the invention) as the supporting ligand and a reaction temperature of 100 ˚C to afford 114 mg (96%) of the title compound as a white solid, mp 71-73 ˚C. $^1H$ NMR (300 MHz, $CDCl_3$) δ 7.43 (d, 2H, *J* = 8.2 Hz), 7:19-7.08 (m, 3H), 7.08 (d, 2H, *J* = 8.2 Hz), 2.05 (s, 6H), 1.38 (s, 9H); $^{13}C$ NMR (75 MHz, $CDCl_3$ δ 1492, 141.9, 137.8, 136.3, 128.6, 127.2, 126.8, 125.1, 34.5, 31.4, 20.9; IR (neat, $cm^{-1}$) 3034, 2952, 2864, 1507, 1462, 1397, 1362, 1113, 1002, 835, 768. Anal. Calcd for $C_{18}H_{22}$: C, 90.70; H, 9.30. Found: C, 91.05; H, 9.02.

**2,6-Dimethyl-4'-acetylbiphenyl.** The coupling of 4'-bromoacetophenone with 2,6-dimethylphenylboronic acid was effected using the general procedure (carried out on a 0.5 mmol scale) with 2 mol % $Pd(OAc)_2$ and 4 mol % **4** (not according to the invention) and a reaction temperature of 80 ˚C to afford 98 mg (87%) of the title compound as a white solid, mp 64-65 ˚C. $^1H$ NMR (300 MHz, $CDCl_3$) δ 8.13 (d, 2H, *J* = 8.2 Hz), 7.36 (d, 2H, *J* = 8.2 Hz), 7.29-7.20 (m, 3H), 2.75 (s, 3H), 2.11 (s, 6H); $^{13}C$ NMR (75 MHz, $CDCl_3$) δ 197.8, 146.4, 140.6, 135.6, 135.5, 129.4, 128.6, 127.5, 127.4, 26.6, 20.7; IR (neat, $cm^{-1}$) 2998, 2947, 1679, 1602, 1398, 1354, 1265, 1109, 1004, 956, 776. Anal Calcd for $C_{16}H_{16}O$: C, 85.68; H, 7.19. Found: C, 85.98; H, 6.93.

**2,6-Dimethyl-2'-methyl biphenyl.**[34] The coupling of 2-bromotoluene with 2,6-dimethylphenylboronic acid was effected using the general procedure (carried out on a 0.5 mmol scale) with **1** (not according to the invention) as the supporting ligand and a reaction temperature of 100 ˚C to afford 88 mg (90%) of the title compound as a colorless oil. $^1H$ NMR (300 MHz, $CDCl_3$) δ 7.34-7.24 (m, 3H), 7.22-7.12 (m, 3H), 7.06-7.03 (m, 1H), 2.00 (s, 3H), 1.98 (s, 6H); $^{13}C$ NMR (75 MHz, $CDCl_3$) δ 141.0, 140.4, 135.8, 135.5, 129.9, 128.7, 127.1, 126.9, 126.8, 126.0, 20.4, 19.4; IR (neat, $cm^{-1}$) 3060, 1583, 1462, 1120, 760. Anal Calcd for $C_{15}H_{16}$: C, 91.78; H, 8.22. Found: C, 91.57; H, 8.20.

**2,6-Dimethyl-2'-methyl biphenyl.**[34] The coupling of 2-chloro-*m*-xylene with *o*-tolylboronic acid was effected using the general procedure with **5** (not according to the invention) as the supporting ligand and a reaction temperature of 100 ˚C to afford 180 mg (92%) of the title compound as a colorless oil.

**2,6-Dimethyl-2'-methyl biphenyl.**[34] The coupling of 2-chloro-*m*-xylene with *o*-tolylboronic acid was effected using the general procedure with **2** (not according to the invention) as the supporting ligand and a reaction temperature of 100 ˚C to afford 174 mg (89%) of the title compound as a colorless oil.

**2-Methoxy-2'-acetylbiphenyl.** The coupling of 2-chloroacetophenone with 2-methoxyphenyl boronic acid was effected using the general procedure to afford 201 mg (89%) of the title compound as a white solid, mp 83 ˚C. $^1H$ NMR (300 MHz, $CDCl_3$) δ 7.62 (d, 1H, *J* = 7.7 Hz), 7.54-7.49 (m, 1H), 7.42-7.26 (m, 4H), 7.06 (t, 1H, *J* = 7.3 Hz), 6.92 (d, 1H, *J* = 8.2Hz), 3.73 (s, 3H), 2.17 (s, 3H); $^{13}C$ NMR (75 MHz, $CDCl_3$) δ 202.2, 155.8, 140.7, 136.7, 131.1, 130.8, 130.5, 129.9, 129.3, 127.3; 127.1, 121.0, 110.6, 55.0, 28.8; IR (neat, $cm^{-1}$) 3003, 1695, 1243, 757. Anal Calcd for $C_{15}H_{14}O_2$: C, 79.62; H, 6.24. Found: C, 79.89; H, 6.02.

**2,5-Dimethyl-2'-Methylbiphenyl.** The coupling of 2-chloro-*p*-xylene with *o*-tolylboronic acid was effected using the general procedure with **2** (not according to the invention) as the supporting ligand and a reaction temperature of 80 ˚C to afford 185 mg (94%) of the title compound a colorless oil. $^1H$ NMR (300 MHz, $CDCl_3$) δ 7.26-7.04 (m, 6H), 6.93 (s, 1H), 2.33 (s, 3H), 2.06 (s, 3H), 2.00 (s, 3H); $^{13}C$ NMR (75 MHz, $CDCl_3$) δ 141.7, 141.4, 135.8, 134.9, 132.6, 129.9, 129.7, 129.6, 129.2, 127.8, 127.0, 125.5, 20.9, 19.8, 19.3; IR (neat, $cm^{-1}$) 3041, 2921, 1482, 1032, 810. Anal Calcd for $C_{15}H_{16}$: C, 91.78; H, 8.22. Found: C, 91.68; H, 8.17.

**2,6-Dimethylbiphenyl.**[34] The coupling of 2-chloro-*m*-xylene with phenylboronic acid was effected using the general procedure with **1** (not according to the invention) as the supporting ligand and a reaction temperature of 100 ˚C to afford 164 mg (90%) of the title compound as a colorless oil. **2,6-Dimethylbiphenyl.**[34] The coupling of 2-chloro-*m*-xylene with phenylboronic acid was effected using the general procedure with **2** as the supporting ligand and a reaction temperature of 100 ˚C to afford 135 mg (85%) of the title compound as a colorless oil. A small amount of the starting aryl chloride (7%) was not consumed during the course of the reaction.

**General Procedure for the Suzuki Coupling of Aryl Halides at Low Catalyst Loadings (0.1 mol % Pd)** An oven-dried resealable Schlenk flask was evacuated and backfilled with argon and charged with the boronic acid (1.5 mmol) and $K_3PO_4$ (2.0 mmol). The flask was evacuated and backfilled with argon, and THF (1.5 mL) and the aryl halide (1.0 mmol) were added through a rubber septum. A separate flask was charged with $Pd_2(dba)_3$ (4.6 mg, 0.005 mmol) and ligand **4** (not according to the invention; 4.5 mmol, 0.015 mmol), and was purged with argon. THF (1 mL) was added, the mixture was stirred for 1 min at rt, then 100 μL of this solution (0.1 mol % Pd, 0.15 mol % ligand **4**) was added to the Schlenk flask followed by additional THF (1.5 mL). The septum was removed; the flask was sealed with a teflon screwcap and the mixture was stirred at rt for 2 min, then heated to 65 ˚C with stirring until the starting aryl bromide had been completely consumed as judged by GC analysis. The reaction mixture was then cooled to rt, diluted with ether (20 mL) and poured into a separatory funnel. The mixture was washed with aqueous NaOH (1 M, 20 mL), and the layers were separated. The aqueous layer was extracted with ether (20 mL), and the combined organic extracts were dried over anhydrous magnesium sulfate, filtered, and concentrated *in vacuo.* The crude material was then purified by flash chromatography on silica gel.

**4-*t*-Butylbiphenyl**[39] (0.1 mol % Pd, $K_3PO_4$ as base). The coupling of 1-bromo-4-*t*-butylbenzene with phenylboronic acid was effected using the general procedure to afford 199 mg (95%) of a glassy solid, mp 47-49 ˚C (lit mp 51-52 ˚C):[39b] $^1H$ NMR (300 MHz, $CDCl_3$) δ 7.58-7:51 (m, 4H), 7.46-7.38 (m, 4H), 7.30-7.26 (m, 1H), 1.34 (s, 9H); $^{13}C$ NMR (75 MHz, $CDCl_3$) δ 150.2, 141.1, 138.3, 128.7, 127.0, 126.9, 126.8, 125.7, 34.5, 31.4; IR (neat, $cm^{-1}$) 2961, 1486, 834, 764. Anal. Calcd for $C_{16}H_{18}$: C, 91.37; H, 8.63. Found: C, 91.42; H, 8.69.

**4-*t*-Butylbiphenyl**[39] (0.05 mol % Pd, CsF as base). The coupling of 1-bromo-4-*t*-butylbenzene with phenylboronic acid was effected using the general procedure with CsF (3.0 mmol) as the base and a 50 μL of a catalyst solution comprised of $Pd_2(dba)_3$ (4.6 mg, 0.005 mmol), ligand **4** (4.5 mmol, 0.015 mmol), in THF (1 mL) to afford 202 mg (96%) of a glassy solid.

**4-*t*-Butylbiphenyl**[39] (0.02 mol % Pd, $K_3PO_4$ as base). The coupling of 1-bromo-4-*t*-butylbenzene with phenylboronic acid was effected using the general procedure with toluene solvent, a reaction temperature of 80 ˚C, and 20 μL of a catalyst solution comprised of $Pd(OAc)_2$ (2.2 mg, 0.01 mmol), ligand **4** (6.0 mg, 0.02 mmol), and THF (2 mL) to afford 196 mg (93 %) of a glassy solid.

**4-*t*-Butylbiphenyl**[39] (0.005 mol % Pd, $K_3PO_4$ as base). The coupling of 1-bromo-4-*t*-butylbenzene with phenylboronic acid was effected using the general procedure with toluene solvent, a reaction temperature of 100 ˚C, and 50 μL of a catalyst solution comprised of $Pd(OAc)_2$(2.2mg, 0.01 mmol), ligand **2** (not according to the invention; 7.0 mg, 0.02 mmol), and THF (10mL) to afford 198 mg (94%) of a glassy solid.

**4-*t*-Butylbiphenyl**[39] (0.001 mol % Pd, $K_3PO_4$ as base). The coupling of 1-bromo-4-*t*-butylbenzene with phenylboronic acid was effected using the general procedure with toluene solvent, a reaction temperature of 100 ˚C, and 10 μL of a catalyst solution comprised of $Pd(OAc)_2$ (2.2 mg, 0.01 mmol), ligand **2** (not according to the invention; 7.0 mg, 0.02 mmol), and THF (10 mL). When the reaction was no longer progressing, the mixture was cooled to room-temperature and dodecane (0.23 mL) was added as an internal standard; GC analysis showed 93% conversion (96% GC yield).

**4-*t*-Butylbiphenyl**[39] (0.001 mol % Pd, $K_3PO_4$ as base, phosphite ligand). The coupling of 1-bromo-4-*t*-butylbenzene with phenylboronic acid was effected using the general procedure with toluene solvent, a reaction temperature of 100 ˚C, and 10 μL of a catalyst solution comprised of $Pd(OAc)_2$ (2.2 mg, 0.01 mmol), tris(2,4-di-*t*-butylphenyl)phosphite (13.0 mg, 0.02 mmol), and THF (10 mL). When the reaction was no longer progressing, the mixture was cooled to room-temperature and dodecane (0.23 mL) was added as an internal standard; GC analysis showed 40% conversion (42% GC yield).

**4-*t*-Butylbiphenyl**[39] (0.005 mol % Pd, $K_3PO_4$ as base, phosphite ligand). The coupling of 1-bromo-4-*t*-butylbenzene with phenylboronic acid was effected using the general procedure with toluene solvent, a reaction temperature of 100 ˚C, and 50 μL of a catalyst solution comprised of $Pd(OAc)_2$ (2.2 mg, 0.01 mmol), tris(2,4-di-*t*-butylphenyl)phosphite (13.0 mg, 0.02 mmol), and THF (10 mL). When the reaction was no longer progressing, the mixture was cooled to room-temperature and dodecane (0.23 mL) was added as an internal standard; GC analysis showed 47% conversion (49% GC yield).

**2-Methyl-4'-t-butylbiphenyl** (0.1 mol % Pd, $K_3PO_4$ as base). The coupling of 1-bromo-4-*t*-butylbenzene with *o*-tolylboronic acid was effected using the general procedure with 100 μL of a catalyst solution comprised of $Pd_2(dba)_3$ (4.6 mg, 0.005 mmol), ligand **4** (4.5 mmol, 0.015 mmol), and THF (1 mL) to afford 210 mg (94%) of a colorless oil: $^1H$ NMR (300 MHz; $CDCl_3$) δ 7:42 (d, 2H, *J* = 8.2 Hz), 7.27-7.22 (m, 6H), 2.29 (s, 3H), 1.37 (s, 9H), $^{13}C$ NMR (75 MHz, $CDCl_3$) δ 149.5, 141.9, 139.0, 135.4, 130.3, 129.9, 128.8, 127.0, 125.7, 124.9, 34.5, 31.4, 20.5; IR (neat, $cm^{-1}$) 2961, 1482, 1112, 838. Anal. Calcd for $C_{17}H_{20}$: C, 91.01; H, 8.99. Found: C, 91.11; H, 9.21.

**2-Methyl-4'-*t*-butylbiphenyl** (0.005 mol % Pd, $K_3PO_4$ as base). The coupling of 1-bromo-4-*t*-butylbenzene with *o*-tolylboronic acid was effected using the general procedure with toluene solvent, a reaction temperature of 100 ˚C, and 50 μL of a catalyst solution comprised of $Pd(OAc)_2$ (2.2 mg, 0.01 mmol), ligand **2** (not according to the invention; 7.0 mg, 0.02 mmol), and THF (10 mL) to afford 216 mg (96%) of a glassy solid.

**2-Methyl-4'-*t*-butylbiphenyl** (0.005 mol % Pd, $K_3PO_4$ as base, phosphite ligand). The coupling of 1-bromo-4-*t*-butylbenzene with *o*-tolylboronic acid was effected using the general procedure with toluene solvent, a reaction temperature of 100˚C; and 50 μL of a catalyst solution comprised of $Pd(OAc)_2$ (2.2 mg, 0.01 mmol), tris(2,4-di-*t*-butylphenyl)phosphite (13.0 mg, 0.02 mmol), and THF (10 mL). When the reaction was no longer progressing, the mixture was cooled to room-temperature and dodecane (0.23 mL) was added as an internal standard; GC analysis showed 87% conversion (84% GC yield).

**2-Methoxy-3-(1,3-dioxolane)-biphenyl** (0.005 mol % Pd, $K_3PO_4$ as base). The coupling of 2-(3-bromophenyl)-1,3-dioxolane with *o*-methoxyphenylboronic acid was effected using the general procedure with toluene solvent, a reaction temperature of 100˚C, and 50 μL of a catalyst solution comprised of $Pd(OAc)_2$ (2.2 mg, 0.01 mmol), ligand **2** (not according to the invention; 7.0 mg, 0.02 mmol), and THF (10 mL) to afford 223 mg (87%) of a glassy solid. See above for NMR data.

**4-Acetylbiphenyl**[32] (0.02 mol % Pd, $K_3PO_4$ as base, from the aryl chloride). The coupling of 4-chloroacetophenone with phenylboronic acid was effected using the general procedure with toluene solvent, a reaction temperature of 100 ˚C, and 100 μL of a catalyst solution comprised of $Pd(OAc)_2$ (2.2 mg, 0.01 mmol), ligand **4** (6.0 mg, 0.02 mmol), and THF (5 mL) to afford 178 mg (91%) of the title compound as a white solid, mp 120-121 ˚C (lit mp 109-110 ˚C):[32] $^1H$ NMR

(300 MHz, $CDCl_3$) δ 8.03 (d, 2H, *J* = 8.6 Hz), 7.71-7.62 (m, 4H), 7.50-7.40 (m, 3H), 2.64 (s, 3H); $^{13}C$ NMR (75 MHz, $CDCl_3$) δ 197.5, 145.7, 139.8, 135.9, 128.9, 128.8, 128.2, 127.2, 127.1, 26.5; IR (neat, $cm^{-1}$) 2999, 1679, 764. Anal. Calcd for $C_{14}H_{12}O$: C, 85.68; H, 6.16. Found: C, 85.62; H, 6.07.

**4-Acetylbiphenyl**[32] (0.001 mol % Pd, $K_3PO_4$ as base, from the aryl bromide). The coupling of 4-bromoacetophenone with phenylboronic acid was effected using the general procedure with toluene solvent, a reaction temperature of 100 ˚C, and 50 μL of a catalyst solution prepared as follows: a flask was charged with $Pd_2(dba)_3$ (4.6 mg, 0.005 mmol) and ligand **4** (4.5 mg, 0.015 mmol) and was purged with argon. THF (5 mL) was added and the mixture was stirred for 1 min at rt, then 50 μL of this solution (0.01 mol % Pd, 0.02 mol % **4**) was added to a second flask containing 1 mL THF. The title compound was obtained as a white solid (187 mg, 95%).

**4-Acetylbiphenyl**[32] (0.000001 mol % Pd, $K_3PO_4$ as base, from the aryl bromide).

The coupling of 4-bromoacetophenone with phenylboronic acid was effected using the general procedure with toluene solvent, a reaction temperature of 100 ˚C, and 10 μL of a catalyst solution prepared as follows: in a flask in a argon filled glovebox, $Pd(OAc)_2$ (4.5 mg, 0.02 mmol) and ligand **4** (12.0 mg, 0.04 mmol) were dissolved in THF (20 mL) under argon. A portion of this solution (10 μL, 0.00001 mmol Pd, 0.001 mol % Pd, 0.002 mol % **4**) was added to a second flask containing THF (10 mL). The title compound was obtained as a white solid (176 mg, 90 %).

**4-Acetylbiphenyl**[32] (0.001 mol % Pd, no ligand). The coupling of 4-bromoacetophenone with phenylboronic acid was effected using the general procedure with toluene solvent, a reaction temperature of 100 ˚C, and 10 μL of a catalyst solution comprised of $Pd(OAc)_2$ (2.2 mg, 0.01 mmol) and THF (10 mL). When the aryl halide had been completely consumed, the mixture was cooled to room-temperature and dodecane (0.23 mL) was added as an internal standard; the GC yield was determined to be 101%.

**4-Methyl biphenyl**[27] (0.1 mol % Pd, ligand **4**). The coupling of 4-chlorotoluene with phenylboronic acid was effected using the general procedure with toluene solvent, a reaction temperature of 100 ˚C, and 100 μL of a catalyst solution comprised of $Pd(OAc)_2$ (4.5 mg, 0.02 mmol), ligand **4** (12.0 mg, 0.04 mmol), and THF (2 mL) to afford 161 mg (96%) of the title compound.

**4-Methyl biphenyl**[27] (0.05 mol % Pd, ligand 5). The coupling of 4-chlorotoluene with phenylboronic acid was effected using the general procedure with toluene solvent, a reaction temperature of 100 ˚C, and 100 μL of a catalyst solution comprised of $Pd(OAc)_2$ (2.2 mg, 0.01 mmol), ligand **2** (not according to the invention; 7.0 mg, 0.02 mmol), and THF (2 mL) to afford 158 mg (94%) of the title compound.

*References and Notes for Example 100*

(1) Suzuki, A. in *Metal-Catalyzed Cross-Coupling Reactions*; Diederich, F; Stang, P.J. Eds.; Wiley-VCH: Weinheim, Germany, 1998; Ch. 2.

(2) Grushin, V. V.; Alper, H. *Chem. Rev.* **1994,** *94*, 1047-1062.

(3) (a) Shen, W. *Tetrahedron Lett.* **1997,** *38*, 5575-5578; (b) Beller, M.; Fischer, H.; Herrmann, W. A.; Öfele, K.; Brossmer, C. *Angew. Chem. Int. Ed. Engl.* **1995**, *34*, 1848-1849; (c) Bumagin, N. A.; Bykov. V. V. *Tetrahedron* **1997,** *53*, 14437-14450; (d) Mitchell, M. B.; Wallbank, P. J. *Tetrahedron Lett.* **1991,** *32*, 2273-2276; (e) Firooznia, F.; Gude, C.; Chan, K.; Satoh, Y. *Tetrahedron Lett.* **1998,** *39*, 3985-3988; (f) Cornils, B. *Orgn. Proc. Res. Dev.* **1998**, *2*, 121-127; (h) Indolese, A. F. *Tetrahedron Lett.* **1997,** *38*, 3513-3516; (i) Saito, S.; Oh-tani, S.; Miyaura, N. *J. Org. Chem.* **1997,** *62*, 8024-8030; (j) Bei, X.; Crevier, T.; Guram, A.S.; Jandeleit, B.; Powers, T. S.; Turner, H. W.; Uno, T.; Weinberg, W. H. *Tetrahedron Lett.* **1999**, *40*, 3855-3858; (k) Herrmann, W. A.; Reisinger, C. -P.; Spiegler, M. *J. Organomet. Chem.* **1998,** *557,* 93-96; (1) Zhang, C.; Huang, J.; Trudell, M. L.; Nolan, S. P. *J. Org, Chem.* **1999,** *64,* 3804-3805.

(4) Fu has recently reported the Suzuki coupling of electron rich aryl chlorides using palladium complexes with P $(t\text{-Bu})_3$ as the supporting ligand. Littke, A. F.; Fu, G. C. *Angew. Chem. Int. Ed. Engl.* **1998,** *37*, 3387-3388.

(5) Genêt has recently reported a biphasic system for the nickel-catalyzed Suzuki coupling of aryl chlorides. In this report he described the synthesis of 2,6,2'-methyl-4'-acetylbiphenyl in 67% yield using 10 mol % $(dppe)NiCl_2$ and 50 mol % of a sulfonated triphenylphosphine ligand. See: Galland, J. -C.; Savignac, M.; Genêt, J. -P. *Tetrahedron Lett.* **1999,** *40*, 2323-2326.

(6) (a) Campi, E. M.; Jackson, W. R.; Marcuccio, S. M.; Naeslund, C. G. M *J. Chem. Soc., Chem. Commun* **1994,** 2395; (b) Anderson, J. C.; Namli, H.; Roberts, C. A. *Tetrahedron* **1997,** *53*, 15123-15134; (c) Uenishi, J.-i.; Beau,

J. -M.; Armstrong, R. W.; Kishi, Y. *J. Am. Chem. Soc.* **1987**, *109,* 4756-4758.

(7) Old, D. W.; Wolfe, J. P.; Buchwald, S. L. *J. Am. Chem. Soc.* **1998**,*120*, 9722-9723.

(8) Aranyos, A.; Old, D. W.; Kiyomori, A.; Wolfe, J. P.; Sadighi, J. P.; Buchwald, S. L. *J. Am. Chem. Soc.* **1999**, *121*, 4369-4378.

(9) (a) Portions of this work have been previously communicated. See: Wolfe, J. P.; Buchwald, S. L. *Angew. Chem. Int. Ed* In press. (b) The 4/$Pd(OAc)_2$ catalyst system has also been shown to be effective for room-temperature catalytic amination of aryl chlorides; see ref 9a.

(10) Ligands **2** and **4** are now commercially available from Strem Chemical Co.

(11) Wright, S. W.; Hageman, D. L.; McClure, L. D. *J. Org. Chem.* **1994,** *59,* 6095-6097.

(12) Miyaura, N.; Ishiyama, T.; Sasaki, H.; Ishikawa, M.; Satoh, M.; Suzuki, A. *J. Am. Chem. Soc.* **1989,** *111,* 314-321.

(13) Primary and secondary alkoxides are known to reduce aryl halides in the presence of palladium catalysts. Zask, A.; Helquist, P. *J. Org. Chem.* **1978,** *43*, 1619.

(14) Albisson, D. A.; Bedford, R. B.; Lawrence, S. E.; Scully, P. N. *J. Chem. Soc. Chem. Commun.* **1998,** 2095-2096.

(15) (a) Bringmann, G.; Götz, R.; Keller, P. A.; Walter, R.; Boyd, M. R.; Lang, F.; Garcia, A.; Walsh, J. J.; Tellitu, I.; Bhaskar, K. V.; Kelly, T. R. *J. Org. Chem.* **1998,** *63,* 1090-1097; (b) Zhang, H.; Kwong, F. Y.; Tian, Y.; Chan, K. S. *J. Org. Chem.* **1998,** *63,* 6886-6890; (c) Johnson, M. G.; Foglesong, R. J. *Tetrahedron Lett.* **1997**, *38*, 7001-7002; (d) Hoye, T. R.; Chen, M. *J. Org. Chem.* **1996,** *61,* 7940-7942; (e) Bahl, A.; Grahn, W.; Stadler, S.; Feiner, F.; Bourhill, G.; Bräuchle, C.; Reisner, A.; Jones, P. G. *Angew. Chem. Int. Ed. Engl.* **1995**, *34*, 1485-1488; (f) Watanabe, T.; Miyaura, N.; Suzuki, A. *Synlett* **1992**, 207-210; (g) Katz, H. *J. Org. Chem.* **1987,** *52*, 3932-3934.

(16) No catalyst has been reported which is effective for Suzuki coupling reactions to form biaryls with four ortho substituents; to the best of our knowledge, only one isolated yield (12%) has been for the synthesis of a tetrasubstituted biaryl (using Suzuki coupling) has been reported. See ref 15c.

(17) Herrmann, W. A.; BroBmer, C.; Priermeier, T.; Öfele, K. *J. Organomet. Chem.* **1994**, *481*, 97-108.

(18) (a) It is well known that the use of electron-rich phosphine ligands accelerates the rate of oxidative addition of aryl halides to Pd(0). See: Spessard, G. O.; Meissler, G. L. *Organometallic Chemistry* Prentice-Hall: Upper Saddle River, New Jersey , 1996; pp 171-175. (b) In his pioneering studies, Milstein demonstrated oxidative addition of aryl chlorides to $Pd(dippp)_2$ (dippp=1,3-bis(diisopropylphosphino)propane) at 38°C. Portnoy, M.; Milstein, D. *Organometallics* **1993**, *12*, 1665-1673.

(19) Hegedus, L. S. *Transition Metals in The Synthesis of Complex Organic Molecules,* University Science Books: Mill Valley, CA, 1994, Ch 2.

(20) (a) Nishiyama, M.; Yamamoto, T.; Koie, Y. *Tetrahedron Lett.* **1998, 39,** 617-620; (b) Yamamoto, T.; Nishiyama, M.; Koie, Y. *Tetrahedron Lett.* **1998**, *39*, 2367-2370.

(21) Metal-π interactions have been observed in other palladium complexes. (a) Ossor, H.; Pfeffer, M.; Jastrzebski, J. T. B. H.; Stam, C. H. *Inorg. Chem.* **1987**, *26,* 1169-1171; (b) Falvello, L. R; Fornies, J.; Navarro, R.; Sicilia, V.; Tomas, M. *Angew. Chem. Int. Ed. Engl.* **1990**, *29*, 891-893; (c) Sommovigo, M.; Pasquali, M.; Leoni, P.; Braga, D.; Sabatino, P. *Chem. Ber.* **1991**, *124,* 97-99; (d) Li, C. -S.; Cheng, C. -H.; Liao, F. -L.; Wang, S. -L. *J. Chem. Soc., Chem. Commun.* **1991**, 710-711.(e) Kannan, S.; James, A. J.; Sharp, P. R. *J. Am. Chem. Soc.* **1998***, 120*, 215-216.

(22) Monophosphine palladium complexes have been demonstrated to be catalytically active intermediates in other palladium-catalyzed processes. Oxidative addition, transmetallation, and reductive elimination often proceed at higher rates if coordinatively unsaturated intermediates are accessible. See (a) Farina, V. in *Comprehensive Organometallic Chemistry,* 2nd Ed, Pergamon Press: Oxford, 1995, Vol 12, pp 161-240; (b) Hartwig, J. F. *Synlett* **1997**, 329-340 and references cited therein.

(23) A detailed mechanistic study on both Suzuki coupling and catalytic amination will be reported separately.

(24) Biaryl-forming reductive elimination from Pt(II) has been postulated to occur via a transition state in which both arenes are perpendicular to the coordination plane. See: Braterman, P. S.; Cross, R. J.; Young, G. B. *J. Chem. Soc. Dalton Trans. I.* **1977**, 1892-1897.

(25) Sadighi, J. P.; Singer, R. A.; Buchwald, S. L. *J. Am. Chem. Soc.* **1998**, *120*, 4960-4976.

(26) Hegedus, L. S. in *Organometallics in Synthesis;* Schlosser, M. Ed.; John Wiley and Sons: West Sussex, England, 1994; p 448.

(27) Schoevarrs, A. M.; Kruizinga, W.; Zijlsbra, R. W. J.; Veldman, N.; Spek, A. L.; Feringa, B. L. *J. Org. Chem.* **199,** *62*, 4943-4948.

(28) Reactions in which argon purges were used instead of the evacuation/backfill cycles (at all points required in the procedure) gave similar results.

(29) Bourelle-Wargnier, F.; Vincent, M.; Chuche, J. *J. Org. Chem.* **1980,** *45*, 428-435.

(30) Badr, M. Z. A.; Aly, M. M.; Fahmy, A. M. *J. Org. Chem.* **1981,** *46*, 4784-4787.

(31) Palik, E. C.; Platz, M. S. *J. Org. Chem.* **1983**, *48*, 963-969.

(32) Häfelinger, G.; Beyer, M.; Burry, P.; Eberle, B.; Ritter, G.; Westermayer, G.; Westermayer, M. *Chem. Ber.* **1984,** *117,* 895-903.

(33) Pelter, A.; Jenkins, I.; Jones, D. E. *Tetrahedron* **1997,** *53,* 10357-10400.

(34) Kamikawa, T.; Hayashi, T. *Synlett* **1997,** 163-164.

(35) Klement, I.; Rottländer, M.; Tucker, C. E.; Majid, T. N.; Knochel, P. *Tetrahedron* **1996**, *52*, 7201-7220.

(36) *Dictionary of Organic Compounds* 6th Ed*.,* Cadogan, J.I. G; Ley, S. V.; Pattenden, G.; Raphael, R. A.; Rees, C. W., eds.; Champan & Hall: London, **1996**, Vol 5, p 4765.

(37) Auria, M. *Tetrahedron Lett.* **1995**, *36*, 6567-6570.

(38) Rieke, R. D.; Daruwala, K. P. *J. Org. Chem.* **1988,** *53*, 2073-2076.

(39) Lipshutz, B. H.; Siegmann, K.; Garcia, E.; Kayser, F. *J. Am. Chem. Soc.* **1993,** *115,* 9276-9282.

(40) (a) Rieke, R D.; Schulte, L. D.; Dawson, B. T.; Yang, S. S. *J. Am. Chem. Soc.* **1990,** *112,* 8388-8398; (b) Clark, F. R. S.; Norman, R. O. C.; Thomas, C. B.; Willson, J. S. *J. Chem. Soc., Perkin Trans. I* **1974,** 1289-1294.

(41) Barba, I.; Chinchilla, R.; Gomez, C. *Tetrahedron* **1990,** *46***,** 7813-7822.

***Example 101***

Asymmetric Suzuki coupling to form axially chiral biaryls

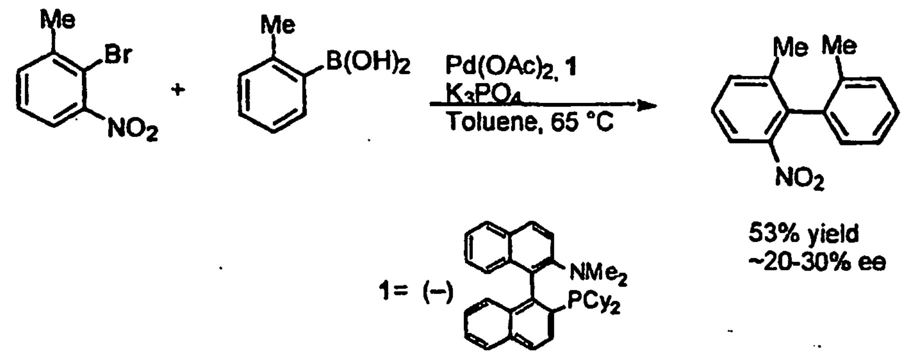


An oven-dried Schlenk flask was evacuated and backfilled with argon and charged with 2-bromo-3-nitrotoluene (108 mg, 0.5 mmol), *o*-tolylboronic acid (102 mg, 0.75 mmol), $K_3PO_4$ (212 mg, 1.0 mmol), $Pd(OAc)_2$ (1.1 mg, 0.005 mmol, 1 mol %), and **1** (according to the invention; 4.9 mg, 0.01 mmol, 2 mol %). The flask was evacuated and backfilled with argon and toluene (1.5 mL) was added through a rubber septum. The flask was sealed with a teflon screwcap and heated to 65 - ˚C with stirring for 20 h, at which time GC analysis showed ~86% conversion. The mixture was cooled to rt, diluted with ether (50 mL) and transferred to a separatory funnel. The mixture was washed with 1 M NaOH (20 mL). The layers were separated and the aqueous layer was extracted with ether (20 mL). The combined organic layers were dried over anhydrous magnesium sulfate, filtered, and concentrated in vacuo. The crude material was purified by flash chromatography on silica gel to afford 60 mg (53%) of the product as a colorless oil. $\alpha_d^{25}$ =+49.6 ˚ (c=0.8) [lit $\alpha_d^{25}$=+153.5 ˚ (c=0.82)].[1] HPLC analysis of the product showed the ee to be ~20-30% (separation of the enantiomers was imperfect). (1) Melillo, J.T.; Mislow, K. *J. Org. Chem.* **1965**, *30*, 2149.

***Example 102***

Palladium-catalyzed cross-coupling of an aryl bromide with an arylzinc bromide

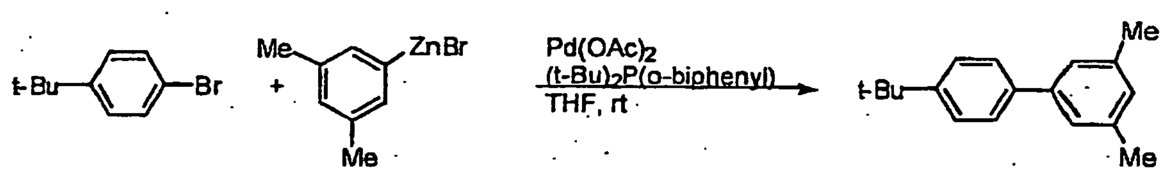


An oven-dried Schlenk flask was purged with argon and charged with 5-bromo-*m*-xylene (1.36 mL, 10.0 mmol), and THF (10 mL) and was cooled to -78 ˚C. A solution of *n*-butyllithium in hexanes (6.25 mL, 1.6 M, 10.0 mmol) was added dropwise. The mixture was stirred at -78 ˚C for 45 min, then a solution of zinc bromide (2.25 g, 10.0 mmol) in THF (5 mL) was added dropwise. The solution was warmed to 0 ˚C. A separate Schlenk flask was charged with Pd $(OAc)_2$ (2.2 mg, 0.01 mmol) and $(t\text{-Bu})_2P$(*o*-biphenyl) (not according to the invention; 6.0 mg, 0.02 mmol), and was evacuated and backfilled with argon. THF (1 mL), 4-*t*-butylbromobenzene (0.17 mL, 1.0 mmol), and a portion of the arylzinc reagent (2.8 mL, 1.3 mmol) were added to the flask. The flask was sealed with a teflon screwcap and stirred at room temperature. GC analysis of the reaction mixture after 16 h showed that the starting aryl bromide had been completely consumed and the desired product had formed. The identity of the product was confirmed by GC/MS.

***Example 103***

Palladium-catalyzed cross-coupling of an aryl bromide with benzylzinc bromide

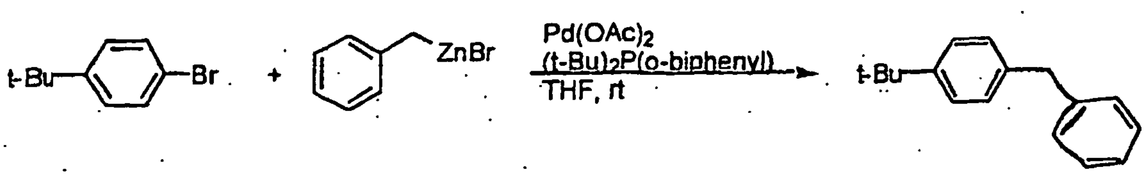


An oven-dried Schlenk flask was charged with zinc dust (763 mg, 12.0 mmol) in a nitrogen-filled glovebox. The flask was removed from the glovebox, charged with THF (1 mL), and cooled to 0 ˚C. A solution of benzyl bromide (0.95 mL, 8.0 mmol) in THF (6 mL) was added dropwise to the flask. The mixture was stirred at 0 ˚C for 1h, at which time GC analysis showed that the benzyl bromide had been completely consumed. A separate Schlenk flask was charged with $Pd(OAc)_2$ (2.2 mg, 0.01 mmol) and ($t$-Bu)$_2$P($o$-biphenyl) (not according to the invention; 6.0 mg, 0.02 mmol), and was evacuated and backfilled with argon. THF (1 mL), 4-$t$-butylbromobenzene (0.17 mL, 1.0 mmol), and a portion of the benzylzinc reagent (1.4 mL, 1.3 mmol) were added to the flask. The flask was sealed with a teflon screwcap and stirred at room temperature. GC analysis of the reaction mixture 21 h showed that the reaction had proceeded to ~99% conversion. The desired product had formed, and it's identity was confirmed by GC/MS.

***Example 104***

Palladium-catalyzed cross-coupling of an aryl chloride with benzylzinc bromide

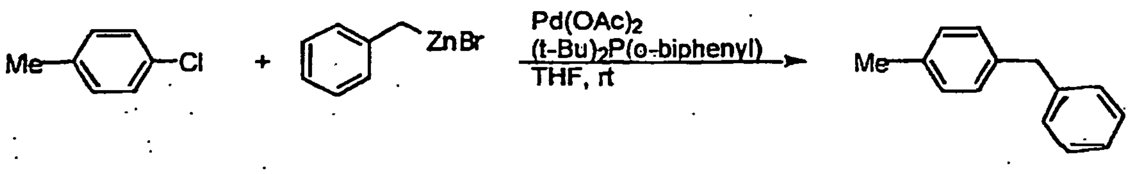


An oven-dried Schlenk flask was charged with zinc dust (763 mg, 12.0 mmol) in a nitrogen-filled glovebox. The flask was removed from the glovebox, charged with THF (1 mL), and cooled to 0 ˚C. A solution of benzyl bromide (0.95 mL, 8.0 mmol) in THF (6 mL) was added dropwise to the flask. The mixture was stirred at 0 ˚C for 1h, at which time GC analysis showed that the benzyl bromide had been completely consumed. A separate Schlenk flask was charged with $Pd(OAc)_2$ (2.2 mg, 0.01 mmol) and ($t$-Bu)$_2$P($o$-biphenyl) (not according to the invention; 6.0 mg, 0.02 mmol), and was evacuated and backfilled with argon. THF (1 mL), 4-chlorotoluene (0.12 mL, 1.0 mmol), and a portion of the benzylzinc reagent (1.4 mL, 1.3 mmol) were added to the flask. The flask was sealed with a teflon screwcap and stirred at room temperature. GC analysis of the reaction mixture after 21 h showed that the reaction had proceeded to ~21% conversion. The desired product had formed, and it's identity was confirmed by GC/MS.

***Example 105***

Synthesis of a tetrasubstituted biaryl from an aryl chloride

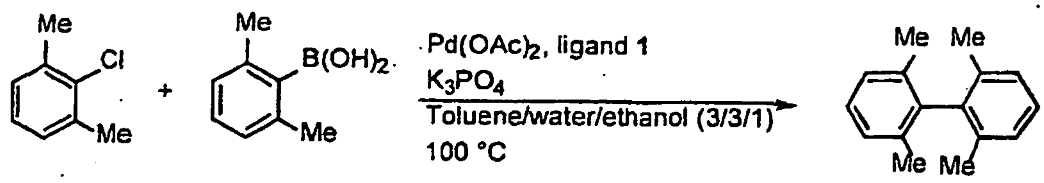


73% conversion

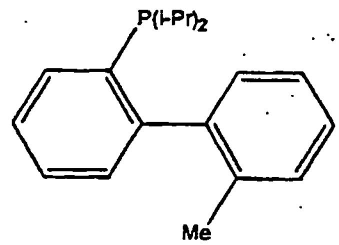

1

**2,2',6,6'-tetramethylbiphenyl** An oven-dried resealable Schlenk flask was evacuated and backfilled with argon. The flask was charged with 2,6-dimethylphenyl boronic acid (224 mg, 1.5 mmol), $K_3PO_4$ (425 mg, 2.0 mmol), $Pd(OAc)_2$ (2.2 mg, 0.01 mmol), and ligand **1** (not according to the invention; 11.4 mg, 0.04 mmol). The tube was evacuated and backfilled with argon and toluene (1 mL), degassed water (1 mL), ethanol (0.3 mL), and 2-chloro-m-xylene (0.13 mL, 1.0 mmol) were added via syringe. The mixture was heated to 100 ˚C for 45 h at which time GC analysis showed that the reaction had proceeded to 73% conversion; the desired product was detected by GC and GC/MS.

***Example 106***

Suzuki Coupling of 1-Chlorocyclopentene with Phenylboronic acid

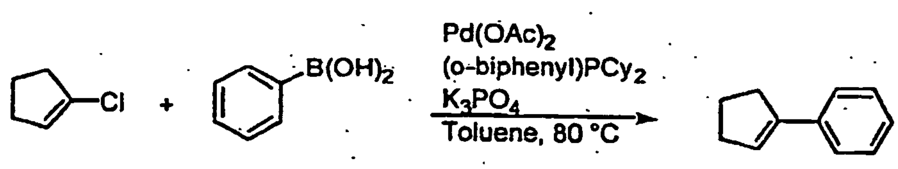


An oven-dried resealable Schlenk flask was evacuated and backfilled with argon. The flask was charged with $Pd(OAc)_2$ (2.2 mg, 0.01 mmol, 1 mol %), (*o*-biphenyl)$PCy_2$ (not according to the invention; 7.0 mg, 0.02 mmol, 2 mol %), phenylboronic acid (183 mg, 1:5 mmol), and $K_3PO_4$ (425 mg, 2.0 mmol). The tube was evacuated and backfilled with argon and toluene (3 mL) and 1-chlorocyclopentene (0.10 mL, 1.0 mmol) were added through a rubber septum. The tube was sealed with a teflon screwcap and the mixture was heated to 80 ˚C with stirring until the starting aryl chloride had been completely consumed as judged by GC analysis. The mixture was cooled to room temperature, diluted with ether (30 mL), and transferred to a separatory funnel. The mixture was washed with 1M aqueous NaOH (20 mL), and the aqueous layer was extracted with ether (20 mL). The combined organic layers were dried over anhydrous magnesium sulfate, filtered, and concentrated in vacuo. The crude material was purified by flash chromatography on silica gel to afford 118 mg (82%) of 1-phenylcyclopentene as a colorless oil.

***Example 107***

Synthesis of cyclohexyl-(triethyl)methyl-*o*-biphenyl phosphine

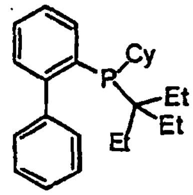

Magnesium turnings (15 g, 0.6 mol) were placed into an flame-dried, round-bottom Schlenk flask The reaction vessel was fitted with a rubber septum and purged with argon. 45 mL ether was added via a syringe followed by 0.5 mL 1,2-dibromoethane. The reaction mixture was stirred for 15 minutes at room temperature then the stirring was switched off, and 2-chloro-2-ethyl pentane (6 mL, 40 mmol) was added via a syringe. The reaction mixture was allowed to reflux for four hours. GC-analysis confirmed complete conversion of the starting halide and showed approximately 60% of the desired alkylmagnesium chloride. This solution was added to a solution of $PCl_3$ (1.3 mL, 15 mmol, in 20 mL ether) and the resulting mixture was stirred at room temperature for 1 hour, then stirred at reflux for 6 hours. The mixture was allowed to cool down to room temperature, filtered through cannula, and the mother liquor was concentrated in vacuo giving dichloro-triethylmethylphosphine as a yellow oil which was used without further purification.

An oven-dried Schlenk tube was charged with magnesium turnings (170 mg, 7.0. mmol) and 2-bromobiphenyl (1.42 mL, 6.9 mmol). The tube was purged with argon, then THF (12 mL) was added through a rubber septum and the reaction mixture was heated to a mild reflux for 3 h. The reaction mixture was then cooled to room temperature, the septum was removed and copper (I) chloride (855 mg, 8.45 mmol) was added. The tube was capped with a septum and purged with argon, then a solution of the dichloro-triethylmethylphosphine (prepared above) in THF (5 mL) was added. The reaction mixture was heated to reflux for 3 h then was allowed to cool to room temperature and ether (50 mL) and pentane (50 mL) were added. The resulting suspension was stirred for 10 min, during which time a heavy dark-brown precipitate formed. The suspension was filtered , the mother liquor was concentrated and column chromatography of the crude material gave 2g of yellow oil which was identified as 2-biphenyl triethylmethyl chlorophosphine (50% pure).

2-Biphenyl triethylmethyl chlorophosphine (prepared above approx: 3.14 mmol) was dissolved in 5 mL THF under argon atmosphere. To this solution cyclohexylmagnesium chloride (2M, 3.14 mL, 6.3 mmol) was added and the resulting mixture was stirred for three hours at room temperature, then copper(I) chloride (0.6 g, 6 mmol) was added and resulting suspension was stirred for 14 hours at 36 ˚C. The reaction mixture was allowed to cool to room temperature and ether (50 mL) and pentane (50 mL) were added. The resulting suspension was stirred for 10 min, during which time a heavy dark-brown precipitate formed. The suspension was filtered and the solid was collected on a fritted funnel. The solid was partitioned between ethyl acetate/ether (100 mL, 1/1 v/v) and 38% aqueous ammonium hydroxide (50 mL) and water (50 mL). The mixture was vigorously shaken several times over 30 min and the layers were separated. The aqueous layer was washed with ether/ethyl acetate (2x100 mL, 1/1 v/v), and the combined organic layers were washed with brine (2x50 mL), dried over anhydrous magnesium sulfate, decanted, and concentrated in vacuo. The product was crystallized from toluene/methanol to afford 300 mg of the title compound as a white solid.

***Example 108***

Synthesis of 2-(5-*m*-xvlyl)-1.3-cyclohexanedione

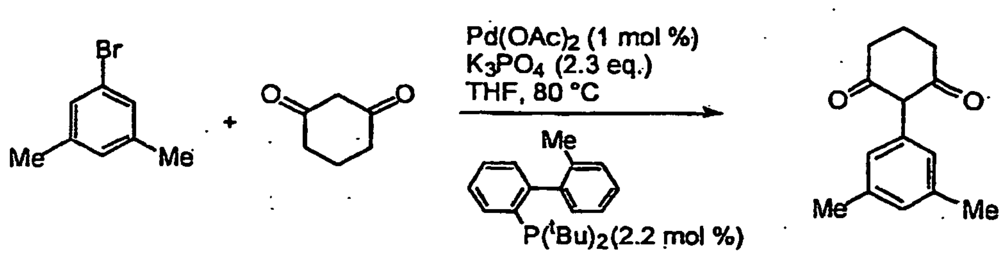


A dry Schlenk tube containing a stirbar was charged with palladium acetate (2.3 mg, 0.01 mmol), 2-di(*t*-butyl) phosphino-2'-methylbiphenyl (not according to the invention; 6.9 mg, 0.022 mmol), 1,3-cyclohexanedione (135 mg, 1.2 mmol), and potassium phosphate (490 mg, 2.3 mmol). After a septum was placed on top of the tube, it was evacuated and refilled with argon three times. THF (3 mL) and 5-bromo-*m*-xylene (186 mg, 0.136 mL, 1.0 mmol) were sequentially

injected. Under a flow of argon, the septum was replaced with a teflon screwcap, and the tube sealed and heated with stirring in an oil bath at 80 °C. After 19 h, the mixture was cooled to rt, filtered, and the solvents were stripped. Chromatography, eluting with 1:1 hexane:ethyl acetate, gave 220 mg (82%) of 2-(5-*m*-xylyl)-1,3-cyclohexanedione, a white solid.

***Example 109***

Synthesis of ethyl α-(5-*m*-xylyl)-acetoacetate

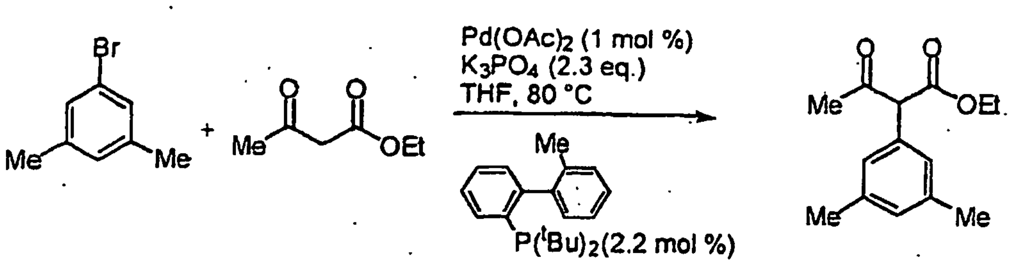


A dry Schlenk tube containing a stirbar was charged with palladium acetate (2.3 mg, 0.01 mmol), 2-di(*t*-butyl) phosphino-2'-methylbiphenyl (not according to the invention; 6.9 mg, 0.022 mmol), and potassium phosphate (490 mg, 2.3 mmol). After a septum was placed on top of the tube, it was evacuated and refilled with argon three times. THF (1 mL), ethyl acetoacetate (156 mg, 0.153 mL, 1.2 mmol) and 5-bromo-*m*-xylene (186 mg, 0.136 mL, 1.0 mmol) were sequentially injected. Under a flow of argon, the septum was replaced with a teflon screwcap, and the tube sealed and heated with stirring in an oil bath at 80 °C. After 23 h, the reaction was stopped. Analysis by GC/MS indicated that Ethyl-α-(5-*m*-xyly)-acetoacetate had been formed in approximately 30% yield.

***Example 110***

Synthesis of α-(4-methylcarboxyphenyl)propiophenone

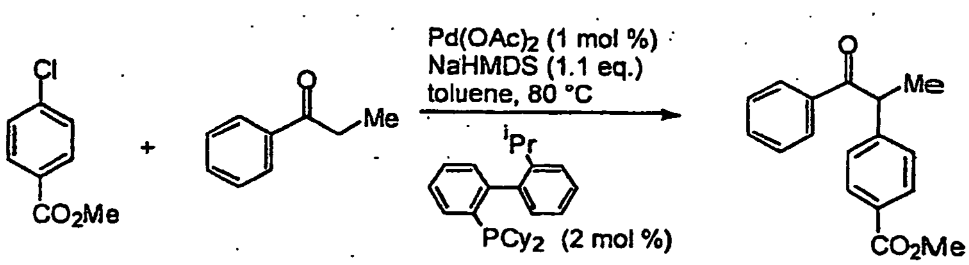


A dry Schlenk tube containing a stirbar was charged with palladium acetate (2.3 mg, 0.01 mmol), 2-dicyclohexylphosphino-2'-isopropylbiphenyl (not according to the invention; 7.9 mg, 0.02 mmol), methyl-4-chlorobenzoate (171 mg, 1.0 .mmol). The tube was then evacuated, filled with argon, sealed with a teflon screwcap, and taken into a glove box, where it was charged with sodium hexamethyldisilazane (213 mg, 1.1 mmol). The tube was again sealed and taken out of the glove box, and under a flow of argon, the screwcap was replaced with a septum. Toluene (1 mL) and propiophenone (161 mg, 0.16 mL, 1.2 mmol) were sequentially injected. Under a flow of argon, the septum was replaced with the screwcap, and the tube sealed and heated with stirring in an oil bath at 80 °C. After 20 h, the mixture was cooled to rt and partitioned between ether and water. The aqueous layer was extracted three times with additional ether, and the combined organics were dried over $Na_2SO_4$, filtered, and the solvents removed. Chromatography, eluting with 20:1 hexane:ethyl acetate, gave 220 mg (82%) of α-(4-methylcarboxyphenyl)propiophenone, a white solid.

***Example 111***

Synthesis of α-(4-ethylcarboxyphenyl)propiophenone

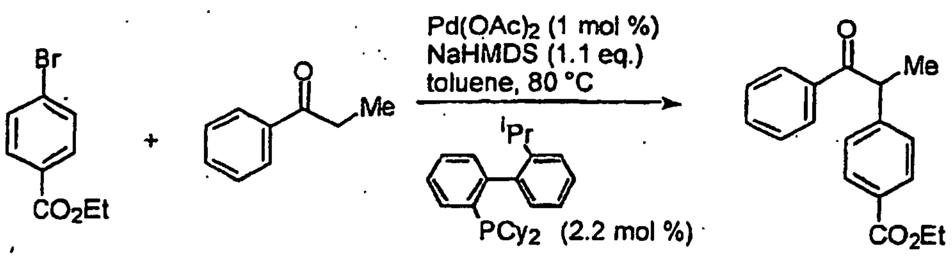


A dry Schlenk tube containing a stirbar was charged with palladium acetate (2.3 mg, 0.01 mmol) and 2-dicyclohexylphosphino-2'-isopropylbiphenyl (not according to the invention; 8.6 mg, 0.022 mmol). A rubber septum was then placed on top of the tube, which was then evacuated and filled with argon. Propiophenone (161 mg, 0.16 mL, 1.2 mmol), sodium hexamethyldisilazane (2.2 mL of a 0.5 M solution in toluene), and ethyl-4-bromobenzoate (230 mg, 163 μL, 1.0 mmol) were sequentially injected. Under a flow of argon, the septum was replaced with a teflon screwcap, and the tube sealed and heated with stirring for 12 h at 80°C. The mixture was then cooled to rt and partitioned between ether and water. The aqueous layer was extracted three times with additional ether, and the combined organics were dried over $Na_2SO_4$, filtered, and the solvents removed. Chromatography, eluting with 10:1 hexane:ethyl acetate, gave 248 mg (88%) of α-(4-ethylcarboxyphenyl)propiophenone, a white solid.

***Example 112***

Synthesis of α-(4-cyanophenyl)propiophenone

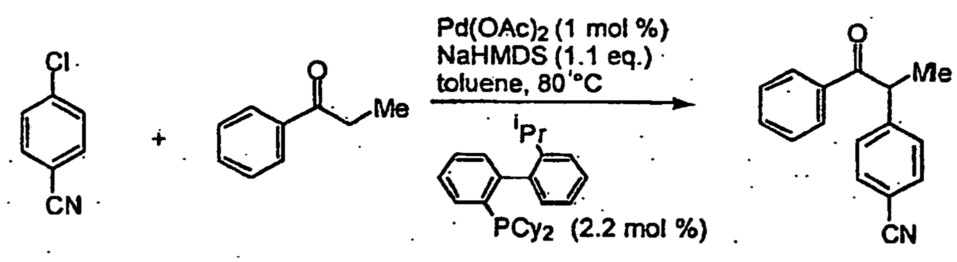


A dry Schlenk tube containing a stirbar was charged with palladium acetate (2.3 mg, 0.01 mmol) and 2-dicyclohexylphosphino-2'-isopropylbiphenyl (not according to the invention; 8.6 mg, 0.022 mmol). A septum was placed on top of the tube, which was then evacuated and filled with argon. Propiophenone (161 mg, 0.16 mL, 12 mmol) and sodium hexamethyldisilazane (2.2 mL of a 0.5 M solution in toluene) were sequentially injected. After this had stirred for 20 min at rt, a solution of 4-chlorobenzonitrile (138 mg, 1.0 mmol) in toluene (1 mL) was injected. Under a flow of argon, the septum was replaced with a teflon screwcap, and the tube sealed and heated with stirring for 18 h at 80 °C. The mixture was then cooled to rt and partitioned between ether and water. The aqueous layer was extracted three times with additional ether, and the combined organics were dried over $Na_2SO_4$, filtered, and the solvents removed. Chromatography, eluting with 10:1 hexane:ethyl acetate, gave 192 mg (82%) of α-(4-cyanophenyl)propiophenone, a clear oil.

***Example 113***

Synthesis of α-(4-cyanophenyl)propiophenone

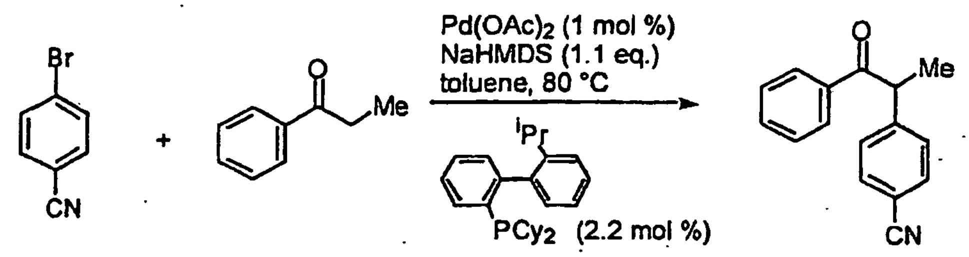


A dry Schlenk tube containing a stirbar was charged with palladium acetate (2.3 mg, 0.01 mmol) and 2-dicyclohexylphosphino-2'-isopropylbiphenyl (not according to the invention; 8.6 mg, 0.022 mmol). A septum was placed on top of the tube, which was then evacuated and filled with argon. Sodium hexamethyldisilazane (2.2 mL of a 0.5 M solution in toluene) and propiophenone (161 mg, 0.16 mL, 1-2 mmol) were sequentially injected. After this had stirred for 20 min at rt, a solution of 4-bromobenzonitrile (182 mg, 1.0 mmol) in toluene (1 mL) was injected. Under a flow of argon, the septum was-replaced with a teflon screwcap, and the tube sealed and heated with stirring for 18 h at 80 ˚C. The mixture was then cooled to rt and partitioned between ether and water. The aqueous layer was extracted three times with additional ether, and the combined organics were dried over $Na_2SO_4$, filtered, and the solvents removed. Chromatography, eluting with 10:1 hexane:ethyl acetate, gave 202 mg (86%) of α-(4-cyanophenyl)propiophenone, a clear oil.

***Example 114***

Synthesis of Diethyl 1-(*t*-butylphenyl)malonate

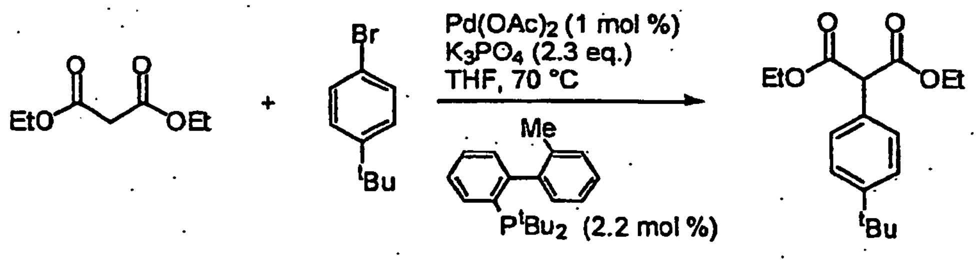


A dry Schlenk tube containing a stirbar was charged with $Pd(OAc)_2$ (2.3 mg, 0.01 mmol), 2-di-*t*-butylphosphino-2'-methylbiphenyl (not according to the invention; 6.9 mg, 0.022 mmol), and potassium phosphate (490 mg, 2.3 mmol). A septum was placed on top of the tube, which was then evacuated and filled with argon. THF (2 mL), diethylmalonate (192 mg, 183 µL, 1.2 mmol), and 4-*t*-butyl-bromobenzene (213 mg, 175µL, 1.0 mmol) were injected sequentially, and, under a flow of argon, the septum was replaced with a teflon screwcap. The tube was then sealed and heated with stirring for 23 h at 70 ˚C. The mixture was partitioned between ether and water, and the aqueous layer extracted three times with additional ether. The combined organics were dried ($Na_2SO_4$), filtered and concentrated. Chromatography of the residue, eluting with 15:1 hexane: ethyl acetate, gave 254 mg (87%) of diethyl 1-(*t*-butylphenyl)malonate, a clear oil.

***Example 115***

2-(3-(1,3-dioxalano)phenyl)cycloheptanone

A Schlenk tube containing a stirbar and capped by a rubber septum was evacuated, flame dried under vacuum, filled with argon, and cooled to rt. The tube was then charged with NaO*t*Bu (211 mg, 2.2 mmol), and the septum was replaced. The tube was again evacuated, filled with argon, and 1 mL of a toluene solution, which was 0.001 M in Pd $(OAc)_2$ and 0.002 M in 2-methyl-2'-(dicyclohexylphosphino)biphenyl (not according to the invention), was injected. 2-(3-bromophenyl)-1,3-dioxalane (229 mg, 151 µL, 1.0 mmol) and cycloheptanone (224 mg, 236 µL, 2.0 mmol) were then injected sequentially, and under a flow of argon, the septum was replaced by a teflon screw cap, and the tube was sealed and heated in an oil bath at 45 ˚C for 20 h. The mixture was then cooled and partitioned between ether and water. After the aqueous layer was extracted three times with ether, the combined organics were dried ($Na_2SO_4$), filtered,

and the solvents removed under reduced pressure. The residue was chromatographed (eluting with 25:75 ethyl acetate: hexane) to give 200 mg (77%) of 2-(3-(1,3-dioxalano)phenyl)cycloheptanone, a clear oil.

A similar reaction, that instead used 2-(dicyclohexylphosphino)biphenyl as the ligand, 60 ˚C as the temperature, and 18.5 h as the reaction time, gave 178 mg (68%) of 2-(3-(1,3-dioxalano)phenyl)cycloheptanone.

### Example 116

2-Methyl-4-(4-*n*-butylphenyl)-3-pentanone

A Schlenk tube containing a stirbar and capped by a rubber septum was evacuated, flame dried under vacuum, filled with argon, and cooled to rt. The tube was then charged with NaO$^t$Bu (125 mg, 1.3 mmol), and the septum was replaced. The tube was again evacuated, filled with argon, and 1 mL of a toluene solution, which was 0.001 M in Pd $(OAc)_2$ and 0.002 M in 2-methyl-2'-(dicyclohexylphosphino)biphenyl (not according to the invention), was injected. 4-*n*-butylchlorobenzene (169 mg, 170 μL, 1.0 mmol) and 2-methyl-3-pentanone (120 mg, 148 μL, 1.3 mmol) were then injected sequentially, and under a flow of argon, the septum was replaced by a teflon screw cap; and the tube was sealed and heated in an oil bath at 80˚C for 16 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 5:95 ethyl acetate: hexane) to give 181 mg (81%) of a clear oil that was a 20:1 mixture of 2-methyl-4-(4-*n*-butylphenyl)-3-pentanone: 2-methyl-2-(4-*n*-butylphenyl)-3-pentanone.

### Example 117

2-(2-*p*-Xylyl)-1-tetralone

A Schlenk tube containing a stirbar and capped by a rubber septum was evacuated, flame dried under vacuum, filled with argon, and cooled to rt. The tube was then charged with NaO$^t$Bu (125 mg, (not according to the invention) 1.3 mmol), and the septum was replaced. The tube was again evacuated, filled with argon, and 1 mL of a toluene solution; which was 0.001 M in $Pd(OAc)_2$ and 0.002 M in 2-methyl-2'-(dicyclohexylphosphino)biphenyl (not according to the invention), was injected. 2-chloro-*p*-xylene (140 mg, 134 μL, 1.0 mmol) and α-tetralone (175 mg, 159 μL, 1.2 mmol) were then injected sequentially, and under a flow of argon, the septum was replaced by a teflon screw cap, and the tube was sealed and heated in an oil bath at 80 ˚C for 5 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 4:1 toluene: hexane) to give 222 mg (89%) of 2-(2-*p*-xylyl)-1-tetralone, a pale yellow oil.

### Example 118

1-(5-m-Xylyl)acetophenone

A Schlenk tube containing a stirbar and capped by a rubber septum was evacuated, flame dried under vacuum, filled with argon, and cooled to rt. The tube was then charged with NaO$^t$Bu (240 mg, 2.5 mmol), and the septum was replaced. The tube was again evacuated, filled with argon, and 1 mL of a toluene solution, which was 0.001 M in Pd $(OAc)_2$ and 0.002 M in 2-isopropyl-2'-(dicyclohexylphosphino)biphenyl (not according to the invention), was injected. Additional toluene (2 mL), 5-bromo-*m*-xylene (185 mg, 136 μL, 1.0 mmol) and acetophenone (144 mg, 140 μL, 1.2 mmol) were then injected sequentially, and under a flow of argon, the septum was replaced by a teflon screw cap, and the tube was sealed and heated in an oil bath at 50 ˚C for 24 h. The mixture was then cooled and partitioned between ether and water. After the aqueous layer was extracted three times with ether, the combined organics were dried ($Na_2SO_4$), filtered, and the solvents removed under reduced pressure: The residue was chromatographed (eluting with 4:1 toluene: hexane) to give 170 mg (76%) of 1-(5-*m*-xylyl)acetophenone, a clear oil.

### Example 119

1-(5-m-Xylyl)propiophenone

A Schlenk tube containing a stirbar and capped by a rubber septum was evacuated, flame dried under vacuum, filled with argon, and cooled to rt. The tube was then charged with NaO$^t$Bu (125 mg, 1.3 mmol), and the septum was replaced. The tube was again evacuated, filled with argon, and 1 mL of a toluene solution, which was 0.001 M in Pd

$(OAc)_2$ and 0.002 M in 2-(di-*t*-butylphosphino)biphenyl (not according to the invention), was injected: 5-bromo-*m*-xylene (185 mg, 136 μL, 1.0 mmol) and propiophenone (161 mg, 160 μL, 1.2 mmol) were then injected sequentially, and under a flow of argon, the septum was replaced by a teflon screw cap, and the tube was sealed and heated in an oil bath at 80 °C for 3 h. The mixture was then cooled and partitioned between ether and water. After the aqueous layer was extracted three times with ether, the combined organics were dried ($Na_2SO_4$) filtered, and the solvents removed under reduced pressure. The residue was chromatographed (eluting with 4:1 toluene: hexane) to give 212 mg (89%) of 1-(5-m-xylyl)propiophenone, a clear oil.

***Example 120***

2-Methyl-4-(4-(*N,N*-dimethylamino)phenyl)-3-pentanone

A Schlenk tube containing a stirbar and capped by a rubber septum was evacuated, flame dried under vacuum, filled with argon, and cooled to rt. The tube was then charged with NaO$^t$Bu (125 mg, (not according to the invention) 1.3 mmol) and 4-bromo-*N,N*-dimethylaminobenzene (200 mg, 1.0 mmol), and the septum was replaced. The tube was again evacuated, filled with argon, and 1 mL of a THF solution, which was 0.001 M in $Pd(OAc)_2$ and 0.002 M in 2-methyl-2'-(dicyclohexylphosphino)biphenyl (not according to the invention), was injected. 2-Methyl-3-pentanone (120 mg, 148 μL, 1.3 mmol) was then injected, and under a flow of argon, the septum was replaced by a teflon screw cap, and the tube was sealed and heated in an oil bath at 83 °C for 22 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 5:95 ethyl acetate: hexane) to give 151 mg (69%) of a pale brown oil that was a 20:1 mixture of 2-methyl-4-(4-(*N,N*-dimethylamino) phenyl)-3-pentanone: 2-methyl-2-(4-(*N,N*-dimethylamino)phenyl)-3-pentanone.

***Example 121***

2-(3-Hydroxyphenyl)-3-pentanone

A Schlenk tube containing stirbar and capped by a rubber septum was evacuated, flame dried under vacuum, filled with argon, and cooled to rt. The tube was then charged with NaO$^t$Bu (211 mg, 2.2 mmol), and the septum was replaced. The tube was again evacuated, filled with argon, and 0.5 mL of a THF solution, which was 0.002 M in Pd $(OAc)_2$ and 0.004 M in 2-methyl-2'-(dicyclohexylphosphino)biphenyl (not according to the invention), was injected. Subsequently injected were 0.5 mL of a 2.0 M solution of 3-bromophenol in THF, followed by 3-pentanone (172 mg, 211 μL, 2.0 mol), and under a flow of argon, the septum was replaced by a teflon screw cap, and the tube was sealed and heated in an oil bath at 70°C for 24 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 1:1 diethyl ether: pentane) to give 157 mg (88%) of 2-(3-hydroxyphenyl)-3-pentanone, a clear oil.

***Example 122***

2,4-Dimethyl-2-(4-*t*-butylphenyl)-3-pentanone

A Schlenk tube containing a stirbar and capped by a rubber septum was evacuated, flame dried under vacuum, filled with argon, and cooled to rt. The tube was then charged with NaO$^t$Bu (125 mg" 1.3 mmol) $Pd(OAc)_2$ (1.1 mg, 0.005 mmol), and 2-methyl-2'-(dicyclohexylphosphino)biphenyl (not according to the invention; 3.6 mg, 0.01 mmol) and the septum was replaced. Toluene (1 mL), 4-bromo-*t*-butylbenzene (213 mg, 173 μL, 1.0 mmol) and 2,4-dimethyl-3-pentanone (137 mg, 170 μL, 12 mmol) were sequentially injected, and under a flow of argon, the septum was replaced by a teflon screw cap, and the tube was sealed and heated in an oil bath at 85 °C for 24 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 3:97 diethyl ether: hexane) to give 150 mg (61%) of 2,4-dimethyl-2-(4-t-butylphenyl)-3-pentanone; a white solid.

***Example 123***

2-(4-Methoxyphenyl)-3-pentanone

A Schlenk tube containing a stirbar and capped by a rubber septum was evacuated, flame dried under vacuum, filled with argon, and cooled to rt. The tube was then charged with NaO$^t$Bu (125 mg, 1.3 mmol); and the septum was replaced. The tube was again evacuated, filled with argon, and 1 mL of a toluene solution, which was 0.001 M in Pd $(OAc)_2$ and 0.002 M in 2-methyl-2'-(dicyclohexylphosphino)biphenyl (not according to the invention), was injected. 4-chloroanisole (143 mg, 123 μL, 1.0 mmol) and 3-pentanone (172 mg, 211 μL, 2.0 mmol) were then injected sequentially, and under a flow of argon, the septum was replaced by a teflon screw cap, and the tube was sealed and heated in an oil bath at 70˚C for 24 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting first with 1:50 ethyl acetate: hexane, then 1:12 ethyl acetate: hexane) to give 144 mg (75%) of 2-(4-methoxyphenyl)-3-pentanone, a clear oil.

***Example 124***

1-(*t*-Butylphenyl)-1-methoxyacetophenone

A Schlenk tube containing a stirbar and capped by a rubber septum was evacuated, flame dried under vacuum, filled with argon, and cooled to rt. The tube was then charged with NaO$^t$Bu (0.125. g, 1.3 mmol), $Pd(OAc)_2$ (1.1 mg, 0.005 mmol), and 2-methyl-2'-(dicyclohexylphosphino)biphenyl (not according to the invention; 3.6 mg, 0.01 mmol) and the septum was replaced. THF (1 mL), 4-bromo-*t*-butylbenzene (213 mg, 173 μL, 1.0 mmol) and α-methoxyacetophenone (180 mg, 165 μL, 1.2 mmol) were sequentially injected, and under a flow of argon, the septum was replaced by a teflon screw cap, and the tube was sealed and heated in an oil bath at 70 ˚C for 17 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 5:95 ethyl acetate: hexane) to give 239 mg (85%) of 1-(*t*-butylphenyl)-1-methoxyacetophenone, a clear oil.

***Example 125***

2,2-Dimethyl-5-(2-*m*-xylyl)cyclopentanone

A Schlenk tube containing a stirbar and capped by a rubber septum was evacuated, flame dried under vacuum, filled with argon, and cooled to rt. The tube was then charged with NaO$^t$Bu (125 mg, 1.3 mmol), $Pd(OAc)_2$ (1.1 mg, 0.005 mmol), and 2-methyl-2'-(dicyclohexylphosphino)biphenyl (not according to the invention; 3.6mg, 0.01 mmol) and the septum was replaced. The tube was again evacuated and filled with argon. Toluene (1 mL), 2-bromo-*m*-xylene (185 mg, 133 μL**,** 1.0 mmol) and 2,2-dimethylcyclopentanone (134 mg, 150 μL, 1.2 mmol) were sequentially injected, and under a flow of argon, the septum was replaced by a teflon screw cap, and the tube was sealed and heated in an oil bath at 70 ˚C for 22.5 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 5:95 ethyl acetate: hexane) to give 145 mg (67%) of 2,2-dimethyl-5-(2-*m*-xylyl)cyclopentanone, a clear oil.

***Example 126***

1-(*t*-Butylphenyl)propiophenone

A Schlenk tube containing a stirbar and capped by a rubber septum was evacuated, flame dried under vacuum, filled with argon, and cooled to rt. The tube was then charged with $Pd(OAc)_2$ (4.8 mg, 0.008 mmol), racemic 2,2'-bis (dicyclohexylphosphino)binaphthyl (not according to the invention; 5.2 mg, 0.008 mmol), NaO$^t$Bu (21 mg, 0.21 mmol), and 4-*t*-butylphenyl-*p*-toluene sulfonate (50 mg, 0.16 mmol), and the septum replaced. The tube was again evacuated and filled with argon. Toluene (1 mL) and propiophenone (26 mg, 26 μL, 0.20 mmol) were sequentially injected, and under a flow of argon, the septum was replaced by a teflon screw cap, and the tube was sealed and heated in an oil bath at 80 ˚C for 40 h. The mixture was then cooled and partitioned between ether and water. After the aqueous layer was extracted three times with ether, the combined organics were dried ($Na_2SO_4$), filtered, and the solvents removed under reduced pressure. The residue was chromatographed (eluting with 5:95 ethyl acetate: hexane) to give 20 mg (45%) of 1-(*t*-butylphenyl)propiophenone, a white solid.

***Example 127***

α-(4-*t*-Butylphenyl)-α-methyl-butyrolactone

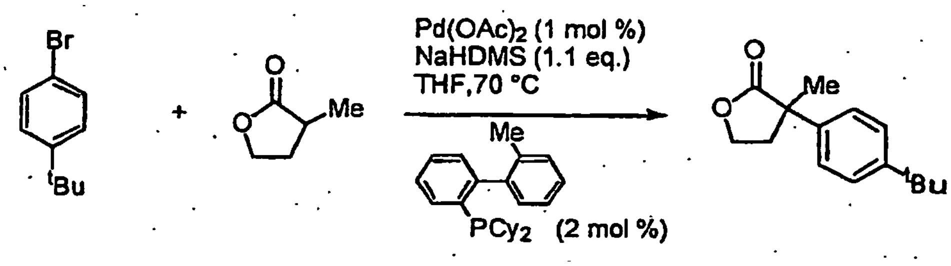


A dry Schlenk tube containing a stirbar was charged with palladium acetate (2.3 mg, 0.01 mmol) and 2-dicyclohexylphosphino-2'-methylbiphenyl (not according to the invention; 7.3 mg, 0.02 mmol). A teflon screwcap was placed on top of the tube, which was then evacuated and filled with argon. The tube was then taken into a glove box, where it was charged with sodium hexamethyldisilizane (201 mg, 1.1 mmol). The tube was sealed and taken out of the glove box. Under a flow of argon, the screwcap was replaced by a septum, and THF (1 mL), 4-bromo-*t*-butylbenzene (213 mg, 0.173 mL, 1.0 mmol), and α-methyl-γ-butyrolactone (120 mg, 0.113 mL, 1.2 mmol) were sequentially injected. The septum was, under a flow of argon, replaced by the screwcap, and the tube was heated, with stirring, for 30 min. Analysis by GC/MS indicated that α-(4-*t*-butylphenyl)-α-methyl-γ-butyrolactone was formed in approximately 20 % yield.

***Example 128***

2-(Dicyclohexylphosphino)-1.1'-binaphthyl

An oven-dried Schlenk tube was cooled under argon then was charged with 2-bromo-1,1'-binaphthyl* (700 mg, 210 mmol) and THF (21 mL). The solution was cooled to -78 ˚C under argon, then *n*-BuLi was added dropwise. After 1 h at -78 ˚C, chlorodicyclohexylphosphine (635 mg, 2.70 mmol) was added as a solution in THF (3 mL). The cooling bath was then removed and the reaction mixture was allowed to warm to room temperature overnight. The reaction was quenched with saturated aqueous $NaHCO_3$ then concentrated to remove THF. The resulting aqueous residue was extracted with $Et_2O$ (2X100 mL). The extracts were dried over $Na_2SO_4$, filtered and concentrated. The resulting crude solid was recrystallized from hot ethanol was to afford 512 mg (54%) of the title compound as a white solid.

***Example 129***

2-(Di-*t*-butylphosphino)-1,1'-binaphthy

An oven-dried Schlenk tube is cooled under argon, then charged with magnesium (56 mg, 2.3 mmol) and a very small crystal of iodine. A solution of 2-bromo-1,1'-binaphthyl* (700 mg, 2.10 mmol) in THF (2.5 mL +1.5 mL) was added via cannula, then the mixture was gently refluxed overnight. The Grignard solution was cooled to room temperature then copper (I) chloride (220 mg, 2.20 mmol) was added. After purging the reaction vessel with argon, dit-butylchlorophosphine (500 μL, 2.60 mmol) was added and the reaction mixture was heated at reflux. After 12 h, the reaction was cooled and diluted with hexanes/$Et_2O$ (1:1 v/v, 30 mL) and stirred vigorously for 15 min. The solid was filtered, then suspended in hexanes / ethyl acetate (1:1 v/v, 30 mL) and water (10 mL), and then 30% aqueous $NH_4OH$ (10 mL) was added. The resulting mixture was stirred for 15 minutes then poured into a separatory funnel and then layers were separated. The organic phase was washed with water and brine, then dried over $Na_2SO_4$, filtered and concentrated. The resulting crude solid was recrystallized from ethanol to afford 280 mg (33%) of the title product as a white solid.

***Example 130***

1-(2-Bromophenyl)naphthalene

Tetrakis(triphenylphosphine)palladium (0) (340 mg, 0.29 mmol, 5 mol% Pd) was suspended in DME (60 mL) in a 250 mL round-bottomed flask, then 2-bromoiodobenzene (750 μL, 5.84 mmol) was added and the mixture was

stirred at room temperature for 15 min. 1-Naphthylboronic acid (1.0 g, 5.8 mmol) was added in a minimum volume of ethanol (ca. 2 mL), followed by aqueous $Na_2CO_3$ (6 mL, 2 M, 12 mmol). The flask was fitted with a reflux condenser and the reaction mixture was refluxed overnight. The reaction was then cooled and concentrated to remove DME. The resulting residue was diluted with water (50 mL) and extracted with $Et_2O$ (2x50 mL). The ethereal extracts were dried over $Na_2SO_4$, filtered and concentrated. Triphenylphosphine was removed by crystallization from ethyl acetate / hexanes, then the resulting crude solid was purified by chromotography on silica gel (1% ethyl acetate-hexanes) to afford 1.35 g (82%) of the title compound as a white solid.

***Example 131***

1-[2-(Dicyclohexylphosphino)phenyl]naphthalene

An oven-dried Schlenk tube was cooled under argon then was charged with 1-(2-bromophenyl)naphthalene (600 mg, 2.12 mmol) and THF (21 mL). The solution was cooled to -78 ˚C under argon, then *n*-BuLi was added dropwise. After 1 h at -78 ˚C, chlorodicyclohexylphosphine (620 mg, 2.66 mmol) was added as a solution in THF (3 mL). The cooling bath was then removed and the reaction mixture was allowed to warm to room temperature overnight. The reaction was quenched with saturated aqueous $NaHCO_3$ then concentrated to remove THF. The resulting aqueous residue was extracted with $Et_2O$ (2X100 mL). The extracts were dried over $Na_2SO_4$, filtered and concentrated. The resulting crude solid was recrystallized from hot ethanol was to afford 493 mg (58%) of the title compound as a white solid.

***Example 132***

1-[2-(Di-*t*-butylphosphino)phenyl]naphthalene

An oven-dried Schlenk tube is cooled under argon, then charged with magnesium (56 mg, 2.30 mmol) and a very small crystal of iodine. A solution of 1-(2-bromophenyl)naphthalene (600 mg, 2.12 mmol) in THF (2.5 mL + 1.5 mL) was added via cannula, then the mixture was gently refluxed for 5 h. The Grignard solution was cooled to room temperature then copper (I) chloride (220 mg, 2.20 mmol) was added. After purging the reaction vessel with argon, di-*t*-butylchlorophosphine (500 μL, 2.60 mmol) was added and the reaction mixture was heated at reflux overnight. The reaction was then cooled and diluted with hexanes/$Et_2O$ (1:1 v/v, 30 mL) and stirred vigorously for 30 min. The solid was filtered, then suspended in hexanes / $Et_2O$ (1:1 v/v, 30 mL) and water (10 mL), and then 30% aqueous $NH_4OH$ (10 mL) was added. The resulting mixture was stirred for 15 minutes then poured into a separatory funnel and then layers were separated. The organic phase was washed with water and brine, then dried over $Na_2SO_4$, filtered and concentrated. The resulting crude solid was recrystallized from ethanol to afford 381 mg (52%) of the title product as a white solid. (2 crops were collected).

***Example 133***

*N*-(4-*t*-Butylphenyl)-2-phenylindole

An oven-dried test tube was cooled under argon then charged with $Pd_2(dba)_3$ (4.5 mg, 0.0049 mmol, 1 mol% Pd), 1-[2-(di-*t*-butylphosphino)phenyl]naphthalene (not according to the invention; 5.1 mg, 0.015 mmol, 1.5 mol%), 2-phenylindole (95%, 205 mg, 1.06 mmol) and NaO*t*-Bu (132 mg, 1.37 mmol). The tube was purged with argon, then toluene (2.0 mL) and 1-bromo-4-*t*-butylbenzene (170 μL, 0.98 mmol) were added through a septum. The reaction mixture was heated at 100 ˚C for 18 h, then allowed to cool to room temperature. The reaction was diluted with ethyl ether, then filtered through a pad of celite and concentrated *in vacuo.* The crude residue was purified by flash chromatography on silica gel (1% ethyl acetate/hexanes) to afford 302 mg (95%) of the title compound as a white solid.

***Example 134***

*N*-(4-Methoxyhenyl)-2-phenylindole

An oven-dried 16x100 mm test tube was cooled to room temperature under argon, then was charged with $Pd_2(dba)_3$ (2.2 mg, 0.0024 mmol, 1 mol% Pd), 2-(di-*t*-butylphosphino)-2'-isopropylbiphenyl (not according to the invention; 2.5 mg, 0.0072 mmol, 1.5 mol%), 2-phenylindole (95%, 105 mg, 0.52 mmol) and NaO*t*-Bu (65 mg, 0.68 mmol). The tube was fitted with a septum and purged with argon, then toluene (1.0 mL) and 4-bromoanisole (60 μL, 0.48 mmol) were added. The reaction was heated at 100 ˚C for 16 h then allowed to cool to room temperature. The reaction was diluted with ethyl ether, then filtered through a pad of celite and concentrated *in vacuo.* The crude residue was purified

by flash chromatography on silica gel (5% ethyl acetate/hexanes) to afford 96 mg (67%) of the title compound as a white solid.

***Example 135***

*N*-(3,5-Dimethylphenyl)-7-ethylindole

An oven-dried test tube was cooled under argon then charged with $Pd_2(dba)_3$ (4.5 mg, 0.0049 mmol, 1 mol% Pd), 1-[2-(di-*t*-butylphosphino)phenyl]naphthalene (not according to the invention; 5.1 mg, 0.015 mmol, 1.5 mol%) and NaO*t*-Bu (134 mg, 1.39 mmol). The tube was purged with argon, then toluene (2.0 mL), 7-ethylindole (150 μL, 1.08 mmol), and 5-bromo-*m*-xylene (135 μL, 0.99 mmol) were added through a septum. The reaction mixture was heated at 100 ˚C for 18 h, then allowed to cool to room temperature. The reaction was diluted with ethyl ether, then filtered through a pad of celite and concentrated *in vacuo.* The crude residue was purified by flash chromatography on silica gel (1% ethyl acetate/hexanes) to afford 132 mg (53%) of the title compound as a pale yellow oil.

***Example 136***

*N*-(2-Pyridyl)-7-ethylindole

An oven-dried test tube was cooled under argon then charged with $Pd_2(dba)_3$ (4.6 mg, 0.005 mmol,1, mol% Pd), 1-[2-(di-*t*-butylphosphino)phenyl]naphthalene (not according to the invention; 5.3 mg, 0.015 mmol, 1.5 mol%) and NaO*t*-Bu (134 mg, 1.39 mmol). The tube was purged with argon, then toluene (2.0 mL), 7-ethylindole (150 μL, 1.08 mmol), and 2-bromopyridine (95 μL, 1.0 mmol) were added through a septum. The reaction mixture was heated at 80 ˚C for 15 h, then allowed to cool to room temperature. The reaction was diluted with ethyl ether, then filtered through a pad of celite and concentrated *in vacuo.* The crude residue was purified by flash chromatography on silica gel (15% ethyl acetate/hexanes) to afford 209 mg (94%) of the title-compound as a white solid.

***Example 137***

*N*-(3,5-Dimethylphenyl)-2,3-dimethylindole

An oven-dried test tube was cooled under argon then charged with $Pd_2(dba)_3$ (2.4 mg, 0.0026 mmol, 1 mol% Pd), 1-[2-(di-*t*-butylphosphino)phenyl]naphthalene (not according to the invention; 2.7 mg, 0.0079 mmol, 1.5 mol%), 2,3-dimethylindole (82 mg, 0.56 mmol) and NaO*t*-Bu (69 mg, 0.72 mmol). The tube was purged with argon, then toluene (1.0 mL) and 5-bromo-*m*-xylene (70 μL, 0.52 mmol) were added through a septum. The reaction mixture was heated at 100˚C for 21.5 h, then allowed to cool to room temperature. The reaction was diluted with ethyl ether, then filtered through a pad of celite and concentrated *in vacuo.* The crude residue was purified by flash chromatography on silica gel (1% ethyl acetate/hexanes) to afford 125 mg (97%) of the title compound as a colorless oil.

***Example 138***

*N*-(3,5-Dimethylphenyl)-2,3-dimethylindole

An oven-dried test tube was cooled under argon then charged with $Pd_2(dba)_3$ (4.7 mg, 0.005 mmol, 2 mol% Pd), 2-(di-*t*-butylphosphino)biphenyl (not according to the invention; 4.6 mg, 0.015 mmol, 3 mol%), 2,3-dimethylindole (82 mg, 0.56 mmol) and NaO*t*-Bu (69 mg, 0.72 mmol). The tube was purged with argon, then toluene (1.0 mL) and 5-bromo-*m*-xylene (70 μL, 0.52 mmol) were added through a septum. The reaction mixture was heated at 100 ˚C for 18 h, then allowed to cool to room temperature. The reaction was diluted with ethyl ether, then filtered through a pad of celite and concentrated *in vacuo.* The crude residue was purified by flash chromatography on silica gel (1% ethyl acetate/hexanes) to afford 116 mg (90%) of the title compound as a colorless oil.

***Example 139***

Ethyl 3-[*N*-(4-methoxyphenyl)indole]acetate

An oven-dried 16x100 mm test tube was cooled to room temperature under argon, then was charged with $Pd_2(dba)_3$ (4.6 mg, 0.005 mmol, 1 mol% Pd), 2-(di-*t*-butylphosphino)-2'-isopropylbiphenyl (not according to the invention; 5.1 mg, 0.015 mmol, 1.5 mol%), ethyl 3-indoleacetate (220 mg, 1.08 mmol) and $K_3PO_4$ (300 mg, 1.41 mmol). The tube

was, fitted with a septum and purged with argon, then toluene (2.0 mL) and 4-bromoanisole (125 μL, 1.0 mmol) were added. The reaction was heated at 100 °C for 16 h then allowed to cool to room temperature. The reaction was diluted with ethyl ether, then filtered through a pad of celite and concentrated *in vacuo.* The crude residue was purified by flash chromatography on silica gel (20% ethyl acetate/hexanes) to afford 270 mg (87%) of the title compound as a yellow oil.

***Example 140***

*N*-(4-*t*-Butylphenyl)-2,3,7-trimethylindole

An oven-dried test tube was cooled under argon then charged with $Pd_2(dba)_3$ (9.0 mg, 0.01 mmol, 4 mol% Pd), 1-[2-(di-*t*-butylphosphino)phenyl]naphthalene (not according to the invention; 10.3 mg, 0.03 mmol, 6 mol%), 2,3,7-trimethylindole (85 mg, 0.53 mmol) and-NaO*t*-Bu (66 mg, 0.69 mmol). The tube was purged with argon, then toluene (1.0 mL) and 1-bromo-4-*t*-butylbenzene (85 μL, 0.49 mmol) were added through a septum. The reaction mixture was heated at 100 °C for 24 h, then allowed to cool to room temperature. The reaction was diluted with ethyl ether, then filtered through a pad of celite and concentrated *in vacuo.* The crude residue was purified by flash chromatography on silica gel (1% ethyl acetate/hexanes) to afford 73 mg (51%) of the title compound as a pale yellow solid.

***Example 141***

*N*-(4-Methoxyphenyl)indole

An oven-dried 16x100 mm test tube was cooled to room temperature under argon, then was charged with $Pd_2(dba)_3$ (4.6 mg, 0.005 mmol, 1 mol% Pd), 2-(di-*t*-butylphosphino)-2'-isopropylbiphenyl (not according to the invention; 5.1 mg, 0.015 mmol, 1.5 mol%), indole (120 mg, 1.02 mmol) and NaO*t*-Bu (135 mg, 1.40 mmol). The tube was fitted with a septum and purged with argon, then toluene (2.0 mL) and 4-bromoanisole (125 μL, 1.0 mmol) were added. The reaction was heated at 100 °C for 7.5 h then allowed to cool to room temperature. The reaction was diluted with ethyl ether; then filtered through a pad of celite and concentrated *in vacuo.* The crude residue was purified by flash chromatography on silica gel (5% ethyl acetate/hexanes) to afford 197 mg (88%) of the title compound as a white solid.

***Example 142***

Coupling Reactions of Indole with Aryl Tosylates

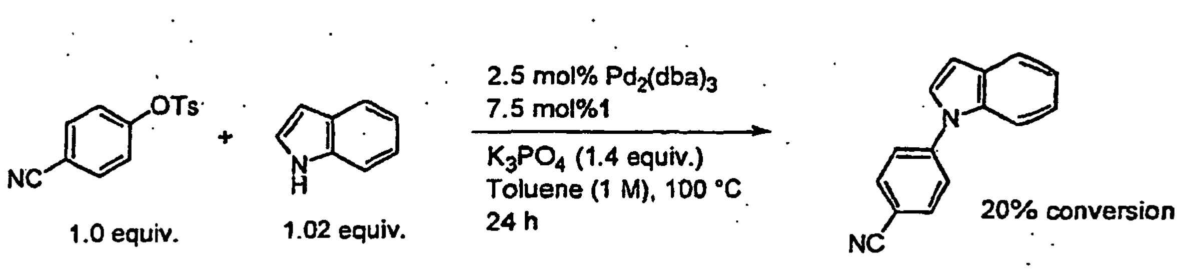


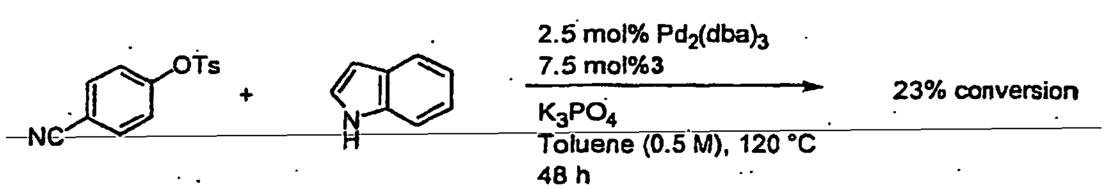

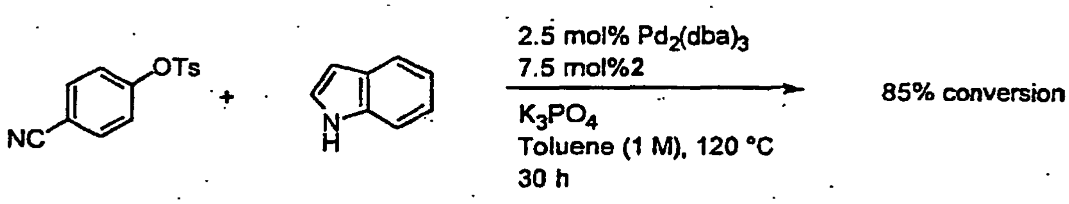


Ligands:

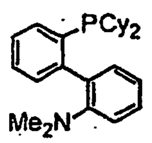
1

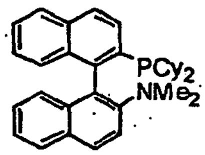
2

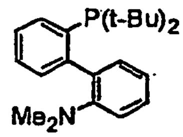
3

***N*-(4-Cyanophenyl)indole** (using ligand **1**; not according to the invention). An oven-dried Schlenk flask was evacuated and backfilled with argon and charged with $Pd_2(dba)_3$ (11.5 mg, 0.013 mmol, 5 mol % Pd), ligand **1** (14.8 mg, 0.0376 mmol, 7.5 mol %), $K_3PO_4$ (150 mg, 0.7 mmol), indole (60 mg, 0.51 mmol), and 4-cyanophenyl toslylate (137 mg, 0.5 mmol). The tube was evacuated and backfilled with argon and toluene (0.5 mL) was added through a rubber septum. The tube was sealed with a teflon screwcap and heated to 100 ˚C with stirring. GC analysis after 24h showed that the desired product had formed and the reaction had proceeded to approximately 20% conversion.

***N*-(4-Cyanophenyl)indole** (using ligand **2**; according to the invention). An oven-dried Schlenk flask was evacuated and backfilled with argon and charged with $Pd_2(dba)_3$ (22.9 mg, 0.025 mmol, 5 mol % Pd), ligand **2**. (37 mg, 0.075 mmol, 7.5 mol %), $K_3PO_4$ (300 mg, 1.4 mmol), indole (120 mg, 1.02 mmol), and 4-cyanophenyl toslylate (273 mg, 1.0 mmol). The tube was evacuated and backfilled with argon and toluene (1 mL) was added through a rubber septum. The tube was sealed with a teflon screwcap and heated to 120 ˚C with stirring. GC analysis after 30h showed that the desired product had formed and the reaction had proceeded to approximately 85% conversion.

***N*-(4-Cyanophenyl)indole** (using ligand **3**; not according to the invention). An oven-dried Schlenk flask was evacuated and backfilled with argon and charged with $Pd_2(dba)_3$ (11.5 mg, 0.013 mmol, 5 mol % Pd), ligand 3 (12.9 mg, 0.038 mmol, 7.5 mol %), $K_3PO_4$ (150 mg, 0.7 mmol), indole (60 mg, 0.51 mmol), and 4-cyanophenyl toslylate (137 mg, 0.5 mmol). The tube was evacuated and backfilled with argon and toluene (1 mL) was added through a rubber septum. The tube was sealed with a teflon screwcap and heated to 120˚C with stirring. GC analysis after 48h showed that the desired product had formed and the reaction had proceeded to approximately 23% conversion.

***Example 143***

Electron-Rich, Bulky Phosphine Ligands that Facilitate the Palladium-Catalyzed Preparation of Diaryl Ethers

A general method for the palladium-catalyzed formation of diaryl ethers is described. Electron-rich, bulky aryldialkylphosphine ligands, in which the two alkyl groups are either tert-butyl or 1-adamantyl, are key to the success of the transformation. A wide range of electron-deficient, electronically neutral and electron-rich aryl bromides, chlorides and triflates can be combined with a variety of phenols using sodium hydride or potassium phosphate as base in toluene at 100 ˚C. The bulky yet basic nature of the phosphine ligand is thought to be responsible for increasing the rate of reductive elimination of the diaryl ether from palladium.

A variety of naturally occurring and medicinally important compounds contain a diaryl ether moiety.[1] Of the methods used for the preparation of diaryl ethers, the classic Ullmann ether synthesis is the most important but it is often limited by the need to employ harsh reaction conditions and stoichiometric amounts of copper.[2] While a number of interesting and useful techniques for diaryl ether formation have been reported in recent years,[3] a need for general methods for their preparation remains. Recently, we reported a general copper-catalyzed preparation of diaryl ethers which constitutes a significant improvement to the Ullmann ether synthesis.[4] The use of palladium catalysis for the combination of phenols and aryl halides or sulfonates is a desirable extension of other recently reported carbon-heteroatom bond forming techniqnes.[5,6] This procedure has been demonstrated,[7] but the scope of the reported process was limited to the reaction of electron-deficient aryl bromides. Moreover, the procedures usually required the use of the sodium salt of the phenol and in most cases the yields were only moderate.

Herein we report that a wide range of electron-deficient, electronically neutral and electron-rich aryl halides and sulfonates can be combined with a variety of phenols using palladium catalysis, representing a substantial improvement in generality and utility of these couplings (eq 1).

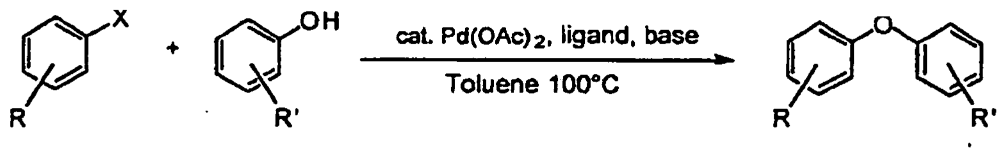


(1)

Critical to the success of the method is the use of electron-rich, sterically bulky aryldialkylphosphines as ligands.[8] Specifically, only the use of catalyst systems with ligands containing a phosphorous center substituted with two tert-butyl or 1-adamantyl groups effects efficiently the desired transformation.

We recently reported that 2-dicyclohexylphosphino-2'-dimethylaminobiphenyl (**1**) serves as an excellent ligand for the palladium-catalyzed amination of aryl halides and for room-temperature Suzuki coupling reactions of aryl chlorides and bromides.[8e] Our results demonstrated that room-temperature

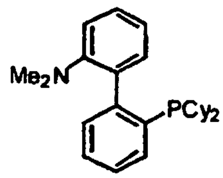

**1**

oxidative addition of aryl chlorides to palladium centers can be achieved using simple electron-rich phosphines.[9,10] Our findings suggested that when this new ligand was used, the rate-limiting step in the catalytic cycle of a cross-coupling process may be shifted from oxidative addition of the aryl halide to a Pd(0) complex to either Pd-N bond formation or the reductive elimination leading to the C-N bond formation.

In our initial studies to extend these results to find improved catalysts for carbon-oxygen bond formation, we sought to utilize **1** for the combination of NaO*t*-Bu and 2-chloro-*p*-xylene to afford the corresponding *t*-butyl ether. Unfortunately, our efforts were met with little success. We reasoned that the problem was due to the recalcitrant nature of the C-O bond reductive elimination from the Pd center.[6d,11] This was predicated on previous studies which suggested that in Pd-catalyzed carbon-oxygen bond-forming reactions, the rate-limiting step most likely involves the formation of the carbon-oxygen bond *via* reductive elimination.[6c-d,7a,11] It is known that increasing the steric bulk of ligands can facilitate reductive elimination processes.[12] We felt that by increasing the size of the dialkylphosphino group, the desired transformation might be induced to occur.[13] With this in mind, we prepared **2** from 2-bromo-2'-dimethylaminobiphenyl[8e] and commercially available di-*t*-butylchlorophosphine.[14] Attempts to prepare **2** using the same conditions developed for the synthesis of **1** (1.1 equiv. *n*-BuLi, THF, -78 °C → rt) were unsuccessful. We found, however, that switching to diethyl ether as the solvent gave **2** in moderate yield. The reaction of the *in situ* generated aryllithium reagent with di-*t*-butylchlorophosphine is quite slow, and we believe that the stability of the aryllithium reagent in ether relative to THF[15] is key to the success of the increased yield of **2**.

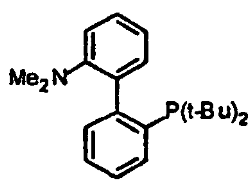

**2**

We initially examined the use of **2** in the reaction of 2-chloro-*p*-xylene and sodium *t*-butoxide (eq. 2). We were surprised to find in addition to the expected product **A**, that 19% (uncorrected GC yield) of diaryl ether **B** was formed. This result encouraged us to explore the use of **2** in coupling reactions to form

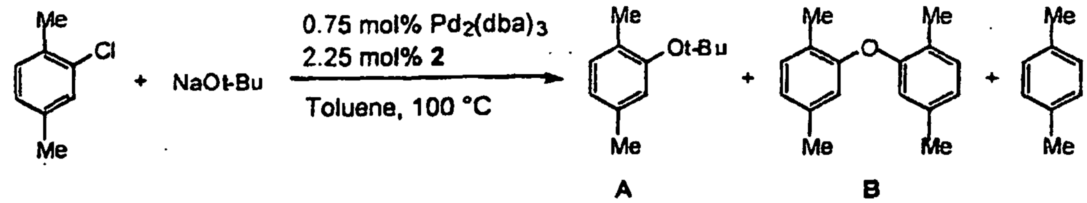

(2)

diaryl ethers. In fact, we found that a smooth reaction of 2-chloro-*p*-xylene and 3,4-dimethylphenol using 1.5 mol % Pd (0) and 2.25 mol % **2** occurred in the presence of sodium hydride in toluene at 100 ˚C to give the desired diaryl ether in 78% isolated yield (eq. 3).

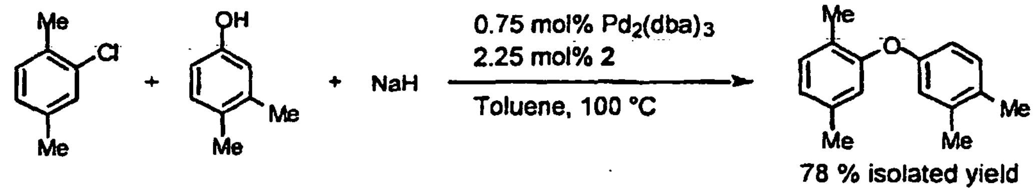

(3)

This result led us to undertake a survey of reaction variables to ascertain the optimum conditions for the transformation. We found that $Pd(OAc)_2$ and $Pd_2(dba)_3$ catalyzed these reactions with comparable efficiency. While in many instances sodium hydride proved to be a suitable base, its use required preheating it with the phenol prior to addition of the other reaction components.[16] This somewhat tedious protocol led us to examine alternative bases for the coupling reaction. Screening a variety of bases, we found that CsF and $K_3PO_4$ were both effective for diaryl ether formation. With respect to reaction rate, yield and cost, $K_3PO_4$ is clearly superior to CsF. Other bases, including $Cs_2CO_3$, $K_2CO_3$, KF, and *n*-BuLi were much less efficient for the process. Comparing $K_3PO_4$ and sodium hydride revealed that reactions with $K_3PO_4$ are significantly slower, but often more efficient than those which use sodium hydride in terms of both the product distribution and the yield. We found that toluene was the only solvent in which the reaction was efficient; THF, DME, dioxane and NMP provided the product in less than 5% yield.

As a control experiment we prepared 2-(di-*t*-butylphosphino)biphenyl (**3**) and were surprised and pleased to find that it is equally efficient in the transformation shown in eq. 3 providing the desired diaryl ether product in 77% isolated yield.[17] While preparation of **2** requires a multi-step sequence, 3 can be obtained in one step from commercially available 2-bromobiphenyl and di-*t*-butylchlorophosphine. Further study showed that this ligand is quite effective in a wide range of palladium-catalyzed diaryl ether-forming reactions.

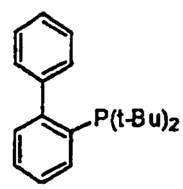

**3**

Compounds 1 to 3 are not ligands according to the invention.

*Synthesis of Diaryl Ethers from Phenols and Electron-Poor Aryl Halides and Triflates*

In our examination of the use of **3** for the combination of electron-poor aryl halides with a variety of phenols, we found that aryl halides or triflates substituted in the para position with electron-withdrawing groups can be coupled with a wide variety of phenols to give the desired product in good to excellent yields (see Table 1). The fact that these activated aryl halides are particularly good substrates is consistent with the results of our previous mechanistic study in which we showed that the presence of para electron-withdrawing groups allow the delocalization of the negative charge which may build up in the transition state of the reductive elimination of the diaryl ether from an intermediate $L_2Pd(OAr)$

Ar' [$L_2$ = chelating phosphine] complex.[11c] The results are also in line with Hartwig's previous findings in the palladium-catalyzed formation of diaryl ethers[7a] and other carbon-heteroatom bond-forming processes.[5d,25] With 4-bromoacetophenone, use of as little as 0.1 mol % Pd was effective; the diaryl ether product was obtained in 95% yield (Table 1, entry 2).[18] Electron-deficient aryl chlorides are also good substrates; the combination of 4-chlorobenzonitrile with 3-isopropylphenol, afforded an 91% yield of the desired product (Table 1, entry 6). Most impressive is that 4-chlorobromobenzene, while only slightly electron-deficient, could be combined with 2-isopropylphenol to give the corresponding diaryl ether in 88% yield; the product results from chemoselective substitution of the bromide substituent (Table 1, entry 7).

In terms of the phenol component of the reaction, use of substrates bearing an ortho alkyl substituent (e.g. *o*-cresol and 2-isopropylphenol) were found to give the highest yields. In the reactions of aryl halides which contain a more weakly electron-withdrawing group on the aromatic ring, only reactions with ortho-substituted phenols are high yielding. The coupling of *N,N*-diethyl 4-bromobenzamide with *o*-cresol is one such case (Table 1, entry 5). This example is particularly significant because copper-mediated procedures with *N,N*-diethyl 4-bromobenzamide fail to yield the desired coupling product.[4]

The reactions of aryl halides having an electron-withdrawing group in the ortho position (e.g. 2-bromoacetophenone and 2-bromobenzonitrile) gave low yields of the desired product with the present catalyst system.[19] At present we have no explanation for these results. The search for a catalyst which will effect these transformations is currently underway in our laboratories.

**Table 1:** Diaryl Ether Formation from Electron-Deficient Aryl Halides using **3**/Pd.[a]

| entry | halide | phenol | product | base | yield (%) |
|---|---|---|---|---|---|
| 1 | 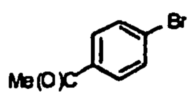 | 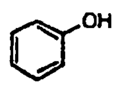 | 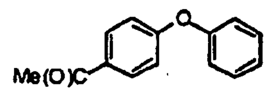 | $K_3PO_4$ | 94 |
| 2 | | 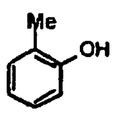 | 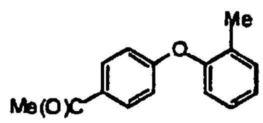 | $K_3PO_4$ | 96 (95)[b] |
| 3 | | 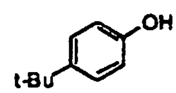 | 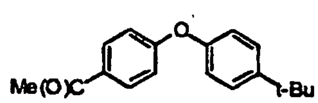 | $K_3PO_4$ | 93 |
| 4 | 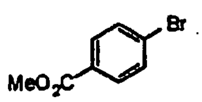 | 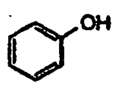 | 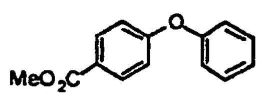 | $K_3PO_4$ | 89 |
| 5[c] | 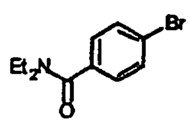 | 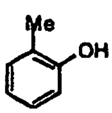 | 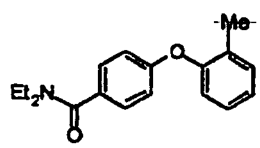 | NaH | 85 |
| 6 | 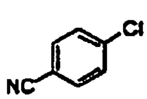 | 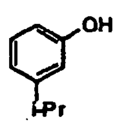 | 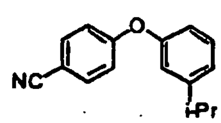 | $K_3PO_4$ | 91 |
| 7[d] | 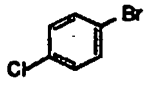 | 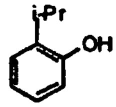 | 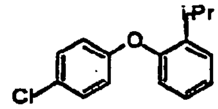 | $K_3PO_4$ | 88 |
| 8 | 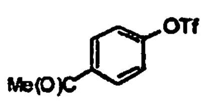 | 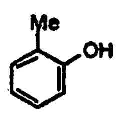 | 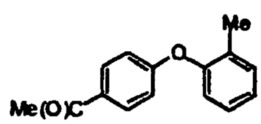 | $K_3PO_4$ | 84 |

(continued)

| entry | halide | phenol | product | base | yield (%) |
|---|---|---|---|---|---|
| 9 | 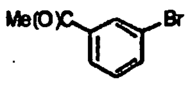 | | 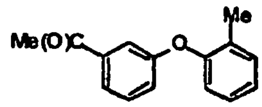 | $K_3PO_4$ | 74 |

(a) Reaction conditions: 1.0 equiv. aryl halide, 1.2 equiv. phenol, 1.4 equiv. NaH or 2.0 equiv. $K_3PO_4$, 2.0 mol % $Pd(OAc)_2$, 3.0 mol % 3, toluene (3 mL), 100 °C, 14-24 h; reaction times were not optimized; (b) Reaction run with 0.1 mol % $Pd(OAc)_2$, 0.15 mol % 3; (c) Reaction nm with 5.0 mol % $Pd(OAc)_2$, 7.5 mol % 3; (d) Reaction run with 1.95 equiv. 2-isopropylphenol.

*Synthesis of Diaryl Ethers from Phenols and Electronically Neutral or Electron-Rich Aryl Halides and Triflates*

We have also examined the reactions of electronically neutral and electron-rich aryl halides and sulfonates in the palladium-catalyzed diaryl ether forming reaction using 3 and related (*vide infra*) ligands. These classes of aryl halides were poor, or in a few cases moderately good substrates in previously reported palladium-catalyzed C-O bond-forming processes.[6,7a] A variety of aryl halides which are unsubstituted at the ortho position are efficiently coupled with a diverse set of phenols using the simple monodentate ligand **3** (Table 2, entries 3-6, 14). Consistent with our results with activated halides, reactions of ortho-substituted phenols with unactivated halides give the highest yields (e.g., Table 2, entries 5-6, 13-14). There are, however, several cases in which ligand **3** is ineffective or affords the desired product in reduced yields. Continuing our search for improved ligands for the synthesis of diaryl ethers, we prepared and evaluated the use of ligands **4-6** in cases for which the use of **3** was unsatisfactory.

**Scheme 1: New Ligands for Diaryl Ether Formation**

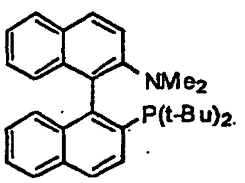

**4**

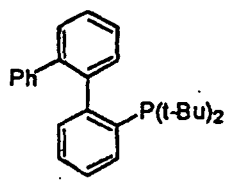

**5**

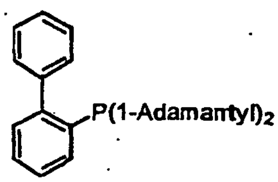

**6**

Compound 4 is a ligand according to the invention.
Compounds 5 and 6 are not ligands according to the invention.

We were pleased to find that binaphthyl ligand **4** was quite effective for the processing of electronically neutral ortho-substituted aryl halides with phenols of several different substitution patterns (Table 2, entries 1, 8-10). In reactions involving these ortho substituted aryl halides, **4** is generally more efficient than **3**.[20]

There are several other difficult cases in which procedures employing **3** and **4** are not satisfactory. For instance, the coupling of an aryl halide lacking an ortho substituent with phenol does not proceed to completion using ligands **3** or **4.** However, using terphenyl ligand **5**, combining 5-bromo-*m*-xylene, and phenol afforded the corresponding diaryl ether in 83% yield (Table 2, entry 2)- This ligand was also quite effective in a number of other cases (Table 2, entries 12, 15-16). Ligands **3, 4**, and **5**, unfortunately, are not effective in the reactions of highly electron-rich aryl chlorides (e.g., 4-chloroanisole). For these transformations, only ligand **6** has been shown to give synthetically useful yields. The 1-adamantyl group was chosen as it occupies a greater volume of space and hence is bulkier than a tert-butyl group. We believe that this is key to the success of **6** in these reactions; 4-chloroanisole and *o*-cresol are converted to the desired product in 73% yield using **6** (Table 2, entry 11), a significantly higher yield than when **3, 4**, or **5** were employed.

**Table 2:** Palladium-Catalyzed Diaryl Ether Formation from Electronically Neutral and Electron-Rich Aryl Halides.[a]

| entry | halide | phenol | product | base | ligand | yield (%) |
|---|---|---|---|---|---|---|
| 1 | 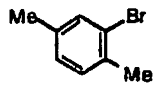 | 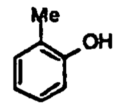 | 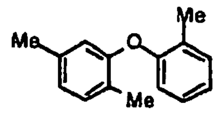 | $K_3PO_4$ | 4 | 95 |
| 2 | 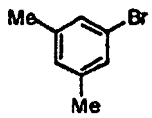 | 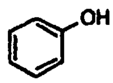 | 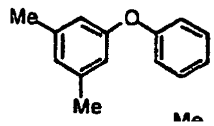 | $K_3PO_4$ | 5 | 83 |
| 3 | | 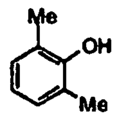 | 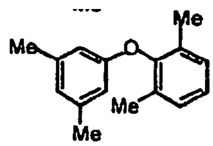 | NaH | 3 | 74 |
| 4 | | 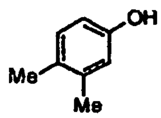 | 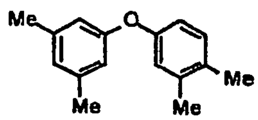 | NaH | 3 | 83 |
| 5 | 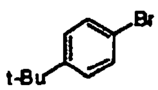 | 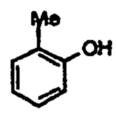 | 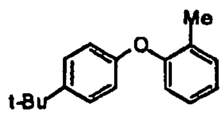 | $K_3PO_4$ | 3 | 85 |
| 6 | 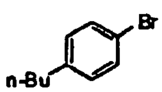 | 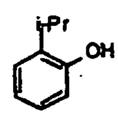 | 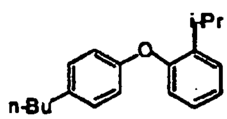 | $K_3PO_4$ | 3 | 92 |
| 7 | 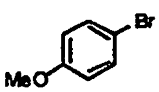 | | 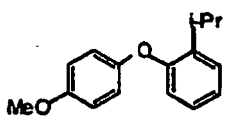 | $K_3PO_4$ | 6 | 87 |
| 8 | 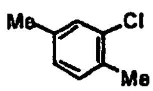 | 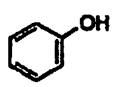 | 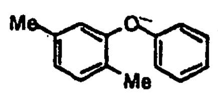 | NaH | 4 | 79 |
| 9[b] | | 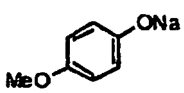 | 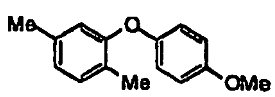 | - | 4 | 92 |
| 10[c] | | 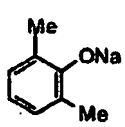 | 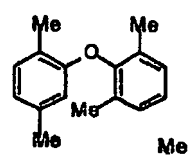 | - | 4 | 87 |
| 11 | 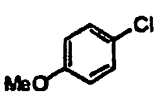 | 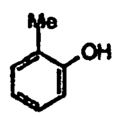 | 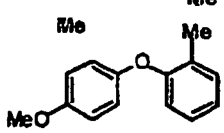 | $K_3PO_4$ | 6 | 73 |
| 12 | 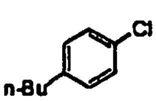 | 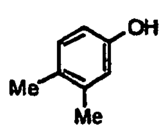 | 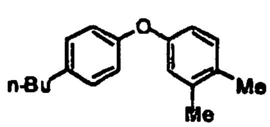 | NaH | 5 | 76 |

(continued)

| entry | halide | phenol | product | base | ligand | yield (%) |
|---|---|---|---|---|---|---|
| 13 | 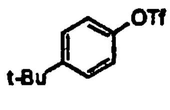 | 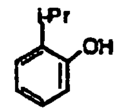 | 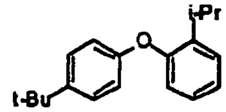 | $K_3PO_4$ | 3 | 84 |
| 14 | 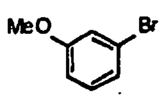 | 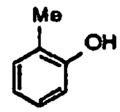 | 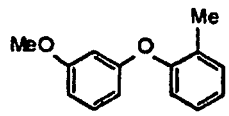 | $K_3PO_4$ | 3 | 87 |
| 15[d] | 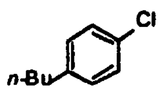 | 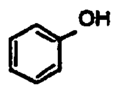 | 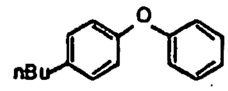 | NaH | 5 | 61 |
| 16 | 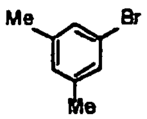 | 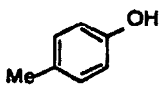 | 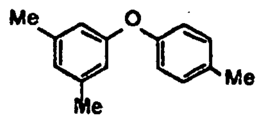 | NaH | 5 | 88 |

(a) Reaction conditions: 1.0 equiv. aryl halide, 1.2 equiv. phenol, 1.4 equiv. NaH or 2.0 equiv. $K_3PO_4$. 2.0 mol % Pd $(OAc)_2$, 3.0 mol % ligand, toluene (3 mL), 100˚C, 14-26 h; reaction times were not optimized; (b) 1.2 equiv. of the phenolate salt was used, 110 ˚C; (c) 1.2 equiv. of the phenolate salt was used, 1.25 mol % $Pd_2(dba)_3$, 3.75 mol % 4, toluene (2 mL), 115 ˚C; (d) 4.0 mol % $Pd(OAc)_2$, 6.0 mol % 5,2.0 equiv. phenol, 2.2 equiv. NaH, 115˚C.

Our proposed catalytic cycle for diaryl ether formation is similar to that proposed for other palladium-catalyzed carbon-carbon and carbon-heteroatom bond forming processes.[5,6,7a] The catalytic cycle consists of three distinct stages: 1) oxidative addition of the aryl halide to $L_nPd(0)$; 2) formation of the Pd-aryloxide complex from the Pd-halide adduct via transmetallation of a metal phenolate; 3) reductive elimination of the diaryl ether product with concomitant regeneration of the active $L_nPd(0)$ species. While the oxidative addition and transmetallation may be expected to be relatively facile,[21] the reductive elimination to form the C-O bond is disfavored due to the Pd-C (LUMO) and Pd-O (HOMO) energy gap.[22]

**Scheme 2: Proposed Catalytic Cycle.**

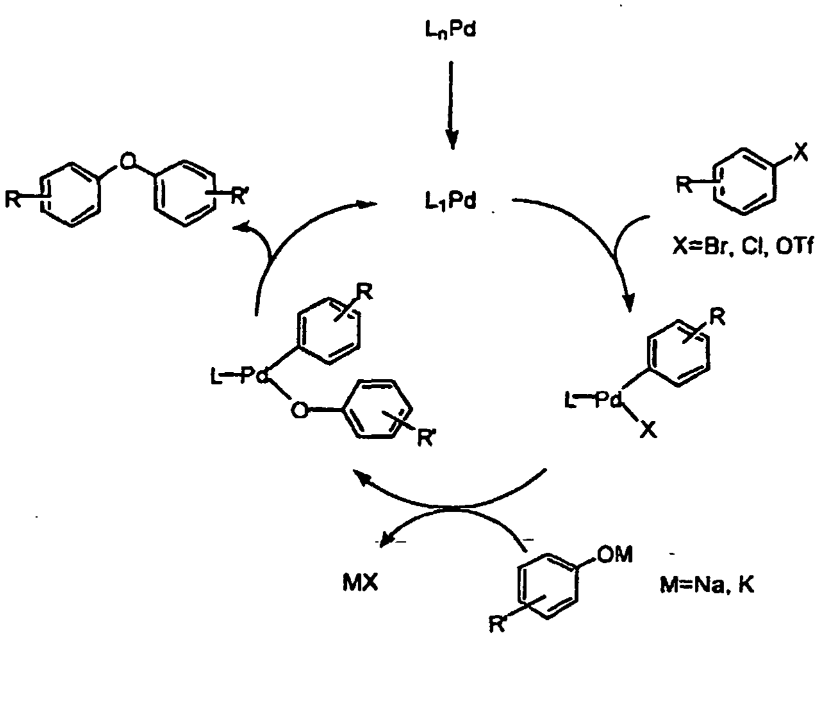

The palladium-catalyzed diaryl ether-forming reactions require only a slight excess of ligand to palladium, and reactions in which the ratio of L/Pd was varied from 1/1 to 1.5/1 to 2/1 gave similar results. This provides circumstantial evidence that the key intermediates in the catalytic cycle are monophosphine palladium complexes.[23] Mechanistic studies by Hartwig of carbon-nitrogen bond-forming reactions catalyzed by palladium complexes with bulky triaryl phosphine ligands demonstrated that the key intermediates in the catalytic cycle were monophosphine palladium complexes.[5b-c,24]

While the exact mechanism(s) for the key reductive elimination step remains unknown, we have previously developed several mechanistic hypotheses for related processes which can be used to account for the observed results. For electron-deficient aryl halides, we still favor a mechanism involving transfer of the phenolate from palladium to the ipso carbon of the aryl halide to form a zwitterionic intermediate which converts to the diaryl ether and a palladium(0) complex.[7a,11c,25] For electronically neutral and electron-rich aryl halides, however, we suggest that a different mechanism for reductive elimination to form the carbon-oxygen bond most likely involves a three-centered transition state.[11c] In these cases, the bulkier ligands are necessary to destabilize the ground state of the LnPd(OAr)Ar' complex, forcing the palladium-bound aryl and aryloxy groups closer together. In this way, the complex is distorted toward the three-centered transition state.[26]

It is informative to compare the results reported here with the reaction of the approximately isosteric primary aniline with the same substrates. The latter processes (using appropriate ligands) are substantially more general and appear to be essentially insensitive to the size or the electronic nature of the substituents on either substrate.[5,27] The rate of reductive elimination to form C-O bonds is significantly slower than the corresponding rate to form C-N bonds.[6d,7a] We speculate that the relatively sluggish rate of this process in the C-O bond-forming reactions, even with ligands (e.g., **3-6**) which give improved results, is the reason for the discrepancy between the efficiencies of the diaryl ether- and diarylamine-forming processes.

What is more complicated is the unraveling of the various factors which contribute to make some of these reactions so facile, while others are inefficient or give none of the desired product It is clear that ortho substitution in either the phenol or the aryl halide is beneficial to the success of the reaction. There are many possible explanations for this observation including enhanced steric interaction of the aryl group(s) with the bulky ligands or improved solubility of key intermediate complexes.[28] While we have significantly expanded the scope of palladium-catalyzed diaryl ether formation, it is not obvious to us, for example, why the presence of ortho electron-withdrawing group on the aryl halide should decrease the efficiency of the process. Additionally, aryl halides bearing a strongly electron-donating group at the ortho position (e.g. 2-bromoanisole, which is a good substrate for related amination reactions)[5,27] and electron-deficient phenols (e.g. 4-hydroxyacetophenone) do not give good results in these coupling reactions.

The current procedure enhances the utility of palladium-catalyzed coupling reactions of phenols with aryl halides in several respects.[7a] These include: 1) for most substrate combinations, the use of preformed sodium phenolates is obviated; 2) the reactions are, in general, more efficient with respect to quantity of catalyst required and the yields obtained; 3) a much wider range of substrates can be utilized including electron-rich and electronically neutral aryl halides and triflates; 4) a higher level of functional group compatibility can be realized. For example, aryl halides containing simple esters or enolizable ketones are now tolerated. Moreover, *o*-substituted phenols, even those with a bulky substituent, are excellent substrates; 5) only 2 mol % $Pd(OAc)_2$ and 3 mol % ligand is required as the catalyst and in special cases a level as low as 0.1 mol % was effective; 6) the mild and inexpensive base $K_3PO_4$ is effective in a large majority of these reactions; 7) inexpensive and readily available aryl chlorides may be used as substrates. We are currently working to improve the scope and generality of palladium-catalyzed diaryl ether formation. We anticipate that existing difficulties will be overcome through the development of new palladium catalyst systems. These efforts and their application to organic synthesis will be reported in due course.

*General Experimental Procedures*

All reactions were performed under argon in oven- or flame-dried glassware. Toluene was distilled under nitrogen from molten sodium. Ethyl ether and THF were distilled under argon from sodium benzophenone ketyl. Reagents were purchased from commercial sources and were used without further purification, unless otherwise noted. Tribasic potassium phosphate was purchased from Fluka Chemical Company. Tetrakis(triphenylphosphine)palladium, palladium acetate, tns(dibenzy!ideneacetone)dipaHadium(0) and 2,2'-dibromo-1,1'-binaphthyl were purchased from Strem Chemicals, Inc. 2-Bromobiphenyl was purchased from Lancaster Synthesis Inc. Di-*t*-butylchlorophosphine was purchased from either Aldrich Chemical Company or Strem Chemicals, Inc. Solutions of tert-Butyllithium were purchased from Aldrich Chemical Company. Sodium salts of phenols were prepared using a slight excess of sodium metal in refluxing THF.[29] Elemental analyses were performed by E & R Microanalytical Laboratory Inc., Parsippany, N.J. IR spectra were obtained by placing neat samples directly on the DiComp probe of an ASI REACTIR *in situ* IR instrument. Yields in the tables refer to isolated yields (average of at least two runs) of compounds which are 95% pure as determined by $^1H$ NMR and GC analysis, or combustion analysis. The products of entries 1,[30,31] 3[30] and 432 from Table 1, and entries

1[4], 4[4] and 16[4] from Table 2 have been described in the literature and were characterized by comparison of their $^1$H NMR spectra to the previously reported data; their purity was confirmed by GC analysis. The procedures described in this section are representative, thus the yields may differ slightly from those given in Tables 1 and 2.

*Ligand Syntheses*

**2-(*N,N*-dimethylamino)-2'-di-*t*-butylphosphinobiphenyl** (2). An oven-dried Schlenk tube was purged with argon and charged with 2-(*N,N*-dimethylamino)-2'-bromobiphenyl[8e] (1.104 g, 4.0 mmol). The tube was purged with argon and ether (18 mL) was added *via* syringe. The resulting solution was cooled to -78 ˚C and *n*-butyllithium in hexanes (1.6 M, 2.75 mL, 4.4 mmol) was added dropwise with stirring. The mixture was stirred at -78˚C for 30 min, then warmed to 0 ˚C. Di-*t*-butylchlorophosphine (0.96 mL, 5.0 mmol) was added *via* syringe, and the mixture was allowed to slowly warm to room temperature overnight (17 h). The mixture was quenched with saturated aqueous ammonium chloride (10 mL), diluted with ether (40 mL), and transferred to a separatory funnel. The layers were separated and the aqueous layer was extracted with ether (1×20 mL). The combined organic layers were dried over anhydrous sodium sulfate, filtered, and concentrated *in vacuo.* The resulting oil was taken up in a small amount of hot methanol (ca. 10 mL), the bottom of the flask was scratched with a spatula, and crystallization was allowed to occur slowly in a -20 ˚C freezer. The resulting crystals were washed with cold methanol and dried under vacuum to afford 683 mg (50%) of a white solid, mp 116-117 ˚C. $^1$H NMR (300 MHz, $CDCl_3$) δ 7.80-7.75 (m, 1H), 7.40-7.26 (m, 4H), 7.00-6.90 (m, 3H), 2.44 (s, 6H), 1.26 (d, 9H, *J*=11.4 Hz), 0.90 (d, 9H, *J*=11.2 Hz); $^{31}$P NMR (121 MHz, $CDCl_3$) δ 25.3; $^{13}$C NMR (75 MHz, $CDCl_3$) δ 151.53, 151.5, 150.3, 149.8, 137.1, 137.0, 136.9, 136.7, 135.6, 135.5, 132.7, 131.0, 130.9, 128.7, 127.8, 125.2, 120.9, 117.4, 43.2, 33.4, 33.1, 31.5, 31.3, 31.1, 30.0, 29.8 (observed complexity due to P-C splitting; definitive assignments have not yet been made); IR (neat, $cm^{-1}$) 2941, 1416, 947, 745; Anal. Calcd for $C_{22}H_{32}NP$: C, 77.38; H, 9.45. Found: C, 77.16; H, 9.56.

**2-(Di-t-butylphosphino)biphenyl (3).** An oven dried round-bottomed flask equipped with a magnetic stirbar and a rubber septum was allowed to cool to room temperature under an argon purge. The flask was charged with magnesium turnings (617 mg, 25.4 mmol) and a small crystal of iodine. The flask was purged with argon and a solution of 2-bromobiphenyl (5.38 g, 23.1 mmol) in THF (40 mL) was added. The mixture was heated to reflux with stirring for 2 h, then allowed to cool to room temperature. The septum was removed and anhydrous copper (I) chloride (2.40 g, 24.2 mmol) was added. The flask was capped with the septum and purged with argon for 2 min. Di-*t*-butylchlorophosphine (5.0 g, 27.7 mmol) was added via syringe, and the mixture was heated to reflux with stirring for 8 h. The mixture was cooled to room temperature and diluted with 1:1 hexanes:ether (200 mL). The resulting suspension was filtered, and the solids were washed with hexanes (60 mL). The solid material was transferred to a flask containing 1:1 hexane:ethyl acetate (150 mL) and water (100 mL) and 30% aqueous ammonium hydroxide (60 mL) were added. The resulting slurry was stirred at room temperature for 5 min then transferred to a separatory funnel. The layers were separated and the organic phase was washed with brine (100 mL), dried over anhydrous sodium sulfate, filtered, and concentrated *in vacuo.* The resulting solid was recrystallized from methanol (2 crops of crystals were collected) to afford 4.46 g (67%) of a white solid, mp 86-86.5 ˚C. $^1$H NMR (300 MHz, $CDCl_3$) δ 7.95-7.85 (m, 1H), 7.40-7.21 (m, 8H), 1.15 (d, 18H, *J*=11.6 Hz); $^{31}$P NMR (121 MHz, $CDCl_3$) δ 18.7; $^{13}$C NMR (75 MHz, $CDCl_3$) δ 151.4, 150.9, 143.6, 143.5, 135.6, 135.2, 135.0, 130.5, 130.4, 130.1, 128.3, 127.0, 126.7, 126.5, 126.2, 126.0, 125.6, 32.7, 32.4, 30.8, 30.6 (observed complexity due to P-C splitting; definitive assignments have not yet been made); IR (neat, $cm^{-1}$) 2956, 1459, 1362, 1173; Anal. Calcd for $C_{20}H_{27}P$: C, 80.50; H, 9.12. Found: C, 80.67; H, 9.36.

**2-*N, N*-Dimethylamino-2'-di-*t*-butylphosphino- 1,1'- binaphthyl (4)**. An oven-dried round-bottomed flask was purged with argon, and charged with 2,2'-dibromo-1,1'-binaphthyl (5.0 g, 12.1 mmol), benzophenone imine (2.90 g, 15.7 mmol), NaO*t*-Bu (1.70 g, 18.0 mmol), $Pd_2(dba)_3$ (110 mg, 0.12 mmol), 2,2'-bis(diphenylphosphino)-1,1'-diphenyl ether[33] (129 mg, 0.24 mmol), and toluene (50 mL). The flask was fitted with a reflux condenser and the mixture was stirred for 18 h at 100 ˚C then cooled to room temperature and two-thirds of the solvent was removed under reduced pressure. Ethanol (25 mL) and water (3 mL) were added to the resulting mixture. The yellow crystals were collected on a Büchner funnel and washed with ethanol (10 mL) to afford 5.7 g (92%) of crude 2-amino-2'-bromo-1,1'-binaphthyl benzophenone imine which was used in the following reaction without further purification.

The crude imine (3.0 g, 5.9 mmol) was suspended in dichloromethane (100 mL) in a round-bottomed flask. Concentrated hydrochloric acid (1.5 mL, 17.6 mmol) was added to the suspension which became homogeneous within 15 min. The reaction mixture was stirred for 18 h at room temperature during which time a precipitate formed. The mixture was then treated with 1M NaOH (25 mL) and the layers were separated. The aqueous layer was extracted with additional dichloromethane (10 mL). The combined organic layers were washed with brine, dried over anhydrous magnesium sulfate, filtered, and concentrated *in vacuo.* The crude material was then purified by flash chromatography on silica gel to give 1.5 g (73%) of 2-amino-2'-bromo-1,1'-binaphthyl as colorless crystals.

A round-bottomed flask was charged with 2-amino-2'-bromo-1,1'-binaphthyl (480 mg, 1.4 mmol), iodomethane (0.25 mL, 4.2 mmol), sodium carbonate (318 mg, 3.0 mmol), and DMF (8 mL) and then purged with argon. The mixture

was heated to 50 °C and stirred until the starting material had been completely consumed. The reaction mixture was diluted with ether (5 mL) and water (1 niL) and then passed through a plug of silica gel. The filtrate was dried over anhydrous magnesium sulfate, filtered, and concentrated *in vacuo* to give 473 mg (91%) of 2-*N,N*-dimethylamino-2'-bromo-1,1'-binaphthyl as colorless crystals.

An oven-dried round-bottomed flask was charged with 2-*N,N*-dimethylamino-2'-bromo-1,1'-binaphthyl (376 mg, 1.0 mmol) and purged with argon. DME (5 mL) was added, the resulting solution was cooled to 0 °C and then *t*-butyllithium in hexanes (1.7 M, 1.2 mL, 2.0 mmol) was added dropwise. The solution was warmed to room temperature and stirred for 30 min. Di-*t*-butylchlorophosphine (397 mg, 0.96 mmol) was then added dropwise and the reaction was stirred at room temperature for 1.8 h. Saturated aqueous ammonium chloride (2 mL) was added and the reaction mixture was extracted with ether (2x10 mL). The combined organic extracts were dried over anhydrous magnesium sulfate, filtered, and concentrated *in vacuo.* The crude material was recrystallized from ether/methanol to give 295 mg (67%) of 2-*N,N*-dimethylamino-2'-di-*t*-butylphosphino-1,1'-binaphthyl as colorless crystals, mp 188-189 °C. $^1$H NMR (500 MHz, $CDCl_3$) δ 8.03 (broad d, 1H, *J*=8.5 Hz), 7.92-7.75 (m, 3H), 7.75 (broad d, 1H, *J*=8.2 Hz), 7.48-7.43 (m, 2H), 7.31 (broad d, 1H, *J*=8.5 Hz), 7.24-7.16 (m, 2H), 6.98 (m, 1H), 6.74 (broad d, 1H, *J*=8.6 Hz), 2.46 (s, 6H), 1.26 (d, 9H, *J*=11.3 Hz), 0.75 (d, 9H, *J*=11.3 Hz); $^{31}$PNMR (121 MHz, $CDCl_3$) δ 26.2; $^{13}$C NMR (125 MHz, $CDCl_3$) δ 149.6, 145.8, 145.5, 136.5, 136.3, 134.6, 134.1, 134.0, 133.4, 132.96, 132.94, 129.2, 128.7, 128.03, 128.01, 127.7, 127.5, 127.1, 126.6, 126.5, 126.0, 125.8, 125.4, 124.9, 122.8, 119.0, 43.3, 32.8, 32.6, 31.8, 31.5, 31.4, 31.3, 30.3, 30.1 (observed complexity due to P-C splitting; definitive assignments have not yet been made); IR (neat, $cm^{-1}$) 3060, 2941, 1596, 1474, 1173; Anal. Calcd for $C_{30}H_{36}NP$: C, 81.60; H, 8.22. Found: C, 81.59; H, 8.60.

**1-(Di-*t*-butylphosphino)-*o*-terphenyl** (5). An oven-dried Schlenk tube was cooled to room temperature under an argon purge and was charged with magnesium turnings (243 mg, 11.0 mmol), ether (7 mL), and 1,2-dibromoethane (38 μL). The mixture was stirred at room temperature until gas evolution ceased, then a solution of 2-bromobiphenyl (1.7 mL, 10.0 mmol) in ether (5 mL) was added dropwise. The mixture was stirred at room temperature for 1.75 h. The solution was then transferred via cannula to a separate flask containing an ice-cooled solution of triisopropyl borate (4.6 mL, 20.0 mmol) in THF (20 mL). The mixture was stirred at 0 °C for 15 min, then warmed to room temperature and stirred for 21 h. The reaction was quenched with 1 M HCl (40 mL) and stirred at room temperature for 10 min. The solution was basified to pH 14 with 6 M NaOH, then extracted with ether (1x10 mL). The organic phase was discarded and the aqueous phase was acidified to pH 2 with 6 M HCl, extracted with ether (3x50 mL), and the combined organic layers were dried over anhydrous sodium sulfate, filtered, and concentrated *in vacuo.* The crude material was recrystallized from ether/pentane at -20 °C to afford 1.0 g (51%) of *o*-biphenyl boronic acid as a white, crystalline solid, which was used without further purification.

An oven-dried Schlenk flask was cooled to room temperature under an argon purge, and was charged with tetrakis(triphenylphosphine)palladium (289 mg, 0.25 mmol, 5 mol %), sodium carbonate (2.86 g, 27 mmol), and *o*-biphenyl boronic acid (1.0 g, 5.0 mmol). The flask was purged with argon and DME (50 mL), ethanol (2 mL), water (15 mL), and 2-bromoiodobenzene (0.83 mL, 6.05 mmol) were added through a rubber septum. The mixture was heated to 85 °C with stirring for 3 d. After cooling to room temperature, the reaction mixture was diluted with ether (100 mL) and poured into a separatory funnel. The layers were separated and the organic phase was washed with 1M NaOH (2x50 mL) and brine, then dried over anhydrous magnesium sulfate, filtered, and concentrated *in vacuo.* The crude material was purified by flash chromatography on silica gel to afford 1.23 g (79%) of 1-bromo-*o*-terphenyl as a colorless oil.

An oven-dried Schlenk tube was cooled to room temperature under an argon purge, and was charged with magnesium turnings (54 mg, 2.2 mmol), THF (2 mL), and 1,2-dibromoethane (9 μL). The mixture was stirred at room temperature for 15 min, then a solution of 1-bromo-*o*-terphenyl (618 mg, 2.0 mmol) in THF (1 mL) was added dropwise. The mixture was stirred at room temperature for 1h, then the septum was removed from the flask, and copper (1) chloride (283 mg, 2.1 mmol) was added. The tube was capped with the septum and purged with argon for 1 min. The tube was charged with di-*t*-butylchlorophosphine (0.46 mL, 2.4 mmol) and additional THF (1 mL). The mixture was heated to 60°C with stirring for 26 h. The mixture was cooled to room temperature and filtered, and the solids were washed with ether/hexanes (50 mL, 1/1 v/v). The organic fraction was poured into a separatory funnel and washed with 38% aqueous ammonium hydroxide (3x50 mL) and brine (50 mL), dried over anhydrous sodium sulfate, filtered, and concentrated. The crude material was recrystallized from hot methanol to afford 191 mg (26%) of the title compound as a white, crystalline solid, mp 95-97 °C. $^1$H NMR (300 MHz, $CDCl_3$) δ 7.56 (d, 1H, *J*=7.5Hz), 7.54-7.07 (m, 12H), 0.92 (d, 9H, *J*=11.1 Hz), 0.68 (d, 9H, *J*=11.1 Hz); $^{31}$P NMR (121 MHz, $CDCl_3$) δ 20.9; $^{13}$C NMR (75 MHz, $CDCl_3$) δ 150.1, 149.8, 142.4, 141.4, 141.3, 140.95, 140.92, 135.8, 135.65, 135.63, 135.6, 132.8, 132.0, 131.9, 130.6, 130.0, 127.9, 127.8, 127.2, 126.1, 125.9, 125.6, 33.5, 33.3, 31.8, 30.89, 30.88, 30.8, 30.0, 29.8 (observed complexity due to P-C splitting; definitive assignments have not yet been made); IR (neat, $cm^{-1}$) 2946, 1459, 1362, 1173; Anal. Calcd for $C_{26}H_{31}P$: C, 83.39; H, 8.34. Found: C, 83.40; H, 8.40.

**2-[Di-(1-adamantyl)phosphino]biphenyl (6)**. An oven-dried, round-bottomed flask was charged with magnesium turnings (15.3 g, 0.63 mol) and 1-bromoadamantane (9.0 g, 0.041 mol). The flask was purged with argon, then ethyl ether (45 mL) was added and the mixture was gently refluxed for 15 h, without stirring.[34] A separate flame-dried, two-necked,

round-bottomed flask equipped with a reflux condenser was charged with $PCl_3$ (0.9 mL, 10 mmol) and ether (15 mL) and was cooled to -40 ˚C. To this solution was added the solution of the Grignard reagent *via* a syringe, slowly enough so that the reaction temperature was kept below -25 ˚C. The resulting mixture was stirred for 30 min at -45 ˚C, then the cooling bath was removed and the reaction mixture was allowed to warm slowly to room temperature. After stirring for an additional 30 min at room temperature, the reaction vessel was placed into a 37 ˚C oil bath and the solution was allowed to gently reflux for 22 h. The mixture was cooled to room temperature and then was cannula filtered into a separate flask. The solvent as well as some of the adamantane byproduct was removed *in vacuo,* without exposing the product to air, to afford a crude mixture of di-(1-adamantyl)chlorophosphine and di-(1-adamantyl)bromophosphine. This mixture was used in the next step without further purification.

An oven-dried Schlenk tube was charged with magnesium turnings (240 mg, 9.89 mmol) and 2-bromobiphenyl (1.55 mL, 7.5 mmol). The tube was purged with argon, then THF (15 mL) was added through a rubber septum and the reaction mixture was heated to a mild reflux for 3 h. The reaction mixture was then cooled to room temperature, the septum was removed and copper (I) chloride (930 mg, 9.45 mmol) was added. The tube was capped with a septum and purged with argon, then a solution of the di-(1-adamantyl)chlorophosphine/di-(1-adamantyl)bromophosphine mixture (prepared above) in THF (5 mL) was added. The reaction mixture was heated to reflux for 3 h then was allowed to cool to room temperature and ether (50 mL) and pentane (50 mL) were added. The resulting suspension was stirred for 10 min, during which time a heavy dark-brown precipitate formed. The suspension was filtered and the solid was collected on a fritted funnel. The solid was partitioned between ethyl acetate/ether (100 mL, 1/1 v/v) and 38% aqueous ammonium hydroxide (50 mL) and water (50 mL). The mixture was vigorously shaken several times over 30 min and the layers were separated. The aqueous layer was washed with ether/ethyl acetate (2x100 mL, 1/1 v/v), and the combined organic layers were washed with brine (2x50 mL), dried over anhydrous magnesium sulfate, decanted, and concentrated in vacuo. The product was crystallized from toluene/methanol to afford 450 mg (6%) of the title compound as a white solid, mp 222-224 ˚C. $^1H$ NMR (300 MHz, $CDCl_3$) δ 7.92-7.87 (m, 1H), 7.41-7.16 (m, 8H), 1.95-1.79 (m, 18H), 1.69-1.62 (m, 12H); $^{31}P$ NMR (121 MHz, $CDCl_3$) δ 21.5; $^{13}C$ NMR (75 MHz, $CDCl_3$) δ 151.9, 151.5, 143.9, 143.8, 136.53, 136.49, 133.1, 132.8, 130.6, 130.55, 130.49, 129.0, 128.15, 128.08, 128.07, 127.0, 126.2, 125.2, 42.0, 41.8, 37.5, 37.14, 36.9, 28.9, 28.78 (observed complexity due to P-C splitting; definitive assignments have not yet been made); IR (neat, $cm^{-1}$) 2898, 1443, 1343, 697; Anal. Calcd for $C_{32}H_{39}P$: C, 84.54; H, 8.65. Found: C, 84.40; H, 8.57.

*Pd-Catalyzed Coupling of Aryl Halides with Phenols*

General Procedure A. An oven-dried resealable Schlenk tube was fitted with a rubber septum and was cooled to room temperature under an argon purge. The septum was removed and the tube was charged with palladium acetate (4.5 mg, 0.02 mmol, 2.0 mol %), ligand (**3** (9.0 mg) or **4** (13.2. mg) or **5** (11.2 mg) or **6** (13.6 mg), 0.03 mmol, 3.0 mol %), potassium phosphate (424 mg 2.0 mmol), the phenol (1.2 mmol) and the aryl halide (1.0 mmol) (Compound 4 is a ligand according to the invention. Compounds 3, 5 and 6 are not ligands according to the invention.). The tube was capped with the septum and purged with argon, then toluene (3 mL) was added through the septum. The tube was sealed with a teflon screwcap, and the reaction mixture was stirred at 100 ˚C for 14-26 h (reaction times were not optimized). The reaction was then subjected to workup method 1 or 2 (see below).

General Procedure B. An oven-dried resealable Schlenk tube was charged with sodium hydride (dry 95%, 36 mg, 1.4 mmol) in a nitrogen-filled glovebox. The tube was sealed and removed from the glovebox, then fitted with a septum and purged with argon. The phenol (1.0 mmol) and toluene (2.5 mL) were added, and the resulting mixture was stirred at 100 ˚C for 15 min under argon. The reaction mixture was allowed to cool to room temperature, then the septum was removed and the tube was charged with palladium acetate (4.5 mg, 0.02 mmol, 2.0 mol %) and ligand (**3** (9.0 mg) or **4** (13.2 mg) or **5** (11.2 mg) or **6** (13.6 mg), 0.03 mmol, 3.0 mol %. (Compound 4 is a ligand according to the invention. Compounds 3, 5 and 6 are not ligands according to the invention.). The tube was capped with the septum and purged with argon. The aryl halide (1.0 mmol) and additional toluene (0.5 mL) were added, the tube was sealed with a teflon screwcap, and the reaction mixture was stirred at 100 ˚C for 14-24 h (reaction times were not optimized). The reaction was then subjected to workup method 1 or 2 (see below).

Workup Method 1. The reaction mixture was allowed to cool to room temperature and was then diluted with ether (40 mL), filtered and concentrated. The crude material was purified by flash chromatography on silica gel.

Workup Method 2. The reaction mixture was allowed to cool to room temperature and was then diluted with ether (40 mL) and poured into a separatory funnel. The mixture was washed with 1 M NaOH (20 mL) and brine (20 mL) and then the organic fraction was dried over anhydrous magnesium sulfate or sodium sulfate, filtered, and concentrated. The crude material was purified by flash chromatography on silica gel.

**2,3',4',5-Tetramethyldiphenyl ether (the reaction shown in equation 3 using ligand 2).** An oven-dried resealable Schlenk tube was charged with sodium hydride (dry 95%, 36 mg, 1.4 mmol) in a nitrogen-filled glovebox. The tube was sealed and removed from the glovebox, then fitted with a rubber septum and purged with argon. Toluene; (1 mL) was added, followed by a solution of 3,4-dimethylphenol (147 mg, 12 mmol) in toluene: (2 mL). The mixture was stirred at

room temperature for 2 min, then heated to 100˚C with stirring for 15 min. The reaction mixture was allowed to cool to room temperature, then the septum was removed and $Pd_2(dba)_3$ (6.9 mg, 0.0075 mmol, 1.5 mol % Pd) and **2** (not according to the invention; 7.7 mg, 0.0225 mmol, 2.25 mol %) were added. The tube was capped with the septum and purged with argon. 2-chloro-*p*-xylene (0.135 mL, 1.0 mmol) and additional toluene (1 mL) were added, and the tube was sealed with a teflon screwcap. The mixture was heated to 100 ˚C with stirring until the starting aryl halide had been completely consumed as judged by GC analysis (19 h). The mixture was cooled to room temperature, diluted with ether (30 mL), filtered through celite, and concentrated *in vacuo.* The crude material was purified by flash chromatography on silica gel to afford 177 mg (78%) of the title compound as a colorless oil.

**2,3',4',5-Tetramethyldiphenyl ether (the reaction shown in equation 3 using ligand 3).** An oven-dried resealable Schlenk tube was charged with sodium hydride (dry 95%, 36 mg, 1.4 mmol) in a nitrogen-filled glovebox. The tube was sealed and removed from the glovebox, then fitted with a rubber septum and purged with argon. 3,4-Dimethylphenol (147 mg, 1.2 mmol) and toluene (2.0 mL) were added and the resulting mixture was stirred at 100 ˚C for 15 min under argon. The reaction mixture was allowed to cool to room temperature, then the septum was removed and $Pd2(dba)_3$ (6.9 mg, 0.0075 mmol, 1.5 mol % Pd), **3** (not according to the invention; 6.7-mg, 0.0225 mmol) were added. The tube was capped with the septum and purged with argon. 2-chloro-*p*-xylene (135 μL, 1.0 mmol) was added, then the tube was sealed with a teflon screwcap and the reaction mixture was stirred at 100 ˚C for 14 h. The mixture was allowed to cool to room temperature, then water (5 mL) and ether (40 mL) were added and the resulting solution was poured into a separatory funnel. The organic phase was separated, dried over anhydrous magnesium sulfate, filtered, and concentrated *in vacuo.* The crude material was purified by flash chromatography on silica gel to afford 175 mg (77%) of the title compound as a colorless oil. $^1H$ NMR ($CDCl_3$, 300 MHz) δ 7:12 (d, 1H, *J*=7.5 Hz), 7.05 (d, 1H, *J*=8.1 Hz), 6.85 (d, 1H, *J*=8.1 Hz), 6.74 (d, 1H, *J*=3.0 Hz), 6.70 (broad s, 1H), 6.64 (dd, 1H, *J*= 8.1, 3.0 Hz), 2.27 (s, 3H), 2.23 (s, 6H), 2.21 (s, 3H); $^{13}C$ NMR ($CDCl_3$, 125 MHz) δ 155.8, 154.7, 138.0, 136.9, 131.0, 130.42, 130.41, 126.4, 124.2, 119.9, 118.8, 114.7, 21.0, 20.0, 18.9, 15.8; IR (neat, $cm^{-1}$) 2923, 1495, 1256; Anal. Calcd for $C_{16}H_{18}O$: C, 84.91; H, 8.02. Found: C, 84.67; H, 8.03.

**4-Phenoxyacetophenone (Table 1, entry 1).**[30,31] General procedure A (workup method 2, ligand **3**; not according to the invention) was used to convert 4-bromoacetophenone and phenol in 16 h to 204 mg (89%) of the title compound, which was obtained as a white solid, mp 50-51 ˚C (lit.[30] mp 51 ˚C).

**4-(2'-Methylphenyloxy)acetophenone (Table 1, entry 2)**.[35] General procedure A (workup method 2, ligand **3**; not according to the invention) was used to convert 4-bromoacetophenone and *o*-cresol in 15 h to 213 mg (96%) of the title compound, which was obtained as a white solid, mp 34.5-35.5 ˚C (lit.[35] oil). $^1H$ NMR ($CDCl_3$, 300 MHz) δ 7.94-7.89 (m, 2H), 7.30-7.12 (m, 3H), 6.99 (broad d, 1H, *J*=7.5 Hz), 6.91-6.87 (m, 2H), 2.57 (s, 3H), 2.19 (s, 3H); $^{13}C$ NMR ($CDCl_3$, 125 MHz) δ 196.5, 162.1, 152.8, 131.6, 131.2, 130.5, 130.4, 127.4, 125.2, 120.9, 115.8, 26.5, 16.2; IR (neat, $cm^{-1}$) 1675; Anal. Calcd for $C_{15}H_{14}O_2$: C, 79.62; H, 6.24. Found: C, 79.75; H, 6.55.

**4-(2'-Methylphenyloxy)acetophenone (Table 1, entry 2, using 0.1 mol % Pd).**[35] General procedure A (workup method 2, ligand **3**; not according to the invention) was employed, with the following changes in the amount of materials used: palladium acetate (1.0 mg, 0.004 mmol, 0.10 mol %), ligand **3** (2.0 mg, 0.007 mmol, 0.15 mol %), 4-bromoacetophenone (890 mg, 4.47 mmol), *o*-cresol (0.55 mL, 5.33 mmol), potassium phosphate (1.90 g, 8.95 mmol) in toluene (13 mL) for 24 h. 955 mg (95%) of the title compound was obtained as a white solid, mp 34.5-35.5 ˚C (lit.[35] oil).

**4-(4'-*tert*-Butylphenoxy)acetophenone (Table 1, entry 3).**[30] General procedure A (workup method 2, ligand **3**; not according to the invention) was used to convert 4-bromoacetophenone and 4-tert-butylphenol in 15 h to 247 mg (92%) of the title compound, which was obtained as a colorless oil.

**Methyl 4-phenoxybenzoate (Table 1, entry 4).**[32] General procedure A (workup method 2, ligand **3**; not according to the invention) was used to convert methyl 4-bromobenzoate and phenol in 24 h to 201 mg (88%) of the title compound, which was obtained as a white solid, mp 59.5-60 ˚C (lit.[32] mp 62.5-63 ˚C).

***N,N*-Diethyl-4-(2'-methylphenoxy)benzamide (Table 1, entry 5)**. General procedure B (workup method 2, ligand **3**; not according to the invention) except that palladium acetate (11 mg, 0.05 mmol, 5.0 mol %) and **3** (22 mg, 0.075 mmol, 7.5 mol %) were employed to convert 4-bromo-*N,N*-diethylbenzamide and *o*-cresol in 22 h to 230 mg (81%) of the title compound, which was obtained as a colorless oil. $^1H$ NMR ($CDCl_3$, 300 MHz) δ 7.35-7.30 (m, 2H), 7.27-7.25 (m, 1H), 7.19 (td, 1H, *J*=8.0, 1.9 Hz), 7.10 (td, 1H, *J*=7.2, 1.2 Hz), 6.94 (dd, 1H, *J*=8.0, 1.2 Hz), 6.90-6.85 (m, 2H), 3.41 (broad s, 4H), 2.21 (s, 3H), 1.18 (broad s, 6H); $^{13}C$ NMR ($CDCl_3$, 75 MHz) δ 171.0, 158.9, 153.8, 131.7, 131.1, 130.4, 128.4, 127.4, 124.7, 120.5, 116.7, 43.0, 39.0, 16.4, 13.0; IR (neat, $cm^{-1}$) 1627,1584, 1424, 1237; Anal. Calcd for $C_{18}H_{21}NO_2$: C, 76.28; H, 7.47. Found: C, 76.11; H, 7.42.

**4-(3'-Isopropylphenoxy)benzonitrile (Table 1, entry 6).** General procedure A (workup method 2, ligand **3**; not according to the invention) was used to convert *p*-chloro benzonitrile and *m*-isopropylphenol in 24 h to 218 mg (91%) of the title compound, which was obtained as a pale yellow oil. $^1H$ NMR ($CDCl_3$, 300 MHz) δ 7.61-7.57 (m, 2H), 7.32 (t, 1H, *J*=7.8 Hz), 7.11 (d, 1H, *J*=7.8 Hz), 7.02-6.97 (m, 2H), 6.95-6.93 (m, 1H), 6.87 (ddd, 1H, *J*=8.1, 2.4, 0.9 Hz), 2.92 (hept, 114, *J*=6.6 Hz), 1.25 (d, 6H, *J*=6.6 Hz); $^{13}C$ NMR ($CDCl_3$, 75 MHz) δ 161.7, 154.6, 151.6, 134.0, 129.9, 123.3, 118.7, 118.4, 117.7, 117.6, 105.5, 34.0, 23.9; IR (neat, $cm^{-1}$) 2962, 2227, 1245; Anal. Calcd for $C_{16}H_{15}O$: C, 80.98; H,

6.37. Found: C, 80.93 H, 6.64.

**4-Chloro-2'-isopropyldiphenyl ether (Table 1, entry 7).** General procedure A (workup method 1, ligand **3**; not according to the invention) was used to convert 4-cholorobromobenzene and *o*-isopropylphenol (260 μL, 1.95 mmol) in 24 h to 223 mg (90%) of the title compound, which was obtained as a colorless oil. $^1H$ NMR ($CDCl_3$, 300 MHz) δ 7.38-7.33 (m, 1H), 728-7.23 (m, 2H), 7.20-7.12 (m, 2H), 6.90-6.82 (m, 3H), 3.25 (hept, 1H, *J*=6.9 Hz), 1.22 (d, 6H, *J*=6.9 Hz); $^{13}C$ NMR ($CDCl_3$, 75 MHz) δ 156.9, 153.0, 140:2, 129.5, 127.15, 127.05, 126.9, 124.5, 119.8, 118.5, 27.1, 23.0; IR (neat, $cm^{-1}$) 2964, 1482,1092; Anal. Calcd for $C_{15}H_{15}ClO$: C, 73.02; H, 6.13. Found: C, 73.00 H, 5.86.

**4-(2'-Methylphenyloxy)acetophenone (Table 1, entry 8).**[35] General procedure A (workup method 2, ligand **3**; not according to the invention) was used to convert 4-acetylphenyl triflate and *o*-cresol in 14 h to 188 mg (83%) of the title compound as a white solid, mp 35-36 °C (lit.[35] oil). See data above (Table 1, entry 2).

**3-(2'-Methylphenoxy)acetophenone (Table 1, entry 9).** General procedure A (workup method 2, ligand **3**; not according to the invention) was used to convert 3'-bromoacetophenone and *o*-cresol in 19 h to 170 mg (75%) of the title compound, which was obtained as a colorless oil. $^1H$ NMR ($CDCl_3$, 300 MHz) δ 7.63 (ddd, 1H, *J*=8.0, 1.5, 1.0 Hz), 7.49 (dd, 1H, *J*=2.6, 1.5 Hz), 7.39 (t, 1H, *J*=8.0 Hz), 7.27 (broad d, 1H, *J*=7.3 Hz), 7.19 (dt, 1H, *J*=7.3, 1.5 Hz), 7.11 (dd, 1H, *J*=8.0, 1.5 Hz), 7.09 (ddd, 1H, *J*=8.0, 2.6, 1.0 Hz), 6.91 (dd, 1H, *J*=8.0, 1.5 Hz), 2.57 (s, 3H), 2.23 (s, 3H); $^{13}C$ NMR ($CDCl_3$, 125 MHz) δ 197.8, 158.5, 154.0, 139.0, 131.9, 130.3, 130.1, 127.6, 124.8, 122.5, 121.9, 120.1, 116.8, 27.0, 16.4; IR (neat, $cm^{-1}$) 1686, 1578, 1437, 1264; Anal. Calcd for $C_{15}H_{14}O_2$: C, 79.62; H, 6.24. Found: C, 80.02; H, 6.28.

**2,2',5-Trimethyldiphenyl ether (Table 2, entry 1).**[4] General procedure B (workup method 1, ligand **4**; according to the invention) was used to convert 5-bromo-*p*-xylene and *o*-cresol in 24 h to 206 mg (96%) of the title compound which was obtained as a colorless oil.

**3,5-Dimethyldiphenyl ether (Table 2, entry 2).**[36] General procedure B (workup method 1, ligand **5**; not according to the invention) was used to convert 5-bromo-*m*-xylene and phenol in 24 h to 164 mg (83%) of the title compound, which was obtained as a colorless oil. $^1H$ NMR ($CDCl_3$, 300 MHz) δ 7.35-7.30 (m, 2H), 7.11-6.98 (m, 3H), 6.74 (broad s, 1H), 6.63 (broad s, 2H), 2.28 (broad s, 6H); $^{13}C$ NMR ($CDCl_3$,75 MHz) δ 157.3, 157.0, 139.4, 129.5, 124.9, 122.8, 118.7, 116.5, 21.3; IR (neat, $cm^{-1}$) 2919, 1584, 1490, 1218; Anal. Calcd for $C_{14}H_{14}O$: C, 84.81; H, 7.12. Found: C, 84.78; H, 6.94.

**2,3',5',6-Tetramethyldiphenyl ether (Table 2, entry 3).**[4] General procedure B (workup method 1, ligand **3**; not according to the invention) was used to convert 5-bromo-*m*-xylene and 2,6-dimethylphenol in 24 h to 157 mg (70%) of the title compound, which was obtained as a colorless oil. $^1H$ NMR ($CDCl_3$, 300 MHz) δ 7.12-7.03 (m, 3H), 6.62 (broad s, 1H), 6.37 (broad s, 2H), 2.24(s, 6H), 2.13 (s, 6H); $^{13}C$ NMR ($CDCl_3$, 125 MHz) δ 157.8, 151.1, 139.4, 131.5, 128.8, 124.8, 123.1, 112.2, 21.4, 16.4; IR (neat, $cm^{-1}$) 2921, 1600, 1194; Anal. Calcd for $C_{16}H_{18}O$: C, 84.91; H, 8.02. Found: C, 84.68; H, 8.18.

**3,3',4',5-Tetramethyldiphenyl ether (Table 2, entry 4)**.[4] General procedure B (workup method 1, ligand **3**; not according to the invention) was used to convert 5-bromo-*m*-xylene and 3,4-dimethylphenol in 24 h to 188 mg (84%) of the title compound, which was obtained as a colorless oil.

**4-*tert*-Butyl-2'-methyldiphenyl ether (Table 2, entry 5).** General procedure A (workup method 1, ligand **3**; not according to the invention) was used to convert 4-*t*-butylbromobenzene and *o*-cresol in 14 h to 204 mg (85%) of the title compound, which was obtained as a colorless oil. $^1H$ NMR ($CDCl_3$, 300 MHz) δ 7.32 (d, 2H, *J*=9.0 Hz), 7.25 (dd, 1H, *J*=8.1, 1.5 Hz), 7:15 (dt, 1H, *J*=7.5,1.5 Hz), 7.04 (dt, 1H, *J*=7.5, 1.2 Hz), 6.89 (dd, 1H, *J*=8.1, 1.2 Hz), 6.84 (d, 2H, *J*=9.0 Hz), 2.26 (s, 3H), 1.31 (s, 9H); $^{13}C$ NMR ($CDCl_3$, 125 MHz) δ 155.6, 155.1, 145.4, 131.5, 130.0, 127.2; 126.6, 123.8, 119.6, 117.2, 34.4, 31.7, 16.4; IR (neat, $cm^{-1}$) 2929; 1586, 1237; Anal. Calcd for $C_{17}H_{20}O$: C, 84.95; H, 8.39. Found: C, 84.84; H, 8.72.

**4-*n*-Butyl-2'-isopropyldiphenyl ether (Table 2, entry 6)**. General procedure A (workup method 1, ligand **3**; not according to the invention) was used to convert 1-bromo-4-*n*-butylbenzene and 2-isopropylphenol in 24 h to 246 mg (92%) of the title compound, which was obtained as a colorless oil. 1H NMR ($CDCl_3$, 300 MHz) δ 7.34-7.31 (dd, 1H, *J*=7.0, 2.4 Hz), 7.13-7.06 (m, 4H), 6.86-6.82 (m, 3H), 3.31 (hept, 1H, *J*=7.0 Hz), 2.57 (t, 2H, *J*=7.7 Hz), 1.63-1.53 (m, 2H), 1.42-1.29 (m, 2H), 1.23 (d, 6H, *J*=7.0 Hz), 0.93 (t, 3H, *J*=7.3 Hz); $^{13}C$ NMR ($CDCl_3$, 75 MHz) δ 156.0, 154.0, 140.0, 136.9, 129.4, 126.7, 126.6, 123.7, 119.2, 117.6, 34.9, 33.9, 27.1, 23.0, 22.4, 14.0; IR (neat, $cm^{-1}$) 2960, 1505, 1486, 1231; Anal. Calcd for $C_{19}H_{24}O$: C, 85.03; H, 9.01. Found: C, 84.88; H, 9.06.

**2-Isopropyl-4'-methoxydiphenyl ether (Table 2, entry 7).** General procedure A (workup method 1, ligand **6**; not according to the invention) was used to convert p-bromoanisole and *o*-isopropylphenol in 24 h to 213 mg (88%) of the title compound, which was obtained as a colorless oil. $^1H$ NMR ($CDCl_3$, 300 MHz) δ 7.32 (dd, 1H, *J*=6.9,2.1), 7.15-7.04 (m, 2H), 6.94-6.84 (m, 4H), 6.79 (dd, 1H, *J*=7.5, 2.1 Hz), 3.80 (s, 3H), 3.37 (hept, 1H, *J*=6.6 Hz), 1.26 (d, 6H *J*=6.6 Hz); $^{13}C$ NMR ($CDCl_3$, 75 MHz) δ 155.1, 154.8, 151.3, 139.2, 126.7, 126.6, 123.2, 119.4, 118.1, 114.7, 55.7, 27.1, 23.0; IR (neat, $cm^{-1}$) 2962, 1503, 1036; Anal. Calcd for $C_{16}H_{18}O_2$: C, 79.31; H, 7.49. Found: C, 79.39; H, 7.30.

**2,5-Dimethyldiphenyl ether (Table 2, entry 8).**[37] General procedure B (workup method 1, ligand **4**; according to the invention) was used to convert 2-chloro-*p*-xylene and phenol in 19 h to 157 mg (79%) of the title compound, which was obtained as a colorless oil. $^1H$ NMR ($CDCl_3$, 300 MHz) δ 7.31 (dd, 2H, *J*=7.5, 8.6 Hz), 7.14 (d, 1H, *J*=7.7 Hz), 7.05 (t, 1H, *J*=7.5 Hz), 6.93-6.89 (m, 1H), 6.91 (d, 2H, *J*=8.6 Hz), 6.76 (s, 1H), 2.29 (s, 3H), 2.21 (s, 3H); $^{13}C$ NMR ($CDCl_3$, 125

MHz) δ 158.2, 154.3, 137.3, 131.3, 129.8, 127.0, 125.0, 122.4, 120.7, 117.4, 21.2, 16.0; IR (neat, $cm^{-1}$) 2923, 1590, 1490; 1252, 1216, 1117; Anal Calcd for $C_{14}H_{14}O$: C, 84.81; H, 7.12. Found: C, 85.02; H, 7.14.

**2,5-Dimethyl-4'-methoxydiphenyl ether (Table 2, entry 9).** An oven-dried resealable Schlenk tube was charged with the sodium salt of *p*-methoxyphenol (88 mg, 0.6 mmol) in a nitrogen-filled glovebox. The tube was sealed and removed from the glovebox, then fitted with a septum and purged with argon. The septum was removed, then palladium acetate (2.2 mg, 0.02 mmol, 2.0 mol %) and **4** (according to the invention; 6.6 mg, 3.0 mol %) were added. The tube was capped with a septum and purged with argon. Toluene (1.5 mL) and 2-chloro-*p*-xylene (67 μL, 0.5 mmol) were added *via* syringe, then the tube was sealed with a teflon screwcap and the reaction mixture was stirred at 110 °C for 24 h. The reaction mixture was allowed to cool to room temperature and was then diluted with ether (20 mL), filtered and concentrated. The crude material was purified by flash chromatography on silica, gel to afford 113 mg (99%) of the title compound as an off-white solid, mp 55-56 °C. $^1$H NMR ($CDCl_3$, 300 MHz) δ 7.11 (broad d, 1H, *J*=7.7 Hz), 6.91-6.81 (m, 5H), 6.62 (broad s, 1H), 3.80 (s, 3H), 2.25 (s, 3H), 2.23 (s, 3H); $^{13}$C NMR ($CDCl_3$, 75 MHz) δ 155.5, 155.1, 151.2, 136.9, 131.0, 125.8, 123.8, 119.2, 118.7, 114.7, 55.6, 21.0, 15.8; IR (neat, $cm^{-1}$) 2923, 1501, 1030; Anal. Calcd for $C_{15}H_{16}O_2$: C, 78.92; H, 7.06. Found: C, 79.01; H, 7.24.

**2,2',5,6'-Tetramethyldiphenyl ether (Table 2, entry 10).** An oven-dried resealable Schlenk tube was charged with sodium 2,6-dimethylphenolate (89 mg, 0.62 mmol) in a nitrogen-filled glovebox. The tube was sealed and removed from the glovebox, then fitted with a septum and purged with argon. The septum was removed, then $Pd_2(dba)_3$ (5.9 mg, 0.0125 mmol, 2.5 mol % Pd) and 4 (according to the invention; 8.5 mg, 3.75 mol %) were added. The tube was capped with a septum and purged with argon. Toluene (1.5 mL) and 2-chloro-*p*-xylene (70 μL, 0.515 mmol) were added via syringe, then the tube was sealed with a teflon screwcap and placed in a 115 °C oil bath and stirred for 24 h. The reaction mixture was allowed to cool to room temperature and was then diluted with ether (20 mL), filtered and concentrated. The crude material was purified by flash chromatography on silica gel to afford 97 mg .(83%) of the title compound as a colorless oil. $^1$H NMR ($CDCl_3$, 500 MHz) δ 7.15-7.07 (m, 4H), 6.72 (broad d, 1H, *J*=7.5 Hz), 6.10 (broad s, 1H), 2.42 (s, 3H), 2.18 (s, 3H), 2.15 (s, 6H); $^{13}$C NMR ($CDCl_3$, 125 MHz) δ 155.5, 151.5, 136.6, 131.4, 130.7, 128.9, 124.7, 122.8, 121.6, 112.4, 21.2, 16.2, 15.9; IR (neat, $cm^{-1}$) 2923, 1507, 1192; Anal. Calcd for $C_{16}H_{18}O$: C, 84.91; H, 8.02. Found: C, 84.99; H, 8.16.

**4-Methoxy-2'-methyldiphenyl ether (Table 2, entry 11).**[38] General procedure A (workup 1, ligand **6**; not according to the invention) was used to convert 4-chloroanisole and *o*-cresol in 26 h to 156 mg (73%) of the title compound, which was obtained as a colorless oil. $^1$H NMR ($CDCl_3$, 300 MHz) δ 7.23 (broad d, 1H, *J*=7.2 Hz), 7.12 (broad t, 1H, *J*=7.8 Hz), 7.00 (broad t, 1H, *J*=7.5 Hz), 6.92-6.83 (m, 4H), 6.79 (broad d, 1H, *J*=8.1 Hz), 3.89 (s, 3H), 2.23 (s, 3H); $^{13}$C NMR ($CDCl_3$, 125 MHz) δ 155.8, 155.2, 151.0, 131.3, 129.0, 126.9, 123.0, 119.3, 118.0, 114.8, 55.7, 16.2; IR (neat, $cm^{-1}$) 2952, 1503, 1225; Anal. Calcd for $C_{14}H_{14}O_2$: C, 78.48; H, 6.59. Found: C, 78.43; H, 6.28.

**4-*n*-Butyl-3',4'-dimethyldiphenyl ether (Table 2, entry 12).** General procedure B (workup method 1, ligand **5**; not according to the invention) was used to convert 1-chloro-4-*n*-butylbenzene and 3,4-dimethylphenol in 22 h to 201 mg (79%) of the title compound, which was obtained as a colorless oil. $^1$H NMR ($CDCl_3$, 300 MHz) δ 7.14-7.03 (m, 3H), 6.92-6.86 (m, 2H), 6.80 (broad d, 1H, *J*=2.4 Hz), 6.80 (broad dd, 1H, *J*=8.4, 2.4 Hz), 2.57 (app t, 2H, *J*=7.8 Hz), 2.22 (s, 6H), 1.66-1.52 (m, 2H), 1.35 (sext, 2H, *J*=7.8 Hz), 0.92 (t, 3H, *J*=7.6 Hz); $^{13}$C NMR ($CDCl_3$, 125 MHz) δ 155.5, 155.3, 138.1, 137:4, 131.2, 130.5, 129.4, 120.1, 118.4, 116.0, 34.9, 33.8, 22.3, 19.9, 19.0, 14.0; IR (neat, $cm^{-1}$) 2927, 1495, 1218; Anal. Calcd for $C_{18}H_{22}O$: C, 84.99; H, 8.72. Found: C, 85.27; H, 8.99.

**2-Isopropyl-4'-*t*-butyldiphenyl ether (Table 2, entry 13).** General procedure A (workup method 1, ligand **3**; not according to the invention) was used to convert 4-*t*-butylphenyl triflate and *o*-isopropylphenol in 24 h to 230 mg (86%) of the title compound, which was obtained as a colorless oil. $^1$H NMR ($CDCl_3$, 300 MHz) δ 7.36-7.30 (m, 1H), 7.33 (d, 2H, *J*=8.6 Hz), 7.18-7.08 (m, 2H), 6.90-6.85 (m, 1H), 6.88 (d, 2H, *J*=8.8 Hz), 3.33 (hept, 1H, *J*=6.9 Hz), 1.33 (s, 9H), 1.25 (d, 6H, *J*=6.9 Hz); $^{13}$C NMR ($CDCl_3$, 125 MHz) δ 155.8, 154.0, 145.2, 140.0, 126.8, 126.7, 126.4, 123.8, 119.4, 117.2, 34.2, 31.5, 27.0, 23.0; IR (neat, $cm^{-1}$) 2962, 1509, 1486, 1233; Anal Calcd for $C_{19}H_{24}O$: C, 85.03; H, 9.01. Found: C, 84.82 H, 8.76.

**3-Methoxy-2'-methyldiphenyl ether (Table 2, entry 14)**.[39] General procedure A (workup method 1, ligand **3**; not according to the invention) was used to convert 3-bromoanisole and *o*-cresol in 19 h to 186 mg (87%) of the title compound, which was obtained as a colorless oil. $^1$H NMR ($CDCl_3$, 300 MHz) δ 7.26 (d, 1H, *J*=7.3 Hz), 7.22-7.16 (m, 2H), 7.08 (dt, 1H, *J*=7.3, 1.3 Hz), 6.95 (dd, 1H, *J*=8.0, 1.3 Hz), 6.61 (ddd, 1H, *J*=8.5, 2.4, 1.3 Hz), 6.50 (d, 1H, *J*=1.3 Hz), 6.48 (ddd, 1H, *J*=8.5, 2.4, 1.3 Hz), 3.78 (s, 3H), 2.25 (s 3H); $^{13}$C NMR ($CDCl_3$, 125 MHz) δ 161.1, 159.4, 154 4, 131.6, 130.3, 130.2, 127.4, 124.4, 120.3, 109.6, 108.0, 103.6, 55.5, 16.3; IR (neat, $cm^{-1}$) 1580, 1486, 1227; Anal. Calcd for $C_{14}H_{14}O_2$: C, 78.48; H, 6.59. Found: C, 78.56; H, 6.84.

**4-*n*-Butyidiphenyl ether (Table 2, entry 15).** General procedure B (workup method 2, ligand **5**; not according to the invention) was used, except that the quantities of phenol (2.0 mmol), sodium hydride (2.4 mmol), palladium acetate (9.0 mg, 0.04 mmol, 4.0 mol %), and ligand **5** (22 mg, 0.06 mmol, 6.0 mol %) were employed to convert 4-*n*-butyl-1-chlorobenzene and phenol in 24 h at 115 °C to 142 mg (63%) of the title compound, which was obtained as a colorless oil. $^1$H NMR ($CDCl_3$, 300 MHz) δ 7.33 (dd, 2H, *J*=8.7, 7.4 Hz), 7.15 (d, 2H, *J*=8.6 Hz), 7.08 (t, 1H, *J*=7.4 Hz), 7:00 (dd,

2H, *J*=8.7, 1.0 Hz), 6.34 (d, 2H, *J*=8.6 Hz), 2.60 (t, 2H, *J*=7.4 Hz), 1.61 (quint, 2H, *J*=7.4 Hz), 1.37 (sext, 2H, *J*=7.4 Hz), 0.95 (t, 3H, *J*=7.4 Hz); $^{13}$C NMR ($CDCl_3$, 125 MHz) δ 157.7, 154.8, 137.9, 129.6, 129.5, 122.8, 118.9, 118.4, 34.9, 33.8, 22.3, 14.0; IR (neat, $cm^{-1}$) 2929, 1590, 1488, 1235; Anal. Calcd for $C_{16}H_{18}O$: C, 84.91; H, 8.02. Found: C, 84.89 H, 7.88. **3,4',5-Trimethyldiphenyl ether (Table 2, entry 16).**[4] General procedure B (workup method 1, ligand **5**; not according to the invention) was used to convert 5-bromo-*m*-xylene and *p*-cresol in 24 h to 186 mg (88%) of the title product, which was obtained as a colorless oil.

*References and Notes for Example 143*

(1) For examples of medicinally important diaryl ethers, see: (a) Evans, D. A., DeVries, K. M. In *Glycopeptide Antibiotics, Drugs and the Pharmaceutical Sciences ;* Nagarajan, R., Ed.; Marcel Decker, Inc.: New York, 1994; Vol. 63, pp 63-104; (b) Deshpande, V. E.; Gohkhale, N. J. *Tetrahedron Lett.* **1992**, *33,* 4213-4216; (c) Singh; S. B.; Pettit, G. R. *J. Org. Chem.* **1990**, *55*, 2797-2800; (d) Pettit, G. R.; Singh, S. B.; Niven, M. L. *J. Am. Chem. Soc.* **1988**, *110,* 8539-8540; (e) Jung, M. E.; Rohloff, J. C. *J Org. Chem.* **1985**, *50*, 4909-4913; (f) Atkinson, D. C.; Godfrey, K. E.; Myers, P. L.; Philips, N. C.; Stillings, M. R.; Welbourn, A. P. *J. Med. Chem.* **1983**, *26*, 1361-1364; For examples of agriculturally important diaryl ethers, see: (g) Seldon, R. A. *Chirotechnology;* Marcel Dekker Inc.: New York, 1998; pp 62-65.

(2) (a) Ullmann, F. *Chem. Ber.* **1904**, *37*, 853-854; (b) Lindley, J. *Tetrahedron* **1984**, *40,* 1433-1456; (c) Moroz, A. A.; Shvartsberg, M. S. *Russ. Chem. Rev.* **1974**, *43*, 679-689.

(3) For examples of recent work in diaryl ether formation, see: (a) Evans, D. A.; Katz, J. L.; West, T. R. *Tetrahedron Lett.* **1998**, 2937-2940; (b) Sawyer, J. S.; Schmittling, E. A.; Palkowitz, J. A.; Smith, W. J. *J Org. Chem.* **1998**, *63*, 6338-6343; (c) . Janetka, J. W.; Rich, D. H. *J. Am. Chem. Soc.* **1997**, *119*, 6488-6495; (d) Palomo, C.; Oairbide, M.; López, R.; Gómez-Bengoa, E. *J. Chem. Soc., Chem. Commun.* **1998**, *19,* 2091-2092; (e) Beugelmans, R.; Zhu, J.; Husson, N.; Bois-Choussy, M.; Singh, G. P. *J Chem. Soc., Chem. Commun.* 1994, 439-440.

(4) Marcoux, J. -F.; Doye, S.; Buchwald, S. L. *J. Am. Chem. Soc.* **1997,** *119,* 10539-10540.

(5) For reviews, see: (a) Wolfe, J. P.; Wagaw, S.; Marcoux, J.-F.; Buchwald, S. L. *Acc. Chem. Res.* **1998**, *31*, 805-818; (b) Hartwig, J. F. *Angew. Chem. Int. Ed. Engl.* **1998**, *37*, 2046-2067; (c) Hartwig, J. F. *Synlett* **1997**, 329-340; (d) Hartwig, J. F. *Acc. Chem. Res.* **1998**, *31*, 852-860; (e) Yang, B. H.; Buchwald, S. L. *J. Organomet. Chem.* In press.

(6) (a) Palucki, M.; Wolfe, J. P.; Buchwald, S. L. *J. Am. Chem. Soc.* **1996**, *118,* 10333-10334; (b) Palucki, M.; Wolfe, J. P.; Buchwald, S. L. *J. Am. Chem. Soc.* **1997**, *119,* 3395-3396; (c) Mann, G.; Hartwig, J. F. *J. Org. Chem.* **1997**, *67,* 5413-5418; (d) Mann, G.; Hartwig, J. F. *J. Am. Chem. Soc.* **1996,** *118,* 13109-13110.

(7) (a) Mann, G.; Hartwig, J. F. *Tetrahedron Lett.* **1997**, *38,* 8005-8008; (b) Doye, S.; Buchwald, S. L. Unpublished Results.

(8) For other examples of bulky, electron-rich ligands for Pd-catalyzed carbon-heteroatom bond forming reactions see: (a) Nishiyama, M.; Yamamoto, T.; Koie, Y. *Tetrahedron Lett.* **1998**, *39*, 617-620; (b) Yamamoto, M.; Nishiyama, M.; Koie, Y. *Tetrahedron Lett.* **1998,** *39,* 2367-2370; (c) Reddy, N. P.; Tanaka, M. *Tetrahedron Lett.* **1998,** *39,* 617-620; (d) Hamann, B. C.; Hartwig, J. F. *J. Am. Chem. Soc.* **1998,** *120,* 7369-7370; (e) Old, D. W.; Wolfe, J. P.; Buchwald, S. L. *J. Am. Chem. Soc.* **1998,** *120,* 9722-9723.

(9) Typically, oxidative addition of aryl chlorides to Pd(0) requires temperatures of 60-140 ˚C. (a) Grushin, V. V.; Alper, H. *Chem. Rev.* **1994,** *94,* 1047-1062; (b) Herrmann, W. A.; Broßmer, C.; Priermeier, T.; Öfele, K. *J. Organomet. Chem.* **1994**, *481*, 97-108; (c) Parshall, G. W. *J. Am. Chem. Soc.* **1974,** *96,* 2360-2366; (d) Huser, M.; Youinou, M. -T.; Osborn, J. A. *Angew. Chem. Int. Ed. Engl.* **1989**, *28*, 1386-1388.

(10) (a) It is well known that the use of electron-rich phosphine ligands accelerates the rate of oxidative addition of aryl halides to Pd(0). See: Spessard, G. O.; Meissler, G. L. *Organometallic Chemistry* Prentice-Hall: Upper Saddle River, New Jersey, 1996; pp 171-175. (b) In his pioneering studies, Milstein demonstrated oxidative addition of aryl chlorides to $Pd(dippp)_2$ (dippp=1,3-bis(diisopropylphosphino)propane) at 38 ˚C. Portnoy, M.; Milstein, D. *Organo-*

*metallics* **1993**, *12*, 1665-1673.

(11) (a) Bryndza, H. E.; Tam, W. *Chem. Rev.* **1988,** *88,* 1163-1188; (b) Widenhoefer, R. A.; Zhong, H. A.; Buchwald, S. L. *J. Am. Chem. Soc.* **1997,** *119,* 6787-6795; (c) Widenhoefer, R. A.; Buchwald, S. L. *J. Am. Chem. Soc.* **1998,** *120,* 6504-6511; (d) Hillhouse has reported C-O bond forming reductive elimination from nickel complexes. Han, R.; Hillhouse, G. L. *J. Am. Chem. Soc.* **1997,** *119,* 8135-8136.

(12) (a) Jones, W. D.; Kuykendall, V. L. *Inorg. Chem.* **1991,** *30*, 2615-2622; (b) Hartwig, J. F.; Richards, S.; Baranano, D.; Paul, F. *J. Am. Chem. Soc.* **1996,** *118,* 3626-3633.

(13) We have previously speculated that improved results in Pd-catalyzed aryl C-O bond forming reactions might be obtained with the use of bulky, electron-deficient, chelating phosphine ligands; see ref 6b.

(14) Di-*t*-butylchlorophosphine is the bulkiest dialkylchlorophosphine which is commercially available.

(15) Schlosser, M. in *Organometallics in Synthesis*; Schlosser, M., Ed.; John Wiley and Sons: Chinchester, 1994; pp 129-133.

(16) Premixing of sodium hydride and the phenol is required to avoid palladium-catalyzed hyride reduction of the aryl halide; Pd-catalyzed reduction of vinyl triflates by LiH and KH has been previously observed: Scott, W. J.; Stille, J. K. *J. Am. Chem. Soc.* **1986**, *108,* 3033-3040.

(17) Previously[8e] we employed dicylclohexylphenylphosphine as a control reaction due the commercial availability of this compound.

(18) Control experiments were carried out in the absence of a palladium salt. For the reaction of 4-bromoacetophenone and phenol with potassium phosphate in toluene at 100 °C, none of the desired product was detected; in DMF at 100 °C, 32% (GC, corrected) of the starting halide was consumed and a 5% GC yield of the desired product was observed.

(19) An exception is the reaction of 2-bromobenzotrifluoride and *o*-cresol to provide the corresponding diaryl ether in 75% isolated yield; the reaction of 2-bromoacetophenone and *o*-cresol proceeded to 25% conversion (GC) with ligand 4, affording <20% of the desired product.

(20) Yields are typically 15-20% higher, and improved product / arene ratio is observed.

(21) (a) Oxidative addition is presumed to be facile based on the utility of **1** in Suzuki couplings at room-temperature; see ref 8e; (b) transmetallation of alkali metal alkoxides to $L_nPd(Ar)X$ complexes has been shown to occur at room temperature when L = BINAP or DPPF; see ref 6d, 11b-c.

(22) Bäckvall, J.E.; Björkman, E. E.; Petterson, L.; Siegbahn, P. *J. Am. Chem. Soc.* **1984**, *106,* 4369-4373.

(23) We cannot rule out a situation where not all of the palladium is ligated in the reaction mixture, and the reactions are proceeding through bis(phosphine) intermediates.

(24) (a) Hartwig, J. F.; Paul, F. *J. Am Chem Soc.* **1995**, *117,* 5373-5374; (b) Paul, F.; Patt, J.; Hartwig, J. F. *J. Am. Chem. Soc.* **1994**, *116*, 5969-5970; (c) Louie, J.; Hartwig, J. F. *J. Am. Chem. Soc.* **1995,** *117*, 11598-11599; (d) Driver, M. S.; Hartwig, J. F. *J. Am. Chem. Soc.* **1995,** *117*, 4708-4709; (e) Louie; J.; Paul, F.; Hartwig, J. F. *Organometallics* **1996**, *15*, 2794-2805.

(25) Hartwig has proposed a similar mechanism to account for electronic effects in C-S and C-N bond forming reductive elimination reactions. (a) Baranano, D.; Hartwig, J. F. *J. Am. Chem. Soc.* **1995,** *117***,** 2937-2938; (b) Driver, M. S.; Hartwig, J. F. *J. Am. Chem. Soc.* **1997**, *119,* 8232-8245.

(26) Brown, J. M.; Guiry, P. J. *Inorg. Chim. Acta.* **1994,** *220,* 249-259.

(27) Sadighi, J. P.; Harris, M. C.; Buchwald, S. L. *Tetrahedron Lett.* **1998,** *39*, 5327-5330.

(28) We thank Dr. Joseph Fox for this suggestion.

(29) Brandt, K. *J. Org. Chem.* **1981**, *46,* 1918-1920.

(30) Yeager, G. W.; Schissel, D. N. *Synthesis* **1991,** 63-68.

(31) This compound is also available from Aldrich Chemical Company.

(32) Haga, N., Takayanagi, H. *J. Org. Chem.* **1996,** *61,* 735-745.

(33) Kranenburg, M.; van der Burgt, Y. E. M.; Kamer, P. C. J.; van Leeuwen, P. W. N. M.; Goubitz, K.; Fraanje, J. *Organometallics* **1995**, *14,* 3081-3089.

(34) Molle, G.; Bauer, P.; Dubios, J. E. *J. Org. Chem.* **1982,** *47,* 4120-4128.

(35) Horii, Z.; Kiuchi, T. *J. Pharm. Soc. Japan* **1937,** *57,* 683-688; *Chem. Abstr.* **1938,** 129.

(36) Smith, K.; Jones, D. *J. Chem. Soc., Perkin Trans. 1* **1992***,* 407-408.

(37) Zeller, K.-P., Berger, S. *J. Chem. Soc., Perkin Trans. 2* **1977**, 54-58.

(38) Van Duzee, E. M.; Adkins, H. *J. Am. Chem. Soc.* **1935**, *57*, 147-150.

(39) Fujikawa, F.; Nakamura, I. *J. Pharm. Soc. Japan* **1944**, *64*, 274-276; *Chem. Abstr.* 1951, 2906.

***Example 144***

4-*tert*-Butyl *tert*-butyloxybenzene

A Schlenk tube containing 2-methyl-2'-di-*tert*-butylphosphinobiphenyl (not according to the invention; 3.7 mg, 0.012 mmol, 1.2 mol%), palladium(II) acetate (2.2 mg, 0.010 mmol, 1.0 mol%), sodium *tert*-butoxide (125 mg, 1.30 mmol, 1.3 equiv), dodecane (internal standard; 0.225 mL, 1.00 mmol, 1.0 equiv), 4-*tert*-butyl bromobenzene (0.175 mL, 1.00 mmol, 1 equiv), and toluene (2.0 mL) was sealed under argon and then placed into a 100 ˚C oil bath. After 17 h, the reaction mixture was cooled to room temperature. The reaction mixture was diluted with ether (6 mL) and was filtered through celite, rinsing with ether (2 x 5 mL). The combined organics were concentrated in vacuo. Purification of the crude oil by flash column chromatography (2% ethyl acetate-hexanes) afforded the product as a pale yellow oil (189 mg, 92%).

***Example 145***

2-*tert*-Butyloxy anisole

A Schlenk tube containing 2-methyl-2'-di-*tert*-butylphosphinobiphenyl (not according to the invention; 3.7 mg, 0.012 mmol, 1.2 mol%), palladium(II) acetate (2.2 mg, 0.010 mmol, 1.0 mol%), sodium *tert*-butoxide (125 mg, 1.30 mmol, 1.3 equiv), dodecane (internal standard; 0.225 mL, 1.00 mmol, 1.0 equiv), 2-chloro anisole (0.120 mL, 1.00 mmol, 1 equiv), and toluene (2.0 mL) was sealed under argon and then placed into a 100 ˚C oil bath. After 19 h, the reaction mixture was cooled to room temperature. The reaction mixture was diluted with ether (6 mL) and was filtered through celite, rinsing with ether (2 x 5 mL). The combined organics were concentrated in vacuo. Purification of the crude oil by flash column chromatography (2% ethyl acetate-hexanes) afforded the product as a colorless oil (152 mg, 85%).

***Example 146***

4-*tert*-Butyl *tert*-butyldimethylsilyloxybenzene

An oven-dried test tube (16 x 100 mm) containing 2-(*N,N*-dimethylamino)-2'-di-*tert*-butylphosphinobiphenyl (not according to the invention; 3.4 mg, 0.010 mmol, 6.2 mol%), $Pd_2(dba)_3$ (3.7 mg, 0.040 mmol, 2.5 mol%), sodium *tert*-butyldimethylsilyloxide (30.0 mg, 0.194 mmol, 1.2 equiv), 4-*tert*-butyl bromobenzene (0.028 mL, 0.162 mmol, 1 equiv), and toluene (1.5 mL) was sealed under argon with a septum and then placed into a 120 ˚C oil bath. After 19.5

h. the reaction mixture was cooled to room temperature. The reaction mixture was diluted with ether (6 mL) and was filtered through celite, rinsing with ether (2 x 5 mL). The combined organics were concentrated in vacuo. Purification of the crude oil by flash column chromatography (2% ethyl acetate-hexanes) afforded the product as a colorless oil (35 mg, 81%).

***Example 147***

2-*n*-Butyloxy-*m*-xylene

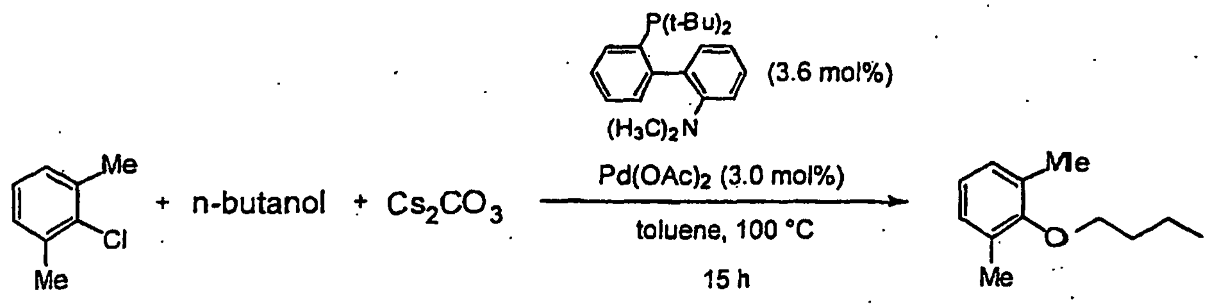


To a Schlenk tube containing 2-(*N,N*-dimethylamino)-2'-di-*tert*-butylphosphinobiphenyl (not according to the invention; 9.2 mg, 0.027 mmol, 3.6 mol%), palladium(II) acetate (5.1 mg, 0.022.mmol, 3.0 mol%), and cesium carbonate (365 mg, 1.12 mmol, 1.5 equiv) was added toluene (1.5 mL), dodecane (internal standard; 0.170 mL, 0.750 mmol, 1.0 equiv), 2-chloro-*m*-xylene (0.099 mL, 0.75 mmol, 1 equiv), and n-butanol (0.170 mL, 1.88 mmol, 2.5 equiv). The reaction flask was sealed under argon and was placed into a 100°C oil bath. After 15 h, the flask was removed from the oil bath and was cooled to room temperature. Analysis of an aliquot from the reaction mixture by GC showed that the reaction had proceeded to completion and indicated an uncorrected ratio of 3.1:1 for the desired product to *m*-xylene. The GC yield of the title product was 63%.

***Example 148***

4-Isopropyloxybenzonitrile

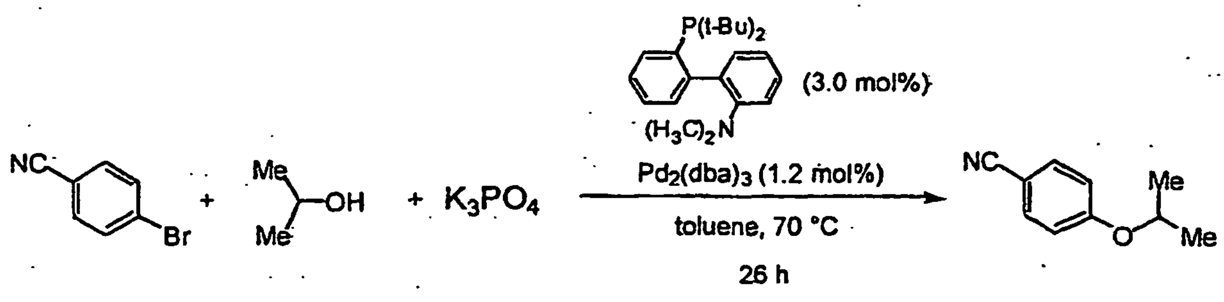


To a Schlenk tube containing 2-(*N,N*-dimethylamino)-2'-di-*tert*-butylphosphinobiphenyl (not according to the invention; 7.7 mg, 0.022 mmol, 3.0 mol%), $Pd_2(dba)_3$ (8.6 mg, 0.0094 mmol, 1.2 mol%), and potassium phosphate (239 mg, 1.12 mmol, 1.5 equiv) was added toluene (1.5 mL), dodecane (internal standard; 0.170 mL, 0.750 mmol, 1.0 equiv), 4-bromobenzonitrile (137 mg, 0.750 mmol, 1 equiv), and isopropanol (0.075 mL, 0.98 mmol, 1.3 equiv). The reaction flask was sealed under argon and was placed into a 70 °C oil bath. After 26 h, the flask was removed from the oil bath and was cooled to room temperature. The reaction mixture was poured into water (40 mL) and the organics were extracted with ether (3 x 35 mL). The combined organic layers were dried over sodium sulfate and were concentrated in vacuo. Purification of the crude residue by flash chromatography (5% ethyl acetate-hexanes) provided the title product as a colorless oil (47.6 mg, 39%).

***Example 149***

Synthesis of 2.5-Dimethylphenyl phenyl ether via Cross-coupling of 2-chloro-p-xylene with phenol

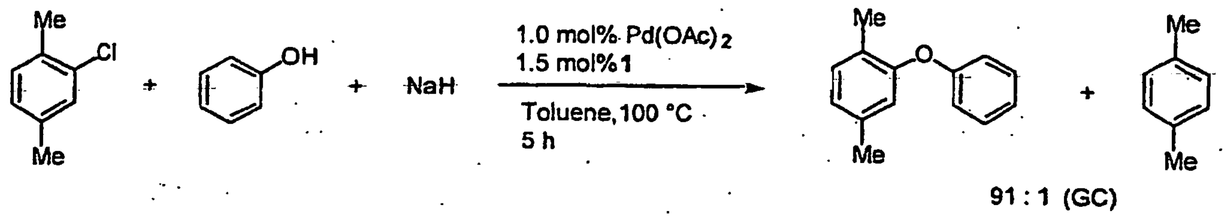


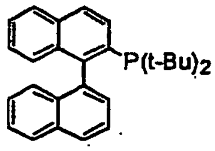

1

An oven-dried resealable Schlenk flask was charged with NaH (95%, 36 mg, 1.4 mmol) in a nitrogen-filled glovebox. The tube was removed from the glovebox and toluene (2 mL) and phenol (113 mg, 1.2 mmol) were added. The mixture was heated to 100 ˚C for 20 minutes under an argon atmosphere, then cooled to room-temperature. The flask was charged with $Pd(OAc)_2$ (2.3 mg, 0.01 mmol, 1 mol %), ligand **1** (not according to the invention; 6.1 mg, 0.015 mmol) and purged with argon. 2-chloro-p-xylene (0.135 mL, 1.0 mmol) was added through a rubber septum and the tube was sealed with a teflon screwcap. The mixture was heated to 100 ˚C with stirring for 5h at which time GC analysis showed that the starting material had been completely consumed and the desired product had formed. The ratio of desired product/xylene was determined to be 91/1 by GC analysis.

***Example 150***

Room temperature Pd-catalyzed coupling of *p*-bromoacetophenone and *o*-cresol

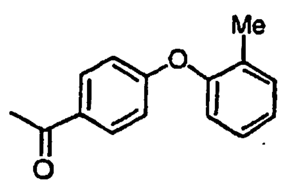

An oven-dried resealable Schlenk tube was fitted with a rubber septum and was cooled to room temperature under an argon purge. The septum was removed and the tube was charged with palladium acetate (5.3 mg, 0.024. mmol, 4.7 mol %), *o*-biphenyl-P(*t*-Bu)$_2$ (not according to the invention; 10.5 mg, 7 mol%), potassium phosphate (224.5 mg 0.94 mmol) and *p*-bromoacetophenone (100 mg, 0.5 mmol): The tube was capped with the septum and purged with argon, then toluene (1-4 mL) and *o*-cresol (60 μL, 0.58 mmol) was added through the septum. The tube was sealed with a teflon screwcap, and the reaction mixture was stirred at room temperature for 24 h. The reaction mixture was then diluted with ether (40 mL), filtered and concentrated. The crude material was purified by flash chromatography on silica gel to afford 108 mg of the title compound (95% yield).

***Example 151***

Room temperature Pd-catalyzed coupling of 4-bromobenzaldehyde and *o*-cresol

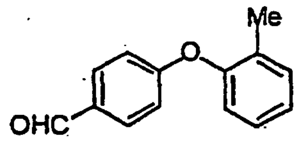

An oven-dried resealable Schlenk tube was fitted with a rubber septum and was cooled to room temperature under an argon purge. The septum was removed and the tube was charged with palladium acetate (5.5 mg, 0.025 mmol, 5 mol %), *o*-biphenyl-P(*t*-BU)$_2$ (not according to the invention; 11.0 mg, 7.6 mol%), potassium phosphate (224.5 mg 0.94 mmol) and 4-bromobenzaldehyde (90 mg, 0.5 mmol). The tube was capped with the septum and purged with argon, then toluene (1.5 mL) and *o*-cresol (60 μL, 0.58 mmol) was added through the septum. The tube was sealed with a teflon screwcap, and the reaction mixture was stirred at room temperature for 24 h. The reaction mixture was then diluted with ether (20 mL). The title compound was detected in 91% GC-yield.

***Example 152***

Room temperature Pd-catalyzed coupling of methyl 4-bromobenzoate and *o*-cresol

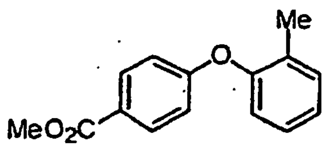

An oven-dried resealable Schlenk tube was fitted with a rubber septum and was cooled to room temperature under an argon purge. The septum was removed and the tube was charged with palladium acetate (5.2 mg, 0.025 mmol, 5 mol %), *o*-biphenyl-P(*t*-Bu)$_2$ (not according to the invention; 10.4 mg, 7.5 mol%), potassium phosphate (212.3 mg 0.91 mmol) and methyl 4-bromobenzoate (100 mg, 0.47 mmol). The tube was capped with the septum and purged with argon, then toluene (1.4 mL) and *o*-cresol (60 μL, 0.58 mmol) was added through the septum. The tube was sealed with a teflon screwcap, and the reaction mixture was stirred at room temperature for 24 h. The reaction mixture was then diluted with ether (20 mL). The title compound was detected in 91% GC-yield.

***Example 153***

Room temperature Pd-catalyzed coupling of 1-bromo-4-butylbenzene and *o*-cresol.

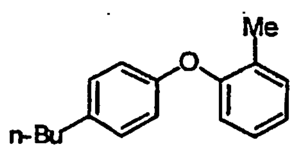

An oven-dried resealable Schlenk tube was fitted with a rubber septum and was cooled to room temperature under an argon purge. The septum was removed and the tube was charged with palladium acetate (5.7 mg, 0.025 mmol, 2.0 mol %), *o*-biphenyl-P(*t*-Bu)$_2$ (not according to the invention; 11.4 mg, 7.5 mol%), and potassium phosphate (224.5

mg 2.0 mmol). The tube was capped with the septum and purged with argon, then toluene (1.5 mL), 1-bromo-4-butylbenzene(0.51 mmol, 90 μl) and *o*-cresol (65 μl, 0.63 mmol) was added through the septum. The tube was sealed with a teflon screwcap, and the reaction mixture was stirred at room temperature for 24 h. Trace of the title product (<5%) was observed by GC-analysis.

***Example 154***

Room temperature Pd-catalyzed coupling of *p*-bromoacetophenone and 3,4-dimethylphenol

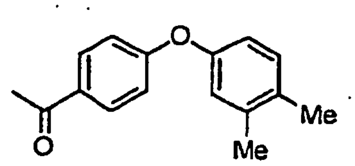

An oven-dried resealable Schlenk tube was fitted with a rubber septum and was cooled to room temperature under an argon purge. The septum was removed and the tube was charged with palladium acetate (5.6 mg, 0.025 mmol, 2.0 mol %), *o*-biphenyl-P(*t*-Bu)$_2$ (not according to the invention; 11.2 mg, 7.5 mol%), potassium phosphate (224.5 mg 2.0 mmol), *p*-bromoacetophenone (0.50 mmol, 100 mg) and 3,4 dimethylphenol (75 mg 0.61 mmol). The tube was capped with the septum and purged with argon, then toluene (1.5 mL) was added through the septum. The tube was sealed with a teflon screwcap, and the reaction mixture was stirred at room temperature for 24 h. The title product was observed by GC analysis in 11% GC yield.

***Example 155***

Synthesis of α-(4-(carboxyethyl)phenyl)-β-tetralone

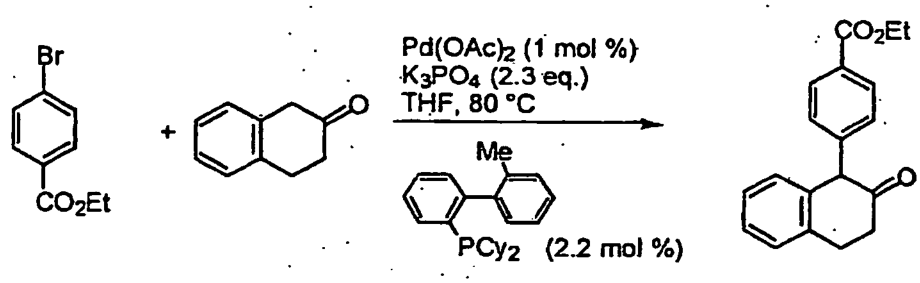


A dry Schlenk tube containing a stirbar was charged with palladium acetate (2.3 mg, 0.01 mmol), 2-dicyclohexylphosphino-2'-methylbiphenyl (not according to the invention; 8.0 mg, 0.022 mmol), and potassium phosphate (490 mg, 2.3 mmol). After a septum was placed on top of the tube, it was evacuated and refilled with argon three times. THF (1 mL), ethyl 4-bromobenzoate (229 mg, 0.163 mL, 1.0 mmol), and β-tetralone (175 mg, 0.150 mL, 1.2 mmol), were sequentially injected. Under a flow of argon, the septum was replaced with a teflon screwcap, and the tube sealed and heated with stirring in an oil bath at 80°C. After 21.5 h, the mixture was cooled to rt and analyzed by GC/MS. No ethyl 4-bromobenzoate was detected, and the only new product that was observed was α-(ethyl-4-carboxyphenyl)-β-tetralone.

***Example 156***

Synthesis of α-(4-(carboxyethyl)phenyl)-β-tetralone using potassium carbonate

A dry Schlenk tube containing a stirbar was charged with palladium acetate (2.3 mg, 0.01 mmol), 2-dicyclohexylphosphino-2'-methylbiphenyl (not according to the invention; 8.0 mg, 0.022 mmol), and $K_2CO_3$ (320 mg, 2.3 mmol). After a septum was placed on top of the tube, it was evacuated and refilled with argon three times. THF (1 mL), ethyl

4-bromobenzoate (229 mg, 0.163 mL, 1.0 mmol), and β-tetralone (175 mg, 0.150 mL, 1.2 mmol), were sequentially injected. Under a flow of argon, the septum was replaced with a teflon screwcap, and the tube sealed and heated with stirring in an oil bath at 80 ˚C. After 21.5 h, the mixture was cooled to rt and analyzed by GC/MS. No ethyl 4-bromobenzoate was detected, and the only new product that was observed was α-(ethyl 4-carboxyphenyl)-β-tetralone.

***Example 157***

Synthesis of α-(4-(carboxyethyl)phenyl)-β-tetralone using cesium carbonate

A dry Schlenk tube containing a stirbar was charged with palladium acetate (23 mg, 0.01 mmol), 2-dicyclohexylphosphino-2'-methylbiphenyl (not according to the invention; 8.0 mg, 0.022 mmol), and cesium carbonate (750 mg, 2.3 mmol). After a septum was placed on top of the tube, it was evacuated and refilled with argon three times. dioxane (1 mL), ethyl 4 bromobenzoate (229 mg, 0.163 mL, 1.0 mmol), and β-tetralone (175 mg, 0.150 mL, 1.2 mmol), were sequentially injected. Under a flow of argon, the septum was replaced with a teflon screwcap, and the tube sealed and heated with stirring in an oil bath at 80 ˚C. After 20 h, the mixture was cooled to rt and analyzed by GC/MS. No ethyl 4-bromobenzoate was detected; and the only new product that was observed was α-(ethyl 4-carboxyphenyl)-β-tetralone.

***Example 158***

2-Methyl-2-(*trans*-β-styryl)-5-(*N*-methyl-anilinomethylene)cyclopentanone ($R_1$ = Me; $R_2$ = Ph)

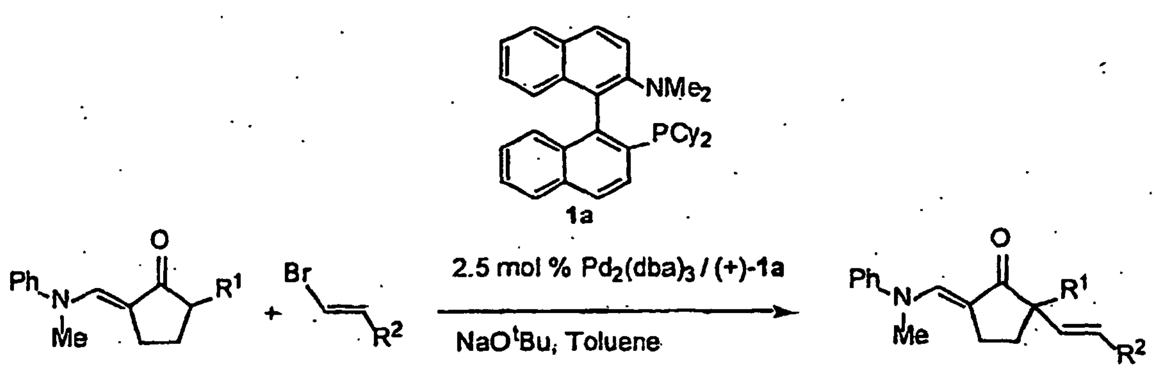


An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 13.6 mg, 0.028 mmol 5.5 mol%). The tube was capped with the septum, purged with: argon, and toluene (2 mL) was added through the septum. The mixture was stirred at room temperature for 10 min, then *trans*-β-bromostyrene (128 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 142 mg (89 %) of the title compound. The ee was determined to be 82 % by chiral HPLC analysis.

***Example 159***

2-*n*-Propyl-2-(*trans*-β-styryl)-5-(*N*-methyl-anilinomethylene) cyclopentanone ($R_1$ = n-Pr; $R_2$ = Ph)

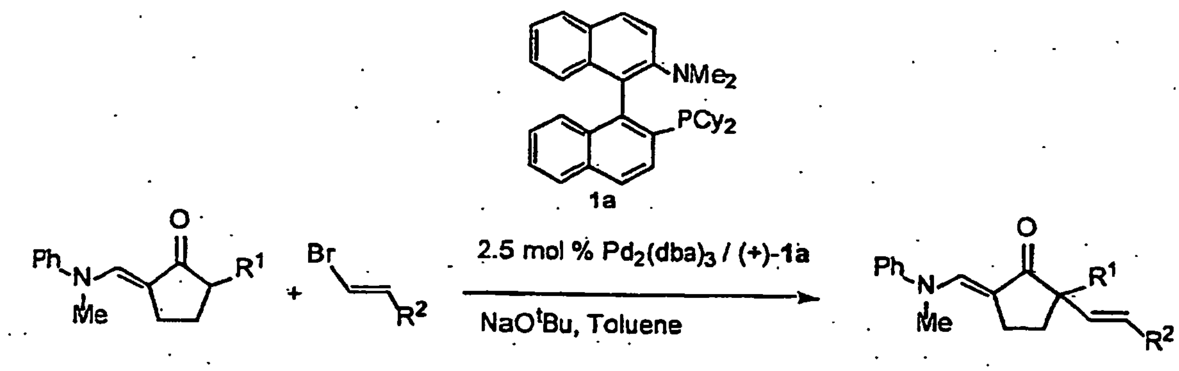


An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 13.6 mg, 0.028 mmol 5.5 mol%). The tube was capped with the septum, purged with argon, and toluene (2 mL) was added through the septum. The mixture was stirred at room temperature for 10 min, then *trans*-β-bromostyrene (128 μL 1.0 mmol) was added through the septum. The septum was removed and 2-*n*-propyl-5-(*N*-methyl-anilinomethylene)cyclopentanone (124 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 170 mg (99 %) of the title compound. The ee was determined to be 86 % by chiral HPLC analysis.

***Example 160***

2-*n*-Pentyl-2-(*trans*-β-styryl)-5-(*N*-methyl-anilinomethylene) cyclopentanone ($R_1$ = n-pentyl; $R_2$ = Ph)

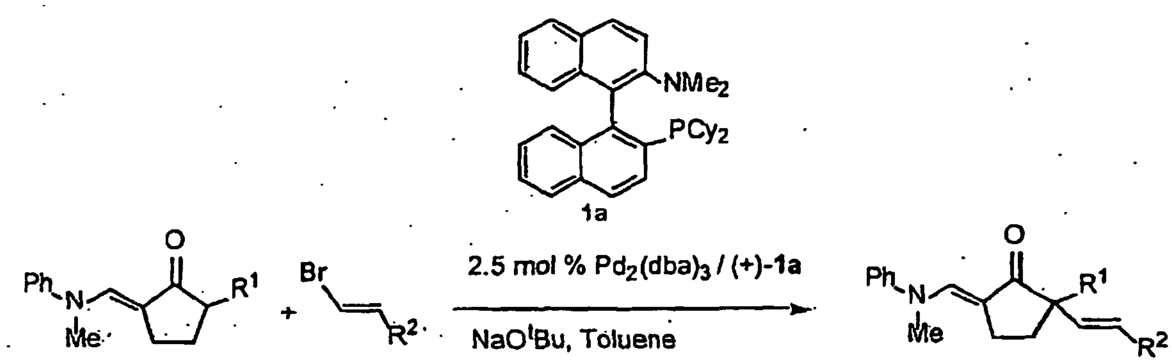


An oven dried Schlenk tube equipped with a rubber septum was purged with argon. The septum was removed; and the tube was charged with trisdibenzylideneacetone dipalladium (11.4 mg, 0.0125 mmol, 5 mol% Pd) and (+)-(**1a**) (according to the invention; 13.6 mg, 0.028 mmol 5.5 mol%). The tube was capped with the septum, purged with argon, and toluene (2 mL) was added through the septum. The mixture was stirred at room temperature for 10 min, then *trans*-β-bromostyrene (128 μL 1.0 mmol) was added through the septum. The septum was removed and 2-*n*-pentyl-5-(*N*-methyl-anilinomethylene)cyclopentanone (136 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 186 mg (98 %) of the title compound. The ee was determined to be 86 % by chiral HPLC analysis.

***Example 161***

2-*n*-Propyl-2-(vinyl)-5-(*N*-methyl-anilinomethylene) cyclopentanone ($R_1$ = n-Pr: $R_2$ = H)

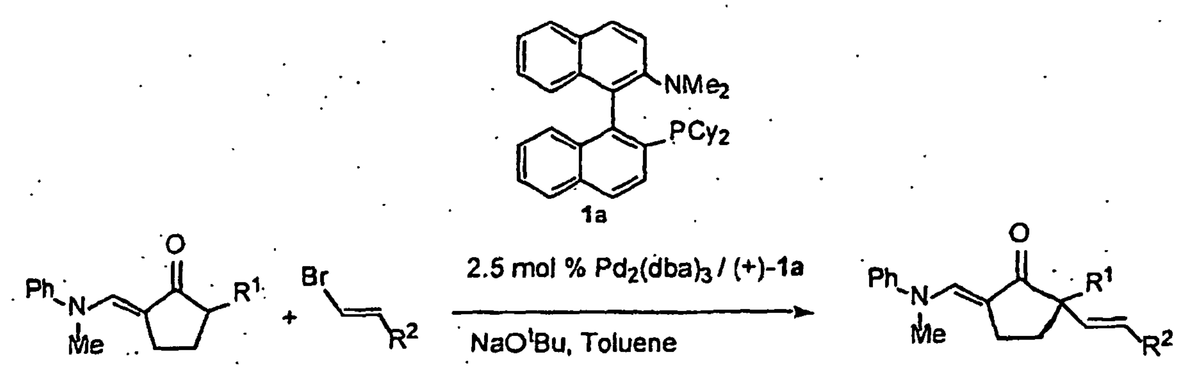


An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)(**1a**) (according to the invention; 13.6 mg, 0.028 mmol 5.5 mol%). The tube was capped with the septum, purged with argon, and toluene (2 mL) was added through the septum. The mixture was stirred at room temperature for 10 min, then vinylbromide (1 mL, I M solution in THF, 1.0 mmol) was added through the septum. The septum was removed and 2-*n*-propyl-5-(*N*-methyl-anilinomethylene)cyclopentanone (136 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 115 mg (86%) of the title compound. The ee was determined to be 86 % by chiral HPLC analysis.

***Example 162***

2-Methyl-2-(vinyl)-5-(*N*-methyl-anilinomethylene)cyclohexanone

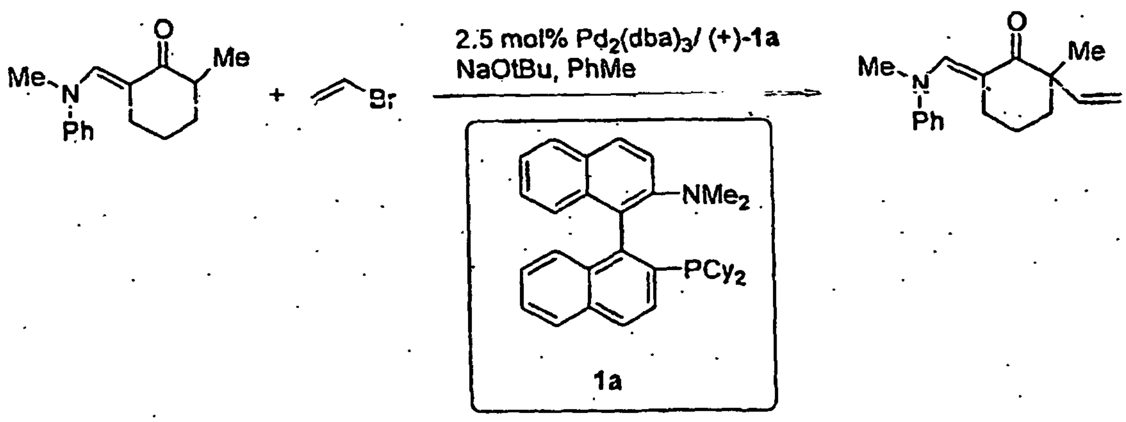


An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 14.8 mg, 0.03 mmol 6 mol%). The tube was capped with the septum, purged with argon,

and toluene (2 mL) was added through the septum. The mixture was stirred at room temperature for 10 min, then vinylbromide (1 mL, 1 M solution in THF, 1.0 mmol) was added through the septum. The septum was removed and 2-methyl-5-(*N*-methyl-anilinomethylene)cyclohexanone (115 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL); diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 103 mg (79%) of the title compound. The ee was determined to be 48 % by chiral HPLC analysis.

***Example 163***

2-Methyl-2-(*trans*-1-propenyl)indanone

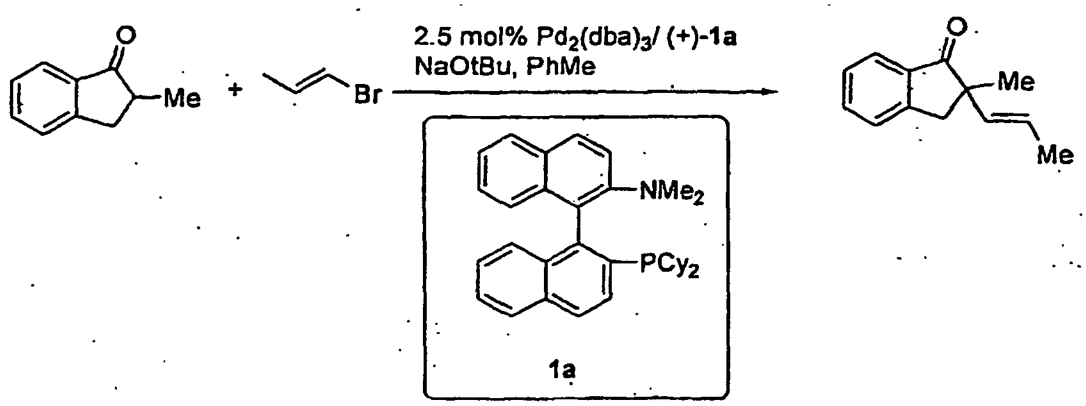


An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 14.8 mg, 0.03 mmol 6 mol%). The tube was capped with the septum, purged with argon, and toluene (2 mL) was added through the septum. The mixture was stirred at room temperature for 10 min, then *trans*-1-bromopropene (86 μL 1.0 mmol) and 2-methylindanone (70 μL, 0.5 mmol) were added through the septum. The septum was removed and sodium *t*-butoxide (96 mg, 1.0 mmol) was added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 90 mg (97 %) of the title compound. The ee was determined to be 72 % by chiral HPLC analysis.

***Example 164***

2-Methyl-2-(*trans*-1-propenyl)-5-(*N*-methyl-anilinomethylene) cyclopentanone

(See Figure 26)

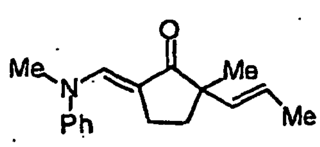

Catalyst solution: An oven dried Schlenk tube equipped with a rubber septum was purged with argon. The septum was removed, and the tube was charged with trisdibenzylideneacetone dipalladium (3.4 mg, 0.00375 mmol) and (-)-(**1a**) (according to the invention; 4.4 mg, 0.090 mmol). The tube was capped with the septum, purged with argon, and toluene (1.50 mL) was added through the septum. The mixture was stirred at room temperature for 10 min.

An oven dried Schlenk tube equipped with a rubber septum was purged with argon. The septum was removed, and the tube was charged with 1 mL of the catalyst solution above and 1 mL of toluene. The mixture was stirred at room temperature for 10 min, then *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0. mmol) were added. The tube was capped with the septum and purged with argon. Additional toluene (4 mL) was added and the mixture was stirred at room temperature for 18h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo*. The crude material was purified by flash chromatography on silica gel to afford 114 mg (89 %) of the title compound. The ee was determined to be 82 % by chiral HPLC analysis.

***Example 165***

2-Methyl-2-(*trans*-1-propenyl)-5-(*N*-methyl-anilinomethylene) cyclopentanone

(See Figure 26)

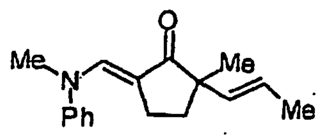

Catalyst solution: An oven dried Schlenk tube equipped with a rubber septum was purged with argon. The septum was removed, and the tube was charged with trisdibenzylideneacetone dipalladium (3.4 mg, 0:00375 mmol) and (-)-(**1a**) (according to the invention; 4.4 mg, 0.090 mmol). The tube was capped with the septum, purged with argon, and toluene (1.50 mL) was added through the septum. The mixture was stirred at room temperature for 10 min.

An oven dried Schlenk tube equipped with a rubber septum was purged with argon. The septum was removed, and the tube was charged with 0.1 mL of the catalyst solution above and 1 mL of toluene. The mixture was stirred at room temperature for 10 min, then *trans*-1-bromopropene (8.7 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 18h. The mixture was quenched with saturated aqueous ammonium chloride (5 mL); diluted with ether (20 mL) and poured into a. separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 110 mg (89 %) of the title compound. The ee was determined to be 82 % by chiral HPLC analysis.

***Example 166***

2-Methyl-2-(*trans*-1-pronenyl)-5-(*N*-methyl-anilinomethylene) cyclopentanone

(See Figure 26).

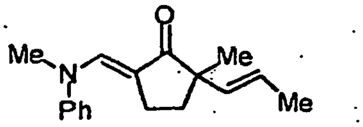

An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 14.8 mg, 0.030 mmol 6 mol%). The tube was capped with the septum, purged with argon, and toluene (2 mL) was added through the septum. The mixture was stirred at room temperature for 10 min, then cooled to 0 C and *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 20h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 109 mg (85%) of the title compound. The ee was determined to be 93 % by chiral HPLC analysis.

***Example 167***

2-Methyl-2-(*trans*-1-propenyl)-5-(*N*-methyl-anilinomethylene) cyclopentanone

(See Figure 26)

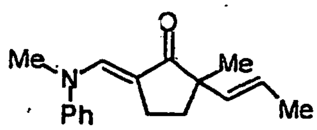

An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (+)-(**1a**) (according to the invention; 14.8 mg, 0.030 mmol 6 mol%). The tube was capped with the septum, purged with argon, and toluene (2 mL) was added through the septum. The mixture was stirred at room temperature for 10 min, then cooled to -20 C and *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 20h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 59 mg (47%) of the title compound. The ee was determined to be 96 % by chiral HPLC analysis.

***Example 168***

2-Methyl-2-(*trans*-1-propenyl)-5-(*N*-methyl-anilinomethylene) cyclopentanone

(See Figure 26).

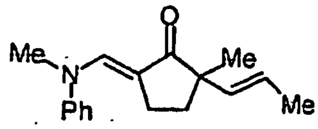

An oven dried Schlenk tube equipped with a rubber septum was purged with argon. The septum was removed, and the tube was charged with trisdibenzylideneacetone dipalladium (4.6 mg, 0.005 mmol, 2 mol% Pd) and (R)-(**1b**) (not according to the invention; 5.8 mg, 0.0125 mmol 2.5 mol%). The tube was capped with the septum, purged with argon, and toluene (2 mL) was added through the septum. The mixture was stirred at room temperature for 10 min, then *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (1 mL) was added and the mixture was stirred at room temperature for 15h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 112 mg (88%) of the title compound. The ee was determined to be 65 % by chiral HPLC analysis.

***Example 169***

2-Methyl-2-(*trans*-1-propenyl)-5-(*N*-methyl-anilinomethylene) cyclopentanone

(See Figure 26)

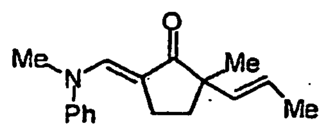

An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (R)-(**1b**) (not according to the invention; 16.3 mg, 0.035 mmol 7mol%). The tube was capped with the septum, purged with argon, and toluene (2 mL) was added through the septum. The mixture was stirred at room temperature for 10 min, then cooled to 0 C and *trans*-1-bromopropene (87 μL 1-0 mmol) was added through the septum. The septum was removed and 2-methyl-5-(*N*-methyl-anilinomethylene)cyclopentanone (108 mg, 0.5 mmol) and sodium *t*-butoxide (96 mg; 1,0 mmol) were added. The tube was capped with the septum and purged with argon. Additional toluene (4 mL) was added and the mixture was stirred at room temperature for 20h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 104 mg (81%) of the title compound. The ee was determined to be 79 % by chiral HPLC analysis.

***Example 170***

2-Methyl-2-(*trans*-1-propenyl)-5-(*N*-methyl-anilinomethylene)cyclopentanone

(See Figure 26)

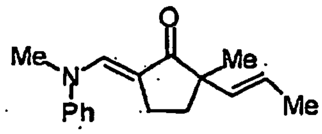

An oven dried Schlenk tube equipped with a rubber septum was purged with argon. The septum was removed, and the tube was charged with trisdibenzylideneacetone dipalladium (11.4-mg, 0.0125 mmol, 5-mol% Pd) and (R)-(**1c**) (not according to the invention; 22.6 mg, 0.035 mmol 7 mol%). The tube was capped with the septum, purged with argon, and toluene (2 mL) was added through the septum. The mixture was stirred at room temperature for 10 min, then cooled to 0 C and *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 20h. The mixture was quenched with saturated aqueous ammonium chloride (5 mL), diluted with ether (20.mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 91 mg (71%) of the title compound. The ee was determined to be 82 % by chiral HPLC analysis.

***Example 171***

2-Methyl-2-(*trans*-1-propenyl)-5-(*N*-methyl-anilinomethylene)cyclopentanone

(See Figure 26)

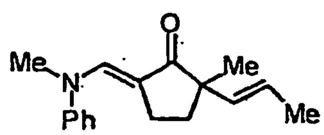

An oven dried Schlenk tube equipped with a rubber septum was purged with argon. The septum was removed, and the tube was charged with trisdibenzylideneacetone dipalladium (11.4 mg, 0.0125 mmol, 5 mol% Pd) and (R)-(**2**) (not according to the invention; 16.3 mg, 0.035 mmol 7 mol%). The tube was capped with the septum, purged with argon, and toluene (2 mL) was added through the septum. The mixture was stirred at room temperature for 10 min, then *trans*-1-bromopropene (87 μL 1.0 mmol) was added through the septum. The septum was removed and 2-methyl-5-(*N*-methyl-anilinomethylene)cyclopentanone (108 mg, 0.5 mmol) and sodium *t*-butoxide (96 mg, 1.0 mmol) were added. The tube was capped with the septum and purged with argon. Additional toluene (4 mL) was added and the mixture was stirred at room temperature for 20h. The mixture was quenched with saturated aqueous ammonium chloride (5 mL), diluted with ether (20 mL) and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 72 mg (56%) of the title compound. The ee was determined to be 35 % by chiral HPLC analysis.

***Example 172***

α-Arylation of *tert*-butyl acetate using Pd without a phosphine ligand (not according to the invention)

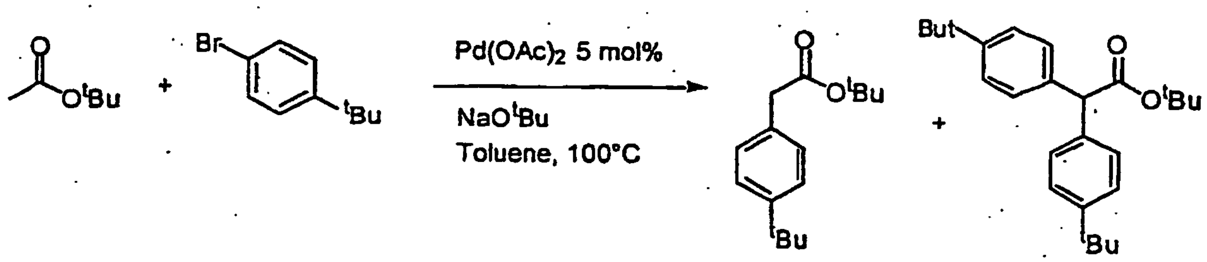


An oven dried Schlenk tube equipped with a rubber septum was cooled under an argon purge. The septum was removed, and the tube was charged with palladium acetate (11.5 mg, 0.05 mmol, 2.5 mol%), the tube was capped with the septum, purged with argon, and toluene (3 mL) was added through the septum. The mixture was stirred at room temperature and I - bromo-4-*t*-butylbenzene (0.34 mL, 2.0 mmol) and *t*-butyl acetate (0.32 mL, 2.5 mmol) were added through the septum. The septum was removed and sodium *t*-butoxide (460 mg, 4.8 mmol) was added. The tube was capped with the septum and purged with argon. Additional toluene (6 mL) was added, and the mixture was heated to 100 °C with stirring until the starting halide had been consumed as judged by GC analysis (48h). The mixture was cooled to room temperature and quenched with saturated aqueous ammonium chloride (35 mL). The mixture was diluted with ether (20 mL), and poured into a separatory funnel. The layers were separated and the aqueous layers were extracted with ether (20 mL). The combined organic layers were washed with brine (20 mL), dried over anhydrous magnesium sulfate, filtered, and concentrated *in vacuo*. The crude material was purified by flash chromatograpy on silica gel to afford 209 mg of a mixture of monoarylated and diarylated compound (2 : 1 by GC analysis).

***Example 173***

Synthesis of 2-Cyano-4'-methylbiphenyl via Suzuki coupling of 2-chlorobenzonitrile with p-tolylboronic acid, using 0.05 mol% Pd and potassium phosphate

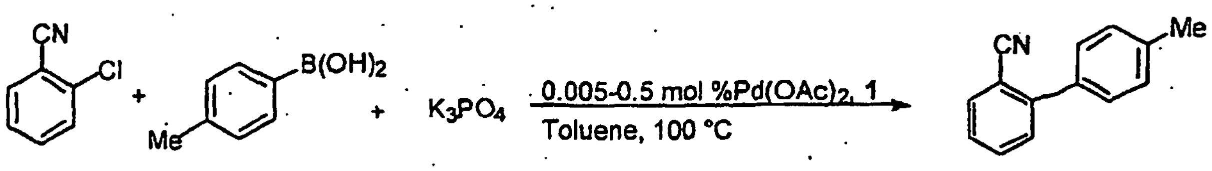


1= 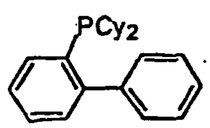

An oven-dried resealable Schlenk tube was evacuated and backfilled with argon and charged with 2-chlorobenzonitrile (138 mg, 1.0 mmol), p-tolylboronic acid (163 mg, 1.5 mmol), and $K_3PO_4$ (425 mg, 2.0 mmol). The tube was evacuated and backfilled with argon and charged with toluene (1.5 mL). A separate flask was purged with argon and charged with $Pd(OAc)_2$ (5.6 mg, 0.025 mmol) and **1** (not according to the invention; 17.5 mg, 0.05 mmol). The flask was purged with argon and THF (5 mL) was added. The mixture was stirred at rt for 5 min then 100 μL of this catalyst solution (0.05 mol % Pd, 0.1 mol % 1) was added to the tube containing the halide/boronic acid/$K_3PO_4$ mixture followed by additional toluene (1.5 mL). The tube was sealed with a teflon screwcap and heated to 100 °C with stirring until the starting aryl chloride had been completely consumed (17h). The mixture was cooled to room temperature, diluted with ether (50 mL) and washed with aqueous NaOH (1M, 50 mL). The layers were separated and the aqueous layer was extracted with ether (50 ml). The combined organic layers were dried over anhydrous magnesium sulfate, filtered, and concentrated *in vacuo.* The crude material was purified by flash chromatography on silica gel to afford 176 mg (91%) of the title compound.

***Example 174***

Synthesis of 2-Cyano-4'-methylbiphenyl via Suzuki coupling of 2-chlorobenzonitrile with p-tolylboronic acid, using 0.01 mol% Pd and potassium phosphate

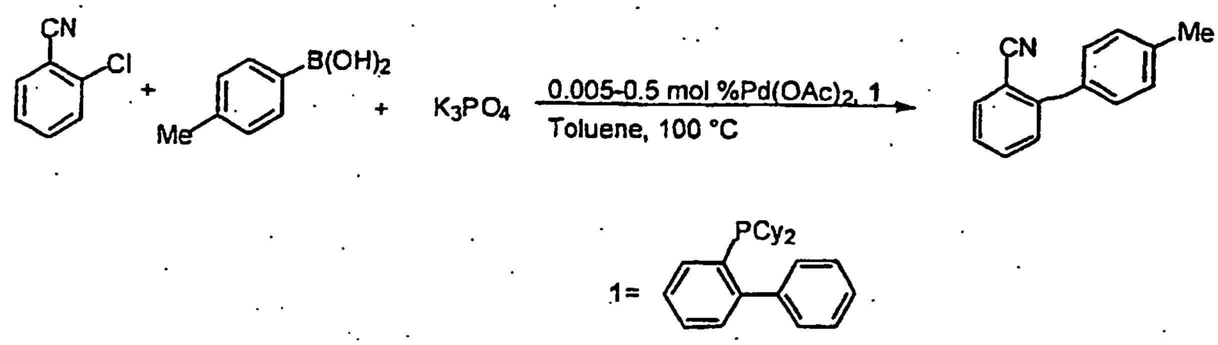


An oven-dried resealable Schlenk tube was evacuated and backfilled with argon and charged with 2-chlorobenzonitrile (138 mg, 1.0 mmol), p-tolylboronic acid (163 mg, 1.5 mmol), and $K_3PO_4$ (425 mg, 2.0 mmol). The tube was evacuated and backfilled with argon and charged with toluene (1.5 mL). A separate flask was purged with argon and charged with $Pd(OAc)_2$ (4.5 mg, 0.02 mmol) and **1** (not according to the invention; 14.0 mg, 0.04 mmol). The flask was purged with argon and THF (10 mL) was added. The mixture was stirred at rt for 5 min then 50 μL of this catalyst solution (0.01 mol % Pd, 0.02 mol % **1**) was added to the tube containing the halide/boronic acid/$K_3PO_4$ mixture followed by additional toluene (1.5 mL). The tube was sealed with a teflon screwcap and heated to 100 ˚C with stirring until the reaction was no longer progressing as judged by GC analysis (17 h). GC analysis showed the reaction had proceeded to ~71 % conversion; the product was detected by GC.

***Example 175***

Synthesis of *N*-(4-Acetylphenyl)morpholine via catalytic amination of an aryl chloride using CsF as base

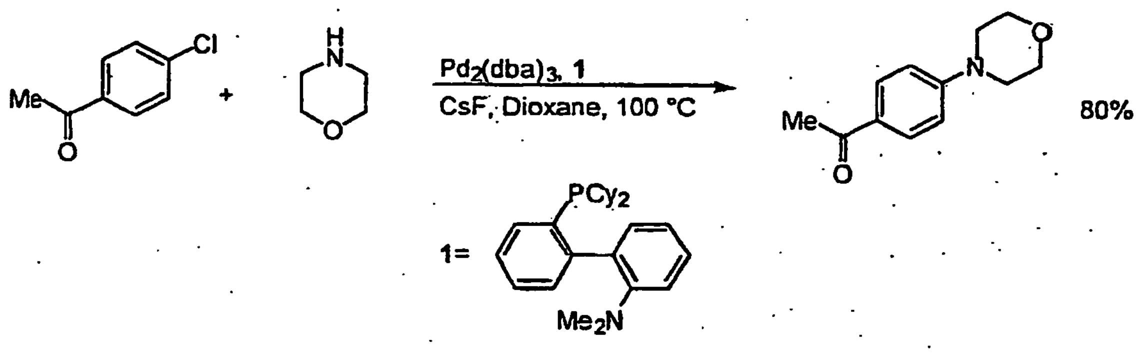


An oven-dried resealable Schlenk tube was evacuated and backfilled with argon. The tube was charged with $Pd_2(dba)_3$ (4.6 mg, 0.005. mmol, 1.0 mol % Pd), **1** (not according to the invention; 5.9 mg, 0.015 mmol, 1.5 mol %), and CsF (304 mg, 2.0 mmol). The tube was evacuated and backfilled with argon and Dioxane (2 mL), 4'-chloroacetophenone (0.13 mL, 1.0 mmol), and morpholine (0.10 mL, 1.2 mmol) were added through a rubber septum. The tube was sealed with a teflon screwcap and heated to 100 ˚C with stirring until the starting material had been completely consumed as judged by GC analysis (31 h). The mixture was cooled to room temperature, diluted with ether/ethyl acetate (1/1 v//v, 50 mL), filtered through celite, and concentrated *in vacuo.* The crude material was purified by flash chromatography on silica gel to afford 165 mg (80%) of the title compound.

***Example 176***

Synthesis of α-(4-*tert*-butylphenyl)-nitroethane via α-arylation of nitroethane.

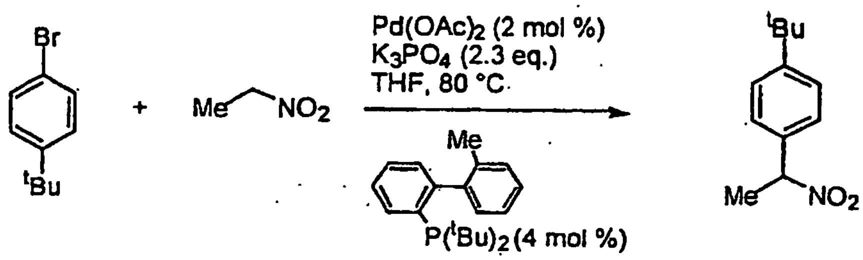


A dry Schlenk tube containing a stirbar was charged with palladium acetate (4.4 mg, 0.02 mmol), 2-di(*tert*-butyl) phosphino-2'-methylbiphenyl (not according to the invention; 12.5 mg, 0.04 mmol), and potassium phosphate (490 mg, 2.3 mmol). After a septum was placed on top of the tube, it was evacuated and refilled with argon. THF (1 mL), 4-bromo-*tert*-butylbenzene (213 mg, 0.173 mL, 1.0 mmol) and nitroethane (90 mg, 0.086 mL, 1.2 mmol) were sequentially injected, and, under a flow of argon, the septum was replaced with a teflon screw cap. The tube was then sealed and heated in an oil bath at 80 °C for 12.5 h. The contents of the tube were partitioned between 1 N HCl and ether, and the aqueous layer twice extracted with ether. The combined organics were washed with water, dried ($Na_2SO_4$) filtered, and the solvents were stripped. Chromatography, eluting with 5 % ethyl acetate/ 95% hexane gave 38 mg (18%) of α-(4-*tert*-butylphenyl)-nitroethane, an oil.

***Example 177***

Synthesis of *N*-(4-*t*-Butylphenyl)morpholine via room-temperature catalytic amination of an aryl triflate

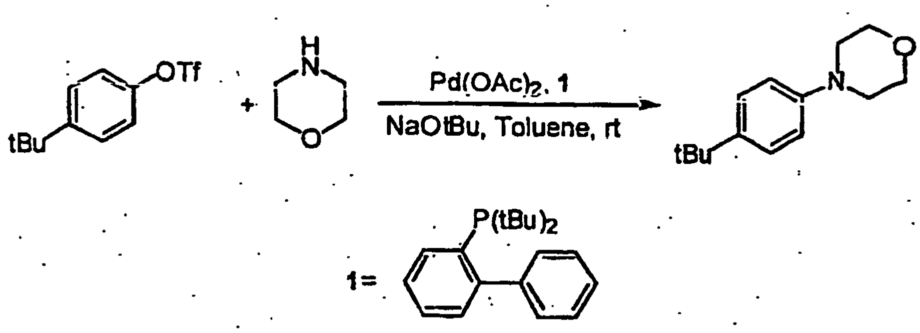


An oven-dried resealable Schlenk tube was evacuated and backfilled with argon. The tube was charged with $Pd(OAc)_2$ (2.2 mg, 0.01 mmol, 1.0 mol %), **1** (not according to the invention; 6.0 mg, 0.02 mmol, 2 mol %), and NaO*t*Bu. (135 mg, 1.4 mmol). The tube was evacuated and backfilled with argon and toluene (2 mL), 4-*t*-butylbromobenzene (0.17 mL. 1.0 mmol), and morpholine (0-10 mL) were added through a rubber septum. The tube was sealed with a teflon screwcap and stirred at rt for 22 h. GC analysis of the reaction mixture showed that the reaction had proceeded to approximately 85% conversion; the desired product had formed as well as 4-t-butylphenol. The ratio of desired product/ 4-t-butylphenol was approximately 6/1.

***Example 178***

Room-Temperature Heck arylation of styrene with an aryl bromide

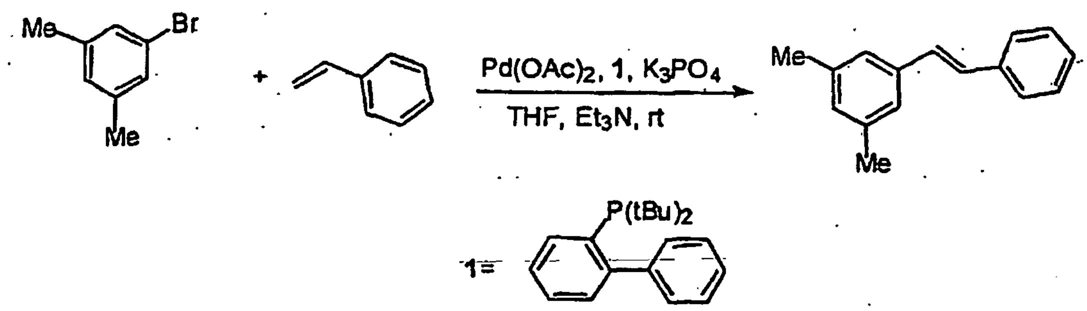


An oven-dried resealable Schlenk tube was evacuated and backfilled with argon. The tube was charged with $Pd(OAc)_2$ (2.2 mg, 0.01 mmol, 1 mol %), **1** (not according to the invention; 6.0 mg (0.02 mmol, 2 mol %), and $K_3PO_4$ (318 mg, 1.5 mmol). The tube was evacuated and backfilled with argon and THF (0.5 mL), triethylamine (0.5 mL), 5-bromo-m-xylene (0.135 mL, 1.0 mmol), and styrene (0.15 mL, 1.3 mmol) were added through a rubber septum. The tube was sealed with a teflon screwcap and stirred at room temperature for 3 days. GC analysis showed that the reaction had proceeded to 75 % conversion and gave a mixture of the desired product along with two olefin isomers in a ratio of 48.8/2.2/1.9 (desired/other/other). The identities of the products were confirmed by GC and GC/MS analysis.

***Example 179***

Asymmetric Alkylation and Vinylation of Ketone Enolates

The creation of all-carbon quaternary centers with absolute control of stereochemistry remains a great challenge in organic synthesis.[1] A number of methods have been developed to accomplish this task, including the Pd-catalyzed asymmetric allylations of soft enolates reported by Hayashi (β-diketones)[2] and Trost (β-ketoesters).[3] We now report the first examples, to our knowledge; of the catalytic asymmetric arylation of ketone enolates to produce all-carbon quaternary centers:[4]

We recently disclosed that nascent ketone enolates generated in presence of an aryl bromide and a catalytic quantity of a Pd catalyst are converted to their α-aryl

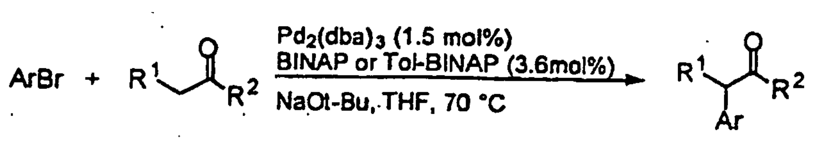

(1)

derivatives with a high degree of regioselectivity (eq 1).[5-7] As our initial protocol employed (S)-Tol-BINAP/$Pd_2(dba)_3$ [dba= dibenzylideneacetone] as catalyst, the application of this methodology to asymmetric arylation processes was of interest (BINAP and Tol-BINAP are not ligands according to the invention). Our initial attempts at asymmetric arylation to produce tertiary stereocenters either by direct arylation or desymmetrization of cyclic ketones gave disappointing results. Our attention then turned to the formation of quaternary centers. In our first experiments we were able to asymmetrically arylate 2-methyl-α-tetralone with 1-bromo-4-*t*-butylbenzene to give the desired product with an ee of 61 %, albeit in low yield. Subsequent experimentation has led to an improvement upon these initial results, which we now report.

We found that both the yield and the enantioselectivity of the arylation of 2-methyl-α-tetralone could be brought to good levels by running the arylation using 10-20 mol % Pd(0)/12-24 mol % BINAP in toluene at 100 °C.[8,9] It was found that an excess of aryl bromide was necessary to ensure complete conversion of the ketone; 2-methyl-1-naphthol, biaryls, and compounds resulting from aldol condensation were formed as side-products. In some reactions, the α-phenylated ketone was also observed as a side product Subsequent experiments demonstrated that the latter side product was a result of aryl transfer from the phosphine ligand.[10] Using the conditions described above, the arylations of 2-methyl-α-tetralone proceeded with enantioselectivities up to 88 % (Table 1).[11] We have also briefly examined the reactions of 2-methyl-1-indanone, **1**. Using 5 mol % $Pd(OAc)_2$/12 mol % BINAP the reaction with

Table 1

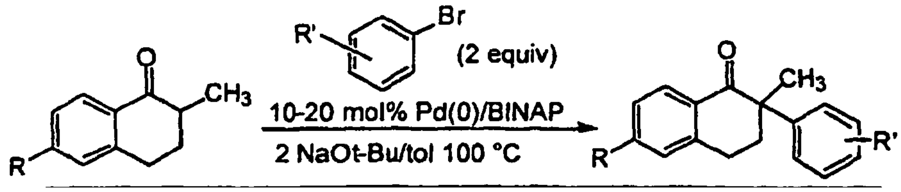


| R | 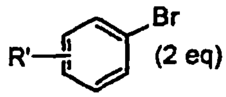 | % ee | % yield |
|---|---|---|---|
| H | 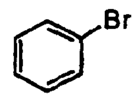 | 73 | 66 |
| H | 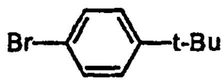 | 88 | 73[a] |
| MeO | 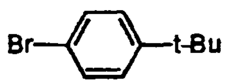 | 77 | 56[b] |
| H | 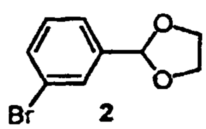 2 | 84 | 74 |
| H | 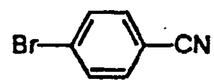 | 61 | 40[c] |

(a) Product contains 4% of 2-methyl-2-phenyl-1-tetralone and 3% of a regioisomer which was present in the starting aryl bromide (percentages determined by GC analysis). (b) Product contains 3% of a regioisomer which was present in the starting aryl bromide (percentages determined by GC analysis). (c) The reaction was run at 70 °C using 5.0 equiv halide and 5.0 equiv NaOtBu.

bromide **2** proceeded smoothly to give **3** in 79 % yield with an ee of 70 % (eq 2).[12] Surprisingly, preliminary attempts to couple para-substituted aryl bromides with **1** gave products which were racemic.

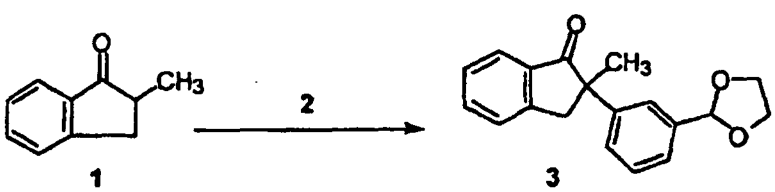


**1** **3**

79 % yield
70 % ee (2)

We next extended our investigation to include some α'-blocked α-methylcycloalkanones. These studies gave some enigmatic, yet intriguing results.

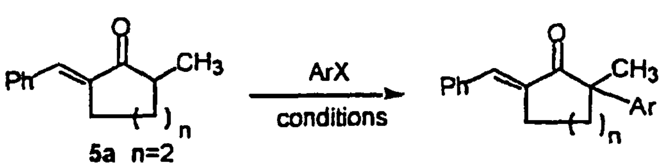


n= 2 low e.e.'s
n= 1 high e.e.'s (3)

**5a** n=2
**5b** n=1

For example, treatment of 2-methylcyclohexanone derivative **5a**[13] with a number of aryl bromides under conditions similar to those described above (or using NaHMDS[14] as base) yielded products with very low ee's (eq 3). However, the reactions of the analogue **5b**[15] proceeded with high yields and with extremely high levels of enantioselecitivity as is shown below (Figure 1). Meta- or para-substituted aryl bromides, coupled

**Figure 1**

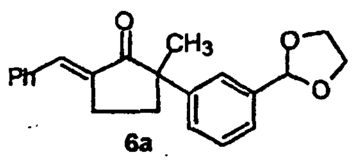

**6a**

**86 % yield, 95 % ee (NaHMDS, 10 mol% $Pd_2(dba)_3$/BINAP)**

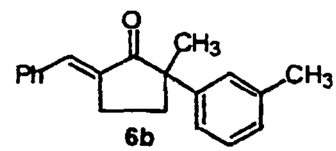

**6b**

**80 % yield, 94 % ee (NaOtBu, 20 mol% $Pd(OAc)_2$/BINAP)**

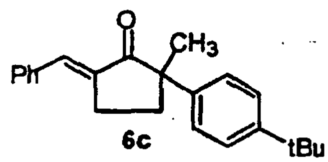

**6c**

**75 % yield, 98 % ee (NaOtBu, 20 mol% $Pd(OAc)_2$/BINAP)**

with **5b** to give the desired products in very good yield and in a highly enantioselective fashion. If NaHMDS and $Pd_2(dba)_3$ was used in place of NaO*t*-Bu and $Pd(OAc)_2$, **6c**[16] was formed in 91 % yield and with an ee of 92 %.

There are a number of mysterious features of these reactions. For example, we currently have no good explanation for the difference in levels of enantioselectivity observed for the reactions of **5a** and **5b** under identical reaction conditions. Moreover, while the reactions of **5b**, shown above, proceed with high levels of enantioselectivity, in preliminary studies, similar reactions with 2-bromopropene, 2,4-dimethylbromobenzene or the triflate derived from 4-hydroxy(methylbenzoate) yielded racemic products. Additionally, while the coupling of **1** and **2** gives **3** with an ee of 70 %, the analogous reactions of **1** with para-substituted aryl bromides yields racemic products.

The mechanism of this reaction presumably follows a similar pathway to the one postulated for the non-asymmetric Pd-catalyzed α-arytation of ketones. At this point in time it is not clear which step or steps in the catalytic cycle determine the enantioselectivity of the overall process.

*References and Footnotes for Example 179*

(1) Fuji, K. *Chem. Rev.* **1993**, *93*, 2037-2066.

(2) Hayashi, T.; Kanehira, K.; Hagihara, T.; Kumada, M. *J. Org. Chem.* **1988**, *53*, 113-120.

(3) Trost, B. M.; Radinov, R.; Grenzer, E. M. *J. Am. Chem. Soc.* **1997**, *119*, 7879-7880.

(4) The palladium-catalyzed asymmetric arylation of a silyl ketene acetal, [E-MeCH=C(OMe)($OSiMe_3$)], using a stoichiometric amount of TIOAc to form tertiary carbon centers has been reported (ee's range from 37-54%; only 2 aryl halides were examined). The asymmetric arylation of the corresponding tin enolate was also studied, although lower ee's were obtained: Galarini, R.; Musco, A.; Pontellini, R. *J. Mol. Cat.* **1992**, *72*, L11-L13.

(5) Palucki, M.; Buchwald, S. L. *J. Am. Chem. Soc.* **1997**, *In Press.*

(6) (a) Similar processes (as in ref 5a) have recently been described by others: Hamann, B. C.; Hartwig, J. F. *J. Am. Chem. Soc.* **1997**, *In Press;* Muratake has recently reported a related Pd-catalyzed intramolecular α-arylation of ketones: (b) Muratake, H.; Hayakawa, A.; Natsume, M. *Tetrahedron Lett.* **1997**, *38*, 7577-7580. (c) Muratake, H.; Natsume, M. *Tetrahedron Lett.* **1997**, *38,* 7581-7582. (d) Satoh has recently reported a single example of the Pd-catalyzed diarylation of 1,3-diphenylacetone with iodobenzene to form 1,1,3,3-tetraphenylacetone: *Angew. Chem. Int. Ed. Engl.* **1997**, *36*, 1740-1742.

(7) For other examples of enolate α-arylation, see references contained in 5 and 6c.

(8) Control experiments were run with no palladium catalyst in the presence of NaHMDS at 100 °C for the reaction of 2-methyl-1-tetralone with 1-bromo-4-*t*-butylbenzene and with 4-bromobenzonitrile. 1-bromo-4-*t*-butylbenzene did not react with the tetralone in the absence of palladium catalyst. The reaction involving 4-bromobenzonitrile showed

~7% conversion after 1 h, but did not proceed further after heating for another 2 h.

(9) Reactions of 2-ethyl-1-tetralone are inefficient under these conditions.

(10) Using Tol-BINAP instead of BINAP gave small amounts of the α-(*p*-tolyl) ketone and none of the phenylated ketone could be detected.

(11) Representative procedure: An oven-dried Schlenk tube was charged with $Pd_2(dba)_3$ or $Pd(OAc)_2$ (10-20 mol% Pd), (S)-(-)-BINAP (12-24 mol%, 1.2 L/Pd), and sodium *t*-butoxide (96 mg, 1.0 mmol). The tube was purged with argon and toluene (6 mL) was added. The mixture was stirred at room temperature for 1 minute. The aryl halide (1.0 mmol) and an internal standard (dodecane, 0.115 mL, 0.5 mmol) were added and the mixture was stirred at room temperature for 1 minute. 2-methyl-1-tetralone (0.075 mL, 0.5 mmol) and additional toluene (3 mL) were added and the reaction mixture was heated to 100 ˚C with stirring until the ketone had been consumed as judged by GC or TLC analysis. The reaction mixture was cooled to room temperature, quenched with saturated aqueous ammonium chloride (~5 mL) and diluted with ether (~10 mL). The layers were separated and the aqueous portion was extracted with ether (~20 mL), and the combined organic layers were washed with saturated brine (~10 mL), dried over anhydrous magnesium sulfate, filtered, and concentrated *in vacuo.* The crude product was purified by flash chromatography on silica gel. Products which were difficult to completely separate from BINAP by silica gel chromatography were purified according to an alternative workup procedure. See supporting information for full experimental details.

(12) In preliminary experiments we have shown that **1** reacts with 2-bromopropene to give product with an ee of ~60-70%. It is worth noting, that an asymmetric vinylation/olefin hydrogenation sequence is the synthetic equivalent of a catalytic asymmetric alkylation.

(13) Johnson, W. S. *J. Am. Chem. Soc.* **1943**, *65*, 1317-1324.

(14) NaHMDS = Sodium Hexamethyldisilazide (sodium bis(trimethylsilyl)amide).

(15) Sato, T.; Hayase, K. *Bull. Chem. Soc. Jpn.* **1991,** *64*, 3384-3389.

(16) This product contained 2 % (as judged by GC analysis) of a regioisomer which was also present in the starting aryl halide. No regioisomers were observed with any of the other compounds reported in this paper which were made from halides other than 1-bromo-4-*t*-butylbenzene.

***Example 180***

Synthesis of 2-methyl-2-[3-(2-dioxolane)phenyl]-1-tetralone (BINAP ligand; not according to the invention )

An oven dried Schlenk tube equipped with a rubber septum was cooled under an argon purge. The septum was removed, and the tube was charged with sodium *t*-butoxide (96 mg, 1.0 mmol), palladium acetate (5.6 mg, 0.025 mmol, 5 mol % Pd), and (S)-BINAP (18.7 mg, 0.03 mmol, 6 mol%): The tube was capped with the septum, and purged with argon. Toluene (6 mL) was added, and the mixture was stirred at room temperature for 1 min. 2-(3-bromophenyl)-1,3-dioxolane (0.15 mL, 1.0 mmol) was added through the septum, and the mixture was stirred at room temperature for 1 min. 2-methyl-1-tetralone (87 mg), and additional toluene (3 mL) were added through the septum, and the mixture was heated to 100 ˚C with stirring until the starting ketone had been completely consumed as judged by GC analysis. The mixture was cooled to room temperature, quenched with saturated aqueous ammonium chloride (5 mL), and diluted with ether (20 mL). The mixture was poured into a separatory funnel and the layers were separated. The aqueous layer was extracted with ether (20 mL), and the combined organic layers were washed with brine (20 mL), dried over anhydrous magnesium sulfate, filtered, and concentrated *in vacuo.* The crude material was purified by flash chromatography on slilica gel to afford 107 mg (69%) of the title compound. The ee was determined to be 84 % by chiral HPLC analysis.

***Example 181***

Synthesis of 2-methyl-2-[3-(2-dioxolane)phenyl]-1-tetralone (BIPHEMP ligand ; not according to the invention)

An oven dried Schlenk tube equipped with a rubber septum was cooled under an argon purge. The septum was removed, and the tube was charged with sodium *t*-butoxide (96 mg, 1.0 mmol), palladium acetate (5.6mg, 0.025

mmol, 5 mol % Pd), and (S)-BIPHEMP (16.5 mg, 0.03 mmol, 6 mol%). The tube was capped with the septum, and purged with argon. Toluene (6 mL) was added, and the mixture was stirred at room temperature for 1 min. 2-(3-bromophenyl)-1,3-dioxolane (0.15 mL, 1.0 mmol) was added through the septum, and the mixture was stirred at room temperature for 1 min. 2-methyl-1-tetralone (87 mg), and additional toluene (3 mL) were added through the septum, and the mixture was heated to 100 ˚C with stirring until the starting ketone had been completely consumed as judged by GC analysis. The mixture was cooled to room temperature, quenched with saturated aqueous ammonium chloride (5 mL), and diluted with ether (20 mL). The mixture was poured into a separatory funnel and the layers were separated. The aqueous layer was extracted with ether (20 mL), and the combined organic layers were washed with brine (20 mL), dried over anhydrous magnesium sulfate, filtered, and concentrated *in vacuo.* The crude material was purified by flash chromatography on silica gel to afford 60 mg (39%).of the title compound. The ee was determined to be 82 % by chiral HPLC analysis.

***Example 182***

Synthesis of 2-methyl-2-[3-(2-dioxolane)phenyl-]-1-tetralone (MeO-BIPHEP ligand; not according to the invention)

An oven dried Schlenk tube equipped with a rubber septum was cooled under an argon purge. The septum was removed, and the tube was charged with sodium *t*-butoxide (96 mg, 1.0 mmol), palladium acetate (5.6 mg, 0.025 mmol, 5 mol % Pd), and (R)-MeO-BIPHEP (17.5. mg, 0.03 mmol, 6 mol%). The tube was capped with the septum, and purged with argon. Toluene (6 mL) was added, and the mixture was stirred at room temperature for 1 min. 2-(3-bromophenyl)-1,3-dioxolane (0.15 mL, 1.0 mmol) was added through the septum, and the mixture was stirred at room temperature for 1 min. 2-methyl-1-tetralone (87 mg), and additional toluene (3 mL) were added through the septum, and the mixture was heated to 100 ˚C with stirring until the starting ketone had been completely consumed as judged by GC analysis. The mixture was cooled to room temperature, quenched with saturated aqueous ammonium chloride (5 mL), and diluted with ether (20 mL). The mixture was poured into a separatory funnel and the layers were separated. The aqueous layer was extracted with ether (20 mL), and the combined organic layers were washed with brine (20 mL), dried over anhydrous magnesium sulfate, filtered, and concentrated *in vacuo.* The crude material was purified by flash chromatography on silica gel to afford 91 mg (59%) of the title compound. The ee was determined to be 85 % by chiral HPLC analysis.

***Example 183***

Synthesis of 2-methyl-2-[3-(2-dioxolane)phenyl]-1-tetralone (QUINAP ligand ; not according to the invention)

An oven dried Schlenk tube equipped with a rubber septum was cooled under an argon purge. The septum was removed, and the tube was charged, with sodium *t*-butoxide (96 mg 1.0 mmol), palladium acetate (5.6 mg, 0.025 mmol, 5 mol % Pd), and (R)-QUINAP (13.2 mg, 0.03 mmol, 6 mol%). The tube was capped with the septum, and purged with argon. Toluene (6 mL) was added, and the mixture was stirred at room temperature for 1 min. 2-(3-bromophenyl)-1,3-dioxolane (0.15 mL, 1.0 mmol) was added through the septum, and the mixture was stirred at room temperature for 1 min. 2-methyl-1-tetralone (87 mg), and additional toluene (3 mL) were added through the septum, and the mixture was heated to 100 ˚C with stirring until the starting ketone had been completely consumed, as judged by GC analysis. The mixture was cooled to room temperature, quenched with saturated aqueous ammonium chloride (5 mL), and diluted with ether (20 mL). The mixture was poured into a separatory funnel and the layers were separated. The aqueous layer was extracted with ether (20 mL), and the combined organic layers were washed with brine (20 mL), dried over anhydrous magnesium sulfate, filtered, and concentrated *in vacuo.* The crude material was purified by flash chromatography on silica gel to afford 66 mg (43%) of the title compound. The ee was determined to be 81 % by chiral HPLC analysis.

***Example 184***

Synthesis of 2-methyl-2-[3-(2-dioxolane)phenyl]-1-tetralone (NORPHOS ligand ; not according to the invention)

An oven dried Schlenk tube equipped with a rubber septum was cooled under an argon purge. The septum was removed, and the tube was charged with sodium *t*-butoxide (96 mg, 1.0 mmol), palladium acetate (5.6 mg, 0.025 mmol, 5 mol % Pd), and (R,R)-NORPHOS (13.9 mg, 0.03 mmol, 6 mol%). The tube was capped with the septum, and purged with argon. Toluene (6 mL) was added, and the mixture was stirred at room temperature for 1 min. 2-(3-bromophenyl)-1,3-dioxolane (0.15 mL, 1.0 mmol) was added through the septum, and the mixture was stirred at room temperature for 1 min. 2-methyl-1-tetralone (87 mg), and additional toluene (3 mL) were added through the septum, and the mixture was heated to 100 ˚C with stirring until the starting ketone had been completely consumed as judged by GC analysis. The mixture was cooled to room temperature, quenched with saturated aqueous ammonium chloride (5 mL), and diluted with ether (20 mL). The mixture was poured into a separatory funnel and the layers were separated. The aqueous layer

was extracted with ether (20 mL), and the combined organic layers were washed with brine (20 mL), dried over anhydrous magnesium sulfate, filtered, and concentrated *in vacuo*. The crude material was purified by flash chromatography on silica gel to afford 58 mg (38%) of the title compound. The ee was determined to be 40% by chiral HPLC analysis.

***Example 185***

Synthesis of 2-methyl-2-vinyl-1-tetralone

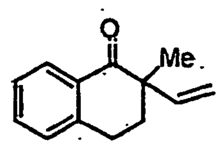

An oven dried Schlenk tube equipped with a rubber septum was purged with argon. The septum was removed, and the tube was charged with palladium acetate (5.6 mg, 0.025 mmol, 5 mol%) and (-)-2-(dicyclohexylphosphino)-2'-(dimethylamino)-1,1'-binaphthyl (according to the invention; 13.6 mg, 0.028 mmol- 5.5 mol%). The tube was capped with the septum, purged with argon, and toluene: (2 mL) and triethylamine (5 mg, 0.05 mmol) were added through the septum. The mixture was stirred at room temperature for 3 min, then vinyl bromide (1.0 mL 1.0 mmol) and 2-methyl-1-tetralone (81 mg, 0.5 mmol) were added through the septum. The septum was removed and sodium *t*-butoxide (96 mg, 1.0 mmol) was added. The tube was capped with the septum and purged with argon. Additional toluene (4 mL) was added and the mixture was stirred at room temperature for 2h. The mixture was quenched with saturated aqueous ammonium chloride (5 mL), diluted with ether (20 mL), and poured into a separatory funnel. The layers were separated and the aqueous layer was extracted with ether (20 mL). The combined organic layers were washed with brine (20 mL), dried over anhydrous magnesium sulfate, filtered, and concentrated *in vacuo.* The crude material was purified by flash chromatography on silica gel to afford 83 mg (88 %) of title compound. The ee was determined to be 79 % by chiral HPLC analysis.

## Claims

1. The compound represented by general structure 7:

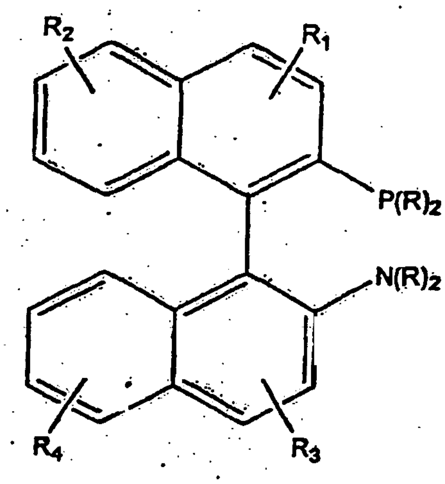

7

wherein

R is selected, independently for each occurrence, from the set consisting of alkyl, heteroalkyl, cycloalkyl, heterocycloalkyl, aryl, heteroaryl, aralkyl, heteroaralkyl, and $-(CH_2)_m-R_{80}$;

the A, B, A', and B' rings of the binaphthyl core independently may be unsubstituted or substituted with $R_1$, $R_2$, $R_3$, and $R_4$, respectively, any number of times up to the limitations imposed by stability and the rules of valence; $R_1$, $R_2$, $R_3$, and $R_4$, are selected, independently for each occurrence, from the set consisting of alkyl, heteroalkyl, cycloalkyl, heterocycloalkyl, aryl, heteroaryl, aralkyl, heteroaralkyl, halogen, $-SiR_3$, and $-(CH_2)_m-R_{80}$;
$R_{80}$ represents an unsubstituted or substituted aryl, a cycloalkyl, a cycloalkenyl, a heterocycle, or a polycycle;
m is an integer in the range 0 to 8 inclusive; and
the ligand, when chiral, may be provided in the form of a mixture of enantiomers or as a single enantiomer.

**2.** The compound of claim 1, wherein:

$R_1$, $R_2$, $R_3$, and $R_4$, are absent;
both instances of R on the N depicted explicitly are lower alkyl, preferably methyl; and
both instances of R on P depicted explicitly are cycloalkyl, preferably cyclohexyl.

**3.** The compound of claim 1, wherein the $PR_2$ group comprises an asymmetric P.

**4.** The compound of claim 1, wherein the $PR_2$ group comprises an asymmetric P; and the binaphthyl core is axially chiral.

**5.** The process represented by the generalized reaction depicted in Scheme 1:

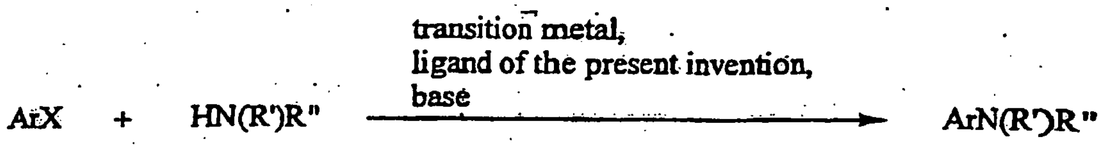


Scheme 1

wherein

Ar is selected from the set consisting of optionally substituted monocyclic and polycyclic aromatic and heteroaromatic moieties;
X is selected from the set consisting of Cl, Br, I, $-OS(O)_2$alkyl, and $-OS(O)_2$aryl;
R' and R" are selected, independently for each occurrence, from the set consisting of H, alkyl, heteroalkyl, aryl, heteroaryl, aralkyl, alkoxyl, amino, trialkylsilyl, and triarylsilyl;
R' and R", taken together, may form an optionally substituted ring consisting of 3-10 backbone atoms inclusive; said ring optionally comprising one of more heteroatoms beyond the nitrogen to which R' and R" are bonded;
R' and/or R" may be covalently linked to Ar such that the amination reaction is intramolecular;
the transition metal is selected from the set of groups 5-12 metals, preferably the Group VIIIA metals;
the ligand is represented by general structure 7; and
the base is selected from the set consisting of hydrides, carbonates, fluorides, phosphates, alkoxides, phenoxides, amides, carbanions, and silyl anions.

**6.** The process of claim 5, wherein the ligand is covalently linked to a solid support or soluble polymer, or is adsorbed onto a solid.

**7.** The process of claim 5, wherein:

X of ArX is Cl, $-OS(O)_2$alkyl, or $-OS(O)_2$aryl; and
the process is conducted at room temperature.

**8.** The process of claim 5, wherein
the transition metal is palladium; and
the base is a phosphate or fluoride.

**9.** The process of claim 5, wherein

the transition metal is palladium; and
the base is potassium phosphate.

**10.** The process of claim 5, wherein: HN(R')R" represents an optionally substituted heteroaromatic compound.

**11.** The process of claim 5, wherein X of ArX is chloride.

**12.** The process represented by the generalized coupling reaction depicted in Scheme 2:

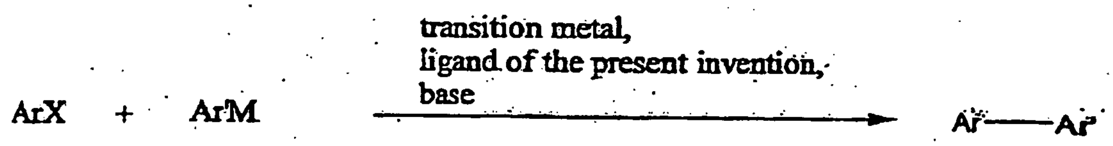


Scheme 2

wherein

Ar and Ar' are independently selected from the set consisting of optionally substituted aromatic, heteroaromatic, and alkenyl moieties;
X is selected from the set consisting of Cl, Br, I, $-OS(O)_2$alkyl, and $-OS(O)_2$aryl;
M represents $B(OR)_2$, Mg(halide), or Zn(halide);
R represents independently for each occurrence H, methyl, alkyl, heteroalkyl, aryl, or heteroaryl; or the two instances of R in an occurrence of $B(OR)_2$, taken together, may represent an optionally substituted two or three carbon tether between the two instances of O;
Ar and Ar' may be covalently linked such that the reaction is intramolecular;
the transition metal is selected from the set of groups 5-12 metals; preferably the Group VIIIA metals;
the ligand is represented by general structure 7; and
the base is selected from the set consisting of carbonates, phosphates, fluorides, alkoxides, amides, carbanions, and silyl anions.

**13.** The process of claim 12, wherein the ligand is covalently linked to a solid support or soluble polymer, or is adsorbed onto a solid.

**14.** The process of claim 12, wherein the ligand is chiral and non-racemic; and the product is not racemic.

**15.** The process of claim 12, wherein:

X of ArX is Cl, $-OS(O)_2$alkyl, or $-OS(O)_2$aryl; and
the process is conducted at room temperature.

**16.** The process of claim 12, wherein
the transition metal is palladium; and
the base is a phosphate or fluoride.

**17.** The process of claim 12, wherein
the transition metal is palladium; and
the base is potassium phosphate.

**18.** The process of claim 12, wherein X of ArX is chloride.

**19.** The process of claim 12, wherein no more than one of the four ortho and ortho' substitutents of Ar-Ar' is hydrogen.

**20.** The process of claim 12, wherein X of ArX is chloride; and no more than one of the four ortho and ortho' substitutents of Ar-Ar' is hydrogen.

**21.** The process of claim 12, wherein M represents $B(OR)_2$.

**22.** The process represented by the generalized coupling reaction depicted in Scheme 3:

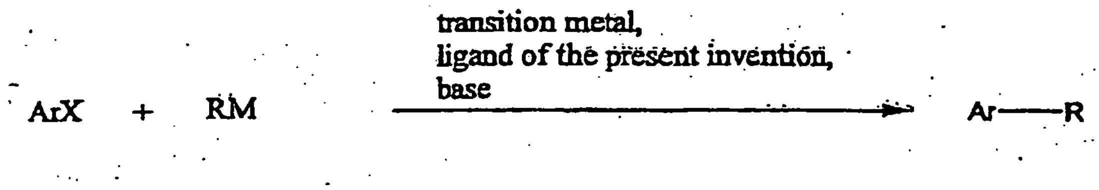


Scheme 3

wherein

Ar is selected from the set consisting of optionally substituted aromatic, heteroaromatic, and alkenyl moieties;
R is selected from the set consisting of optionally substituted alkyl, heteroalkyl, and aralkyl;
R' is selected, independently for each occurrence, from the set of alkyl and heteroalkyl, the carbon-boron bond of said alkyl and heteroalkyl groups being inert under the reaction conditions, e.g., $BR'_2$ taken together represents 9-borobicyclo[3.3.1]nonyl;
M represents $B(R')_2$, Mg(halide), or Zn(halide);
X of ArX is selected from the set consisting of Cl, Br, I, $-OS(O)_2$alkyl, and $-OS(O)_2$aryl;
Ar and R may be covalently linked such that the reaction is intramolecular;
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

**23.** The process of claim 22, wherein the ligand is covalently linked to a solid support or soluble polymer, or is adsorbed onto a solid.

**24.** The process of claim 22, wherein the ligand is chiral and non-racemic; and the product is not racemic.

**25.** The process of claim 22, wherein:

X of ArX is Cl, $-OS(O)_2$alkyl, or $-OS(O)_2$aryl; and
the process is conducted at room temperature.

**26.** The process of claim 22, wherein
the transition metal is palladium; and
the base is a phosphate or fluoride.

**27.** The process of claim 22 , wherein
the transition metal is palladium; and
the base is potassium phosphate.

**28.** The process of claim 22, wherein X of ArX is chloride.

**29.** The process of claim 22, wherein M represents $B(R')_2$.

**30.** The process represented by the generalized $\alpha$-arylation reaction depicted in Scheme 4:

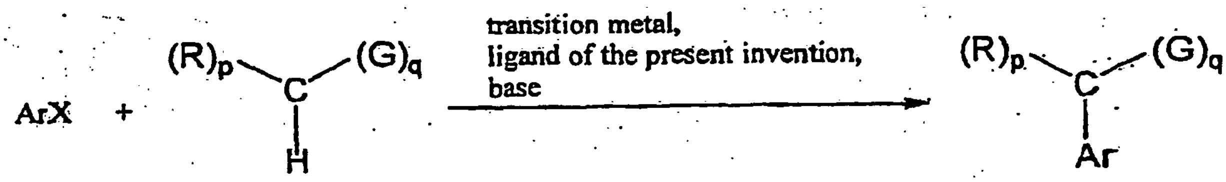


Scheme 4

wherein

Ar is selected from the set consisting of optionally substituted monocyclic and polycyclic aromatic and heteroaromatic moieties;
X is selected from the set consisting of Cl, Br, I, $-OS(O)_2$alkyl, and $-OS(O)_2$aryl;
G represents, independently for each occurrence, an electron withdrawing group selected from the group consisting of formyl, acyl, -CN, -C(O)OR, $-C(O)NR_2$, nitro, nitroso, $-S(O)_2R$, $-SO_3R$, $-S(O)_2NR_2$, -C(NR)-R, -C(NOR)-R, and $-C(NNR_2)$-R;
R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
q is an integer selected from the range 1 to 3 inclusive;
p is an integer equal to (3-q);
Ar and one instance of R may be covalently linked such that the reaction is intramolecular;
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

**31.** The process of claim 30, wherein the ligand is covalently linked to a solid support or soluble polymer, or is adsorbed onto a solid.

**32.** The process of claim 30, wherein the ligand is chiral and non-racemic; and the product is not racemic.

**33.** The process of claim 30, wherein q is 1.

**34.** The process of claim 30, wherein q is 2.

**35.** The process of claim 30, wherein:

X of ArX is Cl, $-OS(O)_2$alkyl, or-$OS(O)_2$aryl; and
the process is conducted at room temperature

**36.** The process of claim 30, wherein:

the transition metal is palladium; and
the base is a phosphate or fluoride.

**37.** The process of claim 30, wherein:

the transition metal is palladium; and
the base is potassium phosphate.

**38.** The process of claim 30, wherein:

X of ArX represents Br, and
the reaction occurs at room temperature.

**39.** The process of claim 30, wherein X of ArX is chloride.

**40.** The process represented by the generalized α-vinylation reaction depicted in Scheme 5:

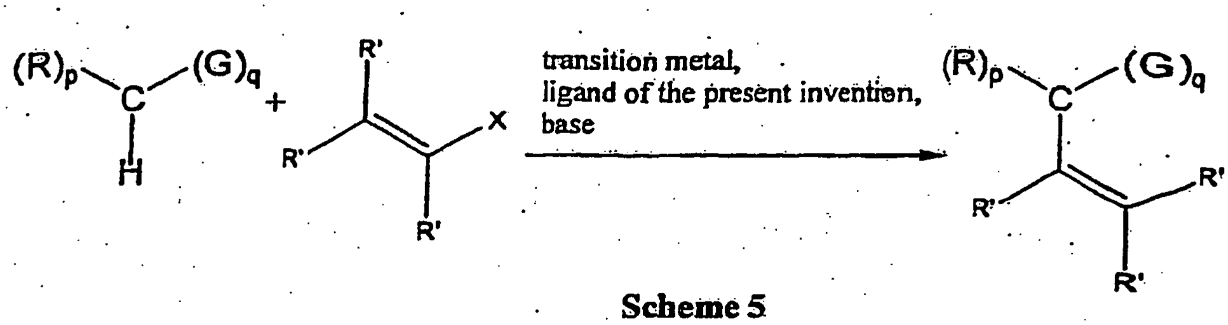


Scheme 5

wherein

X is selected from the set consisting of Cl, Br, I, $-OS(O)_2$alkyl, and $-OS(O)_2$aryl;
G represents, independently for each occurrence, an electron withdrawing group selected from the group consisting of formyl, acyl, -CN, -C(O)OR, $-C(O)NR_2$, nitro, nitroso, $-S(O)_2R$; $-SO_3R$, $-S(O)_2NR_2$, -C(NR)-R, -C(NOR)-R, and $-C(NNR_2)-R$;
R represents, independently for each occurrence, hydrogen, alkyl, aryl, heteroalkyl, heteroaryl, halogen, alkylamino, arylamino, alkylthio, arylthio, alkoxy, aryloxy, or $-(CH_2)_m-R_{80}$;
R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
q is an integer selected from the range 1 to 3 inclusive;
p is an integer equal to (3-q);
an instance of R and an instance of R' may be covalently linked such that the reaction is intramolecular;
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

**41.** The process of claim 40, wherein the ligand is covalently linked to a solid support or soluble polymer, or is adsorbed onto a solid.

**42.** The process of claim 40, wherein the ligand is chiral and non-racemic; and the product is not racemic.

**43.** The process of claim 40, wherein q is 1.

**44.** The process of claim 40, wherein q is 2.

**45.** The process of claim 40, wherein:

X of $(R')_2CC(R')X$ is $-OS(O)_2$alkyl, or $-OS(O)_2$aryl; and
the process is conducted at room temperature.

**46.** The process of claim 40, wherein:

the transition metal is palladium; and
the base is a phosphate or fluoride:

**47.** The process of claim 40, wherein:

the transition metal is palladium; and
the base is potassium phosphate.

**48.** The process of claim 40, wherein:

X of $(R')_2CC(R')X$ represents Br; and
the reaction occurs at room temperature.

**49.** The process of claim 40, wherein X of $(R')_2CC(R')X$ is chloride.

**50.** The process represented by the generalized O-arylation reaction depicted in Scheme 6:

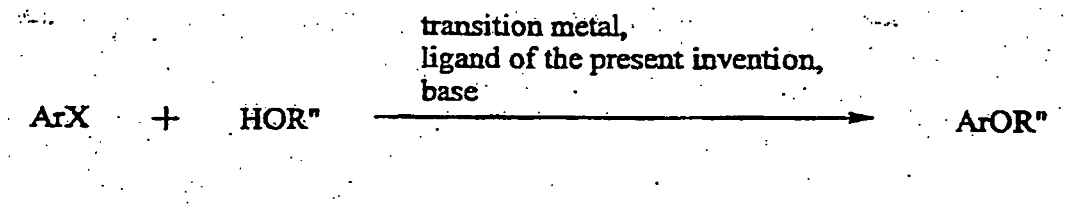


**Scheme 6**

wherein

Ar is selected from the set consisting of optionally substituted monocyclic and polycyclic aromatic and heteroaromatic moieties;
X is selected from the set consisting of Cl, Br, I, $-OS(O)_2$alkyl, and $-OS(O)_2$aryl;
R" represents, independently for each occurrence, alkyl, aryl, aralkyl, heteroalkyl, heteroaryl, heteroaralkyl, -Si(alkyl)$_3$, -Si(aryl)$_3$, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurence, is an integer selected from the range 0 to 8 inclusive;
Ar and R" may be covalently linked such that the reaction is intramolecular;
the transition metal is selected from the set of groups 5-12 metals, preferably the Group VIIIA metals;
the ligand is represented by general structure 7;
the base is selected from the set consisting of carbonates, phosphates, fluorides, alkoxides, amides, carbanions, and silyl anions; and
the process is conducted at room temperature.

**51.** The process of claim 50, wherein the ligand is covalently linked to a solid support or soluble polymer, or is adsorbed onto a solid.

**52.** The process of claim 50, wherein R"OH is a primary alcohol.

**53.** The process of claim 50, wherein:

X of ArX is Cl, $-OS(O)_2$alkyl, or $-OS(O)_2$aryl.

**54.** The process of claim 50, wherein:

the transition metal is palladium; and
the base is a phosphate or fluoride.

**55.** The process of claim 50, wherein:

the transition metal is palladium; and
the base is potassium phosphate.

**56.** The process of claim 50, wherein:

X of ArX represents Br.

**57.** The process claim 50, wherein X of ArX is chloride.

**58.** The process represented by the generalized O-vinylation reaction depicted in Scheme 7:

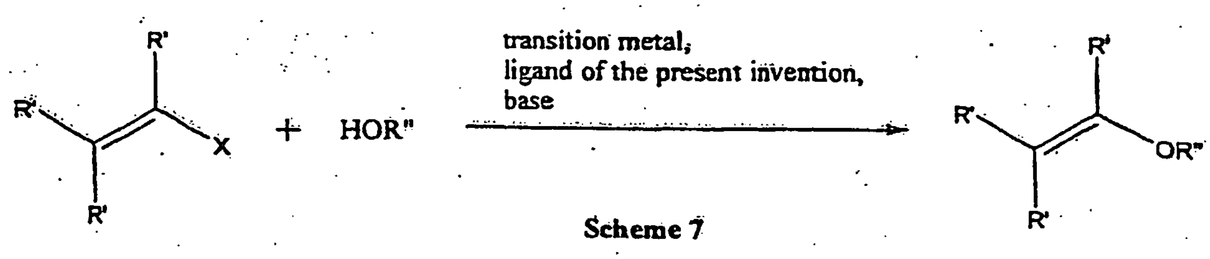


Scheme 7

wherein

X is selected from the set consisting of Cl, Br, I, $-OS(O)_2$alkyl, and $OS(O)_2$aryl;
R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;
R" represents, independently for each occurrence, alkyl, aryl, aralkyl, heteroalkyl, heteroaryl, heteroaralkyl, -Si(alkyl)$_3$, -Si(aryl)$_3$, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
R" and an instance of R' may be covalently linked such that the reaction is intramolecular;
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

**59.** The process of claim 58, wherein the ligand is covalently linked to a solid support or soluble polymer, or is adsorbed onto a solid.

**60.** The process of claim 58, wherein R"OH is a primary alcohol.

**61.** The process of claim 58, wherein:

X of $(R')_2CC(R')X$ is $-OS(O)_2$alkyl, or $-OS(O)_2$aryl; and
the process is conducted at room temperature.

**62.** The process of claim 58, wherein:

the transition metal is palladium; and
the base is a phosphate or fluoride.

**63.** The process of claim 58, wherein:

the transition metal is palladium; and
the base is potassium phosphate.

**64.** The process of claim 58, wherein:

X of $(R')_2CC(R')X$ represents Br; and
the reaction occurs at room temperature.

**65.** The process of claim 58, wherein X of $(R')_2CC(R')X$ is chloride.

**66.** The process represented by the generalized Heck reaction depicted in Scheme 8:

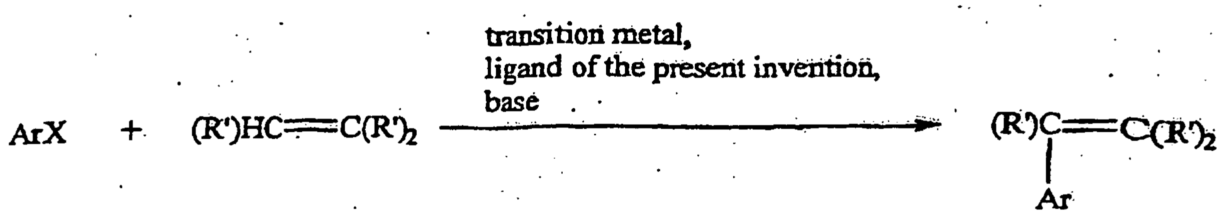


Scheme 8

wherein

Ar is selected from the set consisting of optionally substituted aromatic, heteroaromatic, and alkenyl moieties;
X is selected from the set consisting of Cl, Br, I, $-OS(O)_2$alkyl, and $-OS(O)_2$aryl;
R' represents, independently for each occurrence, hydrogen, alkyl, aryl, aralkyl, heteroalkyl, heteroaryl, heteroaralkyl, alkylamino, arylamino, alkylthio, arylthio, alkoxy, aryloxy, or $-(CH_2)_m-R_{80}$;
$R_{80}$ represents independently for each occurrence a substituted or unsubstituted aryl, cycloalkyl cycloalkenyl, heterocycle or polycycle;
m, independently for each occurrence, is an integer selected from the range 0 to 8 inclusive;
Ar and an instance of R' may be covalently linked such that the reaction is intramolecular,
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions:

**67.** The process of claim 66, wherein the ligand is covalently linked to a solid support or soluble polymer, or is adsorbed onto a solid.

**68.** The process of claim 66, wherein:

X of ArX is Cl, $-OS(O)_2$alkyl, or $-OS(O)_2$aryl; and
the method is conducted at room temperature.

**69.** The process of claim 66, wherein
the transition metal is palladium; and
the base is a phosphate or fluoride.

**70.** The process of claim 66, wherein:

the transition metal is palladium; and
the base is potassium phosphate.

**71.** The process represented by the generalized Heck reaction depicted in Scheme 9:

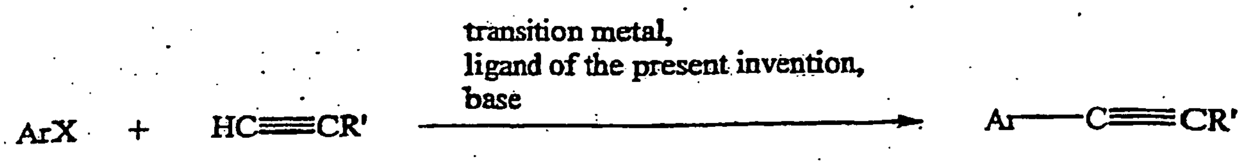


Scheme 9

wherein

Ar is selected from the set consisting of optionally substituted aromatic, heteroaromatic, and alkenyl moieties;
X is selected from the set consisting of Cl, Br, I, $-OS(O)_2$alkyl, and $-OS(O)_2$aryl;
R' represents, independently for each occurrence, hydrogen, alkyl aryl, aralkyl, heteroalkyl, heteroaryl, heteroaralkyl, alkylamino, arylamino, alkylthio, arylthio, alkoxy; aryloxy, or $-(CH_2)_m-R_{80}$;
$R_{80}$ represents independently for each occurrence a substituted or unsubstituted aryl, cycloalkyl, cycloalkenyl, heterocycle or polycycle;
m, independently for each occurrence, is an integer selected from the range 0 to 8 inclusive;
Ar and R' may be covalently linked such that the reaction is intramolecular,
the transition metal is selected from the set of groups 5-12 metals, preferably the Group VIIIA metals;
the ligand is represented by general structure 7; and
the base is selected from the set consisting of carbonates, phosphates, fluorides, alkoxides, amides, carbanions, and silyl anions.

**72.** The process of claim 71, wherein the ligand is covalently linked to a solid support or soluble polymer, or is adsorbed onto a solid.

**73.** The process of claim 71, wherein:

X of ArX is Cl, $-OS(O)_2$alkyl, or $-OS(O)_2$aryl; and
the process is conducted at room temperature.

**74.** The process of claim 71, wherein:

the transition metal is palladium; and
the base is a phosphate or fluoride.

**75.** The process of claim 71, wherein:

the transition metal is palladium; and
die base is potassium phosphate.

**76.** The process of any one of claims 5, 12, 22, 30, 40, 50 or 58, wherein less than 0.01 mol% of the catalyst relative to the limiting reagent is utilized.

**77.** The process of any one of claims 5, 12, 22, 30, 40, 50 or 58, wherein less than 0.0001 mol% of the catalyst relative to the limiting reagent is utilized.

**78.** The process of any one of claims 5, 12, 22, 30, 40, 50 or 58, wherein less than 0.000001 mol% of the catalyst relative to the limiting reagent is utilized.

## Patentansprüche

**1.** Verbindung der allgemeinen Struktur 7:

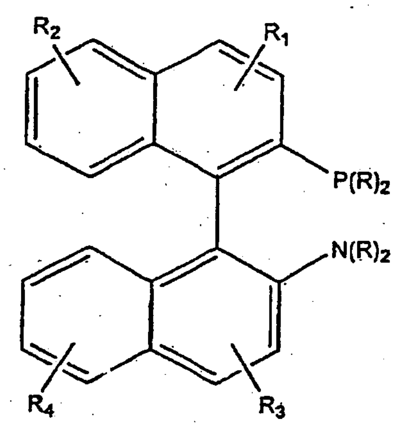

7

wobei

R bei jedem Vorkommen unabhängig voneinander aus Alkyl, Heteroalkyl, Cycloalkyl, Heterocycloalkyl, Aryl, Heteroaryl, Aralkyl, Heteroaralkyl und $-(CH_2)_m-R_{80}$ ausgewählt ist;
die A-, B-, A'- und B'-Ringe des Binaphthylkerns unabhängig voneinander unsubstituiert oder beliebig oft mit jeweils $R_1$, $R_2$, $R_3$ und $R_4$ substituiert sein können, bis zu den Beschränkungen, die durch Stabilität und den Regeln der Valenz auferlegt werden;
$R_1$, $R_2$, $R_3$, und $R_4$ bei jedem Vorkommen unabhängig voneinander aus Alkyl, Heteroalkyl, Cycloalkyl, Heterocycloalkyl, Aryl, Heteroaryl, Aralkyl, Heteroaralkyl, Halogen, $-SiR_3$ und $-(CH_2)_m-R_{80}$ ausgewählt sind;
$R_{80}$ ein unsubstituiertes oder substituiertes Aryl, ein Cycloalkyl, ein Cycloalkenyl, einen Heterocyclus oder einen Polycyclus darstellt;
m eine ganze Zahl im Bereich von 0 bis 8 einschließlich ist; und
der Ligand, wenn er chiral ist, in der Form eines Enantiomergemisches oder als einzelnes Enantiomer bereitgestellt werden kann.

**2.** Verbindung nach Anspruch 1, wobei:

$R_1$, $R_2$, $R_3$ und $R_4$ abwesend sind;
beide Vorkommen von R, die an dem N ausdrücklich dargestellt sind, Niederalkyl, vorzugsweise Methyl, sind; und
beide Vorkommen von R, die an P ausdrücklich dargestellt sind, Cycloalkyl, vorzugsweise Cyclohexyl, sind.

**3.** Verbindung nach Anspruch 1, wobei der $PR_2$-Rest ein asymmetrisches P umfasst.

**4.** Verbindung nach Anspruch 1, wobei der $PR_2$-Rest ein asymmetrisches P umfasst; und der Binaphthylkern axial chiral ist.

**5.** Verfahren der verallgemeinerten Reaktion, die in Schema 1 dargestellt ist:

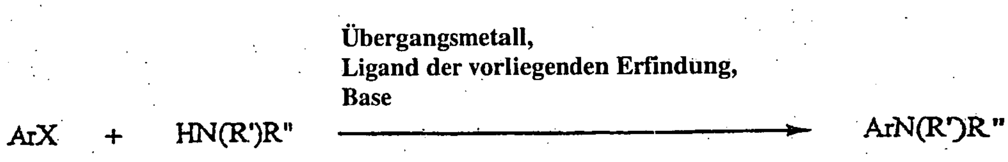


**Schema 1**

wobei

Ar aus gegebenenfalls substituierten monocyclischen und polycyclischen aromatischen und heteroaromatischen Einheiten ausgewählt ist;
X aus Cl, Br, I, $-OS(O)_2$-Alkyl, und $-OS(O)_2$-Aryl ausgewählt ist,
R' und R" bei jedem Vorkommen unabhängig voneinander aus H, Alkyl, Heteroalkyl, Aryl, Heteroaryl, Aralkyl, Alkoxyl, Amino, Trialkylsilyl und Triarylsilyl ausgewählt sind;
R' und R" zusammengenommen einen gegebenenfalls substituierten Ring bilden können, welcher aus 3 bis 10 Gerüstatomen einschließlich besteht, wobei der Ring gegebenenfalls ein oder mehrere Heteroatome zusätzlich zu dem Stickstoff, an den R' und R" gebunden sind, umfasst;
R' und/oder R" kovalent an Ar gebunden sein können, so dass die Aminierungsreaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Hydriden, Carbonaten, Fluoriden, Phosphaten, Alkoxiden, Phenoxiden, Amiden, Carbanionen und Silylanionen ausgewählt ist.

**6.** Verfahren nach Anspruch 5, wobei der Ligand kovalent an einen festen Träger oder an ein lösliches Polymer gebunden ist oder auf einen Feststoff adsorbiert ist.

**7.** Verfahren nach Anspruch 5, wobei:

X von ArX Cl, $-OS(O)_2$-Alkyl oder $-OS(O)_2$-Aryl ist; und
das Verfahren bei Raumtemperatur durchgeführt wird.

**8.** Verfahren nach Anspruch 5, wobei
das Übergangsmetall Palladium ist; und
die Base ein Phosphat oder Fluorid ist.

**9.** Verfahren nach Anspruch 5, wobei
das Übergangsmetall Palladium ist; und
die Base Kaliumphosphat ist.

**10.** Verfahren nach Anspruch 5, wobei: HN(R')R" eine gegebenenfalls substituierte heteroaromatische Verbindung darstellt.

**11.** Verfahren nach Anspruch 5, wobei X von ArX Chlorid ist.

**12.** Verfahren der verallgemeinerten Kupplungsreaktion, die in Schema 2 dargestellt ist:

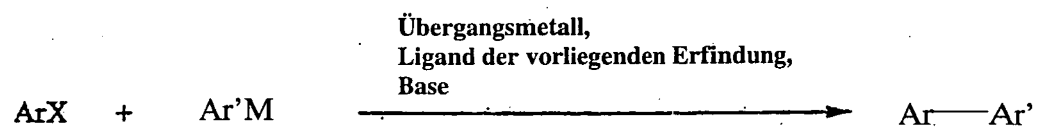


Schema 2

wobei

Ar und Ar' unabhängig voneinander aus gegebenenfalls substituierten aromatischen, heteroaromatischen Einheiten und Alkenyleinheiten ausgewählt sind;
X aus Cl, Br, I, $-OS(O)_2$-Alkyl, und $-OS(O)_2$-Aryl ausgewählt ist;
M $B(OR)_2$, Mg(Halogenid) oder Zn(Halogenid) darstellt;
R bei jedem Vorkommen unabhängig voneinander H, Methyl, Alkyl, Heteroalkyl, Aryl oder Heteroaryl darstellt;
oder die zwei Vorkommen von R, wenn sie in $B(OR)_2$ vorkommen, zusammengenommen eine gegebenenfalls

substituierte Verknüpfung mit zwei oder drei Kohlenstoffatomen zwischen den beiden Vorkommen von O darstellen können;
Ar und Ar' kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**13.** Verfahren nach Anspruch 12, wobei der Ligand kovalent an einen festen Träger oder an ein lösliches Polymer gebunden ist oder auf einen Feststoff adsorbiert ist.

**14.** Verfahren nach Anspruch 12, wobei der Ligand chiral und nicht racemisch ist; und das Produkt nicht racemisch ist.

**15.** Verfahren nach Anspruch 12, wobei:

X von ArX Cl, $-OS(O)_2$-Alkyl oder $-OS(O)_2$-Aryl ist; und
das Verfahren bei Raumtemperatur durchgeführt wird.

**16.** Verfahren nach Anspruch 12, wobei
das Übergangsmetall Palladium ist; und
die Base ein Phosphat oder Fluorid ist.

**17.** Verfahren nach Anspruch 12, wobei
das Übergangsmetall Palladium ist; und
die Base Kaliumphosphat ist.

**18.** Verfahren nach Anspruch 12, wobei X von ArX Chlorid ist.

**19.** Verfahren nach Anspruch 12, wobei nicht mehr als einer der vier ortho- und ortho'-Substituenten von Ar-Ar' Wasserstoff ist.

**20.** Verfahren nach Anspruch 12, wobei X von ArX Chlorid ist; und nicht mehr als einer der vier ortho- und ortho'-Substituenten von Ar-Ar' Wasserstoff ist.

**21.** Verfahren nach Anspruch 12, wobei M $B(OR)_2$ darstellt.

**22.** Verfahren der verallgemeinerten Kupplungsreaktion, die in Schema 3 dargestellt ist:

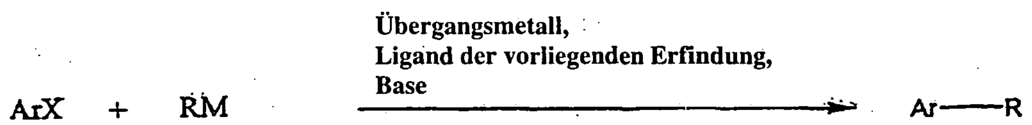


Schema 3

wobei

Ar aus gegebenenfalls substituierten aromatischen, heteroaromatischen Einheiten und Alkenyleinheiten ausgewählt ist;
R aus gegebenenfalls substituiertem Alkyl, Heteroalkyl und Aralkyl ausgewählt ist;
R' bei jedem Vorkommen unabhängig voneinander aus Alkyl und Heteroalkyl ausgewählt ist; wobei die Kohlenstoff-Bor-Bindung der Alkyl- Und Heteroalkylreste unter den Reaktionsbedingungen inert ist, z.B. stellt $BR'_2$ zusammengenommen 9-Borbicyclo[3.3.1]nonyl dar;
M $B(R')_2$, Mg(Halogenid) oder Zn(Halogenid) darstellt;
X von ArX aus Cl, Br, I, $-OS(O)_2$-Alkyl und $-OS(O)_2$-Aryl ausgewählt ist;

Ar und R kovalent gebunden sein können, so dass die Reaktion intramolekular ist; das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**23.** Verfahren nach Anspruch 22, wobei der Ligand kovalent an einen festen Träger oder an ein lösliches Polymer gebunden ist oder auf einen Feststoff adsorbiert ist.

**24.** Verfahren nach Anspruch 22, wobei der Ligand chiral und nicht racemisch ist; und das Produkt nicht racemisch ist.

**25.** Verfahren nach Anspruch 22, wobei:

X von ArX Cl, $-OS(O)_2$-Alkyl oder $-OS(O)_2$-Aryl ist; und
das Verfahren bei Raumtemperatur durchgeführt wird.

**26.** Verfahren nach Anspruch 22, wobei
das Übergangsmetall Palladium ist; und
die Base ein Phosphat oder Fluorid ist.

**27.** Verfahren nach Anspruch 22, wobei
das Übergangsmetall Palladium ist; und
die Base Kaliumphosphat ist.

**28.** Verfahren nach Anspruch 22, wobei X von ArX Chlorid ist.

**29.** Verfahren nach Anspruch 22, wobei M $B(R')_2$ darstellt.

**30.** Verfahren der verallgemeinerten α-Arylierungsreaktion, die in Schema 4 dargestellt ist:

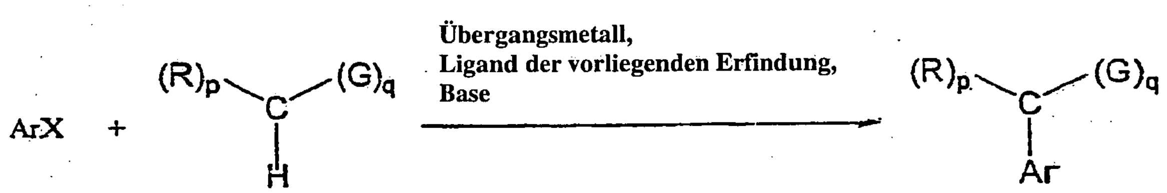


Schema 4

wobei

Ar aus gegebenenfalls substituierten monocyclischen und polycyclischen aromatischen und heteroaromatischen Einheiten ausgewählt ist;
X aus Cl, Br, I, $-OS(O)_2$-Alkyl und $-OS(O)_2$-Aryl ausgewählt ist;
G bei jedem Vorkommen unabhängig voneinander einen elektronenziehenden Rest darstellt, ausgewählt aus Formyl, Acyl, -CN, -C(O)OR, $-C(O)NR_2$, Nitro, Nitroso, $-S(O)_2R$, $-SO_3R$, $-S(O)_2NR_2$, -C(NR)-R, -C(NOR)-R und $-C(NNR_2)$-R;
R bei jedem Vorkommen unabhängig voneinander Wasserstoff, Alkyl, Aryl, Heteroalkyl, Heteroaryl, Halogen, Alkylamino, Arylamino, Alkylthio, Arylthio, Alkoxy, Aryloxy oder $-(CH_2)_m-R_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig eine ganze Zahl im Bereich von 0 bis 8 einschließlich ist;
q eine ganze Zahl im Bereich von 1 bis 3 einschließlich ist;
p eine ganze Zahl von gleich (3-q) ist;
Ar und ein Vorkommen von R kovalent gebunden sein können, so dass die Reaktion intramolekular ist;

das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**31.** Verfahren nach Anspruch 30, wobei der Ligand kovalent an einen festen Träger oder an ein lösliches Polymer gebunden ist oder auf einen Feststoff adsorbiert ist.

**32.** Verfahren nach Anspruch 30, wobei der Ligand chiral und nicht racemisch ist; und das Produkt nicht racemisch ist.

**33.** Verfahren nach Anspruch 30, wobei q 1 ist.

**34.** Verfahren nach Anspruch 30, wobei q 2 ist.

**35.** Verfahren nach Anspruch 30, wobei:

X von ArX Cl, $-OS(O)_2$-Alkyl oder $-OS(O)_2$-Aryl ist; und
das Verfahren bei Raumtemperatur durchgeführt wird.

**36.** Verfahren nach Anspruch 30, wobei:

das Übergangsmetall Palladium ist; und
die Base ein Phosphat oder Fluorid ist.

**37.** Verfahren nach Anspruch 30, wobei:

das Übergangsmetall Palladium ist; und
die Base Kaliumphosphat ist.

**38.** Verfahren nach Anspruch 30, wobei:

X von ArX Br darstellt; und
die Reaktion bei Raumtemperatur stattfindet.

**39.** Verfahren nach Anspruch 30, wobei X von ArX Chlorid ist.

**40.** Verfahren der verallgemeinerten α-Vinylierungsreaktion, die in Schema 5 dargestellt ist:

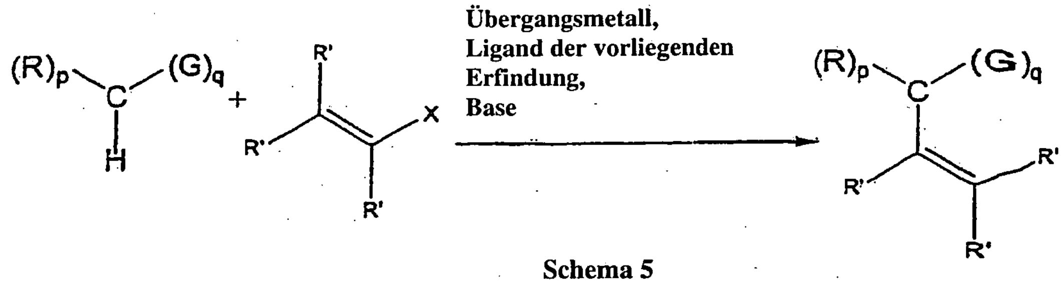


Schema 5

wobei

X aus Cl, Br, I, $-OS(O)_2$-Alkyl und $-OS(O)_2$-Aryl ausgewählt ist;
G bei jedem Vorkommen unabhängig voneinander einen elektronenziehenden Rest darstellt, ausgewählt aus Formyl, Acyl, -CN, -C(O)OR, $-C(O)NR_2$, Nitro, Nitroso, $-S(O)_2R$, $-SO_3R$, $-S(O)_2NR_2$, -C(NR)-R, -C(NOR)-R und $-C(NNR_2)$-R;
R bei jedem Vorkommen unabhängig voneinander Wasserstoff, Alkyl, Aryl, Heteroalkyl, Heteroaryl, Halogen,

Alkylamino, Arylamino, Alkylthio, Arylthio, Alkoxy, Aryloxy oder $-(CH_2)_m-R_{80}$ darstellt;
R' bei jedem Vorkommen unabhängig voneinander Wasserstoff, Alkyl, Aryl, Aralkyl, Heteroalkyl, Heteroaryl, Heteroaralkyl, Alkylamino, Arylamino, Alkylthio, Arylthio, Alkoxy, Aryloxy oder$-(CH_2)_m-R_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig voneinander eine ganze Zahl im Bereich von 0 bis 8 einschließlich ist;
q eine ganze Zahl im Bereich von 1 bis 3 einschließlich ist;
p eine ganze Zahl von gleich (3-q) ist;
ein Vorkommen von R und ein Vorkommen von R' kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**41.** Verfahren nach Anspruch 40, wobei der Ligand kovalent an einen festen Träger oder an ein lösliches Polymer gebunden ist oder auf einen Feststoff adsorbiert ist.

**42.** Verfahren nach Anspruch 40, wobei der Ligand chiral und nicht racemisch ist; und das Produkt nicht racemisch ist.

**43.** Verfahren nach Anspruch 40, wobei q 1 ist.

**44.** Verfahren nach Anspruch 40, wobei q 2 ist.

**45.** Verfahren nach Anspruch 40, wobei:

X von $(R')_2CC(R')X$ $-OS(O)_2$-Alkyl oder $-OS(O)_2$-Aryl ist; und
das Verfahren bei Raumtemperatur durchgeführt wird.

**46.** Verfahren nach Anspruch 40, wobei:

das Übergangsmetall Palladium ist; und
die Base ein Phosphat oder Fluorid ist.

**47.** Verfahren nach Anspruch 40, wobei:

das Übergangsmetall Palladium ist; und
die Base Kaliumphosphat ist.

**48.** Verfahren nach Anspruch 40, wobei:

X von $(R')_2CC(R')X$ Br darstellt; und
die Reaktion bei Raumtemperatur stattfindet.

**49.** Verfahren nach Anspruch 40, wobei X von $(R')_2CC(R')X$ Chlorid ist.

**50.** Verfahren der verallgemeinerten O-Arylierungsreaktion, die in Schema 6 dargestellt ist:

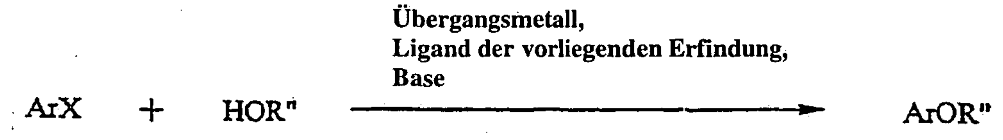


**Schema 6**

wobei

Ar aus gegebenenfalls substituierten monocyclischen und polycyclischen aromatischen und heteroaromatischen Einheiten ausgewählt ist;
X aus Cl, Br, I, $-OS(O)_2$-Alkyl und-$OS(O)_2$-Aryl ausgewählt ist;
R" bei jedem Vorkommen unabhängig voneinander Alkyl, Aryl, Aralkyl, Heteroalkyl, Heteroaryl, Heteroaralkyl, $-Si(Alkyl)_3$, $-Si(Aryl)_3$ oder $-(CH_2)_m-R_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig voneinander eine ganze Zahl im Bereich von 0 bis 8 einschließlich ist;
Ar und R" kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist;
die Base aus Carbonaten, Phosphaten, Fluoriden, Alkoxiden, Amiden, Carbanionen und Silylanionen ausgewählt ist; und
das Verfahren bei Raumtemperatur durchgeführt wird.

**51.** Verfahren nach Anspruch 50, wobei der Ligand kovalent an einen festen Träger oder an ein lösliches Polymer gebunden ist oder auf einen Feststoff adsorbiert ist.

**52.** Verfahren nach Anspruch 50, wobei R"OH ein primärer Alkohol ist.

**53.** Verfahren nach Anspruch 50, wobei:

X von ArX Cl, $-OS(O)_2$-Alkyl oder $-OS(O)_2$-Aryl ist.

**54.** Verfahren nach Anspruch 50, wobei:

das Übergangsmetall Palladium ist; und
die Base ein Phosphat oder Fluorid ist.

**55.** Verfahren nach Anspruch 50, wobei:

das Übergangsmetall Palladium ist; und
die Base Kaliumphosphat ist.

**56.** Verfahren nach Anspruch 50, wobei:

X von ArX Br darstellt.

**57.** Verfahren nach Anspruch 50, wobei X von ArX Chlorid ist.

**58.** Verfahren der verallgemeinerten O-Vinylierungsreaktion, die in Schema 7 dargestellt ist:

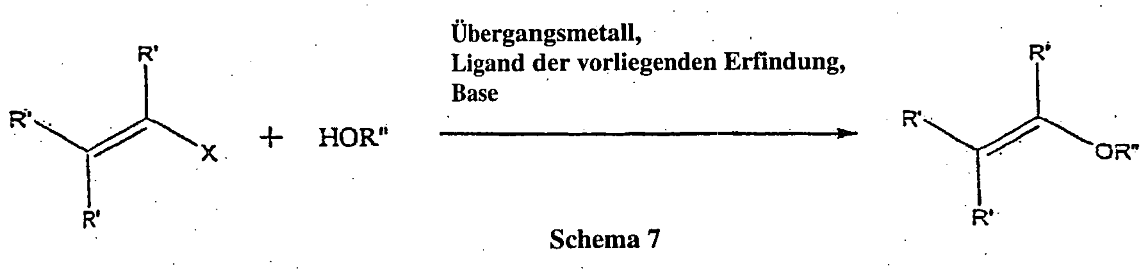


Schema 7

wobei

X aus Cl, Br, I, $-OS(O)_2$-Alkyl und $-OS(O)_2$-Aryl ausgewählt ist;

R' bei jedem Vorkommen unabhängig voneinander Wasserstoff, Alkyl, Aryl, Aralkyl, Heteroalkyl, Heteroaryl, Heteroaralkyl, Alkylamino, Arylamino, Alkylthio, Arylthio, Alkoxy, Aryloxy oder $-(CH_2)_m-R_{80}$ darstellt;
R" bei jedem Vorkommen unabhängig voneinander Alkyl, Aryl, Aralkyl, Heteroalkyl, Heteroaryl, Heteroaralkyl, $-Si(Alkyl)_3$, $-Si(Aryl)_3$ oder $-(CH_2)_mR_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig voneinander eine ganze Zahl im Bereich von 0 bis 8 einschließlich ist;
R" und ein Vorkommen von R' kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**59.** Verfahren nach Anspruch 58, wobei der Ligand kovalent an einen festen Träger oder an ein lösliches Polymer gebunden ist oder auf einen Feststoff adsorbiert ist.

**60.** Verfahren nach Anspruch 58, wobei R"OH ein primärer Alkohol ist.

**61.** Verfahren nach Anspruch 58, wobei:

X von $(R')_2CC(R')X$ $-OS(O)_2$-Alkyl oder $-OS(O)_2$-Aryl ist; und
das Verfahren bei Raumtemperatur durchgeführt wird.

**62.** Verfahren nach Anspruch 58, wobei:

das Übergangsmetall Palladium ist; und
die Base ein Phosphat oder Fluorid ist.

**63.** Verfahren nach Anspruch 58, wobei:

das Übergangsmetall Palladium ist; und
die Base Kaliumphosphat ist.

**64.** Verfahren nach Anspruch 58, wobei:

X von $(R')_2CC(R')X$ Br darstellt; und
die Reaktion bei Raumtemperatur stattfindet.

**65.** Verfahren nach Anspruch 58, wobei X von $(R')_2CC(R')X$ Chlorid ist.

**66.** Verfahren der verallgemeinerten Heck-Reaktion, die in Schema 8 dargestellt ist:

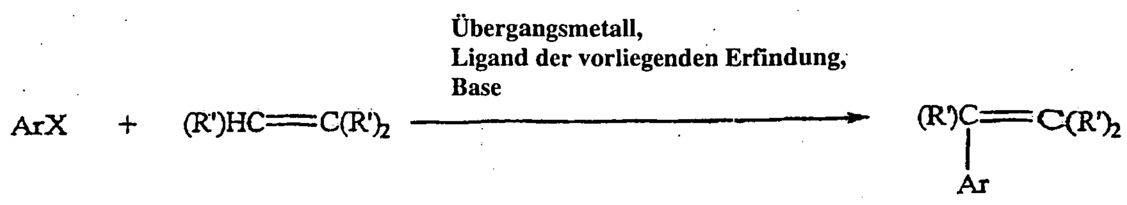


**Schema 8**

wobei

Ar aus gegebenenfalls substituierten aromatischen, heteroaromatischen Einheiten und Alkenyleinheiten ausgewählt ist;

X aus Cl, Br, I, $-OS(O)_2$-Alkyl und $-OS(O)_2$-Aryl ausgewählt ist;
R' bei jedem Vorkommen unabhängig voneinander Wasserstoff, Alkyl, Aryl, Aralkyl, Heteroalkyl, Heteroaryl, Heteroaralkyl, Alkylamino, Arylamino, Alkylthio, Arylthio, Alkoxy, Aryloxy oder $-(CH_2)_m-R_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig voneinander eine ganze Zahl im Bereich von 0 bis 8 einschließlich ist;
Ar und ein Vorkommen von R' kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und
die Base aus Carbonaten, Phosphaten, Fluoriden, Alkoxiden, Amiden, Carbanionen und Silylanionen ausgewählt ist.

**67.** Verfahren nach Anspruch 66, wobei der Ligand kovalent an einen festen Träger oder an ein lösliches Polymer gebunden ist oder auf einen Feststoff adsorbiert ist.

**68.** Verfahren nach Anspruch 66, wobei:

X von ArX Cl, $-OS(O)_2$-Alkyl oder $-OS(O)_2$-Aryl ist; und
das Verfahren bei Raumtemperatur durchgeführt wird.

**69.** Verfahren nach Anspruch 66, wobei:

das Übergangsmetall Palladium ist; und
die Base ein Phosphat oder Fluorid ist.

**70.** Verfahren nach Anspruch 66, wobei:

das Übergangsmetall Palladium ist; und
die Base Kaliumphosphat ist.

**71.** Verfahren der verallgemeinerten Heck-Reaktion, die in Schema 9 dargestellt ist:

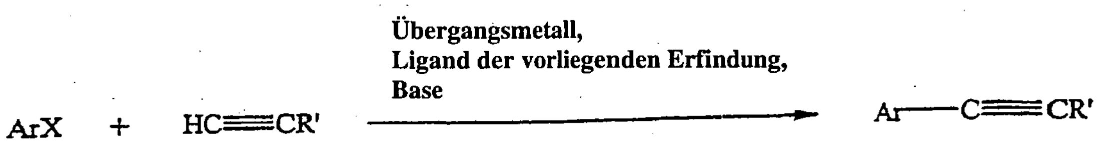


Schema 9

wobei

Ar aus gegebenenfalls substituierten aromatischen, heteroaromatischen Einheiten und Alkenyleinheiten ausgewählt ist;
X aus Cl, Br, I, $-OS(O)_2$-Alkyl und $-OS(O)_2$-Aryl ausgewählt ist;
R' bei jedem Vorkommen unabhängig voneinander Wasserstoff, Alkyl, Aryl, Aralkyl, Heteroalkyl, Heteroaryl, Heteroaralkyl, Alkylamino, Arylamino, Alkylthio, Arylthio, Alkoxy, Aryloxy oder $-(CH_2)_m-R_{80}$ darstellt;
$R_{80}$ bei jedem Vorkommen unabhängig voneinander ein substituiertes oder unsubstituiertes Aryl, Cycloalkyl, Cycloalkenyl, einen Heterocyclus oder einen Polycyclus darstellt;
m bei jedem Vorkommen unabhängig voneinander eine ganze Zahl im Bereich von 0 bis 8 einschließlich ist;
Ar und R' kovalent gebunden sein können, so dass die Reaktion intramolekular ist;
das Übergangsmetall aus Metallen der Gruppen 5 bis 12, vorzugsweise Metallen der Gruppe VIIIA, ausgewählt ist;
der Ligand durch die allgemeine Struktur 7 dargestellt ist; und

die Base aus Carbonaten, Phosphaten, Fluoriden, Alkoxiden, Amiden, Carbanionen und Silylanionen ausgewählt ist.

**72.** Verfahren nach Anspruch 71, wobei der Ligand kovalent an einen festen Träger oder an ein lösliches Polymer gebunden ist oder auf einen Feststoff adsorbiert ist.

**73.** Verfahren nach Anspruch 71, wobei:

X von ArX Cl, $-OS(O)_2$-Alkyl oder $-OS(O)_2$-Aryl ist; und
das Verfahren bei Raumtemperatur durchgeführt wird.

**74.** Verfahren nach Anspruch 71, wobei:

das Übergangsmetall Palladium ist; und
die Base ein Phosphat oder Fluorid ist.

**75.** Verfahren nach Anspruch 71, wobei:

das Übergangsmetall Palladium ist; und
die Base Kaliumphosphat ist.

**76.** Verfahren nach einem der Ansprüche 5, 12, 22, 30, 40, 50 oder 58, wobei weniger als 0,01 Mol-% des Katalysators, bezogen auf das beschränkende Reagens, verwendet wird.

**77.** Verfahren nach einem der Ansprüche 5, 12, 22, 30, 40, 50 oder 58, wobei weniger als 0,0001 Mol-% des Katalysators, bezogen auf das beschränkende Reagens, verwendet wird.

**78.** Verfahren nach einem der Ansprüche 5, 12, 22, 30, 40, 50 oder 58, wobei weniger als 0,000001 Mol-% des Katalysators, bezogen auf das beschränkende Reagens, verwendet wird.

## Revendications

**1.** Composé représenté par la structure générale 7 :

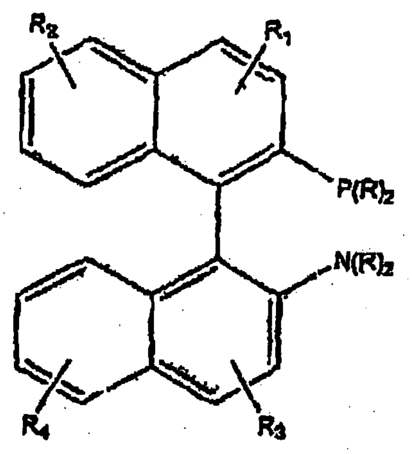

7

dans laquelle

R est choisi, indépendamment pour chaque occurrence, dans l'ensemble constitué d'un groupe alkyle, hétéroalkyle, cycloalkyle, hétérocycloalkyle, aryle, hétéroaryle, aralkyle, hétéroaralkyle et $-(CH_2)_m-R_{80}$ ;

les cycles A, B, A' et B' du noyau binaphtyle peuvent indépendamment être non substitués ou substitués par $R_1$, $R_2$, $R_3$ et $R_4$ respectivement, tout nombre de fois jusqu'aux limitations imposées par la stabilité et les règles de valence ;
$R_1$, $R_2$, $R_3$ et $R_4$ sont choisis, indépendamment pour chaque occurrence, dans l'ensemble constitué d'un groupe alkyle, hétéroalkyle, cycloalkyle, hétérocycloalkyle, aryle, hétéroaryle, aralkyle, hétéroaralkyle, d'un atome d'halogène, $-SiR_3$ et $-(CH_2)_m-R_{80}$;
$R_{80}$ représente un groupe aryle non substitué ou substitué, un cycloalkyle, un cycloalcényle, un hétérocycle ou un polycycle ;
m est un nombre entier dans la gamme de 0 à 8 inclus ; et
le ligand, lorsqu'il est chiral, peut être fourni sous la forme d'un mélange d'énantiomères ou en tant qu'énantiomère unique.

**2.** Composé selon la revendication 1 , dans lequel :

$R_1$, $R_2$, $R_3$ et $R_4$ sont absents;
les deux cas de R sur le N illustrés explicitement sont un groupe alkyle inférieur, de préférence un groupe méthyle ; et
les deux cas de R sur le P illustrés explicitement sont un groupe cycloalkyle, de préférence un groupe cyclohexyle.

**3.** Composé selon la revendication 1, dans lequel le groupe $PR_2$ comprend un P asymétrique.

**4.** Composé selon la revendication 1, dans lequel le groupe $PR_2$ comprend un P asymétrique ; et le noyau binaphtyle est axialement chiral.

**5.** Composé représenté par la réaction généralisée illustrée sur le schéma I :

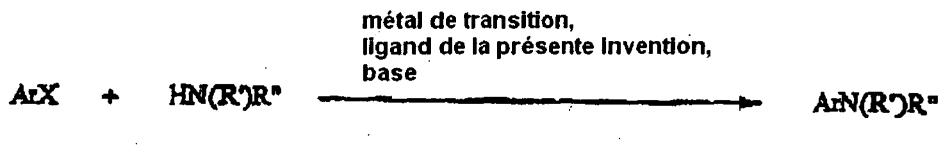


**Schéma 1**

dans lequel

Ar est choisi dans l'ensemble constitué de fractions aromatiques et hétéroaromatiques, monocycliques et polycycliques facultativement substituées ;
X est choisi dans l'ensemble constitué de Cl, Br, I, $-OS(O)_2$alkyle et $-OS(O)_2$aryle ;
R' et R" sont choisis, indépendamment pour chaque occurrence, dans l'ensemble constitué de H, d'un groupe alkyle, hétéroalkyle, aryle, hétéroaryle, aralkyle, alcoxyle, amino, trialkylsilyle et triarylsilyle ;
R' et R", pris ensemble, peuvent former un cycle facultativement substitué constitué de 3 à 10 atomes de squelette inclus ; ledit cycle comprenant facultativement un ou plusieurs hétéroatomes en plus de l'azote auquel R' et R" sont liés ;
R' et/ou R" peuvent être liés de manière covalente à Ar de telle sorte que la réaction d'amination est intramoléculaire ;
le métal de transition est choisi dans l'ensemble constitué des métaux des groupes 5 à 12, de préférence des métaux du groupe VIIIA ;
le ligand est représenté par la structure générale 7 ; et
la base est choisie parmi l'ensemble constitué des hydrures, des carbonates, des fluorures, des phosphates, des alcoxydes, des phénôxydes, des amides, des carbanions et des anions silyle.

**6.** Procédé selon la revendication 5, dans lequel le ligand est lié de manière covalente à un support solide ou un polymère solide, ou est adsorbé sur un solide.

**7.** Procédé selon la revendication 5, dans lequel :

X de ArX est Cl, $-OS(O)_2$alkyle ou $-OS(O)_2$aryle; et

le procédé est effectué à température ambiante.

**8.** Procédé selon la revendication 5, dans lequel

le métal de transition est le palladium ; et
la base est un phosphate ou un fluorure.

**9.** Procédé selon la revendication 5, dans lequel

le métal de transition est le palladium ; et
la base est le phosphate de potassium.

**10.** Procédé selon la revendication 5, dans lequel HN(R')R" représente un composé hétéroaromatique facultativement substitué.

**11.** Procédé selon la revendication 5, dans lequel X de ArX est un chlorure.

**12.** Procédé représenté la réaction de couplage généralisée illustrée sur le schéma 2 :

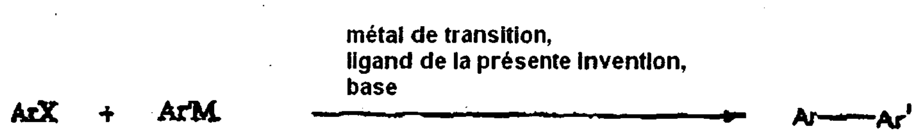


**Schéma 2**

dans lequel

Ar et Ar' sont indépendamment choisis dans l'ensemble constitué de fractions aromatiques, hétéroaromatiques et alcényle facultativement substituées ;
X est choisi dans l'ensemble constitué de Cl, Br, I, $-OS(O)_2$alkyle et $-OS(O)_2$aryle ;
M représente $B(OR)_2$, Mg(halogénure) ou Zn(halogénure);
R représente indépendamment pour chaque occurrence H, un groupe méthyle, alkyle, hétéroalkyle, aryle ou hétéroaryle ; ou les deux exemples de R dans une occurrence de $B(OR)_2$, pris ensemble, peuvent représenter une chaîne à deux ou trois atomes de carbone facultativement substituée entre les deux cas de O;
Ar et Ar' peuvent être liés de manière covalente de telle sorte que la réaction est intramoléculaire ;
le métal de transition est choisi dans l'ensemble constitué des métaux des groupes 5 à 12, de préférence les métaux du groupe VIIIA ;
le ligand est représenté par la structure générale 7; et
la base est choisie dans l'ensemble constitué des carbonates, des phosphates, des fluorures, des alcoxydes, des amides, des carbanions et des anions silyle.

**13.** Procédé selon la revendication 12, dans lequel le ligand est lié de manière covalente à un support solide ou un polymère soluble, ou est adsorbé sur un solide.

**14.** Procédé selon la revendication 12, dans lequel le ligand est chiral ou non racémique ; et le produit est non racémique.

**15.** Procédé selon la revendication 12, dans lequel :

X de ArX est Cl, $-OS(O)_2$alkyle ou $-OS(O)_2$aryle ; et
le procédé est effectué à température ambiante.

**16.** Procédé selon la revendication 12, dans lequel

le métal de transition est le palladium ; et
la base est un phosphate ou un fluorure.

**17.** Procédé selon la revendication 12, dans lequel

le métal de transition est le palladium ; et
la base est le phosphate de potassium.

**18.** Procédé selon la revendication 12, dans lequel X de ArX est un chlorure.

**19.** Procédé selon la revendication 12, dans lequel pas plus d'un des quatre substituants ortho et ortho' de Ar-Ar' est un atome d'hydrogène.

**20.** Procédé selon la revendication 12, dans lequel X de ArX est un chlorure ; et pas plus d'un des quatre substituants ortho et ortho' de Ar-Ar' est un atome d'hydrogène.

**21.** Procédé selon la revendication 12, dans lequel M représente $B(OR)_2$.

**22.** Procédé représente par la réaction de couplage généralisée illustrée sur le schéma 3:

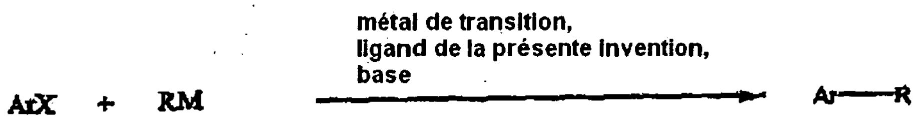


Schéma 3

dans lequel :

Ar est choisi dans l'ensemble constitué de fractions aromatiques, hétéroaromatiques et alcényle facultativement substituées ;
R est choisi dans l'ensemble constitué des groupes alkyle, hétéroalkyle et aralkyle facultativement substitués ;
R' est choisi, indépendamment pour chaque occurrence, dans l'ensemble constitué des groupes alkyle et hétéroalkyle ; la liaison carbone-bore desdits groupes alkyle et hétéroalkyle étant inerte dans les conditions de réaction, par exemple, $BR'_2$ pris ensemble représente le 9-borobicyclo[3.3.1]nonylé ;
M représente $B(R')_2$, Mg(halogénure) ou Zn(halogénure) ;
X de ArX est choisi dans l'ensemble constitué de Cl, Br, I, $-OS(O)_2$alkyle et $-OS(O)_2$aryle ;
Ar et R peuvent être liés de manière covalente de telle sorte que la réaction est intramoléculaire ;
le métal de transition est choisi dans l'ensemble constitué des métaux des groupes 5 à 12, de préférence les métaux du groupe VIIIA ;
le ligand est représenté par la structure générale 7 ; et
la base est choisie parmi l'ensemble constitué des hydrures, des carbonates, des phosphates, des fluorures, des alcoxydes, des amides, des carbanions et des anions silyle.

**23.** Procédé selon la revendication 22, dans lequel le ligand est lié de manière covalente à un support solide ou un polymère soluble, ou est adsorbé sur un solide.

**24.** Procédé selon la revendication 22, dans lequel le ligand est chiral et non racémique; et le produit est non racémique.

**25.** Procédé selon la revendication 22, dans lequel:

X de ArX est Cl, $-OS(O)_2$alkyle ou $-OS(O)_2$aryle ; et
le procédé est effectué à température ambiante.

**26.** Procédé selon la revendication 22, dans lequel

le métal de transition est le palladium ; et
la base est un phosphate ou un fluorure.

**27.** Procédé selon la revendication 22, dans lequel

le métal de transition est le palladium ; et
la base est le phosphate de potassium.

**28.** Procédé selon la revendication 22, dans lequel X de ArX est un chlorure.

**29.** Procédé selon la revendication 22, dans lequel M représente $B(R')_2$.

**30.** Procédé représenté par la réaction d'α-arylation généralisée illustrée sur le schéma 4 :

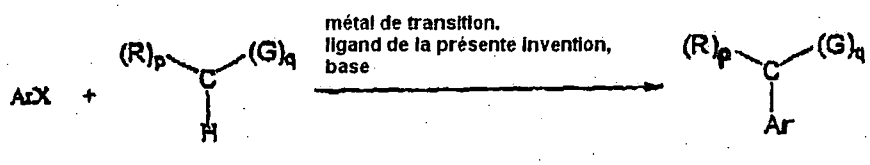


**Schéma 4**

dans lequel

Ar est choisi dans l'ensemble constitué de fractions aromatiques, et hétéroaromatiques, monocycliques et polycycliques facultativement substituées ;
X est choisi dans l'ensemble constitué de Cl, Br, I, $-OS(O)_2$alkyle et $=OS(O)_2$aryle ;
G représente, indépendamment pour chaque occurrence, un groupe attracteur d'électrons choisi dans le groupe constitué d'un groupe formyle, acyle, -CN, -C(O)OR, $-C(O)NR_2$; nitro, nitroso, $-S(O)_2R$, $-SO_3R$, $-S(O)_2NR_2$, -C(NR)-R, -C(NOR)-R et $-C(NNR_2)$- R ;
R représente, indépendamment pour chaque occurrence, un atome d'hydrogène, un groupe alkyle, aryle, héréroalkyle, héréroaryle, un atome d'halogène, un groupe alkylamino, arylamino, alkylthio, arylthio, alcoxy, aryloxy ou $-(CH_2)_m-R_{80}$ ;
$R_{80}$ représente, indépendamment pour chaque occurrence, un groupe aryle substitué ou non substitué, un groupe cycloalkyle, cycloalcényle, un hétérocycle ou un polycycle ;
m, indépendamment pour chaque occurrence, est un nombre entier choisi dans la gamme de 0 à 8 inclus;
q est un nombre entier choisi dans la gamme de 1 à 3 inclus;
p est un nombre entier égal à (3-q).
Ar et un cas de R peuvent être liés de manière covalente de sorte que la réaction est intramoléculaire ;
le métal de transition est choisi dans l'ensemble des métaux des groupes 5 à 12, de préférence les métaux du groupe VIIIA ;
le ligand est représenté par la structure générale 7; et
la base est choisie dans l'ensemble constitué des carbonates, des phosphates, des fluorures, des alcoxydes, des amides, des carbanions et des anions silyle.

**31.** Procédé selon la revendication 30, dans lequel le ligand est lié de manière covalente à un support solide ou un polymère soluble, ou est adsorbé sur un solide.

**32.** Procédé selon la revendication 30, dans lequel le ligand est chiral ou non racémique ; et le produit est non racémique.

**33.** Procédé selon la revendication 30, dans lequel q vaut 1.

**34.** Procédé selon la revendication 30, dans lequel q vaut 2.

**35.** Procédé selon la revendication 30, dans lequel:

X de ArX est Cl, $-OS(O)_2$alkyle ou $-OS(O)_2$aryle; et
le procédé est effectué à température ambiante.

**36.** Procédé selon la revendication 30, dans lequel :

le métal de transition est le palladium ; et
la base est un phosphate ou un fluorure.

**37.** Procédé selon la revendication 30, dans lequel

le métal de transition est le palladium ; et
la base est le phosphate de potassium.

**38.** Procédé selon la revendication 30, dans lequel:

X de ArX représente Br ; et
la réaction a lieu à température ambiante.

**39.** Procédé selon la revendication 30, dans lequel X de ArX est un chlorure.

**40.** Procédé représenté par la réaction d'$\alpha$-vinylation généralisée illustrée sur le schéma 5 :

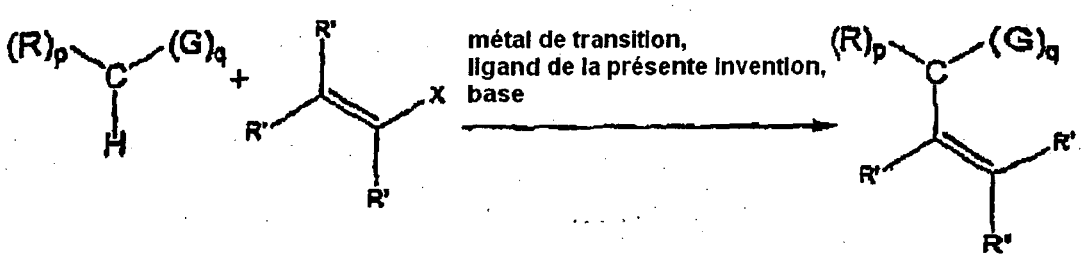


Schéma 5

dans lequel

X est choisi dans l'ensemble constitué de Cl, Br, I, $-OS(O)_2$alkyle et $-OS(O)_2$aryle;
G représente, indépendamment pour chaque occurrence, un groupe attracteur d'électrons choisi dans le groupe constitué d'un groupe formyle, acyle, -CN, -C(O)OR, $-C(O)NR_2$, nitro, nitroso, $-S(O)_2R$, $-SO_3R$, $-S(O)_2NR_2$, -C(NR)-R, -C(NOR)-R et $-C(NNR_2)$- R ;
R représente, indépendamment pour chaque occurrence, un atome d'hydrogène, un groupe alkyle, aryle, héréroalkyle, héréroaryle, un atome d'halogène, un groupe alkylamino, arylamino, alkylthio, arylthio, alcoxy, aryloxy ou $-(CH_2)_m-R_{80}$;
R' représente, indépendamment pour chaque occurrence, un atome d'hydrogène, un groupe alkyle, aryle, aralkyle, hétéroalkyle, hétéroaryle, hétéroaralkyle, alkylamino, arylamino, alkylthio, arylthio, alcoxy, aryloxy ou $-(CH_2)_m-R_{80}$;
$R_{80}$ représente, indépendamment pour chaque occurrence, un groupe aryle substitué ou non substitué, un groupe cycloalkyle, cycloalcényle, un hétérocycle ou un polycycle ;
m, indépendamment pour chaque occurrence, est un nombre entier choisi dans la gamme de 0 à 8 inclus;
q est un nombre entier choisi dans la gamme de 1 à 3 inclus;
p est un nombre entier égal à (3-q).
un cas de R et un cas de R' peuvent être liés de manière covalente de sorte que la réaction est intramoléculaire ;
le métal de transition est choisi dans l'ensemble des métaux des groupes 5 à 12, de préférence les métaux du groupe VIIIA ;
le ligand est représenté par la structure générale 7 ; et
la base est choisie dans l'ensemble constitué des carbonates, des phosphates, des fluorures, des alcoxydes, des amides, des carbanions et des anions silyle.

**41.** Procédé selon la revendication 40, dans lequel le ligand est lié de manière covalente à un support solide ou un polymère soluble, ou est adsorbé sur un solide.

**42.** Procédé selon la revendication 40, dans lequel le ligand est chiral ou non racémique ; et le produit est non racémique.

**43.** Procédé selon la revendication 40, dans lequel q vaut 1.

**44.** Procédé selon la revendication 40, dans lequel q vaut 2.

**45.** Procédé selon la revendication 40, dans lequel :

X de $(R')_2CC(R')X$ est $-OS(O)_2$alkyle ou $-OS(O)_2$aryle ; et
le procédé est effectué à température ambiante.

**46.** Procédé selon la revendication 40, dans lequel :

le métal de transition est le palladium ; et
la base est un phosphate ou un fluorure.

**47.** Procédé selon la revendication 40, dans lequel

le métal de transition est le palladium ; et
la base est le phosphate de potassium.

**48.** Procédé selon la revendication 40, dans lequel :

X de $(R')_2CC(R')X$ représente Br ; et
la réaction a lieu à température ambiante.

**49.** Procédé selon la revendication 40, dans lequel X de $(R')_2CC(R')X$ est un chlorure.

**50.** Procédé représenté par la réaction d'O-arylation généralisée illustrée sur le schéma 6:

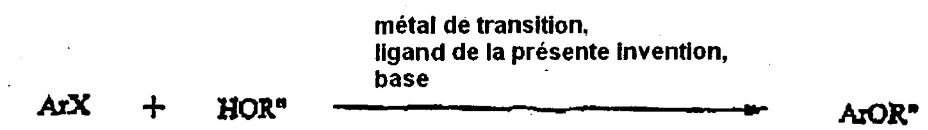


**Schéma 6**

dans lequel

Ar est choisi dans l'ensemble constitué de fractions aromatiques et hétéroaromatiques, monocycliques et polycycliques facultativement substituées ;
X est choisi dans l'ensemble constitué de Cl, Br, I, $-OS(O)_2$alkyle et $-OS(O)_2$aryle ;
R" représente, indépendamment pour chaque occurrence, un groupe alkyle, aryle, aralkyle, hétéroalkyle, hétéroaryle, hétéroaralkyle, $-Si(alkyle)_3$, $-Si(aryle)_3$ ou $-(CH_2)_m-R_{80}$ ;
$R_{80}$ représente, indépendamment pour chaque occurrence, un groupe aryle substitué ou non substitué, un groupe cycloalkyle, cycloalcényle, un hétérocycle ou polycyclique ;
m, indépendamment pour chaque occurrence, est un nombre entier choisi dans la gamme de 0 à 8 inclus ;
Ar et R" peuvent être liés de manière covalente de telle sorte que la réaction est intramoléculaire ;
le métal de transition est choisi dans l'ensemble des métaux des groupes 5 à 12, de préférence les métaux du groupe VIIIA ;
le ligand est représenté par la structure générale 7 ; et
la base est choisie dans l'ensemble constitué des carbonates, des phosphates, des fluorures, des alcoxydes, des amides, des carbanions et des anions silyle ; et
le procédé est effectué à température ambiante.

**51.** Procédé selon la revendication 50, dans lequel le ligand est lié de manière covalente à un support solide ou un polymère soluble; ou est adsorbé sur un solide.

**52.** Procédé selon la revendication 50, dans lequel R"OH est un alcool primaire.

**53.** Procédé selon la revendication 50, dans lequel :

X de ArX est Cl, $-OS(O)_2$alkyle ou $-OS(O)_2$aryle.

**54.** Procédé selon la revendication 50, dans lequel:

le métal de transition est le palladium ; et
la base est un phosphate ou un fluorure.

**55.** Procédé selon la revendication 50, dans lequel

le métal de transition est le palladium ; et
la base est le phosphate de potassium.

**56.** Procédé selon la revendication 50, dans lequel :

X de ArX représente Br.

**57.** Procédé selon la revendication 50, dans lequel X de ArX est un chlorure.

**58.** Procédé représenté par la réaction de O-vinylation généralisée illustrée sur le schéma 7 :

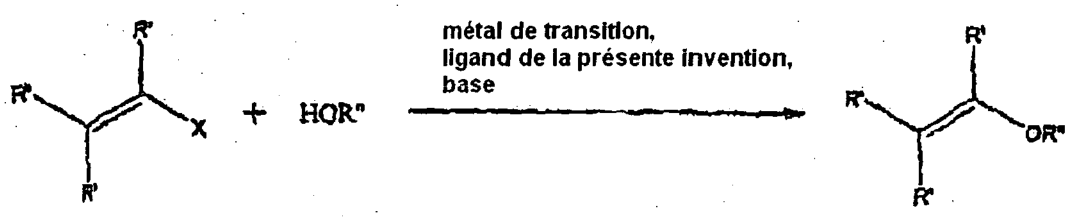


Schéma 7

dans lequel

X est choisi dans l'ensemble constitué de Cl, Br, I, $-OS(O)_2$alkyle et $-OS(O)_2$aryle ;
R' représente, indépendamment pour chaque occurrence, un atome d'hydrogène, un groupe alkyle, aryle, aralkyle, hétéroalkyle, hétéroaryle, hétéroaralkyle, alkylamino, arylamino, alkylthio, arylthio, alcoxy, aryloxy ou $-(CH_2)_m-R_{80}$;
R" représente, indépendamment pour chaque occurrence, un groupe alkyle, aryle, aralkyle, hétéroalkyle, hétéroaryle, hétéroaralkyle, -Si(alkyle)$_3$, -Si(aryle)$_3$ ou $-(CH_2)_m-R_{80}$;
$R_{80}$ représente, indépendamment pour chaque occurrence, un groupe aryle substitué ou non substitué, un groupe cycloalkyle, cycloalcényle, un hétérocycle ou polycycle ;
m, indépendamment pour chaque occurrence, est un nombre entier choisi dans la gamme de 0 à 8 inclus ;
R" et un cas de R' peuvent être liés de manière covalente de telle sorte que la réaction est intramoléculaire ;
le métal de transition est choisi dans l'ensemble des métaux des groupes 5 à 12, de préférence les métaux du groupe VIIIA ;
le ligand est représenté par la structure générale 7 ; et
la base est choisie dans l'ensemble constitué des carbonates, des phosphates, des fluorures, des alcoxydes, des amides, des carbanions et des anions silyle.

**59.** Procédé selon la revendication 58, dans lequel le ligand est lié de manière covalente à un support solide ou un polymère soluble, ou est adsorbé sur un solide.

**60.** Procédé selon la revendication 58, dans lequel R"OH est un alcool primaire.

**61.** Procédé selon la revendication 58, dans lequel :

X de $(R')_2CC(R')X$ est $-OS(O)_2$alkyle ou $-OS(O)_2$aryle ; et
le procédé est effectué à température ambiante.

**62.** Procédé selon la revendication 58, dans lequel:

le métal de transition est le palladium ; et
la base est un phosphate ou un fluorure.

**63.** Procédé selon la revendication 58 dans lequel

le métal de transition est le palladium ; et
la base est le phosphate de potassium.

**64.** Procédé selon la revendication 58, dans lequel :

X de $(R')_2CC(R')X$ représente Br ; et
la réaction a lieu à température ambiante.

**65.** Procédé selon la revendication 58, dans lequel X de $(R')_2CC(R')X$ est un chlorure.

**66.** Procédé représenté par la réaction de Heck généralisée illustrée sur le schéma 8 :

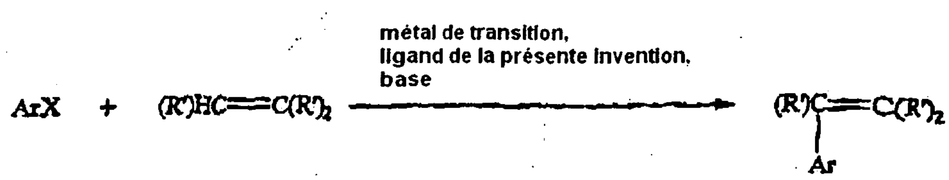


**Schéma 8**

dans lequel

Ar est- choisi dans l'ensemble constitué de fractions aromatiques, hétéroaromatiques et alcényle facultativement substituées ;
X est choisi dans l'ensemble constitué de Cl, Br, I, $-OS(O)_2$alkyle et $-OS(O)_2$aryle ;
R' représente, indépendamment pour chaque occurrence, un atome d'hydrogène, un groupe alkyle, aryle, aralkyle, hétéroalkyle, hétéroaryle, hétéroaralkyle, alkylamino, arylamino, alkylthio, arylthio, alcoxy, aryloxy ou $-(CH_2)_m-R_{80}$ ;
$R_{80}$ représente, indépendamment pour chaque occurrence, un groupe aryle substitué ou non substitué, un groupe cycloalkyle, cycloalcényle, un hétérocycle ou un polycycle ;
m, indépendamment pour chaque occurrence, est un nombre entier choisi dans la gamme de 0 à 8 inclus;
Ar et un cas de R' peuvent être liés de manière covalente de telle sorte que la réaction est intramoléculaire;
le métal de transition est choisi dans l'ensemble des métaux des groupes 5 à 12, de préférence les métaux du groupe VIIIA;
le ligand est représenté par la structure générale 7 ; et
la base est choisie dans l'ensemble constitué des carbonates, des phosphates, des fluorures, des alcoxydes, des amides, des carbanions et des anions silyle.

**67.** Procédé selon la revendication 66, dans lequel le ligand est lié de manière covalente à un support solide ou un polymère soluble, ou est adsorbé sur un solide.

**68.** Procédé selon la revendication 66, dans lequel :

X de ArX est Cl, $-OS(O)_2$alkyle ou $-OS(O)_2$aryle ; et

le procédé est effectué à température ambiante.

**69.** Procédé selon la revendication 66, dans lequel :

le métal de transition est le palladium ; et
la base est un phosphate ou un fluorure.

**70.** Procédé selon la revendication 66, dans lequel

le métal de transition est le palladium ; et
la base est le phosphate de potassium.

**71.** Procédé représenté par la réaction de Heck généralisée illustrée sur le schéma 9 :

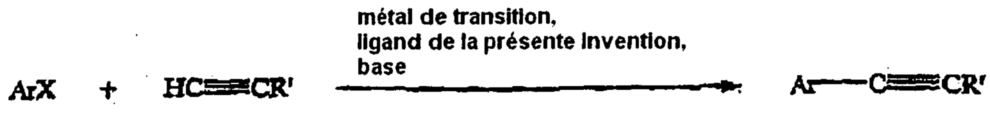


**Schéma 9**

dans lequel

Ar est choisi dans l'ensemble constitué de fractions aromatiques, hétéroaromatiques et alcényle facultativement substituées ;
X est choisi dans l'ensemble constitué de Cl, Br, I, $-OS(O)_2$alkyle et $-OS(O)_2$aryle ;
R' représente, indépendamment pour chaque occurrence, un atome d'hydrogène, un groupe alkyle, aryle, aralkyle, hétéroalkyle, hétéroaryle, hétéroaralkyle, alkylamino, arylamino, alkylthio, arylthio, alcoxy, aryloxy ou $-(CH_2)_m-R_{80}$ ;
$R_{80}$ représente, indépendamment pour chaque occurrence, un groupe aryle substitué ou non substitué, un groupe cycloalkyle, cycloalcényle, un hétérocycle ou un polycycle ;
m, indépendamment pour chaque occurrence, est un nombre entier choisi dans la gamme de 0 à 8 inclus;
Ar et R' peuvent être liés de manière covalente de telle sorte que la réaction est intramoléculaire;
le métal de transition est choisi dans l'ensemble des métaux des groupes 5 à 12, de préférence les métaux du groupe VIIIA ;
le ligand est représenté par la structure générale 7 ; et
la base est choisie dans l'ensemble constitué des carbonates, des phosphates, des fluorures, des alcoxydes, des amides, des carbanions et des anions silyle.

**72.** Procédé selon la revendication 71, dans lequel le ligand est lié de manière covalente à un support solide ou un polymère soluble, ou est adsorbé sur un solide.

**73.** Procédé selon la revendication 71, dans lequel :

X de ArX est Cl, $-OS(O)_2$alkyle ou $-OS(O)_2$aryle ; et
le procédé est effectué à température ambiante.

**74.** Procédé selon la revendication 71 , dans lequel :

le métal de transition est le palladium ; et
la base est un phosphate ou un fluorure.

**75.** Procédé selon la revendication 71, dans lequel

le métal de transition est le palladium ; et

la base est le phosphate de potassium.

**76.** Procédé selon l'une quelconque des revendications 5, 12, 22, 30, 40, 50 ou 58, dans lequel moins de 0,01 % en mol du catalyseur par rapport au réactif limitant est utilisé.

**77.** Procédé selon l'une quelconque des revendications 5, 12, 22, 30, 40, 50 ou 58, dans lequel moins de 0,0001 % en mol du catalyseur par rapport au réactif limitant est utilisé.

**78.** Procédé selon l'une quelconque des revendications 5, 12, 22, 30, 40, 50 où 58, dans lequel moins de 0,000001 % en mol du catalyseur par rapport au réactif limitant est utilisé.

**Figure 1.** Method of Preparation and Reactions Screened for Various Ligands.

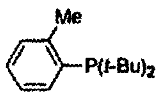
Li, Mg
S,A

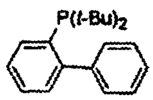
Li, Mg
S,A,K,D,H

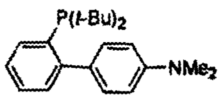
Mg
S,A

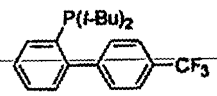
Mg
S,A,D

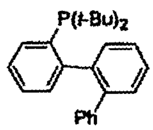
Mg
D,S

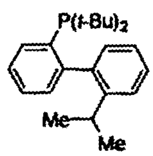
Mg
D,S

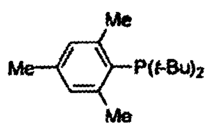
Mg
S

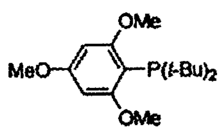
Li
S,A

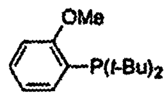
Li
S,A

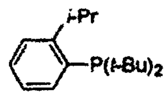
Mg
S,A

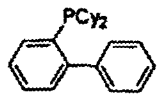
Li
S,A,K

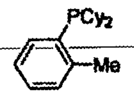
Li
S,A

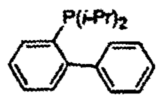
Li
S,A

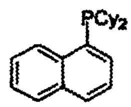
Li
A

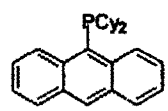
Li
A

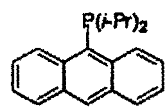
Li
A

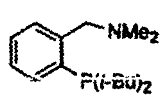
Li
S

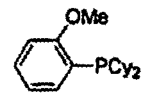
Li
A

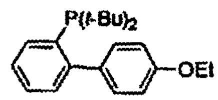
Mg
S

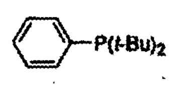
Mg
S,A

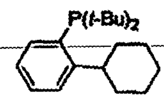
Mg
S,A

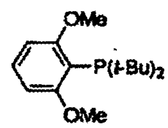
Li
S

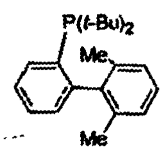
Mg
S,D

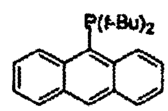
Li
A

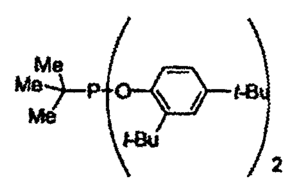
S

**Legend**

*Method of Preparation:*
Li= made from organolithium reagent
Mg= made from Grignard reagent

*Reactions Screened:*
S=Used for Suzuki Coupling
A=Used for amination
D=Used for diaryl ether synthesis
K=Used for ketone arylation
H=Used for Heck reaction

**Figure 2**

Ligands Referred to in Figures 3-11 and Examples 59-65

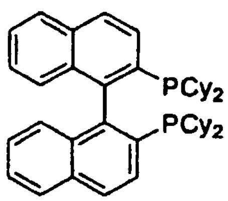

1

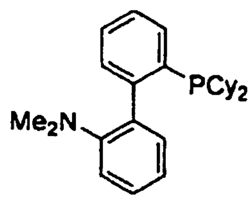

2

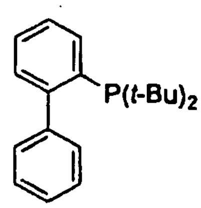

3

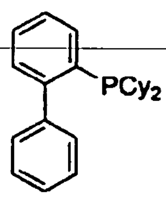

4

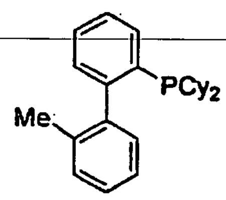

5

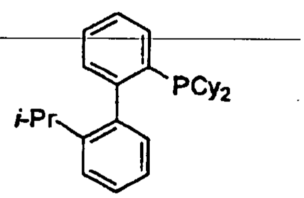

6

**Figure 3**

Room-Temperature Catalytic Aminations of Aryl Chlorides Using Ligand 3[a]

| Entry | Halide | Amine | Product | %Pd | Rxn Time | Yield (%) |
|---|---|---|---|---|---|---|
| 1 | 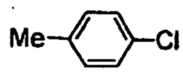 | 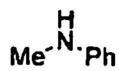 | 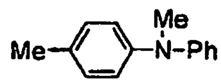 | 1.0 | 19 h | 98 |
| 2 | | 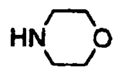 | 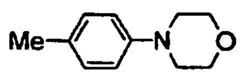 | 1.0 | 20 h | 94 |
| 3 | | $HNBu_2$ | 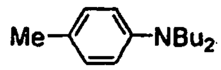 | 2.0 | 18 h | 81 |
| 4 | 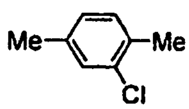 | 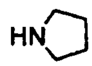 | 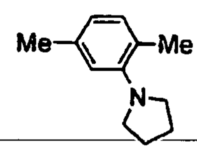 | 1.0 | 21 h | 98 |
| 5 | | $H_2NBn$ | 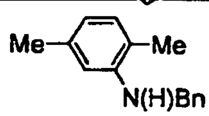 | 2.0 | 18 h | 99 |
| 6 | 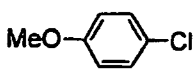 | 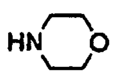 | 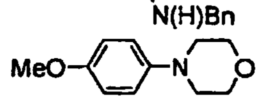 | 2.0 | 20 h | 90 |
| 7 | 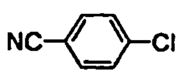 | 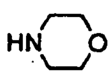 | 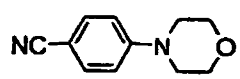 | 1.0 | 15 h | 86 |
| 8 | 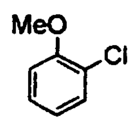 | $H_2NBn$ | 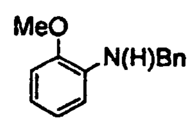 | 1.0 | 14 h | 99 |
| 9 | 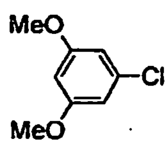 | 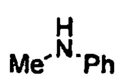 | 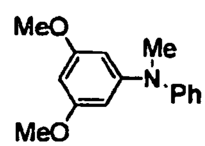 | 1.0 | 16 h | 97 |

(a) Reaction conditions: 1.0 equiv. aryl chloride, 1.2 equiv amine, 1.4 equiv NaOtBu, 1-2 mol % $Pd(OAc)_2$, 2-4 mol % 3, toluene (1 mL/mmol halide), rt. Reaction times have not been minimized. Yields represent isolated yields (average of two or more experiments) of compounds estimated to be >95 % pure as judged by $^1H$ NMR and GC analysis (known compounds) and combustion analysis (new compounds). (b) The reaction was run at 100 °C using $Pd_2(dba)_3$ in place of $Pd(OAc)_2$.

**Figure 4**

Palladium-catalyzed amination of unactivated aryl chlorides using ligand 3[a]

| Entry | Halide | Amine | Product | mol % Pd | Rxn Time | Yield (%) |
|---|---|---|---|---|---|---|
| 1 | 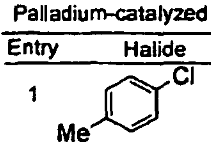 | $H_2NHex$ | 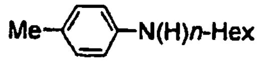 | 0.5 | 19 h | 85 |
| 2 | | 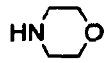 | 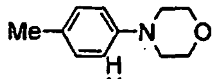 | 0.5 | 4 h | 93 |
| 3 | | 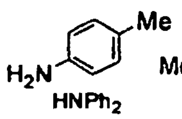 | 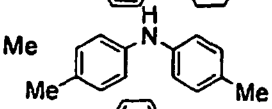 | 0.5 | 2.5 h | 90[b] |
| 4 | | $HNPh_2$ | 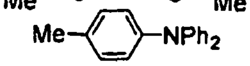 | 0.5 | 12 h | 90 |
| 5 | | $H_2NBn$ | 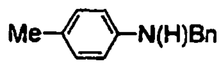 | 0.5 | 5 h | 89 |
| 6 | | $HNBu_2$ | 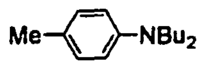 | 0.5/5<br>0.5/6 | 22 h<br>22 h | 89[b]<br>91[b] |
| 7 | | HN(Et)Ph | 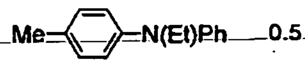 | 0.5 | 18 h | 93 |
| 8 | | 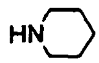 | 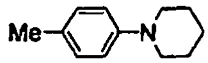 | 0.5 | 23 h | 86 |
| 9 | 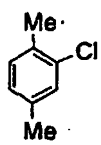 | 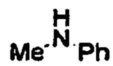 | 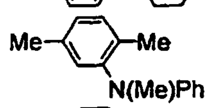 | 0.5 | 3 h | 90 |
| 10 | | $H_2NCy$ | 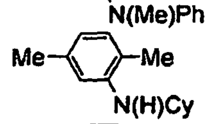 | 1.0 | 19 h | 98 |
| 11 | | 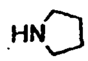 | 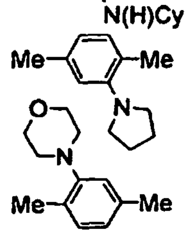 | 0.5 | 3h | 98 |
| 12 | | 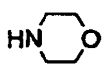 | | 0.5 | 24 h | 89 |
| 13 | | 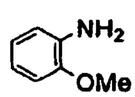 | 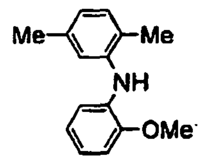 | 0.5 | 2.5 h | 97[b] |
| 14 | | $H_2NBn$ | 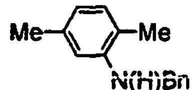 | 0.5 | 24 h | 96 |
| 15 | | 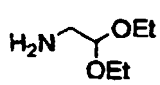 | 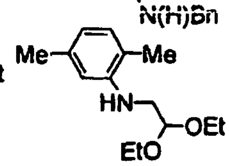 | 0.5 | 15 h | 100[b] |

(a) Reaction conditions: 1.0 equiv aryl halide, 1.2 equiv amine, 1.4 equiv NaO*t*Bu, cat. $Pd(OAc)_2$, cat 3 (2L/Pd), toluene (2mL/mmol halide), 80 °C. Reaction times have not been minimized. (b) $Pd_2(dba)_3$ used in place of $Pd(OAc)_2$; (c) The reaction was conducted at 100 °C; (d) The reaction was conducted at 110 °C.

## Figure 5

Palladium-catalyzed amination of unactivated aryl chlorides using ligand 3[a]

| Entry | Halide | Amine | Product | mol % Pd | Rxn Time | Yield (%) |
|---|---|---|---|---|---|---|
| 16 | 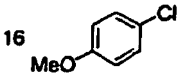 | 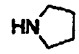 | 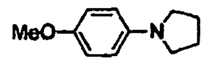 | 1.0 | 23 h | 92 |
| 17 | | 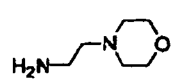 | 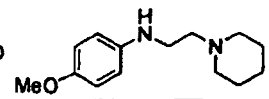 | 0.5 | 24 h | 86[d] |
| 18 | | 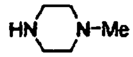 | 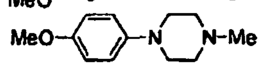 | 0.5/4 | 21 h | 82[c] |
| 19 | | 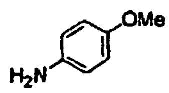 | 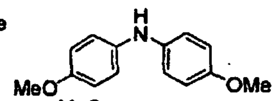 | 0.5 | 8 h | 94[b] |
| 20 | 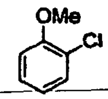 | 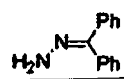 | 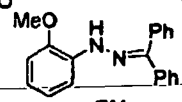 | 0.5 | 2.5 h | 91[b] |
| 21 | | $H_2NBn$ | 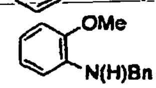 | 0.5 | 18 h | 96 |
| 22 | | 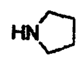 | 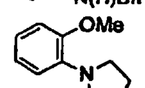 | 0.5 | 16 h | 88 |
| 23 | 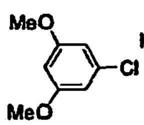 | 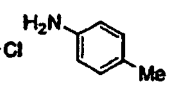 | 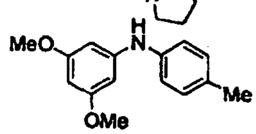 | 0.5 | 2.5 h | 95[b] |
| 24 | | 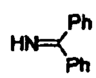 | 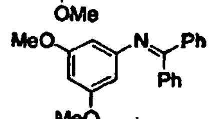 | 1.0/4 | 18 h | >99[b] |
| 25 | 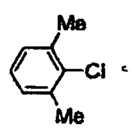 | 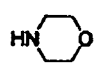 | 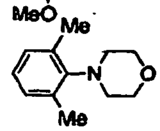 | 1.0/2 | 20 h | 86[d] |
| 26 | | $H_2NBn$ | 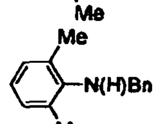 | 1.0 | 24 h | 86[d] |
| 27 | | 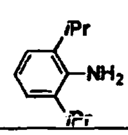 | 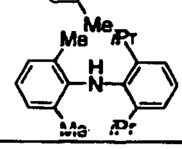 | 4.0 | 20 h | 73[b] |

(a) Reaction conditions: 1.0 equiv aryl halide, 1.2 equiv amine, 1.4 equiv NaO*t*Bu, cat. $Pd(OAc)_2$, cat 3 (2L/Pd), toluene (2mL/mmol halide), 80 °C. Reaction times have not been minimized. (b) $Pd_2(dba)_3$ used in place of $Pd(OAc)_2$; (c) The reaction was conducted at 100 °C; (d) The reaction was conducted at 110 °C.

**Figure 6**

Amination of Aryl Chlorides at Low Catalyst Loading Using Ligand 3[a]

| Entry | Halide | Amine | Product | %Pd | Rxn Time | Yield |
|---|---|---|---|---|---|---|
| 1 | 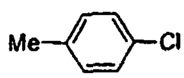 | 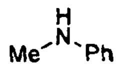 | 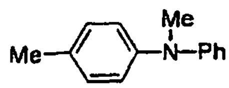 | 0.05 | 22 h | 95[b] |
| 2 | 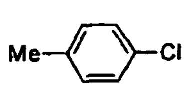 | 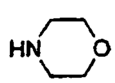 | 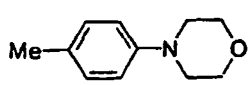 | 0.05/4 | 19 h | 89[b] |
| 3 | 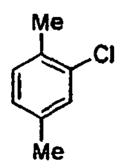 | 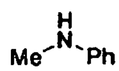 | 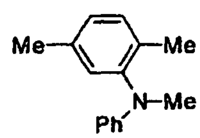 | 0.05 | 22 h | 95 |
| 4 | 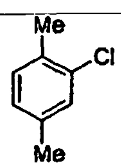 | 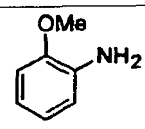 | 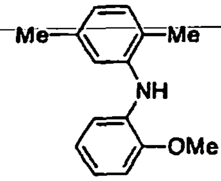 | 0.05 | 14 h | 97[c] |

(a) Reaction conditions: 1.0 equiv aryl chloride, 1.2 equiv amine, 1.4 equiv NaO*t*Bu, 0.025 mol % $Pd_2(dba)_3$, 0.1 mol % 3, toluene (1 mL/mmol halide),110 °C. Reaction times have not been minimized. Yields represent isolated yields (average of two or more experiments) of compounds estimated to be >95 % pure as judged by $^1H$ NMR and GC analysis (known compounds) and combustion analysis (new compounds); (b) The reaction was conducted at 100 °C; (c) The reaction was conducted at 80 °C.

## Figure 7

Palladium-catalyzed amination of chloropyridines using ligand 3[a]

| Entry | Halide | Amine | Product | mol % Pd | Rxn Time | Yield (%) |
|---|---|---|---|---|---|---|
| 1 | 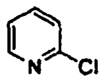 | $H_2NBn$ | 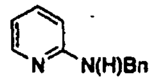 | 0.5 | 3.5h | 89 (GC)[b] |
| 2 | | 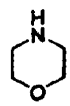 | 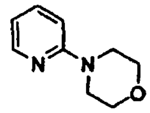 | 0.5/4 | 4h | 95[b] |
| 3 | 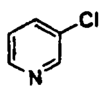 | 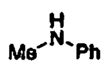 | 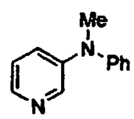 | 1.0/4<br>1.0/2 | 22h<br>22h | 97<br>94 |
| 4 | | $H_2NBn$ | 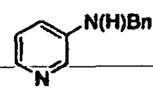 | 1.0 | 22h | 86 |
| 5 | | *n*-Hexyl$NH_2$ (3 eq) | 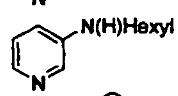 | 1.0/2 | 22h | 76[c] |
| 6 | | 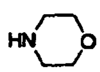 | 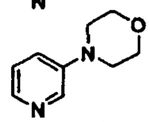 | 1.0 | 22h | 70 |
| 7 | | $Bu_2NH$ | 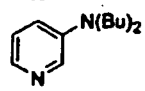 | 1.0/2 | 22h | 77 |

(a) Reaction condintions: 1.0 equiv chloropyridine, 1.2 equiv amine, 1.4 equiv NaO*t*Bu, cat $Pd(OAc)_2$, cat 3, toluene (2 mL/mmol halide), 110 °C; (b) reaction conducted at 100 °C; (c) reaction conducted using 3.0 equiv of amine.

## Figure 8

Palladium-catalyzed amination of functionalized aryl chlorides[a]

| Entry | Halide | Amine | Product | mol % Pd | Rxn Time | Yield (%) |
|---|---|---|---|---|---|---|
| 1 | 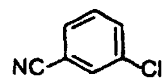 | $H_2NBn$ | 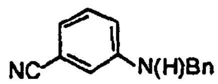 | 2.0/4 | 25 h | 80[b] |
| 2 | | 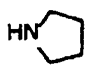 | 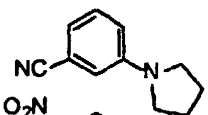 | 1.0/4 | 22 h | 81 |
| 3 | 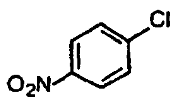 | 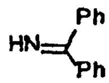 | 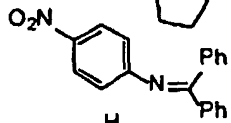 | 1.0/2 | 16 h | 82[c] |
| 4 | | 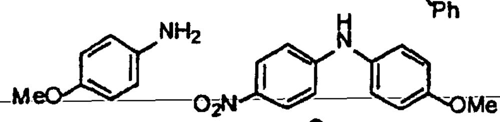 | | 1.0 | 17 h | 91[c] |
| 5 | 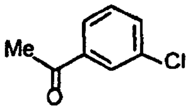 | 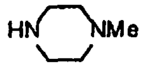 | 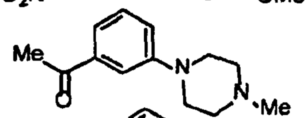 | 1.0/2 | 21 h | 77[c] |
| 6 | | 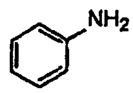 | 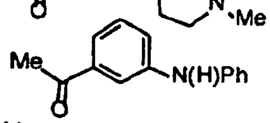 | 1.0/4<br>1.0/2 | 22 h<br>22 h | 81[c]<br>84[c] |
| 7 | 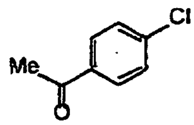 | 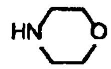 | 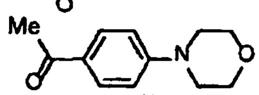 | 1.0 | 21 h | 91[d] |
| 8 | | 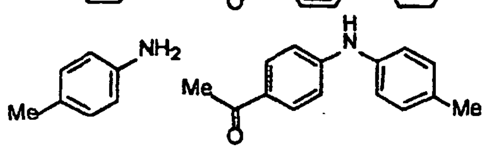 | | 1.0/4 | 18 h | 95[d] |
| 9 | 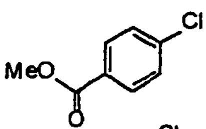 | 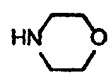 | 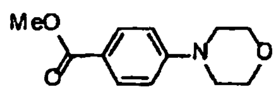 | 1.0/4 | 18 h | 90 [c,d] |
| 10 | 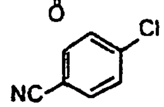 | n-HexylNH$_2$ | 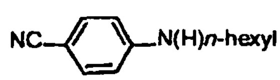 | 1.0 | 40 h | 72 [c,e,g] |
| 11 | 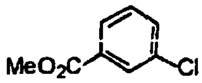 | 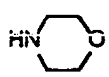 | 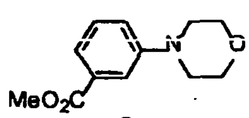 | 1.0/4 | 20 h | 93[c] |
| 12 | | 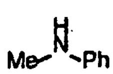 | 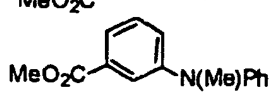 | 1.0/4<br>1.0/2 | 20 h<br>20 h | 88 [b,c]<br>83[c] |

(a) Reaction conditions: 1.0 equiv aryl chloride, 1.2 equiv amine, 1.4 equiv $K_3PO_4$, cat $Pd(OAc)_2$, cat 3, DME (2mL/mmol halide), 100 C; (b) The reaction proceeded to 99% conversion; (c) $Pd_2(dba)_3$ used in place of $Pd(OAc)_2$; (d) The reaction was conducted at 80 C; (e) The reaction was conducted at 110 C; (f) The reaction was conducted with 3.0 equiv amine; (g) NaOtBu used in place of $K_3PO_4$.

# Figure 9

Palladium-catalyzed amination of aryl bromides using ligand 3[a]

| Entry | Halide | Amine | Product | mol % Pd | Rxn Time | Yield (%) |
|---|---|---|---|---|---|---|
| 1 | 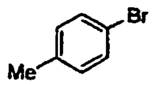 | 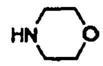 | 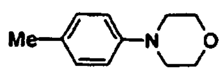 | 0.5 | 20 h | 92 |
| 2 | 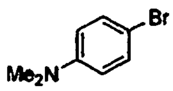 | 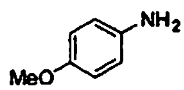 | 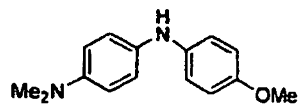 | 0.5 | 2 h | 93[b] |
| 3 | 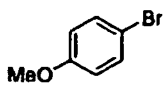 | 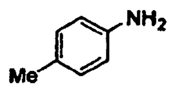 | 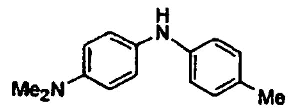 | 0.5 | 2.5h | 90[b] |
| 4 | 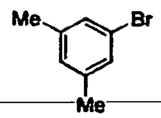 | 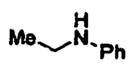 | 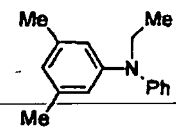 | 0.5 | 3h | 82 |
| 5 | | 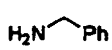 | 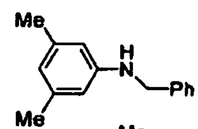 | 0.5 | 42h | 82[b] |
| 6 | 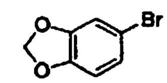 | 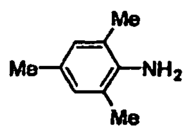 | 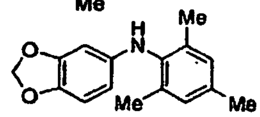 | 0.5 | 2h | 97[b] |
| 7 | 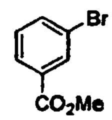 | 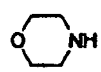 | 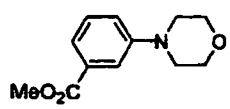 | 1.0/2 | 20h | 86[b,c] |
| 8 | 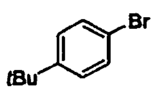 | 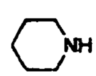 | 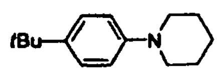 | 0.5/4 | 17h | 85[b] |
| 9 | | $HN(n\text{-}Bu)_2$ | 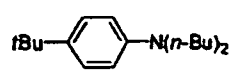 | 0.5/4<br>0.5/5 | 20h<br>18h | 85[b]<br>91[b] |
| 10 | 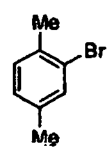 | 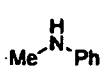 | 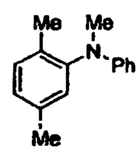 | 0.5/4 | 16h | 75[b] |

(a) Reaction conditions: 1.0 equivl aryl bromide, 1.2 equiv amine, 1.4 equiv NaO*t*Bu, cat $Pd(OAc)_2$, cat ligand 3, toluene (2 mL/mmol halide), 80 °C; (b) $Pd_2(dba)_3$ used in place of $Pd(OAc)_2$; (c) reaction conducted at 100 °C; (d) $K_3PO_4$ used in place of NaO*t*Bu.

**Figure 10**

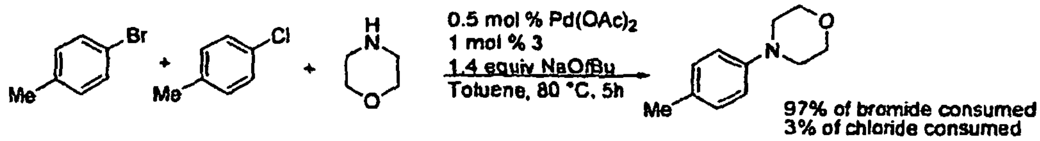


1.0 equiv 1.0 equiv 1.2 equiv

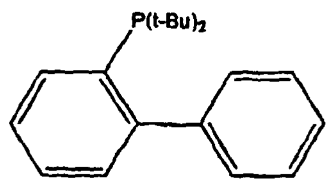

## Figure 11

Palladium-catalyzed amination of aryl triflates using ligand 3[a]

| Entry | Halide | Amine | Product | mol % Pd | Rxn Time | Yield (%) |
|---|---|---|---|---|---|---|
| 1 | 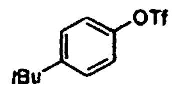 | 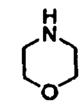 | 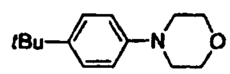 | 1.0 | 26h | 92 |
| 2 | 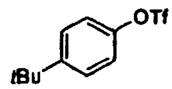 | 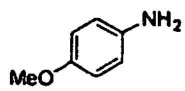 | 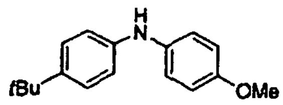 | 1.0 | 16h | 87[b] |
| 3 | 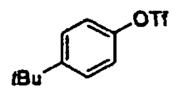 | $H_2NBn$ | 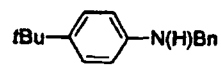 | 1.0 | 19h | 68[d] |
| 4 | 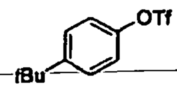 | HN(*n*-Bu)$_2$ | 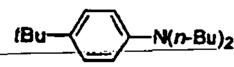 | 1.0 | 19h | 71[c,d] |
| 5 | 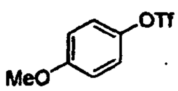 | 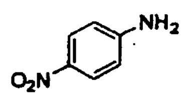 | 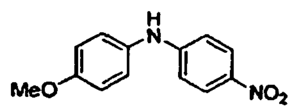 | 1.0 | 16h | 75[b] |
| 6 | 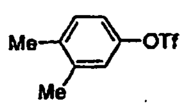 | 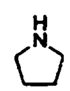 | 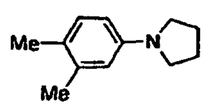 | 1.0 | 25h | 89[b,c] |
| 7 | 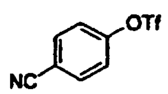 | 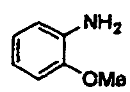 | 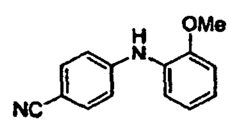 | 1.0 | 1.5h | 85[b] |
| 8 | 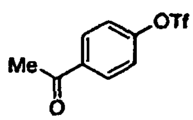 | 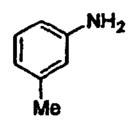 | 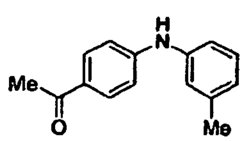 | 1.0 | 1.5h | 94[b] |
| 9 | 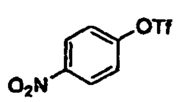 | 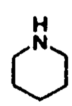 | 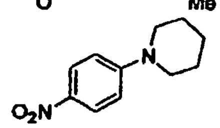 | 1.0 | 17h | 82[b,c] |

(a) Reaction conditions: 1.0 equivl aryl bromide, 1.2 equiv amine, 1.4 equiv$K_3PO_4$, cat $Pd(OAc)_2$, cat ligand 3, toluene (2 mL/mmol halide), 80 °C; (b) $Pd_2(dba)_3$ used in place of $Pd(OAc)_2$; (c) Ligand 4 used in place of ligand 3; (d) NaO*t*Bu used in place of $K_3PO_4$.

**Figure 12**

**One-pot synthesis of triarylamines (cf. Examples 73-82).**

| Entry | Amine | ArBr | ArCl | Product | mol % Pd | Yield(%) |
|---|---|---|---|---|---|---|
| 1 | 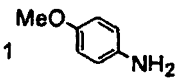 | 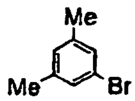 | 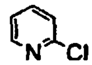 | 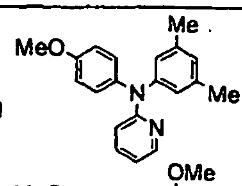 | 3 | 94 |
| 2 | 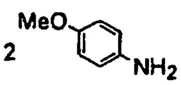 | 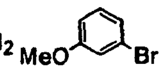 | 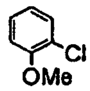 | 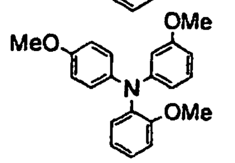 | 3 | 74 |
| ~~3~~ | 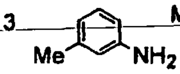 | 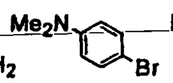 | 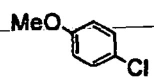 | 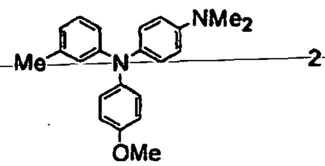 | ~~2~~ | ~~91~~ |
| 4 | 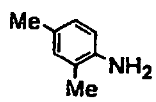 | 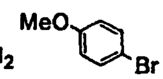 | 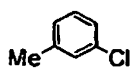 | 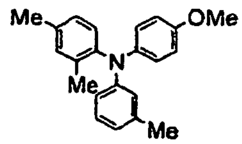 | 1 | 82 |
| 5 | 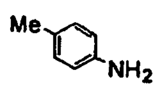 | 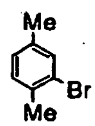 | 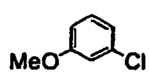 | 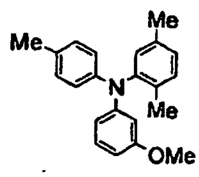 | 1 | 90 |
| 6 | 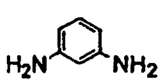 | 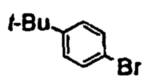 | 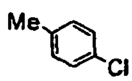 | 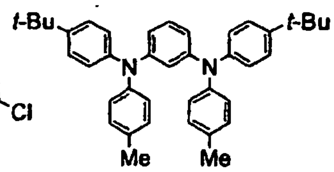 | 3 | 95 |
| 7 | 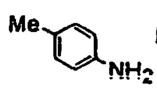 | 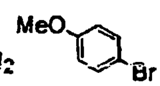 | 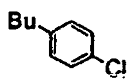 | 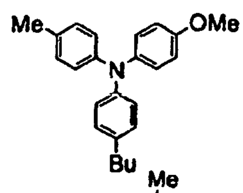 | 1 | 85 |
| 8 | 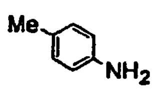 | 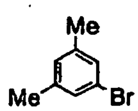 | 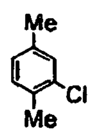 | 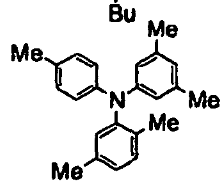 | 1 | 84 |

**Figure 13**

## One-pot synthesis of triarylamines (cf. Examples 84-86).

| Entry | Amine | ArBr | ArCl | Product | Yield(%) |
|---|---|---|---|---|---|
| 9 | 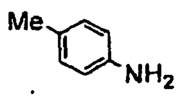 | 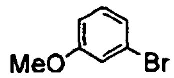 | 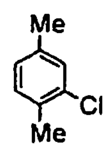 | 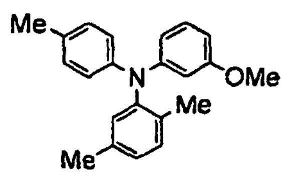 | 89 |
| 10 | 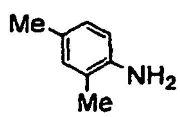 | — | 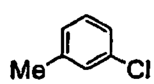 | 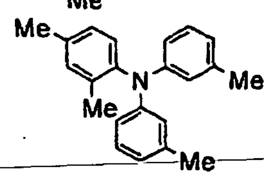 | 87 |
| 11 | 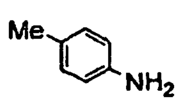 | 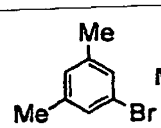 | 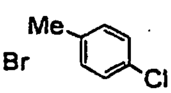 | 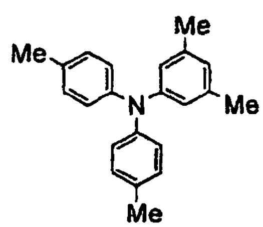 | 89 |
| 12 | 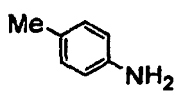 | — | 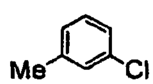 | 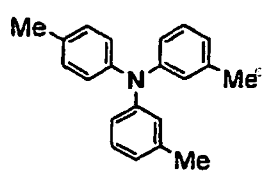 | 68 |

**Figure 14**
See Example 99.

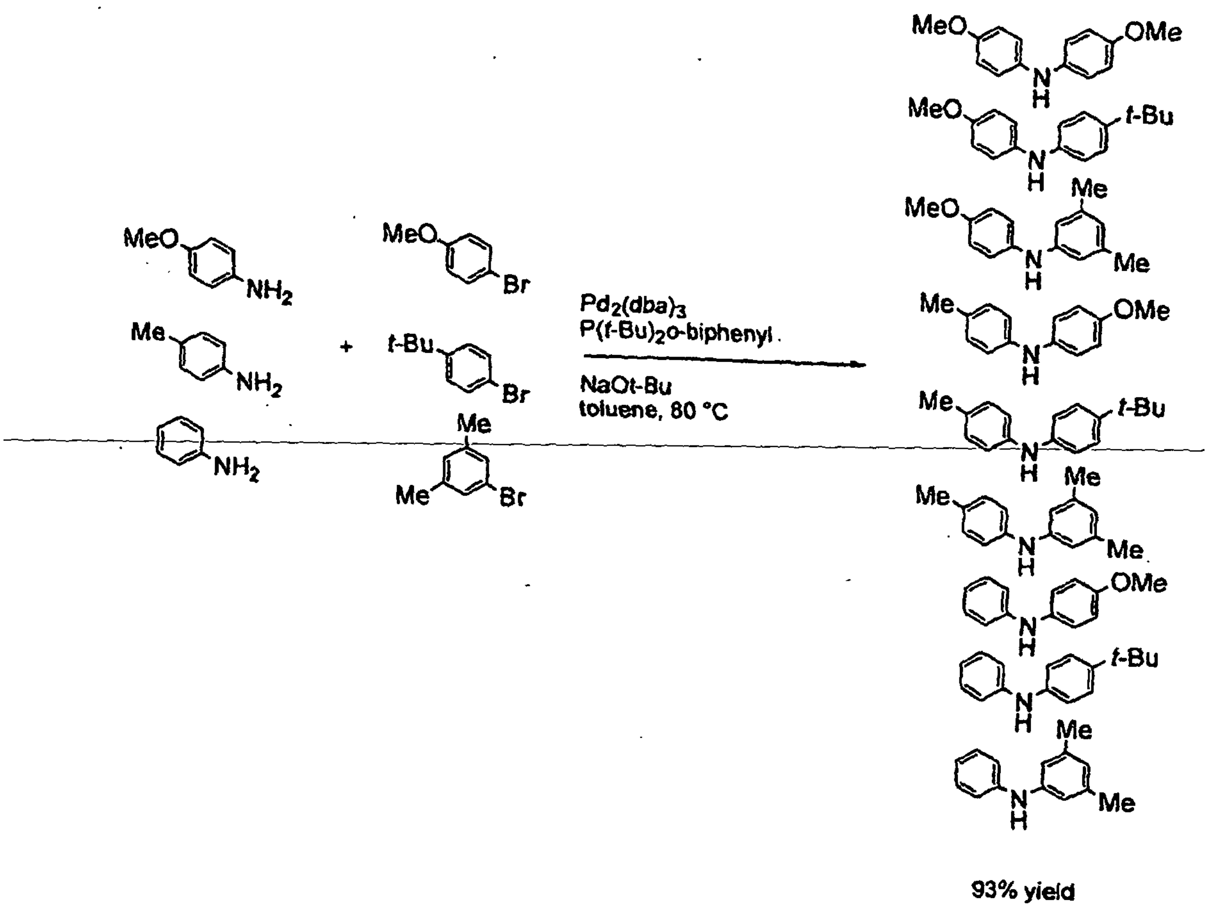

**Figure 15**
See Examples 87-89.

Arylation of diphenylamine.

| Entry | Amine | ArX | Product | mol % Pd | Yield(%) |
|---|---|---|---|---|---|
| 1 | 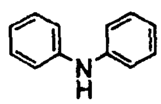 | 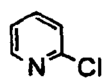 | 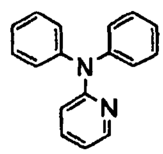 | 1 | 99 |
| 2 | 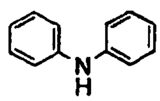 | 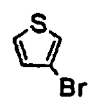 | 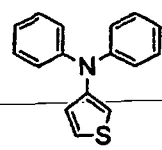 | 2 | 72 (GC yield) |
| 3 | 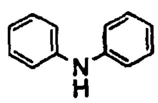 | 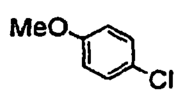 | 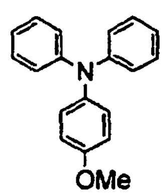 | 0.05 | 88 |

**Figure 16**
See Examples 97 and 98.

Arylations of cyclopropylamine.

| Entry | Amine | ArBr | Products | Yield(%) |
|---|---|---|---|---|
| 1 | 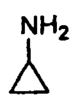 | 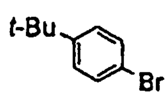 | 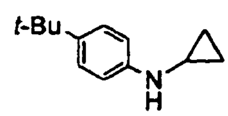 | 79 |
| 2 | 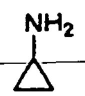 | 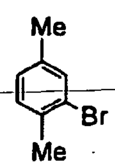 | 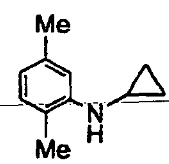 | 86 |

**Figure 17**
<u>See</u> Examples 90-96.

*N*-Arylations of indole.

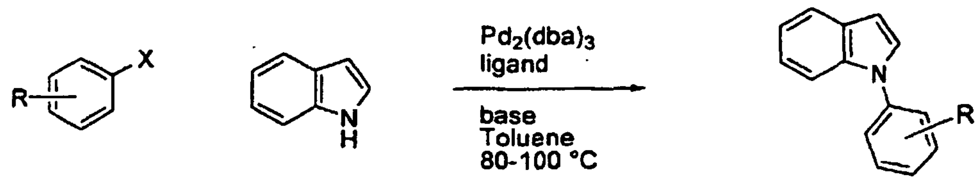


| Entry | X | R | mol% Pd | ligand | yield |
|---|---|---|---|---|---|
| 1 | Cl | 4-Me | 1 | 1 | 92 |
| 2 | Br | 4-(*t*-Bu) | 1 | 1 | 90 |
| 3 | Br | 4-OMe | 1 | 1 | 84 |
| 4 | Br | $4\text{-NMe}_2$ | 1 | 1 | 90 |
| 5 | Br | 4-F | 1 | 1 | 63 |
| 6 | Br | 3-pyridyl | 3 | 1 | 78 |
| 7 | Br | $4\text{-CO}_2\text{Me}$ | 1 | 1 | 83 |

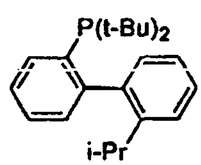

**1**

**Figure 18**

Certain Ligands

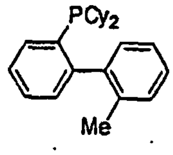
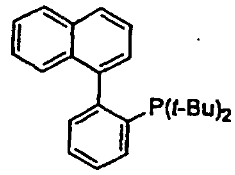
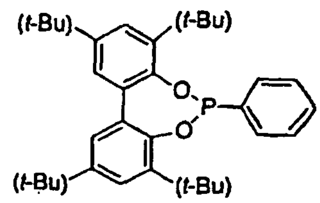
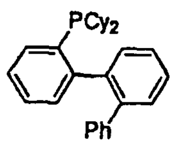
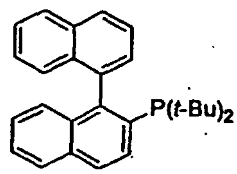
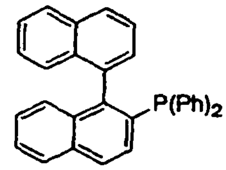
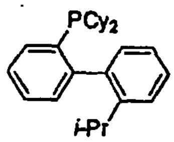

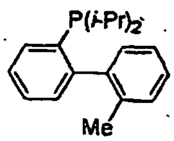
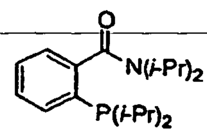
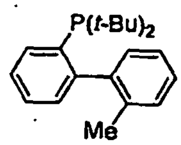
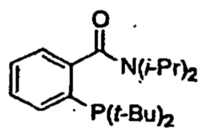
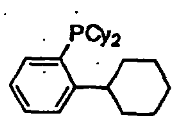
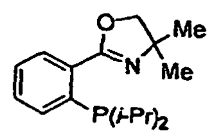
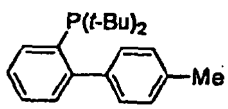
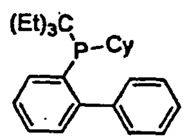
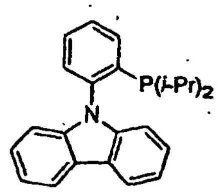
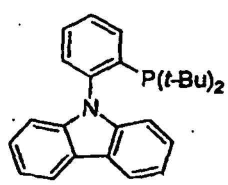
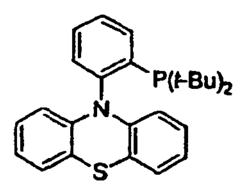
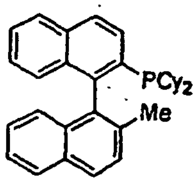
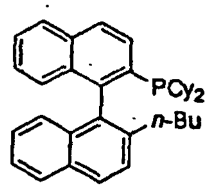
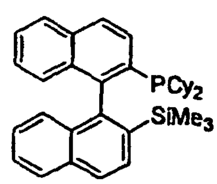

**Figure 19**

Certain Ligands

| | | |
|---|---|---|
| 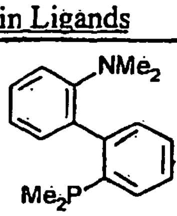 | 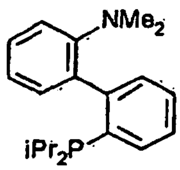 | 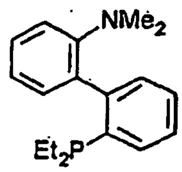 |
| 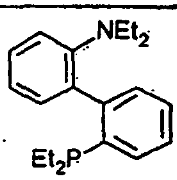 | 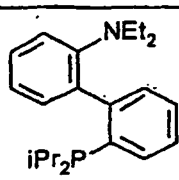 | 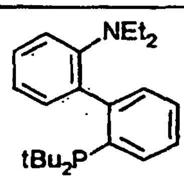 |
| 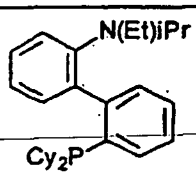 | 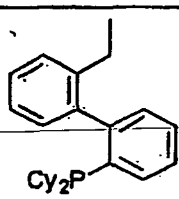 | 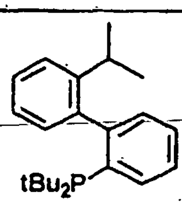 |
| 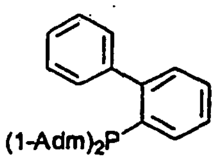 | 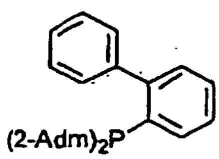 | 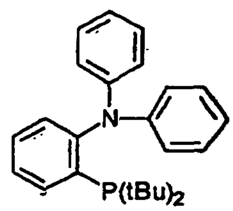 |
| 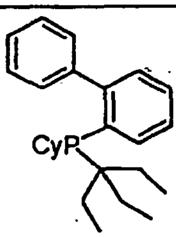 | 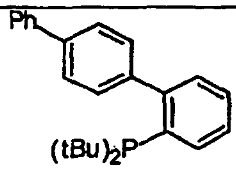 | 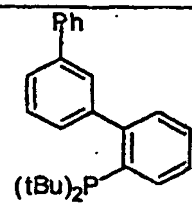 |

## Figure 20

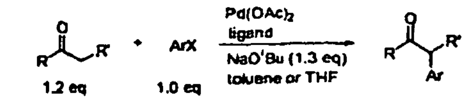


| entry | ketone | arylhalide | mol % Pd/ligand | solvent, temperature | time (h) | yield |
|---|---|---|---|---|---|---|
| 1[a,f] | 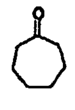 | 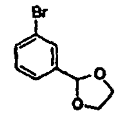 | 0.1% $Pd(OAc)_2$ 0.2 % 1 | toluene 45 °C | 20 | 77 % |
| | | | 0.1% $Pd(OAc)_2$ 0.2 % 4 | toluene 60 °C | 18.5 | 68 % |
| 2 | 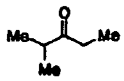 | 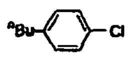 | 0.1% $Pd(OAc)_2$ 0.2 % 1 | toluene 80 °C | 16 | 81 %[b] |
| 3 | 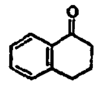 | 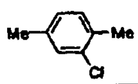 | 0.1% $Pd(OAc)_2$ 0.2 % 1 | toluene 80 °C | 5 | 89 % |
| 4[g] | 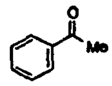 | 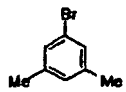 | 0.1% $Pd(OAc)_2$ 0.2 % 2 | toluene 50 °C | 24 | 76 %[c] |
| 5 | 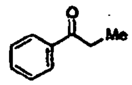 | 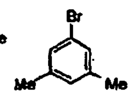 | 0.1% $Pd(OAc)_2$ 0.2 % 3 | toluene 80 °C | 3 | 89 % |
| 6 | 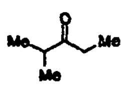 | 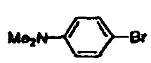 | 0.1% $Pd(OAc)_2$ 0.2 % 1 | THF 83 °C | 22 | 69 %[d] |
| 7[a,f] | 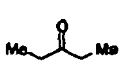 | 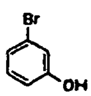 | 0.1% $Pd(OAc)_2$ 0.2 % 1 | THF 70 °C | 24 | 68 % |
| 8 | 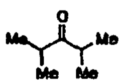 | 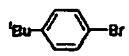 | 0.5% $Pd(OAc)_2$ 1.0 % 1 | toluene 85 °C | 24 | 61 % |
| 9[e] | 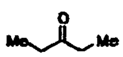 | 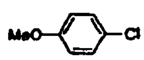 | 0.1% $Pd(OAc)_2$ 0.2 % 1 | toluene 70 °C | 24 | 75 % |
| 10 | 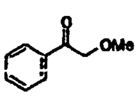 | 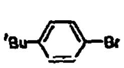 | 0.5 % $Pd(OAc)_2$ 1 % 1 | THF 70 °C | 17 | 85 % |
| 11 | 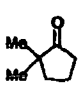 | 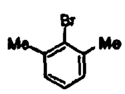 | 0.5 % $Pd(OAc)_2$ 1 % 1 | toluene 70 °C | 22.5 | 67 % |
| 12 | 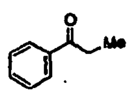 | 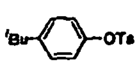 | 5 % $Pd(OAc)_2$ 5 % 5 | toluene 80 °C | 40 | 45 % |

*Ligands*

1 = 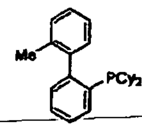

2 = 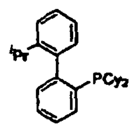

3 = 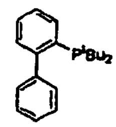

4 = 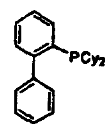

5 = 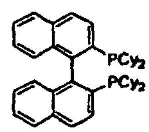

[a] 2.0 equivalents of ketone were used. [b] Isolated as a 20:1 mixture of regioisomers. [c] Only 95 % of the arylhalide was consumed. [d] Only 92 % of the arylhalide was consumed. [e] NaHDMS (1.1 eq) was used as the base. [f] 2.2 eq. of NaOtBu were used. [g] 2.5 eq. of NaOtBu were used.

**Figure 21**

Arylation of Substituted Indoles

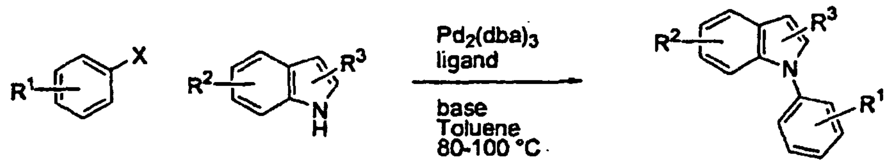


| Entry | aryl halide | indole substituent | mol% Pd | base | ligand | yield |
|---|---|---|---|---|---|---|
| 1 | 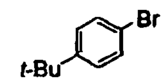 | 2-Ph | 1 | NaO*t*-Bu | 4 | 91 |
| 2 | 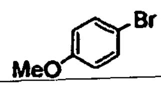 | 2-Ph | 1 | NaO*t*-Bu | 4 | 65 |
| 3 | 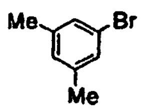 | 2-(4-fluorophenyl) | 1 | NaO*t*-Bu | 4 | 74 |
| 4 | 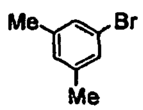 | $2,3\text{-}Me_2$ | 1 | NaO*t*-Bu | 4 | 97 |
| 5 | | | 2 | NaO*t*-Bu | 6 | 90 |
| 6 | 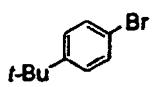 | $2,3,7\text{-}Me_3$ | 4 | NaO*t*-Bu | 4 | 51 |
| 7 | 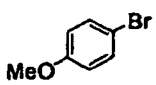 | $3\text{-}CH_2CO_2Et$ | 1 | $K_3PO_4$ | 2 | 87 |
| 8 | 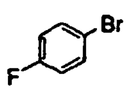 | 5-F | 3 | NaO*t*-Bu | 2 | 85 |
| 9 | 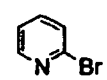 | 7-Et | 1 | NaO*t*-Bu | 4 | 94 |
| 10 | 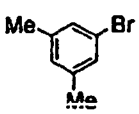 | 7-Et | 1 | NaO*t*-Bu | 4 | 53 |

Ligands:

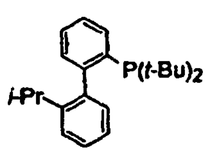
2

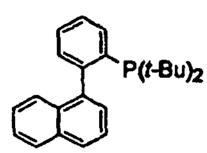
4

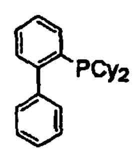
5

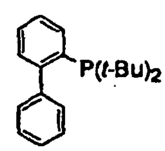
6

**Figure 22**

Arylations of Indole

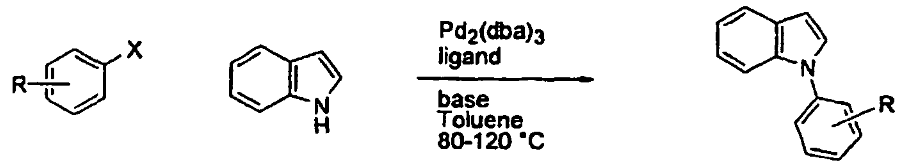


| Entry | X | R | mol% Pd | ligand | temp. | yield |
|---|---|---|---|---|---|---|
| 1 | Cl | 4-Me | 5 | **1** | 100 °C | 87 |
| 2 | | | 1 | **2** | 100 °C | 92 |
| 3 | Br | 4-(*t*-Bu) | 1 | **2** | 80 °C | 90 |
| 4 | I | 4-Me | 2.5 | **1** | 100 °C | 90 |
| 5 | OTf | 4-OMe | 5 | **1** | 100 °C | 87 |
| 6 | Br | 4-OMe | 1 | **2** | 80 °C | 84 |
| 7 | Br | 4-NMe$_2$ | 1 | **2** | 80 °C | 90 |
| 8 | Br | 4-F | 1 | **2** | 80 °C | 63 |
| 9 | Br | 3-pyridyl | 3 | **2** | 100 °C | 78 |
| 10 | Br | 2,5-Me$_2$ | 5 | **3** | 120 °C | 81 |
| 11 | Br | 4-CO$_2$Me | 1 | **2** | 100 °C | 83 |
| 12 | Cl | 4-CO$_2$Me | 2 | **2** | 100 °C | 61 |
| 13 | OTf | 2-CO$_2$Me | 3 | **3** | 100 °C | 78 |

Ligands:

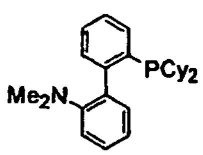
**1**

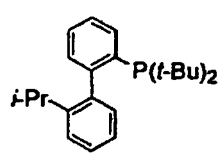
**2**

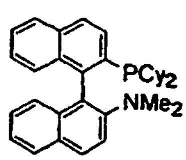
**3**

**Figure 23**

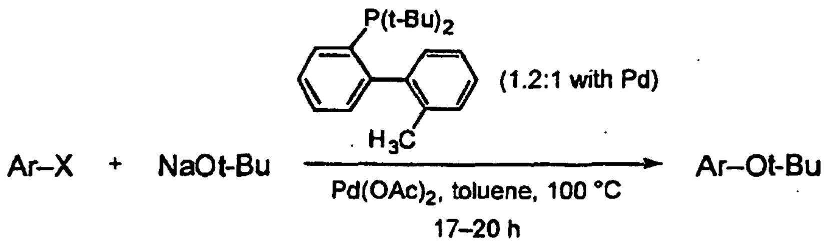


| Ar–X | mol% Pd | Isolated Yield |
|---|---|---|
| 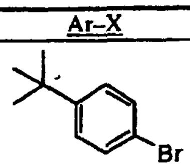 | 1.0[a] | 90% |
| 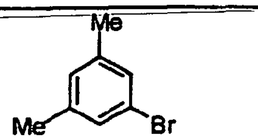 | 1.0 | 88% |
| 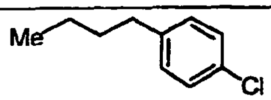 | 2.5 | 92% |
| 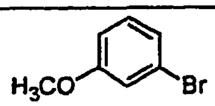 | 1.0[a] | 84% |
| 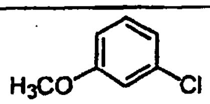 | 1.0[a] | 84% |

[a]These reactions perform equally well with only 0.5 mol% Pd. [b]The corresponding diaryl ether is a significant byproduct in this reaction. [c]Reaction was performed with dicyclohexylphosphine ligand A.

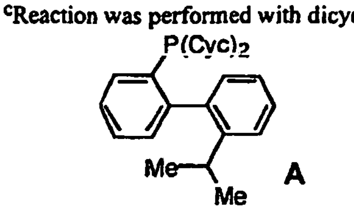

A

**Figure 24**

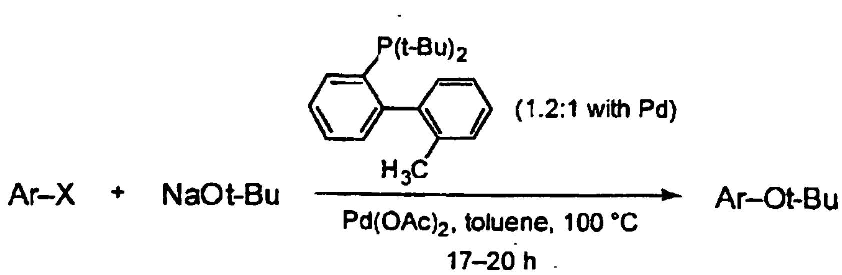


| Ar–X | mol% Pd | Isolated Yield |
|---|---|---|
| 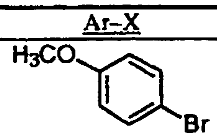 | 2.5 | 84% |
| 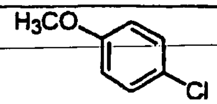 | 2.5 | 81% |
| 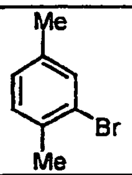 | 2.5<br>2.5[c] | 63%[b]<br>86% |
| 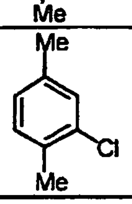 | 2.5<br>2.5[c] | 76%[b]<br>88% |

[a]These reactions perform equally well with only 0.5 mol% Pd. [b]The corresponding diaryl ether is a significant byproduct in this reaction. [c]Reaction was performed with dicyclohexylphosphine ligand A.

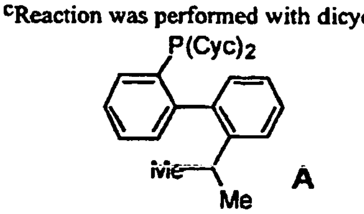

**A**

**Figure 25**

ligand (1.25:1 with Pd), $Pd_2(dba)_3$

Ar–X + NaOTBS → Ar–OTBS

toluene, 120 °C

17–20 h

| Ar–X | Ligand | mol% Pd | Isolated Yield |
|---|---|---|---|
| 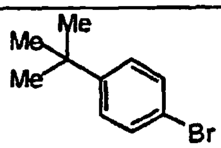 | 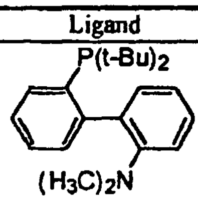 | 5.0 | 81% |
| 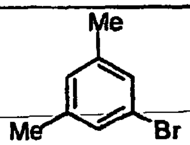 | 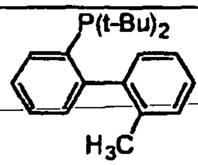 | 5.0 | 63%[a] |
| 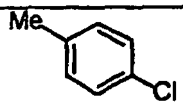 | 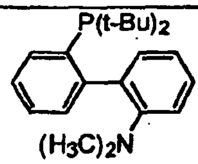 | 5.0 | 58%[a] |
| 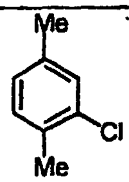 | 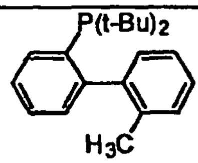 | 5.0[b] | 65% |

[a]Reported is a GC yield. [b]$Pd(OAc)_2$ was used as the palladium source.

**Figure 26**
See Examples 164-171.

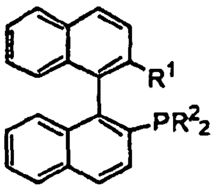

1(a) $R^1 = NMe_2$, $R^2 = Cy$
1(b) $R^1 = Me$, $R^2 = Cy$
1(c) $R^1 = Bu$, $R^2 = Cy$

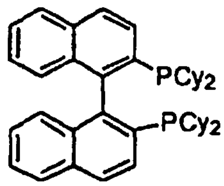

1

2

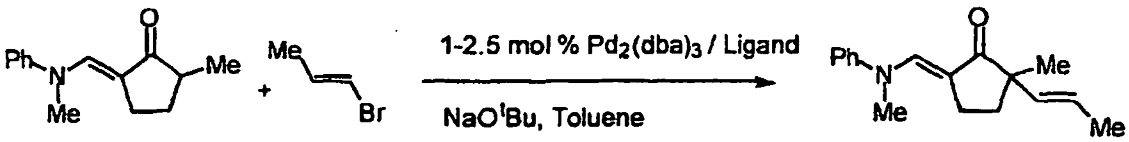


| entry | ligand | temperature | Pd (%) | yld (%) | ee (%) |
|---|---|---|---|---|---|
| 1 | 1(a) | r.t. | 1 | 89 | 90 |
| 2 | 1(a) | 50°C | 0.1 | 86 | 70 |
| 3 | 1(a) | 0°C | 5 | 85 | 94 |
| 4 | 1(a) | -20°C | 5 | 47 | 96 |
| 5 | 1(b) | rt | 5 | 88 | 65 |
| 6 | 1(b) | 0°C | 5 | 81 | 79 |
| 7 | 1(c) | 0°C | 5 | 71 | 82 |
| 8 | 2 | 50°C | 5 | 56 | 35 |

## Figure 27 α-*Arylation of 2-Methyl-1-Tetralone–Ligand Effects*

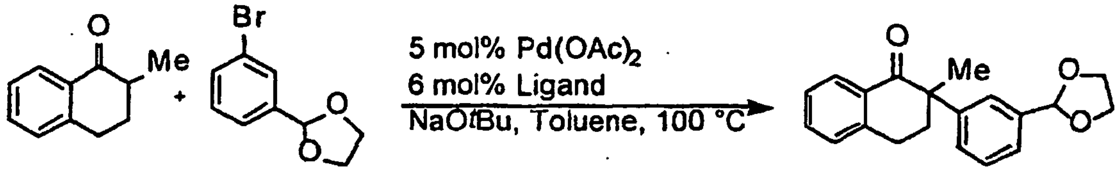


| Ligand | Yield | ee |
|---|---|---|
| (S)-BINAP | 69 | 84 |
| (R)-QUINAP | 43 | 84 |
| (R)-MeO-BIPHEP | 59 | 85 |
| (S)-BIPHEMP | 39 | 82 |
| (R,R)-NORPHOS | 38 | 40 |
| (R)-MOP | 19 | 8 |
| (R)-PPF-OMe | 38 | 1 |
| (R)-(S)-JOSIPHOS | 38 | 2 |
| (S)-Et-DUPHOS[a] | 8 | 12 |
| (S)-Tol-BINAP[b] | 26 | 30 |

(a) Reaction run with 10 mol% $Pd_2(dba)_3$, 24 mol% (S)-Tol-BINAP, NaHMDS as base. (b) Reaction run with 2.5 mol% $Pd_2(dba)_3$, 6 mol% (S)-Et-DUPHOS, NaHMDS as base.

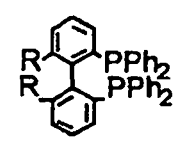

BIPHEMP (R=Me)
MeO-BIPHEP (R=OMe)

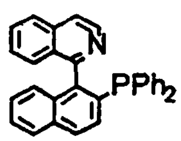

QUINAP

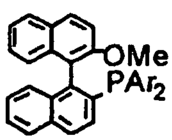

MOP

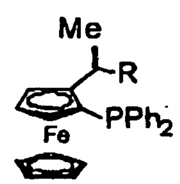

PPF-OMe (R=OMe)
JOSIPHOS (R=PCy2)

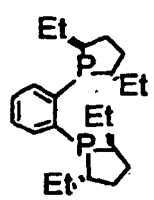

Et-DUPHOS

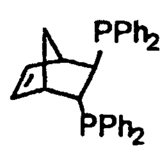

NORPHOS

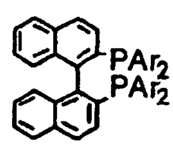

BINAP (Ar=Ph)
Tol-BINAP (Ar=*p*-tolyl)

**Figure 28.** α-Arylations of Ketones in the Absence of a Phosphine Ligand

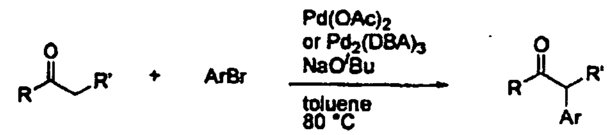


| entry | Ketone | Aryl Bromide | Pd Source (mol %) | (% yield) |
|---|---|---|---|---|
| 1 | 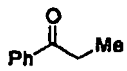 | 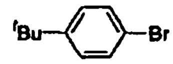 | $Pd_2(DBA)_3$ (1.5 mol %) | 55% |
| 2 | 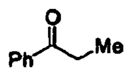 | 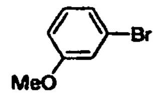 | $Pd_2(DBA)_3$ (1.5 mol %) | 54% |
| 3 | 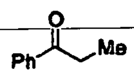 | 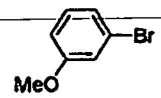 | $Pd(OAc)_2$ (1 mol %) | 79% |
| 4 | 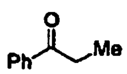 | 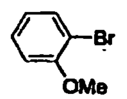 | $Pd_2(DBA)_3$ (1.5 mol %) | 46% |
| 5 | 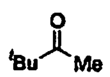 | 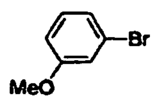 | $Pd_2(DBA)_3$ (1.5 mol %) | 48% |
| 6 | 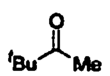 | 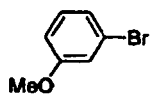 | $Pd(OAc)_2$ (1 mol %) | 71% |
| 7 | 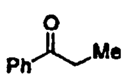 | 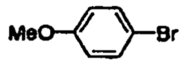 | $Pd(OAc)_2$ (1 mol %) | 79% |
| 8 | 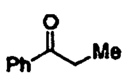 | 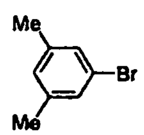 | $Pd(OAc)_2$ (1 mol %) | 83% |
| 9 | 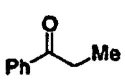 | 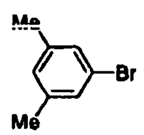 | $Pd_2(DBA)_3$ (0.5 mol %) | 79% |
| 10 | 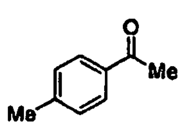 | 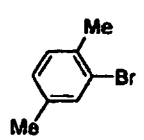 | $Pd(OAc)_2$ (1.0 mol %) | 64% |